(19) Europäisches Patentamt
European Patent Office
Office européen des brevets

(11) EP 2 463 384 A2

(12) **EUROPEAN PATENT APPLICATION**

(43) Date of publication:
**13.06.2012 Bulletin 2012/24**

(51) Int Cl.:
***C12Q 1/68*** *(2006.01)*

(21) Application number: **11192355.3**

(22) Date of filing: **07.12.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.12.2010 US 961596**
**07.12.2010 US 961694**

(71) Applicant: **Medtronic, Inc.**
**Minneapolis, MN 55342 (US)**

(72) Inventors:
- **Soykan, Orhan**
  **Shoreview, MN 55410 (US)**
- **Nahey, Tara**
  **Minneapolis, MN 55410 (US)**
- **Lande, Jeffrey**
  **Minneapolis, MN 55406 (US)**

(74) Representative: **Grünecker, Kinkeldey, Stockmair & Schwanhäusser**
**Leopoldstrasse 4**
**80802 München (DE)**

# (54) Diagnostic kits, genetic markers and methods for SCD or SCA therapy selection

(57) Variations in certain genomic sequences useful as genetic markers of Sudden Cardiac Death ("SCD") or Sudden Cardiac Arrest ("SCA") risk are described. Novel diagnostic kits, DNA microarrays, and methods employing these genetic markers are used in assessing the risk of SCD or SCA. Methods of distinguishing patients having an increased susceptibility to SCD or SCA, through use of these markers, alone or in combination with other markers, are also provided. Further, methods of detecting a polymorphism associated with SCD or SCA are taught.

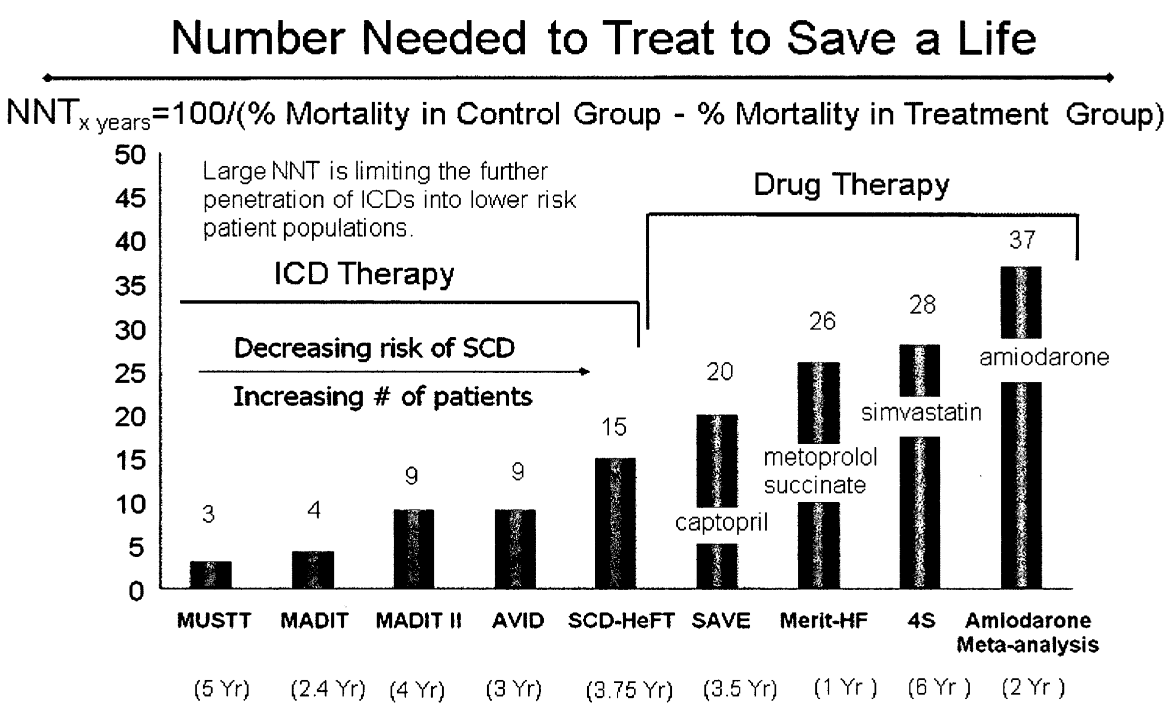


Fig. 1

EP 2 463 384 A2

## Description

### CROSS-REFERENCE TO RELATED APPLICATIONS

This application claims benefit of priority to U.S. Patent Application Serial No. 12/961,596, filed December 7, 2010, and U.S. Patent Application Serial No. 12/961,694, filed December 7, 2010,

### REFERENCE TO SEQUENCE LISTING

This application contains a Sequence Listing submitted as an electronic text file named ST25.txt. The information contained in the Sequence Listing is hereby incorporated by reference.

### BACKGROUND

Implantable cardioverter-defibrillator (ICD) therapy is effective in primary and secondary prevention for patients at high risk of Sudden Cardiac Arrest (SCA). ICDs can effectively terminate life threatening ventricular tachy-arrhythmias, such as ventricular tachycardia ("VT") and ventricular fibrillation ("VF"). For many patients, ICDs are indicated for various cardiac related ailments including myocardial infarction, ischemic heart disease, coronary artery disease, and heart failure. The use of these devices, however, remains low due in part to lack of reliable markers to select patients who are in need of these devices. Left ventricular function, clinical comorbidity, QRS duration, and various electrophysiological testing methods have been proposed as criteria for the screening of patients potentially at high risk for arrhythmic death. But risk stratification remains unsatisfactory because it is mainly performed using a single clinical marker, namely left ventricular ejection fraction. Hence, despite the effectiveness of ICDs in Sudden Cardiac Death ("SCD") or Sudden Cardiac Arrest ("SCA") prevention, many susceptible patients who might benefit from an ICD do not receive one due to a lack of reliable methods for the identification of SCD or SCA. The financial burden and potential risks associated with this therapy make better identification of patients with a propensity towards SCA a desirable goal.

One possible type of genetic maker for improved risk stratification is a Single Nucleotide Polymorphism (SNP). Human beings share 99.9% of their gene sequences. Given the approximate size of the human genome, which is approximately 3 billion base pairs, it is believed that there can be as many as 3 million sequence differences between any two individuals. These base pair differences predominantly show up as polymorphisms, defined as variants that occur at a frequency >1% in the population. If these polymorphisms result from the substitution of one nucleotide for another in the DNA sequence, it is called a single nucleotide polymorphism (SNP). Polymorphisms affecting the coding region of a gene may influence the structure of the protein product, whereas others located within the regulatory sequences (also referred to as the promoter region) of a gene can influence the regulation of expression levels of the protein product. In some cases, these genetic variations may alter phenotypic expression following a change in physiological conditions, such as an ischemic event or the administration of a medication. Diagnostic data from a medical device such as an ICD can be used to obtain information of various diagnostic markers, including information about tachyarrhythmia episodes for the identification of possible genetic markers for SCA.

### SUMMARY OF THE INVENTION

Novel genetic markers and novel diagnostic kits and methods for assessing the risk of Sudden Cardiac Death ("SCD") and Sudden Cardiac Arrest ("SCA") and useful genetic markers thereof are provided. Methods of distinguishing patients having an increased susceptibility to SCD and SCA using these genetic markers, diagnostic kits and methods, including various DNA microarrays, through use of the genetic markers, alone or in combination with other markers, are also provided. The DNA microarrays can be *in situ* synthesized oligonucleotides, randomly or non-randomly assembled bead-based arrays, and mechanically assembled arrays of spotted material where the materials can be an oligonucleotide, a cDNA clone, or a Polymerase Chain Reaction (PCR) amplicon.

Specifically, a diagnostic kit for detecting one or more Sudden Cardiac Arrest (SCA)-associated polymorphisms in a genetic sample having at least one probe or a plurality of probes for assessing the presence of a Single Nucleotide Polymorphism (SNP) in any one of SEQ ID Nos. 1-858 is provided. Preferably, the SNP is selected from the group of SEQ ID Nos. 850-855 and 858. Also provided is a DNA microarray for detecting one or more Sudden Cardiac Arrest (SCA)-associated polymorphisms in a genetic sample made up of at least one probe for assessing the presence of a Single Nucleotide Polymorphism (SNP) in any one of SEQ ID Nos. 1-858, more preferably SEQ ID Nos. 850-855 and 858.

The SNPs in the kits, compositions, and methods of the invention include any one or more selected from the group of SEQ ID Nos. 1-858. The SNPs are preferably selected from the group of SEQ ID Nos. 850-855 and 858. It is also understood that the group of SNPs may further include any of the following groups of SEQ ID Nos.: 850-851, 850-852, 850-853, 850-854, 850-855, 851-852, 851-853, 851-854, 851-855, 851-855 and 858, 852-853, 852-854, 852-855, 852-855 and 858, 853-854, 853-855, 853-855 and 858, 854-855, 854-855 and 858, 855 and 858. It is also understood that the group of SNPs may further include any of the following groups of SEQ ID Nos.: 850 and 852; 850 and 853; 850 and 854; 850 and 855; 850 and 858; 851 and 853; 851 and 854; 851 and 855; 851 and 858; 852 and 854; 852 and 855; 852 and 858; 853 and 855; 853 and 858; 854 and 858. It is also understood that the group of SNPs may further include any of the following groups of SEQ ID Nos.: 850 and 852-853; 850 and 853-854; 850 and 854-855; 850, 855 and 858; 851 and 853-854; 851 and 854-855; 851, 855 and 858; 852 and 854-855; 852, 855 and 858; 853, 855 and 858. It is also understood that the group of SNPs may further include any of the following groups of SEQ ID Nos.: 850 and 852-854; 850 and 853-855; 850, 854-855 and 858; 851 and 853-855; 851, 854-855 and 858; 852, 854-855 and 858; 850 and 852-855; 850, 853-855 and 858; 851, 853-855 and 858; 850, 852-855 and 858.

A system for detecting one or more Single Nucleotide Polymorphisms (SNPs) associated with SCA is also provided. The system comprises a computer system having a computer processor programmed with an algorithm and one or more genetic databases in communication the programmed processor. The system imputes p-values for one or more known SNPs that are detected from one or more genetic samples obtained from a patient. Additionally or alternatively, the system imputes p-values for one or more known SNPs obtained from the one or more genetic databases. A p-value of less than a specified range indicates association with SCA.

Novel genetic markers useful in assessing the risk of Sudden Cardiac Death ("SCD") and Sudden Cardiac Arrest ("SCA") are provided. Methods of distinguishing patients having an increased susceptibility to SCD, or SCA, through use of these markers, alone or in combination with other markers, are also provided. Further, methods of assessing the need for an ICD in a patient are taught. Specifically, an isolated nucleic acid molecule is contemplated that is useful to predict SCD, or SCA risk, and Single Nucleotide Polymorphisms ("SNPs") selected from the group of SEQ ID Nos. 1-858 that can be used in the diagnosis, distinguishing, and detection thereof.

Provided are isolated nucleotides, to be used in the diagnostic kits and methods that are useful to predict SCD, or SCA risk, which are complementary to any one of SEQ ID Nos. 1-849 where the complement is between 3 to 101 nucleotides in length and overlaps a position 51 in any of the SEQ ID Nos. 1-849, which represents a SNP. The invention also contemplates isolated nucleotides useful to predict SCD or SCA risk, complementary to any one of SEQ ID Nos. 850-858, where the complement is between 3 to 101 nucleotides in length and overlaps at position 26 or 27 in any of SEQ ID Nos. 850-858, each of which represent a SNP. An amplified nucleotide is further contemplated for use in the diagnostic kits containing a SNP embodied in any one of SEQ ID Nos. 1-849, or a complement thereof, overlapping position 51, wherein the amplified nucleotide is between 3 and 101 base pairs in length. An amplified nucleotide is contemplated containing a SNP embodied in any one of SEQ ID Nos. 850-858, or a complement thereof, overlapping position 26 or 27, wherein the amplified nucleotide is between 3 and 101 base pairs in length. The lower limit of the number of nucleotides in the isolated nucleotides, and complements thereof, can range from about 3 base pairs from position 50 to 52 in any one of SEQ ID Nos. 1-849 such that the SNP at position 51 is flanked on either the 5' and 3' side by a single base pair, to any number of base pairs flanking the 5' and 3' side of the SNP sufficient to adequately identify, or result in hybridization. The lower limit of the number of nucleotides in the isolated nucleotides, and complements thereof, can range from about 3 base pairs from position 26 to 27 in any one of SEQ ID Nos. 850-858 such that the SNP at position 26 or 27 is flanked on either the 5' and 3' side by a single base pair, to any number of base pairs flanking the 5' and 3' side of the SNP sufficient to adequately identify, or result in hybridization. This lower limit of nucleotides can be from about 3 to 99 base pairs, the optimal length being determinable by a person of ordinary skill in the art. For example, the isolated nucleotides or complements thereof, can be from about 5 to 101 nucleotides in length, or from about 7 to 101, or from about 9 to 101, or from about 15 to 101, or from about 20 to 101, or from about 25 to 101, or from about 30 to 101, or from about 40 to 101, or from about 50 to 101, or from about 60 to 101, or from about 70 to 101, or from about 80 to 101, or from about 90 to 101, or from about 99 to 101 nucleotides, so long as position 51 in any of SEQ ID Nos. 1-849 and position 26 or 27 of SEQ ID Nos. 850-858 are overlapped. Preferred primer lengths can be from 25 to 35, 18 to 30, and 17 to 24 nucleotides.

The nucleotide lengths can be described by n for the lower bound, and (n+i) for the upper bound for $n = \{x \in \mathbb{Z} \mid 3 \leq x \leq 101\}$ and $i = \{y \in \mathbb{Z} \mid 0 \leq y \leq (101 - n)\}$. For example, the isolated nucleotides or complements thereof, can be for n = 3, for every $i = \{y \in \mathbb{Z} \mid 0 \leq y \leq (98)\}$ from about 3 to 4 nucleotides in length, or from about 3 to 5, 3 to 6, 3 to 7, 3 to 8, ... , 3 to 99, 3 to 100, 3 to 101, where position 51 in any of SEQ ID Nos. 1-849 is overlapped, or where positions 26 or 27 in any of SEQ ID Nos. 850-858 is overlapped. Some preferred primer and nucleotide lengths can be from 25 to 35, 18 to 30, and 17 to 24 nucleotides. Preferred primer lengths can be from 25 to 35, 18 to 30, and 17 to 24 nucleotides. A preferred length is 52 nucleotides with the polymorphism at position 27 for SEQ ID Nos. 850-858. An amplified nucleotide is further contemplated containing a SNP embodied in any one of SEQ ID Nos. 1-4, or a complement thereof, overlapping position 27, wherein the amplified nucleotide is between 3 and 101 base pairs in length described by n for the lower bound, and (*n*+*i*) for the upper bound for

$n = \{x \in \mathbb{Z} \mid 3 \leq x \leq 101\}$ and $i = \{y \in \mathbb{Z} \mid 0 \leq y \leq (101 - n)\}$.

The isolated nucleic acid molecules of the invention may also consist of nucleotide sequences having a SNP that is selected as being associated with SCA using the system of the invention.

A method of distinguishing patients having an increased or decreased susceptibility to SCD or SCA from patients who do not is provided, and a diagnostic kit or method thereof, where at least one SNP is detected at position 51 in any of SEQ ID Nos. 1-858 in a nucleic acid sample from the patients using any of the diagnostic kits described herein. The presence or absence of the SNP can be used to assess increased susceptibility to SCD or SCA.

A method of determining SCA or SCD risk in a patient, and a diagnostic thereof, is contemplated which requires identifying one or more SNP at position 51 in any of SEQ ID Nos. 1-858 in a nucleic acid sample from the patient.

A method for determining whether a patient needs an Implantable Cardio Defibrillator ("ICD"), and a diagnostic thereof is contemplated by identifying one or more SNPs at position 51 in any of SEQ ID Nos. 1-858 in a nucleic acid sample from the patient.

A method of detecting SCA or SCD-associated polymorphisms, and a diagnostic kit or method thereof, is further contemplated by extracting genetic material from a biological sample and screening the genetic material for at least one SNP in any of SEQ ID Nos. I-858, which is at position 51 using any of the diagnostic kits described herein.

Those skilled in the art will recognize that the analysis of the nucleotides present in one or several of the SNP markers in an individual's nucleic acid can be done by any method or technique capable of determining nucleotides present at a polymorphic site. One of skill in the art would also know that the nucleotides present in SNP markers can be determined from either nucleic acid strand or from both strands.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

## BRIEF DESCRIPTION OF THE DRAWINGS

The foregoing and other features and aspects of the present disclosure will be best understood with reference to the following detailed description of a specific embodiment of the disclosure, when read in conjunction with the accompanying drawings, wherein:

Figure 1 depicts increase in the Number Needed to Treat ("NNT") observed for the ICD therapy as devices are implanted in patients with lower risks.

Figure 2 is a flow chart of a MAPP sub-study design. MAPP was a preliminary genetic association study conducted to search for markers of SCA. The study involved collection of blood samples from 240 ICD patients who were then followed for more than 2 years for their arrhythmic outcomes. Resulting data was used for the search of statistical associations between life threatening events and SNPs.

Figure 3 is a statistical plot of Single Nucleotide Polymorphisms ("SNPs").

Figure 4 is a decision tree based on a recursive partitioning algorithm.

Figures 5A and 5B are genomic groupings of MAPP based on the recursive partitioning algorithm.

Figure 6 is a chromosomal plot of 849 SNPs with p=0.1 for both MAPP and an IDEA-VF study. IDEA-VF was a pilot study to demonstrate the feasibility of collecting blood samples from post Myocardial Infarction ("MI") patients to search for genetic markers that indicate the patient risk for SCA. Approximately 100 post-MI patients participated in the study and roughly half of them were ICD patients with life threatening arrhythmias and the rest were patients without ICDs.

Figure 7A represents a listing of SNPs potentially useful as genetic markers based on logical criteria (CART tree).

Figure 7B represents a listing of SNPs potentially useful as genetic markers based on biological criteria (clustering in genome).

Figure 7C represents a listing of SNPs potentially useful as genetic markers based on statistical criteria (min radius).

Figure 8 shows graphically the operation of a genetic screen in conjunction with existing medical tests.

Figure 9 shows 25 SNPs identified as SCD or SCA-associated SNPs having p-values less than 0.0001 from the analysis of the MAPP data.

Figure 10 shows the SNPs identified by the MAPP and IDEA-VF studies associated with risk at SCD.

Figure 11 is a list of rs numbers and corresponding SEQ ID Nos.

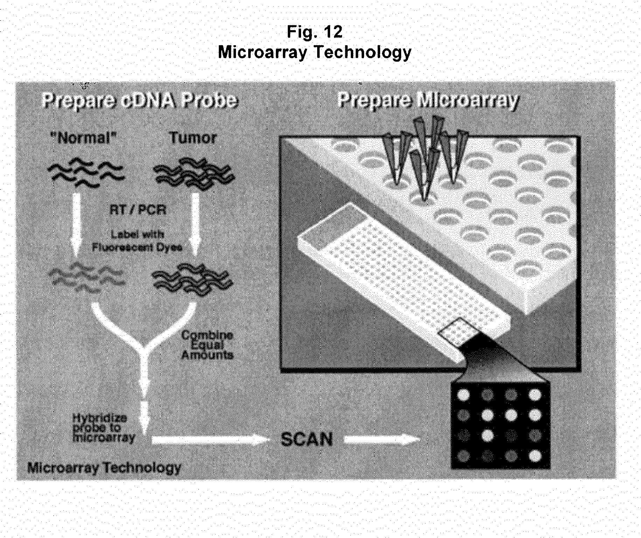

**[0033]** Figure 12 is a schematic of a two-color analysis of SNPs using microarray technology.

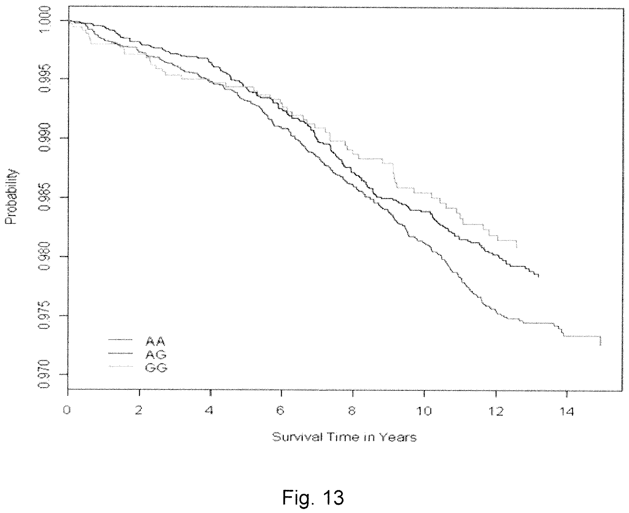

Figure 13 is a Cox proportional hazards model adjusted for age, sex, and race/ethnicity for GPC5. Individuals homozygous for the protective allele (GG) are shown in green, heterozygotes (AG) in blue, and homozygous for the risk allele (AA) are in red.

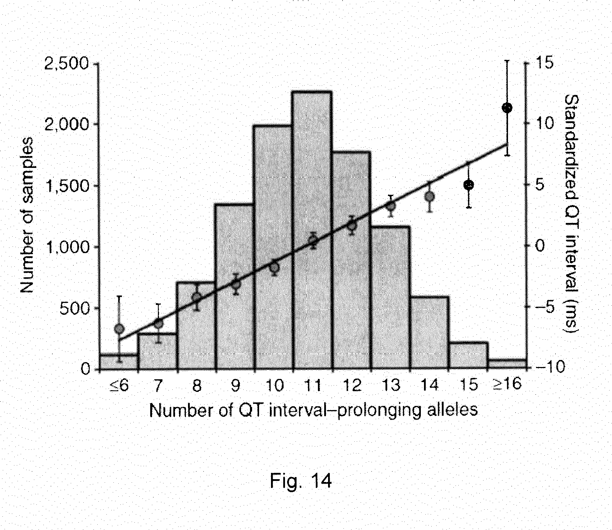

Figure 14 shows individuals classified by counting their number of QT-prolonging alleles in all ten identified markers (max score 20). Dosages for the QT-prolonging allele as calculated by MACH1 were added and then rounded to the nearest integer.

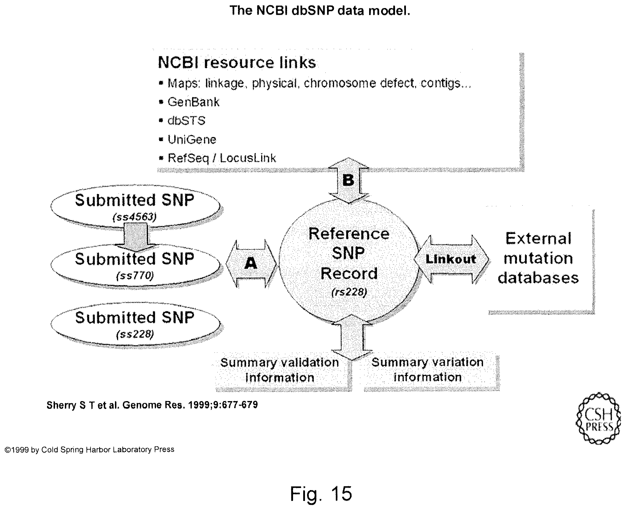

Figure 15 depicts schematics showing the National Center for Biotechnology Information (NCBI) SNP database model.

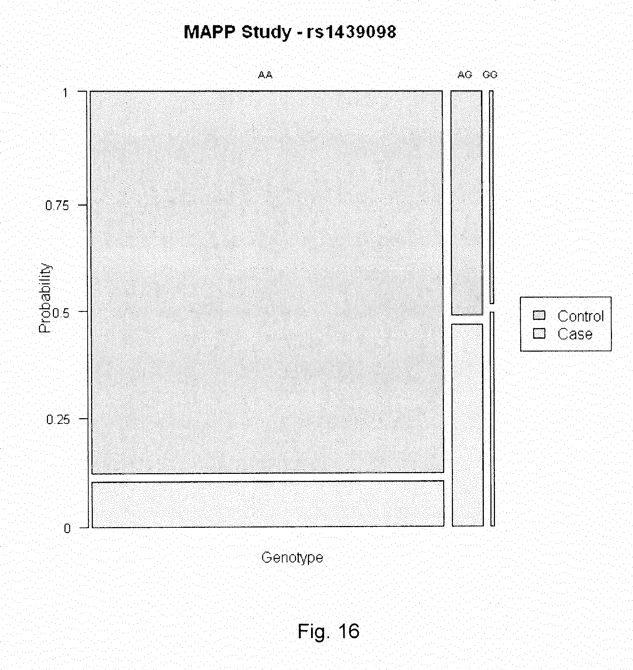

Figure 16 is mosaic plot illustrating the probability of experiencing life threatening arrhythmia (LTA) as a function of allele specific inheritance of the SNP rs1439098. The horizontal width corresponds to the three genotypes and is proportional to their percentage distribution within the study. The vertical axis divides the case and control groups.

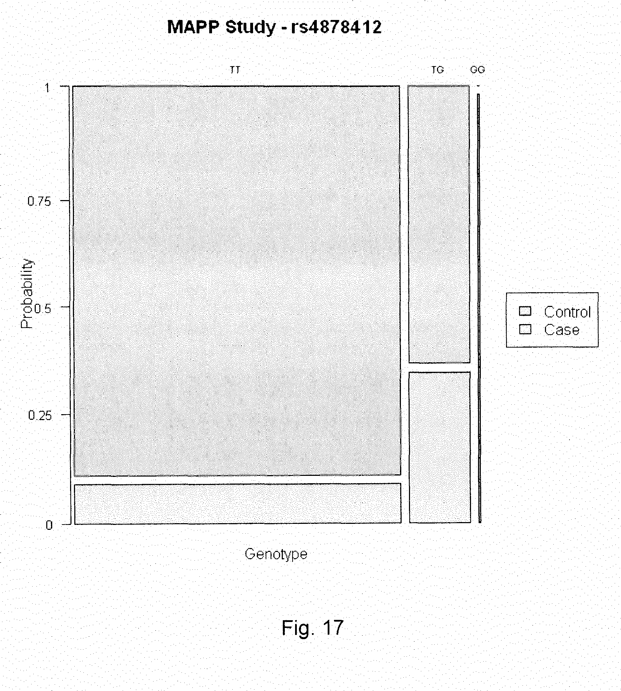

Figure 17 is a mosaic plot illustrating the probability of experiencing LTA as a function of allele specific inheritance of the SNP rs4878412. The horizontal width corresponds to the three genotypes and is proportional to their percentage distribution within the study. The vertical axis divides the case and control groups.

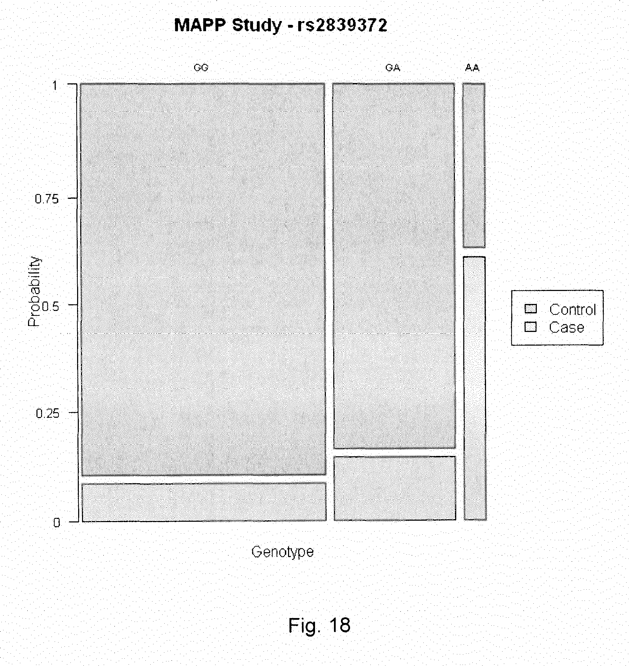

Figure 18 is a mosaic plot illustrating the probability of experiencing LTA as a function of allele specific inheritance of the SNP rs2839372. The horizontal width corresponds to the three genotypes and is proportional to their percentage distribution within the study. The vertical axis divides the case and control groups.

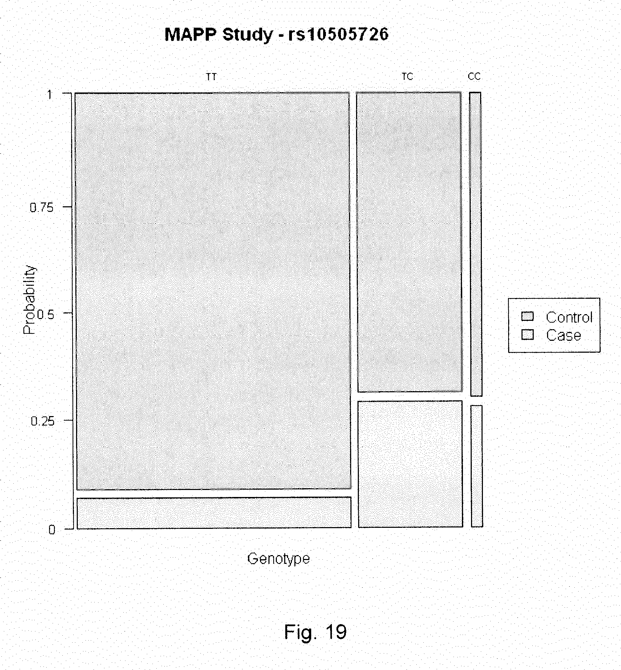

Figure 19 is a mosaic plot illustrating the probability of experiencing LTA as a function of allele specific inheritance of the SNP rs10505726. The horizontal width corresponds to the three genotypes and is proportional to their percentage distribution within the study. The vertical axis divides the case and control groups.

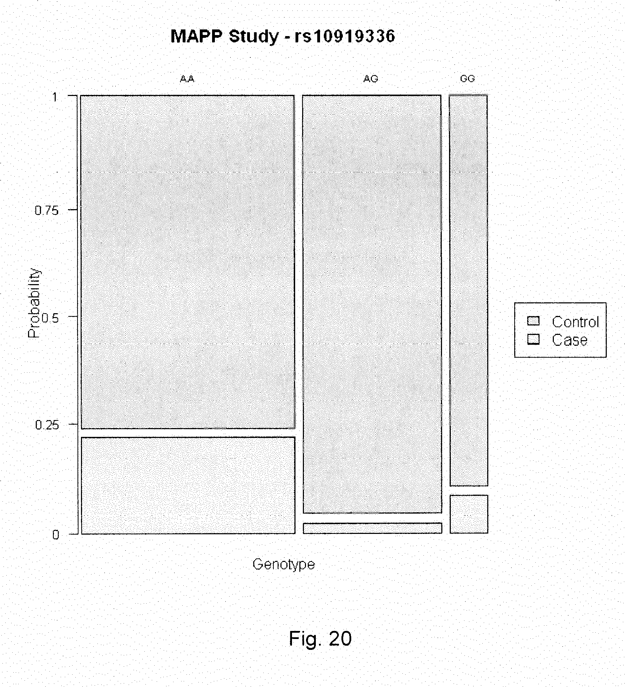

Figure 20 is a mosaic plot illustrating the probability of experiencing LTA as a function of allele specific inheritance of the SNP rs10919336. The horizontal width corresponds to the three genotypes and is proportional to their percentage distribution within the study. The vertical axis divides the case and control groups.

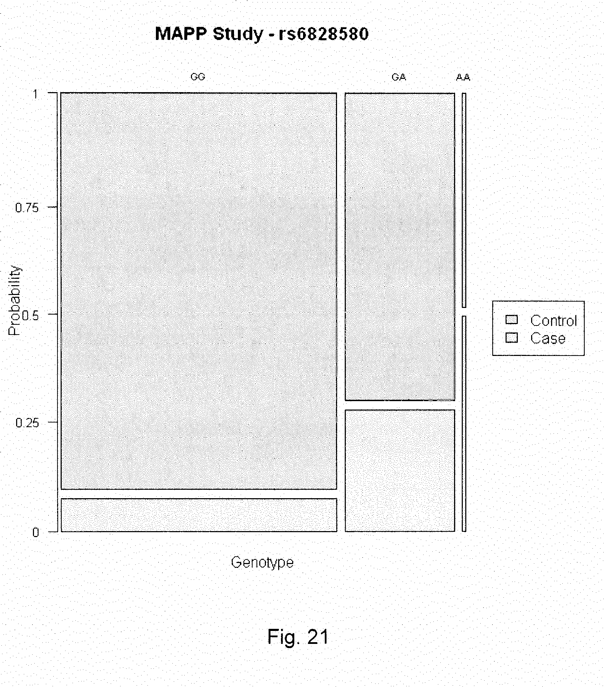

Figure 21 is a mosaic plot illustrating the probability of experiencing LTA as a function of allele specific inheritance of the SNP rs6828580. The horizontal width corresponds to the three genotypes and is proportional to their percentage distribution within the study. The vertical axis divides the case and control groups.

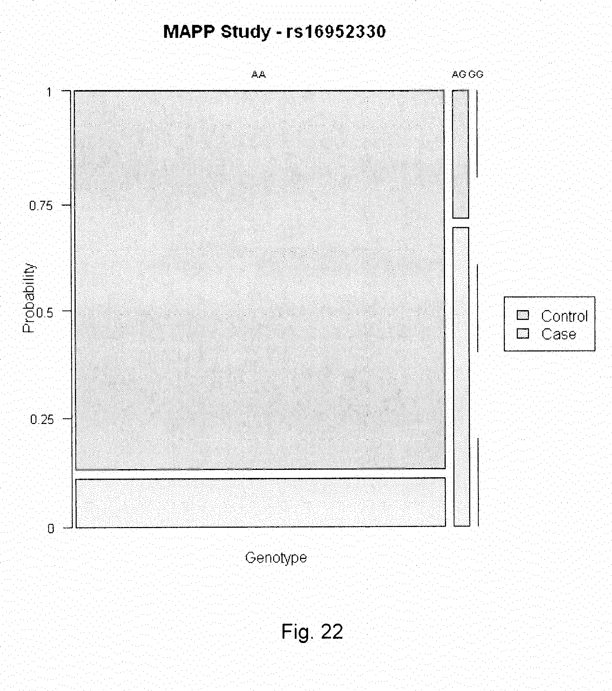

Figure 22 is a mosaic plot illustrating the probability of experiencing LTA as a function of allele specific inheritance of the SNP rs16952330. The horizontal width corresponds to the three genotypes and is proportional to their percentage distribution within the study. The vertical axis divides the case and control groups.

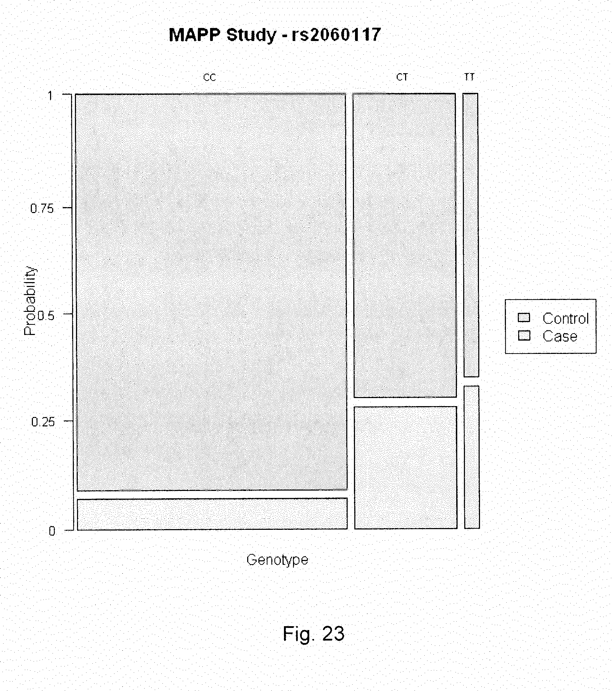

Figure 23 is a mosaic plot illustrating the probability of experiencing LTA as a function of allele specific inheritance of the SNP rs2060117. The horizontal width corresponds to the three genotypes and is proportional to their percentage distribution within the study. The vertical axis divides the case and control groups.

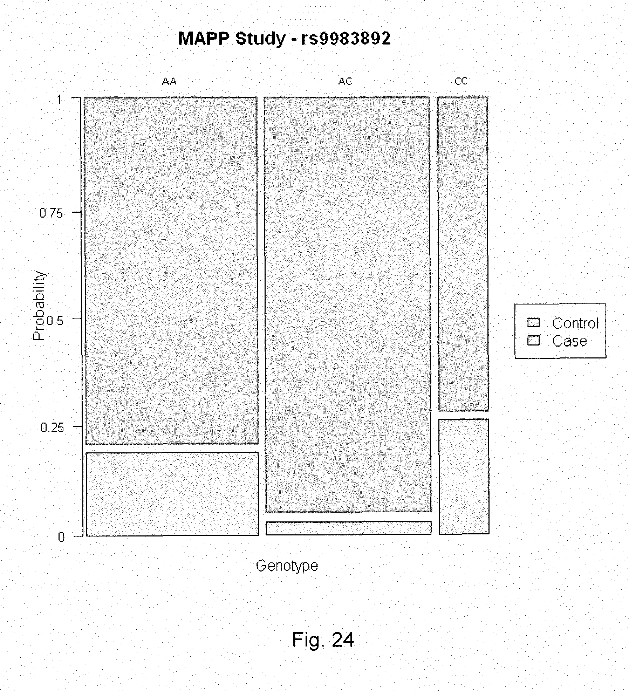

Figure 24 is a mosaic plot illustrating the probability of experiencing LTA as a function of allele specific inheritance of the SNP rs9983892. The horizontal width corresponds to the three genotypes and is proportional to their percentage distribution within the study. The vertical axis divides the case and control groups.

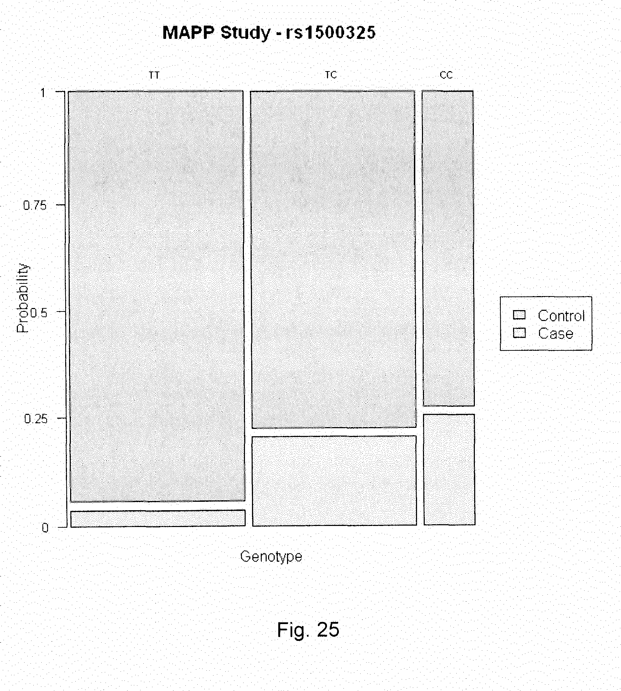

Figure 25 is a mosaic plot illustrating the probability of experiencing LTA as a function of allele specific inheritance of the SNP rs 1500325. The horizontal width corresponds to the three genotypes and is proportional to their percentage distribution within the study. The vertical axis divides the case and control groups.

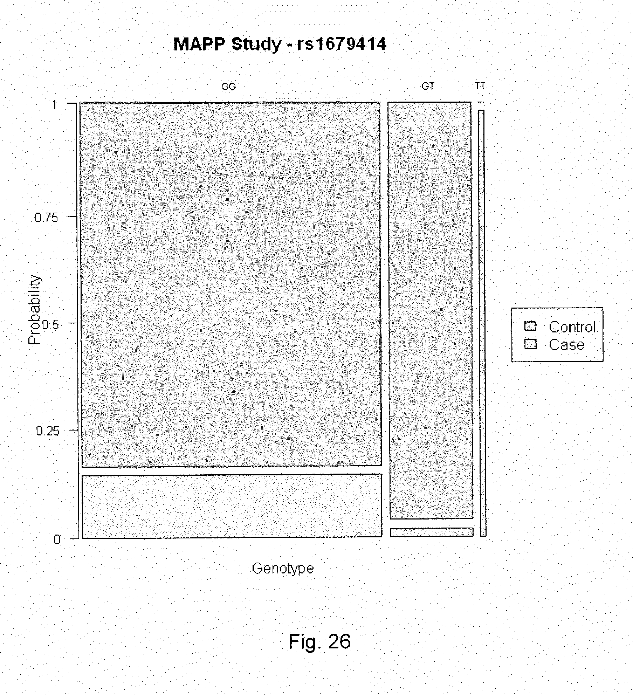

Figure 26 is a mosaic plot illustrating the probability of experiencing LTA as a function of allele specific inheritance of the SNP rs1679414. The horizontal width corresponds to the three genotypes and is proportional to their percentage distribution within the study. The vertical axis divides the case and control groups.

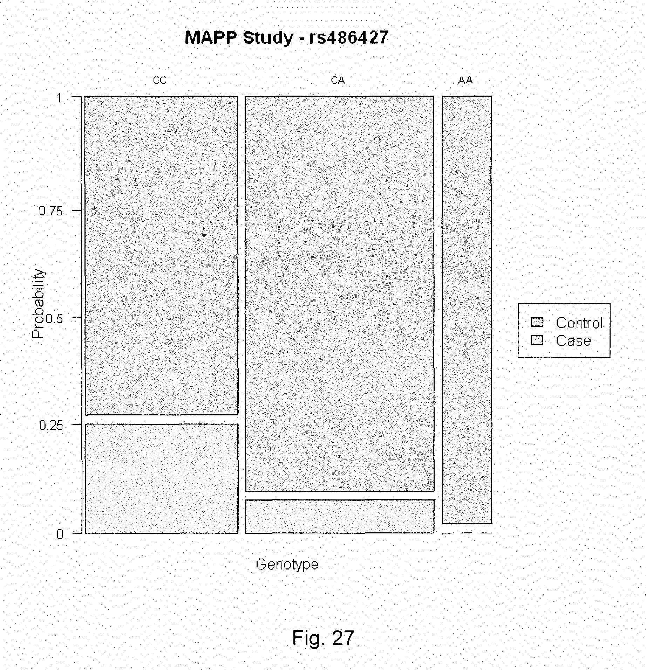

Figure 27 is a mosaic plot illustrating the probability of experiencing LTA as a function of allele specific inheritance of the SNP rs486427. The horizontal width corresponds to the three genotypes and is proportional to their percentage distribution within the study. The vertical axis divides the case and control groups.

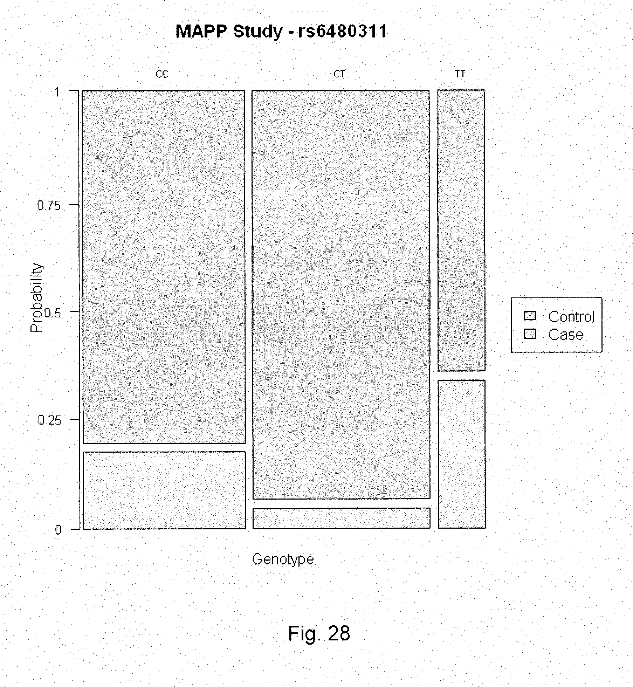

Figure 28 is a mosaic plot illustrating the probability of experiencing LTA as a function of allele specific inheritance of the SNP rs6480311. The horizontal width corresponds to the three genotypes and is proportional to their percentage distribution within the study. The vertical axis divides the case and control groups.

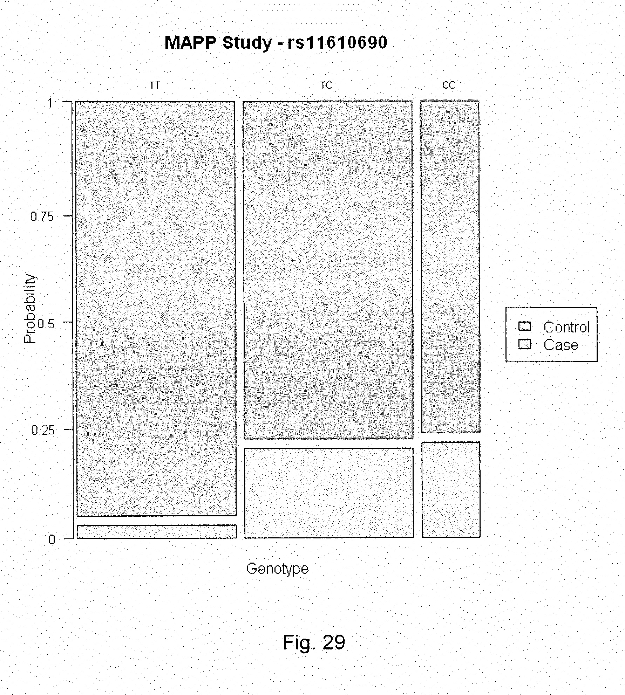

Figure 29 is a mosaic plot illustrating the probability of experiencing LTA as a function of allele specific inheritance of the SNP rs11610690. The horizontal width corresponds to the three genotypes and is proportional to their percentage distribution within the study. The vertical axis divides the case and control groups.

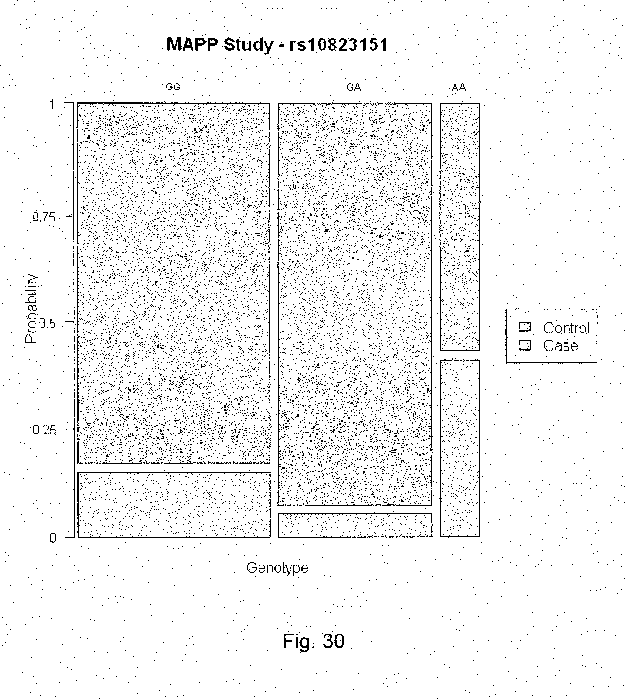

Figure 30 is a mosaic plot illustrating the probability of experiencing LTA as a function of allele specific inheritance of the SNP rs10823151. The horizontal width corresponds to the three genotypes and is proportional to their percentage distribution within the study. The vertical axis divides the case and control groups.

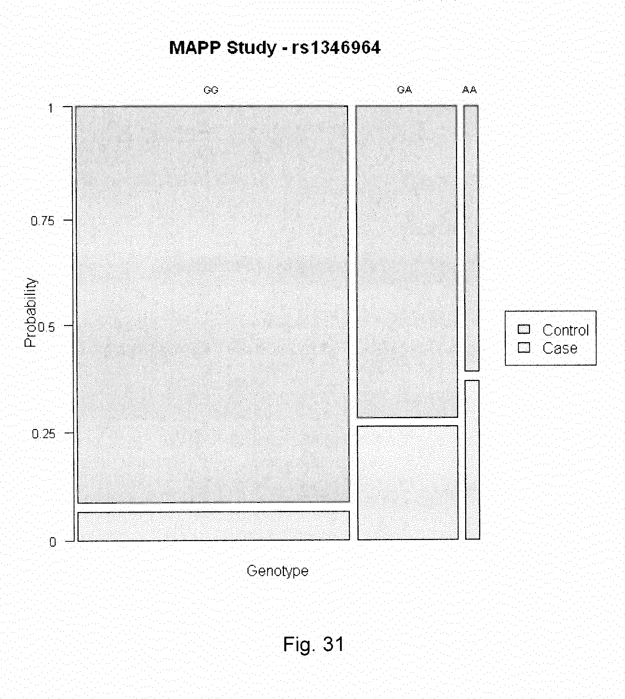

Figure 31 is a mosaic plot illustrating the probability of experiencing LTA as a function of allele specific inheritance of the SNP rs1346964. The horizontal width corresponds to the three genotypes and is proportional to their percentage distribution within the study. The vertical axis divides the case and control groups.

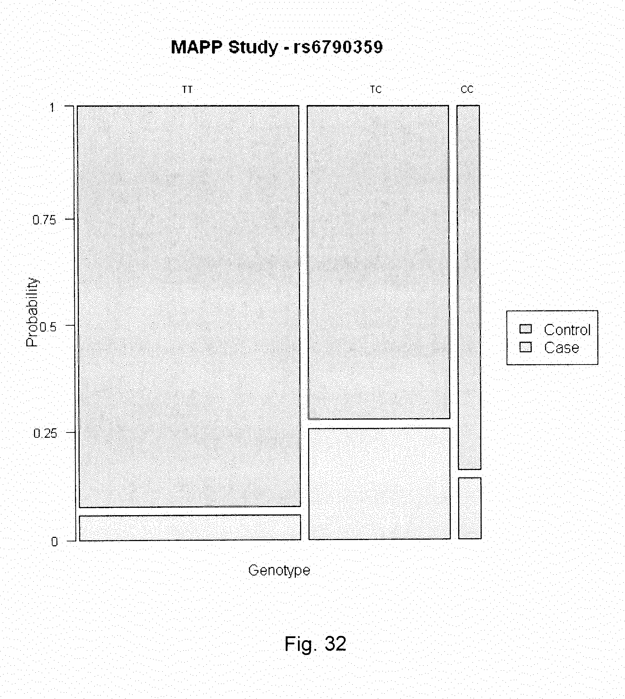

Figure 32 is a mosaic plot illustrating the probability of experiencing LTA as a function of allele specific inheritance of the SNP rs6790359. The horizontal width corresponds to the three genotypes and is proportional to their percentage distribution within the study. The vertical axis divides the case and control groups.

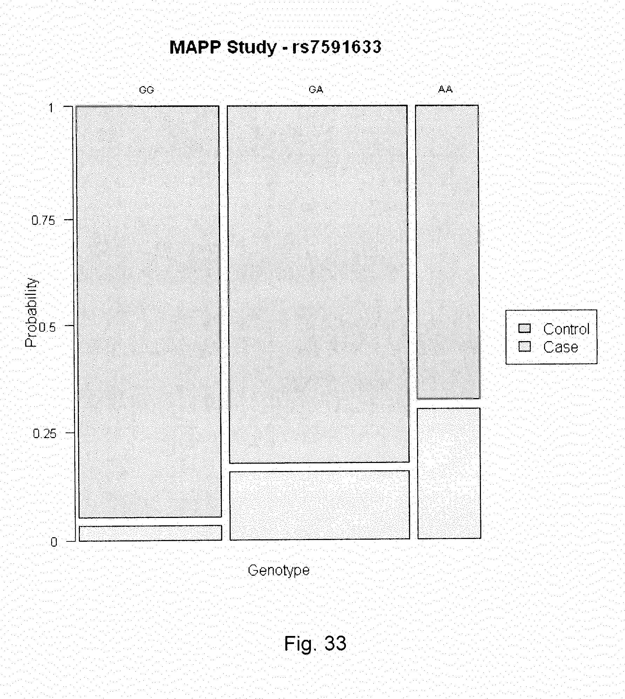

Figure 33 is a mosaic plot illustrating the probability of experiencing LTA as a function of allele specific inheritance of the SNP rs7591633. The horizontal width corresponds to the three genotypes and is proportional to their percentage distribution within the study. The vertical axis divides the case and control groups.

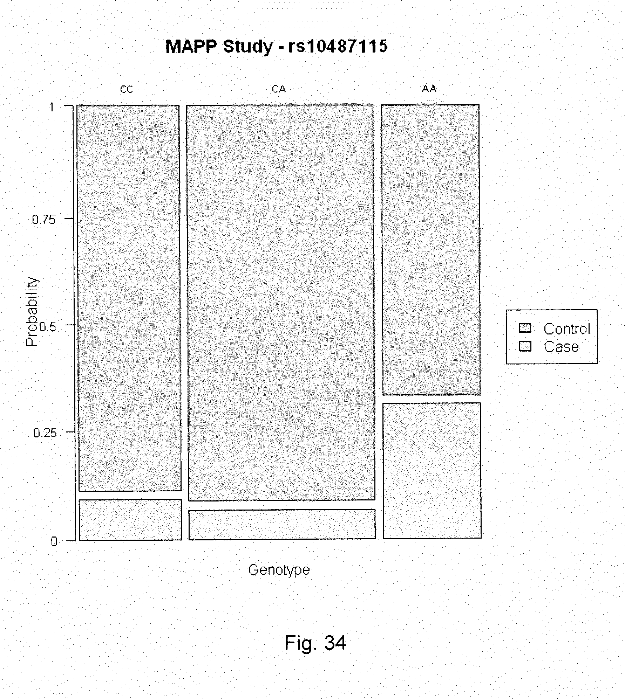

Figure 34 is a mosaic plot illustrating the probability of experiencing LTA as a function of allele specific inheritance of the SNP rs10487115. The horizontal width corresponds to the three genotypes and is proportional to their percentage distribution within the study. The vertical axis divides the case and control groups.

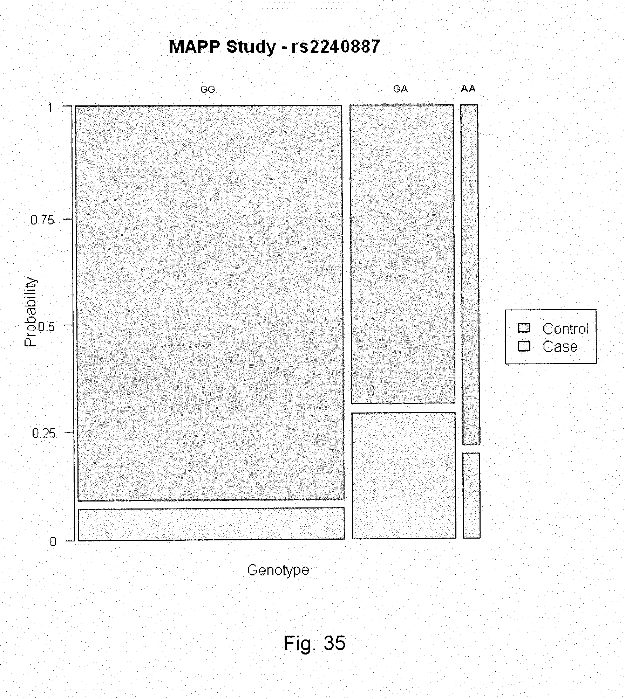

Figure 35 is a mosaic plot illustrating the probability of experiencing LTA as a function of allele specific inheritance of the SNP rs2240887. The horizontal width corresponds to the three genotypes and is proportional to their percentage distribution within the study. The vertical axis divides the case and control groups.

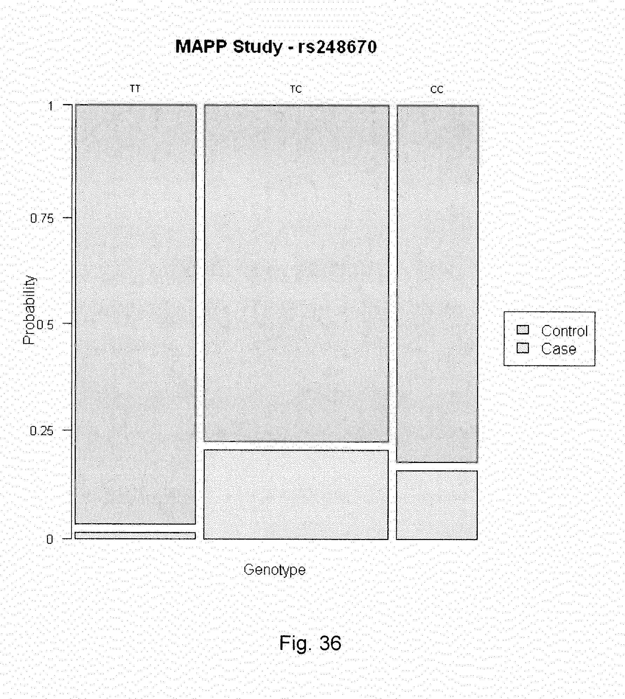

Figure 36 is a mosaic plot illustrating the probability of experiencing LTA as a function of allele specific inheritance of the SNP rs248670. The horizontal width corresponds to the three genotypes and is proportional to their percentage distribution within the study. The vertical axis divides the case and control groups.

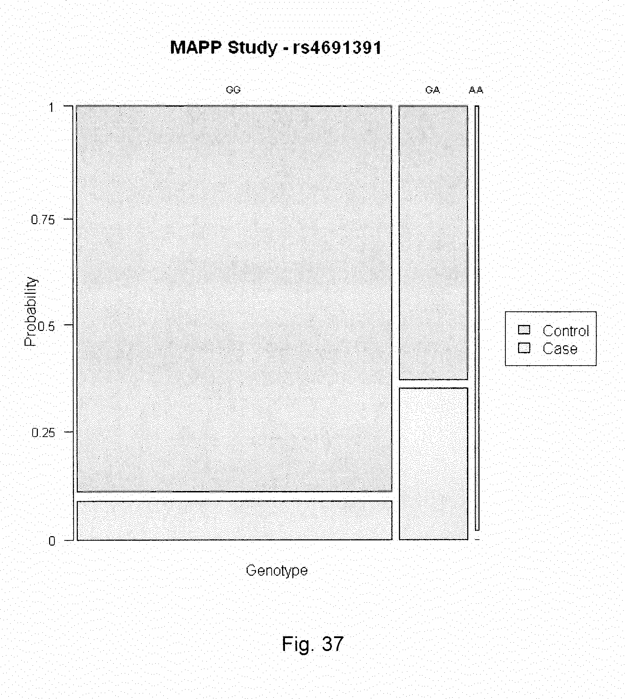

Figure 37 is a mosaic plot illustrating the probability of experiencing LTA as a function of allele specific inheritance of the SNP rs4691391. The horizontal width corresponds to the three genotypes and is proportional to their percentage distribution within the study. The vertical axis divides the case and control groups.

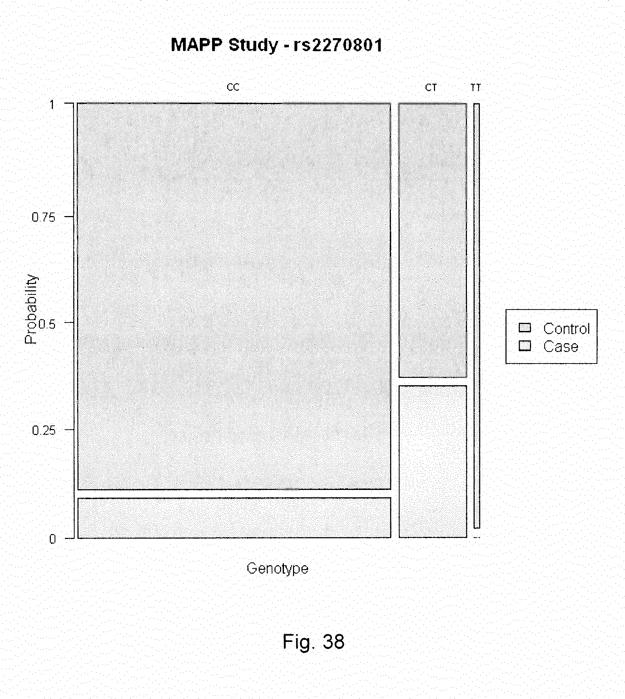

Figure 38 is a mosaic plot illustrating the probability of experiencing LTA as a function of allele specific inheritance of the SNP rs2270801. The horizontal width corresponds to the three genotypes and is proportional to their percentage distribution within the study. The vertical axis divides the case and control groups.

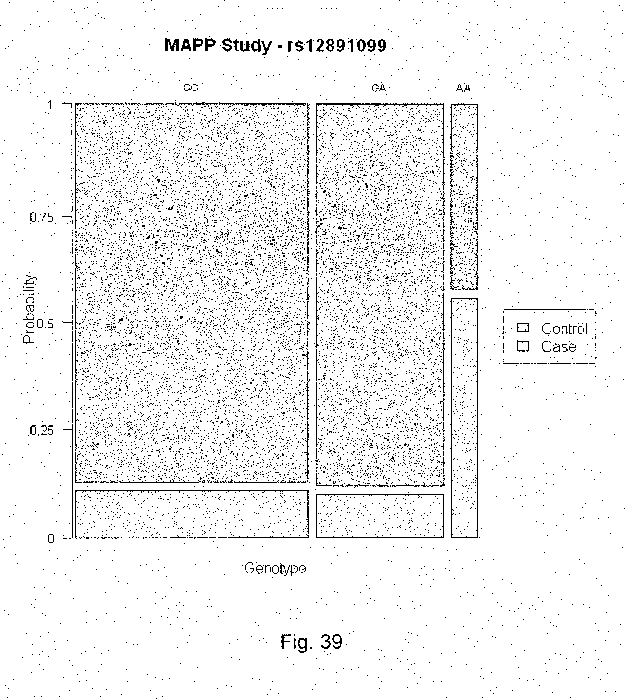

Figure 39 is a mosaic plot illustrating the probability of experiencing LTA as a function of allele specific inheritance of the SNP rs12891099. The horizontal width corresponds to the three genotypes and is proportional to their percentage distribution within the study. The vertical axis divides the case and control groups.

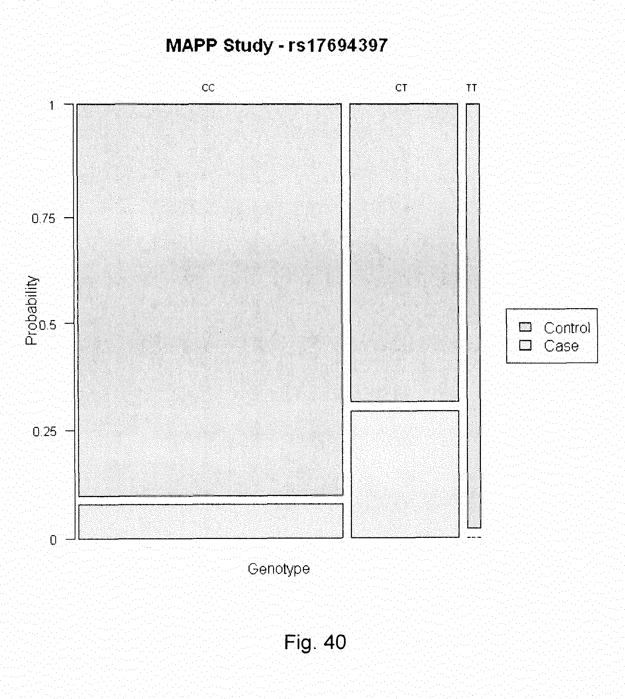

Figure 40 is a mosaic plot illustrating the probability of experiencing LTA as a function of allele specific inheritance of the SNP rs17694397. The horizontal width corresponds to the three genotypes and is proportional to their percentage distribution within the study. The vertical axis divides the case and control groups.

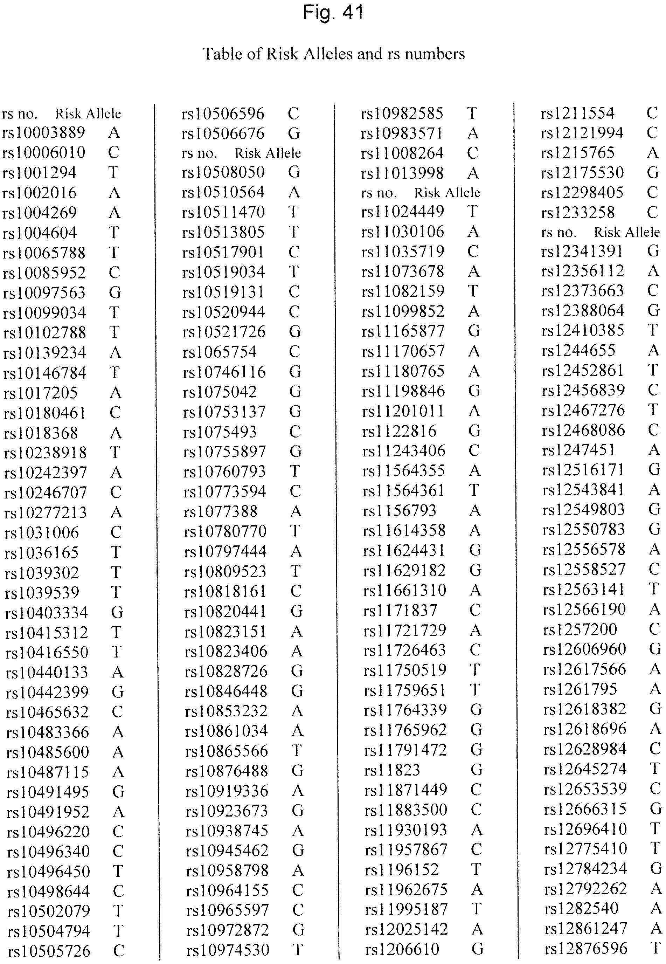

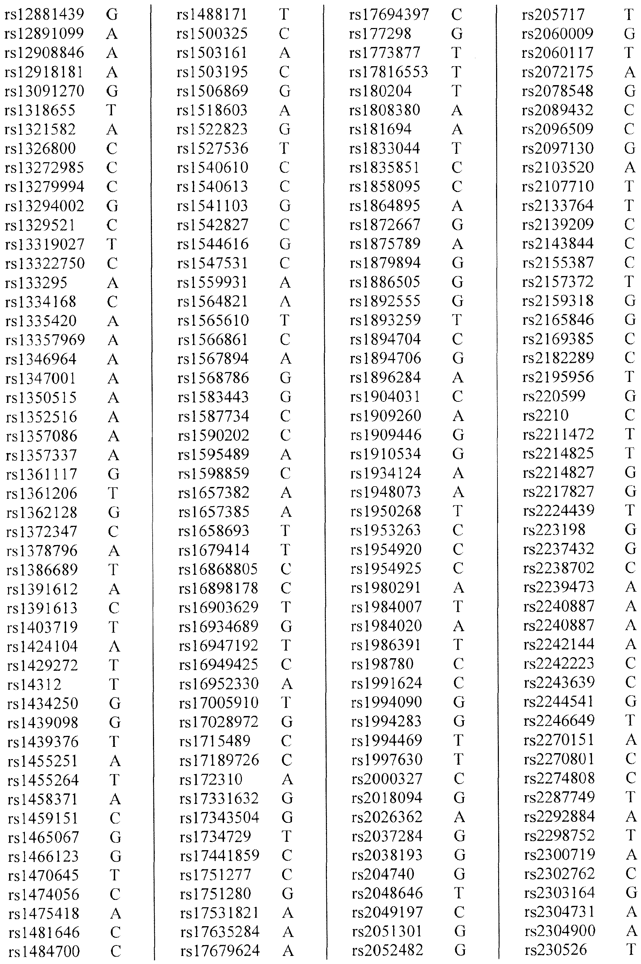

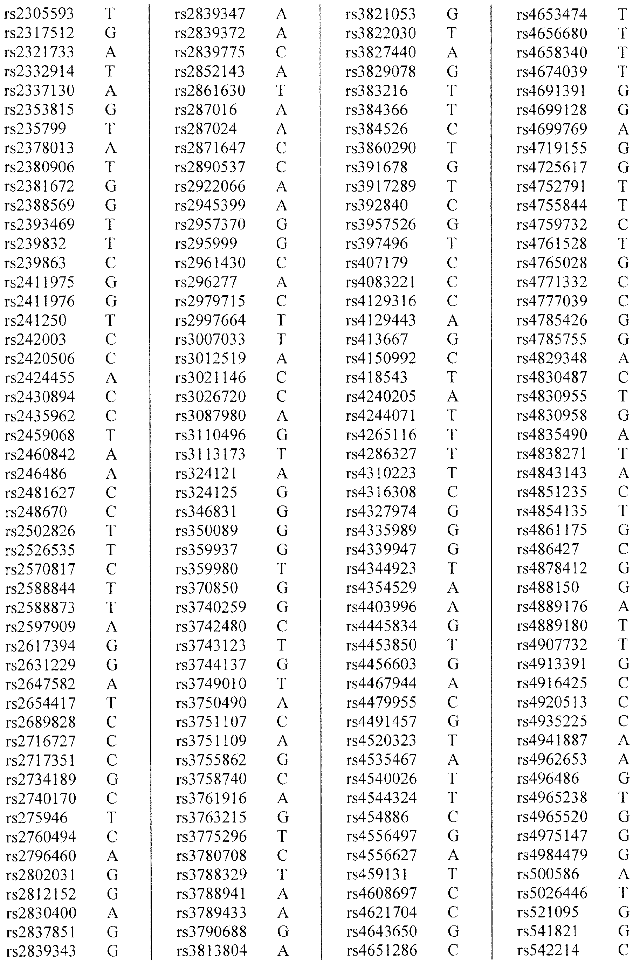

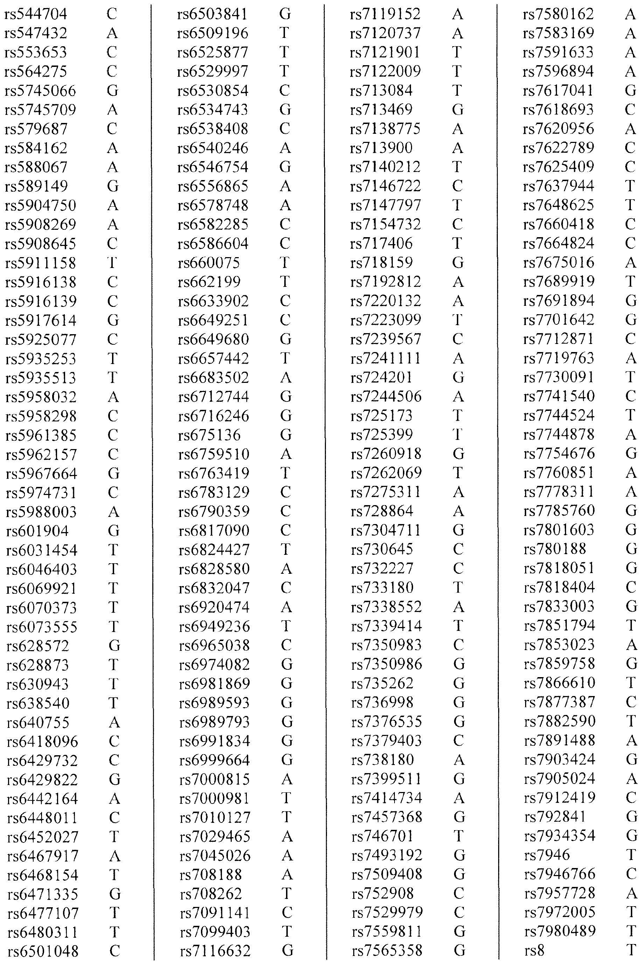

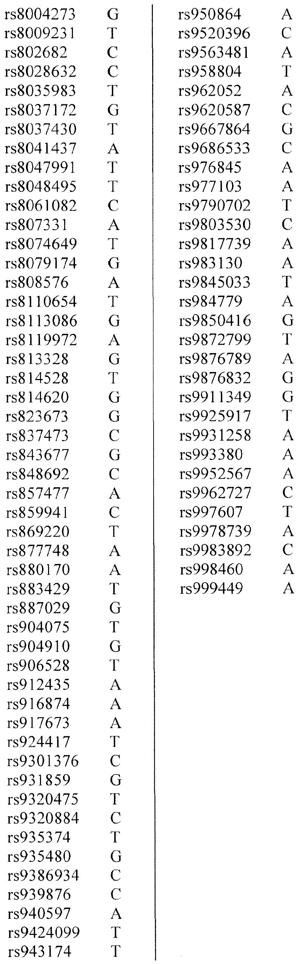

Figure 41 is a list of rs numbers and corresponding risk alleles.

## DETAILED DESCRIPTION OF THE INVENTION

The invention relates to diagnostic kits and methods using a nucleic acid molecule that can predict Sudden Cardiac Death ("SCA") or Sudden Cardiac Arrest ("SCA") risk having a single nucleotide polymorphisms ("SNPs") selected from the group of SEQ ID Nos. 1-858 that can be used in the diagnosis, distinguishing, and detection thereof. The invention further relates to an isolated nucleic acid molecule or molecules useful to predict SCD or SCA risk and SNPs selected from SEQ ID Nos. 1-858 that can be used in the diagnosis, distinguishing, and detecting thereof.

### Definitions

Unless defined otherwise, all technical and scientific terms used herein have the same meaning as commonly understood by one of ordinary skill in the art to which this invention belongs. For purposes of the present invention, the following terms are defined below.
The terms "a," "an," and "the" include plural referents unless the context clearly dictates otherwise.
The term "comprising" includes, but is not limited to, whatever follows the word "comprising." Thus, use of the term indicates that the listed elements are required or mandatory but that other elements are optional and may or may not be present.
The term "consisting of" includes and is limited to whatever follows the phrase the phrase "consisting of." Thus, the phrase indicates that the limited elements are required or mandatory and that no other elements may be present.
The phrase "consisting essentially of" includes any elements listed after the phrase and is limited to other elements that do not interfere with or contribute to the activity or action specified in the disclosure for the listed elements. Thus, the phrase indicates that the listed elements are required or mandatory but that other elements are optional and may or may not be present, depending upon whether or not they affect the activity or action of the listed elements.
The term "plurality" as described herein means more than one, and also defines a multiple of items.
The term "isolated" refers to nucleic acid, or a fragment thereof, that has been removed from its natural cellular environment.
The term "nucleic acid" refers to a deoxyribonucleotide or ribonucleotide polymer in either single- or double-stranded form, and unless otherwise limited, encompasses known analogues of natural nucleotides that hybridize to nucleic acids in a manner similar to naturally occurring nucleotides. The term "nucleic acid" encompasses the terms "oligonucleotide" and "polynucleotide."
The term "amplified polynucleotide" or "amplified nucleotide" as used herein refers to polynucleotides or nucleotides that are copies of a portion of a particular polynucleotide sequence and/or its complementary sequence, which correspond to a template polynucleotide sequence and its complementary sequence. An "amplified polynucleotide" or "amplified nucleotide" according to the present invention, may be DNA or RNA, and it may be double-stranded or single-stranded.

"Synthesis" and "amplification" as used herein are used interchangeably to refer to a reaction for generating a copy of a particular polynucleotide sequence or increasing in copy number or amount of a particular polynucleotide sequence. It may be accomplished, without limitation, by the in vitro methods of polymerase chain reaction (PCR), ligase chain reaction (LCR), polynucleotide-specific based amplification (NSBA), or any other method known in the art. For example, polynucleotide amplification may be a process using a polymerase and a pair of oligonucleotide primers for producing any particular polynucleotide sequence, i.e., the target polynucleotide sequence or target polynucleotide, in an amount which is greater than that initially present.

As used herein, the term "primer pair" means two oligonucleotides designed to flank a region of a polynucleotide to be amplified.

As used herein, an implantable cardioverter-defibrillator (ICD) is a small battery-powered electrical impulse generator implanted in patients who are at risk of sudden cardiac death due to ventricular fibrillation and/or ventricular tachycardia. The device is programmed to detect cardiac arrhythmia and correct it by delivering a jolt of electricity. In known variants, the ability to revert ventricular fibrillation has been extended to include both atrial and ventricular arrhythmias as well as the ability to perform biventricular pacing in patients with congestive heart failure or bradycardia.

"Single nucleotide polymorphisms" (SNPs) refers to a variation in the sequence of a gene in the genome of a population that arises as the result of a single base change, such as an insertion, deletion or, a change in a single base. A locus is the site at which divergence occurs.

An "rs number" refers to a SNP database record archived and curated on dbSNP, which is a database for Single Polymorphism Polynucleotides and Other Classes of Minor Genetic Variations. The dbSNP database maintains two types of records: ss records of each original submission and rs records. The ss records may represent variations in submissions for the same genome location. The rs numbers represent a unique record for a SNP and are constructed and periodically reconstructed based on subsequent submissions and Builds. In each new build cycle, the set of new data entering each build typically includes all submissions received since the close of data in the previous build. Some refSNP (rs) numbers might have been merged if they are found to map the same location at a later build, however, it is understood that a particular rs number with a Build number provides the requisite detail so that one of ordinary skill in the art will be able to make and use the invention as contemplated herein. Hence, one of ordinary skill will generally be able to determine a particular SNP by reviewing the entries for an rs number and related ss numbers. Data submitted to the NCBI database are clustered and provide a non-redundant set of variations for each organism in the database. The clusters are maintained as rs numbers in the database in parallel to the underlying submitted data. Reference Sequences, or RefSeqs, are a curated, non-redundant set of records for mRNAs, proteins, contigs, and gene regions constructed from a GenBank exemplar for that protein or sequence. The accession numbers under "Submitter-Referenced Accessions" is annotation that is included with a submitted SNP (ss) when it is submitted to dbSNP as shown in Figure 15 (Sherry et al., "dbSNP— Database for Single Polymorphism Polynucleotides and Other Classes of Minor Genetic Variation," GENOME RES. 1999; 9: 677-679). However, other alternate forms of the rs number as provided in refseqs, ss numbers, etc. are contemplated by the invention such that one of ordinary skill in the art would understand that the scope and nature of the invention is not departed by using follow-on builds of dbSNP.

The term "MACH" or "MACH 1.0" refers to a haplotyper program using a Hidden Markov Model (HMM) that can resolve long haplotypes or infer missing genotypes in samples of unrelated individuals as known within the art.

The term "Hidden Markov Model (HMM)" describes a statistical method for determining a state, which has not been observed or "hidden." The HMM is generally based on a Markov chain, which describes a series of observations in which the probability of an observation depends on a number of previous observations. For a HMM, the Markov process itself cannot be observed, but only the steps in the sequence.

"Probes" or "primers" refer to single-stranded nucleic acid sequences that are complementary to a desired target nucleic acid. The 5' and 3' regions flanking the target complement sequence reversibly interact by means of either complementary nucleic acid sequences or by attached members of another affinity pair. Hybridization can occur in a base-specific manner where the primer or probe sequence is not required to be perfectly complementary to all of the sequences of a template. Hence, non-complementary bases or modified bases can be interspersed into the primer or probe, provided that base substitutions do not inhibit hybridization. The nucleic acid template may also include "nonspecific priming sequences" or "nonspecific sequences" to which the primers or probes have varying degrees of complementarity. As used in the phrase "priming polynucleotide synthesis," a probe is described that is of sufficient length to initiate synthesis during PCR. In certain embodiments, a probe or primer comprises from about 3 to 101 nucleotides.

The following formula is provided in support of every possible range within 3 to 101 nucleotides. The formula is intended to provide express support for ranges such as 3 to 4 nucleotides in length, or from about 3 to 5, 3 to 6, 3 to 7, 3 to 8, ..., 3 to 99, 3 to 100, 3 to 101, 4 to 5, 4 to 6, *etc.,* with no limitation on the permutations of various ranges that can be selected from the range of about 3 to 101 nucleotides. Thus, in certain embodiments, a probe or primer comprises from about 3 to 101 nucleotides, wherein the length of the complement is described by a length n for the lower bound, and (*n+i*) for the upper bound for $n = \{x \in \mathbb{Z} \mid 3 \leq x \leq 101\}$ and $i = \{y \in \mathbb{Z} \mid 0 \leq y \leq (101 - n)\}$, or from about any number of base pairs flanking the 5' and 3' side of a region of interest to sufficiently identify, or result in hybridization. Hence, where x is the integer 3, the lower bound (n) is 3, and the upper bound (n + i) ranges from 3 to 101 where i ranges from 0 to 98, so that the following ranges of nucleotides are provided: 3 to 3, 3 to 4, 3 to 5, 3 to 6, ... 3 to 101.

Similarly, where x is the integer 4, the lower bound (n) is 4, and the upper bound (n + i) ranges from 4 to 101 for i equals 0 to 97, so that the following ranges of nucleotides are provided: 4 to 4, 4 to 5, 4 to 6, 4 to 7, ... 4 to 101.

Similarly, where x is the integer 5, the lower bound (n) is 5, and the upper bound (n + i) ranges from 5 to 101 for i equals 0 to 96, so that the following ranges of nucleotides are provided: 5 to 5, 5 to 6, 5 to 7, 5 to 8, ... 5 to 101, and so forth for each x.

Hence, where x is the integer 100, the lower bound (n) is 100, and the upper bound (n + i) ranges from 100 to 101 for i equals 0 to 1, so that the following ranges of nucleotides are provided: 100 to 100 and 100 to 101.

Finally, where x is the integer 101, the lower bound (n) is 101 and the upper bound (n + i) is 101 because i equals 0.

Further, the ranges can be chosen from group A and B where for A: the probe or primer is greater than 5, greater than 10, greater than 15, greater than 20, greater than 25, greater than 30, greater than 40, greater than 50, greater than 60, greater than 70, greater than 80, greater than 90 and greater than 100 base pairs in length. For B, the probe or primer is less than 102, less than 95, less than 90, less than 85, less than 80, less than 75, less than 70, less than 65, less than 60, less than 55, less than 50, less than 45, less than 40, less than 35, less than 30, less than 25, less than 20, less than 15, or less than 10 base pairs in length. In other embodiments, the probe or primer is at least 70% identical to the contiguous nucleic acid sequence or to the complement of the contiguous nucleotide sequence, for example, at least 80% identical, at least 90°/a identical, at least 95% identical, and is capable of selectively hybridizing to the contiguous nucleic acid sequence or to the complement of the contiguous nucleotide sequence. Preferred primer lengths include 25 to 35, 18 to 30, and 17 to 24 nucleotides. Often, the probe or primer further comprises a label, e.g., radioisotope, fluorescent compound, enzyme, or enzyme co-factor. One primer is complementary to nucleotides present on the sense strand at one end of a polynucleotide to be amplified and another primer is complementary to nucleotides present on the antisense strand at the other end of the polynucleotide to be amplified. The polynucleotide to be amplified can be referred to as the template polynucleotide. The nucleotide of a polynucleotide to which a primer is complementary is referred to as a target sequence. A primer can have at least about 15 nucleotides, preferably, at least about 20 nucleotides, most preferably, at least about 25 nucleotides. Typically, a primer has at least about 95% sequence identity, preferably at least about 97% sequence identity, most preferably, about 100% sequence identity with the target sequence to which the primer hybridizes. The conditions for amplifying a polynucleotide by PCR vary depending on the nucleotide sequence of primers used, and methods for determining such conditions are routine in the art.

To obtain high quality primers, primer length, melting temperature ($T_m$), GC content, specificity, and intra- or inter-primer homology are taken into account in the present invention. You et al., BatchPrimer3: A high throughput web application for PCR and sequencing primer design, BMC BIOINFORMATICS, 2008, 9:253; Yang X, Scheffler BE, Weston LA, Recent developments in primer design for DNA polymorphism and mRNA profiling in higher plants, PLANT METHODS, 2006, 2(1):4. Primer specificity is related to primer length and the final 8 to 10 bases of the 3" end sequence where a primer length of 18 to 30 bases is one possible embodiment. Abd-Elsalam KA, Bioinformatics tools and guideline for PCR primer design, AFRICA JOURNAL OF BIOTECHNOLOGY, 2003, 2(5):91-95. $T_m$ is closely correlated to primer length, GC content and primer base composition. One preferred primer $T_m$ is in the range of 50 to 65°C with GC content in the range of 40 to 60% for standard primer pairs. Dieffenbatch CW, Lowe TMJ, Dveksler GS, General concepts for PCR primer design, PCR PRIMER, A LABORATORY MANUAL, Edited by: Dieffenbatch CW, Dveksler GS. New York , Cold Spring Harbor Laboratory Press; 1995:133-155. However, an optimal primer length varies depending on different types of primers. For example, SNP genotyping primers may require a longer primer length of 25 to 35 bases to enhance their specificity, and thus the corresponding $T_m$ might be higher than 65°C. Also, a suitable $T_m$ can be obtained by setting a broader GC content range (20 to 80%).

The probes or primers can also be variously referred to as "antisense nucleic acid molecules," "polynucleotides," or "oligonucleotides" and can be constructed using chemical synthesis and enzymatic ligation reactions known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids. The primers or probes can further be used in "Polymerase Chain Reaction" (PCR), a well known amplification and analytical technique that generally uses two "primers" of short, single-stranded DNA synthesized to correspond to the beginning of a DNA stretch to be copied, and a polymerase enzyme that moves along the segment of DNA to be copied that assembles the DNA copy.

The term "genetic material" refers to a nucleic acid sequence that is sought to be obtained from any number of sources, including without limitation, whole blood, a tissue biopsy, lymph, bone marrow, hair, skin, saliva, buccal swabs, purified samples generally, cultured cells, and lysed cells, and can comprise any number of different compositional components (e.g., DNA, RNA, tRNA, siRNA, mRNA, or various non-coding RNAs). The nucleic acid can be isolated from samples using any of a variety of procedures known in the art. In general, the target nucleic acid will be single stranded, though in some embodiments the nucleic acid can be double stranded, and a single strand can result from denaturation. It will be appreciated that either strand of a double-stranded molecule can serve as a target nucleic acid to be obtained. The nucleic acid sequence can be methylated, non-methylated, or both, and can contain any number of modifications. Further, the nucleic acid sequence can refer to amplification products as well as to the native sequences.

The term "screening" within the phrase "screening for a genetic sample" means any testing procedure known to those of ordinary skill in the art to determine the genetic make-up of a genetic sample.

As used herein, "hybridization" is defined as the ability of two nucleotide sequences to bind with each other based on a degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific will be the binding of the two sequences. Increased stringency is achieved by elevating the temperature, increasing the ratio of co-solvents, lowering the salt concentration, and the like. Stringent conditions are conditions under which a probe can hybridize to its target subsequence, but to no other sequences. Stringent conditions are sequence-dependent and are different in different circumstances. Longer sequences hybridize specifically at higher temperatures. Generally, stringent conditions are selected to be about 5° C. lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. Typically, stringent conditions include a salt concentration of at least about 0.01 to 1.0 M Na ion concentration (or other salts) at pH 7.0 to 8.3 and the temperature is at least about 30° C. for short probes (e.g., 10 to 50 nucleotides). Stringent conditions can also be achieved with the addition of destabilizing agents such as formamide or tetraalkyl ammonium salts. For example, conditions of SxSSPE (750 mM NaCl, 50 mM Na Phosphate, 5 mM EDTA, pH 7.4) and a temperature of 25-30° C. are suitable for allele-specific probe hybridizations. Sambrook et al., MOLECULAR CLONING, 1989.

Allele Specific Oligomer ("ASO") refers to a primary oligonucleotide having a target specific portion and a target-identifying portion, which can query the identity of an allele at a SNP locus. The target specific portion of the ASO of a primary group can hybridize adjacent to the target specific portion and can be made by methods well known to those of ordinary skill. The ordinary meaning of the term "allele" is one of two or more alternate forms of a gene occupying the same locus in a particular chromosome or linkage structure and differing from other alleles of the locus at one or more mutational sites. (Rieger et al., GLOSSARY OF GENETICS, 5th Ed., Springer-herlag, Berlin 1991; 16*).*

The ordinary meaning of the term "allele" is one of two or more alternate forms of a gene occupying the same locus in a particular chromosome or linkage structure and differing from other alleles of the locus at one or more mutational sites. (Rieger et al., GLOSSARY OF GENETICS, 5TH ED., Springer-Verlag, Berlin 1991; 16).

Bi-allelic and multi-allelic refers to two, or more than two alternate forms of a SNP, respectively, occupying the same locus in a particular chromosome or linkage structure and differing from other alleles of the locus at a polymorphic site.

The phrase "assessing the presence of said one or more SNPs in a genetic sample" encompasses any known process that can be implemented to determine if a polymorphism is present in a genetic sample. For example, amplified DNA obtained from a genetic sample can be labeled before it is hybridized to a probe on a solid support. The amplified DNA is hybridized to probes which are immobilized to known locations on a solid support, e.g., in an array, microarray, high density array, beads or microtiter dish. The presence of labeled amplified DNA products hybridized to the solid support indicates that the nucleic acid sample contains at the polymorphic locus a nucleotide which is indicative of the polymorphism. The quantities of the label at distinct locations on the solid support can be compared, and the genotype can be determined for the sample from which the DNA was obtained. Two or more pairs of primers can be used for determining the genotype of a sample. Each pair of primers specifically amplifies a different allele possible at a given SNP. The hybridized nucleic acids can be detected, e.g., by detecting one or more labels attached to the target nucleic acids. The labels can be incorporated by any convenient means. For example, a label can be incorporated by labeling the amplified DNA product using a terminal transferase and a fluorescently labeled nucleotide. Useful detectable labels include labels that can be detected by spectroscopic, photochemical, biochemical, immunochemical, and electrical, optical, or chemical means. Radioactive labels can be detected using photographic film or scintillation counters. Fluorescent labels can be detected using a photodetector.

The term "detecting" as used in the phrase "detecting one or more Single Nucleotide Polymorphisms (SNPs)" refers to any suitable method for determining the identity of a nucleotide at a position including, but not limited to, sequencing, allele specific hybridization, primer specific extension, oligonucleotide ligation assay, restriction enzyme site analysis and single- stranded conformation polymorphism analysis.

In double-stranded DNA, only one strand codes for the RNA that is translated into protein. This DNA strand is referred to as the "antisense" strand. The strand that does not code for RNA is called the "sense" strand. Another way of defining antisense DNA is that it is the strand of DNA that carries the information necessary to make proteins by binding to a corresponding messenger RNA (mRNA). Although these strands are exact mirror images of one another, only the antisense strand contains the information for making proteins. "Antisense compounds" are oligomeric compounds that are at least partially complementary to a target nucleic acid molecule to which they hybridize. In certain embodiments, an antisense compound modulates (increases or decreases) expression of a target nucleic acid. Antisense compounds include, but are not limited to, compounds that are oligonucleotides, oligonucleosides, oligonucleotide analogs, oligonucleotide mimetics, and chimeric combinations of these. Consequently, while all antisense compounds are oligomeric compounds, not all oligomeric compounds are antisense compounds.

Mutations are changes in a genomic sequence. As used herein, "naturally occurring mutants" refers to any preexisting, not artificially induced change in a genomic sequence. Mutations, mutant sequences, or, simply, "mutants" include additions, deletions and substitutions or one or more alleles.

The optimal probe length, position, and number of probes for detection of a single nucleotide polymorphism or for hybridization may vary depending on various hybridization conditions. Thus, the phrase "sufficient to identify the SNP or result in a hybridization" is understood to encompass design and use of probes such that there is sufficient specificity and sensitivity to detect and identify a SNP sequence or result in a hybridization. Hybridization is described in further detail below.

The phrases "increased susceptibility," "decreased susceptibility," or the term "risk," generally, relates to the possibility or probability of a particular event occurring either presently or at some point in the future. Determining an increase or decrease in susceptibility to a medical disease, disorder or condition involves "risk stratification" or "assessing susceptibility," which refers to an arraying of known clinical risk factors that allow physicians and others of skill in the relevant art to classify patients from a low to high range of risk of developing a particular disease, disorder, or condition.

."cDNA" refers to DNA that is synthesized to be complementary to a mRNA molecule, and that represents a portion of the DNA that specifies a protein (is translated). If the sequence of the cDNA is known, by complementarity, the sequence of the DNA is known.

The phrase "selectively hybridizing" refers to the ability of a probe used in the invention to hybridize, with a target nucleotide sequence with specificity.

The term "treatable" means that a patient is potentially or would be expected to be responsive to a particular form of treatment.

In statistical significance testing, the "p-value" is the probability of obtaining a test statistic at least as extreme as the one that was actually observed, assuming that the null hypothesis is true. The lower the p-value, the less likely the result is if the null hypothesis is true, and consequently the more "significant" the result is, in the sense of statistical significance.

As used herein, to impute a p-value to one or more SNPs outside of a test sample means to mathematically attribute a p-value to one or more known and documented SNPs, using the methods described herein, that are not present on the test microchips used in a specific experiment or study. Using the p-values obtained from the tested microchips, p-values may be mathematically imputed to other known SNPs using algorithms such as those described herein.

By the phrase "indicate association," it is meant that the statistical analysis suggests, by, for example, a p-value, that a SNP may be linked to or associated with a particular medical disease, condition, or disorder.

The term "isolated" as used herein with reference to a nucleic acid molecule refers to a nucleic acid that is not immediately contiguous with both of the sequences with which it is immediately contiguous in the naturally occurring genome of the organism from which it is derived. The term "isolated" also includes any non-naturally occurring nucleic acid because such engineered or artificial nucleic acid molecules do not have immediately contiguous sequences in a naturally occurring genome.

DNA microarrays

Numerous forms of diagnostic kits employing arrays of nucleotides are known in the art. They can be fabricated by any number of known methods including photolithography, pipette, drop-touch, piezoelectric, spotting and electric procedures. The DNA microarrays generally have probes that are supported by a substrate so that a target sample is bound or hybridized with the probes. In use, the microarray surface is contacted with one or more target samples under conditions that promote specific, high-affinity binding of the target to one or more of the probes as shown in Fig. 12. A sample solution containing the target sample typically contains radioactively, chemoluminescently or fluorescently labeled molecules that are detectable. The hybridized targets and probes can also be detected by voltage, current, or electronic means known in the art.

Optionally, a plurality of microarrays may be formed on a larger array substrate. The substrate can be diced into a plurality of individual microarray dies in order to optimize use of the substrate. Possible substrate materials include siliceous compositions where a siliceous substrate is generally defined as any material largely comprised of silicon dioxide. Natural or synthetic assemblies can also be employed. The substrate can be hydrophobic or hydrophilic or capable of being rendered hydrophobic or hydrophilic and includes inorganic powders such as silica, magnesium sulfate, and alumina; natural polymeric materials, particularly cellulosic materials and materials derived from cellulose, such as fiber-containing papers, e.g., filter paper, chromatographic paper, *etc*.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), *etc*.; either used by themselves or in conjunction with other materials; glass available as Bioglass, ceramics, metals, and the like. The surface of the substrate is then chemically prepared or derivatized to enable or facilitate the attachment of the molecular species to the surface of the array substrate. Surface derivatizations can differ for immobilization of prepared biological material, such as cDNA, and *in situ* synthesis of the biological material on the microarray substrate. Surface treatment or derivatization techniques are well known in the art. The surface of the substrate can have any number of shapes, such as strip, plate, disk, rod, particle, including bead, and the like. In modifying siliceous or metal oxide surfaces, one technique that has been used is derivatization with bifunctional silanes, i.e., silanes having a first functional group enabling covalent binding to the surface and a second functional group that can impart the desired chemical and/or physical modifications to the surface to covalently or non-covalently attach ligands and/or the polymers or monomers for the biological probe array. Adsorbed polymer surfaces are used on siliceous substrates for attaching nucleic acids, for example cDNA, to the substrate surface. Since a microarray die may be quite small and difficult to handle for processing, an individual microarray die can also be packaged for further handling and processing. For example, the microarray may be processed by subjecting the microarray to a hybridization assay while retained in a package.

Various techniques can be employed for preparing an oligonucleotide for use in a microarray. *In situ* synthesis of oligonucleotide or polynucleotide probes on a substrate is performed in accordance with well-known chemical processes, such as sequential addition of nucleotide phosphoramidites to surface-linked hydroxyl groups. Indirect synthesis may also be performed in accordance with biosynthetic techniques such as Polymerase Chain Reaction ("PCR"). Other methods of oligonucleotide synthesis include phosphotriester and phosphodiester methods and synthesis on a support, as well as phosphoramidate techniques. Chemical synthesis via a photolithographic method of spatially addressable arrays of oligonucleotides bound to a substrate made of glass can also be employed. The probes or oligonucleotides, themselves, can be obtained by biological synthesis or by chemical synthesis. Chemical synthesis provides a convenient way of incorporating low molecular weight compounds and/or modified bases during specific synthesis steps. Furthermore, chemical synthesis is very flexible in the choice of length and region of target polynucleotides binding sequence. The oligonucleotide can be synthesized by standard methods such as those used in commercial automated nucleic acid synthesizers.

Immobilization of probes or oligonucleotides on a substrate or surface may be accomplished by well-known techniques. One type of technology makes use of a bead-array of randomly or non-randomly arranged beads. A specific oligonucleotide or probe sequence is assigned to each bead type, which is replicated any number of times on an array. A series of decoding hybridizations is then used to identify each bead on the array. The concept of these assays is very similar to that of DNA chip based assays. However, oligonucleotides are attached to small microspheres rather than to a fixed surface of DNA chips. Bead-based systems can be combined with most of the allele-discrimination chemistry used in DNA chip based array assays, such as single-base extension and oligonucleotide ligation assays. The bead-based format has flexibility for multiplexing and SNP combination. In bead-based assays, the identity of each bead needs is determined where that information is combined with the genotype signal from the bead to assign a "genotype call" to each SNP and individual.

One bead-based genotyping technology uses fluorescently coded microspheres developed by Luminex. Fulton R, McDade R, Smith P, Kienker L, Keltman J. J. Advanced multiplexed analysis with the FlowMetrix system, CLIN. CHEM. 1997; 43: 1749-1756. These beads are coated with two different dyes (red and orange), and can be identified and separated using flow cytometry, based on the amount of these two dyes on the surface. By having a hundred types of microspheres with a different red:orange signal ratio, a hundred-plex detection reaction can be performed in a single tube. After the reaction, these microspheres are distinguished using a flow fluorimeter where a genotyping signal (green) from each group of microspheres is measured separately. This bead-based platform is useful in allele-specific hybridization, single-base extension, allele-specific primer extension, and oligonucleotide ligation assay. In a different bead-based platform commercialized by Illumina, microspheres are captured in solid wells created from optical fibers. Michael K. , Taylor L., Schultz S, Walt D. Randomly ordered addressable high-density optical sensor arrays, ANAL. CHEM.., 1998; 70: 1242-1248; Steemers F. , Ferguson J, Walt D., Screening unlabeled DNA targets with randomly ordered fiber-optic gene arrays, NAT. BIOTECHNOL., 2000; 18: 91-94. The diameter of each well is similar to that of the spheres, allowing only a single sphere to fit in one well. Once the microspheres are set in these wells, all of the spheres can be treated like a high-density microarray. The high degree of replication in DNA microarray technology makes robust measurements for each bead type possible. Bead-array technology is particularly useful in SNP genotyping. Software used to process raw data from a DNA microarray or chip is well known in the art and employs various known methods for image processing, background correction and normalization. Many available public and proprietary software packages are available for such processing whereby a quality assessment of the raw data can be carried out, and the data then summarized and stored in a format which can be used by other software to perform additional analyses.

The diagnostic kit, microarray or probes or nucleotides immobilized on a substrate or surface, a bead-based platform, and any of the methods for detecting an SNP described above can contain or be provided, or be performed with a limited number of probes. Probes include, but are not limited to, nucleotides that hybridize with the locus of a SNP, allele specific oligomers (OAS) or primers that bind to a flanking region relative to the locus of the SNP to assist in determining the identity of the SNP by Sanger sequencing or similar technique. As described, a plurality of probes can be provided on a common or shared substrate or as part of the same kit.

In certain embodiments, the limited number of probes is from about 1 to about 50 probes. In other embodiments, the limited number of probes is less than about 10 probes, or any of less than about 100 probes, less than about 50 probes, less than about 30 probes or less than about 10 probes. In some embodiments, the limited number of probes is any of from about 2 to about 100 probes, from about 2 to about 50 probes, from 2 to about 30 probes, and from 2 to about 6 probes.

In certain embodiments, the limited number of probes is from about 1 to about 1000 probes. In other embodiments, the limited number of probes is less than about 1000 probes, or any of less than about 900 probes, less than about 700 probes, less than about 500 probes or less than about 30 probes. In some embodiments, the limited number of probes is any selected from about 2 to about 1000 probes, from about 2 to about 900 probes, from 2 to about 500 probes, and from 2 to about 30 probes.

In certain embodiments, the limited number of probes in the diagnostic kit or microarray is selected from probes for detecting an SNP at position number 51 in any of SEQ ID Nos. 1-849 or at position 26 or 27 in any of SEQ ID Nos. 850-858. In certain embodiments, at least about 90% of the limited number of probes in the diagnostic kit or microarray is selected from probes for detecting an SNP at position number 51 in any of SEQ ID Nos. 1-849 or at position number 26 or 27 in SEQ ID Nos. 850-858. In certain embodiments, at least about 80% of the limited number of probes in the diagnostic kit or microarray is selected from probes for detecting an SNP at position number 51 in any of SEQ ID Nos. 1-849 or at position number 26 or 27 in any of SEQ ID Nos. 850-858. In certain embodiments, the limited number of probes in the diagnostic kit or microarray includes at least one probe for determining an SNP at position 51 in any of SEQ ID Nos. 835, 832, 844, 846, 838, 848, 829, 842, 827, 828, 824, 836, 840, 845, 826, 837, 841, 843, 117, 535, 823, 834, 830, 847, and 849. In certain embodiments, the limited number of probes in the diagnostic kit or microarray contains probes for detecting an SNP at position 51 in all of SEQ ID Nos. 835, 832, 844, 846, 838, 848, 829, 842, 827, 828, 824, 836, 840, 845, 826, 837, 841, 843, 117, 535, 823, 834, 830, 847, and 849.

In certain embodiments, the limited number of probes in the diagnostic kit or microarray includes at least one probe for determining an SNP at position 26 or 27 in any of SEQ ID Nos. 850-855 and 858. In certain embodiments, the limited number of probes in the diagnostic kit or microarray contains probes for detecting an SNP at position 26 or 27 in all of SEQ ID Nos. 850-855 and 858.

In certain embodiments, the limited number of probes in the diagnostic kit or microarray includes at least one probe for determining an SNP at position 51 in any of SEQ ID Nos. 844, 831, 825, 839 and 833. In certain embodiments, the limited number of probes in the diagnostic kit or microarray contains probes for detecting an SNP at position 51 in all of SEQ ID Nos. 844, 831, 825, 839 and 833.

In certain embodiments, the limited number of probes in the diagnostic kit or microarray includes at least one probe for determining an SNP at position 51 in any of SEQ ID Nos. 535, 505 and 515. In certain embodiments, the limited number of probes in the diagnostic kit or microarray contains probes for detecting an SNP at position 51 in all of SEQ ID Nos. 535, 505 and 515.

Those having skill in the art will recognize that humans are diploid organisms containing two copies of each chromosome which can be referred to as homologous chromosomes, except for the X and Y chromosomes in males. As such, any individual or genetic sample can be heterozygous or homozygous at any particular genomic locus corresponding with an SNP at position 51 in any of SEQ ID Nos. 1-849 or at position 26 or 27 in any of SEQ ID Nos. 850-858 or any subset thereof. In certain embodiments, the probes in the diagnostic kits or DNA microarrays described herein including methods for determining the presence of SNPs can determine the heterozygous or homozygous status of an individual or genetic sample by assessing SNPs at the same locus on homologous chromosomes. That is, probes to determine and identify different alleles at position 51 in any of SEQ ID Nos. 1-849 or at position 26 or 27 in any of SEQ ID Nos. 850-858 can be present in the diagnostic kit or microarray, where more than one probe is provided for determining the presence of bi-allelic or multi-allelic SNPs in any one of SEQ ID Nos. 1-858 or any subset thereof.

Single Nucleotide Polymorphism ("SNP")

Generally, genetic variations are associated with human phenotypic diversity and sometimes disease susceptibility. As a result, variations in genes may prove useful as markers for disease or other disorder or condition. Variation at a particular genomic location is due to a mutation event in the conserved human genome sequence, leading to two possible nucleotide variants at that genetic locus. If both nucleotide variants are found in at least 1% of the population, that location is defined as a Single Nucleotide Polymorphism ("SNP"). Moreover, SNPs in close proximity to one another are often inherited together in blocks called haplotypes. These single base nucleotide exchanges result in modified amino acid sequences, altering the structure and function of the coded protein. They also influence the splicing process when present at exon-intron transitions and modify gene transcription when part of promoters. This leads to an altered level of protein expression.

One phenomenon of SNPs is that they can undergo linkage disequilibrium, which refers to the tendency of specific alleles at different genomic locations to occur together more frequently than would be expected by random change. Alleles at given loci are said to be in complete equilibrium if the frequency of any particular set of alleles (or haplotype) is the product of their individual population frequencies. Several statistical measures can be used to quantify this relationship. *(*Devlin and Risch, A comparison of linkage disequilibrium measures for fine-scale mapping, Genomics, 1995 Sep. 20; 29(2): 311-22*)*.

With respect to alleles, a more common nucleotide is known as the major allele and the less common nucleotide is known as the minor allele. An allele found to have a higher than expected prevalence among individuals positive for a given outcome is considered a risk allele for that outcome. An allele found to have a lower than expected prevalence among individuals positive for an outcome is considered a protective allele for that outcome. But while the human genome harbors 10 million "common" SNPs, minor alleles indicative of heart disease are often only shared by as little as one percent of a population.

Hence, as provided herein, certain SNPs found by one or a combination of these methods have been found useful as genetic markers for risk-stratification of SCD or SCA in individuals. Genome-wide association studies are used to identify disease susceptibility genes for common diseases and involve scanning thousands of samples, either as case-control cohorts or in family trios, utilizing hundreds of thousands of SNP markers located throughout the human genome. Algorithms can then be applied that compare the frequencies of single SNP alleles, genotypes, or multi-marker haplotypes between disease and control cohorts. Regions (loci) with statistically significant differences in allele or genotype frequencies between cases and controls, pointing to their role in disease, are then analyzed. For example, following the completion of a whole genome analysis of patient samples, SNPs for use as clinical markers can be identified by any, or combination, of the following three methods:

(1) Statistical SNP Selection Method: Univariate or multivariate analysis of the data is carried out to determine the correlation between the SNPs and the study outcome, life threatening arrhythmias for the present invention. SNPs that yield low p-values are considered as markers. These techniques can be expanded by the use of other statistical methods such as linear regression.

(2) Logical SNP Selection Method: Clustering algorithms are used to segregate the SNP markers into categories which would ultimately correlate with the patient outcomes. Classification and Regression Tree ("CART") is one of the clustering algorithms that can be used. In that case, SNPs forming the branching nodes of the tree will be the markers of interest.

(3) Biological SNP Selection Method: SNP markers are chosen based on the biological effect of the SNP, as it might affect the function of various proteins. For example, a SNP located on a transcribed or a regulatory portion of a gene that is involved in ion channel formation would be good candidates. Similarly, a group of SNPs that are shown to be located closely on the genome would also hint the importance of the region and would constitute a set of markers.

Genetic markers are non-invasive, cost-effective and conducive to mass screening of individuals. The SNPs identified herein can be effectively used alone or in combination with other SNPs as well as with other clinical markers for risk-stratification/assessment and diagnosis of SCD, or SCA. Further, these genetic markers in combination with other clinical markers for SCA are effectively used for identification and implantation of ICDs in individuals at risk for SCA. The genetic markers taught herein provide greater specificity and sensitivity in identification of individuals at risk.

An explanation of an rs number and the National Center for Biotechnology Information (NCBI) SNP database is provided herein. In collaboration with the National Human Genome Research Institute, The National Center for Biotechnology Information has established the dbSNP database to serve as a central repository for both single base nucleotide substitutions, single nucleotide polymorphisms (SNPs) and short deletion and insertion polymorphisms. Reference Sequences, or RefSeqs (rs), are a curated, non-redundant set of records for mRNAs, proteins, contigs, and gene regions constructed from a GenBank exemplar for that protein or sequence. The rs numbers represent a unique record for a SNP. Submitted SNPs (ss) are records that are independently submitted to NCBI, are used to construct the rs record, and are cross-referenced with the rs record for the corresponding genome location. Submitter-Referenced Accession numbers are annotations that are included with a SS number. For rs records relevant to the present invention, these accession numbers may be associated with a GenBank accession record, which will start with one or two letters, such as "AL" or "AC," followed by five or six numbers. The NCBI RefSeq database accession numbers have different formatting: "NT_123456." The RefSeq accession numbers are unique identifiers for a sequence, and when minor changes are made to a sequence, a new version number is assigned, such as "NT_123456.1," where the version is represented by the number after the decimal. The rs number represents a specific range of bases at a certain contig position. Although the contig location of the rs sequence may move relative to the length of the larger sequence encompassed by the accession number, that sequence of bases represented by the rs number, *i.e.,* the SNP, will remain constant. Hence, it is understood that rs numbers can be used to uniquely identify a SNP and fully enables one of ordinary skill in the art to make and use the invention using rs numbers. The sequences provided in the Sequence Listing each correspond to a unique sequence represented by an rs number known at the time of invention. Thus, the SEQ ID Nos. and the rs numbers claimed disclosed herein are understood to represent uniquely identified sequences for identified SNPs and may be used interchangeably.

Sudden Cardiac Arrest ("SCA")

SCA, also known as Sudden Cardiac Death ("SCD"), results from an abrupt loss of heart function. It is commonly brought on by an abnormal heart rhythm. Sudden cardiac death occurs within a short time period, generally less than an hour from the onset of symptoms. Despite recent progress in the management of cardiovascular disorders generally, and cardiac arrhythmias in particular, SCA remains both a problem for the practicing clinician and a major public health issue.

In the United States, SCA accounts for approximately 325,000 deaths per year. More deaths are attributable to SCA than to lung cancer, breast cancer, or AIDS. This represents an incidence of 0.1-0.2% per year in the adult population. Myerburg, RJ et a/., Cardiac arrest and sudden cardiac death, Braunwald E, ed., A TEXTBOOK OF CARDIOVASCULAR MEDICINE. 6th ed. Philadelphia, Saunders, WB., 2001: 890-931; American Cancer Society*,* CANCER FACTS AND FIGURES 2003: 4*,* Center for Disease Control 2004.

In 60% to 80% of cases, SCA occurs in the setting of Coronary Artery Disease ("CAD"). Most instances involve Ventricular Tachycardias ("VT") degenerating to Ventricular Fibrillation ("VF") and subsequent asystole. Fibrillation occurs when transient neural triggers impinge upon an unstable heart causing normally organized electrical activity in the heart to become disorganized and chaotic. Complete cardiac dysfunction results. Non-ischemic cardiomyopathy and infiltrative, inflammatory, and acquired valvular diseases account for most other SCA, or SCD, events. A small percentage of SCAs occur in the setting of ion channel mutations responsible for inherited abnormalities such as the long/short QT syndromes, Brugada syndrome, and catecholaminergic vent ricular tachycardia. These conditions account for a small number of SCAs. In addition, other genetic abnormalities such as hypertrophic cardiomyopathy and congenital heart defects such as anomalous coronary arteries are responsible for SCA.

Currently, five arrhythmia markers are often used for risk assessment in Myocardial Infarction ("MI") patients: (1) Heart Rate ("HR") Variability, (2) severe ventricular arrhythmia, (3) signal averaged Electro Cardio Gram ("ECG"), (4) left ventricular Ejection Fraction ("EF") and (5) electrophysiology ("EP") (studies). Table 1 illustrates the mean sensitivity and specificity values for each of these five arrhythmia markers. As shown, these markers have relatively high specificity values, but low sensitivity values.

TABLE 1

| Test | HR Variability on AECG | Severe Ventricular Arrhythmia on AECG | Signal Averaged ECG | Left Ventricular Ejection Fraction (EF) | Electrophysiology (EP) Studies |
|---|---|---|---|---|---|
| Sensitivity | 49.8% | 42.8% | 62.4% | 59.1% | 61.8% |
| Specificity | 85.8% | 81.2% | 77.4% | 77.8% | 84.1% |

The most commonly used marker, EF, has a sensitivity of 59%, meaning that 41% of the patients would be missed if EF were the only marker used. Although EP studies provide slightly better indications, they are not performed very frequently due to their rather invasive nature. Hence, the identification of patients who have a propensity toward SCA remains as an unmet medical need.

ECG parameters indicative of SCA, or SCD, are QRS duration, late potentials, QT dispersion, T-wave morphology, Heart rate variability and T-wave alternans. Electrical alternans is a pattern of variation in the shape of the ECG waveform that appears on an every-other-beat basis. In humans, alternation in ventricular repolarization, namely, repolarization alternans, has been associated with increased vulnerability to ventricular tachycardia/ventricular fibrillation and sudden cardiac death. Pham, Q., et al., T-wave alternans: marker, mechanism, and methodology for predicting sudden cardiac death. JOURNAL OF ELECTROCARDIOLOGY, 36: 75-81. Analysis of the morphology of an ECG (*i.e.,* T-wave alternans and QT interval dispersion) has been recognized as means for assessing cardiac vulnerability.

Certain biological factors are predictive of risk for SCA such as a previous clinical event, ambient arrhythmias, cardiac response to direct stimulations, and patient demographics. Similarly, analysis of heart rate variability has been proposed as a means for assessing autonomic nervous system activity, the neural basis for cardiac vulnerability. Heart rate variability, a measure of beat-to-beat variations of sinus-initiated RR intervals, with its Fourier transform-derived parameters, is blunted in patients at risk for SCD. Bigger, JT. Heart rate variability and sudden cardiac death, Zipes DP, Jalife J, Eds. CARDIAC ELECTROPHYSIOLOGY: FROM CELL TO BEDSIDE, Philadelphia, Pa: WB Saunders; 1999.

Patient history is helpful to analyze the risk of SCA, or SCD. For example, in patients with ventricular tachycardia after myocardial infarction, on the basis of clinical history, the following four variables identify patients at increased risk of sudden cardiac death: (1) syncope at the time of the first documented episode of arrhythmia, (2) New York Heart Association ("NYHA") Classification class III or IV, (3) ventricular tachycardia/fibrillation occurring early after myocardial infarction (3 days to 2 months), and (4) history of previous myocardial infarctions. Unfortunately, most of these clinical indicators lack sufficient sensitivity, specificity, and predictive accuracy to pinpoint the patient at risk for SCA, with a degree of accuracy that would permit using a specific therapeutic intervention before an actual event.

For example, the disadvantage of focusing solely on ejection fraction is that many patients whose ejection fractions exceed commonly used cut offs still experience sudden death or cardiac arrest. Since EF is not specific in predicting mode of death, decision making for the implantation of an ICD solely on ejection fraction will not be optimal. Buxton, AE et al., Risk stratification for sudden death: do we need anything more than ejection fraction? CARD. ELECTROPHYSIOLOGY REV. 2003; 7: 434-7. Although electrophysiological ("EP") studies provide slightly better indication, they are not performed very frequently due to their invasive nature and high cost.

Conventional methods for assessing vulnerability to SCA, or SCD, often rely on power spectral analysis (Fourier analysis) of the cardiac electrogram. However, the power spectrum lacks the ability to track many of the rapid arrhythmogenic changes which characterize T-wave alternans, dispersions and heart rate variability. As a result, a non-invasive diagnostic method of predicting vulnerability to SCA, or SCD, by the analysis of ECG has not achieved wide spread clinical acceptance.

Similarly, both, baroflex sensitivity and heart rate variability, judge autonomic modulation at the sinus node, which is taken as a surrogate for autonomic actions at the ventricular level. Autonomic effects at the sinus node and ventricle can easily be dissociated experimentally and may possibly be a cause of false-positive or false-negative test results. Zipes, DP et al., Sudden Cardiac Death, CIRCULATION, 1998;98:2334-2351.

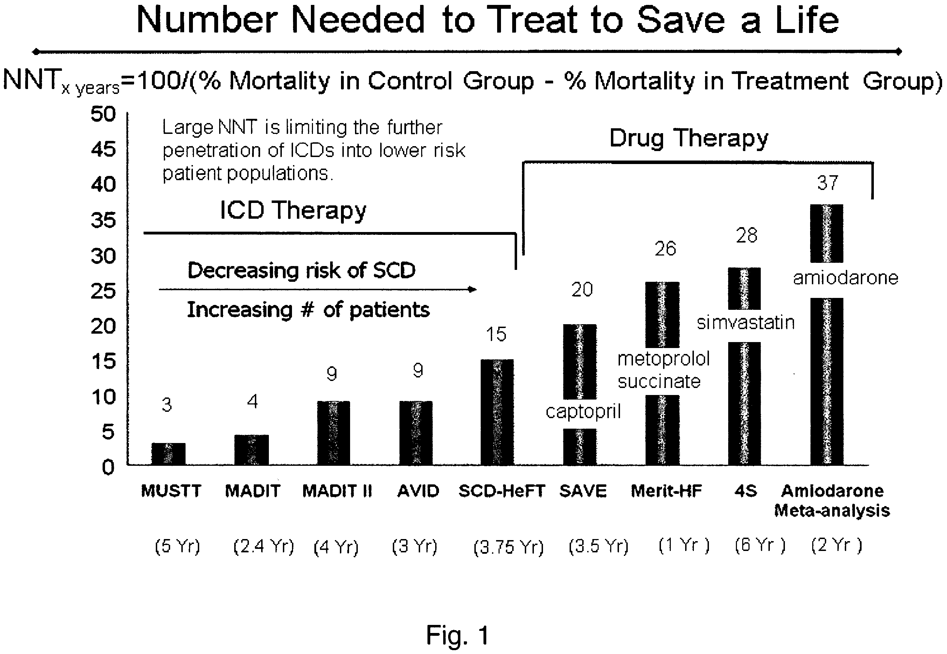

Moreover, as shown in Figure 1, an increase in the Number Needed to Treat ("NNT") has been observed for the ICD therapy as the devices are implanted in patients with lower risks. NNT is an epidemiological measure used in assessing the effectiveness of a health-care intervention. The NNT is the number of patients who need to be treated in order to prevent a single negative outcome. In the case of ICDs, currently, devices must be implanted in approximately 17 patients to prevent one death. The other 16 patients may not experience a life threatening arrhythmia and may not receive a treatment. Reduction of the NNT for ICDs would yield to better patient identification methods and allow delivery of therapies to individuals who need them. As a result, it is believed that the need for risk stratification of patients might increase over time as the ICDs are implanted in patients who are generally considered to be at lower risk categories. The net result of the lack of more specific markers for life threatening arrhythmias is the presence of a population of patients who would benefit from ICD therapy, but are not currently indicated, and a subgroup of patients who receive ICD implants, but may not benefit from them.

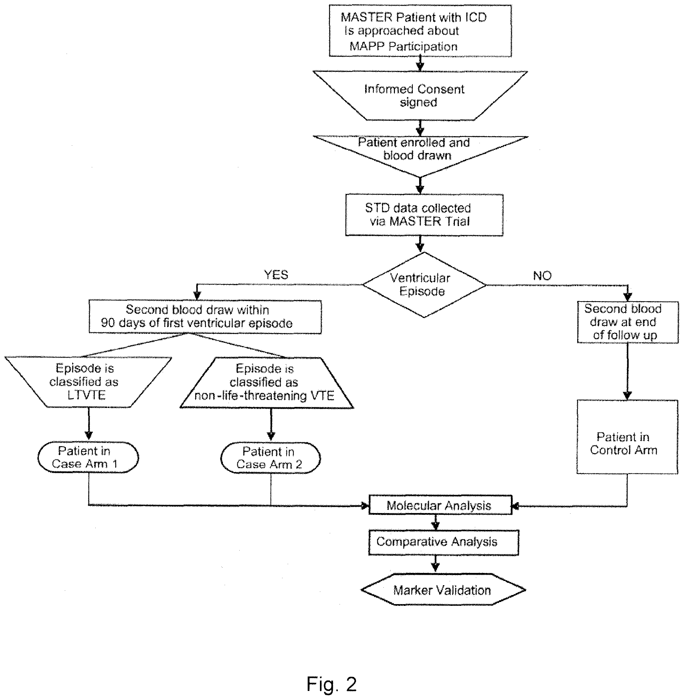

Therefore, in order to identify genetic markers associated with SCA, or SCD, a sub-study (also referred to herein as "MAPP") to an ongoing clinical trial (also referred to herein as "MASTER") was designed and implemented. The MASTER study was undertaken to determine the utility of T-wave-alternans test for the prediction of SCA in patients who have had a heart attack and are in heart failure. The overall aim of the study was to assist in identification of patients most likely to benefit from receiving an ICD. Resulting data was used for the search of statistical associations between life threatening events and SNPs. Figure 2 is a graphical representation of the study design. All patients participating in the MAPP study had defibrillators (ICD) implanted at enrollment and they were followed up for an average of 2.6 years following the ICD implantation. Based on the arrhythmic events that the patients had during this follow-up, they were categorized in three groups as shown in Table 2.

TABLE 2

| Outcome of MAPP Patients | |
|---|---|
| Patient Category | Number |
| CASE 1- Life Threatening Left Ventricular Event | 33 |
| CASE 2- Non-life Threatening Left Ventricular Events | 2 |
| CONTROL - No Events | 205 |
| Total | 240 |

Table 3 provides a brief summary of the demographic and physiologic variables that were recorded at the time of enrollment. Except for the Ejection Fraction ("EF"), none of the variables were found to be predictive of the patient outcome, as shown by the large p-values in Table 3. Although the EF gave a p-value less than 0.05, indicating a correlation with the presence of arrhythmic events, it did not provide a sufficient separation of the two groups to act as a prognostic predictor for individual patients, which in turn further confirmed the initial assessment that there is no strong predictor for SCA.

TABLE 3

| Demographic and Physiologic Variable Summary For the MAPP Patient Population | | | | |
|---|---|---|---|---|
| Variable Name | Entire MAPP N=240 | Case 1 N=33 | Control N=205 | p-value |
| | Mean (SD) | | | |
| Age (years) | 63.2 (11.0) | 61.6 (8.5) | 63.5 (11.3) | 0.3694 |
| EF (%) | 27.1 (6.5) | 25.0 (6.3) | 27.5 (6.4) | 0.0449 |
| NYHA Class | 2.7 (1.4) | 2.9 (1.4) | 2.7 (1.4) | 0.4015 |
| QRS Width (msec) | 115.4 (29.8) | 115.0 (23,8) | 115.5 (30.7) | 0.9443 |
| | N (%) | | | |
| Sex (Male) | 209 (87.1) | 26 (78.8) | 183 (88.4) | 0.1582 |
| MTWA (Negative) | 77 (32.2) | 13 (39.4) | 64 (31.0) | 0.4223 |
| Race (Caucasian) | 224 (93.3) | 31 (93.9) | 193 (93.2) | 1 |
| (EF: Ejection fraction; NYHC: New York Heart Class; MTWA: Microvolt T-Wave Alternans test) | | | | |

Association of genetic variation and disease can be a function of many factors, including, but not limited to, the frequency of the risk allele or genotype, the relative risk conferred by the disease-associated allele or genotype, the correlation between the genotyped marker and the risk allele, sample size, disease prevalence, and genetic heterogeneity of the sample population. In order to search for associations between SNPs and patient outcomes, genomic DNA was isolated from the blood samples collected from the 240 patients who participated in this study. Following the DNA isolation, a whole genome scan consisting of 317,503 SNPs was conducted using Illumina 300K HapMap gene chips. For each locus, two nucleic acid reads were done from each patient, representing the nucleotide variants on two chromosomes, except for the loci chromosomes on male patients. Four letter symbols were used to represent the nucleotides that were read: cytosine (C), guanine (G), adenine (A), and thymine (T). The structure of the various alleles is described by any one of the nucleotide symbols of Table 4.

TABLE 4

Allele Key used in Sequence Listings

| Nucleotide symbol | Full Name |
|---|---|
| R | Guanine / Adenine (purine) |
| Y | Cytosine / Thymine (pyrimidine) |
| K | Guanine / Thymine |
| M | Adenine / Cytosine |

(continued)

Allele Key used in Sequence Listings

| Nucleotide symbol | Full Name |
|---|---|
| S | Guanine / Cytosine |
| W | Adenine / Thymine |
| B | Guanine / Thymine / Cytosine |
| D | Guanine / Adenine / Thymine |
| H | Adenine / Cytosine / Thymine |
| V | Guanine / Cytosine / Adenine |
| N | Adenine / Guanine / Cytosine / Thymine |

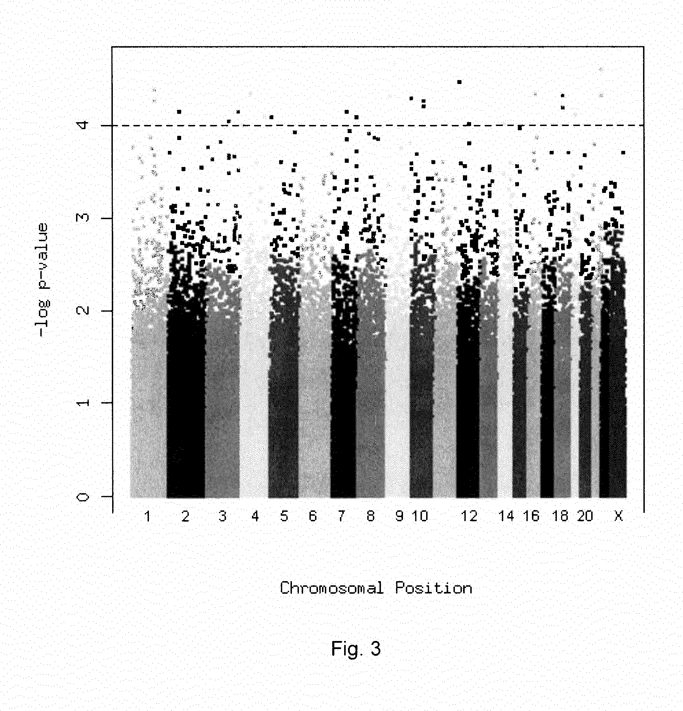

Following the compilation of the genetic data into an electronic database, statistical analysis was carried out. Results from this analysis are provided in Figure 3. As shown in Figure 3, a statistical plot of SNPs: p-values graphed as a function of chromosomal position. The dotted line corresponds to a p-value of 0.0001. There were 25 SNPs found in this analysis with a p-value at or less than 0.0001. The y-axis is the negative base 10 logarithm of the p-value. The x-axis is the chromosome and chromosomal position of each SNP on the Illumina gene chip for which a chromosomal location could be determined (N = 314,635).

For each SNP, Fisher exact test p-value was calculated. Fisher's exact test is a statistical significance test used in the analysis of categorical data where sample sizes are small. For 2 by 2 tables, the null of conditional independence is equivalent to the hypothesis that the odds ratio equals one. "Exact" inference can be based on observing that in general given all marginal totals are fixed, the first element of the contingency table has a non-central hypergeometric distribution with non-centrality parameter given by the odds ratio (Fisher, 1935). The alternative for a one-sided test is based on the odds ratio, so alternative = "greater" is a test of the odds ratio being bigger than one.

For a 2 x 2 contingency table

| | |
|---|---|
| a | C |
| b | D |

the probability of the observed table is calculated by the hypergeometric distribution formula

$$p = \binom{a+b}{a}\binom{c+d}{c} \Big/ \binom{n}{a+c} = \frac{(a+b)!(c+d)!(a+c)!(b+d)!}{n!a!b!c!d!}$$

Two-sided tests are based on the probabilities of the tables, and take as 'more extreme' all tables with probabilities less than or equal to that of the observed table, the p-value being the sum of all such probabilities. Simulation is done conditional on the row and column marginals, and works only if the marginals are strictly positive. Fisher, R. A., The Logic of Inductive Inference, JOURNAL OF THE ROYAL STATISTICAL SOCIETY SERIES A, 1935; 98, 39-54.

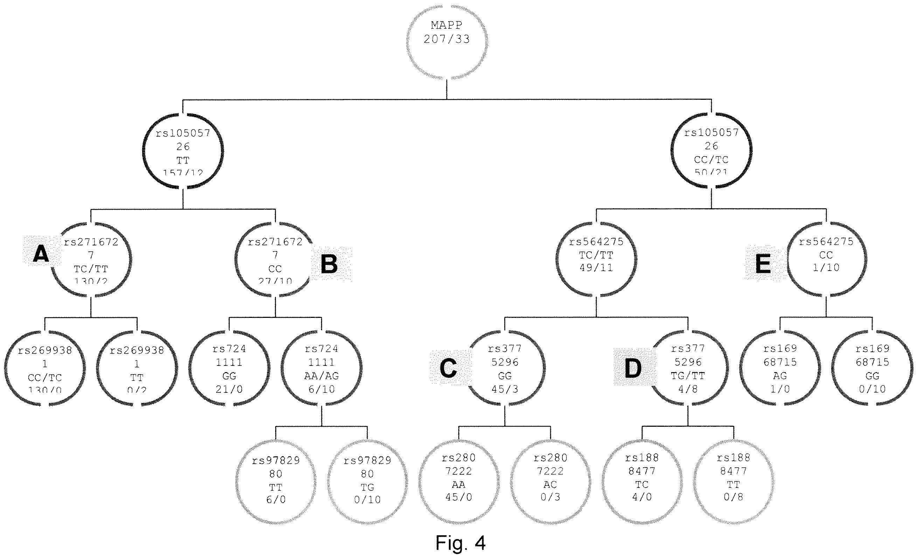

Statistical analysis of the data continued with the use of a recursive partitioning algorithm. Recursive partitioning is a nonparametric technique that recursively partitions the data up into homogeneous subsets (with regard to the response). A multi-level "tree" is formed by bisecting each subset of patients based on their value of a given predictor variable. This point of bisection is called a "node." In this analysis, SNPs were the predictors and the three potential genotypes for each SNP (major allele homozygotes. heterozygotes and minor allele homozygotes) were split into two groups, where the heterozygotes were compacted with one of the two homozygote groups. For a prospectively defined response (in this case, whether a patient is a case or control patient) and set of predictors (SNPs), this method recursively splits the data at each node until either the patients at the resulting end nodes are homogeneous with respect to the response or the end nodes contain too few observations. The decision tree is a visual diagram of the results of recursive partitioning, with the topmost nodes indicating the most discriminatory SNP and each node further split into subnodes accordingly. When this algorithm was applied to 317,498 SNPs, at least a subset of the patients in the analysis cohort was successfully genotyped, and the decision tree shown in Figure 4 resulted. Figure 4 provides the decision tree resulting from the application of the recursive partitioning algorithm to the SNPs that were found to be correlated with the patient outcomes in the MAPP study. The two numbers shown in each node correspond to the case and the control patients grouped in that node.

Using only the non-shaded decision nodes on the tree shown in Figure 4, patients can be categorized in five groups as illustrated in Table 5.

TABLE 5 Genomic Grouping of MAPP Patients Based on the Results of the Recursive Partitioning Algorithm

| Group | Genome | SCD Risk | ICD Recommendation |
|---|---|---|---|
| A | rs10505726=TT<br>rs2716727=TC/TT | $\frac{2}{132}=1.5\%$ | Do not implant |
| B | rs10505726=TT<br>rs2716727=CC | $\frac{10}{37}=27\%$ | Implant |
| C | rs10505726=CC/TC<br>rs564275=TC/TT<br>rs3775296=GG | $\frac{3}{48}=6.3\%$ | Do not implant |
| D | rs10505726=CC/TC<br>rs564275=TC/TT<br>rs3775296=TG/TT | $\frac{8}{12}=66.7\%$ | Implant |
| E | rs10505726=CC/TC<br>rs564275=CC | $\frac{10}{11}=90.1\%$ | Implant |

The overall specificity and sensitivity of the combined tests described by Groups A through E in Table 5 can be determined by examining the contingency table (Table 6) of the combined test and MAPP patients in Case 1 patients, who experienced a life threatening VT/VF event versus Case 2 and Control patients who did not. It is desirable that the given test should have a high sensitivity and specificity value. Furthermore, it is not acceptable to sacrifice either one of these features to enhance the other. Therefore, values that are high enough to improve the clinical patient selection process, but low enough to be achievable with current research capabilities were chosen as indicative of SCA. The goal is to have 80% sensitivity and 80% specificity, which is met by 84.8% and 84.5%, respectively, based on calculations from the data in Table 6.

TABLE 6

| Sensitivity and Specificity of the Combined Tests Enumerated in Table 5, Based on the Results of the Recursive Partitioning Algorithm | | | | |
|---|---|---|---|---|
| | | Experienced VT/VF | | Total |
| | | Yes | No | |
| Combined Tests | Implant | A=28 | B=32 | 60 |
| | Do not Implant | C=5 | D=175 | 180 |
| | Total | 33 | 207 | 240 |

$$Sensitivity_of_combined_test = \frac{A}{A+C} = \frac{28}{28+5} = 84.8\%$$

$$Specificity_of_combined_test = \frac{D}{B+D} = \frac{175}{175+32} = 84.5\%$$

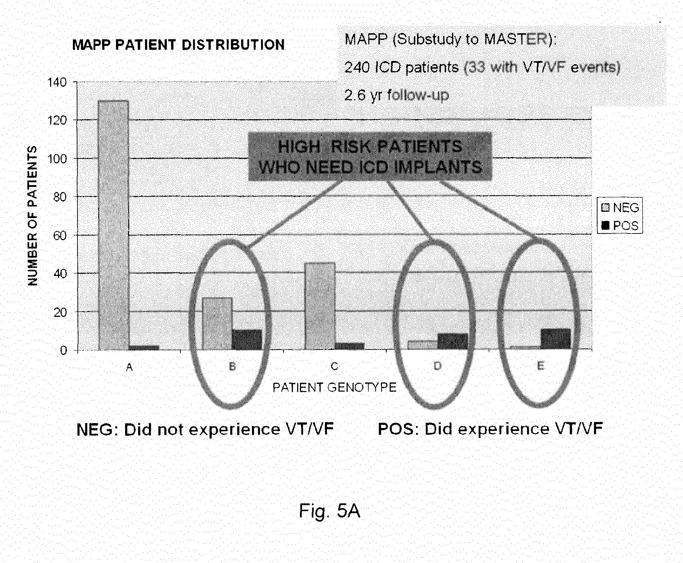

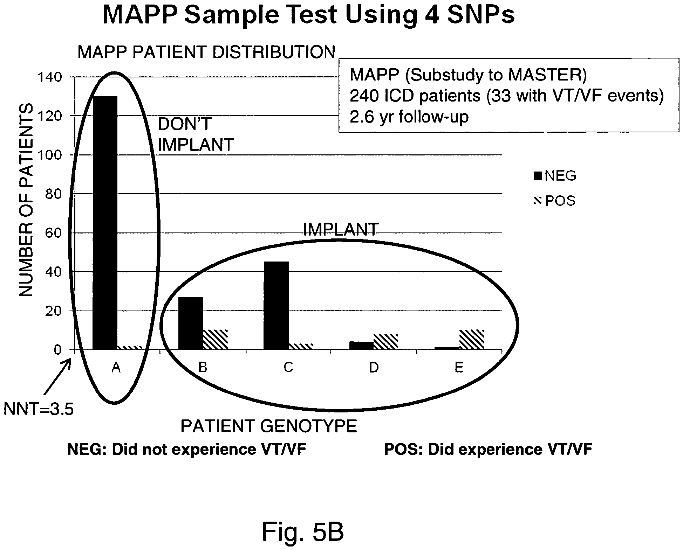

The same results are also shown in the graphical format provided in Figures 5A and 5B.

Figures 5A and 5B indicates how 4 SNP markers could potentially be used to differentiate patients into high risk and low risk groups. The five SNPs indicated in Table 7 are shown visually among the SNPs in the decision tree in Figure 4. Group A consists of patients with the TT genotype for rs10505726 and the TC or TT genotype for rs2716727. As indicated by Figure 5B, these patients would not be considered to be at relatively high risk for a life threatening VT/VF based solely on the genetic diagnostic test. Alternatively, Group B consists of patients with the TT genotype for rs10505726, but with the CC genotype for rs2716727. As indicated by Figure 5A, these patients would be considered to be at relatively high risk for a life threatening VT/VF based solely on the genetic test and would be considered to be candidates for ICD implantation. Similar logic dictates that Groups D and E are relatively high risk and Group C is relatively low risk for life threatening VT/VF based on the genotypes of rs10505726, rs564275 and rs3775296. Rs7241111 from Table 7 is not used in Figure 5A, but could be used to further risk stratify the patients.

Additional investigations were conducted using references to determine the nature of the five polymorphisms that were identified by the recursive partitioning algorithm. Results of this work are summarized in Table 7.

TABLE 7

| SNPs That Were Found to Be Statistically Significant Using the Recursive Partitioning Analysis | | | | | | |
|---|---|---|---|---|---|---|
| SNP | Fisher Exact Test p-value | Chromosome number | Gene Name | Entrez ID | Functional Class | Chromosome Position |
| rs10505726 | $3.46x10^{-5}$ | 12 | PARP 11 | 57097 | Intron | 12:3848218 |
| rs2716727 | $3.67x10^{-3}$ | 2 | - | - | - | 2:39807249 |
| rs564275 | $3.72.10^{-3}$ | 9 | GLIS3 | 169792 | Intron | 9:4084320 |
| rs7241111 | $7.33x10^{-3}$ | 18 | - | - | - | 18:63002332 |
| rs3775296 | $6.01x10^{-2}$ | 4 | TLR3 | 7098 | Mrna-utr | 4:187234760 |

Persons skilled in the art of medical diagnosis will appreciate that there are multiple methods for the combination of measurements from a patient contemplated by the present invention. For example, a triple test given during pregnancy utilizes the three factors measured from a female subject, and a medical decision is made by further addition of the age of the subject. Similarly, SNPs described in this invention can be combined with other patient information, such as co-morbidities (*e.g.,* diabetes, obesity, cholesterol, family history), parameters derived from electrophysiological measurements such as T-wave alternans, heart rate variability and heart rate turbulence, hemodynamic variables such as ejection fraction and end diastolic left ventricular volume, to yield a superior diagnostic technique. Furthermore, such a combination of a set markers can be achieved by multiple methods, including logical, linear, or non-linear combination of these markers, or by the use of clustering algorithms known in the art.

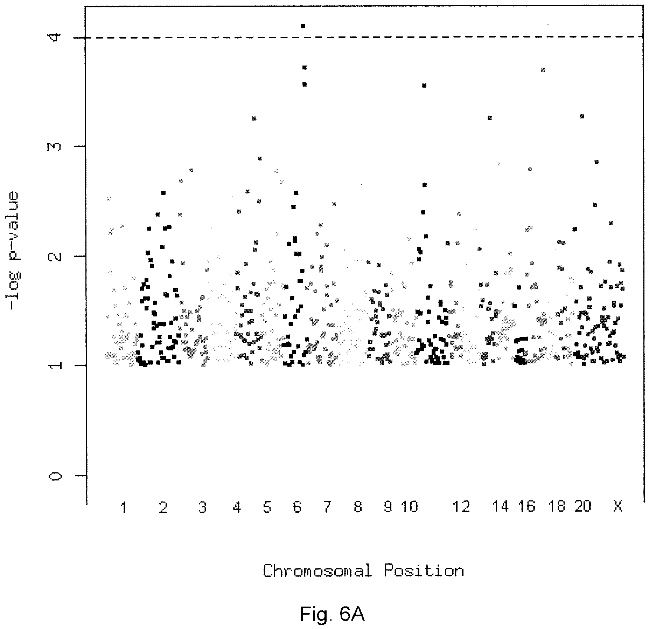

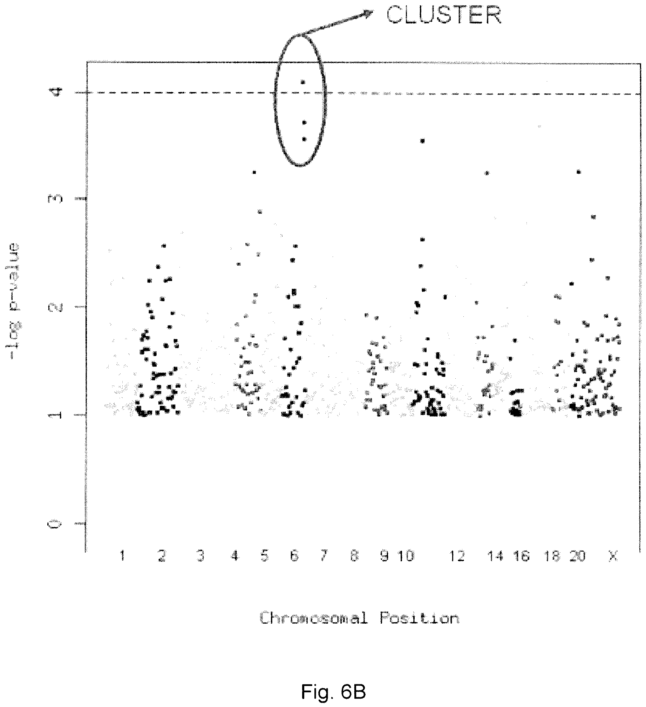

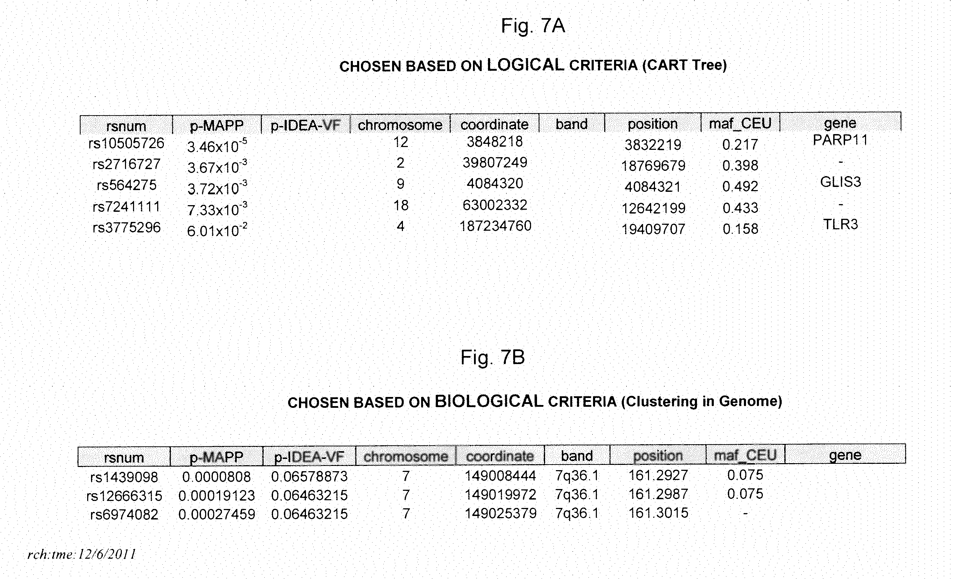

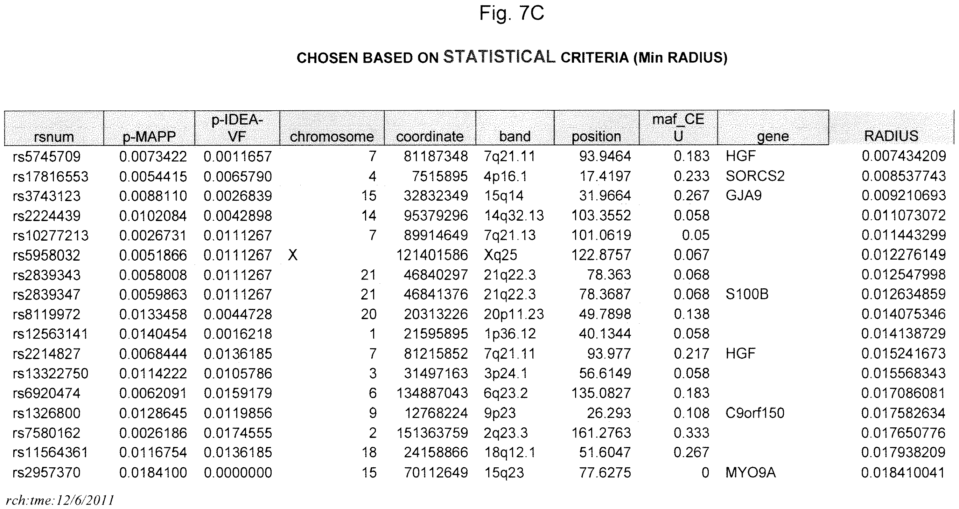

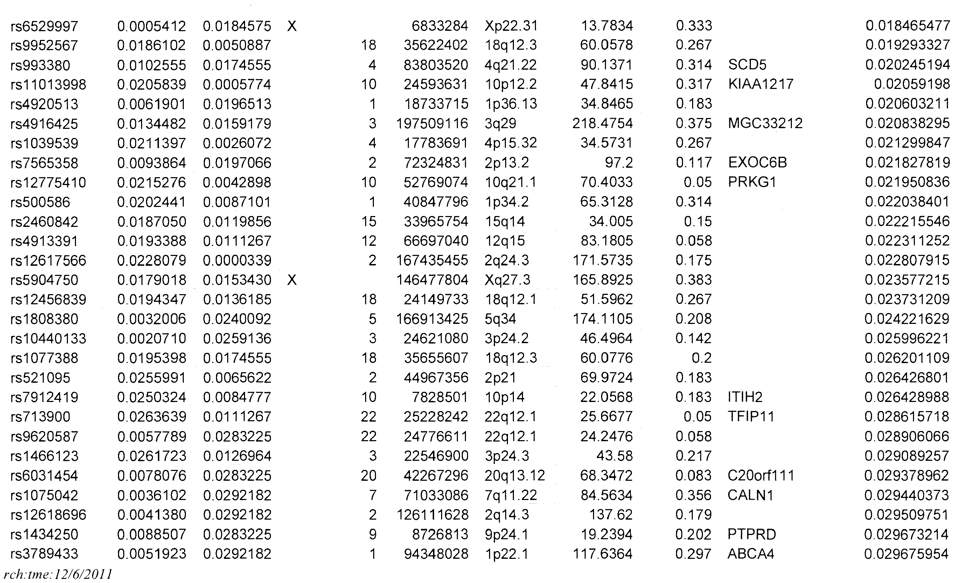

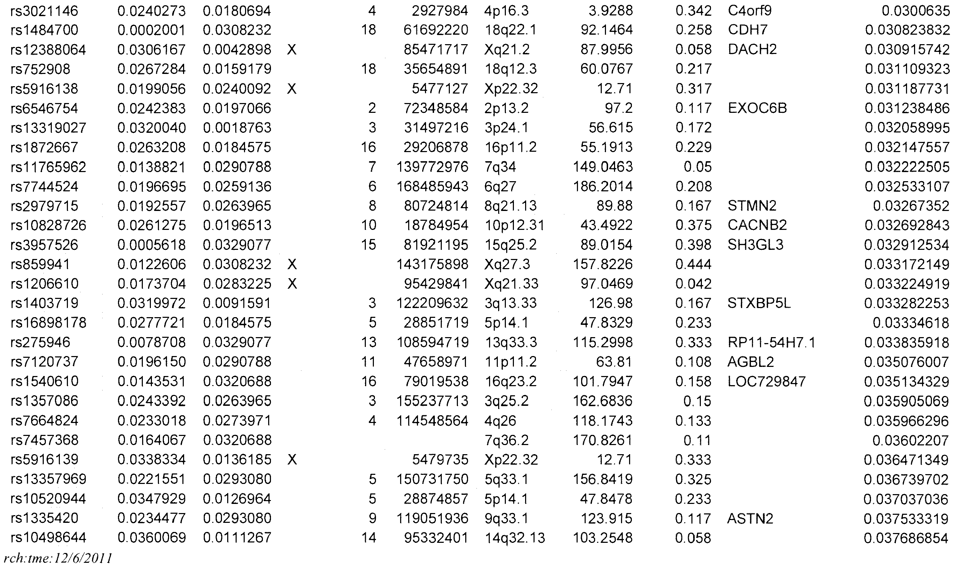

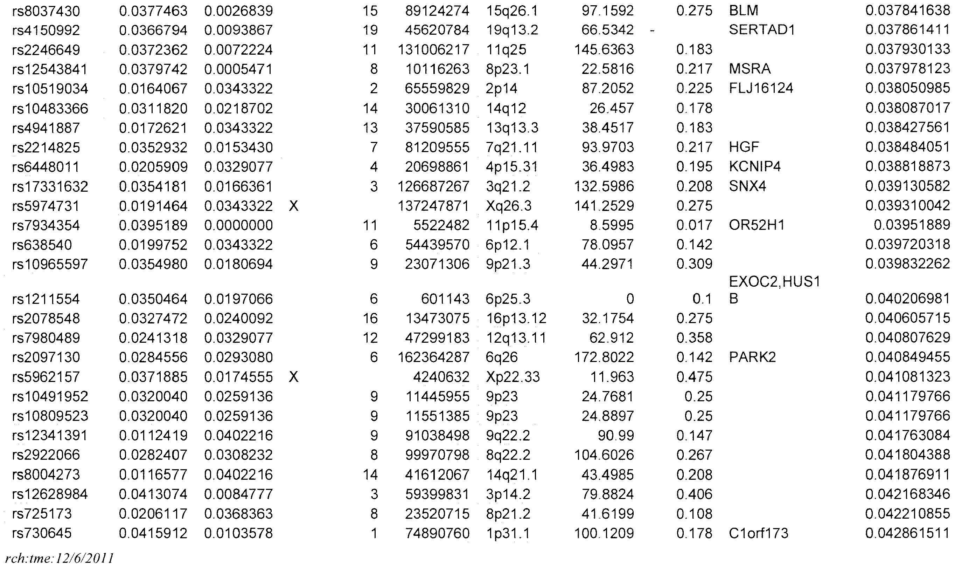

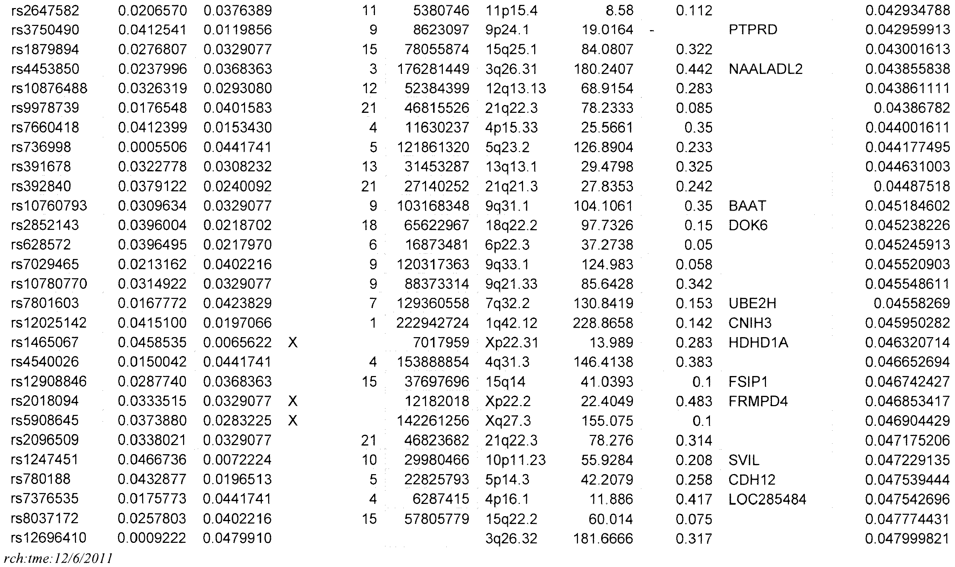

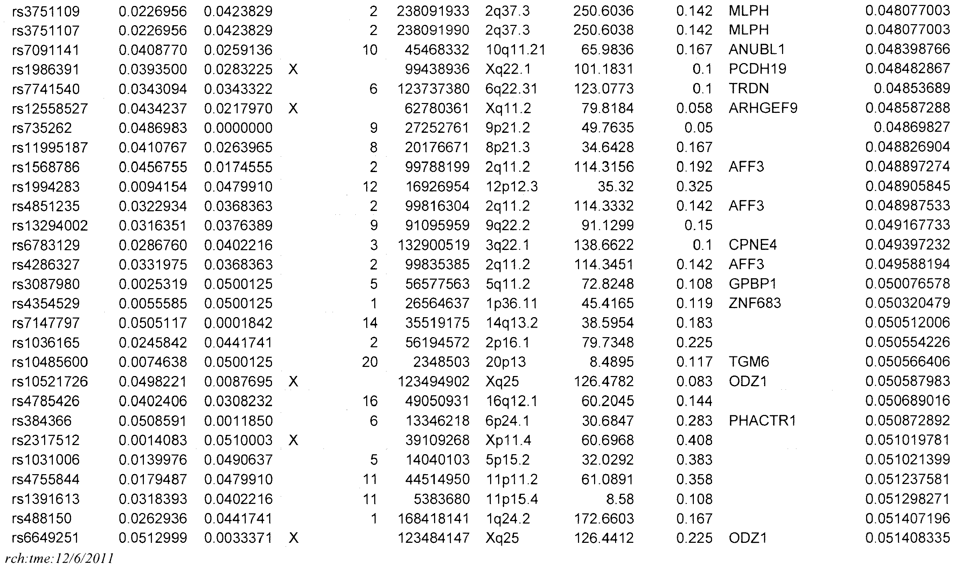

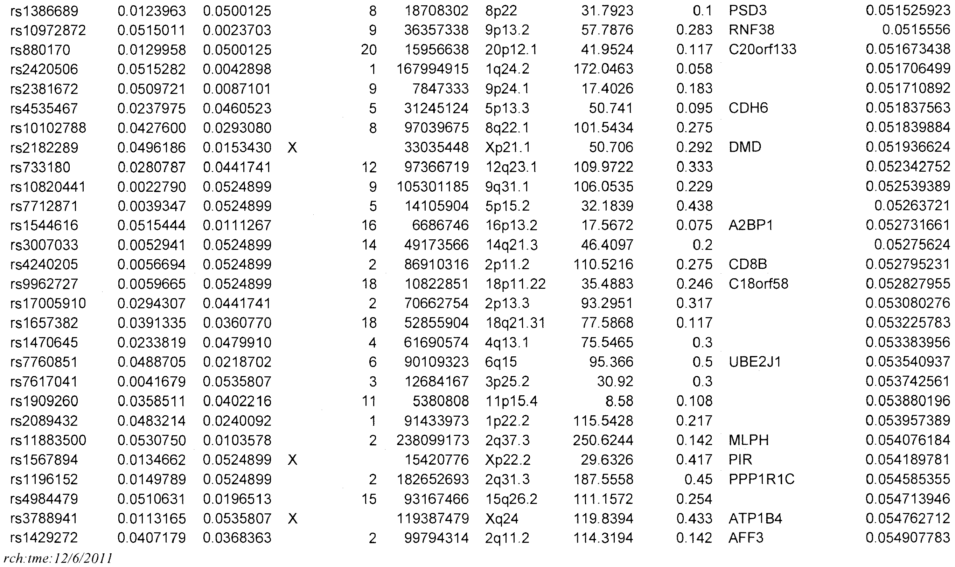

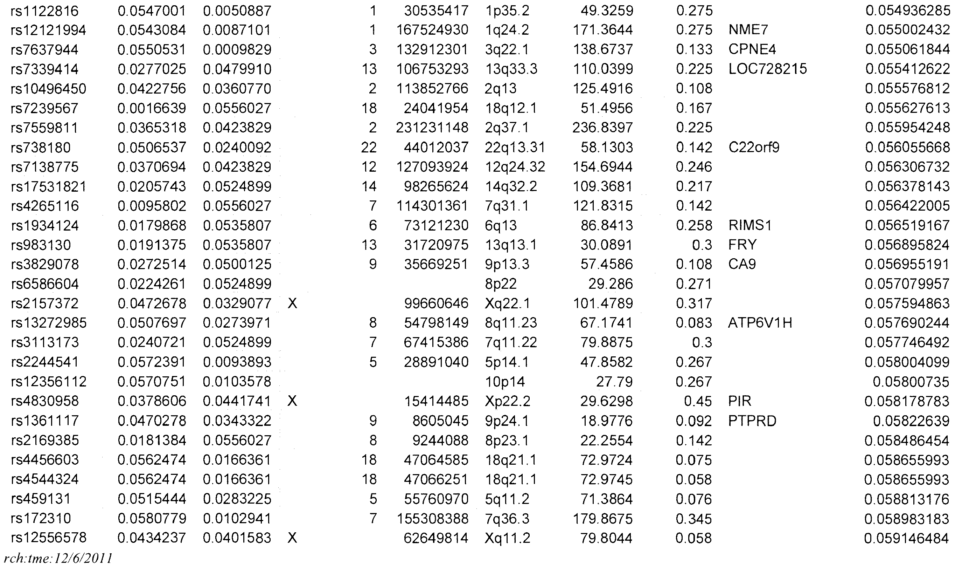

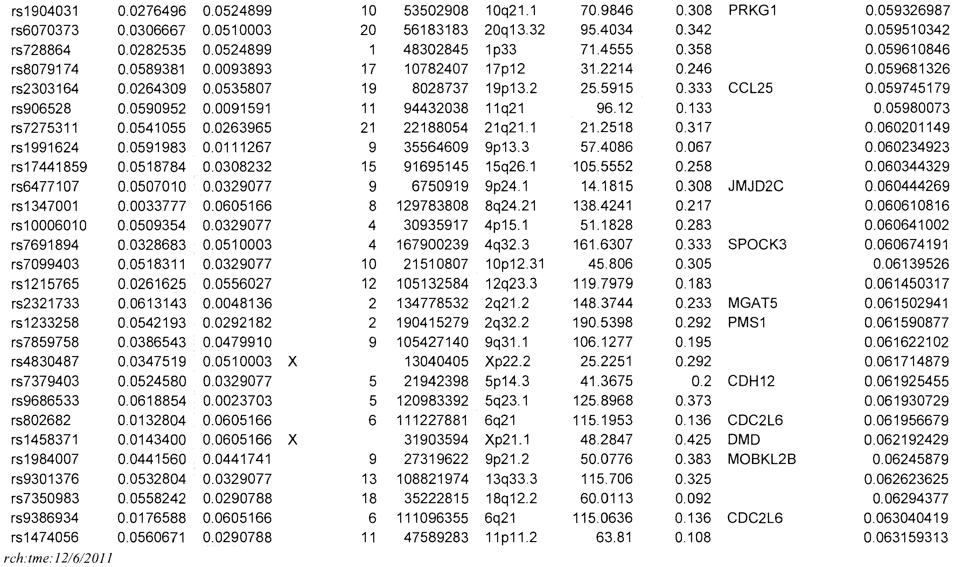

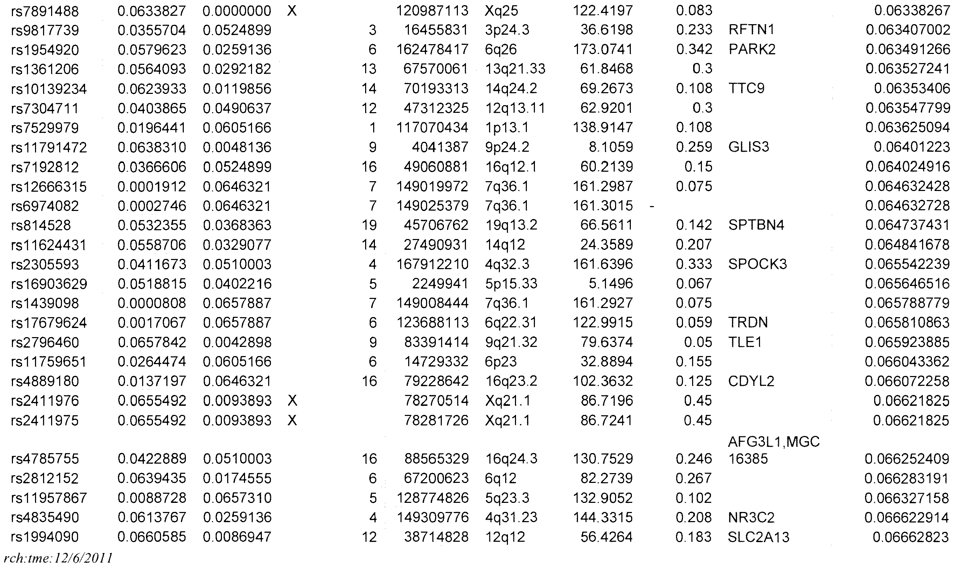

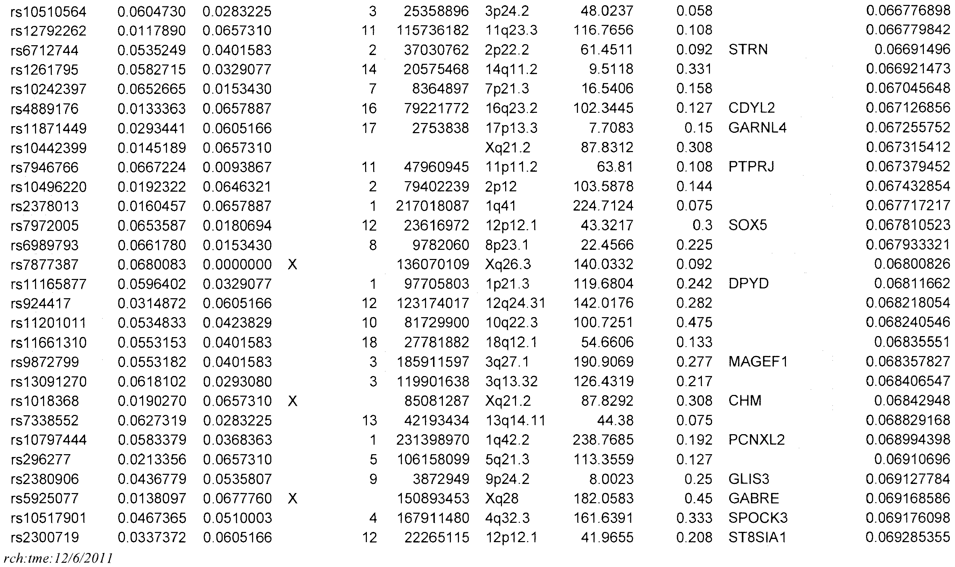

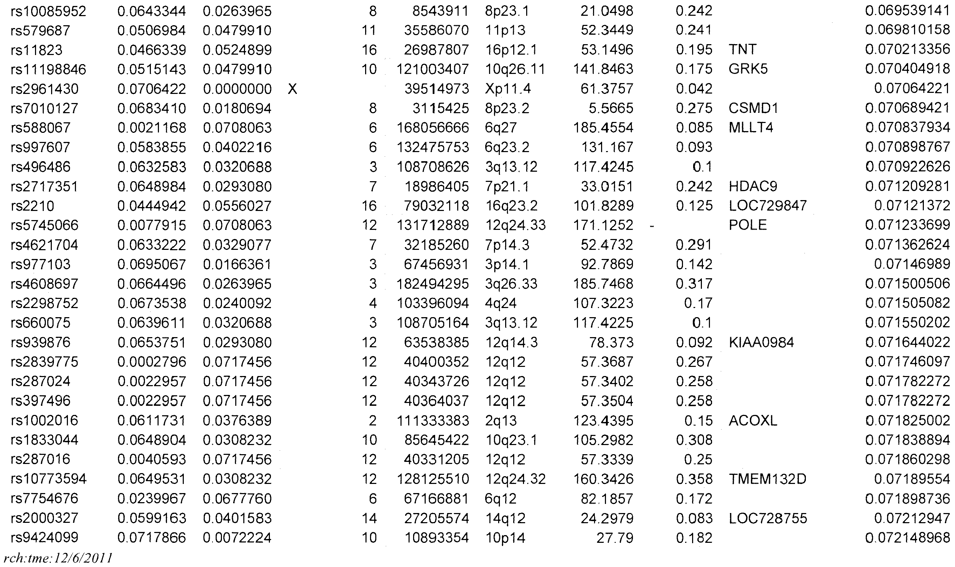

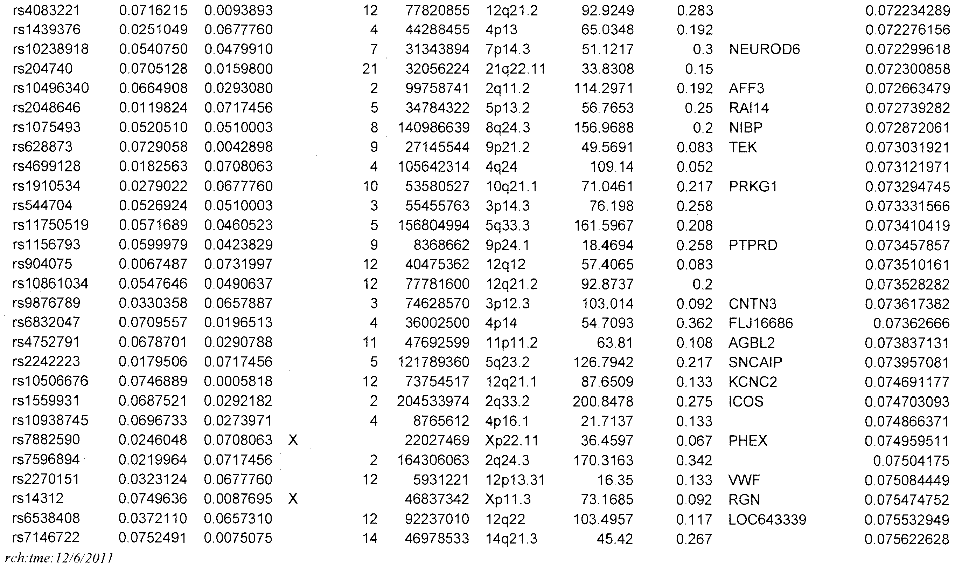

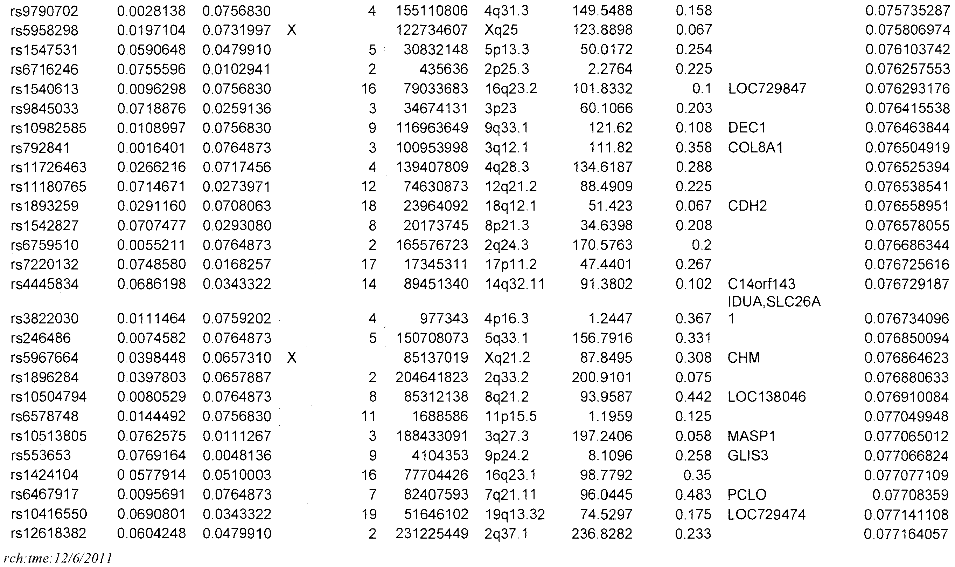

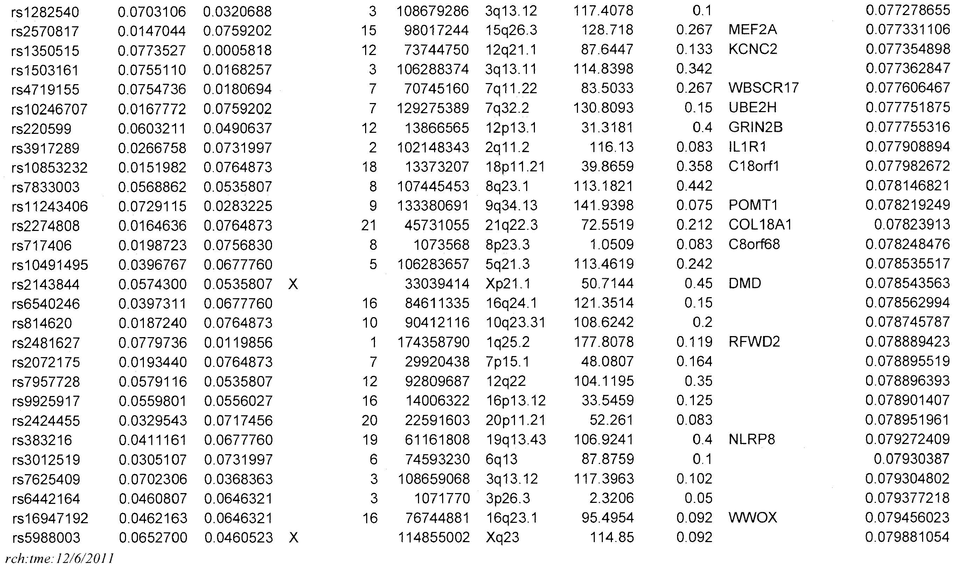

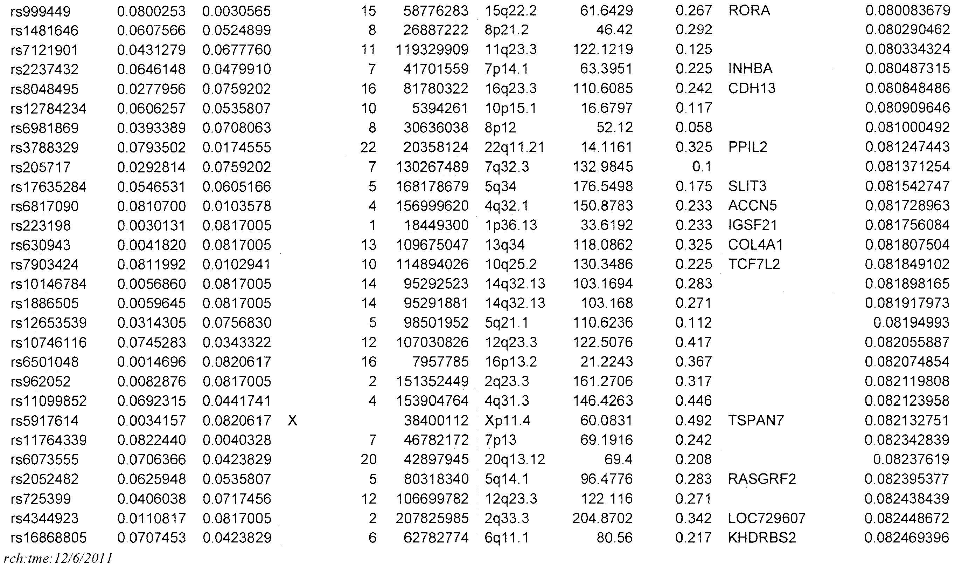

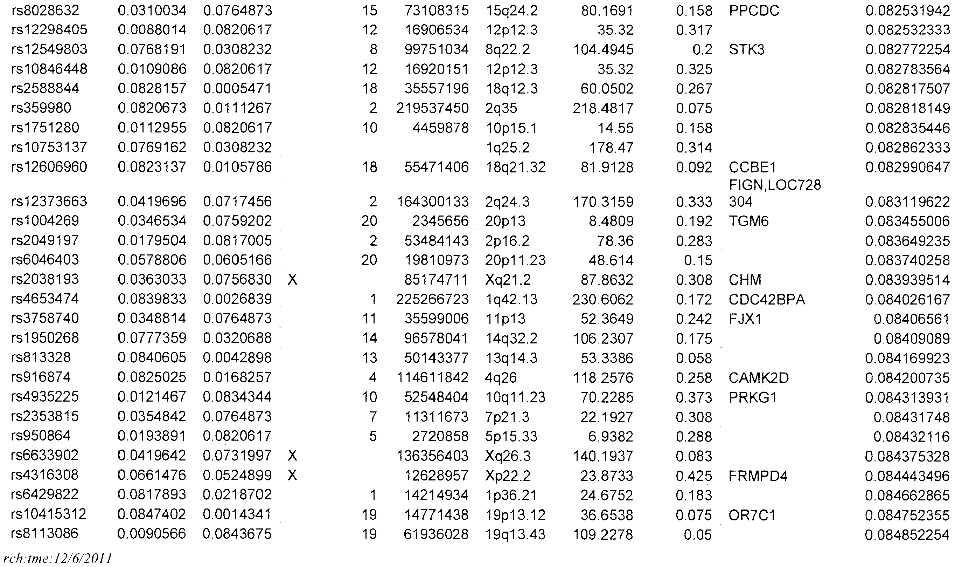

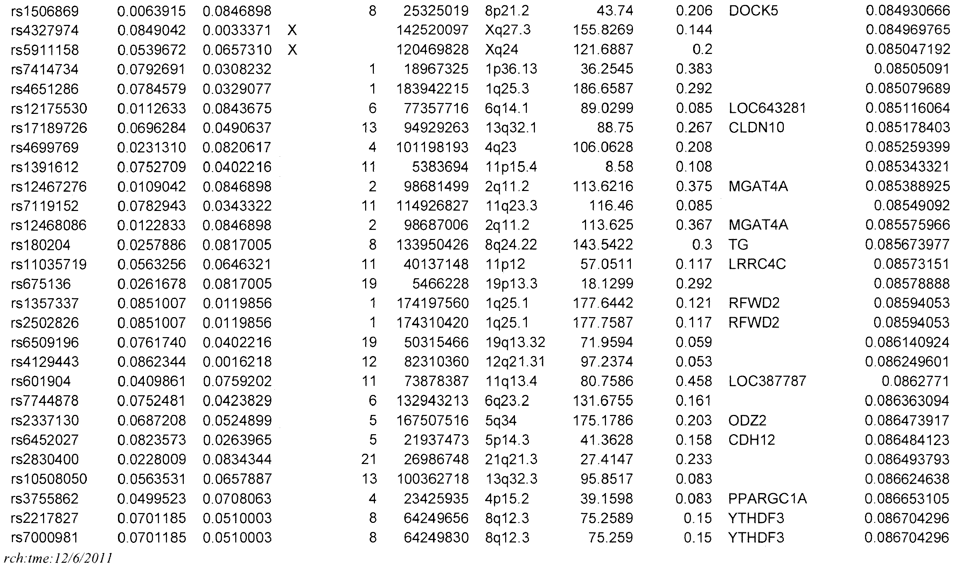

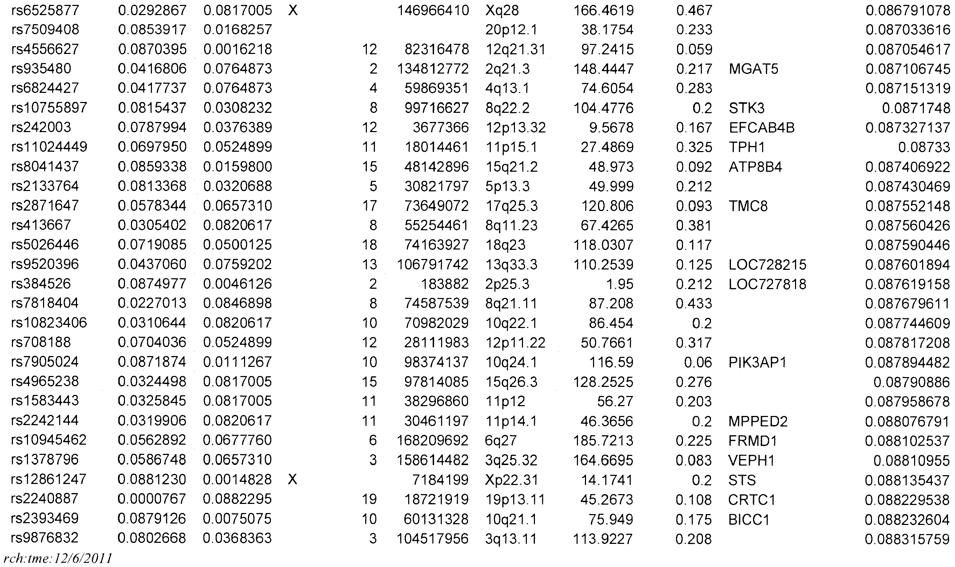

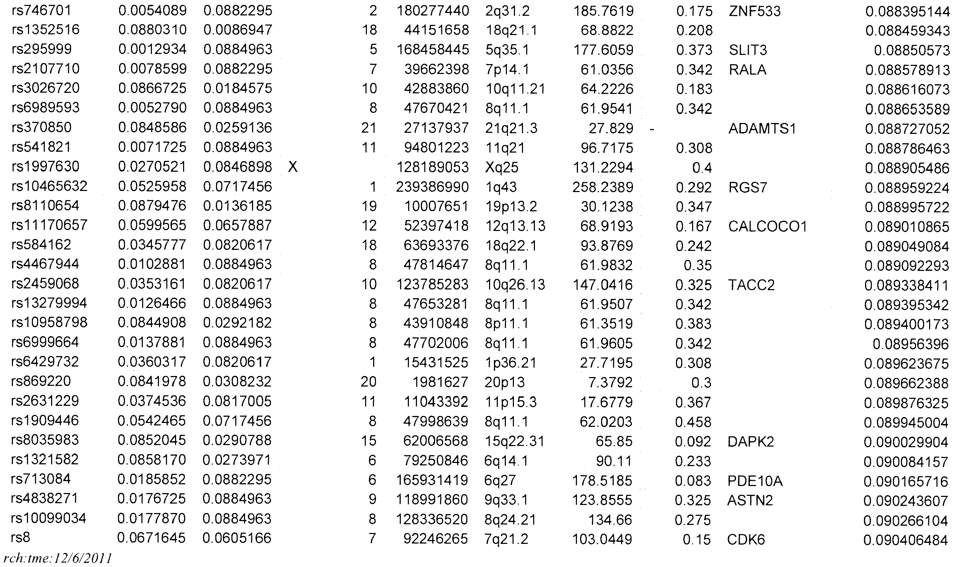

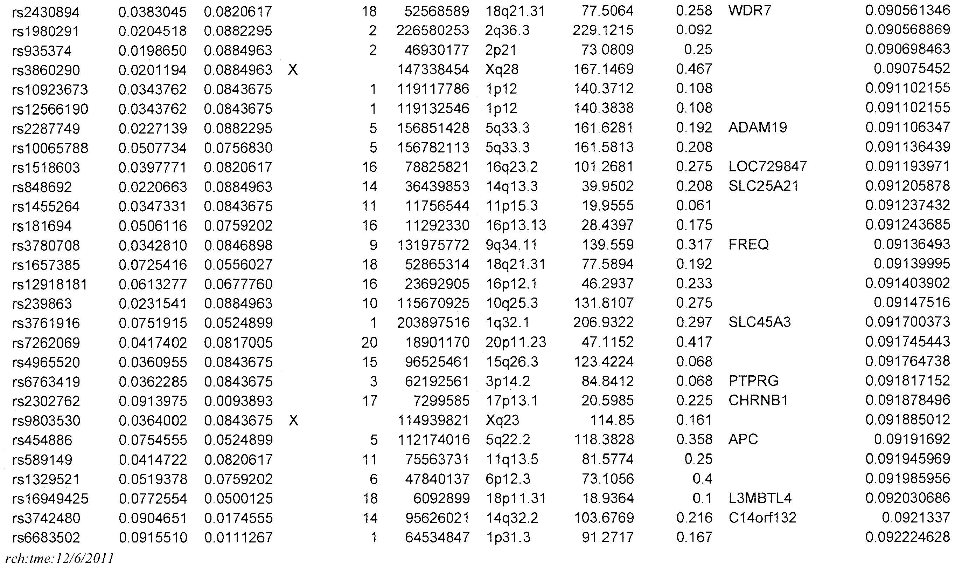

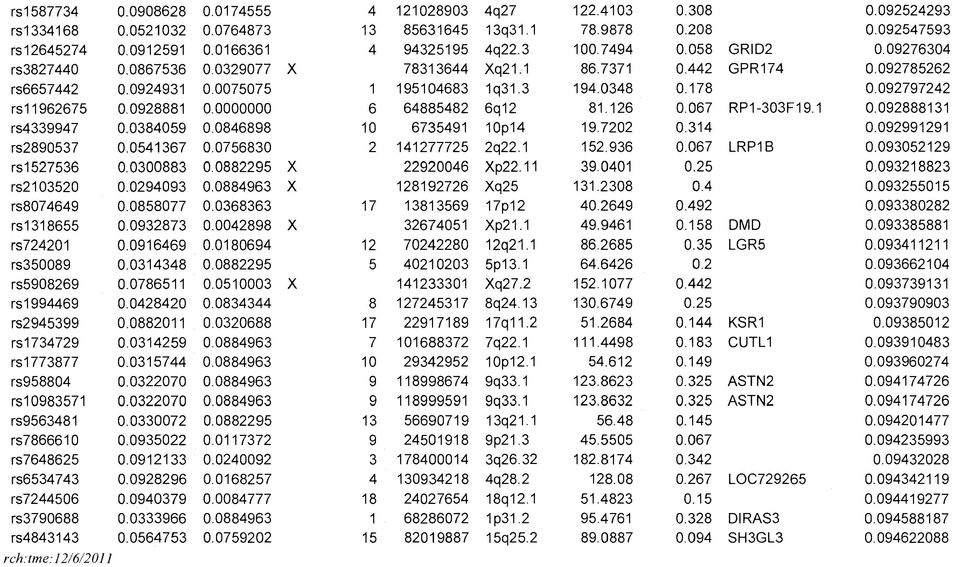

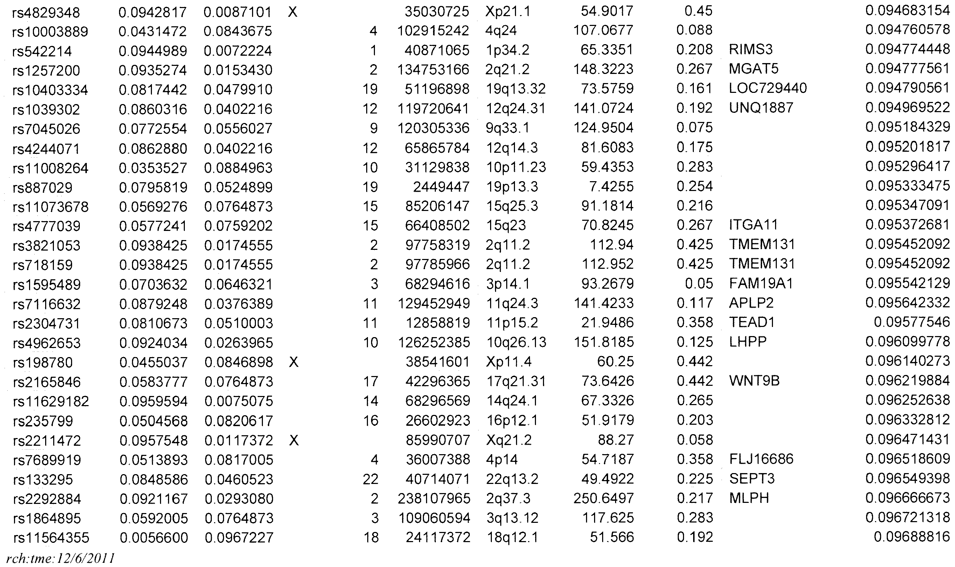

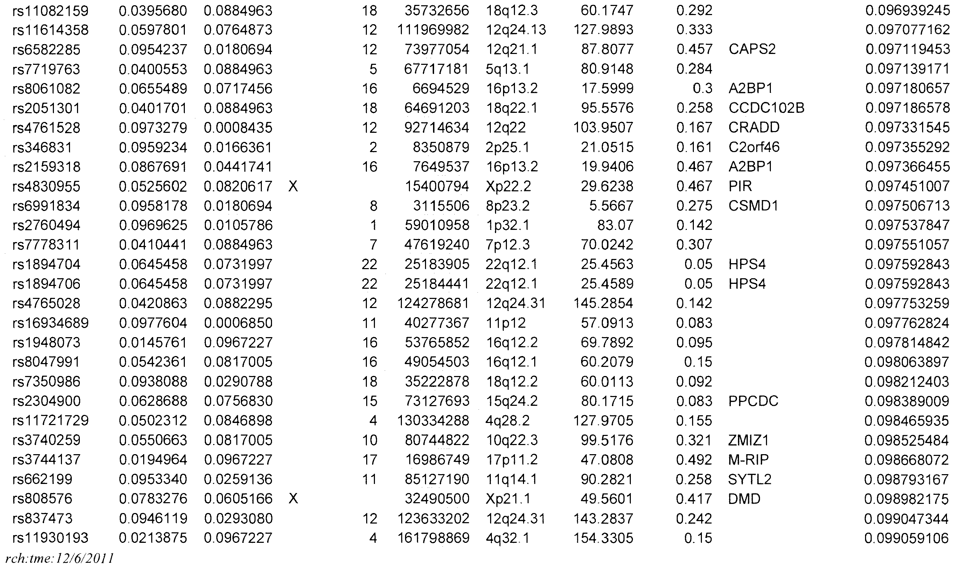

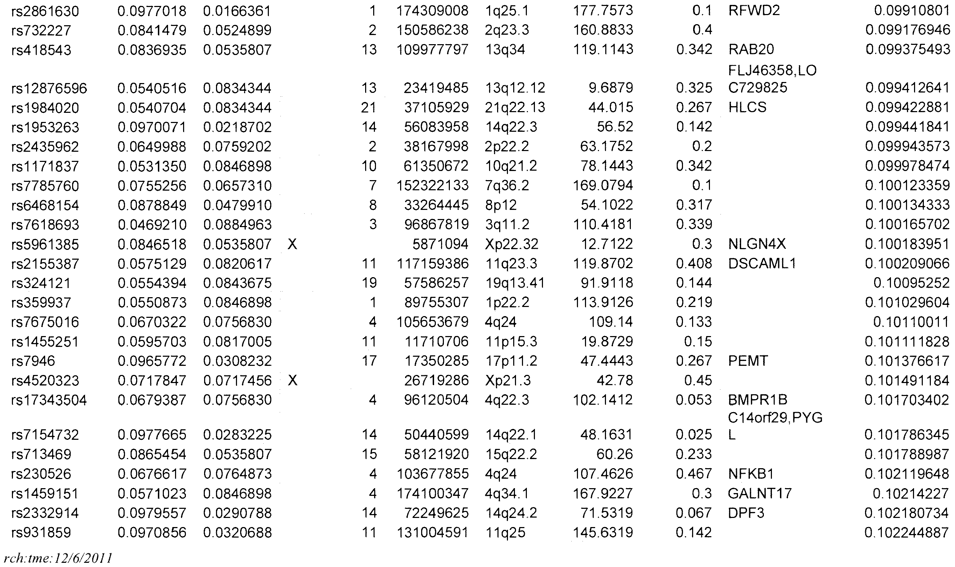

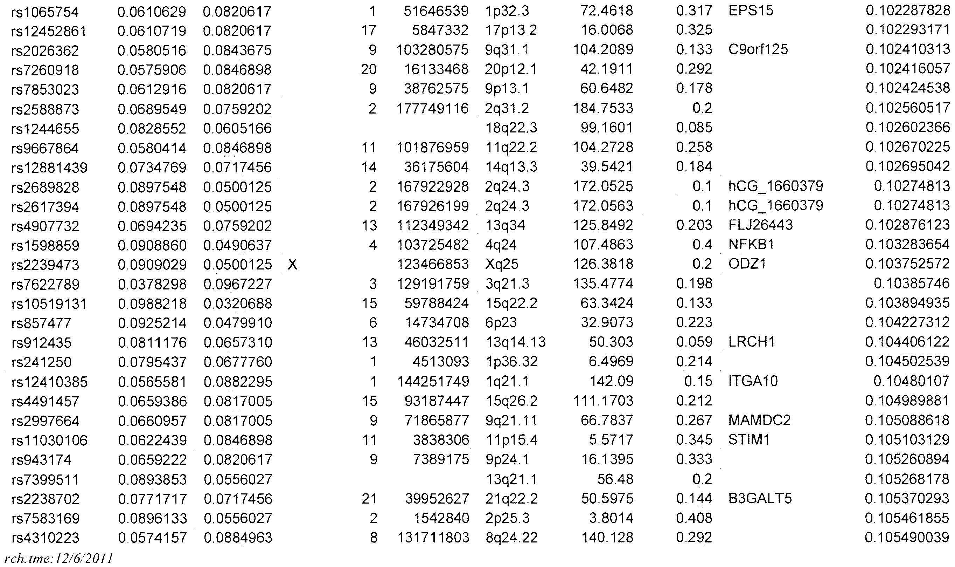

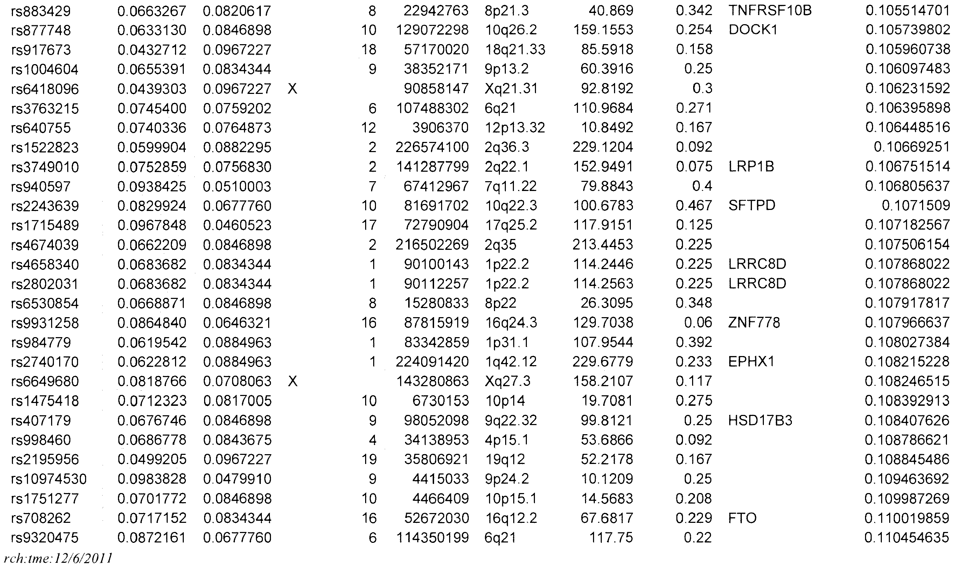

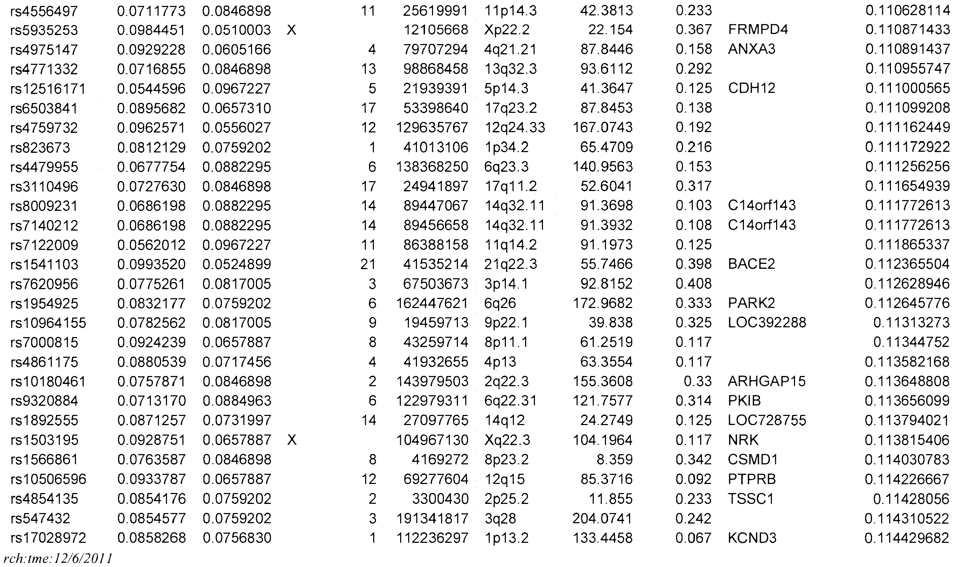

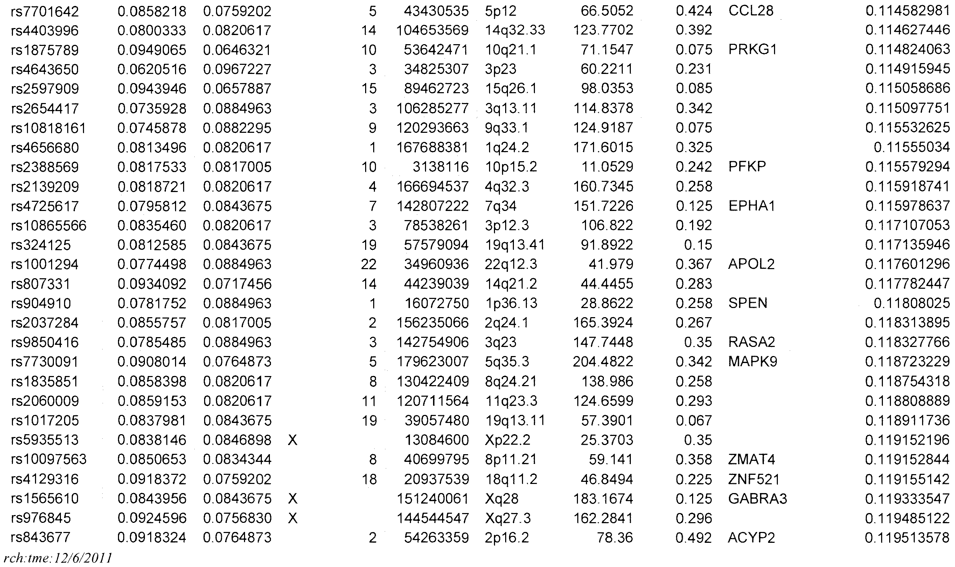

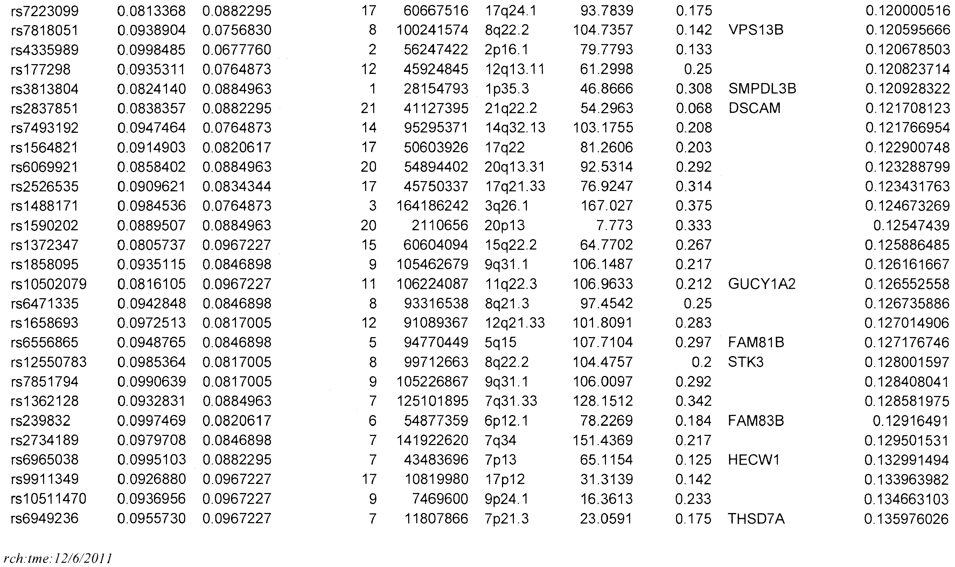

Furthermore, analysis was done using the data obtained from another study, namely the IDEA-VF, where SNP data from 37 ICD and 51 control patients was available. Again, the 317,503 SNPs in the MAPP study were scanned to identify the SNPs with $p \leq 0.1$, and 31,008 SNPs were found. These SNPs were tested in the IDEA-VF set, and only 849 of them were found to have $p \leq 0.1$, meaning that all 849 SNPs showed p values that were less than 0.1 in two independent studies. The chromosomal plot for these 849 SNPs with $p \leq 0.1$ for both MAPP and IDEA-VF are shown in Figure 6. Figures 7A, 7B and 7C contain a detailed table of all the 849 SNPs (SEQ ID Nos. 1 to 849) chosen based on logical, biological and statistical criteria. For SEQ ID Nos. 1-849 of the Sequence Listing of the invention, the SNP is located at position 51.

To determine the presence or absence of an SNP in an individual or patient, an array having nucleotide probes from each of the sequences listed in SEQ ID Nos. 1 to 849 can be constructed where each probe is a different nucleotide sequence from 3 to 101 base pairs overlapping the SNP at position 51. In a further embodiment, the nucleotide probes are from each of the sequences listed in SEQ ID Nos. 850-858 and can be constructed where each probe is a different nucleotide sequence from 3 to 101 base pairs overlapping the SNP at position 26 or 27. In a further embodiment, the sequences of SEQ ID Nos. 1 to 858 can be individually used to monitor loss of heterozygosity, identify imprinted genes; genotype polymorphisms, determine allele frequencies in a population, characterize bi-allelic or multi-allelic markers, produce genetic maps, detect linkage disequilibrium, determine allele frequencies, do association studies, analyze genetic variation, or to identify markers linked to a phenotype or, compare genotypes between different individuals or populations.

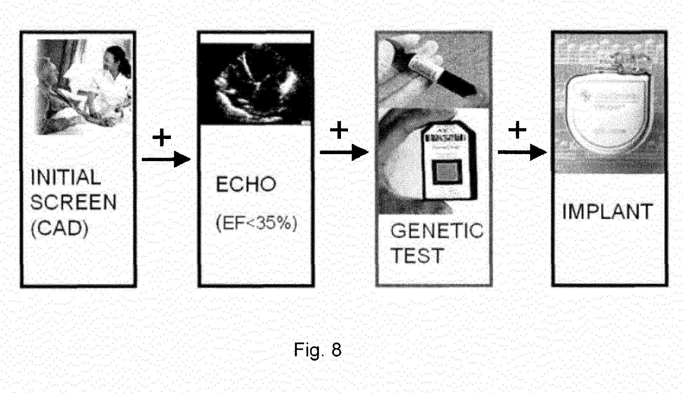

Figure 8 depicts one embodiment of a clinical utilization of the genetic test created for screening of patients for susceptibility to life threatening arrhythmias. In this embodiment, patients already testing positively for CAD and a low EF would undergo the test for genetic susceptibility using any of the methods described herein. Positive genetic test results would then be used in conjunction with the other test, such as the ones based on the analysis of ECG, and be used to make the ultimate decision of whether or not to implant an ICD.

Patients who are presenting a cardiac condition such as MI are usually subjected to echocardiographic examination to determine the need for an ICD. Based on the present invention, blood samples could also be taken from the patients who have low left ventricular EF. If the genetic tests in combination with the hemodynamic and demographic parameters indicate a high risk for sudden cardiac arrest, then a recommendation is made for an ICD implant. A schematic of this overall process is shown in Figure 8. A similar recommendation can be made for individuals with no previous history of cardiovascular disease based on a positive genetic screen for one or more of the SNPs taught herein in combination with one or more biological factors including markers, clinical parameters and the like.

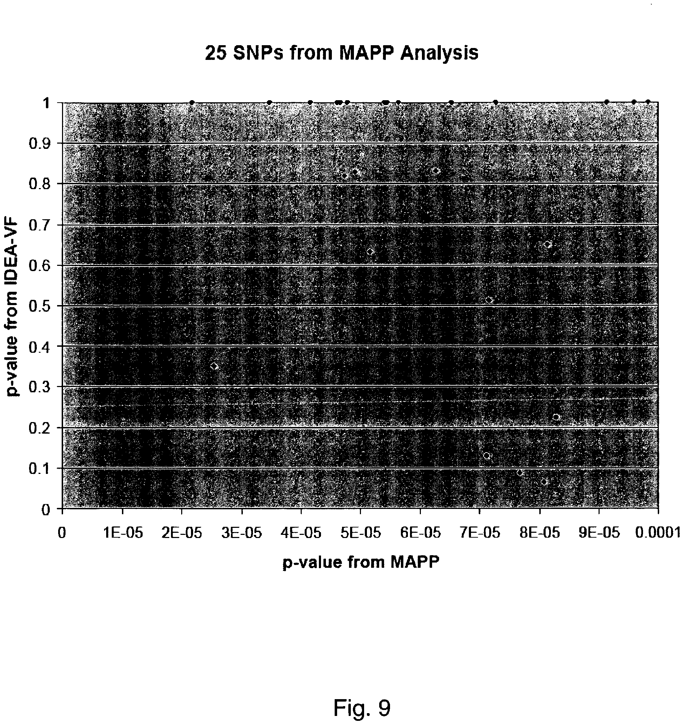

Figure 9 shows the performance of the genetic markers obtained from the MAPP Study when they were applied to the IDEA-VF patient population. Only the markers with MAPP p-values that are less than 0.0001 were tested. As it can be seen from this graph, not all the markers identified as highly significant in MAPP did not give low p-values when they are applied to the IDEA-VF population. A total of 25 SNPs are represented in Figure 9: rs4878412, rs2839372, rs10505726, rs10919336, rs6828580, rs16952330, rs2060117, rs9983892, rs1500325, rs1679414, rs486427, rs6480311, rs7305353, rs10823151, rs1346964, rs6790359, rs7591633, rs10487115, rs2240887, rs1439098, rs248670, rs4691391, rs2270801, rs12891099, and rs17694397.

Figures 16-40 contain mosaic plots illustrating the probability of experiencing LTA as a function of allele specific inheritance of the 25 SNPs represented in Figure 9. Figure 16 illustrates the resulting risk stratification of rs1439098. As shown in the plot, the presence of a at the SNP position indicates decreased susceptibility to SCA or SCD as compared to the presence of g at the SNP position. Patients with genotype a/a have about an 11% probability of experiencing SCA or SCD, while the a/g genotype indicates about a 47% probability, and the g/g genotype indicates about a 50% probability of experiencing SCA or SCD. Table 8 shows the statistical breakdown of the genotypes for this SNP.

The first (top) value in each cell in each of the statistical tables is the number, or count, of patients placed in that set. The second value is the percentage of the total number of patients placed in the set. The third value is the percentage of control or case patients (depending on the column) having a specific genotype from the total number of patients having that specific genotype. The fourth value is the percentage of patients from either the control or case patients (depending on the column) placed in the set. The bottom right cell is the total number of patients (100%) utilized for the SNP analysis.

TABLE 8

| Table of rs1439098 by arm | | | |
|---|---|---|---|
| rs1439098 | arm | | |
| Count<br>Frequency %<br>Row %<br>Col % | Control | Case | Total |
| AA | 195<br>81.59<br>89.45<br>94.66 | 23<br>9.62<br>10.55<br>69.70 | 218<br>91.21 |
| AG | 10<br>4.18<br>52.63<br>4.85 | 9<br>3.77<br>47.37<br>27.27 | 19<br>7.95 |
| GG | 1<br>0.42<br>50.00<br>0.49 | 1<br>0.42<br>50.00<br>3.03 | 2<br>0.84 |
| Total | 206<br>86.19 | 33<br>13.81 | 239<br>100.00 |
| Frequency Missing = 1 | | | |

Figure 17 illustrates the resulting risk stratification of rs4878412. As shown in the plot, the presence of t at the SNP position indicates decreased susceptibility to SCA or SCD as compared to the presence of g at the SNP position. Patients with genotype t/t have about a 9% probability of experiencing SCA or SCD, while the t/g genotype indicates about a 35% probability, and the g/g genotype indicates greater than 99% probability of experiencing SCA or SCD. Table 9 shows the statistical breakdown of the genotypes for this SNP.

TABLE 9

| Table of rs4878412 by arm | | | |
|---|---|---|---|
| rs4878412 | arm | | |
| Count<br>Frequency %<br>Row %<br>Col % | Control | Case | Total |
| GG | 0<br>0.00<br>0.00<br>0.00 | 1<br>0.42<br>100.00<br>3.13 | 1<br>0.42 |
| GT | 24<br>10.13<br>64.86<br>11.71 | 13<br>5.49<br>35.14<br>40.63 | 37<br>15.61 |
| TT | 181<br>76.37<br>90.95<br>88.29 | 18<br>7.59<br>9.05<br>56.25 | 199<br>83.97 |
| Total | 205<br>86.50 | 32<br>13.50 | 237<br>100.00 |
| Frequency Missing = 3 | | | |

Figure 18 illustrates the resulting risk stratification of rs2839372. As shown in the plot, the presence of g at the SNP position indicates decreased susceptibility to SCA or SCD as compared to the presence of a at the SNP position. Patients with genotype g/g have about a 9% probability of experiencing SCA or SCD, while the g/a genotype indicates about a 15% probability, and the a/a genotype indicates about a 62% probability of experiencing SCA or SCD. Table 10 shows the statistical breakdown of the genotypes for this SNP.

TABLE 10

| Table of rs2839372 by arm | | | |
|---|---|---|---|
| rs2839372 | arm | | |
| Count<br>Frequency %<br>Row %<br>Col % | Control | Case | Total |
| AA | 5<br>2.10<br>38.46<br>2.43 | 8<br>3.36<br>61.54<br>25.00 | 13<br>5.46 |
| AG | 64<br>26.89<br>85.33<br>31.07 | 11<br>4.62<br>14.67<br>34.38 | 75<br>31.51 |
| GG | 137<br>57.56<br>91.33<br>66.50 | 13<br>5.46<br>8.67<br>40.63 | 150<br>63.03 |
| Total | 206<br>86.55 | 32<br>13.45 | 238<br>100.00 |
| Frequency Missing = 2 | | | |

Figure 19 illustrates the resulting risk stratification of rs10505726. As shown in the plot, the presence of t at the SNP position indicates decreased susceptibility to SCA or SCD as compared to the presence of c at the SNP position. Patients with genotype t/t have about a 7% probability of experiencing SCA or SCD, while the t/c genotype indicates about a 30% probability, and the c/c genotype indicates about a 29% probability of experiencing SCA or SCD. Table 11 shows the statistical breakdown of the genotypes for this SNP.

TABLE 11

| Table of rs10505726 by arm | | | |
|---|---|---|---|
| rs10505726 | arm | | |
| Count<br>Frequency %<br>Row %<br>Col % | Control | Case | Total |
| CC | 5<br>2.08<br>71.43<br>2.42 | 2<br>0.83<br>28.57<br>6.06 | 7<br>2.92 |
| CT | 45<br>18.75<br>70.31<br>21.74 | 19<br>7.92<br>29.69<br>57.58 | 64<br>26.67 |
| TT | 157<br>65.42<br>92.90<br>75.85 | 12<br>5.00<br>7.10<br>36.36 | 169<br>70.42 |
| Total | 207<br>86.25 | 33<br>13.75 | 240<br>100.00 |

Figure 20 illustrates the resulting risk stratification of rs 10919336. As shown in the plot, the presence of a at the SNP position indicates increased susceptibility to SCA or SCD as compared to the presence of g at the SNP position. Patients with genotype a/a have about a 22% probability of experiencing SCA or SCD, while the a/g genotype indicates less than 5% probability, and the g/g genotype indicates about a 9% probability of experiencing SCA or SCD. Table 12 shows the statistical breakdown of the genotypes for this SNP.

TABLE 12

| Table of rs10919336 by arm | | | |
|---|---|---|---|
| rs10919336 | arm | | |
| Count<br>Frequency %<br>Row %<br>Col % | Control | Case | Total |
| AA | 101<br>42.62<br>77.69<br>49.51 | 29<br>12.24<br>22.31<br>87.88 | 130<br>54.85 |
| AG | 82<br>34.60<br>97.62<br>40.20 | 2<br>0.84<br>2.38<br>6.06 | 84<br>35.44 |
| GG | 21<br>8.86<br>91.30<br>10.29 | 2<br>0.84<br>8.70<br>6.06 | 23<br>9.70 |
| Total | 204<br>86.08 | 33<br>13.92 | 237<br>100.00 |
| Frequency Missing = 3 | | | |

Figure 21 illustrates the resulting risk stratification of rs6828580. As shown in the plot, the presence of g at the SNP position indicates decreased susceptibility to SCA or SCD as compared to the presence of a at the SNP position. Patients with genotype g/g have about an 8% probability of experiencing SCA or SCD, while the g/a genotype indicates about a 28% probability, and the a/a genotype indicates about a 50% probability of experiencing SCA or SCD. Table 13 shows the statistical breakdown for the genotypes of this SNP.

TABLE 13

| Table of rs6828580 by arm | | | |
|---|---|---|---|
| rs6828580 | arm | | |
| Count<br>Frequency %<br>Row %<br>Col % | Control | Case | Total |
| AA | 1<br>0.42<br>50.00<br>0.49 | 1<br>0.42<br>50.00<br>3.03 | 2<br>0.84 |
| AG | 48<br>20.08<br>71.64<br>23.30 | 19<br>7.95<br>28.36<br>57.58 | 67<br>28.03 |
| GG | 157<br>65.69<br>92.35<br>76.21 | 13<br>5.44<br>7.65<br>39.39 | 170<br>71.13 |
| Total | 206<br>86.19 | 33<br>13.81 | 239<br>100.00 |
| Frequency Missing = 1 | | | |

Figure 22 illustrates the resulting risk stratification of rs16952330. As shown in the plot, the presence of a at the SNP position indicates increased susceptibility to SCA or SCD as compared to the presence of g at the SNP position. Patients with genotype a/a have about an 11% probability of experiencing SCA or SCD, while the a/g genotype indicates about a 70% probability, and no patients in the case or control populations had the genotype g/g. Table 14 shows the statistical breakdown of the genotypes for this SNP.

TABLE 14

| Table of rs16952330 by arm | | | |
|---|---|---|---|
| rs16952330 | arm | | |
| Count<br>Frequency %<br>Row Pct<br>Col Pct | Control | Case | Total |
| AA | 203<br>84.94<br>88.65<br>98.54 | 26<br>10.88<br>11.35<br>78.79 | 229<br>95.82 |
| AG | 3<br>1.26<br>30.00<br>1.46 | 7<br>2.93<br>70.00<br>21.21 | 10<br>4.18 |
| Total | 206<br>86.19 | 33<br>13.81 | 239<br>100.00 |
| Frequency Missing = 1 | | | |

Figure 23 illustrates the resulting risk stratification of rs2060117. As shown in the plot, the presence of c at the SNP position indicates decreased susceptibility to SCA or SCD as compared to the presence of t at the SNP position. Patients with genotype c/c have about a 7% probability of experiencing SCA or SCD, while the c/t genotype indicates about a 29% probability, and the t/t genotype indicates about a 33% probability of experiencing SCA or SCD. Table 15 shows the statistical breakdown of the genotypes for this SNP.

TABLE 15

| Table of rs2060117 by arm | | | |
|---|---|---|---|
| rs2060117 | arm | | |
| Count<br>Frequency %<br>Row Pct<br>Col Pct | Control | Case | Total |
| CC | 156<br>65.00<br>92.86<br>75.36 | 12<br>5.00<br>7.14<br>36.36 | 168<br>70.00 |
| CT | 45<br>18.75<br>71.43<br>21.74 | 18<br>7.50<br>28.57<br>54.55 | 63<br>26.25 |
| TT | 6<br>2.50<br>66.67<br>2.90 | 3<br>1.25<br>33.33<br>9.09 | 9<br>3.75 |
| Total | 207<br>86.25 | 33<br>13.75 | 240<br>100.00 |

Figure 24 illustrates the resulting risk stratification of rs9983892. As shown in the plot, the presence of a at the SNP position indicates decreased susceptibility to SCA or SCD as compared to the presence of c at the SNP position. Patients with genotype a/a have about a 19% probability of experiencing SCA or SCD, while the a/c genotype indicates less than 5% probability, and the c/c genotype indicates about a 27% probability of experiencing SCD or SCA. Table 16 shows the statistical breakdown of the genotypes for this SNP.

TABLE 16

| Table of rs9983892 by arm | | | |
|---|---|---|---|
| rs9983892 | arm | | |
| Count<br>Frequency %<br>Row %<br>Col % | Control | Case | Total |
| AA | 84<br>35.90<br>80.77<br>41.38 | 20<br>8.55<br>19.23<br>64.52 | 104<br>44.44 |
| AC | 97<br>41.45<br>97.00<br>47.78 | 3<br>1.28<br>3.00<br>9.68 | 100<br>42.74 |
| CC | 22<br>9.40<br>73.33<br>10.84 | 8<br>3.42<br>26.67<br>25.81 | 30<br>12.82 |
| Total | 203<br>86.75 | 31<br>13.25 | 234<br>100.00 |
| Frequency Missing = 6 | | | |

Figure 25 illustrates the resulting risk stratification of rs1500325. As shown in the plot, the presence of t at the SNP position indicates decreased susceptibility to SCA or SCD as compared to the presence of c at the SNP position. Patients with genotype t/t have less than 5% probability of experiencing SCA or SCD, while the t/c genotype indicates about a 21% probability, and the c/c genotype indicates about a 26% probability of experiencing SCA or SCD. Table 17 shows the statistical breakdown of the genotypes for this SNP.

TABLE 17

| Table of rs1500325 by arm | | | |
|---|---|---|---|
| rs1500325 | arm | | |
| Count<br>Frequency %<br>Row %<br>Col % | Control | Case | Total |
| CC | 23<br>9.58<br>74.19<br>11.11 | 8<br>3.33<br>25.81<br>24.24 | 31<br>12.92 |
| CT | 80<br>33.33<br>79.21<br>38.65 | 21<br>8.75<br>20.79<br>63.64 | 101<br>42.08 |
| TT | 104<br>43.33<br>96.30<br>50.24 | 4<br>1.67<br>3.70<br>12.12 | 108<br>45.00 |
| Total | 207<br>86.25 | 33<br>13.75 | 240<br>100.00 |

Figure 26 illustrates the resulting risk stratification of rs1679414. As shown in the plot, the presence of g at the SNP position indicates decreased susceptibility to SCA or SCD as compared to the presence of t at the SNP position. Patients with genotype g/g have about a 15% probability of experiencing SCA or SCD, while the g/t genotype indicates less than 5% probability, and the t/t genotype indicates greater than 99% probability of experiencing SCA or SCD. Table 18 shows the statistical breakdown of the genotypes for this SNP.

TABLE 18

| Table of rs1679414 by arm | | | |
|---|---|---|---|
| rs1679414 | arm | | |
| Count<br>Frequency %<br>Row %<br>Col % | Control | Case | Total |
| GG | 157<br>65.97<br>85.33<br>75.85 | 27<br>11.34<br>14.67<br>87.10 | 184<br>77.31 |
| GT | 50<br>21.01<br>98.04<br>24.15 | 1<br>0.42<br>1.96<br>3.23 | 51<br>21.43 |
| TT | 0<br>0.00<br>0.00<br>0.00 | 3<br>1.26<br>100.00<br>9.68 | 3<br>1.26 |
| Total | 207<br>86.97 | 31<br>13.03 | 238<br>100.00 |
| Frequency Missing = 2 | | | |

Figure 27 illustrates the resulting risk stratification of rs486427. As shown in the plot, the presence of c at the SNP position indicates increased susceptibility to SCA or SCD as compared to the presence of a the SNP position. Patients with genotype c/c have about a 26% probability of experiencing SCA or SCD, while the c/a genotype indicates about an 8% probability, and the a/a genotype indicates less than 1% probability of experiencing SCA or SCD. Table 19 shows the statistical breakdown of the genotypes for this SNP.

TABLE 19

| Table of rs486427 by arm | | | |
|---|---|---|---|
| rs486427 | arm | | |
| Count<br>Frequency %<br>Row %<br>Col % | Control | Case | Total |
| AA | 30<br>12.50<br>100.00<br>14.49 | 0<br>0.00<br>0.00<br>0.00 | 30<br>12.50 |
| AC | 107<br>44.58<br>92.24<br>51.69 | 9<br>3.75<br>7.76<br>27.27 | 116<br>48.33 |
| CC | 70<br>29.17<br>74.47<br>33.82 | 24<br>10.00<br>25.53<br>72.73 | 94<br>39.17 |
| Total | 207<br>86.25 | 33<br>13.75 | 240<br>100.00 |

Figure 28 illustrates the resulting risk stratification of rs6480311. As shown in the plot, the presence of c at the SNP position indicates decreased susceptibility to SCA or SCD as compared to the presence of t at the SNP position. Patients with genotype c/c have about an 18% probability of experiencing SCA or SCD, while the c/t genotype indicates about a 5% probability, and the t/t genotype indicates about a 35% probability of experiencing SCA or SCD. Table 20 shows the statistical breakdown of the genotypes for this SNP.

TABLE 20

| Table of rs6480311 by arm | | | |
|---|---|---|---|
| rs6480311 | arm | | |
| Count<br>Frequency %<br>Row %<br>Col % | Control | Case | Total |
| CC | 83<br>34.58<br>82.18<br>40.10 | 18<br>7.50<br>17.82<br>54.55 | 101<br>42.08 |
| CT | 105<br>43.75<br>95.45<br>50.72 | 5<br>2.08<br>4.55<br>15.15 | 110<br>45.83 |
| TT | 19<br>7.92<br>65.52<br>9.18 | 10<br>4.17<br>34.48<br>30.30 | 29<br>12.08 |
| Total | 207<br>86.25 | 33<br>13.75 | 240<br>100.00 |

Figure 29 illustrates the resulting risk stratification of rs11610690. As shown in the plot, the presence of t at the SNP position indicates decreased susceptibility to SCA or SCD as compared to the presence of c at the SNP position. Patients with genotype t/t have less than 5% probability of experiencing SCA or SCD, while the t/c genotype indicates about a 21% probability, and the c/c genotype indicates about a 22% probability of experiencing SCA or SCD. Table 21 shows the statistical breakdown of the genotypes for this SNP.

TABLE 21

| Table of rs11610690 by arm | | | |
|---|---|---|---|
| rs11610690 | arm | | |
| Count<br>Frequency %<br>Row %<br>Col % | Control | Case | Total |
| CC | 28<br>11.67<br>77.78<br>13.53 | 8<br>3.33<br>22.22<br>24.24 | 36<br>15.00 |
| CT | 83<br>34.58<br>79.05<br>40.10 | 22<br>9.17<br>20.95<br>66.67 | 105<br>43.75 |
| TT | 96<br>40.00<br>96.97<br>46.38 | 3<br>1.25<br>3.03<br>9.09 | 99<br>41.25 |
| Total | 207<br>86.25 | 33<br>13.75 | 240<br>100.00 |

Figure 30 illustrates the resulting risk stratification of rs10823151. As shown in the plot, the presence of g at the SNP position indicates decreased susceptibility to SCA or SCD as compared to the presence of a at the SNP position. Patients with genotype g/g have about a 15% probability of experiencing SCA or SCD, while the g/a genotype indicates about a 5% probability, and the a/a genotype indicates about a 42% probability of experiencing SCA or SCD. Table 22 shows the statistical breakdown of the genotypes for this SNP.

TABLE 22

| Table of rs10823151 by arm | | | |
|---|---|---|---|
| rs10823151 | arm | | |
| Count<br>Frequency %<br>Row %<br>Col % | Control | Case | Total |
| AA | 14<br>5.93<br>58.33<br>6.90 | 10<br>4.24<br>41.67<br>30.30 | 24<br>10.17 |
| AG | 89<br>37.71<br>94.68<br>43.84 | 5<br>2.12<br>5.32<br>15.15 | 94<br>39.83 |
| GG | 100<br>42.37<br>84.75<br>49.26 | 18<br>7.63<br>15.25<br>54.55 | 118<br>50.00 |
| Total | 203<br>86.02 | 33<br>13.98 | 236<br>100.00 |
| Frequency Missing = 4 | | | |

Figure 31 illustrates the resulting risk stratification of rs 1346964. As shown in the plot, the presence of g at the SNP position indicates decreased susceptibility to SCA or SCD as compared to the presence of a at the SNP position. Patients with genotype g/g have about a 7% probability of experiencing SCA or SCD, while the g/a genotype indicates about a 27% probability, and the a/a genotype indicates about a 37% probability of experiencing SCA or SCD. Table 23 shows the statistical breakdown of the genotypes for this SNP.

TABLE 23

| Table of rs1346964 by arm | | | |
|---|---|---|---|
| rs1346964 | arm | | |
| Count<br>Frequency %<br>Row %<br>Col % | Control | Case | Total |
| AA | 5<br>2.16<br>62.50<br>2.49 | 3<br>1.30<br>37.50<br>10.00 | 8<br>3.46 |
| AG | 44<br>19.05<br>73.33<br>21.89 | 16<br>6.93<br>26.67<br>53.33 | 60<br>25.97 |
| GG | 152<br>65.80<br>93.25<br>75.62 | 11<br>4.76<br>6.75<br>36.67 | 163<br>70.56 |
| Total | 201<br>87.01 | 30<br>12.99 | 231<br>100.00 |
| Frequency Missing = 9 | | | |

Figure 32 illustrates the resulting risk stratification of rs6790359. As shown in the plot, the presence of t at the SNP position indicates decreased susceptibility to SCA or SCD as compared to the presence of c at the SNP position. Patients with genotype t/t have about a 65% probability of experiencing SCA or SCD, while the t/c genotype indicates about a 26% probability, and the c/c genotype indicates about a 14% probability of experiencing SCA or SCD. Table 24 shows the statistical breakdown of the genotypes for this SNP.

TABLE 24

| Table of rs6790359 by arm | | | |
|---|---|---|---|
| rs6790359 | arm | | |
| Count<br>Frequency %<br>Row Pct<br>Col Pct | Control | Case | Total |
| CC | 12<br>5.00<br>85.71<br>5.80 | 2<br>0.83<br>14.29<br>6.06 | 14<br>5.83 |
| CT | 65<br>27.08<br>73.86<br>31.40 | 23<br>9.58<br>26.14<br>69.70 | 88<br>36.67 |
| TT | 130<br>54.17<br>94.20<br>62.80 | 8<br>3.33<br>5.80<br>24.24 | 138<br>57.50 |
| Total | 207<br>86.25 | 33<br>13.75 | 240<br>100.00 |

Figure 33 illustrates the resulting risk stratification of rs7591633. As shown in the plot, the presence of g at the SNP position indicates decreased susceptibility to SCA or SCD as compared to the presence of a at the SNP position. Patients with genotype g/g have less than 5% probability of experiencing SCA or SCD, while the g/a genotype indicates about a 16% probability, and the a/a genotype indicates about a 31% probability of experiencing SCA or SCD. Table 25 shows the statistical breakdown of the genotypes for this SNP.

TABLE 25

| Table of rs7591633 by arm | | | |
|---|---|---|---|
| rs7591633 | arm | | |
| Count<br>Frequency %<br>Row %<br>Col % | Control | Case | Total |
| AA | 27<br>11.25<br>69.23<br>13.04 | 12<br>5.00<br>30.77<br>36.36 | 39<br>16.25 |
| AG | 94<br>39.17<br>83.93<br>45.41 | 18<br>7.50<br>16.07<br>54.55 | 112<br>46.67 |
| GG | 86<br>35.83<br>96.63<br>41.55 | 3<br>1.25<br>3.37<br>9.09 | 89<br>37.08 |
| Total | 207<br>86.25 | 33<br>13.75 | 240<br>100.00 |

Figure 34 illustrates the resulting risk stratification of rs10487115. As shown in the plot, the presence of c at the SNP position indicates decreased susceptibility to SCA or SCD as compared to the presence of a at the SNP position. Patients with genotype c/c have about a 10% probability of experiencing SCA or SCD, while the c/a genotype indicates about a 7% probability, and the a/a genotype indicates about a 32% probability of experiencing SCA or SCD. Table 26 shows the statistical breakdown of the genotypes for this SNP.

TABLE 26

| Table of rs10487115 by arm | | | |
|---|---|---|---|
| rs10487115 | arm | | |
| Count<br>Frequency %<br>Row %<br>Col % | Control | Case | Total |
| AA | 41<br>17.23<br>68.33<br>20.00 | 19<br>7.98<br>31.67<br>57.58 | 60<br>25.21 |
| AC | 107<br>44.96<br>93.04<br>52.20 | 8<br>3.36<br>6.96<br>24.24 | 115<br>48.32 |
| CC | 57<br>23.95<br>90.48<br>27.80 | 6<br>2.52<br>9.52<br>18.18 | 63<br>26.47 |
| Total | 205<br>86.13 | 33<br>13.87 | 238<br>100.00 |
| Frequency Missing = 2 | | | |

Figure 35 illustrates the resulting risk stratification of rs2240887. As shown in the plot, the presence of g at the SNP position indicates decreased susceptibility to SCA or SCD as compared to the presence of a at the SNP position. Patients with genotype g/g have about a 7% probability of experiencing SCA or SCD, while the g/a genotype indicates about a 30% probability, and the a/a genotype indicates about a 20% probability of experiencing SCA or SCD. Table 27 shows the statistical breakdown of the genotypes for this SNP.

TABLE 27

| Table of rs2240887 by arm | | | |
|---|---|---|---|
| rs2240887 | arm | | |
| Count<br>Frequency %<br>Row %<br>Col % | Control | Case | Total |
| AA | 8<br>3.33<br>80.00<br>3.86 | 2<br>0.83<br>20.00<br>6.06 | 10<br>4.17 |
| AG | 45<br>18.75<br>70.31<br>21.74 | 19<br>7.92<br>29.69<br>57.58 | 64<br>26.67 |
| GG | 154<br>64.17<br>92.77<br>74.40 | 12<br>5.00<br>7.23<br>36.36 | 166<br>69.17 |
| Total | 207<br>86.25 | 33<br>13.75 | 240<br>100.00 |

Figure 36 illustrates the resulting risk stratification of rs248670. As shown in the plot, the presence of t at the SNP position indicates decreased susceptibility to SCA or SCD as compared to the presence of c at the SNP position. Patients with genotype t/t have less than 5% probability of experiencing SCA or SCD, while the t/c genotype indicates about a 21 % probability, and the c/c genotype indicates about a 16% probability of experiencing SCA or SCD. Table 28 shows the statistical breakdown of the genotypes for this SNP.

TABLE 28

| Table of rs248670 by arm | | | |
|---|---|---|---|
| rs248670 | arm | | |
| Count<br>Frequency %<br>Row %<br>Col % | Control | Case | Total |
| CC | 42<br>17.50<br>84.00<br>20.29 | 8<br>3.33<br>16.00<br>24.24 | 50<br>20.83 |
| CT | 91<br>37.92<br>79.13<br>43.96 | 24<br>10.00<br>20.87<br>72.73 | 115<br>47.92 |
| TT | 74<br>30.83<br>98.67<br>35.75 | 1<br>0.42<br>1.33<br>3.03 | 75<br>31.25 |
| Total | 207<br>86.25 | 33<br>13.75 | 240<br>100.00 |

Figure 37 illustrates the resulting risk stratification of rs4691391. As shown in the plot, the presence of g at the SNP position indicates increased susceptibility to SCA or SCD as compared to the presence of a at the SNP position. Patients with genotype g/g have about a 9% probability of experiencing SCA or SCD, while the g/a genotype indicates about a 36% probability, and the a/a genotype indicates less than 1% probability of experiencing SCA or SCD. Table 29 shows the statistical breakdown of the genotypes for this SNP.

TABLE 29

| Table of rs4691391 by arm | | | |
|---|---|---|---|
| rs4691391 | arm | | |
| Count<br>Frequency %<br>Row %<br>Col % | Control | Case | Total |
| AA | 2<br>0.83<br>100.00<br>0.97 | 0<br>0.00<br>0.00<br>0.00 | 2<br>0.83 |
| AG | 27<br>11.25<br>64.29<br>13.04 | 15<br>6.25<br>35.71<br>45.45 | 42<br>17.50 |
| GG | 178<br>74.17<br>90.82<br>85.99 | 18<br>7.50<br>9.18<br>54.55 | 196<br>81.67 |
| Total | 207<br>86.25 | 33<br>13.75 | 240<br>100.00 |

Figure 38 illustrates the resulting risk stratification of rs2270801. As shown in the plot, the presence of c at the SNP position indicates increased susceptibility to SCA or SCD as compared to the presence of t at the SNP position. Patients with genotype c/c have about a 9% probability of experiencing SCA or SCD, while the c/t genotype indicates about a 36% probability, and the t/t genotype indicates less than 1% probability of experiencing SCA or SCD. Table 30 shows the statistical breakdown of the genotypes for this SNP.

TABLE 30

| Table of rs2270801 by arm | | | |
|---|---|---|---|
| rs2270801 | arm | | |
| Count<br>Frequency %<br>Row %<br>Col % | Control | Case | Total |
| CC | 177<br>73.75<br>90.77<br>85.51 | 18<br>7.50<br>9.23<br>54.55 | 195<br>81.25 |
| CT | 27<br>11.25<br>64.29<br>13.04 | 15<br>6.25<br>35.71<br>45.45 | 42<br>17.50 |
| TT | 3<br>1.25<br>100.00<br>1.45 | 0<br>0.00<br>0.00<br>0.00 | 3<br>1.25 |
| Total | 207<br>86.25 | 33<br>13.75 | 240<br>100.00 |

Figure 39 illustrates the resulting risk stratification of rs12891099. As shown in the plot, the presence of g at the SNP position indicates decreased susceptibility to SCA or SCD as compared to the presence of a at the SNP position. Patients with genotype g/g have about an 11% probability of experiencing SCA or SCD, while the g/a genotype indicates about a 10% probability, and the a/a genotype indicates about a 56% probability of experiencing SCA or SCD. Table 31 shows the statistical breakdown of the genotypes for this SNP.

TABLE 31

| Table of rs12891099 by arm | | | |
|---|---|---|---|
| rs12891099 | arm | | |
| Count<br>Frequency %<br>Row %<br>Col % | Control | Case | Total |
| AA | 7<br>2.92<br>43.75<br>3.38 | 9<br>3.75<br>56.25<br>27.27 | 16<br>6.67 |
| AG | 71<br>29.58<br>89.87<br>34.30 | 8<br>3.33<br>10.13<br>24.24 | 79<br>32.92 |
| GG | 129<br>53.75<br>88.97<br>62.32 | 16<br>6.67<br>11.03<br>48.48 | 145<br>60.42 |
| Total | 207<br>86.25 | 33<br>13.75 | 240<br>100.00 |

Figure 40 illustrates the resulting risk stratification of rs17694397. As shown in the plot, the presence of c at the SNP position indicates increased susceptibility to SCA or SCD as compared to the presence of t at the SNP position. Patients with genotype c/c have about an 8% probability of experiencing SCA or SCD, while the c/t genotype indicates about a 30% probability, and the t/t genotype indicates less than 1% probability of experiencing SCA or SCD. Table 32 shows the statistical breakdown of the genotypes for this SNP.

TABLE 32

| Table of rs17694397 by arm | | | |
|---|---|---|---|
| rs17694397 | arm | | |
| Count<br>Frequency %<br>Row %<br>Col % | Control | Case | Total |
| CC | 151<br>63.18<br>92.07<br>73.30 | 13<br>5.44<br>7.93<br>39.39 | 164<br>68.62 |
| CT | 47<br>19.67<br>70.15<br>22.82 | 20<br>8.37<br>29.85<br>60.61 | 67<br>28.03 |
| TT | 8<br>3.35<br>100.00<br>3.88 | 0<br>0.00<br>0.00<br>0.00 | 8<br>3.35 |
| Total | 206<br>86.19 | 33<br>13.81 | 239<br>100.00 |
| Frequency Missing = 1 | | | |

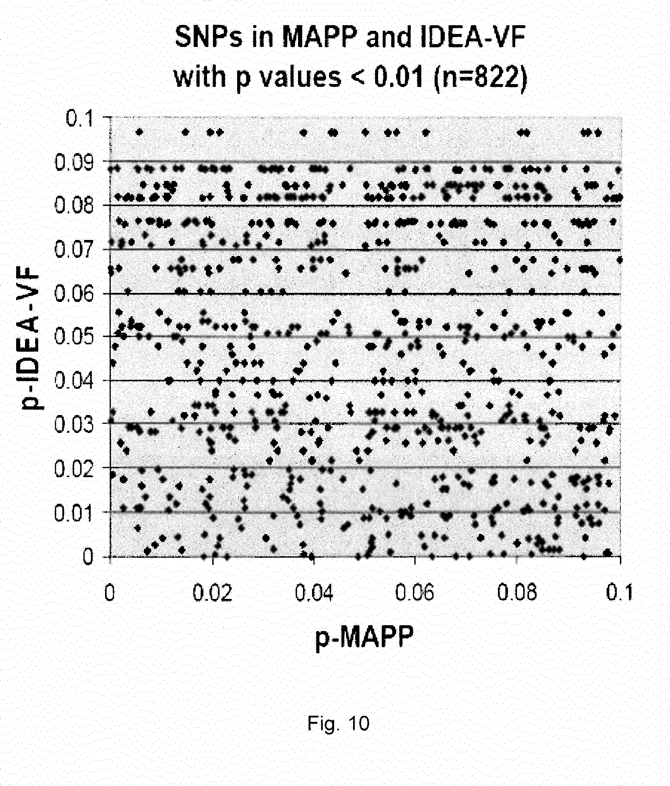

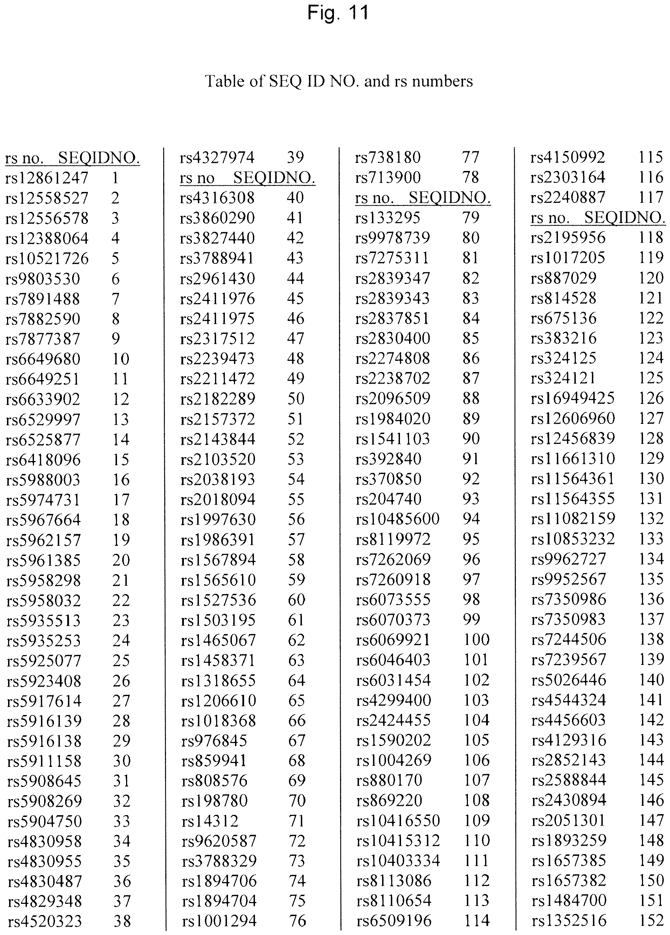

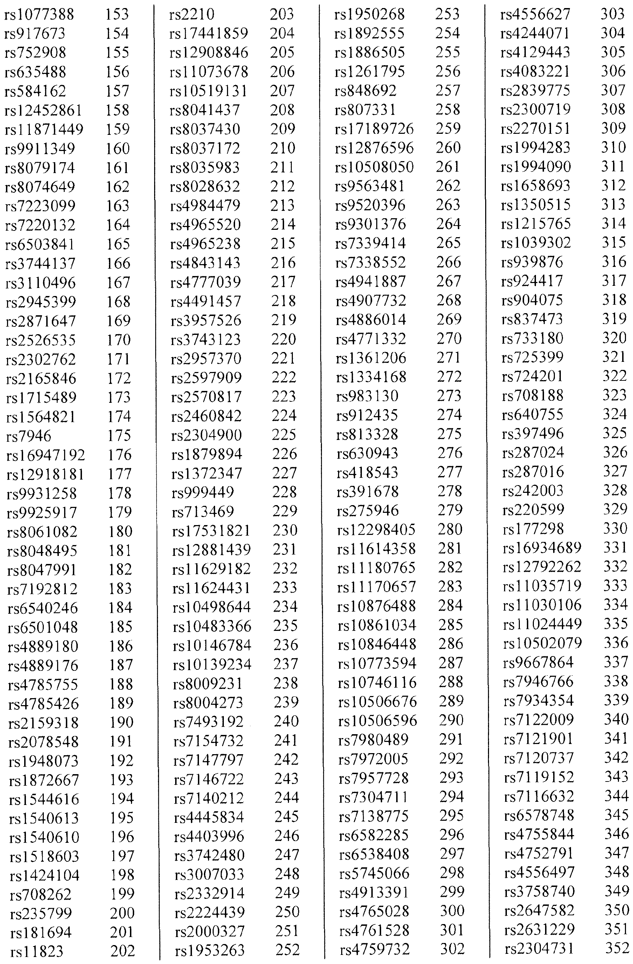

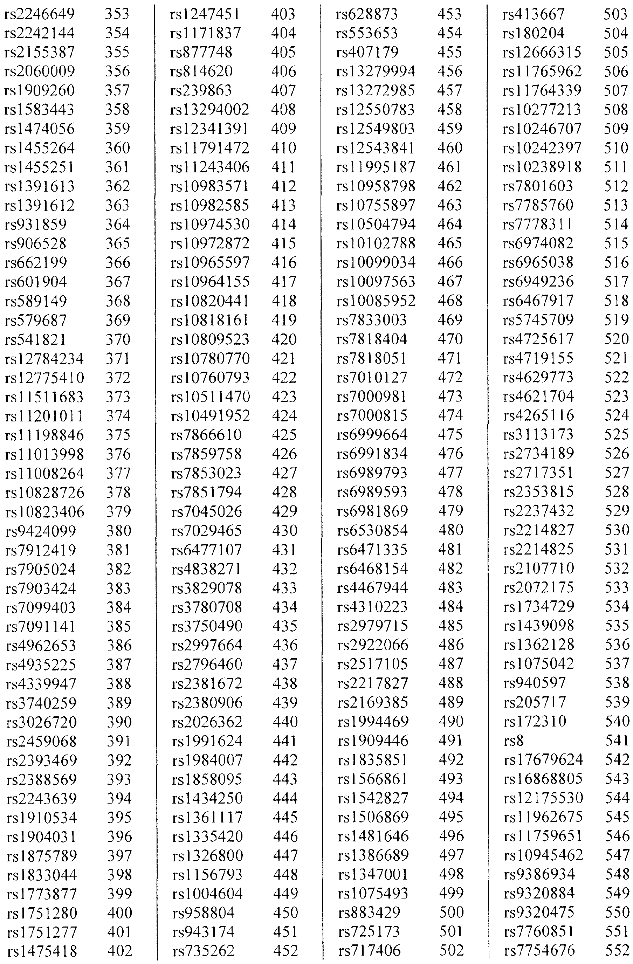

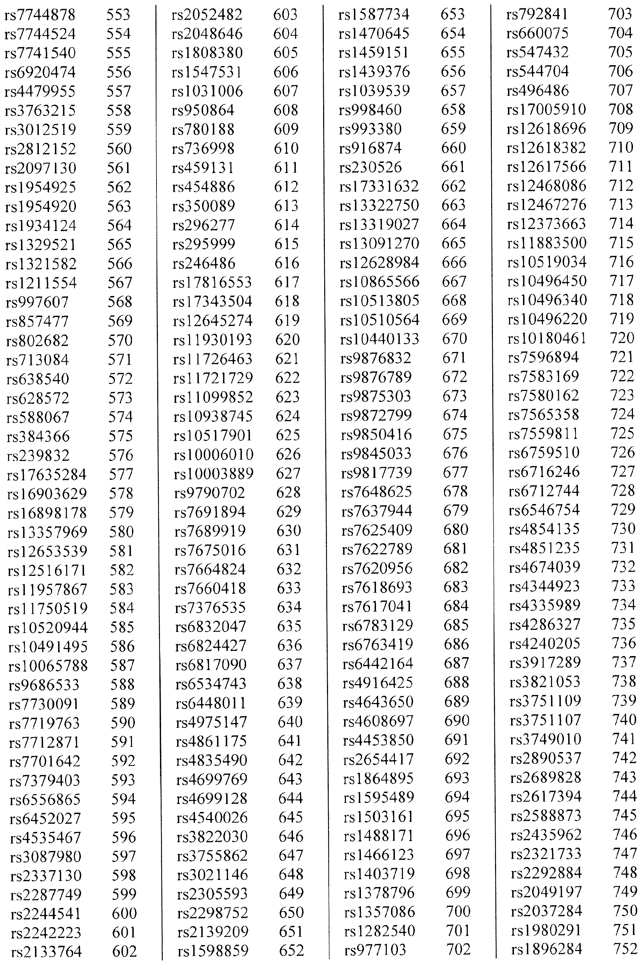

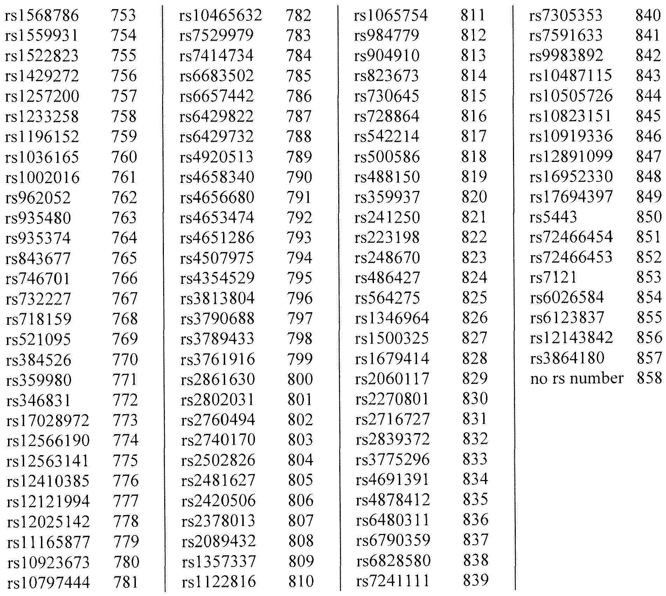

Figure 10 shows 849 SNPs identified by the MAPP and IDEA-VF studies that are associated with risk of SCA, and is a subset of the total number of 317,503 SNPs scanned from the whole genome using the Illumina 300K HapMap gene chips described herein. Figure 11 is a list of rs numbers and corresponding SEQ ID Nos. Both the rs numbers and the SEQ ID Nos. can be used interchangeably to identify a particular SNP.

A third study to identify genetic markers associated with SCA or SCD (referred to herein as "DISCOVERY") has been designed and implemented. The DISCOVERY study is undertaken to determine if certain cardiac ion-channel genetic polymorphisms predispose a patient to ventricular and atrial arrhythmia. In particular, the study aimed to identify which polymorphism combinations, optionally in further combination with other markers, serve as prognostics that identify appropriate candidates for ICD therapy. The DISCOVERY study's primary objectives are to correlate genetic polymorphisms with a diagnostic stratification of patients through a determination of risk of ventricular tachycardia and to evaluate the utility of ICD-based diagnostic information on the long-term treatment and management of primary prevention ICD patients. In particular, the predictive utility of SNPs in specific genes for ventricular arrhythmia of <400ms was evaluated. In particular, the genes studied were GNB3, GNAS and GNAQ genes, and the positive value was determined for SNPs as predictor for death, sudden cardiac death and atrial fibrillation or flutter in the genes GNB3, GNAS, GNAQ and other SNPs involving signal transduction components that have an impact on the activity of cardiac ion channels. Other genes under consideration include the CAPON and GPC5 genes. These data may be used to determine the optimal combination of all genetic parameters, including the presence or absence of any of the SNPs disclosed herein or otherwise known to be markers for cardiovascular diseases or disorders, patient baseline data, and patient follow-up data as a predictor for use in diagnostic and treatment methods and further methods of classification or stratification of patients based on the likelihood of SCA or SCD.

Polymorphism in GNB3

The GNB3 gene consists of 12 exons localized on chromosome 12p13. It codes for the $\beta_3$-subunit of the heterotrimeric G-proteins. The widely distributed C825T polymorphism exhibits exchange between Cytosine (C) and Thymine (T) in nucleotide position 825 of the cDNA as shown in Table 33. (Siffert, W. et al., Association of a Human G-Protein beta3 Subunit Variant with Hypertension, NAT. GENET., 1998; 18:45-48). This SNP is localized in exon 10 and associated with changes in cellular signal transduction. *Id.* This polymorphism is represented by rs5443 (SEQ ID No. 850) and is known by the following sequence: SEQ ID No. 850: 5'-gag agc atc atc tgc ggc atc acg tc [c/t] gtg gcc ttc tcc ctc agt ggc cgc c-3'.

As G-proteins participate in signal transduction in almost all body cells, it was shown that the C825T polymorphism is correlated with arterial hypertension, *(*Hengstenberg, C. et al., Association Between a Polymorphism in the G protein beta3 Subunit Gene (GNB3) with Arterial Hypertension but not with Myocardial Infarction, CARDIVASC. RES., 2001; 49:820-827) arteriosclerosis and obesity (Gutersohn, A. et al., G Protein beta3 Subunit 825 TT Genotype and Post-Pregnancy Weight Retention, LANCET, 2000; 355:1240-1241) along with changes in the response to hormones and drugs (Mitchell, A. et al., Increased Haemodynamic Response to Clonidine in Subjects Carrying the 825T-allele of the G Protein beta3 Subunit, Abstract, NAUNYN-SCHMIEDEBERG'S ARCH. PHARACOL., 2002; 265: Suppl. 1*;* Mitchell, A. et al., Insulin-mediated Venodilation Is Impaired in Young, Healthy Carriers of the 825T-allele of the G-protein beta3 Subunit Gene (GNB3), CLIN. PHARMACOL. THER., 2005; 77:495-502*;* Mitchell, A. et al., Effects of Systemic Endothelin A Receptor Antagonism in Various Vascular Beds in Men: In Vivo Interactions of the Major Blood Pressure-regulating Systems and Associations with the GNB3 C825T Polymorphism, CLIN. PHARMACOL. THER., 2004; 76:396-408*;* Sarrazin, C. et al., GNB3 C825T Polymorphism and Response to Interferon-alfa/ribavirin Treatment in Patients with Hepatitis C Virus Genotype 1 (CHV-1) Injection, J. HEPATOL., 2005; 43:388-393*;* Sperling, H. el al., Sildenafil Response is Influenced by the G Protein beta3 Subunit GNB3 C825T Polymorphism: A Pilot Study, J. UROL., 2003; 169:1048-1051). US 6,924,100 describes a method for evaluating responsiveness of an individual to treatment with an in vivo pharmaceutical wherein the in vivo pharmaceutical is one which activates G protein heterodimers containing a G protein subunit wherein the genetic modification is a substitution of cytosine by thymidine at position 825. Similarly, US 6,242,181 describes a method for diagnosing an increased likelihood of hypertension in a human subject comprising determining the presence of a genetic modification in a gene obtained from said subject which encodes a human G protein $\beta_3$ subunit wherein a genetic modification is a substitution of cytosine by thymine at position 825. Homozygotes of the 825T-allele exhibit changes in ion current in atrial cells (Dobrev, D. et al., G-Protein beta(3)-Subunit 825T Allele Is Associated with Enhanced Human Atrial Inward Rectifier Potassium Currents, CIRCULATION, 2000; 102:692-697) and results in reduced risk of atrial fibrillation. (Schreieck, J. et al., C825T Polymorphism of the G-protein beta3 Subunit Gene and Atrial Fibrillation: Association of the TT Genotype with a Reduced Risk for Atrial Fibrillation, AM. HEART. J., 2004; 148: 545-550). A pilot study has shown that ventricular arrhythmias are more prevalent in CC-homozygotes than in TC-heterozygotes and TT-homozygotes. (Wieneke, H. et al., Better Identification of Patients Who Benefit from Implantable Cardioverter Defibrillators by Genotyping the G Protein beta3 Subunit (GNB3) C825T Polymorphism, BASIC RES. CARDIOL., 2006).

Polymorphisms in GNAQ

The GNAQ gene codes for the Gaq subunit of hetero-trimeric G-proteins. The Gaq protein transmits signals over $\alpha$l-adrenoceptors (nor-adrenaline), endothelin receptors and similar receptors. Gaq directly regulates many ion channels. Hyper-expression of Gaq in the heart leads to cardiac hypertrophy (Adams, J. W. et al., Enhanced Galphaq signaling: A Common Pathway Mediates Cardiac Hypertrophy and Apoptotic Heart Failure, Proc. Natl. Acad. Sci. U.S.A., 1998; 95:10140-10145), whereas the knockout of Gaq (plus G$\alpha$11) counteracts pressure-induced hypertrophy. (Wettschureck, N. et al., Absence of Pressure Overload Induced Myocardial Hypertrophy After Conditional Inactivation of Galphaq/Galpha11 in Cardiomyocytes, NAT. MED., 2001; 7:1236-1240). Three polymorphisms have recently been described in the promoter of gene GNAQ that cause alterations in the expression of the Gaq protein: (GC (-909/-908) TT), G (-382)A and G (-387)A as shown in Table 33. GC (-909/-908TT) (SEQ ID No. 858) has the following sequence: 5'-gcg tcc gca gag ccc gcg ggg gcc g [g/t] [c/t] cca gcc cgg gag ccg cgc ggg cga g-3'. The polymorphism G (-382)A is known by rs72466454 (SEQ ID No. 851) and has the following sequence: 5'-cgc cgc cag gcg cac ggc gta ggg ga [a/g] cct cgc agg cgg cgg cgg cgg cgg c-3'. The polymorphism G (-387)A is known by rs72466453 (SEQ ID No. 852) and has the following sequence: 5'-gct ctc gcc gcc agg cgc acg gcg ta [a/g] ggg agc ctc gca ggc ggc ggc ggc g-3'.

Polymorphisms in GNAS

The GNAS gene codes for the Gas-subunit of hetero-trimeric G-proteins. Activation of Gas (formerly the stimulating G-protein), activates adenyl cyclase, leading to increases in cAMP. Gas is activated by many hormone receptors. The activation of $\beta$1 - adenoceptors is particularly important for the heart, as this leads to positive chronotropy and inotropy. Several somatic mutations in GNAS lead to rare endocrinological diseases *(*Weinstein, L.S. et al., Genetic Diseases associated with Heterotrimeric G Proteins, TRENDS PHARMACOL. SCI., 2006; 27: 260-266). There is also a silent C393T polymorphism thought to influence the response to beta-blocker medications (Jia, H, et al., Association of the G(s)alpha Gene with Essential Hypertension and Response to beta-blockade. HYPERTENSION, 1999l; 34:8-14). A series of polymorphisms in the promoter and intron-1 of gene GNAS has recently been described that modify the transcription rate and protein expression (C393T, G-1211A, C2291T) as shown in Table 33. C393T is known by rs7121 (SEQ ID No. 853) and has the following sequence: 5'-gag aac cag ttc aga gtg gac tac at [c/t] ctg agt gtg atg aac gtg cct gac t-3'. The polymorphism G-1211A is known by rs6123837 (SEQ ID No. 855) and has the following sequence: 5'-ctg gtc ttc tcg gtg cgc agc ccc tc [a/g] tgg gtg ctc aac ttc ctg ctg cag a-3'. The polymorphism C2291T is known by rs6026584 (SEQ ID No. 854) and has the following sequence: 5'-atc tgc agc tta agc cag tga cac aa [c/t] att ttg cat ttt taa atg gtg att c-3'.

TABLE 33

| Prevalence of the SNPs analyzed in the DISCOVERY study | |
|---|---|
| **SNP** | **Frequency of minor allele** |
| GNB3 c.825C >T | 30% T |
| GNAQ c.-909/-908GC>TT | 50% TT |
| GNAQ c.-382G>A | 5% A |
| GNAQ c.-387G>A | 8% A |
| GNAS c.393C>T | 50% T |
| GNAS c.2291C>T | 30% T |
| GNAS c.-1211 G>A | 25% T |

Polymorphism in GPC5

The minor allele of GPC5 (GLYPICAN 5, rs3864180) was associated with a lower risk of SCA in Oregon-SUDS, an effect that was also observed in ARIC/CHS whites (p<0.05) and blacks (p<0.04). Genome-Wide Association Study Identifies GPC5 as a Novel Genetic Locus Protective against Sudden Cardiac Arrest, Arking et al., PLosOne 2010 http://www.plosone.org/article/info:doi%2F10.1371%2Fjournal.pone.0009879. In a combined Cox proportional hazards model analysis that adjusted for race, the minor allele exhibited a hazard ratio of 0.85 (95% CI 0.74 to 0.98; p<0.01). Figure 13 shows Cox proportional hazards model was adjusted for age, sex, and race/ethnicity. Individuals homozygous for the protective allele (GG) are shown in green, heterozygotes (AG) in blue, and homozygous for the risk allele (AA) are in red. Further, a statistically significant interaction between rs3864180 and sex (P<0.012), with a stronger effect in women, has been reported in the association with SCA. However, the GPC5 association to SCD was shown in a non-ICD population. The polymorphism of GPC5 is known by rs3864180 (SEQ ID No. 856) and has the following sequence: 5'-tgt tca tct att caa aat gta gta ta [a/g] ttt tat ttg aga ttg tct ttt ttt a-3'.

Polymorphisms in the CAPON (NOS1AP) gene

The CAPON (NOS1AP) gene was shown to modulate the QT duration. "Common variants at ten loci modulate the QT interval (A. Pfeufer et al., Nature Genetics 2009). Figure 14 shows individuals classified by counting their number of QT-prolonging alleles in all ten identified markers (max score 20). Dosages for the QT-prolonging allele as calculated by MACH1 were added and then rounded to the nearest integer. Gray bars indicate the number of individuals in each score class, blue dots indicate the mean QT interval for each class, and the black line is the linear regression though these points. The polymorphism of GPC5 is known by rs12143842 (SEQ ID No. 857) and has the following sequence: 5'-tta gca ccc agg gtc aca tcc cag tt [c/t] aaa aat atc cca tgg agt gca gtc a-3'.

DISCOVERY study

The DISCOVERY study determined whether a correlation exists between genotypes and the incidence of atrial and ventricular arrhythmia, as measured by a dual chamber ICD produced by Medtronic, Inc. The differentiated diagnostic data (Saoudi, N. et al., How Smart Should Pacemakers Be? AM. J. CARDIOL., 1999; 83:180-186) afforded by the ICDs can produce information on arrhythmia trigger (Marshall, A.J., et al., Pacemaker Diagnostics to Determine Treatment and Outcome in Sick Sinus Syndrome with Paroxysmal Atrial Fibrillation, PACE, 2004; 27: 1130-1135*)* and IEGMs (Mitrani, R.D., et al., The Use Of Pacemaker Diagnostic Data To Guide Clinical Decision Making, Presented at Cardiostim, 2006), supraventricular tachycardia which are a known independent risk factor for mortality and stroke. (Benjamin, E.J. et a/., Impact Of Atrial Fibrillation On The Risk Of Death: The Framingham Heart Study, Circulation, 1998; 98:946-952*;* Glotzer, T.V. et al., Atrial High Rate Episodes Detected By Pacemaker Diagnostics Predicts Death And Stroke, Circulation 2003; 107(12):1614-1619). These data complement the follow-up data collected during unscheduled cardiology, specialized pacing and electrophysiology examinations.

The second part of the DISCOVERY study evaluated the therapeutic utility of ICD-based diagnostic information on patient treatment or management of symptoms related to cardiovascular disease. Recently developed ICD algorithms target improved patient-specific therapies. However, ICDs can also provide physicians with increasingly differentiated diagnostic information (Saoudi, N. *et al.,* 1999). First, the diagnostic information available in the ICDs is separated into system-related and patient-related diagnoses. (Nowak, B., Taking Advantage of Sophisticated Pacemaker Diagnostics, AM. J. CARDIOL., 1999; 83:172-179). This separation provided a systematic approach for the classification of the information generated.

System-related diagnostic data includes device query, battery and lead status, thresholds, sensing, and related long-term trends. This data enables early detection of hardware dysfunction. Patient-related diagnostics include intracardiac EGM, sensor data, and channel markers. This data supports the evaluation of device reactions to a patient's intrinsic rhythm and provides information on arrhythmia and heart disease progression. Patient-related diagnostic data may also be used to evaluate device programming and the impact of medication on the treatment or suppression of cardiovascular disease. The study evaluated the use of system-based and patient-based diagnostics and the resulting medical consequences, including medical interventions, prescription of medication and changes in medication, surgery, additional diagnostics, and changes in ICD programming. Similarly, the frequency of programming changes involving AF-prevention or AF-therapy algorithms and programming changes involving changes in pacing parameters were evaluated along with the resulting medical consequences.

The Medtronic, Inc. ICDs used in the study stored long-term trends for numerous diagnostic parameters over a period of up to 14 months. The device long-term diagnostics complement the information collected during patient follow-up examinations, which reflect only a brief exposure to a physician. For example, early identification of lead defects is improved by examining long-term impedance and sensing trends where major fluctuations are visible (Soudi, N. *et al.,* 1999). Arrhythmia therapy also significantly relies on stored ICD information and can be qualified by device-based system diagnostics (Mitriani, R.D. *et al.,* 2006). For example, the stored information related to atrial arrhythmia trends permits differentiated diagnosis of atrial arrhythmias, which comprise an independent risk factor for morality, stroke, and atrial fibrillation ("AF") in pacemaker patients with sinus node disease (Benjamin, E.J. *et al.,* 1998; Glotzer, T.V. *et al.,* 2003). Understanding the triggers of atrial arrhythmias can be of decisive importance in the treatment or reduction of atrial arrhythmias (Marshall, A.J. *et al.,* 2004). Additionally, assessment of atrial coherence is important for the diagnostic interpretation of atrial arrhythmias provided by ICDs. Atrial leads with long-term trends in sensing values and EGM episodes support the evaluation of sensing integrity and of atrial arrhythmia episodes by highlighting sensing malfunction on atrial channels and leads which would otherwise result in a faulty assessment of arrhythmias.

The DISCOVERY study was an interventional non-randomized, longitudinal, prospective, multi-centric, diagnostic study. It was composed of two parts: Part One was a double-blind study, and analyzed data on genetic polymorphisms as prognostic of ventricular and atrial tachyarrhythmia. Part Two of the study evaluated the influence of ICD-based diagnostic information on long-term patient management and treatment. Subjects were enrolled for a period of approximately 24 months, and the total study duration was 48 months. The DISCOVERY study intended to determine the diagnostic value obtained from SNPs studied within the framework of Cardiac Compass and other diagnostic tools available in the commercially released Medtronic, Inc. produced ICD devices. The devices were manufactured in accordance with the provisions of the Active Medical Device Directive (90/385/EEC) and comply with all relevant legal requirements. The devices and leads were market released and used within labeling.

Subjects who were included in the study first had implantation of a market approved Medtronic, Inc. Dual-chamber ICD with long-term clinical trends as Cardiac Compass including, but not limited to, Marquis DR (7274), Maximo DR (7278), Intrinsic DR (7288), EnTrust DR (D153ATG), and Virtuoso DR (D164AWG). The components used were programmer 2090 and 2090W (Medtronic, Inc.), all market released leads, and all Medtronic, Inc. market released software. However, other leads and software known to those of skill in the art are contemplated. The 2090 and 2090W programmer and Medtronic, Inc. software was used to interrogate and program the parameters of the devices. The software was market released in Europe. Additional ICDs, leads, programmers, software and accessories were optionally incorporated into the study as they became commercially available. As part of the CE conformity assessment, a notified body evaluated the biocompatibility, clinical performance, and safety of all the devices and leads used in the DISCOVERY study.

The subjects had ICD indication for primary prevention of ventricular arrhythmia according to the current AHA/ACC/ESC guidelines (A report of the ACC/AHA Task Force and the ESC Committee for Practice Guidelines: ACC/AHA/ESC 2006 Guidelines For Management Of Patients With Ventricular Arrhythmia and the Prevention of Sudden Cardiac Death - Executive Summary, European Heart J, 2006; 27:2099-2140). Subjects were also willing and able to comply with the Clinical Investigation Plan, remained available for follow-up examinations, and signed an informed consent form within 10 days of receiving the implant.

Excluded subjects included pregnant women; women of childbearing potential who did not use a reliable form of birth control; subjects enrolled in a concurrent study that may confound the results of this study; minors; subjects with a life expectancy of less than two years; subjects who have had or were awaiting heart transplantation; subjects having syndromes known to be associated with Ion channelopathies such as Long- and Short-QT Syndrome, Brugada Syndrome, Catecholaminergic Polymorphic Ventricular Tachycardia (CPTV); and subjects otherwise deemed appropriate for exclusion based on an expectation of poor compliance.

The ICD devices used in the study were multi-programmable, Dual-chamber ICDs as previously described. All devices automatically detect and treat episodes of VT, VF, fast ventricular tachycardia and bradyarrhythmia. When a cardiac arrhythmia is detected, the implantable device delivers defibrillation, cardioversion, anti-tachycardia pacing or standard pacing therapy. The devices collect and store various types of data and provide a range of diagnostic tools to manage patient care.

A summary of the data and diagnostic tools, which are available during follow-up examination, is provided herein. The devices provided a Quick Look screen which supplies a summary of the episode data, device and lead status information, programmed bradycardia pacing parameters, conduction status, and device observations since the last patient session. The Quick Look screen is displayed after the software application is started. The Observations section of the Quick Look screen highlights significant device status events, lead status events, Patient Alert events, parameter programming, diagnostic data, and clinical status data.

The Cardiac Compass report provides up to 14 months of clinically significant data including arrhythmia episodes, therapies delivered, physical activity, heart rate, and bradycardia pacing activity. The report can be useful in correlating changes in data trends to changes in programmed parameters, drug regimen, or patient condition. The Cardiac Compass report provides an overall view based on the following daily checks or measurements: VT/VF episodes; indication of a cardioversion or defibrillation therapy delivered; ventricular rate during VF, FVT, or VT episodes; the number of VT-NS episodes per day; the total time in AT or AF (EnTrust and Virtuoso devices only); ventricular rate during AT or AF (EnTrust and Virtuoso devices only); percentage of atrial and ventricular pacing; average day and night ventricular rate; overall patient activity; heart rate variability; and OptiVol fluid index (Virtuoso device only).

The Cardiac Compass report also provides the following trend data. The "VT/VF episodes per day trend" provides a history of ventricular tachyarrhythmia and may reveal correlations between clusters of episodes and other clinical trends. Each day, the ICD records the total number of spontaneous VT and VF episodes. The episode counts are provided in histogram format on the report.

The device also records a shock indicator for any day on which it delivers an automatic defibrillation therapy, cardioversion therapy, or atrial shock therapy. The Cardiac Compass report displays an annotation for the day on which a defibrillation therapy, cardioversion therapy, or atrial shock therapy was delivered.

The Cardiac Compass displays a graph of the daily median ventricular rate for spontaneous VF, FVT and VT episodes, which may have occurred. This may provide an indication of the effects of anti- arrhythmic drugs on VF, FVT, and VT rates and a better understanding of the safety margins for detection.

The "non-sustained VT episodes" trend may reveal correlations between patient symptoms (such as palpitations) and VT-NS episodes and may indicate a need for further investigation of the status of the patient. Each day, the ICD records the total number of spontaneous VT-NS episodes. The episode counts are provided in histogram format on the report.

The "AT/AF total hour per day" trend for EnTrust and Virtuoso devices helps in the assessment of the need for anti-arrhythmic drugs or ablations to reduce AT/AF episode occurrences or for anti-coagulant drugs to reduce the risk of stroke. The device records a daily total for the time the patient spent in AT or AF.

The "ventricular rate during AT/AF" trend reveals correlations between patient symptoms and rapid ventricular responses to AT/AF. It is also useful in the assessment of the efficacy of an AV node ablation procedure or in the assessment VT/VF detection safety margins so that programming may be modified to avoid treating rapidly conducted AT/AF as VT/VF. The trend may be used further to prescribe or titrate anti-arrhythmic and rate control drugs. The device records average and maximum ventricular rates during episodes of AT and AF each day. The values are plotted on the Cardiac Compass report along with the average ventricular rates.

The percent pacing per day graph provides a view of pacing over time that can help identify pacing changes and trends. It displays the percentage of events occurring during each day that are atrial paces (AT and DR devices only) and ventricular paces. It can be useful to program the pacing parameters in a way that helps to avoid unnecessary ventricular stimulation in those patients that have no indication for ventricular pacing.

The "patient activity" trend can be evaluated and used for the following types of information. The trend can act as an early indicator of symptoms due to progressive diseases like heart failure, which causes fatigue and a consequent reduction in patient activity. Similarly, the trend allows monitoring of a patient's exercise regimen. The trend is an objective measurement of a patient's response to changes in therapy. The trend may also be used to study outcomes in ICD patients, along with additional parameters such as quality of life. The device uses activity count data derived from the rate response accelerometer signal to determine patient activity. The activity values are stored daily. For each seven days of stored data, the device calculates a seven-day average. This average is plotted for the Cardiac Compass report.

The night and day heart rate trend provides the following clinically useful information: gradual increase in heart rate, which may indicate cardiac decompensation as a symptom of heart failure; objective data that may be correlated with patient symptoms; indications of autonomic dysfunction or heart failure; and information regarding diurnal variations.

In AT and DR devices, the device measures the median atrial interval value every five minutes and calculates a variability value each day. The heart rate variability value, in milliseconds, is plotted on the Cardiac Compass report.

The "OptiVol" fluid index trend for Virtuoso devices displays the accumulation of the time and magnitude that the daily impedance is less than the reference impedance. If the daily impedance is less than the reference impedance, then the OptiVol fluid index trend increases. This may indicate that the patient's thoracic fluid has increased. The OptiVol fluid monitoring feature is an additional source of information for patient management.

The "rate histograms report" counts and collects atrial or ventricular events and classifies them by rate range and the percentage of time. The device automatically collects the histogram data without any programming by the clinician. This diagnostic is intended for ambulatory monitoring uses, such as monitoring rate distribution. Rate histograms also collect the ventricular rate during AT/AF. This diagnostic may be used to evaluate drug titration.

Flashback Memory allows analysis of heart rates leading to a VF, VT, or AT/AF episode and compares the pre-VF, pre-VT, and pre-AT/AF rhythms to the normal sinus rhythm and to other episodes. In AT and DR devices, Flashback Memory automatically records V-V and A-A intervals and stored marker data for the following events: the most recent VF episode, the most recent VT episode, the most recent AT/AF episode, and the most recent interrogation.

The ICD automatically and continuously monitors battery and lead status. The Battery and Lead Measurements screens after interrogation views and prints the following data: current battery voltage, last capacitor formation, last capacitor charge, sensing integrity counter data, last atrial lead position check (EnTrust and Virtuoso devices only), last lead impedance data, last sensing data, and last high-voltage therapy.

The automatically performed daily lead impedance and sensing measurements are used to generate lead performance graphs based on up to 82 weeks of measurements. A separate graph is provided for each of the following measurements: atrial placing lead impedance (AT and DR devices only), ventricular pacing lead impedance, defibrillation lead impedance, SVC lead impedance (if used), P-wave sensing amplitude (AT and DR devices only), and R-wave sensing amplitude.

Methods of stratifying patients into diagnostic groups based on a determination of risk of ventricular tachycardia are provided. Methods of evaluating ICD-based diagnostic information for the long-term treatment and management of primary prevention ICD patients are also provided. Subjects suitable for evaluation are first identified. A review of each subject is required to determine preliminary eligibility according to subject inclusion and exclusion criteria. Clinical data is collected via study electronic or paper case report forms ("eCRF" or "CRF") at the time of subject's baseline, planned follow-up examinations, unscheduled follow-up examinations, system modification and subject exit, including deaths, as applicable.

The Baseline CRF is used to record the baseline data for all subjects. The information documented may include, for example, (1) verification of inclusion and exclusion criteria; (2) recording of the subject's demographic and medical history; (3) cardiovascular status and history, including arrhythmia history; (4) ICD implant indication; (5) physical assessment such as LV ejection fraction, 12-lead ECG; (6) New York Heart Association (NYHA) classification; (7) Recording of cardiovascular medications; and (8) date of blood test for the genetics analysis. A subject's cardiovascular history includes heart failure ("HF") etiology, previous surgery and history of arrhythmia.

Cardiac medications are recorded at the baseline evaluation, but recordation of non-cardiac medications is not required. During every follow-up visit, however, only medication changes as a result of the use of patient-related diagnostics will be recorded, as described below. Cardiac medications include, but are not limited to, angiotensin-converting enzyme inhibitors and angiotensin receptor blockers, anti-arrhythmic medications, beta-blockers, diuretics, calcium channel blockers, anticoagulants, inotropes, nitrates, cardiac glycosides, and anti-lipidemics, e.g., statins.

New York Heart Association classification of functional capacity is based on a classification system originating in 1928, when the NYHA published a classification of subjects with cardiac disease based on clinical severity and prognosis. This classification has been updated in seven subsequent editions of Nomenclature and Criteria for Diagnosis of Diseases of the Heart and Great Vessels (Little, Brown & Co.). The ninth edition, revised by the Criteria Committee of the American Heart Association, New York City Affiliate, was released March 4, 1994. These classifications are summarized below in Table 34.

TABLE 34

| Functional Capacity | |
|---|---|
| **Class I** | Subjects with cardiac disease but without resulting limitation of physical activity.<br>Ordinary physical activity does not cause undue fatigue, palpitation, dyspnea, or angina. |
| **Class II** | Subjects with cardiac disease resulting in slight limitation of physical activity.<br>They are comfortable at rest. Ordinary physical activity results in fatigue, palpitation, dyspnea, or angina. |

(continued)

| Functional Capacity | |
|---|---|
| **Class III** | Subjects with cardiac disease resulting in marked limitation of physical activity.<br>They are comfortable at rest. Less than ordinary activity causes fatigue, palpitation, dyspnea, or angina. |
| **Class IV** | Subjects with cardiac disease resulting in inability to carry on any physical activity without discomfort.<br>Symptoms of HF or the anginal syndrome may be present even at rest. If any physical activity is undertaken, discomfort is increased. |

A left ventricular ejection fraction ("LVEF") measurement is also performed at baseline if one has not been performed within 30 days prior to subject enrollment. The following information is collected: LVEF measurement, the method of LVEF measurement, radionucleotide entriculo-cardiography/MUGA, echocardiography, and ventricularcardiography via catheterization.

A 12-lead ECG is also performed during the baseline evaluation if one has not been performed within 30 days prior to subject enrollment. The QRS width and the lead used for measurement will be circled and maintained in the patient's file.

After a subject receives an IMD, the device is programmed. The programming is done according to the applicable Medtronic ICD System Reference Manual for the programs for detection and therapy parameters for bradycardia pacing and anti-tachycardia therapy. Table 35 outlines the required programming parameters and concern only the diagnostic quality of the data collected. Deviation from these settings must be recorded with the clinical evidence justifying deviation from the programming requirements.

TABLE 35

| Parameter | Feature | Value |
|---|---|---|
| VF | Detection | ON |
| | initial beats to detect (NID) | 18/24 |
| VT | Detection | ON or Monitor |
| | Initial beats to detect (NID) | 16 |
| | V Interval | > 400 ms |
| | EGM 1 Source | Atip - Aring |
| | EGM 2 Source | Vtip - Vring |

Recommended programs are shown in Table 36 and may be subject to change by a subject's physician.

TABLE 36

| Parameter | Feature | Value |
|---|---|---|
| VF | V interval | 300 ms |
| | Redetect beats to detect | 9/12 |
| | Therapies | 6 x max. energy [J] |
| FVT | Detection | via VF |
| | V Interval | 240 ms |
| | Therapies | Burst (1 sequence)*, 5 x max. energy [J] |
| VT | Redetect beats to detect | 8 |
| | Therapies | Burst (2)*. Ramp (1)**,20 J, 3 x max. energy [J] |
| SVT criteria | PR Logic: AFib/ AFlutter | On |
| | PR Logic: Sinus Tach | On |
| | 1:1 VT-ST boundarv | 66 % (except EnTrust. Virtuoso) |

(continued)

| Parameter | Feature | Value |
|---|---|---|
| | SVT Limit | 260 ms |
| Pacing | MVP | On (only InTrinsic. EnTrust, Virtuoso) |
| | PAV ( where applicable) | > 230 ms |
| | SAV ( where applicable) | > 200 ms |
| * Burst ATP: 8 intervals, R-S1 = 88%, 20 ms decrement<br>** Ramp ATP: 8 intervals, R-S1 = 81%, 10 ms decrement | | |

At the conclusion of the implantation, as well as at the beginning and conclusion of every follow-up examination, the device is interrogated, and the data is collected via the device's Save-to-Disk function. A full interrogation is performed without clearing any episodes. If any Save-to-Disk files related to a follow-up visit are permanently missing, a Study Deviation form is completed. Subject data may be excluded from analysis if a sequential device-based arrhythmic history cannot be provided at a later time.

Clinical data is also collected at the time of the subject's planned follow-up, unscheduled follow-up, system modification and subject exit. Regular follow-up examinations take place at 6, 12, 18, and 24 months after device implantation. To obtain sufficient incidence of ventricular arrhythmia, a follow-up duration of 24 months per subject is required. If a follow-up visit falls outside the acceptable target day +/- 30 days, the original follow-up schedule will be maintained for the remaining visits. Table 37 shows the method for determining appropriate follow-up visit scheduling. Medical treatment and device programming not included in Table 9 are left to the discretion of the examining physician during follow-up examinations.

TABLE 37

| | Days post Device-Implantation | | |
|---|---|---|---|
| Visits | Window start | Target day | Window end |
| 6 month follow-up | 153 | 183 | 213 |
| 12 month follow-up | 335 | 365 | 395 |
| 18 month follow-up | 518 | 548 | 578 |
| 24 month follow-up | 700 | 730 | 760 |

At every scheduled and unscheduled follow-up visit, the following information is recorded: (1) cardiac symptoms, (2) occurrence and classification of arrhythmia, (3) use of system-related diagnostics such as battery status, impedance, pacing threshold, and sensing, (4) use of patient-related diagnostics such as arrhythmia information, heart frequency and stimulation that may lead to a change in treatment, (5) programming changes of the device using the device diagnostic, (6) follow-up duration, (7) number of medication changes as a result of the use of patient-related diagnostics, and (8) NYHA classification. The procedures for collecting the subject demographic and medical history and NYHA classification and cardiac medication information are as previously described. A Save-to-Disk function is performed using the ICD.

If applicable, the following reports are also completed: a Study Deviation report and/or an Adverse Event report. An Adverse Event ("AE") is any untoward medical occurrence in a subject. An Adverse Device Effect (ADE) is any untoward and unintended response to a medical device, including any event resulting from insufficiencies or inadequacies in the instructions for use or the deployment of the device, which also includes an event that is a result of user error. A Serious Adverse Event (SAE) is an AE that (1) leads to death, (2) leads to fetal distress, fetal death, or a congenital abnormality or birth defect, or (3) leads to a serious deterioration in the health of a subject that (i) resulted in a life-threatening illness or injury, (ii) resulted in a permanent impairment of a body structure or a body function, (iii) required in-patient hospitalization or prolongation of existing hospitalization, or (iv) resulted in medical or surgical intervention to prevent permanent impairment to a body structure or body function.

The Adverse Event report is only for serious adverse device effects ("SADE") and serious procedure-related adverse events. An SADE is an event that has resulted in any of the consequences characteristic of a Serious Adverse Event or that might have led to any of those consequences if (i) suitable action had not been taken, (ii) intervention had not been made, or (iii) if circumstances had been less opportune. Serious procedure related adverse events are those that occur due to any procedure specific to the treatment and examination of the subject, including the implantation or modification of the system. Ventricular or supraventricular arrhythmias that are detected are not treated as adverse events, whether or not treated, because they constitute material events analysis.

Information reported on the Adverse Event form includes a description of the event, the diagnosis, the date of event onset, the relationship of the event to the procedure, the relationship of the event to the device or system, actions taken as a result of the event, and the outcome of the event. Adverse events are to be reported as soon as possible after the event occurs.

In the event that the device or leads require invasive modification (e.g., ICD or lead explants, ICD or lead replacement, or lead repositioning), a system modification CRF is completed. An Adverse Event CRF is likewise completed to document the underlying cause of the system modification. When possible, explanted ICD devices and/or leads are returned to Medtronic, Inc. for analysis.

The devices and other components contemplated by the invention are those described as being used in and intended for the DISCOVERY study. If the device or lead is not replaced with those not meeting these criteria, then the following steps are performed. First, prior to explant, the device is interrogated and the data is saved onto one or more diskettes as needed using the Save-to-Disk function. The subject is then followed over the next 30 days or until all Adverse Events associated with the initial system are either resolved or unresolved with no further action required, whichever occurs last. Finally, a study termination CRF is completed for the subject.

If the ICD or leads are replaced with those meeting the above criteria, then the following steps are performed. First, prior to explant, the device is interrogated and the data is saved onto one or more diskettes as needed using the Save-to-Disk function. The subject is then followed according to his or her regular examination schedule.

Each patient death is classified as follows: (1) cardiac, non-cardiac, or unknown; and (2) sudden, non-sudden, or unknown. All deaths are reported. In addition, deaths will be classified based on whether they are related to the device or lead system and whether they were arrhythmic, non-arrhythmic (vascular) or unknown. The following information will also be collected when a death occurs, if available: a medical report, EGM or IEGM related to the death, an autopsy report, and a full device interrogation using Save-to-Disk.

A cardiac death is defined as a death related to the electrical or mechanical dysfunction of the heart. The initiating event, which may be preventable, is differentiated from the terminal event. For this purpose, the initiating event of cardiac death thus requires further classification as either arrhythmic or vascular: (1) initiating event is arrhythmic, non-arrhythmic, or unknown; (2) initiating event is vascular, non-vascular or unknown. Non-cardiac deaths are all deaths with a known cause not classified as cardiac deaths. If insufficient information is available to classify a death as cardiac or non-cardiac, the death is classified as unknown.

Sudden death is a witnessed death within one hour after onset of acute symptoms, or un-witnessed death, that is unexpected and without other apparent cause, including death during sleep. Non-sudden death is a death that is not classified as sudden death, including cardiac death of hospitalized subjects on inotropic support.

Blood samples are collected from each subject and analyzed for seven single nucleotide polymorphisms in the genes GNB3, GNAQ, GNAS. Then, the subject data is analyzed to determine the existence of a correlation between these SNPs and the occurrence of arrhythmia. During the course of the evaluation, additional genetic factors related to other medical conditions, which may or may not be cardiac related, may also be revealed. The genetic profiles of the subjects may be used in additional research and analysis. This additional research involves medical research related to genetic effects on diseases and diagnostic and therapeutic applications of that research.

Statistical analysis is then performed on the data collected. For all analyses, a two-sided p-value or 0.05 is considered to be statistically significant. Groups of patients for which statistical analysis is performed must contain no fewer than four patients. No populations are defined for the statistical analyses. All data is analyzed as collected. There is no imputation of missing data. Continuous variables are reported using N, N missing, mean, standard deviation, minimum, median and maximum. Categorical variables are reported using N per category and percentages. In addition, for categorical variables where more than one category can be crossed, a report of how often a combination of categories has been checked is made. The analyses are performed after two years of follow-up have been completed for each subject. There is no correction for multiple testing.

After the baseline and follow-up patient data is collected, a determination is made of the value of a SNP in the genes GNB3, GNAS and GNAQ as a predictor for ventricular arrhythmia < 400 ms. For the analysis of the predictive power of the various SNPs, sensitivity, specificity, and positive and negative predictive value are calculated as a predictor of the primary endpoint in patients without a history of spontaneous VT/VF (primary prevention indication for ICD implantation). Confidence intervals are calculated (95%, 2-sided). A primary endpoint is a ventricular arrhythmia which can be detected within a Tachycardia Detection Interval (TDI) programmed as follows: 18 out of 24 for ventricular fibrillation or 16 consecutive beats for ventricular tachycardia with a maximum cycle length of 400 ms. A cutoff value of 400 ms has been selected in order to capture a high amount of ventricular tachy-arrhythmias and at the same time avoid inappropriate ICD therapies. Sweeney *et al.* have shown in the PainFREE RX II trial (Appropriate and Inappropriate Ventricular Therapies, Quality of Life, and Mortality Among Primary and Secondary Prevention Implantable Cardioverter Defibrillator Patients: Results From the Pacing Fast VT Reduces Shock Therapies [PinFREE Rx II] Trial, CIRCULATION, 2005; 111:2898-2905*)* that the mean cycle length of ventricular tachycardia in primary prevention patients is 351 msec. In the PainFree Rx II and in the EMPIRIC trial, (Wilkoff, B. L. et al., A Comparison of Empiric to Physician-Tailored Programming of Implantable Cardioverter-Defibrillators, J. AM. COLL. CARDIOL., 2006; 48:3330-339) a cutoff value of 400 ms was chosen, allowing heart rates as low as 150 beats per minute. A cutoff value of 400 ms lead to an amount of only 11.9 % inappropriately treated SVTs in the EMPIRIC trial.

The sample size calculation is based upon a required accuracy of the estimate of the positive predictive value (PPV) of the potential risk stratifiers under study. A 95% confidence interval with a maximal width of ±5 % is deemed appropriate. This level of accuracy of the estimated PPV requires a sample of 386 patients with a positive risk stratifier, assuming actual PPV is 40%. The bisection method is used along with the proportion confidence interval formulas found in Johnson and Kotz (Discrete Distributions, Houghton Mifflin Company, Boston, 1969, 58-60*).* This focus is on those markers that have incidence greater than one third of all patients. If 386 patients are required to reach the primary endpoint, 3 x 386 = 1158 patients are needed with a primary indication for ICD implantation. With an approximated 10% of the patients lost to follow-up, a total of 1287 patients are required. Alternatively, it may be acceptable to use a 95% confidence interval with a maximal width of ± 7,5%, requiring 583 patients in total.

The positive value of SNPs as predictor for death, cardiac death and atrial fibrillation or flutter in genes GNB3, GNAS and GNAQ is determined. A determination of the positive predictive value is also made for other SNPs having signal transduction components that impact on the activity of cardiac ion channels. To evaluate the predictive power of the various SNPs, sensitivity, specificity, positive and negative predictive value will be calculated as predictor of the endpoints: death, cardiac death, and atrial fibrillation or flutter.

Finally, a determination as to the most useful combination of genetic parameters, baseline data and follow-up data is made regarding the predictor of primary endpoint, all-cause mortality, cardiac death, and atrial arrhythmia. This determination involves analysis of the following parameters: (1) available genetic tests, (2) QRS width, (3) baseline medication, (4) age, (5) heart rate variability documented by the device diagnostics such as Cardiac Compass, (6) history of AT/AF, and (7) documented AT/AF by the device or Holter ECG. NYHA and EF data are related to ICD implantation indication and are assessed as eventual baseline correction. Usefulness is evaluated in terms of the greatest positive predictive value for the prognosis of sustained ventricular arrhythmia, non-cardiac death, cardiac death, and atrial arrhythmia.

For each combination of test and endpoint parameters, a univariate Cox proportional hazards model is used to assess the predictive value of the test. For each endpoint, the tests with a univariate correlation of $p<0.05$ are included in the multivariate Cox proportional hazard regression analysis. The best combination of these tests is selected by an automatic algorithm applied to a Cox proportional hazard model.

During performance of the methods of the invention, diagnostic data collected by the implanted devices is observed, compared, and analyzed. The results of such analyses may lead to conclusions and insights that, in turn, could result in device programming that might be more favorable for an individual patient or patient subgroups compared to the settings originally chosen. If new aspects regarding optimization of treatment such as the programming of the devices arise, appropriate changes in treatment may be made that will provide a benefit to the patient(s). The correlations made between the results of the genetic analyses and the amount of ventricular and atrial arrhythmia which are statistically significant benefit patients by providing for more appropriate patient selection for ICD therapy.

A method of determining the medical consequences of using ICD-based diagnostics is also provided. In the method, patient treatment using ICD-based diagnostics is evaluated along with the medical consequences of such treatment. Alternatively, the medical consequences of ICD-based rhythmic diagnostic data are evaluated. This analysis is performed using the subject follow-up CRFs. The determination is made by setting a value on diagnostic and treatment utility of the diagnostics based on the medical consequences.

A method of evaluating the frequency of programming changes involving AF-prevention and AF-therapy algorithms triggered by device diagnostics is also provided. Device interrogations documented at the beginning and end of each subject follow-up examination are used to identify changes in device programming regarding AF-prevention and AF-therapy. A determination is made as to the frequency of changes in device programming. Additionally, the frequency of pacing parameter programming changes and the resulting medical consequences is also evaluated. A determination is made of the medical consequences of such programming changes.

The medical consequences of the use of ICD systems and ICD-based diagnostics may include certain potential risks. These risks may include, but are not limited to, the following types of events and medical consequences. Adverse events associated with ICD systems include, but are not limited to: acceleration of arrhythmia (caused by the ICD), inappropriate detection of tachy-arrhythmia, inappropriate therapy for tachy-arrhythmia including shocks, potential sudden death due to failure to detect and/or inability to defibrillate or pace, air embolism, bleeding, chronic nerve damage, erosion, excessive fibrotic tissue growth, extrusion, fluid accumulation, hematoma or cysts, infection, body rejection, keloid formation, lead abrasion and discontinuity, lead migration/dislodgement, movement of the device from its original location, myocardial damage, pain, pneumothorax, seroma, thromboemboli, venous occlusion, venous or cardiac perforation, shunting current or insulating myocardium during defibrillation.

Patient conditions may also change. Even when there has been a satisfactory response to tachyarrhythmia therapies during clinically conducted electrophysiology studies, underlying or accompanying diseases or a change in anti-arrhythmic drug therapy may, over time, alter electrophysiologic characteristics of the heart. As a result of the changes, the programmed therapies may become ineffective and possibly dangerous, e.g., initiate an atrial tachyarrhythmia or accelerate a ventricular tachycardia to flutter or fibrillation. Changing patient conditions may also require modification of the ICD system due to factors such as increased defibrillation requirements, unacceptable sensing, elevated pacing thresholds, loss of pacing capture and diaphragmatic stimulation. Patients receiving frequent shocks despite anti-arrhythmic medical management could develop psychological intolerance to an ICD system that might include the following: dependency, depression, fear of premature battery depletion, fear of shocking while conscious, fear that shocking capability may be lost, imagined shocking, *i.e.*, phantom shock.

Similarly, potential adverse events related to the use of leads include, but are not limited to, the following patient related conditions: cardiac perforation, cardiac tamponade, constrictive pericarditis, embolism, endocarditis, fibrillation or other arrhythmia, heart wall rupture, hemothorax, infection, pneumothorax, thrombosis and tissue necrosis. Other potential adverse events related to the lead include, but are not limited to, the following: insulation failure, lead conductor or electrode fracture, lead dislodgement, and poor connection to the ICD. These may lead to oversensing, undersensing, or loss of therapy.

In performing the methods of the invention, risks have been minimized by the careful assessment of each subject prior to, during, and after implantation of the ICD. The devices contemplated by the invention have independently selectable parameters available to maximize the detection and/or rejection of tachyarrhythmia. The efficacy of programmed detection and therapies for the treatment of episodes of ventricular tachycardia is routinely evaluated prior to permanent implantation and programming of any device. The risk of failure to terminate an arrhythmia by the ICD is minimized by demonstrating an adequate defibrillation safety margin at the time of implant and the ability to select and deliver up to six therapies for detected episodes of ventricular tachycardia or fibrillation.

Careful follow-up of patients receiving ICD systems also helps to minimize risks associated with the device (such as battery depletion) or associated with the patient (such as altered drug regimen). Patients are followed at regular intervals to confirm that the programmed parameters are appropriate and to monitor the implanted system. At each follow-up examination, the ICD is interrogated, and verification of an adequate pacing threshold margin is made as well as an evaluation of pacing and sensing characteristics.

Telemetry reports, such as device status data, episode counter data, therapy counter data, episode data reports, lead trend data, daily automatic lead measurements and Patient Alert™ reports provide information about the operation and status of the ICD system. Various programmable EGM recording sources can be useful for troubleshooting possible lead and connector problems.

Specific SNPs, either alone or in combination, can be used to predict SCA, or SCD, risk and to select to which drugs or device therapies a patients may be more or less likely to respond. Identification of therapies to which a subject is unlikely to respond allows for quicker access to a more appropriate drug or device therapy. The genetic information can be taken from a biological specimen containing the patient DNA to be used for SNP detection, or from a previously obtained genetic sequence specific to the given patient. Once it is determined that the given patient has a high risk for SCA, then evaluation of possible therapies can be performed. Specific anti-arrhythmic drugs and device therapies including ICD, cardiac resynchronization therapy, anti-tachycardiac pacing therapy and Subcutaneous ICD, or similar therapies can be assessed for their likely effect on the individual patient.

## EXAMPLES

### Bead-based genotyping and haplotyping

A template can be generated by obtaining genomic DNA probes representing the SNPs of SEQ ID Nos. 1-849. Nested PCR can be used to generate a template for typing where amplifications could be performed using PCR Mastermix (Abgene, Inc., Rochester, NY). Primary PCRs can be carried out with 20 ng genomic DNA in 10 $\mu$l 1 $\times$ PCR Mastermix, 0.2 $\mu$M of primers, and 2 mM $MgCl_2$ with the following cycling conditions: 95˚C for 5 min; 40 cycles at 95˚C for 30 s, 58˚C for 30 s, 72˚C for 2 min 30 s; 72˚C for 10 min. The product can then be diluted 1:500 in 1 $\times$ TE and re-amplified using asymmetric PCR. The amplified products can then be analyzed by gel electrophoresis and then used directly in a bead-based genotyping and haplotyping reaction.

### Allele-specific hybridization

For genotyping and haplotyping, allele-specific oligonucleotides (ASOs), representing the SNPs of SEQ ID Nos. 1-849 can be synthesized. The ASO can be 25 nucleotides long with a 5' Uni-Link amino modifier where each ASO can be attached to a different colored bead. Genotyping can be performed in a 30 μl hybridization reaction containing 5 μl unpurified PCR product, 83 nM biotinylated sequence-specific oligonucleotide and beads corresponding to each allele of the SNPs of SEQ ID NO.'s 1-849 reacted in 1 × TMAC buffer (4.5 M TMAC, 0.15% Sarkosyl, 75 mM Tris-HCl, pH 8.0 and 6 mM EDTA, pH 8.0). The reactions can then be denatured at 95°C for 2 min and incubated at 54°C for 30 min. An equal volume of 20 μg/ml streptavidin-R-phycoerythrin (RPE) (Molecular Probes, Inc., Eugene, OR) in 1 × TMAC buffer can be added and the reaction be incubated at 54°C for 20 min prior to analysis on a Luminex 100. The data collection software can be set to analyze 100 beads from each set and the median relative fluorescent intensity can be used for analysis. Visual genotypes and haplotypes can be generated using the online software applications found at http://pga.gs.washington.edu/software.html.

It should be understood that the above-described embodiments and examples are merely illustrative of some of the many specific embodiments that represent the principles of the present invention. Numerous other versions can be readily devised by those skilled in the art without departing from the scope of the present invention.

**Claims**

1. A diagnostic kit, comprising a plurality of probes that determines the presence or absence of one or more Single Nucleotide Polymorphisms (SNPs) associated with Sudden Cardiac Arrest (SCA) in a genetic sample, said one or more SNPs being selected from position 51 in any one of SEQ ID Nos. 1-849 or position 26 or 27 in any one of SEQ ID Nos. 850-858, wherein the diagnostic kit comprises less than 1000 probes.

2. A diagnostic kit, comprising a plurality of probes that determines the presence or absence of one or more Single Nucleotide Polymorphisms (SNPs) associated with Sudden Cardiac Arrest (SCA) in a genetic sample, said one or more SNPs being selected from position 26 or 27 in any one of SEQ ID Nos. 850-858, wherein the diagnostic kit comprises less than 1000 probes.

3. A diagnostic kit, comprising a plurality of probes that determines the presence or absence of one or more Single Nucleotide Polymorphisms (SNPs) associated with Sudden Cardiac Arrest (SCA) in a genetic sample, said one or more SNPs being selected from position 51 in any one of SEQ ID Nos. 844, 831, 825, 839 and 833, wherein the diagnostic kit comprises less than 1000 probes.

4. A diagnostic kit, comprising a plurality of probes that determines the presence or absence of one or more Single Nucleotide Polymorphisms (SNPs) associated with Sudden Cardiac Arrest (SCA) in a genetic sample, said one or more SNPs being selected from position 51 in any one of SEQ ID Nos. 835, 832, 844, 846, 838, 848, 829, 842, 827, 828, 824, 836, 840, 845, 826, 837, 841, 843, 117, 535, 823, 834, 830, 847, and 849, wherein the diagnostic kit comprises less than 1000 probes.

5. A diagnostic kit, comprising a plurality of probes that determines the presence or absence of one or more Single Nucleotide Polymorphisms (SNPs) associated with Sudden Cardiac Arrest (SCA) in a genetic sample, said one or more SNPs being selected from position 51 in any one of SEQ ID Nos. 535, 505, and 515, wherein the diagnostic kit comprises less than 1000 probes.

6. The diagnostic kit of any of claims 1-5, wherein at least one probe of the plurality of probes has a length selected from the group of from 25 to 35, 18 to 30, and 17 to 24 nucleotides.

7. The diagnostic kit of any of claims 1-6, wherein at least one probe of the plurality of probes has a label capable of being detected by electrical, fluorescent or radioactive means.

8. The diagnostic kit of any of claims 1-7, wherein the plurality of probes are affixed to a substrate.

**9.** The diagnostic kit of any of claims 1-8, wherein the kit comprises probes for the detection of a bi-allelic or multi-allelic SNP on homologous chromosomes in a diploid organism.

**10.** A method of distinguishing a human patient having susceptibility to Sudden Cardiac Arrest (SCA), comprising the step of analyzing a genetic sample from the human patient and detecting at least one Single Nucleotide Polymorphism (SNP) in a genetic sample from the human patient at position 51 in any of SEQ ID Nos. 1-849 or at position 26 or 27 in any of SEQ ID Nos. 850-858 using the diagnostic kit of any of claims 1-6.

**11.** A method of distinguishing a human patient having susceptibility to Sudden Cardiac Arrest (SCA), comprising the steps of analyzing a genetic sample from the human patient and detecting Single Nucleotide Polymorphisms (SNPs) in a genetic sample from the human patient at position 51 in SEQ ID No. 844, and detecting at least one SNP at position 51 in any of SEQ ID Nos. 825, 831, and 833 using the diagnostic kit of any of claims 1-6, wherein the presence or absence of the SNPs can be used to assess susceptibility to SCA.

**12.** The method of claim 11, wherein the human patient is assigned to one of the following groups (A)-(E) based upon bi-allelic or multi-allelic SNPs detected at position 51 in any of SEQ ID Nos. 825, 831, 833, and 844:

(A) a genotype TC or TT for SEQ ID No. 831 and a genotype TT for SEQ ID No. 844,
(B) a genotype CC for SEQ ID No. 831 and a genotype TT for SEQ ID No. 844,
(C) a genotype TC or TT for SEQ ID No. 825, a genotype GG for SEQ ID No. 833, and a genotype CC or TC for SEQ ID No. 844,
(D) a genotype TC or TT for SEQ ID No. 825, a genotype TG or TT for SEQ ID No. 833 and a genotype CC or TC for SEQ ID No. 844, and
(E) a genotype CC for SEQ ID No. 825 and a genotype CC or TC for SEQ ID No. 844,

wherein the assignment to one of groups (A)-(E) can be used to distinguish susceptibility to SCA and where assignment of the human patient to one of groups B,
D and E indicates susceptibility to SCA.

**13.** A method of distinguishing a human patient having susceptibility to Sudden Cardiac Arrest (SCA), comprising the steps of analyzing a genetic sample from the human patient and detecting at least one Single Nucleotide Polymorphism (SNP) at position 51 in any of SEQ ID Nos. 835, 832, 844, 846, 838, 848, 829, 842, 827, 828, 824, 836, 840, 845, 826, 837, 841, 843, 117, 535, 823, 834, 830, 847, and 849 using the diagnostic kit of any of claims 1-6.

**14.** The method of claim 10, wherein the method comprises detecting at least one SNP in the genetic from the human patient at position 26 or 27 in any of SEQ ID Nos. 850-855 and 858 and evaluating a number of minor alleles present in the genetic sample.

**15.** The method of any of claims 10-14, wherein the step of detecting an SNP is performed using a diagnostic kit comprising at least one probe that determines the presence or absence of one or more SNPs associated with SCA in the genetic sample, said one or more SNPs being selected from position 51 in any one of SEQ ID Nos. 1-849 or position 26 or 27 in any one of SEQ ID Nos. 850-855 and 858.

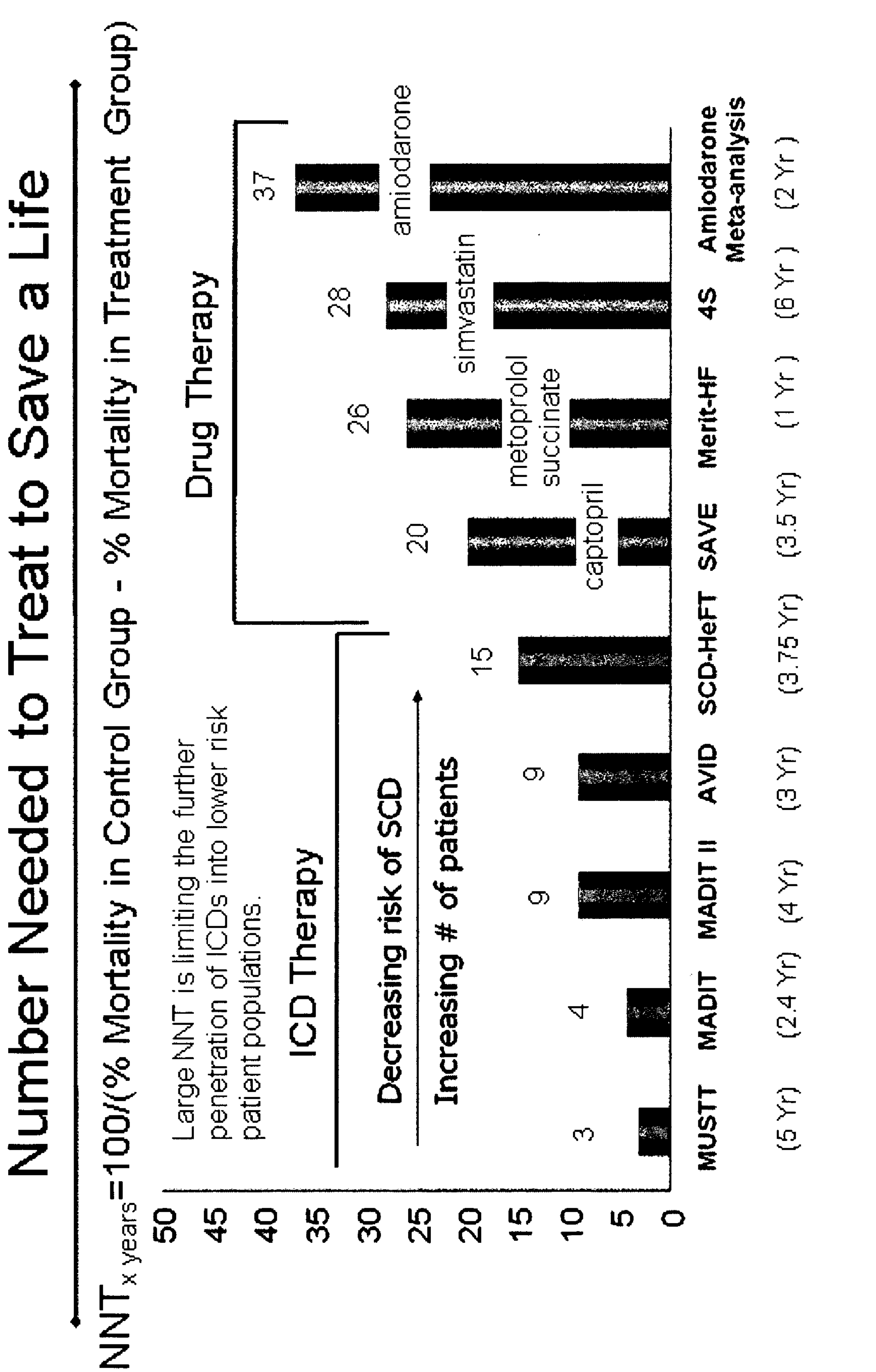
Number Needed to Treat to Save a Life
$NNT_{x\ years}$=100/(% Mortality in Control Group - % Mortality in Treatment Group)
50
45
40
35
30
25
20
15
10
5
0
Large NNT is limiting the further penetration of ICDs into lower risk patient populations.
ICD Therapy
Decreasing risk of SCD
Increasing # of patients
Drug Therapy
3
4
9
9
15
20
26
28
37
captopril
metoprolol succinate
simvastatin
amiodarone
MUSTT
MADIT
MADIT II
AVID
SCD-HeFT
SAVE
Merit-HF
4S
Amiodarone Meta-analysis
(5 Yr)
(2.4 Yr)
(4 Yr)
(3 Yr)
(3.75 Yr)
(3.5 Yr)
(1 Yr)
(6 Yr)
(2 Yr)

Fig. 1

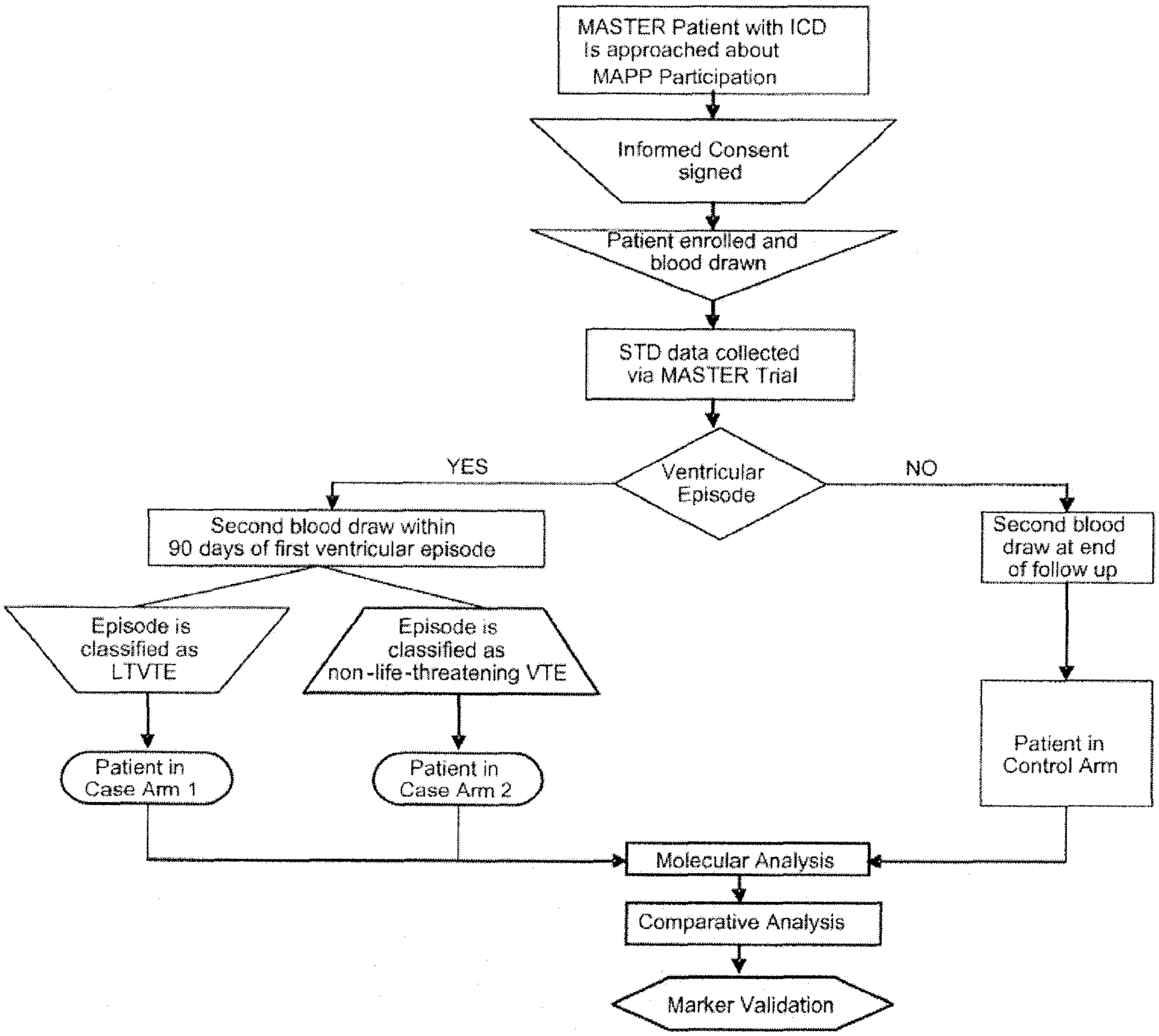
MASTER Patient with ICD
Is approached about
MAPP Participation
Informed Consent
signed
Patient enrolled and
blood drawn
STD data collected
via MASTER Trial
Ventricular
Episode
YES
NO
Second blood draw within
90 days of first ventricular episode
Second blood
draw at end
of follow up
Episode is
classified as
LTVTE
Episode is
classified as
non-life-threatening VTE
Patient in
Case Arm 1
Patient in
Case Arm 2
Patient in
Control Arm
Molecular Analysis
Comparative Analysis
Marker Validation

Fig. 2

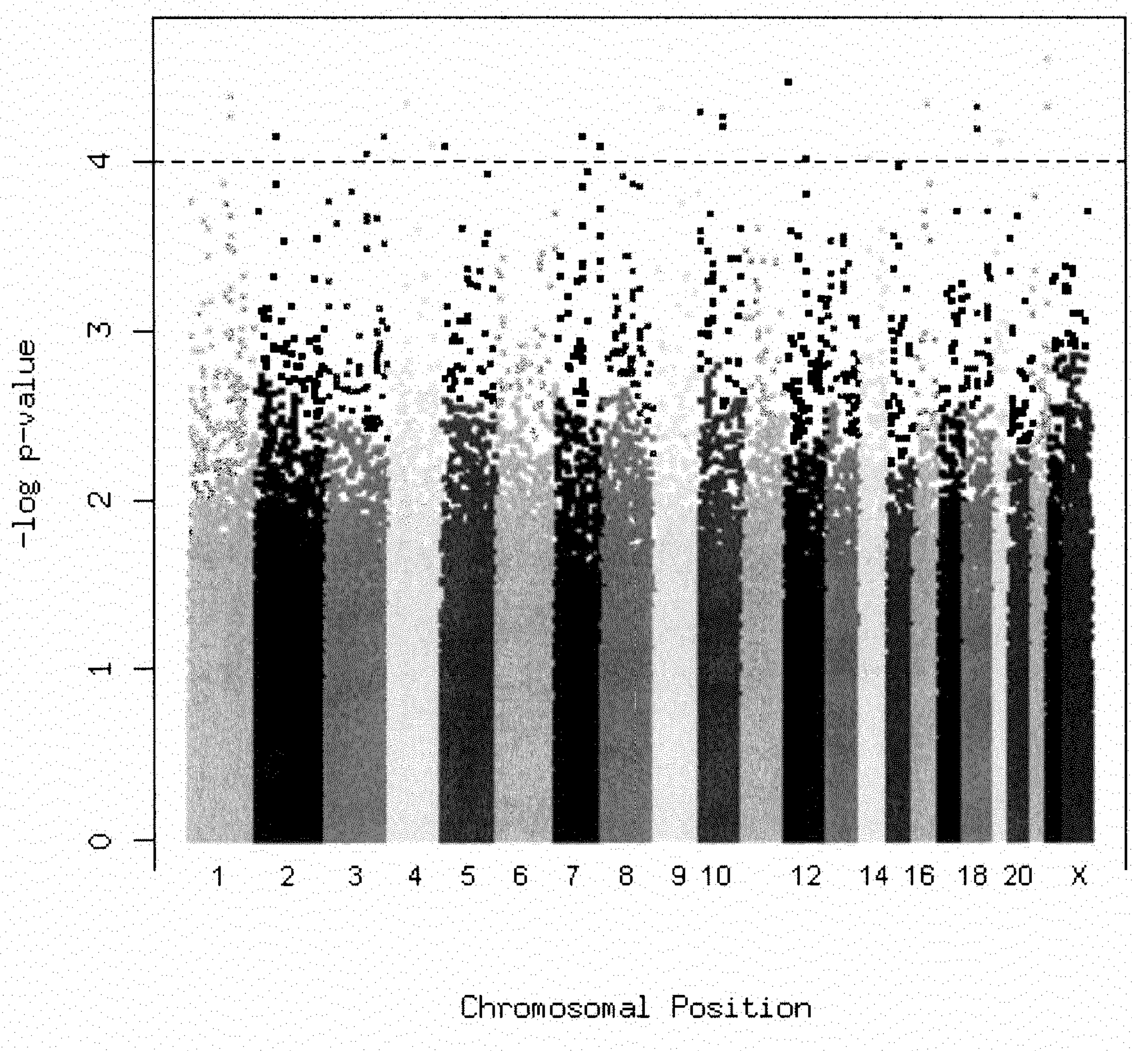
-log p-value
0
1
2
3
4
1
2
3
4
5
6
7
8
9
10
12
14
16
18
20
X
Chromosomal Position

Fig. 3

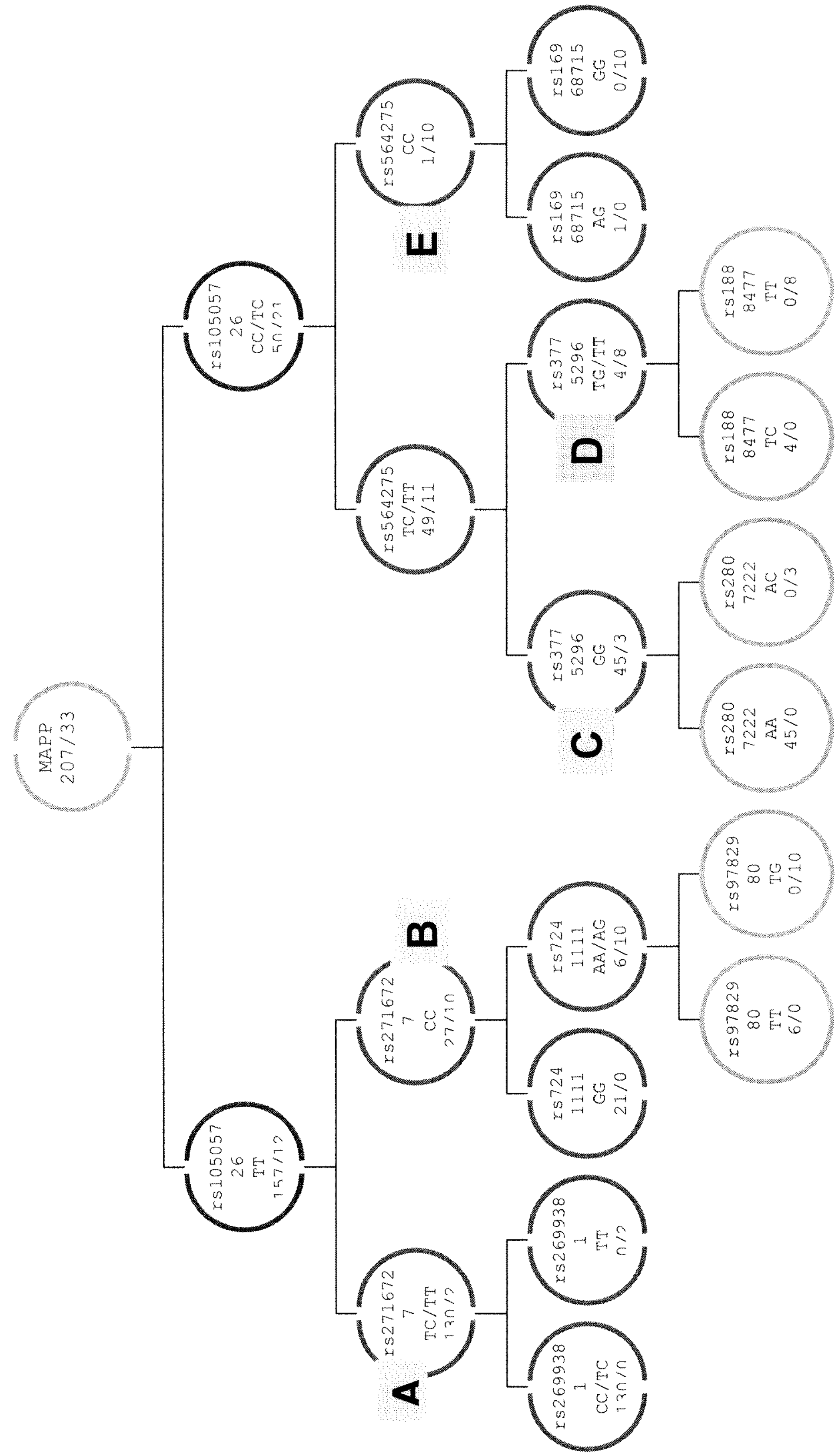
MAPP 207/33
rs105057 26 TT
rs105057 26 CC/TC
rs271672 7 TC/TT
rs271672 7 CC
rs564275 TC/TT 49/11
rs564275 CC 1/10
rs269938 1 CC/TC
rs269938 1 TT
rs724 1111 GG 21/0
rs724 1111 AA/AG 6/10
rs97829 80 TT 6/0
rs97829 80 TG 0/10
rs377 5296 GG 45/3
rs377 5296 TG/TT 4/8
rs280 7222 AA 45/0
rs280 7222 AC 0/3
rs188 8477 TC 4/0
rs188 8477 TT 0/8
rs169 68715 AG 1/0
rs169 68715 GG 0/10
A
B
C
D
E

Fig. 4

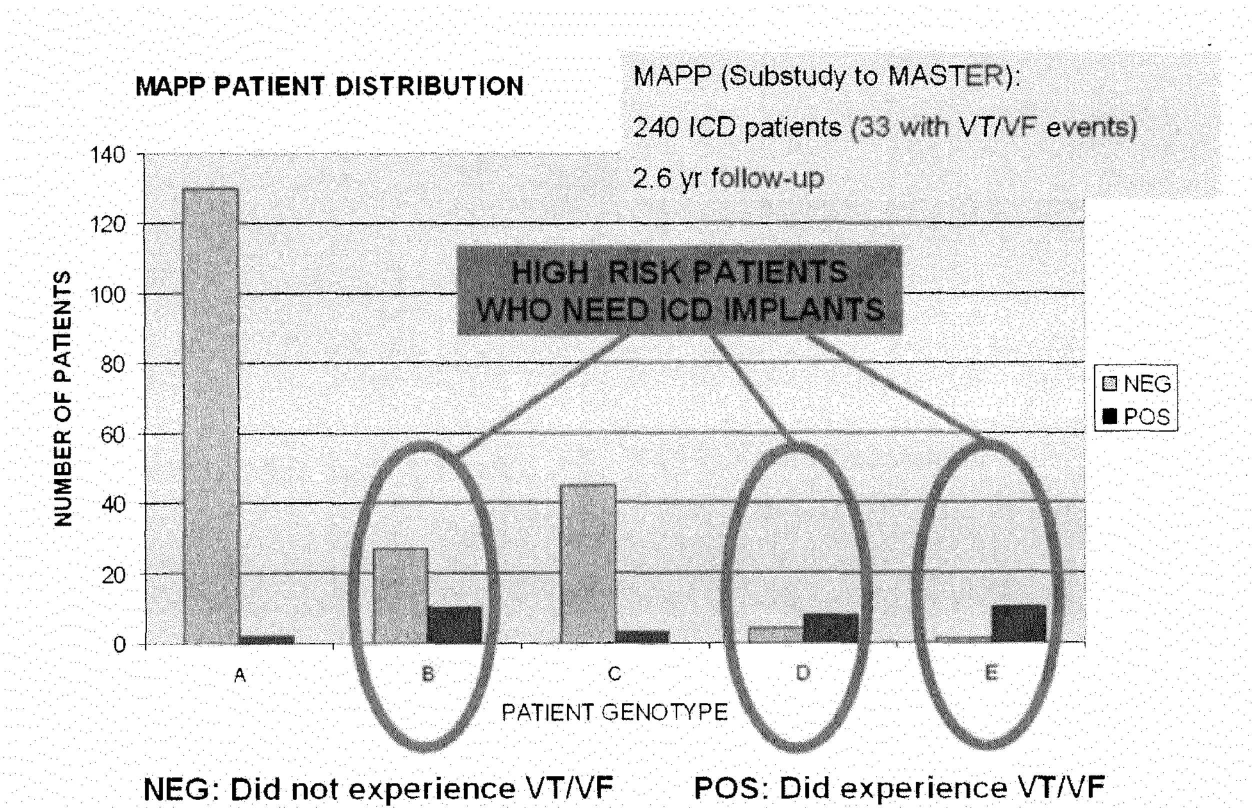
MAPP PATIENT DISTRIBUTION
MAPP (Substudy to MASTER):
240 ICD patients (33 with VT/VF events)
2.6 yr follow-up
HIGH RISK PATIENTS WHO NEED ICD IMPLANTS
NUMBER OF PATIENTS
140
120
100
80
60
40
20
0
A
B
C
D
E
PATIENT GENOTYPE
NEG
POS
NEG: Did not experience VT/VF
POS: Did experience VT/VF

Fig. 5A

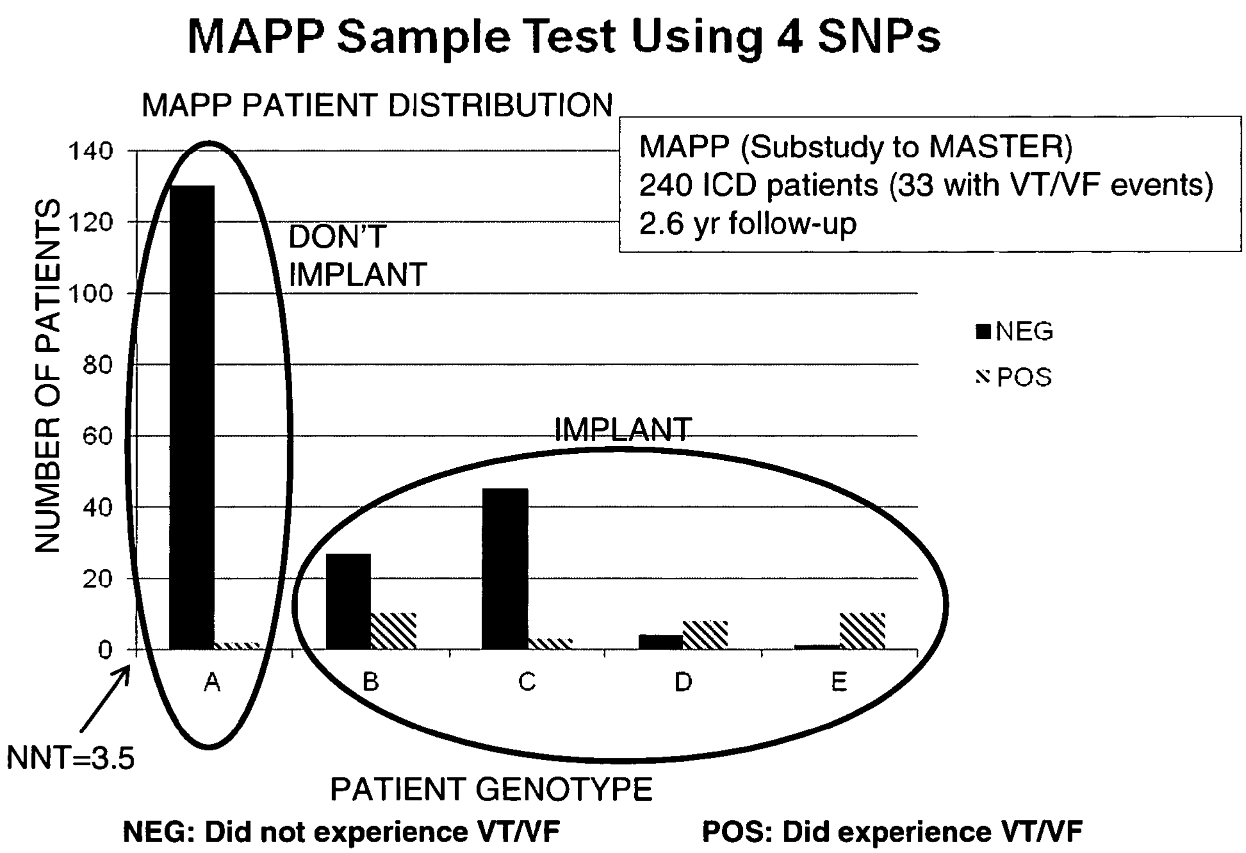
MAPP Sample Test Using 4 SNPs
MAPP PATIENT DISTRIBUTION
MAPP (Substudy to MASTER)
240 ICD patients (33 with VT/VF events)
2.6 yr follow-up
NUMBER OF PATIENTS
140
120
100
80
60
40
20
0
DON'T
IMPLANT
IMPLANT
NEG
POS
A
B
C
D
E
NNT=3.5
PATIENT GENOTYPE
NEG: Did not experience VT/VF
POS: Did experience VT/VF

Fig. 5B

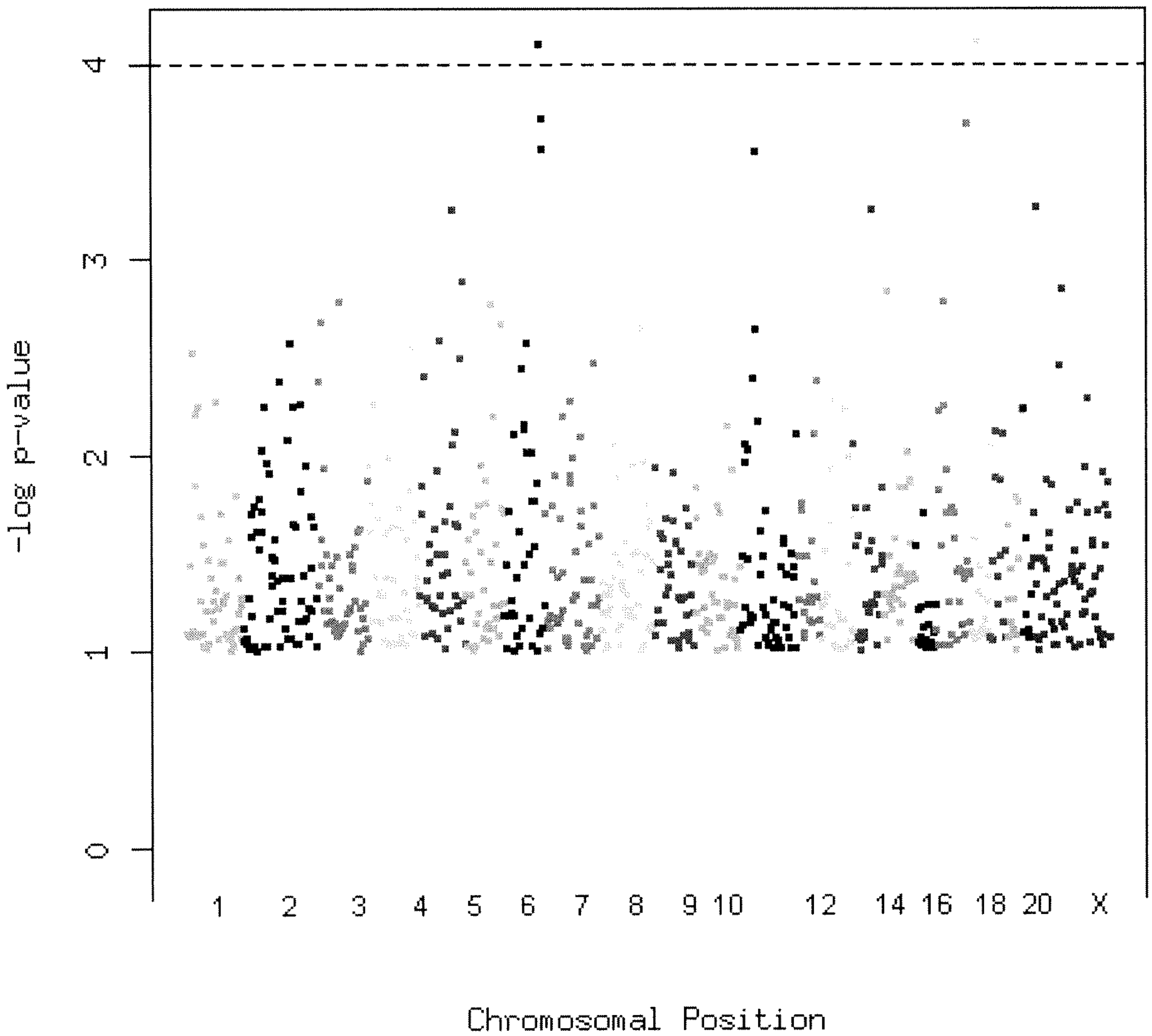
4
3
2
1
0
-log p-value
1
2
3
4
5
6
7
8
9
10
12
14
16
18
20
X
Chromosomal Position

Fig. 6A

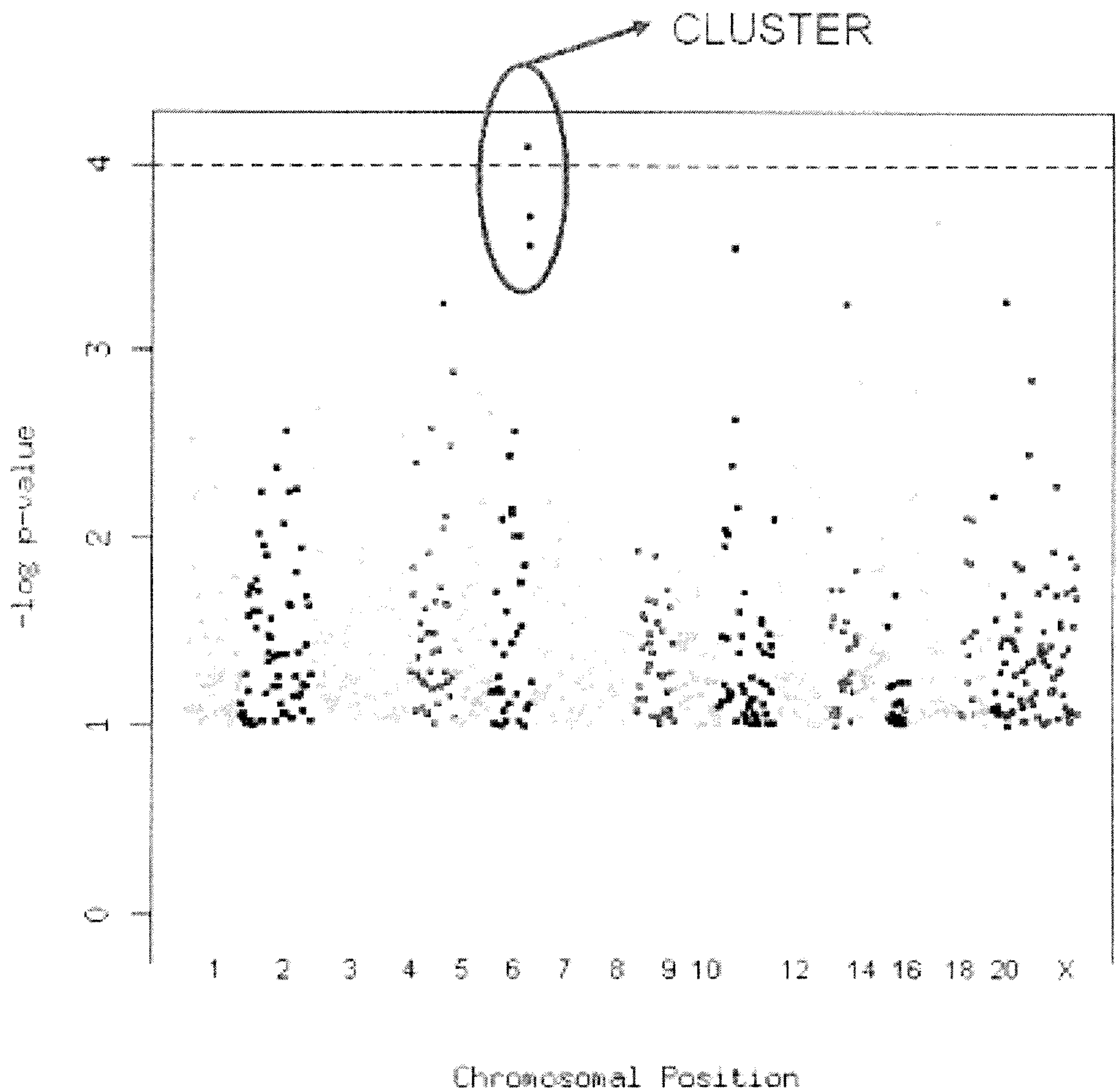
CLUSTER
-log p-value
0
1
2
3
4
1 2 3 4 5 6 7 8 9 10 12 14 16 18 20 X
Chromosomal Position

Fig. 6B

Fig. 7A

**CHOSEN BASED ON LOGICAL CRITERIA (CART Tree)**

| rsnum | p-MAPP | p-IDEA-VF | chromosome | coordinate | band | position | maf_CEU | gene |
|---|---|---|---|---|---|---|---|---|
| rs10505726 | $3.46x10^{-5}$ | | 12 | 3848218 | | 3832219 | 0.217 | PARP11 |
| rs2716727 | $3.67x10^{-3}$ | | 2 | 39807249 | | 18769679 | 0.398 | - |
| rs564275 | $3.72x10^{-3}$ | | 9 | 4084320 | | 4084321 | 0.492 | GLIS3 |
| rs7241111 | $7.33x10^{-3}$ | | 18 | 63002332 | | 12642199 | 0.433 | - |
| rs3775296 | $6.01x10^{-2}$ | | 4 | 187234760 | | 19409707 | 0.158 | TLR3 |

Fig. 7B

**CHOSEN BASED ON BIOLOGICAL CRITERIA (Clustering in Genome)**

| rsnum | p-MAPP | p-IDEA-VF | chromosome | coordinate | band | position | maf_CEU | gene |
|---|---|---|---|---|---|---|---|---|
| rs1439098 | 0.0000808 | 0.06578873 | 7 | 149008444 | 7q36.1 | 161.2927 | 0.075 | |
| rs12666315 | 0.00019123 | 0.06463215 | 7 | 149019972 | 7q36.1 | 161.2987 | 0.075 | |
| rs6974082 | 0.00027459 | 0.06463215 | 7 | 149025379 | 7q36.1 | 161.3015 | - | |

*rch:tme: 12/6/2011*

Fig. 7C

**CHOSEN BASED ON STATISTICAL CRITERIA (Min RADIUS)**

| rsnum | p-MAPP | p-IDEA-VF | chromosome | coordinate | band | position | maf_CEU | gene | RADIUS |
|---|---|---|---|---|---|---|---|---|---|
| rs5745709 | 0.0073422 | 0.0011657 | 7 | 81187348 | 7q21.11 | 93.9464 | 0.183 | HGF | 0.007434209 |
| rs17816553 | 0.0054415 | 0.0065790 | 4 | 7515895 | 4p16.1 | 17.4197 | 0.233 | SORCS2 | 0.008537743 |
| rs3743123 | 0.0088110 | 0.0026839 | 15 | 32832349 | 15q14 | 31.9664 | 0.267 | GJA9 | 0.009210693 |
| rs2224439 | 0.0102084 | 0.0042898 | 14 | 95379296 | 14q32.13 | 103.3552 | 0.058 | | 0.011073072 |
| rs10277213 | 0.0026731 | 0.0111267 | 7 | 89914649 | 7q21.13 | 101.0619 | 0.05 | | 0.011443299 |
| rs5958032 | 0.0051866 | 0.0111267 | X | 121401586 | Xq25 | 122.8757 | 0.067 | | 0.012276149 |
| rs2839343 | 0.0058008 | 0.0111267 | 21 | 46840297 | 21q22.3 | 78.363 | 0.068 | | 0.012547998 |
| rs2839347 | 0.0059863 | 0.0111267 | 21 | 46841376 | 21q22.3 | 78.3687 | 0.068 | S100B | 0.012634859 |
| rs8119972 | 0.0133458 | 0.0044728 | 20 | 20313226 | 20p11.23 | 49.7898 | 0.138 | | 0.014075346 |
| rs12563141 | 0.0140454 | 0.0016218 | 1 | 21595895 | 1p36.12 | 40.1344 | 0.058 | | 0.014138729 |
| rs2214827 | 0.0068444 | 0.0136185 | 7 | 81215852 | 7q21.11 | 93.977 | 0.217 | HGF | 0.015241673 |
| rs13322750 | 0.0114222 | 0.0105786 | 3 | 31497163 | 3p24.1 | 56.6149 | 0.058 | | 0.015568343 |
| rs6920474 | 0.0062091 | 0.0159179 | 6 | 134887043 | 6q23.2 | 135.0827 | 0.183 | | 0.017086081 |
| rs1326800 | 0.0128645 | 0.0119856 | 9 | 12768224 | 9p23 | 26.293 | 0.108 | C9orf150 | 0.017582634 |
| rs7580162 | 0.0026186 | 0.0174555 | 2 | 151363759 | 2q23.3 | 161.2763 | 0.333 | | 0.017650776 |
| rs11564361 | 0.0116754 | 0.0136185 | 18 | 24158866 | 18q12.1 | 51.6047 | 0.267 | | 0.017938209 |
| rs2957370 | 0.0184100 | 0.0000000 | 15 | 70112649 | 15q23 | 77.6275 | 0 | MYO9A | 0.018410041 |

*rch:tme:12/6/2011*

| | | | | | | | | | | |
|---|---|---|---|---|---|---|---|---|---|---|
| rs6529997 | 0.0005412 | 0.0184575 | X | | 6833284 | Xp22.31 | 13.7834 | 0.333 | | 0.018465477 |
| rs9952567 | 0.0186102 | 0.0050887 | | 18 | 35622402 | 18q12.3 | 60.0578 | 0.267 | | 0.019293327 |
| rs993380 | 0.0102555 | 0.0174555 | | 4 | 83803520 | 4q21.22 | 90.1371 | 0.314 | SCD5 | 0.020245194 |
| rs11013998 | 0.0205839 | 0.0005774 | | 10 | 24593631 | 10p12.2 | 47.8415 | 0.317 | KIAA1217 | 0.02059198 |
| rs4920513 | 0.0061901 | 0.0196513 | | 1 | 18733715 | 1p36.13 | 34.8465 | 0.183 | | 0.020603211 |
| rs4916425 | 0.0134482 | 0.0159179 | | 3 | 197509116 | 3q29 | 218.4754 | 0.375 | MGC33212 | 0.020838295 |
| rs1039539 | 0.0211397 | 0.0026072 | | 4 | 17783691 | 4p15.32 | 34.5731 | 0.267 | | 0.021299847 |
| rs7565358 | 0.0093864 | 0.0197066 | | 2 | 72324831 | 2p13.2 | 97.2 | 0.117 | EXOC6B | 0.021827819 |
| rs12775410 | 0.0215276 | 0.0042898 | | 10 | 52769074 | 10q21.1 | 70.4033 | 0.05 | PRKG1 | 0.021950836 |
| rs500586 | 0.0202441 | 0.0087101 | | 1 | 40847796 | 1p34.2 | 65.3128 | 0.314 | | 0.022038401 |
| rs2460842 | 0.0187050 | 0.0119856 | | 15 | 33965754 | 15q14 | 34.005 | 0.15 | | 0.022215546 |
| rs4913391 | 0.0193388 | 0.0111267 | | 12 | 66697040 | 12q15 | 83.1805 | 0.058 | | 0.022311252 |
| rs12617566 | 0.0228079 | 0.0000339 | | 2 | 167435455 | 2q24.3 | 171.5735 | 0.175 | | 0.022807915 |
| rs5904750 | 0.0179018 | 0.0153430 | X | | 146477804 | Xq27.3 | 165.8925 | 0.383 | | 0.023577215 |
| rs12456839 | 0.0194347 | 0.0136185 | | 18 | 24149733 | 18q12.1 | 51.5962 | 0.267 | | 0.023731209 |
| rs1808380 | 0.0032006 | 0.0240092 | | 5 | 166913425 | 5q34 | 174.1105 | 0.208 | | 0.024221629 |
| rs10440133 | 0.0020710 | 0.0259136 | | 3 | 24621080 | 3p24.2 | 46.4964 | 0.142 | | 0.025996221 |
| rs1077388 | 0.0195398 | 0.0174555 | | 18 | 35655607 | 18q12.3 | 60.0776 | 0.2 | | 0.026201109 |
| rs521095 | 0.0255991 | 0.0065622 | | 2 | 44967356 | 2p21 | 69.9724 | 0.183 | | 0.026426801 |
| rs7912419 | 0.0250324 | 0.0084777 | | 10 | 7828501 | 10p14 | 22.0568 | 0.183 | ITIH2 | 0.026428988 |
| rs713900 | 0.0263639 | 0.0111267 | | 22 | 25228242 | 22q12.1 | 25.6677 | 0.05 | TFIP11 | 0.028615718 |
| rs9620587 | 0.0057789 | 0.0283225 | | 22 | 24776611 | 22q12.1 | 24.2476 | 0.058 | | 0.028906066 |
| rs1466123 | 0.0261723 | 0.0126964 | | 3 | 22546900 | 3p24.3 | 43.58 | 0.217 | | 0.029089257 |
| rs6031454 | 0.0078076 | 0.0283225 | | 20 | 42267296 | 20q13.12 | 68.3472 | 0.083 | C20orf111 | 0.029378962 |
| rs1075042 | 0.0036102 | 0.0292182 | | 7 | 71033086 | 7q11.22 | 84.5634 | 0.356 | CALN1 | 0.029440373 |
| rs12618696 | 0.0041380 | 0.0292182 | | 2 | 126111628 | 2q14.3 | 137.62 | 0.179 | | 0.029509751 |
| rs1434250 | 0.0088507 | 0.0283225 | | 9 | 8726813 | 9p24.1 | 19.2394 | 0.202 | PTPRD | 0.029673214 |
| rs3789433 | 0.0051923 | 0.0292182 | | 1 | 94348028 | 1p22.1 | 117.6364 | 0.297 | ABCA4 | 0.029675954 |

*rch:tme:12/6/2011*

| | | | | | | | | | | |
|---|---|---|---|---|---|---|---|---|---|---|
| rs3021146 | 0.0240273 | 0.0180694 | | 4 | 2927984 | 4p16.3 | 3.9288 | 0.342 | C4orf9 | 0.0300635 |
| rs1484700 | 0.0002001 | 0.0308232 | | 18 | 61692220 | 18q22.1 | 92.1464 | 0.258 | CDH7 | 0.030823832 |
| rs12388064 | 0.0306167 | 0.0042898 | X | | 85471717 | Xq21.2 | 87.9956 | 0.058 | DACH2 | 0.030915742 |
| rs752908 | 0.0267284 | 0.0159179 | | 18 | 35654891 | 18q12.3 | 60.0767 | 0.217 | | 0.031109323 |
| rs5916138 | 0.0199056 | 0.0240092 | X | | 5477127 | Xp22.32 | 12.71 | 0.317 | | 0.031187731 |
| rs6546754 | 0.0242383 | 0.0197066 | | 2 | 72348584 | 2p13.2 | 97.2 | 0.117 | EXOC6B | 0.031238486 |
| rs13319027 | 0.0320040 | 0.0018763 | | 3 | 31497216 | 3p24.1 | 56.615 | 0.172 | | 0.032058995 |
| rs1872667 | 0.0263208 | 0.0184575 | | 16 | 29206878 | 16p11.2 | 55.1913 | 0.229 | | 0.032147557 |
| rs11765962 | 0.0138821 | 0.0290788 | | 7 | 139772976 | 7q34 | 149.0463 | 0.05 | | 0.032222505 |
| rs7744524 | 0.0196695 | 0.0259136 | | 6 | 168485943 | 6q27 | 186.2014 | 0.208 | | 0.032533107 |
| rs2979715 | 0.0192557 | 0.0263965 | | 8 | 80724814 | 8q21.13 | 89.88 | 0.167 | STMN2 | 0.03267352 |
| rs10828726 | 0.0261275 | 0.0196513 | | 10 | 18784954 | 10p12.31 | 43.4922 | 0.375 | CACNB2 | 0.032692843 |
| rs3957526 | 0.0005618 | 0.0329077 | | 15 | 81921195 | 15q25.2 | 89.0154 | 0.398 | SH3GL3 | 0.032912534 |
| rs859941 | 0.0122606 | 0.0308232 | X | | 143175898 | Xq27.3 | 157.8226 | 0.444 | | 0.033172149 |
| rs1206610 | 0.0173704 | 0.0283225 | X | | 95429841 | Xq21.33 | 97.0469 | 0.042 | | 0.033224919 |
| rs1403719 | 0.0319972 | 0.0091591 | | 3 | 122209632 | 3q13.33 | 126.98 | 0.167 | STXBP5L | 0.033282253 |
| rs16898178 | 0.0277721 | 0.0184575 | | 5 | 28851719 | 5p14.1 | 47.8329 | 0.233 | | 0.03334618 |
| rs275946 | 0.0078708 | 0.0329077 | | 13 | 108594719 | 13q33.3 | 115.2998 | 0.333 | RP11-54H7.1 | 0.033835918 |
| rs7120737 | 0.0196150 | 0.0290788 | | 11 | 47658971 | 11p11.2 | 63.81 | 0.108 | AGBL2 | 0.035076007 |
| rs1540610 | 0.0143531 | 0.0320688 | | 16 | 79019538 | 16q23.2 | 101.7947 | 0.158 | LOC729847 | 0.035134329 |
| rs1357086 | 0.0243392 | 0.0263965 | | 3 | 155237713 | 3q25.2 | 162.6836 | 0.15 | | 0.035905069 |
| rs7664824 | 0.0233018 | 0.0273971 | | 4 | 114548564 | 4q26 | 118.1743 | 0.133 | | 0.035966296 |
| rs7457368 | 0.0164067 | 0.0320688 | | | | 7q36.2 | 170.8261 | 0.11 | | 0.03602207 |
| rs5916139 | 0.0338334 | 0.0136185 | X | | 5479735 | Xp22.32 | 12.71 | 0.333 | | 0.036471349 |
| rs13357969 | 0.0221551 | 0.0293080 | | 5 | 150731750 | 5q33.1 | 156.8419 | 0.325 | | 0.036739702 |
| rs10520944 | 0.0347929 | 0.0126964 | | 5 | 28874857 | 5p14.1 | 47.8478 | 0.233 | | 0.037037036 |
| rs1335420 | 0.0234477 | 0.0293080 | | 9 | 119051936 | 9q33.1 | 123.915 | 0.117 | ASTN2 | 0.037533319 |
| rs10498644 | 0.0360069 | 0.0111267 | | 14 | 95332401 | 14q32.13 | 103.2548 | 0.058 | | 0.037686854 |

*rch:tme:12/6/2011*

| | | | | | | | | | | |
|---|---|---|---|---|---|---|---|---|---|---|
| rs8037430 | 0.0377463 | 0.0026839 | | 15 | 89124274 | 15q26.1 | 97.1592 | 0.275 | BLM | 0.037841638 |
| rs4150992 | 0.0366794 | 0.0093867 | | 19 | 45620784 | 19q13.2 | 66.5342 | - | SERTAD1 | 0.037861411 |
| rs2246649 | 0.0372362 | 0.0072224 | | 11 | 131006217 | 11q25 | 145.6363 | 0.183 | | 0.037930133 |
| rs12543841 | 0.0379742 | 0.0005471 | | 8 | 10116263 | 8p23.1 | 22.5816 | 0.217 | MSRA | 0.037978123 |
| rs10519034 | 0.0164067 | 0.0343322 | | 2 | 65559829 | 2p14 | 87.2052 | 0.225 | FLJ16124 | 0.038050985 |
| rs10483366 | 0.0311820 | 0.0218702 | | 14 | 30061310 | 14q12 | 26.457 | 0.178 | | 0.038087017 |
| rs4941887 | 0.0172621 | 0.0343322 | | 13 | 37590585 | 13q13.3 | 38.4517 | 0.183 | | 0.038427561 |
| rs2214825 | 0.0352932 | 0.0153430 | | 7 | 81209555 | 7q21.11 | 93.9703 | 0.217 | HGF | 0.038484051 |
| rs6448011 | 0.0205909 | 0.0329077 | | 4 | 20698861 | 4p15.31 | 36.4983 | 0.195 | KCNIP4 | 0.038818873 |
| rs17331632 | 0.0354181 | 0.0166361 | | 3 | 126687267 | 3q21.2 | 132.5986 | 0.208 | SNX4 | 0.039130582 |
| rs5974731 | 0.0191464 | 0.0343322 | X | | 137247871 | Xq26.3 | 141.2529 | 0.275 | | 0.039310042 |
| rs7934354 | 0.0395189 | 0.0000000 | | 11 | 5522482 | 11p15.4 | 8.5995 | 0.017 | OR52H1 | 0.03951889 |
| rs638540 | 0.0199752 | 0.0343322 | | 6 | 54439570 | 6p12.1 | 78.0957 | 0.142 | | 0.039720318 |
| rs10965597 | 0.0354980 | 0.0180694 | | 9 | 23071306 | 9p21.3 | 44.2971 | 0.309 | | 0.039832262 |
| rs1211554 | 0.0350464 | 0.0197066 | | 6 | 601143 | 6p25.3 | 0 | 0.1 | EXOC2,HUS1B | 0.040206981 |
| rs2078548 | 0.0327472 | 0.0240092 | | 16 | 13473075 | 16p13.12 | 32.1754 | 0.275 | | 0.040605715 |
| rs7980489 | 0.0241318 | 0.0329077 | | 12 | 47299183 | 12q13.11 | 62.912 | 0.358 | | 0.040807629 |
| rs2097130 | 0.0284556 | 0.0293080 | | 6 | 162364287 | 6q26 | 172.8022 | 0.142 | PARK2 | 0.040849455 |
| rs5962157 | 0.0371885 | 0.0174555 | X | | 4240632 | Xp22.33 | 11.963 | 0.475 | | 0.041081323 |
| rs10491952 | 0.0320040 | 0.0259136 | | 9 | 11445955 | 9p23 | 24.7681 | 0.25 | | 0.041179766 |
| rs10809523 | 0.0320040 | 0.0259136 | | 9 | 11551385 | 9p23 | 24.8897 | 0.25 | | 0.041179766 |
| rs12341391 | 0.0112419 | 0.0402216 | | 9 | 91038498 | 9q22.2 | 90.99 | 0.147 | | 0.041763084 |
| rs2922066 | 0.0282407 | 0.0308232 | | 8 | 99970798 | 8q22.2 | 104.6026 | 0.267 | | 0.041804388 |
| rs8004273 | 0.0116577 | 0.0402216 | | 14 | 41612067 | 14q21.1 | 43.4985 | 0.208 | | 0.041876911 |
| rs12628984 | 0.0413074 | 0.0084777 | | 3 | 59399831 | 3p14.2 | 79.8824 | 0.406 | | 0.042168346 |
| rs725173 | 0.0206117 | 0.0368363 | | 8 | 23520715 | 8p21.2 | 41.6199 | 0.108 | | 0.042210855 |
| rs730645 | 0.0415912 | 0.0103578 | | 1 | 74890760 | 1p31.1 | 100.1209 | 0.178 | C1orf173 | 0.042861511 |

*rch:tme:12/6/2011*

| | | | | | | | | | | | |
|---|---|---|---|---|---|---|---|---|---|---|---|
| rs2647582 | 0.0206570 | 0.0376389 | | 11 | 5380746 | 11p15.4 | 8.58 | | 0.112 | | 0.042934788 |
| rs3750490 | 0.0412541 | 0.0119856 | | 9 | 8623097 | 9p24.1 | 19.0164 | - | | PTPRD | 0.042959913 |
| rs1879894 | 0.0276807 | 0.0329077 | | 15 | 78055874 | 15q25.1 | 84.0807 | | 0.322 | | 0.043001613 |
| rs4453850 | 0.0237996 | 0.0368363 | | 3 | 176281449 | 3q26.31 | 180.2407 | | 0.442 | NAALADL2 | 0.043855838 |
| rs10876488 | 0.0326319 | 0.0293080 | | 12 | 52384399 | 12q13.13 | 68.9154 | | 0.283 | | 0.043861111 |
| rs9978739 | 0.0176548 | 0.0401583 | | 21 | 46815526 | 21q22.3 | 78.2333 | | 0.085 | | 0.04386782 |
| rs7660418 | 0.0412399 | 0.0153430 | | 4 | 11630237 | 4p15.33 | 25.5661 | | 0.35 | | 0.044001611 |
| rs736998 | 0.0005506 | 0.0441741 | | 5 | 121861320 | 5q23.2 | 126.8904 | | 0.233 | | 0.044177495 |
| rs391678 | 0.0322778 | 0.0308232 | | 13 | 31453287 | 13q13.1 | 29.4798 | | 0.325 | | 0.044631003 |
| rs392840 | 0.0379122 | 0.0240092 | | 21 | 27140252 | 21q21.3 | 27.8353 | | 0.242 | | 0.04487518 |
| rs10760793 | 0.0309634 | 0.0329077 | | 9 | 103168348 | 9q31.1 | 104.1061 | | 0.35 | BAAT | 0.045184602 |
| rs2852143 | 0.0396004 | 0.0218702 | | 18 | 65622967 | 18q22.2 | 97.7326 | | 0.15 | DOK6 | 0.045238226 |
| rs628572 | 0.0396495 | 0.0217970 | | 6 | 16873481 | 6p22.3 | 37.2738 | | 0.05 | | 0.045245913 |
| rs7029465 | 0.0213162 | 0.0402216 | | 9 | 120317363 | 9q33.1 | 124.983 | | 0.058 | | 0.045520903 |
| rs10780770 | 0.0314922 | 0.0329077 | | 9 | 88373314 | 9q21.33 | 85.6428 | | 0.342 | | 0.045548611 |
| rs7801603 | 0.0167772 | 0.0423829 | | 7 | 129360558 | 7q32.2 | 130.8419 | | 0.153 | UBE2H | 0.04558269 |
| rs12025142 | 0.0415100 | 0.0197066 | | 1 | 222942724 | 1q42.12 | 228.8658 | | 0.142 | CNIH3 | 0.045950282 |
| rs1465067 | 0.0458535 | 0.0065622 | X | | 7017959 | Xp22.31 | 13.989 | | 0.283 | HDHD1A | 0.046320714 |
| rs4540026 | 0.0150042 | 0.0441741 | | 4 | 153888854 | 4q31.3 | 146.4138 | | 0.383 | | 0.046652694 |
| rs12908846 | 0.0287740 | 0.0368363 | | 15 | 37697696 | 15q14 | 41.0393 | | 0.1 | FSIP1 | 0.046742427 |
| rs2018094 | 0.0333515 | 0.0329077 | X | | 12182018 | Xp22.2 | 22.4049 | | 0.483 | FRMPD4 | 0.046853417 |
| rs5908645 | 0.0373880 | 0.0283225 | X | | 142261256 | Xq27.3 | 155.075 | | 0.1 | | 0.046904429 |
| rs2096509 | 0.0338021 | 0.0329077 | | 21 | 46823682 | 21q22.3 | 78.276 | | 0.314 | | 0.047175206 |
| rs1247451 | 0.0466736 | 0.0072224 | | 10 | 29980466 | 10p11.23 | 55.9284 | | 0.208 | SVIL | 0.047229135 |
| rs780188 | 0.0432877 | 0.0196513 | | 5 | 22825793 | 5p14.3 | 42.2079 | | 0.258 | CDH12 | 0.047539444 |
| rs7376535 | 0.0175773 | 0.0441741 | | 4 | 6287415 | 4p16.1 | 11.886 | | 0.417 | LOC285484 | 0.047542696 |
| rs8037172 | 0.0257803 | 0.0402216 | | 15 | 57805779 | 15q22.2 | 60.014 | | 0.075 | | 0.047774431 |
| rs12696410 | 0.0009222 | 0.0479910 | | | | 3q26.32 | 181.6666 | | 0.317 | | 0.047999821 |

*rch:tme:12/6/2011*

| | | | | | | | | | | |
|---|---|---|---|---|---|---|---|---|---|---|
| rs3751109 | 0.0226956 | 0.0423829 | | 2 | 238091933 | 2q37.3 | 250.6036 | 0.142 | MLPH | 0.048077003 |
| rs3751107 | 0.0226956 | 0.0423829 | | 2 | 238091990 | 2q37.3 | 250.6038 | 0.142 | MLPH | 0.048077003 |
| rs7091141 | 0.0408770 | 0.0259136 | | 10 | 45468332 | 10q11.21 | 65.9836 | 0.167 | ANUBL1 | 0.048398766 |
| rs1986391 | 0.0393500 | 0.0283225 | X | | 99438936 | Xq22.1 | 101.1831 | 0.1 | PCDH19 | 0.048482867 |
| rs7741540 | 0.0343094 | 0.0343322 | | 6 | 123737380 | 6q22.31 | 123.0773 | 0.1 | TRDN | 0.04853689 |
| rs12558527 | 0.0434237 | 0.0217970 | X | | 62780361 | Xq11.2 | 79.8184 | 0.058 | ARHGEF9 | 0.048587288 |
| rs735262 | 0.0486983 | 0.0000000 | | 9 | 27252761 | 9p21.2 | 49.7635 | 0.05 | | 0.04869827 |
| rs11995187 | 0.0410767 | 0.0263965 | | 8 | 20176671 | 8p21.3 | 34.6428 | 0.167 | | 0.048826904 |
| rs1568786 | 0.0456755 | 0.0174555 | | 2 | 99788199 | 2q11.2 | 114.3156 | 0.192 | AFF3 | 0.048897274 |
| rs1994283 | 0.0094154 | 0.0479910 | | 12 | 16926954 | 12p12.3 | 35.32 | 0.325 | | 0.048905845 |
| rs4851235 | 0.0322934 | 0.0368363 | | 2 | 99816304 | 2q11.2 | 114.3332 | 0.142 | AFF3 | 0.048987533 |
| rs13294002 | 0.0316351 | 0.0376389 | | 9 | 91095959 | 9q22.2 | 91.1299 | 0.15 | | 0.049167733 |
| rs6783129 | 0.0286760 | 0.0402216 | | 3 | 132900519 | 3q22.1 | 138.6622 | 0.1 | CPNE4 | 0.049397232 |
| rs4286327 | 0.0331975 | 0.0368363 | | 2 | 99835385 | 2q11.2 | 114.3451 | 0.142 | AFF3 | 0.049588194 |
| rs3087980 | 0.0025319 | 0.0500125 | | 5 | 56577563 | 5q11.2 | 72.8248 | 0.108 | GPBP1 | 0.050076578 |
| rs4354529 | 0.0055585 | 0.0500125 | | 1 | 26564637 | 1p36.11 | 45.4165 | 0.119 | ZNF683 | 0.050320479 |
| rs7147797 | 0.0505117 | 0.0001842 | | 14 | 35519175 | 14q13.2 | 38.5954 | 0.183 | | 0.050512006 |
| rs1036165 | 0.0245842 | 0.0441741 | | 2 | 56194572 | 2p16.1 | 79.7348 | 0.225 | | 0.050554226 |
| rs10485600 | 0.0074638 | 0.0500125 | | 20 | 2348503 | 20p13 | 8.4895 | 0.117 | TGM6 | 0.050566406 |
| rs10521726 | 0.0498221 | 0.0087695 | X | | 123494902 | Xq25 | 126.4782 | 0.083 | ODZ1 | 0.050587983 |
| rs4785426 | 0.0402406 | 0.0308232 | | 16 | 49050931 | 16q12.1 | 60.2045 | 0.144 | | 0.050689016 |
| rs384366 | 0.0508591 | 0.0011850 | | 6 | 13346218 | 6p24.1 | 30.6847 | 0.283 | PHACTR1 | 0.050872892 |
| rs2317512 | 0.0014083 | 0.0510003 | X | | 39109268 | Xp11.4 | 60.6968 | 0.408 | | 0.051019781 |
| rs1031006 | 0.0139976 | 0.0490637 | | 5 | 14040103 | 5p15.2 | 32.0292 | 0.383 | | 0.051021399 |
| rs4755844 | 0.0179487 | 0.0479910 | | 11 | 44514950 | 11p11.2 | 61.0891 | 0.358 | | 0.051237581 |
| rs1391613 | 0.0318393 | 0.0402216 | | 11 | 5383680 | 11p15.4 | 8.58 | 0.108 | | 0.051298271 |
| rs488150 | 0.0262936 | 0.0441741 | | 1 | 168418141 | 1q24.2 | 172.6603 | 0.167 | | 0.051407196 |
| rs6649251 | 0.0512999 | 0.0033371 | X | | 123484147 | Xq25 | 126.4412 | 0.225 | ODZ1 | 0.051408335 |

*rch:tme:12/6/2011*

| | | | | | | | | | | |
|---|---|---|---|---|---|---|---|---|---|---|
| rs1386689 | 0.0123963 | 0.0500125 | | 8 | 18708302 | 8p22 | 31.7923 | 0.1 | PSD3 | 0.051525923 |
| rs10972872 | 0.0515011 | 0.0023703 | | 9 | 36357338 | 9p13.2 | 57.7876 | 0.283 | RNF38 | 0.0515556 |
| rs880170 | 0.0129958 | 0.0500125 | | 20 | 15956638 | 20p12.1 | 41.9524 | 0.117 | C20orf133 | 0.051673438 |
| rs2420506 | 0.0515282 | 0.0042898 | | 1 | 167994915 | 1q24.2 | 172.0463 | 0.058 | | 0.051706499 |
| rs2381672 | 0.0509721 | 0.0087101 | | 9 | 7847333 | 9p24.1 | 17.4026 | 0.183 | | 0.051710892 |
| rs4535467 | 0.0237975 | 0.0460523 | | 5 | 31245124 | 5p13.3 | 50.741 | 0.095 | CDH6 | 0.051837563 |
| rs10102788 | 0.0427600 | 0.0293080 | | 8 | 97039675 | 8q22.1 | 101.5434 | 0.275 | | 0.051839884 |
| rs2182289 | 0.0496186 | 0.0153430 | X | | 33035448 | Xp21.1 | 50.706 | 0.292 | DMD | 0.051936624 |
| rs733180 | 0.0280787 | 0.0441741 | | 12 | 97366719 | 12q23.1 | 109.9722 | 0.333 | | 0.052342752 |
| rs10820441 | 0.0022790 | 0.0524899 | | 9 | 105301185 | 9q31.1 | 106.0535 | 0.229 | | 0.052539389 |
| rs7712871 | 0.0039347 | 0.0524899 | | 5 | 14105904 | 5p15.2 | 32.1839 | 0.438 | | 0.05263721 |
| rs1544616 | 0.0515444 | 0.0111267 | | 16 | 6686746 | 16p13.2 | 17.5672 | 0.075 | A2BP1 | 0.052731661 |
| rs3007033 | 0.0052941 | 0.0524899 | | 14 | 49173566 | 14q21.3 | 46.4097 | 0.2 | | 0.05275624 |
| rs4240205 | 0.0056694 | 0.0524899 | | 2 | 86910316 | 2p11.2 | 110.5216 | 0.275 | CD8B | 0.052795231 |
| rs9962727 | 0.0059665 | 0.0524899 | | 18 | 10822851 | 18p11.22 | 35.4883 | 0.246 | C18orf58 | 0.052827955 |
| rs17005910 | 0.0294307 | 0.0441741 | | 2 | 70662754 | 2p13.3 | 93.2951 | 0.317 | | 0.053080276 |
| rs1657382 | 0.0391335 | 0.0360770 | | 18 | 52855904 | 18q21.31 | 77.5868 | 0.117 | | 0.053225783 |
| rs1470645 | 0.0233819 | 0.0479910 | | 4 | 61690574 | 4q13.1 | 75.5465 | 0.3 | | 0.053383956 |
| rs7760851 | 0.0488705 | 0.0218702 | | 6 | 90109323 | 6q15 | 95.366 | 0.5 | UBE2J1 | 0.053540937 |
| rs7617041 | 0.0041679 | 0.0535807 | | 3 | 12684167 | 3p25.2 | 30.92 | 0.3 | | 0.053742561 |
| rs1909260 | 0.0358511 | 0.0402216 | | 11 | 5380808 | 11p15.4 | 8.58 | 0.108 | | 0.053880196 |
| rs2089432 | 0.0483214 | 0.0240092 | | 1 | 91433973 | 1p22.2 | 115.5428 | 0.217 | | 0.053957389 |
| rs11883500 | 0.0530750 | 0.0103578 | | 2 | 238099173 | 2q37.3 | 250.6244 | 0.142 | MLPH | 0.054076184 |
| rs1567894 | 0.0134662 | 0.0524899 | X | | 15420776 | Xp22.2 | 29.6326 | 0.417 | PIR | 0.054189781 |
| rs1196152 | 0.0149789 | 0.0524899 | | 2 | 182652693 | 2q31.3 | 187.5558 | 0.45 | PPP1R1C | 0.054585355 |
| rs4984479 | 0.0510631 | 0.0196513 | | 15 | 93167466 | 15q26.2 | 111.1572 | 0.254 | | 0.054713946 |
| rs3788941 | 0.0113165 | 0.0535807 | X | | 119387479 | Xq24 | 119.8394 | 0.433 | ATP1B4 | 0.054762712 |
| rs1429272 | 0.0407179 | 0.0368363 | | 2 | 99794314 | 2q11.2 | 114.3194 | 0.142 | AFF3 | 0.054907783 |

*rch:tme:12/6/2011*

| | | | | | | | | | | |
|---|---|---|---|---|---|---|---|---|---|---|
| rs1122816 | 0.0547001 | 0.0050887 | | 1 | 30535417 | 1p35.2 | 49.3259 | 0.275 | | 0.054936285 |
| rs12121994 | 0.0543084 | 0.0087101 | | 1 | 167524930 | 1q24.2 | 171.3644 | 0.275 | NME7 | 0.055002432 |
| rs7637944 | 0.0550531 | 0.0009829 | | 3 | 132912301 | 3q22.1 | 138.6737 | 0.133 | CPNE4 | 0.055061844 |
| rs7339414 | 0.0277025 | 0.0479910 | | 13 | 106753293 | 13q33.3 | 110.0399 | 0.225 | LOC728215 | 0.055412622 |
| rs10496450 | 0.0422756 | 0.0360770 | | 2 | 113852766 | 2q13 | 125.4916 | 0.108 | | 0.055576812 |
| rs7239567 | 0.0016639 | 0.0556027 | | 18 | 24041954 | 18q12.1 | 51.4956 | 0.167 | | 0.055627613 |
| rs7559811 | 0.0365318 | 0.0423829 | | 2 | 231231148 | 2q37.1 | 236.8397 | 0.225 | | 0.055954248 |
| rs738180 | 0.0506537 | 0.0240092 | | 22 | 44012037 | 22q13.31 | 58.1303 | 0.142 | C22orf9 | 0.056055668 |
| rs7138775 | 0.0370694 | 0.0423829 | | 12 | 127093924 | 12q24.32 | 154.6944 | 0.246 | | 0.056306732 |
| rs17531821 | 0.0205743 | 0.0524899 | | 14 | 98265624 | 14q32.2 | 109.3681 | 0.217 | | 0.056378143 |
| rs4265116 | 0.0095802 | 0.0556027 | | 7 | 114301361 | 7q31.1 | 121.8315 | 0.142 | | 0.056422005 |
| rs1934124 | 0.0179868 | 0.0535807 | | 6 | 73121230 | 6q13 | 86.8413 | 0.258 | RIMS1 | 0.056519167 |
| rs983130 | 0.0191375 | 0.0535807 | | 13 | 31720975 | 13q13.1 | 30.0891 | 0.3 | FRY | 0.056895824 |
| rs3829078 | 0.0272514 | 0.0500125 | | 9 | 35669251 | 9p13.3 | 57.4586 | 0.108 | CA9 | 0.056955191 |
| rs6586604 | 0.0224261 | 0.0524899 | | | | 8p22 | 29.286 | 0.271 | | 0.057079957 |
| rs2157372 | 0.0472678 | 0.0329077 | X | | 99660646 | Xq22.1 | 101.4789 | 0.317 | | 0.057594863 |
| rs13272985 | 0.0507697 | 0.0273971 | | 8 | 54798149 | 8q11.23 | 67.1741 | 0.083 | ATP6V1H | 0.057690244 |
| rs3113173 | 0.0240721 | 0.0524899 | | 7 | 67415386 | 7q11.22 | 79.8875 | 0.3 | | 0.057746492 |
| rs2244541 | 0.0572391 | 0.0093893 | | 5 | 28891040 | 5p14.1 | 47.8582 | 0.267 | | 0.058004099 |
| rs12356112 | 0.0570751 | 0.0103578 | | | | 10p14 | 27.79 | 0.267 | | 0.05800735 |
| rs4830958 | 0.0378606 | 0.0441741 | X | | 15414485 | Xp22.2 | 29.6298 | 0.45 | PIR | 0.058178783 |
| rs1361117 | 0.0470278 | 0.0343322 | | 9 | 8605045 | 9p24.1 | 18.9776 | 0.092 | PTPRD | 0.05822639 |
| rs2169385 | 0.0181384 | 0.0556027 | | 8 | 9244088 | 8p23.1 | 22.2554 | 0.142 | | 0.058486454 |
| rs4456603 | 0.0562474 | 0.0166361 | | 18 | 47064585 | 18q21.1 | 72.9724 | 0.075 | | 0.058655993 |
| rs4544324 | 0.0562474 | 0.0166361 | | 18 | 47066251 | 18q21.1 | 72.9745 | 0.058 | | 0.058655993 |
| rs459131 | 0.0515444 | 0.0283225 | | 5 | 55760970 | 5q11.2 | 71.3864 | 0.076 | | 0.058813176 |
| rs172310 | 0.0580779 | 0.0102941 | | 7 | 155308388 | 7q36.3 | 179.8675 | 0.345 | | 0.058983183 |
| rs12556578 | 0.0434237 | 0.0401583 | X | | 62649814 | Xq11.2 | 79.8044 | 0.058 | | 0.059146484 |

rch:tme:12/6/2011

| | | | | | | | | | | |
|---|---|---|---|---|---|---|---|---|---|---|
| rs1904031 | 0.0276496 | 0.0524899 | | 10 | 53502908 | 10q21.1 | 70.9846 | 0.308 | PRKG1 | 0.059326987 |
| rs6070373 | 0.0306667 | 0.0510003 | | 20 | 56183183 | 20q13.32 | 95.4034 | 0.342 | | 0.059510342 |
| rs728864 | 0.0282535 | 0.0524899 | | 1 | 48302845 | 1p33 | 71.4555 | 0.358 | | 0.059610846 |
| rs8079174 | 0.0589381 | 0.0093893 | | 17 | 10782407 | 17p12 | 31.2214 | 0.246 | | 0.059681326 |
| rs2303164 | 0.0264309 | 0.0535807 | | 19 | 8028737 | 19p13.2 | 25.5915 | 0.333 | CCL25 | 0.059745179 |
| rs906528 | 0.0590952 | 0.0091591 | | 11 | 94432038 | 11q21 | 96.12 | 0.133 | | 0.05980073 |
| rs7275311 | 0.0541055 | 0.0263965 | | 21 | 22188054 | 21q21.1 | 21.2518 | 0.317 | | 0.060201149 |
| rs1991624 | 0.0591983 | 0.0111267 | | 9 | 35564609 | 9p13.3 | 57.4086 | 0.067 | | 0.060234923 |
| rs17441859 | 0.0518784 | 0.0308232 | | 15 | 91695145 | 15q26.1 | 105.5552 | 0.258 | | 0.060344329 |
| rs6477107 | 0.0507010 | 0.0329077 | | 9 | 6750919 | 9p24.1 | 14.1815 | 0.308 | JMJD2C | 0.060444269 |
| rs1347001 | 0.0033777 | 0.0605166 | | 8 | 129783808 | 8q24.21 | 138.4241 | 0.217 | | 0.060610816 |
| rs10006010 | 0.0509354 | 0.0329077 | | 4 | 30935917 | 4p15.1 | 51.1828 | 0.283 | | 0.060641002 |
| rs7691894 | 0.0328683 | 0.0510003 | | 4 | 167900239 | 4q32.3 | 161.6307 | 0.333 | SPOCK3 | 0.060674191 |
| rs7099403 | 0.0518311 | 0.0329077 | | 10 | 21510807 | 10p12.31 | 45.806 | 0.305 | | 0.06139526 |
| rs1215765 | 0.0261625 | 0.0556027 | | 12 | 105132584 | 12q23.3 | 119.7979 | 0.183 | | 0.061450317 |
| rs2321733 | 0.0613143 | 0.0048136 | | 2 | 134778532 | 2q21.2 | 148.3744 | 0.233 | MGAT5 | 0.061502941 |
| rs1233258 | 0.0542193 | 0.0292182 | | 2 | 190415279 | 2q32.2 | 190.5398 | 0.292 | PMS1 | 0.061590877 |
| rs7859758 | 0.0386543 | 0.0479910 | | 9 | 105427140 | 9q31.1 | 106.1277 | 0.195 | | 0.061622102 |
| rs4830487 | 0.0347519 | 0.0510003 | X | | 13040405 | Xp22.2 | 25.2251 | 0.292 | | 0.061714879 |
| rs7379403 | 0.0524580 | 0.0329077 | | 5 | 21942398 | 5p14.3 | 41.3675 | 0.2 | CDH12 | 0.061925455 |
| rs9686533 | 0.0618854 | 0.0023703 | | 5 | 120983392 | 5q23.1 | 125.8968 | 0.373 | | 0.061930729 |
| rs802682 | 0.0132804 | 0.0605166 | | 6 | 111227881 | 6q21 | 115.1953 | 0.136 | CDC2L6 | 0.061956679 |
| rs1458371 | 0.0143400 | 0.0605166 | X | | 31903594 | Xp21.1 | 48.2847 | 0.425 | DMD | 0.062192429 |
| rs1984007 | 0.0441560 | 0.0441741 | | 9 | 27319622 | 9p21.2 | 50.0776 | 0.383 | MOBKL2B | 0.06245879 |
| rs9301376 | 0.0532804 | 0.0329077 | | 13 | 108821974 | 13q33.3 | 115.706 | 0.325 | | 0.062623625 |
| rs7350983 | 0.0558242 | 0.0290788 | | 18 | 35222815 | 18q12.2 | 60.0113 | 0.092 | | 0.06294377 |
| rs9386934 | 0.0176588 | 0.0605166 | | 6 | 111096355 | 6q21 | 115.0636 | 0.136 | CDC2L6 | 0.063040419 |
| rs1474056 | 0.0560671 | 0.0290788 | | 11 | 47589283 | 11p11.2 | 63.81 | 0.108 | | 0.063159313 |

*rch:tme:12/6/2011*

| | | | | | | | | | | |
|---|---|---|---|---|---|---|---|---|---|---|
| rs7891488 | 0.0633827 | 0.0000000 | X | | 120987113 | Xq25 | 122.4197 | 0.083 | | 0.06338267 |
| rs9817739 | 0.0355704 | 0.0524899 | | 3 | 16455831 | 3p24.3 | 36.6198 | 0.233 | RFTN1 | 0.063407002 |
| rs1954920 | 0.0579623 | 0.0259136 | | 6 | 162478417 | 6q26 | 173.0741 | 0.342 | PARK2 | 0.063491266 |
| rs1361206 | 0.0564093 | 0.0292182 | | 13 | 67570061 | 13q21.33 | 61.8468 | 0.3 | | 0.063527241 |
| rs10139234 | 0.0623933 | 0.0119856 | | 14 | 70193313 | 14q24.2 | 69.2673 | 0.108 | TTC9 | 0.06353406 |
| rs7304711 | 0.0403865 | 0.0490637 | | 12 | 47312325 | 12q13.11 | 62.9201 | 0.3 | | 0.063547799 |
| rs7529979 | 0.0196441 | 0.0605166 | | 1 | 117070434 | 1p13.1 | 138.9147 | 0.108 | | 0.063625094 |
| rs11791472 | 0.0638310 | 0.0048136 | | 9 | 4041387 | 9p24.2 | 8.1059 | 0.259 | GLIS3 | 0.06401223 |
| rs7192812 | 0.0366606 | 0.0524899 | | 16 | 49060881 | 16q12.1 | 60.2139 | 0.15 | | 0.064024916 |
| rs12666315 | 0.0001912 | 0.0646321 | | 7 | 149019972 | 7q36.1 | 161.2987 | 0.075 | | 0.064632428 |
| rs6974082 | 0.0002746 | 0.0646321 | | 7 | 149025379 | 7q36.1 | 161.3015 | - | | 0.064632728 |
| rs814528 | 0.0532355 | 0.0368363 | | 19 | 45706762 | 19q13.2 | 66.5611 | 0.142 | SPTBN4 | 0.064737431 |
| rs11624431 | 0.0558706 | 0.0329077 | | 14 | 27490931 | 14q12 | 24.3589 | 0.207 | | 0.064841678 |
| rs2305593 | 0.0411673 | 0.0510003 | | 4 | 167912210 | 4q32.3 | 161.6396 | 0.333 | SPOCK3 | 0.065542239 |
| rs16903629 | 0.0518815 | 0.0402216 | | 5 | 2249941 | 5p15.33 | 5.1496 | 0.067 | | 0.065646516 |
| rs1439098 | 0.0000808 | 0.0657887 | | 7 | 149008444 | 7q36.1 | 161.2927 | 0.075 | | 0.065788779 |
| rs17679624 | 0.0017067 | 0.0657887 | | 6 | 123688113 | 6q22.31 | 122.9915 | 0.059 | TRDN | 0.065810863 |
| rs2796460 | 0.0657842 | 0.0042898 | | 9 | 83391414 | 9q21.32 | 79.6374 | 0.05 | TLE1 | 0.065923885 |
| rs11759651 | 0.0264474 | 0.0605166 | | 6 | 14729332 | 6p23 | 32.8894 | 0.155 | | 0.066043362 |
| rs4889180 | 0.0137197 | 0.0646321 | | 16 | 79228642 | 16q23.2 | 102.3632 | 0.125 | CDYL2 | 0.066072258 |
| rs2411976 | 0.0655492 | 0.0093893 | X | | 78270514 | Xq21.1 | 86.7196 | 0.45 | | 0.06621825 |
| rs2411975 | 0.0655492 | 0.0093893 | X | | 78281726 | Xq21.1 | 86.7241 | 0.45 | | 0.06621825 |
| rs4785755 | 0.0422889 | 0.0510003 | | 16 | 88565329 | 16q24.3 | 130.7529 | 0.246 | AFG3L1,MGC 16385 | 0.066252409 |
| rs2812152 | 0.0639435 | 0.0174555 | | 6 | 67200623 | 6q12 | 82.2739 | 0.267 | | 0.066283191 |
| rs11957867 | 0.0088728 | 0.0657310 | | 5 | 128774826 | 5q23.3 | 132.9052 | 0.102 | | 0.066327158 |
| rs4835490 | 0.0613767 | 0.0259136 | | 4 | 149309776 | 4q31.23 | 144.3315 | 0.208 | NR3C2 | 0.066622914 |
| rs1994090 | 0.0660585 | 0.0086947 | | 12 | 38714828 | 12q12 | 56.4264 | 0.183 | SLC2A13 | 0.06662823 |

*rch:tme:12/6/2011*

| | | | | | | | | | | |
|---|---|---|---|---|---|---|---|---|---|---|
| rs10510564 | 0.0604730 | 0.0283225 | | 3 | 25358896 | 3p24.2 | 48.0237 | 0.058 | | 0.066776898 |
| rs12792262 | 0.0117890 | 0.0657310 | | 11 | 115736182 | 11q23.3 | 116.7656 | 0.108 | | 0.066779842 |
| rs6712744 | 0.0535249 | 0.0401583 | | 2 | 37030762 | 2p22.2 | 61.4511 | 0.092 | STRN | 0.06691496 |
| rs1261795 | 0.0582715 | 0.0329077 | | 14 | 20575468 | 14q11.2 | 9.5118 | 0.331 | | 0.066921473 |
| rs10242397 | 0.0652665 | 0.0153430 | | 7 | 8364897 | 7p21.3 | 16.5406 | 0.158 | | 0.067045648 |
| rs4889176 | 0.0133363 | 0.0657887 | | 16 | 79221772 | 16q23.2 | 102.3445 | 0.127 | CDYL2 | 0.067126856 |
| rs11871449 | 0.0293441 | 0.0605166 | | 17 | 2753838 | 17p13.3 | 7.7083 | 0.15 | GARNL4 | 0.067255752 |
| rs10442399 | 0.0145189 | 0.0657310 | | | | Xq21.2 | 87.8312 | 0.308 | | 0.067315412 |
| rs7946766 | 0.0667224 | 0.0093867 | | 11 | 47960945 | 11p11.2 | 63.81 | 0.108 | PTPRJ | 0.067379452 |
| rs10496220 | 0.0192322 | 0.0646321 | | 2 | 79402239 | 2p12 | 103.5878 | 0.144 | | 0.067432854 |
| rs2378013 | 0.0160457 | 0.0657887 | | 1 | 217018087 | 1q41 | 224.7124 | 0.075 | | 0.067717217 |
| rs7972005 | 0.0653587 | 0.0180694 | | 12 | 23616972 | 12p12.1 | 43.3217 | 0.3 | SOX5 | 0.067810523 |
| rs6989793 | 0.0661780 | 0.0153430 | | 8 | 9782060 | 8p23.1 | 22.4566 | 0.225 | | 0.067933321 |
| rs7877387 | 0.0680083 | 0.0000000 | X | | 136070109 | Xq26.3 | 140.0332 | 0.092 | | 0.06800826 |
| rs11165877 | 0.0596402 | 0.0329077 | | 1 | 97705803 | 1p21.3 | 119.6804 | 0.242 | DPYD | 0.06811662 |
| rs924417 | 0.0314872 | 0.0605166 | | 12 | 123174017 | 12q24.31 | 142.0176 | 0.282 | | 0.068218054 |
| rs11201011 | 0.0534833 | 0.0423829 | | 10 | 81729900 | 10q22.3 | 100.7251 | 0.475 | | 0.068240546 |
| rs11661310 | 0.0553153 | 0.0401583 | | 18 | 27781882 | 18q12.1 | 54.6606 | 0.133 | | 0.06835551 |
| rs9872799 | 0.0553182 | 0.0401583 | | 3 | 185911597 | 3q27.1 | 190.9069 | 0.277 | MAGEF1 | 0.068357827 |
| rs13091270 | 0.0618102 | 0.0293080 | | 3 | 119901638 | 3q13.32 | 126.4319 | 0.217 | | 0.068406547 |
| rs1018368 | 0.0190270 | 0.0657310 | X | | 85081287 | Xq21.2 | 87.8292 | 0.308 | CHM | 0.06842948 |
| rs7338552 | 0.0627319 | 0.0283225 | | 13 | 42193434 | 13q14.11 | 44.38 | 0.075 | | 0.068829168 |
| rs10797444 | 0.0583379 | 0.0368363 | | 1 | 231398970 | 1q42.2 | 238.7685 | 0.192 | PCNXL2 | 0.068994398 |
| rs296277 | 0.0213356 | 0.0657310 | | 5 | 106158099 | 5q21.3 | 113.3559 | 0.127 | | 0.06910696 |
| rs2380906 | 0.0436779 | 0.0535807 | | 9 | 3872949 | 9p24.2 | 8.0023 | 0.25 | GLIS3 | 0.069127784 |
| rs5925077 | 0.0138097 | 0.0677760 | X | | 150893453 | Xq28 | 182.0583 | 0.45 | GABRE | 0.069168586 |
| rs10517901 | 0.0467365 | 0.0510003 | | 4 | 167911480 | 4q32.3 | 161.6391 | 0.333 | SPOCK3 | 0.069176098 |
| rs2300719 | 0.0337372 | 0.0605166 | | 12 | 22265115 | 12p12.1 | 41.9655 | 0.208 | ST8SIA1 | 0.069285355 |

*rch:tme:12/6/2011*

| | | | | | | | | | | |
|---|---|---|---|---|---|---|---|---|---|---|
| rs10085952 | 0.0643344 | 0.0263965 | | 8 | 8543911 | 8p23.1 | 21.0498 | 0.242 | | 0.069539141 |
| rs579687 | 0.0506984 | 0.0479910 | | 11 | 35586070 | 11p13 | 52.3449 | 0.241 | | 0.069810158 |
| rs11823 | 0.0466339 | 0.0524899 | | 16 | 26987807 | 16p12.1 | 53.1496 | 0.195 | TNT | 0.070213356 |
| rs11198846 | 0.0515143 | 0.0479910 | | 10 | 121003407 | 10q26.11 | 141.8463 | 0.175 | GRK5 | 0.070404918 |
| rs2961430 | 0.0706422 | 0.0000000 | X | | 39514973 | Xp11.4 | 61.3757 | 0.042 | | 0.07064221 |
| rs7010127 | 0.0683410 | 0.0180694 | | 8 | 3115425 | 8p23.2 | 5.5665 | 0.275 | CSMD1 | 0.070689421 |
| rs588067 | 0.0021168 | 0.0708063 | | 6 | 168056666 | 6q27 | 185.4554 | 0.085 | MLLT4 | 0.070837934 |
| rs997607 | 0.0583855 | 0.0402216 | | 6 | 132475753 | 6q23.2 | 131.167 | 0.093 | | 0.070898767 |
| rs496486 | 0.0632583 | 0.0320688 | | 3 | 108708626 | 3q13.12 | 117.4245 | 0.1 | | 0.070922626 |
| rs2717351 | 0.0648984 | 0.0293080 | | 7 | 18986405 | 7p21.1 | 33.0151 | 0.242 | HDAC9 | 0.071209281 |
| rs2210 | 0.0444942 | 0.0556027 | | 16 | 79032118 | 16q23.2 | 101.8289 | 0.125 | LOC729847 | 0.07121372 |
| rs5745066 | 0.0077915 | 0.0708063 | | 12 | 131712889 | 12q24.33 | 171.1252 | - | POLE | 0.071233699 |
| rs4621704 | 0.0633222 | 0.0329077 | | 7 | 32185260 | 7p14.3 | 52.4732 | 0.291 | | 0.071362624 |
| rs977103 | 0.0695067 | 0.0166361 | | 3 | 67456931 | 3p14.1 | 92.7869 | 0.142 | | 0.07146989 |
| rs4608697 | 0.0664496 | 0.0263965 | | 3 | 182494295 | 3q26.33 | 185.7468 | 0.317 | | 0.071500506 |
| rs2298752 | 0.0673538 | 0.0240092 | | 4 | 103396094 | 4q24 | 107.3223 | 0.17 | | 0.071505082 |
| rs660075 | 0.0639611 | 0.0320688 | | 3 | 108705164 | 3q13.12 | 117.4225 | 0.1 | | 0.071550202 |
| rs939876 | 0.0653751 | 0.0293080 | | 12 | 63538385 | 12q14.3 | 78.373 | 0.092 | KIAA0984 | 0.071644022 |
| rs2839775 | 0.0002796 | 0.0717456 | | 12 | 40400352 | 12q12 | 57.3687 | 0.267 | | 0.071746097 |
| rs287024 | 0.0022957 | 0.0717456 | | 12 | 40343726 | 12q12 | 57.3402 | 0.258 | | 0.071782272 |
| rs397496 | 0.0022957 | 0.0717456 | | 12 | 40364037 | 12q12 | 57.3504 | 0.258 | | 0.071782272 |
| rs1002016 | 0.0611731 | 0.0376389 | | 2 | 111333383 | 2q13 | 123.4395 | 0.15 | ACOXL | 0.071825002 |
| rs1833044 | 0.0648904 | 0.0308232 | | 10 | 85645422 | 10q23.1 | 105.2982 | 0.308 | | 0.071838894 |
| rs287016 | 0.0040593 | 0.0717456 | | 12 | 40331205 | 12q12 | 57.3339 | 0.25 | | 0.071860298 |
| rs10773594 | 0.0649531 | 0.0308232 | | 12 | 128125510 | 12q24.32 | 160.3426 | 0.358 | TMEM132D | 0.07189554 |
| rs7754676 | 0.0239967 | 0.0677760 | | 6 | 67166881 | 6q12 | 82.1857 | 0.172 | | 0.071898736 |
| rs2000327 | 0.0599163 | 0.0401583 | | 14 | 27205574 | 14q12 | 24.2979 | 0.083 | LOC728755 | 0.07212947 |
| rs9424099 | 0.0717866 | 0.0072224 | | 10 | 10893354 | 10p14 | 27.79 | 0.182 | | 0.072148968 |

*rch:tme:12/6/2011*

| | | | | | | | | | | |
|---|---|---|---|---|---|---|---|---|---|---|
| rs4083221 | 0.0716215 | 0.0093893 | | 12 | 77820855 | 12q21.2 | 92.9249 | 0.283 | | 0.072234289 |
| rs1439376 | 0.0251049 | 0.0677760 | | 4 | 44288455 | 4p13 | 65.0348 | 0.192 | | 0.072276156 |
| rs10238918 | 0.0540750 | 0.0479910 | | 7 | 31343894 | 7p14.3 | 51.1217 | 0.3 | NEUROD6 | 0.072299618 |
| rs204740 | 0.0705128 | 0.0159800 | | 21 | 32056224 | 21q22.11 | 33.8308 | 0.15 | | 0.072300858 |
| rs10496340 | 0.0664908 | 0.0293080 | | 2 | 99758741 | 2q11.2 | 114.2971 | 0.192 | AFF3 | 0.072663479 |
| rs2048646 | 0.0119824 | 0.0717456 | | 5 | 34784322 | 5p13.2 | 56.7653 | 0.25 | RAI14 | 0.072739282 |
| rs1075493 | 0.0520510 | 0.0510003 | | 8 | 140986639 | 8q24.3 | 156.9688 | 0.2 | NIBP | 0.072872061 |
| rs628873 | 0.0729058 | 0.0042898 | | 9 | 27145544 | 9p21.2 | 49.5691 | 0.083 | TEK | 0.073031921 |
| rs4699128 | 0.0182563 | 0.0708063 | | 4 | 105642314 | 4q24 | 109.14 | 0.052 | | 0.073121971 |
| rs1910534 | 0.0279022 | 0.0677760 | | 10 | 53580527 | 10q21.1 | 71.0461 | 0.217 | PRKG1 | 0.073294745 |
| rs544704 | 0.0526924 | 0.0510003 | | 3 | 55455763 | 3p14.3 | 76.198 | 0.258 | | 0.073331566 |
| rs11750519 | 0.0571689 | 0.0460523 | | 5 | 156804994 | 5q33.3 | 161.5967 | 0.208 | | 0.073410419 |
| rs1156793 | 0.0599979 | 0.0423829 | | 9 | 8368662 | 9p24.1 | 18.4694 | 0.258 | PTPRD | 0.073457857 |
| rs904075 | 0.0067487 | 0.0731997 | | 12 | 40475362 | 12q12 | 57.4065 | 0.083 | | 0.073510161 |
| rs10861034 | 0.0547646 | 0.0490637 | | 12 | 77781600 | 12q21.2 | 92.8737 | 0.2 | | 0.073528282 |
| rs9876789 | 0.0330358 | 0.0657887 | | 3 | 74628570 | 3p12.3 | 103.014 | 0.092 | CNTN3 | 0.073617382 |
| rs6832047 | 0.0709557 | 0.0196513 | | 4 | 36002500 | 4p14 | 54.7093 | 0.362 | FLJ16686 | 0.07362666 |
| rs4752791 | 0.0678701 | 0.0290788 | | 11 | 47692599 | 11p11.2 | 63.81 | 0.108 | AGBL2 | 0.073837131 |
| rs2242223 | 0.0179506 | 0.0717456 | | 5 | 121789360 | 5q23.2 | 126.7942 | 0.217 | SNCAIP | 0.073957081 |
| rs10506676 | 0.0746889 | 0.0005818 | | 12 | 73754517 | 12q21.1 | 87.6509 | 0.133 | KCNC2 | 0.074691177 |
| rs1559931 | 0.0687521 | 0.0292182 | | 2 | 204533974 | 2q33.2 | 200.8478 | 0.275 | ICOS | 0.074703093 |
| rs10938745 | 0.0696733 | 0.0273971 | | 4 | 8765612 | 4p16.1 | 21.7137 | 0.133 | | 0.074866371 |
| rs7882590 | 0.0246048 | 0.0708063 | X | | 22027469 | Xp22.11 | 36.4597 | 0.067 | PHEX | 0.074959511 |
| rs7596894 | 0.0219964 | 0.0717456 | | 2 | 164306063 | 2q24.3 | 170.3163 | 0.342 | | 0.07504175 |
| rs2270151 | 0.0323124 | 0.0677760 | | 12 | 5931221 | 12p13.31 | 16.35 | 0.133 | VWF | 0.075084449 |
| rs14312 | 0.0749636 | 0.0087695 | X | | 46837342 | Xp11.3 | 73.1685 | 0.092 | RGN | 0.075474752 |
| rs6538408 | 0.0372110 | 0.0657310 | | 12 | 92237010 | 12q22 | 103.4957 | 0.117 | LOC643339 | 0.075532949 |
| rs7146722 | 0.0752491 | 0.0075075 | | 14 | 46978533 | 14q21.3 | 45.42 | 0.267 | | 0.075622628 |

*rch:tme:12/6/2011*

| | | | | | | | | | | |
|---|---|---|---|---|---|---|---|---|---|---|
| rs9790702 | 0.0028138 | 0.0756830 | | 4 | 155110806 | 4q31.3 | 149.5488 | 0.158 | | 0.075735287 |
| rs5958298 | 0.0197104 | 0.0731997 | X | | 122734607 | Xq25 | 123.8898 | 0.067 | | 0.075806974 |
| rs1547531 | 0.0590648 | 0.0479910 | | 5 | 30832148 | 5p13.3 | 50.0172 | 0.254 | | 0.076103742 |
| rs6716246 | 0.0755596 | 0.0102941 | | 2 | 435636 | 2p25.3 | 2.2764 | 0.225 | | 0.076257553 |
| rs1540613 | 0.0096298 | 0.0756830 | | 16 | 79033683 | 16q23.2 | 101.8332 | 0.1 | LOC729847 | 0.076293176 |
| rs9845033 | 0.0718876 | 0.0259136 | | 3 | 34674131 | 3p23 | 60.1066 | 0.203 | | 0.076415538 |
| rs10982585 | 0.0108997 | 0.0756830 | | 9 | 116963649 | 9q33.1 | 121.62 | 0.108 | DEC1 | 0.076463844 |
| rs792841 | 0.0016401 | 0.0764873 | | 3 | 100953998 | 3q12.1 | 111.82 | 0.358 | COL8A1 | 0.076504919 |
| rs11726463 | 0.0266216 | 0.0717456 | | 4 | 139407809 | 4q28.3 | 134.6187 | 0.288 | | 0.076525394 |
| rs11180765 | 0.0714671 | 0.0273971 | | 12 | 74630873 | 12q21.2 | 88.4909 | 0.225 | | 0.076538541 |
| rs1893259 | 0.0291160 | 0.0708063 | | 18 | 23964092 | 18q12.1 | 51.423 | 0.067 | CDH2 | 0.076558951 |
| rs1542827 | 0.0707477 | 0.0293080 | | 8 | 20173745 | 8p21.3 | 34.6398 | 0.208 | | 0.076578055 |
| rs6759510 | 0.0055211 | 0.0764873 | | 2 | 165576723 | 2q24.3 | 170.5763 | 0.2 | | 0.076686344 |
| rs7220132 | 0.0748580 | 0.0168257 | | 17 | 17345311 | 17p11.2 | 47.4401 | 0.267 | | 0.076725616 |
| rs4445834 | 0.0686198 | 0.0343322 | | 14 | 89451340 | 14q32.11 | 91.3802 | 0.102 | C14orf143 | 0.076729187 |
| rs3822030 | 0.0111464 | 0.0759202 | | 4 | 977343 | 4p16.3 | 1.2447 | 0.367 | IDUA,SLC26A 1 | 0.076734096 |
| rs246486 | 0.0074582 | 0.0764873 | | 5 | 150708073 | 5q33.1 | 156.7916 | 0.331 | | 0.076850094 |
| rs5967664 | 0.0398448 | 0.0657310 | X | | 85137019 | Xq21.2 | 87.8495 | 0.308 | CHM | 0.076864623 |
| rs1896284 | 0.0397803 | 0.0657887 | | 2 | 204641823 | 2q33.2 | 200.9101 | 0.075 | | 0.076880633 |
| rs10504794 | 0.0080529 | 0.0764873 | | 8 | 85312138 | 8q21.2 | 93.9587 | 0.442 | LOC138046 | 0.076910084 |
| rs6578748 | 0.0144492 | 0.0756830 | | 11 | 1688586 | 11p15.5 | 1.1959 | 0.125 | | 0.077049948 |
| rs10513805 | 0.0762575 | 0.0111267 | | 3 | 188433091 | 3q27.3 | 197.2406 | 0.058 | MASP1 | 0.077065012 |
| rs553653 | 0.0769164 | 0.0048136 | | 9 | 4104353 | 9p24.2 | 8.1096 | 0.258 | GLIS3 | 0.077066824 |
| rs1424104 | 0.0577914 | 0.0510003 | | 16 | 77704426 | 16q23.1 | 98.7792 | 0.35 | | 0.077077109 |
| rs6467917 | 0.0095691 | 0.0764873 | | 7 | 82407593 | 7q21.11 | 96.0445 | 0.483 | PCLO | 0.07708359 |
| rs10416550 | 0.0690801 | 0.0343322 | | 19 | 51646102 | 19q13.32 | 74.5297 | 0.175 | LOC729474 | 0.077141108 |
| rs12618382 | 0.0604248 | 0.0479910 | | 2 | 231225449 | 2q37.1 | 236.8282 | 0.233 | | 0.077164057 |

*rch:tme:12/6/2011*

| | | | | | | | | | | |
|---|---|---|---|---|---|---|---|---|---|---|
| rs1282540 | 0.0703106 | 0.0320688 | | 3 | 108679286 | 3q13.12 | 117.4078 | 0.1 | | 0.077278655 |
| rs2570817 | 0.0147044 | 0.0759202 | | 15 | 98017244 | 15q26.3 | 128.718 | 0.267 | MEF2A | 0.077331106 |
| rs1350515 | 0.0773527 | 0.0005818 | | 12 | 73744750 | 12q21.1 | 87.6447 | 0.133 | KCNC2 | 0.077354898 |
| rs1503161 | 0.0755110 | 0.0168257 | | 3 | 106288374 | 3q13.11 | 114.8398 | 0.342 | | 0.077362847 |
| rs4719155 | 0.0754736 | 0.0180694 | | 7 | 70745160 | 7q11.22 | 83.5033 | 0.267 | WBSCR17 | 0.077606467 |
| rs10246707 | 0.0167772 | 0.0759202 | | 7 | 129275389 | 7q32.2 | 130.8093 | 0.15 | UBE2H | 0.077751875 |
| rs220599 | 0.0603211 | 0.0490637 | | 12 | 13866565 | 12p13.1 | 31.3181 | 0.4 | GRIN2B | 0.077755316 |
| rs3917289 | 0.0266758 | 0.0731997 | | 2 | 102148343 | 2q11.2 | 116.13 | 0.083 | IL1R1 | 0.077908894 |
| rs10853232 | 0.0151982 | 0.0764873 | | 18 | 13373207 | 18p11.21 | 39.8659 | 0.358 | C18orf1 | 0.077982672 |
| rs7833003 | 0.0568862 | 0.0535807 | | 8 | 107445453 | 8q23.1 | 113.1821 | 0.442 | | 0.078146821 |
| rs11243406 | 0.0729115 | 0.0283225 | | 9 | 133380691 | 9q34.13 | 141.9398 | 0.075 | POMT1 | 0.078219249 |
| rs2274808 | 0.0164636 | 0.0764873 | | 21 | 45731055 | 21q22.3 | 72.5519 | 0.212 | COL18A1 | 0.07823913 |
| rs717406 | 0.0198723 | 0.0756830 | | 8 | 1073568 | 8p23.3 | 1.0509 | 0.083 | C8orf68 | 0.078248476 |
| rs10491495 | 0.0396767 | 0.0677760 | | 5 | 106283657 | 5q21.3 | 113.4619 | 0.242 | | 0.078535517 |
| rs2143844 | 0.0574300 | 0.0535807 | X | | 33039414 | Xp21.1 | 50.7144 | 0.45 | DMD | 0.078543563 |
| rs6540246 | 0.0397311 | 0.0677760 | | 16 | 84611335 | 16q24.1 | 121.3514 | 0.15 | | 0.078562994 |
| rs814620 | 0.0187240 | 0.0764873 | | 10 | 90412116 | 10q23.31 | 108.6242 | 0.2 | | 0.078745787 |
| rs2481627 | 0.0779736 | 0.0119856 | | 1 | 174358790 | 1q25.2 | 177.8078 | 0.119 | RFWD2 | 0.078889423 |
| rs2072175 | 0.0193440 | 0.0764873 | | 7 | 29920438 | 7p15.1 | 48.0807 | 0.164 | | 0.078895519 |
| rs7957728 | 0.0579116 | 0.0535807 | | 12 | 92809687 | 12q22 | 104.1195 | 0.35 | | 0.078896393 |
| rs9925917 | 0.0559801 | 0.0556027 | | 16 | 14006322 | 16p13.12 | 33.5459 | 0.125 | | 0.078901407 |
| rs2424455 | 0.0329543 | 0.0717456 | | 20 | 22591603 | 20p11.21 | 52.261 | 0.083 | | 0.078951961 |
| rs383216 | 0.0411161 | 0.0677760 | | 19 | 61161808 | 19q13.43 | 106.9241 | 0.4 | NLRP8 | 0.079272409 |
| rs3012519 | 0.0305107 | 0.0731997 | | 6 | 74593230 | 6q13 | 87.8759 | 0.1 | | 0.07930387 |
| rs7625409 | 0.0702306 | 0.0368363 | | 3 | 108659068 | 3q13.12 | 117.3963 | 0.102 | | 0.079304802 |
| rs6442164 | 0.0460807 | 0.0646321 | | 3 | 1071770 | 3p26.3 | 2.3206 | 0.05 | | 0.079377218 |
| rs16947192 | 0.0462163 | 0.0646321 | | 16 | 76744881 | 16q23.1 | 95.4954 | 0.092 | WWOX | 0.079456023 |
| rs5988003 | 0.0652700 | 0.0460523 | X | | 114855002 | Xq23 | 114.85 | 0.092 | | 0.079881054 |

*rch:tme:12/6/2011*

| | | | | | | | | | | |
|---|---|---|---|---|---|---|---|---|---|---|
| rs999449 | 0.0800253 | 0.0030565 | | 15 | 58776283 | 15q22.2 | 61.6429 | 0.267 | RORA | 0.080083679 |
| rs1481646 | 0.0607566 | 0.0524899 | | 8 | 26887222 | 8p21.2 | 46.42 | 0.292 | | 0.080290462 |
| rs7121901 | 0.0431279 | 0.0677760 | | 11 | 119329909 | 11q23.3 | 122.1219 | 0.125 | | 0.080334324 |
| rs2237432 | 0.0646148 | 0.0479910 | | 7 | 41701559 | 7p14.1 | 63.3951 | 0.225 | INHBA | 0.080487315 |
| rs8048495 | 0.0277956 | 0.0759202 | | 16 | 81780322 | 16q23.3 | 110.6085 | 0.242 | CDH13 | 0.080848486 |
| rs12784234 | 0.0606257 | 0.0535807 | | 10 | 5394261 | 10p15.1 | 16.6797 | 0.117 | | 0.080909646 |
| rs6981869 | 0.0393389 | 0.0708063 | | 8 | 30636038 | 8p12 | 52.12 | 0.058 | | 0.081000492 |
| rs3788329 | 0.0793502 | 0.0174555 | | 22 | 20358124 | 22q11.21 | 14.1161 | 0.325 | PPIL2 | 0.081247443 |
| rs205717 | 0.0292814 | 0.0759202 | | 7 | 130267489 | 7q32.3 | 132.9845 | 0.1 | | 0.081371254 |
| rs17635284 | 0.0546531 | 0.0605166 | | 5 | 168178679 | 5q34 | 176.5498 | 0.175 | SLIT3 | 0.081542747 |
| rs6817090 | 0.0810700 | 0.0103578 | | 4 | 156999620 | 4q32.1 | 150.8783 | 0.233 | ACCN5 | 0.081728963 |
| rs223198 | 0.0030131 | 0.0817005 | | 1 | 18449300 | 1p36.13 | 33.6192 | 0.233 | IGSF21 | 0.081756084 |
| rs630943 | 0.0041820 | 0.0817005 | | 13 | 109675047 | 13q34 | 118.0862 | 0.325 | COL4A1 | 0.081807504 |
| rs7903424 | 0.0811992 | 0.0102941 | | 10 | 114894026 | 10q25.2 | 130.3486 | 0.225 | TCF7L2 | 0.081849102 |
| rs10146784 | 0.0056860 | 0.0817005 | | 14 | 95292523 | 14q32.13 | 103.1694 | 0.283 | | 0.081898165 |
| rs1886505 | 0.0059645 | 0.0817005 | | 14 | 95291881 | 14q32.13 | 103.168 | 0.271 | | 0.081917973 |
| rs12653539 | 0.0314305 | 0.0756830 | | 5 | 98501952 | 5q21.1 | 110.6236 | 0.112 | | 0.08194993 |
| rs10746116 | 0.0745283 | 0.0343322 | | 12 | 107030826 | 12q23.3 | 122.5076 | 0.417 | | 0.082055887 |
| rs6501048 | 0.0014696 | 0.0820617 | | 16 | 7957785 | 16p13.2 | 21.2243 | 0.367 | | 0.082074854 |
| rs962052 | 0.0082876 | 0.0817005 | | 2 | 151352449 | 2q23.3 | 161.2706 | 0.317 | | 0.082119808 |
| rs11099852 | 0.0692315 | 0.0441741 | | 4 | 153904764 | 4q31.3 | 146.4263 | 0.446 | | 0.082123958 |
| rs5917614 | 0.0034157 | 0.0820617 | X | | 38400112 | Xp11.4 | 60.0831 | 0.492 | TSPAN7 | 0.082132751 |
| rs11764339 | 0.0822440 | 0.0040328 | | 7 | 46782172 | 7p13 | 69.1916 | 0.242 | | 0.082342839 |
| rs6073555 | 0.0706366 | 0.0423829 | | 20 | 42897945 | 20q13.12 | 69.4 | 0.208 | | 0.08237619 |
| rs2052482 | 0.0625948 | 0.0535807 | | 5 | 80318340 | 5q14.1 | 96.4776 | 0.283 | RASGRF2 | 0.082395377 |
| rs725399 | 0.0406038 | 0.0717456 | | 12 | 106699782 | 12q23.3 | 122.116 | 0.271 | | 0.082438439 |
| rs4344923 | 0.0110817 | 0.0817005 | | 2 | 207825985 | 2q33.3 | 204.8702 | 0.342 | LOC729607 | 0.082448672 |
| rs16868805 | 0.0707453 | 0.0423829 | | 6 | 62782774 | 6q11.1 | 80.56 | 0.217 | KHDRBS2 | 0.082469396 |

*rch:tme:12/6/2011*

| | | | | | | | | | | |
|---|---|---|---|---|---|---|---|---|---|---|
| rs8028632 | 0.0310034 | 0.0764873 | | 15 | 73108315 | 15q24.2 | 80.1691 | 0.158 | PPCDC | 0.082531942 |
| rs12298405 | 0.0088014 | 0.0820617 | | 12 | 16906534 | 12p12.3 | 35.32 | 0.317 | | 0.082532333 |
| rs12549803 | 0.0768191 | 0.0308232 | | 8 | 99751034 | 8q22.2 | 104.4945 | 0.2 | STK3 | 0.082772254 |
| rs10846448 | 0.0109086 | 0.0820617 | | 12 | 16920151 | 12p12.3 | 35.32 | 0.325 | | 0.082783564 |
| rs2588844 | 0.0828157 | 0.0005471 | | 18 | 35557196 | 18q12.3 | 60.0502 | 0.267 | | 0.082817507 |
| rs359980 | 0.0820673 | 0.0111267 | | 2 | 219537450 | 2q35 | 218.4817 | 0.075 | | 0.082818149 |
| rs1751280 | 0.0112955 | 0.0820617 | | 10 | 4459878 | 10p15.1 | 14.55 | 0.158 | | 0.082835446 |
| rs10753137 | 0.0769162 | 0.0308232 | | | | 1q25.2 | 178.47 | 0.314 | | 0.082862333 |
| rs12606960 | 0.0823137 | 0.0105786 | | 18 | 55471406 | 18q21.32 | 81.9128 | 0.092 | CCBE1 | 0.082990647 |
| rs12373663 | 0.0419696 | 0.0717456 | | 2 | 164300133 | 2q24.3 | 170.3159 | 0.333 | FIGN,LOC728304 | 0.083119622 |
| rs1004269 | 0.0346534 | 0.0759202 | | 20 | 2345656 | 20p13 | 8.4809 | 0.192 | TGM6 | 0.083455006 |
| rs2049197 | 0.0179504 | 0.0817005 | | 2 | 53484143 | 2p16.2 | 78.36 | 0.283 | | 0.083649235 |
| rs6046403 | 0.0578806 | 0.0605166 | | 20 | 19810973 | 20p11.23 | 48.614 | 0.15 | | 0.083740258 |
| rs2038193 | 0.0363033 | 0.0756830 | X | | 85174711 | Xq21.2 | 87.8632 | 0.308 | CHM | 0.083939514 |
| rs4653474 | 0.0839833 | 0.0026839 | | 1 | 225266723 | 1q42.13 | 230.6062 | 0.172 | CDC42BPA | 0.084026167 |
| rs3758740 | 0.0348814 | 0.0764873 | | 11 | 35599006 | 11p13 | 52.3649 | 0.242 | FJX1 | 0.08406561 |
| rs1950268 | 0.0777359 | 0.0320688 | | 14 | 96578041 | 14q32.2 | 106.2307 | 0.175 | | 0.08409089 |
| rs813328 | 0.0840605 | 0.0042898 | | 13 | 50143377 | 13q14.3 | 53.3386 | 0.058 | | 0.084169923 |
| rs916874 | 0.0825025 | 0.0168257 | | 4 | 114611842 | 4q26 | 118.2576 | 0.258 | CAMK2D | 0.084200735 |
| rs4935225 | 0.0121467 | 0.0834344 | | 10 | 52548404 | 10q11.23 | 70.2285 | 0.373 | PRKG1 | 0.084313931 |
| rs2353815 | 0.0354842 | 0.0764873 | | 7 | 11311673 | 7p21.3 | 22.1927 | 0.308 | | 0.08431748 |
| rs950864 | 0.0193891 | 0.0820617 | | 5 | 2720858 | 5p15.33 | 6.9382 | 0.288 | | 0.08432116 |
| rs6633902 | 0.0419642 | 0.0731997 | X | | 136356403 | Xq26.3 | 140.1937 | 0.083 | | 0.084375328 |
| rs4316308 | 0.0661476 | 0.0524899 | X | | 12628957 | Xp22.2 | 23.8733 | 0.425 | FRMPD4 | 0.084443496 |
| rs6429822 | 0.0817893 | 0.0218702 | | 1 | 14214934 | 1p36.21 | 24.6752 | 0.183 | | 0.084662865 |
| rs10415312 | 0.0847402 | 0.0014341 | | 19 | 14771438 | 19p13.12 | 36.6538 | 0.075 | OR7C1 | 0.084752355 |
| rs8113086 | 0.0090566 | 0.0843675 | | 19 | 61936028 | 19q13.43 | 109.2278 | 0.05 | | 0.084852254 |

*rch:tme:12/6/2011*

| | | | | | | | | | | |
|---|---|---|---|---|---|---|---|---|---|---|
| rs1506869 | 0.0063915 | 0.0846898 | | 8 | 25325019 | 8p21.2 | 43.74 | 0.206 | DOCK5 | 0.084930666 |
| rs4327974 | 0.0849042 | 0.0033371 | X | | 142520097 | Xq27.3 | 155.8269 | 0.144 | | 0.084969765 |
| rs5911158 | 0.0539672 | 0.0657310 | X | | 120469828 | Xq24 | 121.6887 | 0.2 | | 0.085047192 |
| rs7414734 | 0.0792691 | 0.0308232 | | 1 | 18967325 | 1p36.13 | 36.2545 | 0.383 | | 0.08505091 |
| rs4651286 | 0.0784579 | 0.0329077 | | 1 | 183942215 | 1q25.3 | 186.6587 | 0.292 | | 0.085079689 |
| rs12175530 | 0.0112633 | 0.0843675 | | 6 | 77357716 | 6q14.1 | 89.0299 | 0.085 | LOC643281 | 0.085116064 |
| rs17189726 | 0.0696284 | 0.0490637 | | 13 | 94929263 | 13q32.1 | 88.75 | 0.267 | CLDN10 | 0.085178403 |
| rs4699769 | 0.0231310 | 0.0820617 | | 4 | 101198193 | 4q23 | 106.0628 | 0.208 | | 0.085259399 |
| rs1391612 | 0.0752709 | 0.0402216 | | 11 | 5383694 | 11p15.4 | 8.58 | 0.108 | | 0.085343321 |
| rs12467276 | 0.0109042 | 0.0846898 | | 2 | 98681499 | 2q11.2 | 113.6216 | 0.375 | MGAT4A | 0.085388925 |
| rs7119152 | 0.0782943 | 0.0343322 | | 11 | 114926827 | 11q23.3 | 116.46 | 0.085 | | 0.08549092 |
| rs12468086 | 0.0122833 | 0.0846898 | | 2 | 98687006 | 2q11.2 | 113.625 | 0.367 | MGAT4A | 0.085575966 |
| rs180204 | 0.0257886 | 0.0817005 | | 8 | 133950426 | 8q24.22 | 143.5422 | 0.3 | TG | 0.085673977 |
| rs11035719 | 0.0563256 | 0.0646321 | | 11 | 40137148 | 11p12 | 57.0511 | 0.117 | LRRC4C | 0.08573151 |
| rs675136 | 0.0261678 | 0.0817005 | | 19 | 5466228 | 19p13.3 | 18.1299 | 0.292 | | 0.08578888 |
| rs1357337 | 0.0851007 | 0.0119856 | | 1 | 174197560 | 1q25.1 | 177.6442 | 0.121 | RFWD2 | 0.08594053 |
| rs2502826 | 0.0851007 | 0.0119856 | | 1 | 174310420 | 1q25.1 | 177.7587 | 0.117 | RFWD2 | 0.08594053 |
| rs6509196 | 0.0761740 | 0.0402216 | | 19 | 50315466 | 19q13.32 | 71.9594 | 0.059 | | 0.086140924 |
| rs4129443 | 0.0862344 | 0.0016218 | | 12 | 82310360 | 12q21.31 | 97.2374 | 0.053 | | 0.086249601 |
| rs601904 | 0.0409861 | 0.0759202 | | 11 | 73878387 | 11q13.4 | 80.7586 | 0.458 | LOC387787 | 0.0862771 |
| rs7744878 | 0.0752481 | 0.0423829 | | 6 | 132943213 | 6q23.2 | 131.6755 | 0.161 | | 0.086363094 |
| rs2337130 | 0.0687208 | 0.0524899 | | 5 | 167507516 | 5q34 | 175.1786 | 0.203 | ODZ2 | 0.086473917 |
| rs6452027 | 0.0823573 | 0.0263965 | | 5 | 21937473 | 5p14.3 | 41.3628 | 0.158 | CDH12 | 0.086484123 |
| rs2830400 | 0.0228009 | 0.0834344 | | 21 | 26986748 | 21q21.3 | 27.4147 | 0.233 | | 0.086493793 |
| rs10508050 | 0.0563531 | 0.0657887 | | 13 | 100362718 | 13q32.3 | 95.8517 | 0.083 | | 0.086624638 |
| rs3755862 | 0.0499523 | 0.0708063 | | 4 | 23425935 | 4p15.2 | 39.1598 | 0.083 | PPARGC1A | 0.086653105 |
| rs2217827 | 0.0701185 | 0.0510003 | | 8 | 64249656 | 8q12.3 | 75.2589 | 0.15 | YTHDF3 | 0.086704296 |
| rs7000981 | 0.0701185 | 0.0510003 | | 8 | 64249830 | 8q12.3 | 75.259 | 0.15 | YTHDF3 | 0.086704296 |

*rch:tme:12/6/2011*

| | | | | | | | | | | |
|---|---|---|---|---|---|---|---|---|---|---|
| rs6525877 | 0.0292867 | 0.0817005 | X | | 146966410 | Xq28 | 166.4619 | 0.467 | | 0.086791078 |
| rs7509408 | 0.0853917 | 0.0168257 | | | | 20p12.1 | 38.1754 | 0.233 | | 0.087033616 |
| rs4556627 | 0.0870395 | 0.0016218 | | 12 | 82316478 | 12q21.31 | 97.2415 | 0.059 | | 0.087054617 |
| rs935480 | 0.0416806 | 0.0764873 | | 2 | 134812772 | 2q21.3 | 148.4447 | 0.217 | MGAT5 | 0.087106745 |
| rs6824427 | 0.0417737 | 0.0764873 | | 4 | 59869351 | 4q13.1 | 74.6054 | 0.283 | | 0.087151319 |
| rs10755897 | 0.0815437 | 0.0308232 | | 8 | 99716627 | 8q22.2 | 104.4776 | 0.2 | STK3 | 0.0871748 |
| rs242003 | 0.0787994 | 0.0376389 | | 12 | 3677366 | 12p13.32 | 9.5678 | 0.167 | EFCAB4B | 0.087327137 |
| rs11024449 | 0.0697950 | 0.0524899 | | 11 | 18014461 | 11p15.1 | 27.4869 | 0.325 | TPH1 | 0.08733 |
| rs8041437 | 0.0859338 | 0.0159800 | | 15 | 48142896 | 15q21.2 | 48.973 | 0.092 | ATP8B4 | 0.087406922 |
| rs2133764 | 0.0813368 | 0.0320688 | | 5 | 30821797 | 5p13.3 | 49.999 | 0.212 | | 0.087430469 |
| rs2871647 | 0.0578344 | 0.0657310 | | 17 | 73649072 | 17q25.3 | 120.806 | 0.093 | TMC8 | 0.087552148 |
| rs413667 | 0.0305402 | 0.0820617 | | 8 | 55254461 | 8q11.23 | 67.4265 | 0.381 | | 0.087560426 |
| rs5026446 | 0.0719085 | 0.0500125 | | 18 | 74163927 | 18q23 | 118.0307 | 0.117 | | 0.087590446 |
| rs9520396 | 0.0437060 | 0.0759202 | | 13 | 106791742 | 13q33.3 | 110.2539 | 0.125 | LOC728215 | 0.087601894 |
| rs384526 | 0.0874977 | 0.0046126 | | 2 | 183882 | 2p25.3 | 1.95 | 0.212 | LOC727818 | 0.087619158 |
| rs7818404 | 0.0227013 | 0.0846898 | | 8 | 74587539 | 8q21.11 | 87.208 | 0.433 | | 0.087679611 |
| rs10823406 | 0.0310644 | 0.0820617 | | 10 | 70982029 | 10q22.1 | 86.454 | 0.2 | | 0.087744609 |
| rs708188 | 0.0704036 | 0.0524899 | | 12 | 28111983 | 12p11.22 | 50.7661 | 0.317 | | 0.087817208 |
| rs7905024 | 0.0871874 | 0.0111267 | | 10 | 98374137 | 10q24.1 | 116.59 | 0.06 | PIK3AP1 | 0.087894482 |
| rs4965238 | 0.0324498 | 0.0817005 | | 15 | 97814085 | 15q26.3 | 128.2525 | 0.276 | | 0.08790886 |
| rs1583443 | 0.0325845 | 0.0817005 | | 11 | 38296860 | 11p12 | 56.27 | 0.203 | | 0.087958678 |
| rs2242144 | 0.0319906 | 0.0820617 | | 11 | 30461197 | 11p14.1 | 46.3656 | 0.2 | MPPED2 | 0.088076791 |
| rs10945462 | 0.0562892 | 0.0677760 | | 6 | 168209692 | 6q27 | 185.7213 | 0.225 | FRMD1 | 0.088102537 |
| rs1378796 | 0.0586748 | 0.0657310 | | 3 | 158614482 | 3q25.32 | 164.6695 | 0.083 | VEPH1 | 0.08810955 |
| rs12861247 | 0.0881230 | 0.0014828 | X | | 7184199 | Xp22.31 | 14.1741 | 0.2 | STS | 0.088135437 |
| rs2240887 | 0.0000767 | 0.0882295 | | 19 | 18721919 | 19p13.11 | 45.2673 | 0.108 | CRTC1 | 0.088229538 |
| rs2393469 | 0.0879126 | 0.0075075 | | 10 | 60131328 | 10q21.1 | 75.949 | 0.175 | BICC1 | 0.088232604 |
| rs9876832 | 0.0802668 | 0.0368363 | | 3 | 104517956 | 3q13.11 | 113.9227 | 0.208 | | 0.088315759 |

*rch:tme:12/6/2011*

| | | | | | | | | | | | |
|---|---|---|---|---|---|---|---|---|---|---|---|
| rs746701 | 0.0054089 | 0.0882295 | | 2 | 180277440 | 2q31.2 | 185.7619 | | 0.175 | ZNF533 | 0.088395144 |
| rs1352516 | 0.0880310 | 0.0086947 | | 18 | 44151658 | 18q21.1 | 68.8822 | | 0.208 | | 0.088459343 |
| rs295999 | 0.0012934 | 0.0884963 | | 5 | 168458445 | 5q35.1 | 177.6059 | | 0.373 | SLIT3 | 0.08850573 |
| rs2107710 | 0.0078599 | 0.0882295 | | 7 | 39662398 | 7p14.1 | 61.0356 | | 0.342 | RALA | 0.088578913 |
| rs3026720 | 0.0866725 | 0.0184575 | | 10 | 42883860 | 10q11.21 | 64.2226 | | 0.183 | | 0.088616073 |
| rs6989593 | 0.0052790 | 0.0884963 | | 8 | 47670421 | 8q11.1 | 61.9541 | | 0.342 | | 0.088653589 |
| rs370850 | 0.0848586 | 0.0259136 | | 21 | 27137937 | 21q21.3 | 27.829 | - | | ADAMTS1 | 0.088727052 |
| rs541821 | 0.0071725 | 0.0884963 | | 11 | 94801223 | 11q21 | 96.7175 | | 0.308 | | 0.088786463 |
| rs1997630 | 0.0270521 | 0.0846898 | X | | 128189053 | Xq25 | 131.2294 | | 0.4 | | 0.088905486 |
| rs10465632 | 0.0525958 | 0.0717456 | | 1 | 239386990 | 1q43 | 258.2389 | | 0.292 | RGS7 | 0.088959224 |
| rs8110654 | 0.0879476 | 0.0136185 | | 19 | 10007651 | 19p13.2 | 30.1238 | | 0.347 | | 0.088995722 |
| rs11170657 | 0.0599565 | 0.0657887 | | 12 | 52397418 | 12q13.13 | 68.9193 | | 0.167 | CALCOCO1 | 0.089010865 |
| rs584162 | 0.0345777 | 0.0820617 | | 18 | 63693376 | 18q22.1 | 93.8769 | | 0.242 | | 0.089049084 |
| rs4467944 | 0.0102881 | 0.0884963 | | 8 | 47814647 | 8q11.1 | 61.9832 | | 0.35 | | 0.089092293 |
| rs2459068 | 0.0353161 | 0.0820617 | | 10 | 123785283 | 10q26.13 | 147.0416 | | 0.325 | TACC2 | 0.089338411 |
| rs13279994 | 0.0126466 | 0.0884963 | | 8 | 47653281 | 8q11.1 | 61.9507 | | 0.342 | | 0.089395342 |
| rs10958798 | 0.0844908 | 0.0292182 | | 8 | 43910848 | 8p11.1 | 61.3519 | | 0.383 | | 0.089400173 |
| rs6999664 | 0.0137881 | 0.0884963 | | 8 | 47702006 | 8q11.1 | 61.9605 | | 0.342 | | 0.08956396 |
| rs6429732 | 0.0360317 | 0.0820617 | | 1 | 15431525 | 1p36.21 | 27.7195 | | 0.308 | | 0.089623675 |
| rs869220 | 0.0841978 | 0.0308232 | | 20 | 1981627 | 20p13 | 7.3792 | | 0.3 | | 0.089662388 |
| rs2631229 | 0.0374536 | 0.0817005 | | 11 | 11043392 | 11p15.3 | 17.6779 | | 0.367 | | 0.089876325 |
| rs1909446 | 0.0542465 | 0.0717456 | | 8 | 47998639 | 8q11.1 | 62.0203 | | 0.458 | | 0.089945004 |
| rs8035983 | 0.0852045 | 0.0290788 | | 15 | 62006568 | 15q22.31 | 65.85 | | 0.092 | DAPK2 | 0.090029904 |
| rs1321582 | 0.0858170 | 0.0273971 | | 6 | 79250846 | 6q14.1 | 90.11 | | 0.233 | | 0.090084157 |
| rs713084 | 0.0185852 | 0.0882295 | | 6 | 165931419 | 6q27 | 178.5185 | | 0.083 | PDE10A | 0.090165716 |
| rs4838271 | 0.0176725 | 0.0884963 | | 9 | 118991860 | 9q33.1 | 123.8555 | | 0.325 | ASTN2 | 0.090243607 |
| rs10099034 | 0.0177870 | 0.0884963 | | 8 | 128336520 | 8q24.21 | 134.66 | | 0.275 | | 0.090266104 |
| rs8 | 0.0671645 | 0.0605166 | | 7 | 92246265 | 7q21.2 | 103.0449 | | 0.15 | CDK6 | 0.090406484 |

*rch:tme:12/6/2011*

| | | | | | | | | | | |
|---|---|---|---|---|---|---|---|---|---|---|
| rs2430894 | 0.0383045 | 0.0820617 | | 18 | 52568589 | 18q21.31 | 77.5064 | 0.258 | WDR7 | 0.090561346 |
| rs1980291 | 0.0204518 | 0.0882295 | | 2 | 226580253 | 2q36.3 | 229.1215 | 0.092 | | 0.090568869 |
| rs935374 | 0.0198650 | 0.0884963 | | 2 | 46930177 | 2p21 | 73.0809 | 0.25 | | 0.090698463 |
| rs3860290 | 0.0201194 | 0.0884963 | X | | 147338454 | Xq28 | 167.1469 | 0.467 | | 0.09075452 |
| rs10923673 | 0.0343762 | 0.0843675 | | 1 | 119117786 | 1p12 | 140.3712 | 0.108 | | 0.091102155 |
| rs12566190 | 0.0343762 | 0.0843675 | | 1 | 119132546 | 1p12 | 140.3838 | 0.108 | | 0.091102155 |
| rs2287749 | 0.0227139 | 0.0882295 | | 5 | 156851428 | 5q33.3 | 161.6281 | 0.192 | ADAM19 | 0.091106347 |
| rs10065788 | 0.0507734 | 0.0756830 | | 5 | 156782113 | 5q33.3 | 161.5813 | 0.208 | | 0.091136439 |
| rs1518603 | 0.0397771 | 0.0820617 | | 16 | 78825821 | 16q23.2 | 101.2681 | 0.275 | LOC729847 | 0.091193971 |
| rs848692 | 0.0220663 | 0.0884963 | | 14 | 36439853 | 14q13.3 | 39.9502 | 0.208 | SLC25A21 | 0.091205878 |
| rs1455264 | 0.0347331 | 0.0843675 | | 11 | 11756544 | 11p15.3 | 19.9555 | 0.061 | | 0.091237432 |
| rs181694 | 0.0506116 | 0.0759202 | | 16 | 11292330 | 16p13.13 | 28.4397 | 0.175 | | 0.091243685 |
| rs3780708 | 0.0342810 | 0.0846898 | | 9 | 131975772 | 9q34.11 | 139.559 | 0.317 | FREQ | 0.09136493 |
| rs1657385 | 0.0725416 | 0.0556027 | | 18 | 52865314 | 18q21.31 | 77.5894 | 0.192 | | 0.09139995 |
| rs12918181 | 0.0613277 | 0.0677760 | | 16 | 23692905 | 16p12.1 | 46.2937 | 0.233 | | 0.091403902 |
| rs239863 | 0.0231541 | 0.0884963 | | 10 | 115670925 | 10q25.3 | 131.8107 | 0.275 | | 0.09147516 |
| rs3761916 | 0.0751915 | 0.0524899 | | 1 | 203897516 | 1q32.1 | 206.9322 | 0.297 | SLC45A3 | 0.091700373 |
| rs7262069 | 0.0417402 | 0.0817005 | | 20 | 18901170 | 20p11.23 | 47.1152 | 0.417 | | 0.091745443 |
| rs4965520 | 0.0360955 | 0.0843675 | | 15 | 96525461 | 15q26.3 | 123.4224 | 0.068 | | 0.091764738 |
| rs6763419 | 0.0362285 | 0.0843675 | | 3 | 62192561 | 3p14.2 | 84.8412 | 0.068 | PTPRG | 0.091817152 |
| rs2302762 | 0.0913975 | 0.0093893 | | 17 | 7299585 | 17p13.1 | 20.5985 | 0.225 | CHRNB1 | 0.091878496 |
| rs9803530 | 0.0364002 | 0.0843675 | X | | 114939821 | Xq23 | 114.85 | 0.161 | | 0.091885012 |
| rs454886 | 0.0754555 | 0.0524899 | | 5 | 112174016 | 5q22.2 | 118.3828 | 0.358 | APC | 0.09191692 |
| rs589149 | 0.0414722 | 0.0820617 | | 11 | 75563731 | 11q13.5 | 81.5774 | 0.25 | | 0.091945969 |
| rs1329521 | 0.0519378 | 0.0759202 | | 6 | 47840137 | 6p12.3 | 73.1056 | 0.4 | | 0.091985956 |
| rs16949425 | 0.0772554 | 0.0500125 | | 18 | 6092899 | 18p11.31 | 18.9364 | 0.1 | L3MBTL4 | 0.092030686 |
| rs3742480 | 0.0904651 | 0.0174555 | | 14 | 95626021 | 14q32.2 | 103.6769 | 0.216 | C14orf132 | 0.0921337 |
| rs6683502 | 0.0915510 | 0.0111267 | | 1 | 64534847 | 1p31.3 | 91.2717 | 0.167 | | 0.092224628 |

rch:tme:12/6/2011

| | | | | | | | | | | |
|---|---|---|---|---|---|---|---|---|---|---|
| rs1587734 | 0.0908628 | 0.0174555 | | 4 | 121028903 | 4q27 | 122.4103 | 0.308 | | 0.092524293 |
| rs1334168 | 0.0521032 | 0.0764873 | | 13 | 85631645 | 13q31.1 | 78.9878 | 0.208 | | 0.092547593 |
| rs12645274 | 0.0912591 | 0.0166361 | | 4 | 94325195 | 4q22.3 | 100.7494 | 0.058 | GRID2 | 0.09276304 |
| rs3827440 | 0.0867536 | 0.0329077 | X | | 78313644 | Xq21.1 | 86.7371 | 0.442 | GPR174 | 0.092785262 |
| rs6657442 | 0.0924931 | 0.0075075 | | 1 | 195104683 | 1q31.3 | 194.0348 | 0.178 | | 0.092797242 |
| rs11962675 | 0.0928881 | 0.0000000 | | 6 | 64885482 | 6q12 | 81.126 | 0.067 | RP1-303F19.1 | 0.092888131 |
| rs4339947 | 0.0384059 | 0.0846898 | | 10 | 6735491 | 10p14 | 19.7202 | 0.314 | | 0.092991291 |
| rs2890537 | 0.0541367 | 0.0756830 | | 2 | 141277725 | 2q22.1 | 152.936 | 0.067 | LRP1B | 0.093052129 |
| rs1527536 | 0.0300883 | 0.0882295 | X | | 22920046 | Xp22.11 | 39.0401 | 0.25 | | 0.093218823 |
| rs2103520 | 0.0294093 | 0.0884963 | X | | 128192726 | Xq25 | 131.2308 | 0.4 | | 0.093255015 |
| rs8074649 | 0.0858077 | 0.0368363 | | 17 | 13813569 | 17p12 | 40.2649 | 0.492 | | 0.093380282 |
| rs1318655 | 0.0932873 | 0.0042898 | X | | 32674051 | Xp21.1 | 49.9461 | 0.158 | DMD | 0.093385881 |
| rs724201 | 0.0916469 | 0.0180694 | | 12 | 70242280 | 12q21.1 | 86.2685 | 0.35 | LGR5 | 0.093411211 |
| rs350089 | 0.0314348 | 0.0882295 | | 5 | 40210203 | 5p13.1 | 64.6426 | 0.2 | | 0.093662104 |
| rs5908269 | 0.0786511 | 0.0510003 | X | | 141233301 | Xq27.2 | 152.1077 | 0.442 | | 0.093739131 |
| rs1994469 | 0.0428420 | 0.0834344 | | 8 | 127245317 | 8q24.13 | 130.6749 | 0.25 | | 0.093790903 |
| rs2945399 | 0.0882011 | 0.0320688 | | 17 | 22917189 | 17q11.2 | 51.2684 | 0.144 | KSR1 | 0.09385012 |
| rs1734729 | 0.0314259 | 0.0884963 | | 7 | 101688372 | 7q22.1 | 111.4498 | 0.183 | CUTL1 | 0.093910483 |
| rs1773877 | 0.0315744 | 0.0884963 | | 10 | 29342952 | 10p12.1 | 54.612 | 0.149 | | 0.093960274 |
| rs958804 | 0.0322070 | 0.0884963 | | 9 | 118998674 | 9q33.1 | 123.8623 | 0.325 | ASTN2 | 0.094174726 |
| rs10983571 | 0.0322070 | 0.0884963 | | 9 | 118999591 | 9q33.1 | 123.8632 | 0.325 | ASTN2 | 0.094174726 |
| rs9563481 | 0.0330072 | 0.0882295 | | 13 | 56690719 | 13q21.1 | 56.48 | 0.145 | | 0.094201477 |
| rs7866610 | 0.0935022 | 0.0117372 | | 9 | 24501918 | 9p21.3 | 45.5505 | 0.067 | | 0.094235993 |
| rs7648625 | 0.0912133 | 0.0240092 | | 3 | 178400014 | 3q26.32 | 182.8174 | 0.342 | | 0.09432028 |
| rs6534743 | 0.0928296 | 0.0168257 | | 4 | 130934218 | 4q28.2 | 128.08 | 0.267 | LOC729265 | 0.094342119 |
| rs7244506 | 0.0940379 | 0.0084777 | | 18 | 24027654 | 18q12.1 | 51.4823 | 0.15 | | 0.094419277 |
| rs3790688 | 0.0333966 | 0.0884963 | | 1 | 68286072 | 1p31.2 | 95.4761 | 0.328 | DIRAS3 | 0.094588187 |
| rs4843143 | 0.0564753 | 0.0759202 | | 15 | 82019887 | 15q25.2 | 89.0887 | 0.094 | SH3GL3 | 0.094622088 |

*rch:tme:12/6/2011*

| | | | | | | | | | | |
|---|---|---|---|---|---|---|---|---|---|---|
| rs4829348 | 0.0942817 | 0.0087101 | X | | 35030725 | Xp21.1 | 54.9017 | 0.45 | | 0.094683154 |
| rs10003889 | 0.0431472 | 0.0843675 | | 4 | 102915242 | 4q24 | 107.0677 | 0.088 | | 0.094760578 |
| rs542214 | 0.0944989 | 0.0072224 | | 1 | 40871065 | 1p34.2 | 65.3351 | 0.208 | RIMS3 | 0.094774448 |
| rs1257200 | 0.0935274 | 0.0153430 | | 2 | 134753166 | 2q21.2 | 148.3223 | 0.267 | MGAT5 | 0.094777561 |
| rs10403334 | 0.0817442 | 0.0479910 | | 19 | 51196898 | 19q13.32 | 73.5759 | 0.161 | LOC729440 | 0.094790561 |
| rs1039302 | 0.0860316 | 0.0402216 | | 12 | 119720641 | 12q24.31 | 141.0724 | 0.192 | UNQ1887 | 0.094969522 |
| rs7045026 | 0.0772554 | 0.0556027 | | 9 | 120305336 | 9q33.1 | 124.9504 | 0.075 | | 0.095184329 |
| rs4244071 | 0.0862880 | 0.0402216 | | 12 | 65865784 | 12q14.3 | 81.6083 | 0.175 | | 0.095201817 |
| rs11008264 | 0.0353527 | 0.0884963 | | 10 | 31129838 | 10p11.23 | 59.4353 | 0.283 | | 0.095296417 |
| rs887029 | 0.0795819 | 0.0524899 | | 19 | 2449447 | 19p13.3 | 7.4255 | 0.254 | | 0.095333475 |
| rs11073678 | 0.0569276 | 0.0764873 | | 15 | 85206147 | 15q25.3 | 91.1814 | 0.216 | | 0.095347091 |
| rs4777039 | 0.0577241 | 0.0759202 | | 15 | 66408502 | 15q23 | 70.8245 | 0.267 | ITGA11 | 0.095372681 |
| rs3821053 | 0.0938425 | 0.0174555 | | 2 | 97758319 | 2q11.2 | 112.94 | 0.425 | TMEM131 | 0.095452092 |
| rs718159 | 0.0938425 | 0.0174555 | | 2 | 97785966 | 2q11.2 | 112.952 | 0.425 | TMEM131 | 0.095452092 |
| rs1595489 | 0.0703632 | 0.0646321 | | 3 | 68294616 | 3p14.1 | 93.2679 | 0.05 | FAM19A1 | 0.095542129 |
| rs7116632 | 0.0879248 | 0.0376389 | | 11 | 129452949 | 11q24.3 | 141.4233 | 0.117 | APLP2 | 0.095642332 |
| rs2304731 | 0.0810673 | 0.0510003 | | 11 | 12858819 | 11p15.2 | 21.9486 | 0.358 | TEAD1 | 0.09577546 |
| rs4962653 | 0.0924034 | 0.0263965 | | 10 | 126252385 | 10q26.13 | 151.8185 | 0.125 | LHPP | 0.096099778 |
| rs198780 | 0.0455037 | 0.0846898 | X | | 38541601 | Xp11.4 | 60.25 | 0.442 | | 0.096140273 |
| rs2165846 | 0.0583777 | 0.0764873 | | 17 | 42296365 | 17q21.31 | 73.6426 | 0.442 | WNT9B | 0.096219884 |
| rs11629182 | 0.0959594 | 0.0075075 | | 14 | 68296569 | 14q24.1 | 67.3326 | 0.265 | | 0.096252638 |
| rs235799 | 0.0504568 | 0.0820617 | | 16 | 26602923 | 16p12.1 | 51.9179 | 0.203 | | 0.096332812 |
| rs2211472 | 0.0957548 | 0.0117372 | X | | 85990707 | Xq21.2 | 88.27 | 0.058 | | 0.096471431 |
| rs7689919 | 0.0513893 | 0.0817005 | | 4 | 36007388 | 4p14 | 54.7187 | 0.358 | FLJ16686 | 0.096518609 |
| rs133295 | 0.0848586 | 0.0460523 | | 22 | 40714071 | 22q13.2 | 49.4922 | 0.225 | SEPT3 | 0.096549398 |
| rs2292884 | 0.0921167 | 0.0293080 | | 2 | 238107965 | 2q37.3 | 250.6497 | 0.217 | MLPH | 0.096666673 |
| rs1864895 | 0.0592005 | 0.0764873 | | 3 | 109060594 | 3q13.12 | 117.625 | 0.283 | | 0.096721318 |
| rs11564355 | 0.0056600 | 0.0967227 | | 18 | 24117372 | 18q12.1 | 51.566 | 0.192 | | 0.09688816 |

*rch:tme:12/6/2011*

| | | | | | | | | | | |
|---|---|---|---|---|---|---|---|---|---|---|
| rs11082159 | 0.0395680 | 0.0884963 | | 18 | 35732656 | 18q12.3 | 60.1747 | 0.292 | | 0.096939245 |
| rs11614358 | 0.0597801 | 0.0764873 | | 12 | 111969982 | 12q24.13 | 127.9893 | 0.333 | | 0.097077162 |
| rs6582285 | 0.0954237 | 0.0180694 | | 12 | 73977054 | 12q21.1 | 87.8077 | 0.457 | CAPS2 | 0.097119453 |
| rs7719763 | 0.0400553 | 0.0884963 | | 5 | 67717181 | 5q13.1 | 80.9148 | 0.284 | | 0.097139171 |
| rs8061082 | 0.0655489 | 0.0717456 | | 16 | 6694529 | 16p13.2 | 17.5999 | 0.3 | A2BP1 | 0.097180657 |
| rs2051301 | 0.0401701 | 0.0884963 | | 18 | 64691203 | 18q22.1 | 95.5576 | 0.258 | CCDC102B | 0.097186578 |
| rs4761528 | 0.0973279 | 0.0008435 | | 12 | 92714634 | 12q22 | 103.9507 | 0.167 | CRADD | 0.097331545 |
| rs346831 | 0.0959234 | 0.0166361 | | 2 | 8350879 | 2p25.1 | 21.0515 | 0.161 | C2orf46 | 0.097355292 |
| rs2159318 | 0.0867691 | 0.0441741 | | 16 | 7649537 | 16p13.2 | 19.9406 | 0.467 | A2BP1 | 0.097366455 |
| rs4830955 | 0.0525602 | 0.0820617 | X | | 15400794 | Xp22.2 | 29.6238 | 0.467 | PIR | 0.097451007 |
| rs6991834 | 0.0958178 | 0.0180694 | | 8 | 3115506 | 8p23.2 | 5.5667 | 0.275 | CSMD1 | 0.097506713 |
| rs2760494 | 0.0969625 | 0.0105786 | | 1 | 59010958 | 1p32.1 | 83.07 | 0.142 | | 0.097537847 |
| rs7778311 | 0.0410441 | 0.0884963 | | 7 | 47619240 | 7p12.3 | 70.0242 | 0.307 | | 0.097551057 |
| rs1894704 | 0.0645458 | 0.0731997 | | 22 | 25183905 | 22q12.1 | 25.4563 | 0.05 | HPS4 | 0.097592843 |
| rs1894706 | 0.0645458 | 0.0731997 | | 22 | 25184441 | 22q12.1 | 25.4589 | 0.05 | HPS4 | 0.097592843 |
| rs4765028 | 0.0420863 | 0.0882295 | | 12 | 124278681 | 12q24.31 | 145.2854 | 0.142 | | 0.097753259 |
| rs16934689 | 0.0977604 | 0.0006850 | | 11 | 40277367 | 11p12 | 57.0913 | 0.083 | | 0.097762824 |
| rs1948073 | 0.0145761 | 0.0967227 | | 16 | 53765852 | 16q12.2 | 69.7892 | 0.095 | | 0.097814842 |
| rs8047991 | 0.0542361 | 0.0817005 | | 16 | 49054503 | 16q12.1 | 60.2079 | 0.15 | | 0.098063897 |
| rs7350986 | 0.0938088 | 0.0290788 | | 18 | 35222878 | 18q12.2 | 60.0113 | 0.092 | | 0.098212403 |
| rs2304900 | 0.0628688 | 0.0756830 | | 15 | 73127693 | 15q24.2 | 80.1715 | 0.083 | PPCDC | 0.098389009 |
| rs11721729 | 0.0502312 | 0.0846898 | | 4 | 130334288 | 4q28.2 | 127.9705 | 0.155 | | 0.098465935 |
| rs3740259 | 0.0550663 | 0.0817005 | | 10 | 80744822 | 10q22.3 | 99.5176 | 0.321 | ZMIZ1 | 0.098525484 |
| rs3744137 | 0.0194964 | 0.0967227 | | 17 | 16986749 | 17p11.2 | 47.0808 | 0.492 | M-RIP | 0.098668072 |
| rs662199 | 0.0953340 | 0.0259136 | | 11 | 85127190 | 11q14.1 | 90.2821 | 0.258 | SYTL2 | 0.098793167 |
| rs808576 | 0.0783276 | 0.0605166 | X | | 32490500 | Xp21.1 | 49.5601 | 0.417 | DMD | 0.098982175 |
| rs837473 | 0.0946119 | 0.0293080 | | 12 | 123633202 | 12q24.31 | 143.2837 | 0.242 | | 0.099047344 |
| rs11930193 | 0.0213875 | 0.0967227 | | 4 | 161798869 | 4q32.1 | 154.3305 | 0.15 | | 0.099059106 |

*rch:tme:12/6/2011*

| | | | | | | | | | | |
|---|---|---|---|---|---|---|---|---|---|---|
| rs2861630 | 0.0977018 | 0.0166361 | | 1 | 174309008 | 1q25.1 | 177.7573 | 0.1 | RFWD2 | 0.09910801 |
| rs732227 | 0.0841479 | 0.0524899 | | 2 | 150586238 | 2q23.3 | 160.8833 | 0.4 | | 0.099176946 |
| rs418543 | 0.0836935 | 0.0535807 | | 13 | 109977797 | 13q34 | 119.1143 | 0.342 | RAB20 | 0.099375493 |
| rs12876596 | 0.0540516 | 0.0834344 | | 13 | 23419485 | 13q12.12 | 9.6879 | 0.325 | FLJ46358,LOC729825 | 0.099412641 |
| rs1984020 | 0.0540704 | 0.0834344 | | 21 | 37105929 | 21q22.13 | 44.015 | 0.267 | HLCS | 0.099422881 |
| rs1953263 | 0.0970071 | 0.0218702 | | 14 | 56083958 | 14q22.3 | 56.52 | 0.142 | | 0.099441841 |
| rs2435962 | 0.0649988 | 0.0759202 | | 2 | 38167998 | 2p22.2 | 63.1752 | 0.2 | | 0.099943573 |
| rs1171837 | 0.0531350 | 0.0846898 | | 10 | 61350672 | 10q21.2 | 78.1443 | 0.342 | | 0.099978474 |
| rs7785760 | 0.0755256 | 0.0657310 | | 7 | 152322133 | 7q36.2 | 169.0794 | 0.1 | | 0.100123359 |
| rs6468154 | 0.0878849 | 0.0479910 | | 8 | 33264445 | 8p12 | 54.1022 | 0.317 | | 0.100134333 |
| rs7618693 | 0.0469210 | 0.0884963 | | 3 | 96867819 | 3q11.2 | 110.4181 | 0.339 | | 0.100165702 |
| rs5961385 | 0.0846518 | 0.0535807 | X | | 5871094 | Xp22.32 | 12.7122 | 0.3 | NLGN4X | 0.100183951 |
| rs2155387 | 0.0575129 | 0.0820617 | | 11 | 117159386 | 11q23.3 | 119.8702 | 0.408 | DSCAML1 | 0.100209066 |
| rs324121 | 0.0554394 | 0.0843675 | | 19 | 57586257 | 19q13.41 | 91.9118 | 0.144 | | 0.10095252 |
| rs359937 | 0.0550873 | 0.0846898 | | 1 | 89755307 | 1p22.2 | 113.9126 | 0.219 | | 0.101029604 |
| rs7675016 | 0.0670322 | 0.0756830 | | 4 | 105653679 | 4q24 | 109.14 | 0.133 | | 0.10110011 |
| rs1455251 | 0.0595703 | 0.0817005 | | 11 | 11710706 | 11p15.3 | 19.8729 | 0.15 | | 0.101111828 |
| rs7946 | 0.0965772 | 0.0308232 | | 17 | 17350285 | 17p11.2 | 47.4443 | 0.267 | PEMT | 0.101376617 |
| rs4520323 | 0.0717847 | 0.0717456 | X | | 26719286 | Xp21.3 | 42.78 | 0.45 | | 0.101491184 |
| rs17343504 | 0.0679387 | 0.0756830 | | 4 | 96120504 | 4q22.3 | 102.1412 | 0.053 | BMPR1B | 0.101703402 |
| rs7154732 | 0.0977665 | 0.0283225 | | 14 | 50440599 | 14q22.1 | 48.1631 | 0.025 | C14orf29,PYGL | 0.101786345 |
| rs713469 | 0.0865454 | 0.0535807 | | 15 | 58121920 | 15q22.2 | 60.26 | 0.233 | | 0.101788987 |
| rs230526 | 0.0676617 | 0.0764873 | | 4 | 103677855 | 4q24 | 107.4626 | 0.467 | NFKB1 | 0.102119648 |
| rs1459151 | 0.0571023 | 0.0846898 | | 4 | 174100347 | 4q34.1 | 167.9227 | 0.3 | GALNT17 | 0.10214227 |
| rs2332914 | 0.0979557 | 0.0290788 | | 14 | 72249625 | 14q24.2 | 71.5319 | 0.067 | DPF3 | 0.102180734 |
| rs931859 | 0.0970856 | 0.0320688 | | 11 | 131004591 | 11q25 | 145.6319 | 0.142 | | 0.102244887 |

*rch:tme:12/6/2011*

| | | | | | | | | | | |
|---|---|---|---|---|---|---|---|---|---|---|
| rs1065754 | 0.0610629 | 0.0820617 | | 1 | 51646539 | 1p32.3 | 72.4618 | 0.317 | EPS15 | 0.102287828 |
| rs12452861 | 0.0610719 | 0.0820617 | | 17 | 5847332 | 17p13.2 | 16.0068 | 0.325 | | 0.102293171 |
| rs2026362 | 0.0580516 | 0.0843675 | | 9 | 103280575 | 9q31.1 | 104.2089 | 0.133 | C9orf125 | 0.102410313 |
| rs7260918 | 0.0575906 | 0.0846898 | | 20 | 16133468 | 20p12.1 | 42.1911 | 0.292 | | 0.102416057 |
| rs7853023 | 0.0612916 | 0.0820617 | | 9 | 38762575 | 9p13.1 | 60.6482 | 0.178 | | 0.102424538 |
| rs2588873 | 0.0689549 | 0.0759202 | | 2 | 177749116 | 2q31.2 | 184.7533 | 0.2 | | 0.102560517 |
| rs1244655 | 0.0828552 | 0.0605166 | | | | 18q22.3 | 99.1601 | 0.085 | | 0.102602366 |
| rs9667864 | 0.0580414 | 0.0846898 | | 11 | 101876959 | 11q22.2 | 104.2728 | 0.258 | | 0.102670225 |
| rs12881439 | 0.0734769 | 0.0717456 | | 14 | 36175604 | 14q13.3 | 39.5421 | 0.184 | | 0.102695042 |
| rs2689828 | 0.0897548 | 0.0500125 | | 2 | 167922928 | 2q24.3 | 172.0525 | 0.1 | hCG_1660379 | 0.10274813 |
| rs2617394 | 0.0897548 | 0.0500125 | | 2 | 167926199 | 2q24.3 | 172.0563 | 0.1 | hCG_1660379 | 0.10274813 |
| rs4907732 | 0.0694235 | 0.0759202 | | 13 | 112349342 | 13q34 | 125.8492 | 0.203 | FLJ26443 | 0.102876123 |
| rs1598859 | 0.0908860 | 0.0490637 | | 4 | 103725482 | 4q24 | 107.4863 | 0.4 | NFKB1 | 0.103283654 |
| rs2239473 | 0.0909029 | 0.0500125 | X | | 123466853 | Xq25 | 126.3818 | 0.2 | ODZ1 | 0.103752572 |
| rs7622789 | 0.0378298 | 0.0967227 | | 3 | 129191759 | 3q21.3 | 135.4774 | 0.198 | | 0.10385746 |
| rs10519131 | 0.0988218 | 0.0320688 | | 15 | 59788424 | 15q22.2 | 63.3424 | 0.133 | | 0.103894935 |
| rs857477 | 0.0925214 | 0.0479910 | | 6 | 14734708 | 6p23 | 32.9073 | 0.223 | | 0.104227312 |
| rs912435 | 0.0811176 | 0.0657310 | | 13 | 46032511 | 13q14.13 | 50.303 | 0.059 | LRCH1 | 0.104406122 |
| rs241250 | 0.0795437 | 0.0677760 | | 1 | 4513093 | 1p36.32 | 6.4969 | 0.214 | | 0.104502539 |
| rs12410385 | 0.0565581 | 0.0882295 | | 1 | 144251749 | 1q21.1 | 142.09 | 0.15 | ITGA10 | 0.10480107 |
| rs4491457 | 0.0659386 | 0.0817005 | | 15 | 93187447 | 15q26.2 | 111.1703 | 0.212 | | 0.104989881 |
| rs2997664 | 0.0660957 | 0.0817005 | | 9 | 71865877 | 9q21.11 | 66.7837 | 0.267 | MAMDC2 | 0.105088618 |
| rs11030106 | 0.0622439 | 0.0846898 | | 11 | 3838306 | 11p15.4 | 5.5717 | 0.345 | STIM1 | 0.105103129 |
| rs943174 | 0.0659222 | 0.0820617 | | 9 | 7389175 | 9p24.1 | 16.1395 | 0.333 | | 0.105260894 |
| rs7399511 | 0.0893853 | 0.0556027 | | | | 13q21.1 | 56.48 | 0.2 | | 0.105268178 |
| rs2238702 | 0.0771717 | 0.0717456 | | 21 | 39952627 | 21q22.2 | 50.5975 | 0.144 | B3GALT5 | 0.105370293 |
| rs7583169 | 0.0896133 | 0.0556027 | | 2 | 1542840 | 2p25.3 | 3.8014 | 0.408 | | 0.105461855 |
| rs4310223 | 0.0574157 | 0.0884963 | | 8 | 131711803 | 8q24.22 | 140.128 | 0.292 | | 0.105490039 |

rch:tme:12/6/2011

| | | | | | | | | | | |
|---|---|---|---|---|---|---|---|---|---|---|
| rs883429 | 0.0663267 | 0.0820617 | | 8 | 22942763 | 8p21.3 | 40.869 | 0.342 | TNFRSF10B | 0.105514701 |
| rs877748 | 0.0633130 | 0.0846898 | | 10 | 129072298 | 10q26.2 | 159.1553 | 0.254 | DOCK1 | 0.105739802 |
| rs917673 | 0.0432712 | 0.0967227 | | 18 | 57170020 | 18q21.33 | 85.5918 | 0.158 | | 0.105960738 |
| rs1004604 | 0.0655391 | 0.0834344 | | 9 | 38352171 | 9p13.2 | 60.3916 | 0.25 | | 0.106097483 |
| rs6418096 | 0.0439303 | 0.0967227 | X | | 90858147 | Xq21.31 | 92.8192 | 0.3 | | 0.106231592 |
| rs3763215 | 0.0745400 | 0.0759202 | | 6 | 107488302 | 6q21 | 110.9684 | 0.271 | | 0.106395898 |
| rs640755 | 0.0740336 | 0.0764873 | | 12 | 3906370 | 12p13.32 | 10.8492 | 0.167 | | 0.106448516 |
| rs1522823 | 0.0599904 | 0.0882295 | | 2 | 226574100 | 2q36.3 | 229.1204 | 0.092 | | 0.10669251 |
| rs3749010 | 0.0752859 | 0.0756830 | | 2 | 141287799 | 2q22.1 | 152.9491 | 0.075 | LRP1B | 0.106751514 |
| rs940597 | 0.0938425 | 0.0510003 | | 7 | 67412967 | 7q11.22 | 79.8843 | 0.4 | | 0.106805637 |
| rs2243639 | 0.0829924 | 0.0677760 | | 10 | 81691702 | 10q22.3 | 100.6783 | 0.467 | SFTPD | 0.1071509 |
| rs1715489 | 0.0967848 | 0.0460523 | | 17 | 72790904 | 17q25.2 | 117.9151 | 0.125 | | 0.107182567 |
| rs4674039 | 0.0662209 | 0.0846898 | | 2 | 216502269 | 2q35 | 213.4453 | 0.225 | | 0.107506154 |
| rs4658340 | 0.0683682 | 0.0834344 | | 1 | 90100143 | 1p22.2 | 114.2446 | 0.225 | LRRC8D | 0.107868022 |
| rs2802031 | 0.0683682 | 0.0834344 | | 1 | 90112257 | 1p22.2 | 114.2563 | 0.225 | LRRC8D | 0.107868022 |
| rs6530854 | 0.0668871 | 0.0846898 | | 8 | 15280833 | 8p22 | 26.3095 | 0.348 | | 0.107917817 |
| rs9931258 | 0.0864840 | 0.0646321 | | 16 | 87815919 | 16q24.3 | 129.7038 | 0.06 | ZNF778 | 0.107966637 |
| rs984779 | 0.0619542 | 0.0884963 | | 1 | 83342859 | 1p31.1 | 107.9544 | 0.392 | | 0.108027384 |
| rs2740170 | 0.0622812 | 0.0884963 | | 1 | 224091420 | 1q42.12 | 229.6779 | 0.233 | EPHX1 | 0.108215228 |
| rs6649680 | 0.0818766 | 0.0708063 | X | | 143280863 | Xq27.3 | 158.2107 | 0.117 | | 0.108246515 |
| rs1475418 | 0.0712323 | 0.0817005 | | 10 | 6730153 | 10p14 | 19.7081 | 0.275 | | 0.108392913 |
| rs407179 | 0.0676746 | 0.0846898 | | 9 | 98052098 | 9q22.32 | 99.8121 | 0.25 | HSD17B3 | 0.108407626 |
| rs998460 | 0.0686778 | 0.0843675 | | 4 | 34138953 | 4p15.1 | 53.6866 | 0.092 | | 0.108786621 |
| rs2195956 | 0.0499205 | 0.0967227 | | 19 | 35806921 | 19q12 | 52.2178 | 0.167 | | 0.108845486 |
| rs10974530 | 0.0983828 | 0.0479910 | | 9 | 4415033 | 9p24.2 | 10.1209 | 0.25 | | 0.109463692 |
| rs1751277 | 0.0701772 | 0.0846898 | | 10 | 4466409 | 10p15.1 | 14.5683 | 0.208 | | 0.109987269 |
| rs708262 | 0.0717152 | 0.0834344 | | 16 | 52672030 | 16q12.2 | 67.6817 | 0.229 | FTO | 0.110019859 |
| rs9320475 | 0.0872161 | 0.0677760 | | 6 | 114350199 | 6q21 | 117.75 | 0.22 | | 0.110454635 |

*rch:tme:12/6/2011*

| | | | | | | | | | | |
|---|---|---|---|---|---|---|---|---|---|---|
| rs4556497 | 0.0711773 | 0.0846898 | | 11 | 25619991 | 11p14.3 | 42.3813 | 0.233 | | 0.110628114 |
| rs5935253 | 0.0984451 | 0.0510003 | X | | 12105668 | Xp22.2 | 22.154 | 0.367 | FRMPD4 | 0.110871433 |
| rs4975147 | 0.0929228 | 0.0605166 | | 4 | 79707294 | 4q21.21 | 87.8446 | 0.158 | ANXA3 | 0.110891437 |
| rs4771332 | 0.0716855 | 0.0846898 | | 13 | 98868458 | 13q32.3 | 93.6112 | 0.292 | | 0.110955747 |
| rs12516171 | 0.0544596 | 0.0967227 | | 5 | 21939391 | 5p14.3 | 41.3647 | 0.125 | CDH12 | 0.111000565 |
| rs6503841 | 0.0895682 | 0.0657310 | | 17 | 53398640 | 17q23.2 | 87.8453 | 0.138 | | 0.111099208 |
| rs4759732 | 0.0962571 | 0.0556027 | | 12 | 129635767 | 12q24.33 | 167.0743 | 0.192 | | 0.111162449 |
| rs823673 | 0.0812129 | 0.0759202 | | 1 | 41013106 | 1p34.2 | 65.4709 | 0.216 | | 0.111172922 |
| rs4479955 | 0.0677754 | 0.0882295 | | 6 | 138368250 | 6q23.3 | 140.9563 | 0.153 | | 0.111256256 |
| rs3110496 | 0.0727630 | 0.0846898 | | 17 | 24941897 | 17q11.2 | 52.6041 | 0.317 | | 0.111654939 |
| rs8009231 | 0.0686198 | 0.0882295 | | 14 | 89447067 | 14q32.11 | 91.3698 | 0.103 | C14orf143 | 0.111772613 |
| rs7140212 | 0.0686198 | 0.0882295 | | 14 | 89456658 | 14q32.11 | 91.3932 | 0.108 | C14orf143 | 0.111772613 |
| rs7122009 | 0.0562012 | 0.0967227 | | 11 | 86388158 | 11q14.2 | 91.1973 | 0.125 | | 0.111865337 |
| rs1541103 | 0.0993520 | 0.0524899 | | 21 | 41535214 | 21q22.3 | 55.7466 | 0.398 | BACE2 | 0.112365504 |
| rs7620956 | 0.0775261 | 0.0817005 | | 3 | 67503673 | 3p14.1 | 92.8152 | 0.408 | | 0.112628946 |
| rs1954925 | 0.0832177 | 0.0759202 | | 6 | 162447621 | 6q26 | 172.9682 | 0.333 | PARK2 | 0.112645776 |
| rs10964155 | 0.0782562 | 0.0817005 | | 9 | 19459713 | 9p22.1 | 39.838 | 0.325 | LOC392288 | 0.11313273 |
| rs7000815 | 0.0924239 | 0.0657887 | | 8 | 43259714 | 8p11.1 | 61.2519 | 0.117 | | 0.11344752 |
| rs4861175 | 0.0880539 | 0.0717456 | | 4 | 41932655 | 4p13 | 63.3554 | 0.117 | | 0.113582168 |
| rs10180461 | 0.0757871 | 0.0846898 | | 2 | 143979503 | 2q22.3 | 155.3608 | 0.33 | ARHGAP15 | 0.113648808 |
| rs9320884 | 0.0713170 | 0.0884963 | | 6 | 122979311 | 6q22.31 | 121.7577 | 0.314 | PKIB | 0.113656099 |
| rs1892555 | 0.0871257 | 0.0731997 | | 14 | 27097765 | 14q12 | 24.2749 | 0.125 | LOC728755 | 0.113794021 |
| rs1503195 | 0.0928751 | 0.0657887 | X | | 104967130 | Xq22.3 | 104.1964 | 0.117 | NRK | 0.113815406 |
| rs1566861 | 0.0763587 | 0.0846898 | | 8 | 4169272 | 8p23.2 | 8.359 | 0.342 | CSMD1 | 0.114030783 |
| rs10506596 | 0.0933787 | 0.0657887 | | 12 | 69277604 | 12q15 | 85.3716 | 0.092 | PTPRB | 0.114226667 |
| rs4854135 | 0.0854176 | 0.0759202 | | 2 | 3300430 | 2p25.2 | 11.855 | 0.233 | TSSC1 | 0.11428056 |
| rs547432 | 0.0854577 | 0.0759202 | | 3 | 191341817 | 3q28 | 204.0741 | 0.242 | | 0.114310522 |
| rs17028972 | 0.0858268 | 0.0756830 | | 1 | 112236297 | 1p13.2 | 133.4458 | 0.067 | KCND3 | 0.114429682 |

*rch:tme:12/6/2011*

| | | | | | | | | | | |
|---|---|---|---|---|---|---|---|---|---|---|
| rs7701642 | 0.0858218 | 0.0759202 | | 5 | 43430535 | 5p12 | 66.5052 | 0.424 | CCL28 | 0.114582981 |
| rs4403996 | 0.0800333 | 0.0820617 | | 14 | 104653569 | 14q32.33 | 123.7702 | 0.392 | | 0.114627446 |
| rs1875789 | 0.0949065 | 0.0646321 | | 10 | 53642471 | 10q21.1 | 71.1547 | 0.075 | PRKG1 | 0.114824063 |
| rs4643650 | 0.0620516 | 0.0967227 | | 3 | 34825307 | 3p23 | 60.2211 | 0.231 | | 0.114915945 |
| rs2597909 | 0.0943946 | 0.0657887 | | 15 | 89462723 | 15q26.1 | 98.0353 | 0.085 | | 0.115058686 |
| rs2654417 | 0.0735928 | 0.0884963 | | 3 | 106285277 | 3q13.11 | 114.8378 | 0.342 | | 0.115097751 |
| rs10818161 | 0.0745878 | 0.0882295 | | 9 | 120293663 | 9q33.1 | 124.9187 | 0.075 | | 0.115532625 |
| rs4656680 | 0.0813496 | 0.0820617 | | 1 | 167688381 | 1q24.2 | 171.6015 | 0.325 | | 0.11555034 |
| rs2388569 | 0.0817533 | 0.0817005 | | 10 | 3138116 | 10p15.2 | 11.0529 | 0.242 | PFKP | 0.115579294 |
| rs2139209 | 0.0818721 | 0.0820617 | | 4 | 166694537 | 4q32.3 | 160.7345 | 0.258 | | 0.115918741 |
| rs4725617 | 0.0795812 | 0.0843675 | | 7 | 142807222 | 7q34 | 151.7226 | 0.125 | EPHA1 | 0.115978637 |
| rs10865566 | 0.0835460 | 0.0820617 | | 3 | 78538261 | 3p12.3 | 106.822 | 0.192 | | 0.117107053 |
| rs324125 | 0.0812585 | 0.0843675 | | 19 | 57579094 | 19q13.41 | 91.8922 | 0.15 | | 0.117135946 |
| rs1001294 | 0.0774498 | 0.0884963 | | 22 | 34960936 | 22q12.3 | 41.979 | 0.367 | APOL2 | 0.117601296 |
| rs807331 | 0.0934092 | 0.0717456 | | 14 | 44239039 | 14q21.2 | 44.4455 | 0.283 | | 0.117782447 |
| rs904910 | 0.0781752 | 0.0884963 | | 1 | 16072750 | 1p36.13 | 28.8622 | 0.258 | SPEN | 0.11808025 |
| rs2037284 | 0.0855757 | 0.0817005 | | 2 | 156235066 | 2q24.1 | 165.3924 | 0.267 | | 0.118313895 |
| rs9850416 | 0.0785485 | 0.0884963 | | 3 | 142754906 | 3q23 | 147.7448 | 0.35 | RASA2 | 0.118327766 |
| rs7730091 | 0.0908014 | 0.0764873 | | 5 | 179623007 | 5q35.3 | 204.4822 | 0.342 | MAPK9 | 0.118723229 |
| rs1835851 | 0.0858398 | 0.0820617 | | 8 | 130422409 | 8q24.21 | 138.986 | 0.258 | | 0.118754318 |
| rs2060009 | 0.0859153 | 0.0820617 | | 11 | 120711564 | 11q23.3 | 124.6599 | 0.293 | | 0.118808889 |
| rs1017205 | 0.0837981 | 0.0843675 | | 19 | 39057480 | 19q13.11 | 57.3901 | 0.067 | | 0.118911736 |
| rs5935513 | 0.0838146 | 0.0846898 | X | | 13084600 | Xp22.2 | 25.3703 | 0.35 | | 0.119152196 |
| rs10097563 | 0.0850653 | 0.0834344 | | 8 | 40699795 | 8p11.21 | 59.141 | 0.358 | ZMAT4 | 0.119152844 |
| rs4129316 | 0.0918372 | 0.0759202 | | 18 | 20937539 | 18q11.2 | 46.8494 | 0.225 | ZNF521 | 0.119155142 |
| rs1565610 | 0.0843956 | 0.0843675 | X | | 151240061 | Xq28 | 183.1674 | 0.125 | GABRA3 | 0.119333547 |
| rs976845 | 0.0924596 | 0.0756830 | X | | 144544547 | Xq27.3 | 162.2841 | 0.296 | | 0.119485122 |
| rs843677 | 0.0918324 | 0.0764873 | | 2 | 54263359 | 2p16.2 | 78.36 | 0.492 | ACYP2 | 0.119513578 |

rch:tme:12/6/2011

| | | | | | | | | | |
|---|---|---|---|---|---|---|---|---|---|
| rs7223099 | 0.0813368 | 0.0882295 | 17 | 60667516 | 17q24.1 | 93.7839 | 0.175 | | 0.120000516 |
| rs7818051 | 0.0938904 | 0.0756830 | 8 | 100241574 | 8q22.2 | 104.7357 | 0.142 | VPS13B | 0.120595666 |
| rs4335989 | 0.0998485 | 0.0677760 | 2 | 56247422 | 2p16.1 | 79.7793 | 0.133 | | 0.120678503 |
| rs177298 | 0.0935311 | 0.0764873 | 12 | 45924845 | 12q13.11 | 61.2998 | 0.25 | | 0.120823714 |
| rs3813804 | 0.0824140 | 0.0884963 | 1 | 28154793 | 1p35.3 | 46.8666 | 0.308 | SMPDL3B | 0.120928322 |
| rs2837851 | 0.0838357 | 0.0882295 | 21 | 41127395 | 21q22.2 | 54.2963 | 0.068 | DSCAM | 0.121708123 |
| rs7493192 | 0.0947464 | 0.0764873 | 14 | 95295371 | 14q32.13 | 103.1755 | 0.208 | | 0.121766954 |
| rs1564821 | 0.0914903 | 0.0820617 | 17 | 50603926 | 17q22 | 81.2606 | 0.203 | | 0.122900748 |
| rs6069921 | 0.0858402 | 0.0884963 | 20 | 54894402 | 20q13.31 | 92.5314 | 0.292 | | 0.123288799 |
| rs2526535 | 0.0909621 | 0.0834344 | 17 | 45750337 | 17q21.33 | 76.9247 | 0.314 | | 0.123431763 |
| rs1488171 | 0.0984536 | 0.0764873 | 3 | 164186242 | 3q26.1 | 167.027 | 0.375 | | 0.124673269 |
| rs1590202 | 0.0889507 | 0.0884963 | 20 | 2110656 | 20p13 | 7.773 | 0.333 | | 0.12547439 |
| rs1372347 | 0.0805737 | 0.0967227 | 15 | 60604094 | 15q22.2 | 64.7702 | 0.267 | | 0.125886485 |
| rs1858095 | 0.0935115 | 0.0846898 | 9 | 105462679 | 9q31.1 | 106.1487 | 0.217 | | 0.126161667 |
| rs10502079 | 0.0816105 | 0.0967227 | 11 | 106224087 | 11q22.3 | 106.9633 | 0.212 | GUCY1A2 | 0.126552558 |
| rs6471335 | 0.0942848 | 0.0846898 | 8 | 93316538 | 8q21.3 | 97.4542 | 0.25 | | 0.126735886 |
| rs1658693 | 0.0972513 | 0.0817005 | 12 | 91089367 | 12q21.33 | 101.8091 | 0.283 | | 0.127014906 |
| rs6556865 | 0.0948765 | 0.0846898 | 5 | 94770449 | 5q15 | 107.7104 | 0.297 | FAM81B | 0.127176746 |
| rs12550783 | 0.0985364 | 0.0817005 | 8 | 99712663 | 8q22.2 | 104.4757 | 0.2 | STK3 | 0.128001597 |
| rs7851794 | 0.0990639 | 0.0817005 | 9 | 105226867 | 9q31.1 | 106.0097 | 0.292 | | 0.128408041 |
| rs1362128 | 0.0932831 | 0.0884963 | 7 | 125101895 | 7q31.33 | 128.1512 | 0.342 | | 0.128581975 |
| rs239832 | 0.0997469 | 0.0820617 | 6 | 54877359 | 6p12.1 | 78.2269 | 0.184 | FAM83B | 0.12916491 |
| rs2734189 | 0.0979708 | 0.0846898 | 7 | 141922620 | 7q34 | 151.4369 | 0.217 | | 0.129501531 |
| rs6965038 | 0.0995103 | 0.0882295 | 7 | 43483696 | 7p13 | 65.1154 | 0.125 | HECW1 | 0.132991494 |
| rs9911349 | 0.0926880 | 0.0967227 | 17 | 10819980 | 17p12 | 31.3139 | 0.142 | | 0.133963982 |
| rs10511470 | 0.0936956 | 0.0967227 | 9 | 7469600 | 9p24.1 | 16.3613 | 0.233 | | 0.134663103 |
| rs6949236 | 0.0955730 | 0.0967227 | 7 | 11807866 | 7p21.3 | 23.0591 | 0.175 | THSD7A | 0.135976026 |

*rch:tme:12/6/2011*

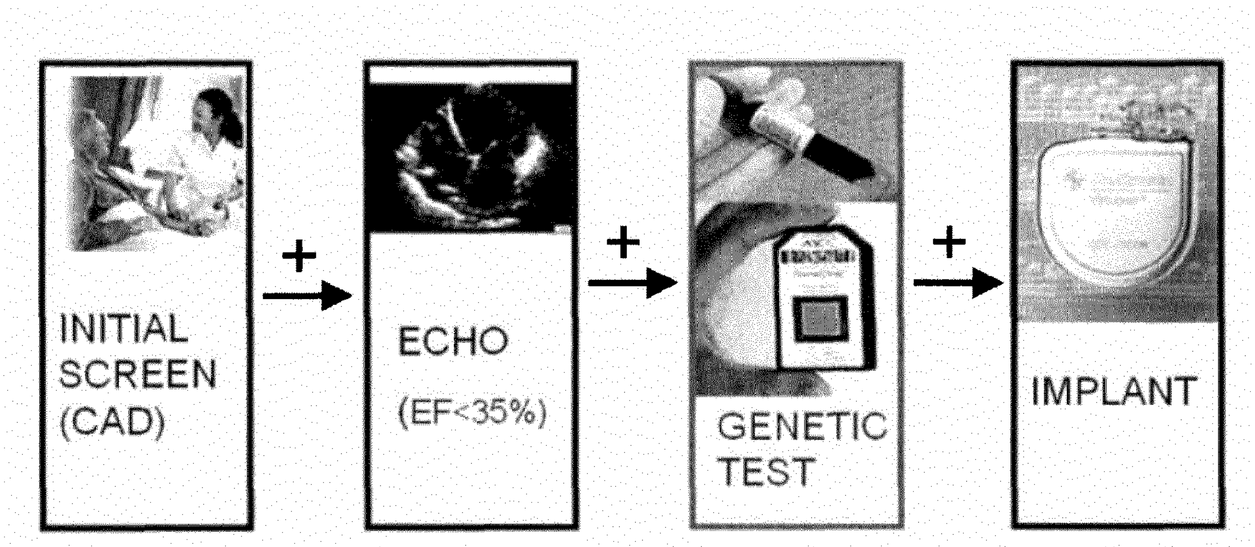


Fig. 8

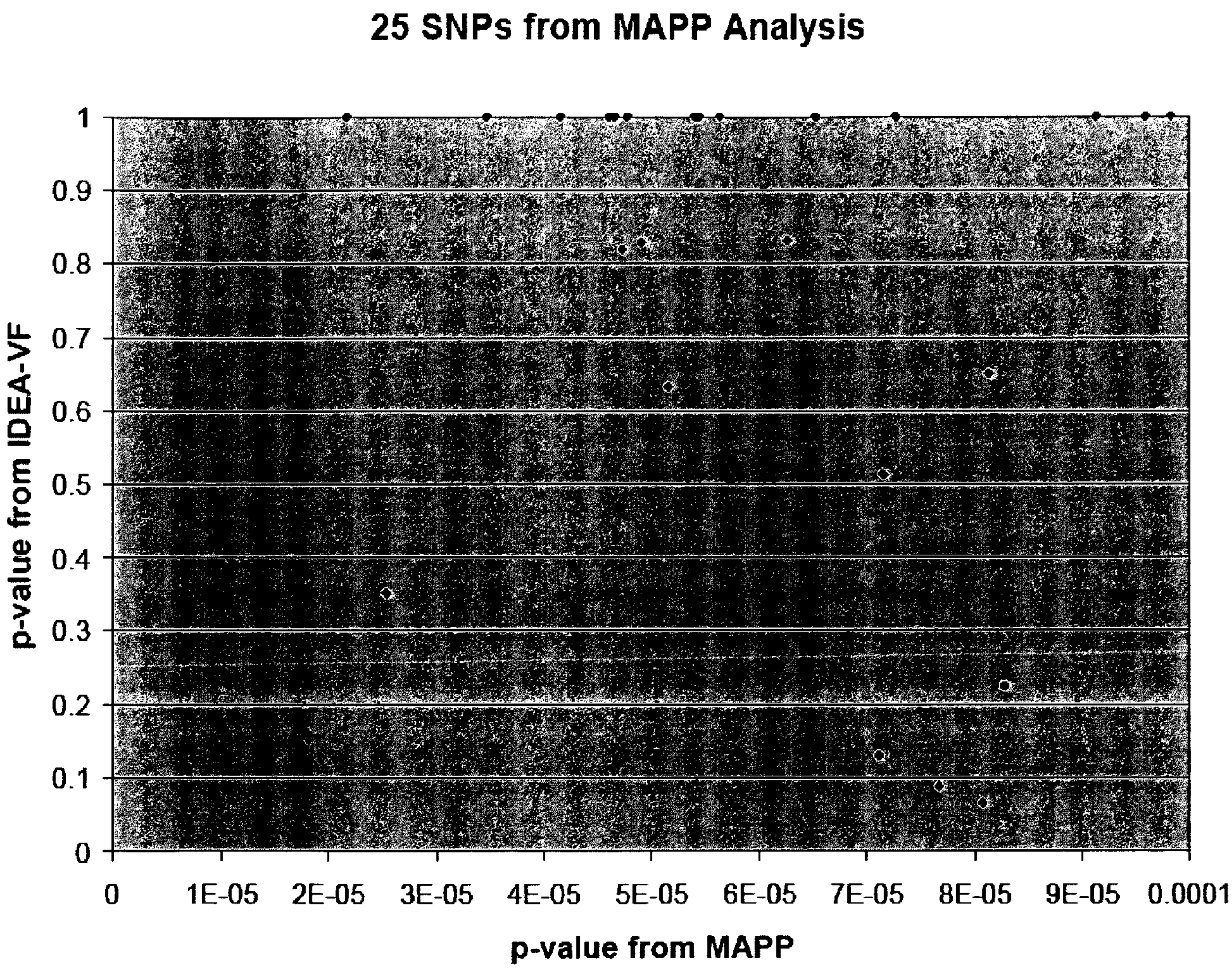
25 SNPs from MAPP Analysis
p-value from IDEA-VF
1
0.9
0.8
0.7
0.6
0.5
0.4
0.3
0.2
0.1
0
0
1E-05
2E-05
3E-05
4E-05
5E-05
6E-05
7E-05
8E-05
9E-05
0.0001
p-value from MAPP

Fig. 9

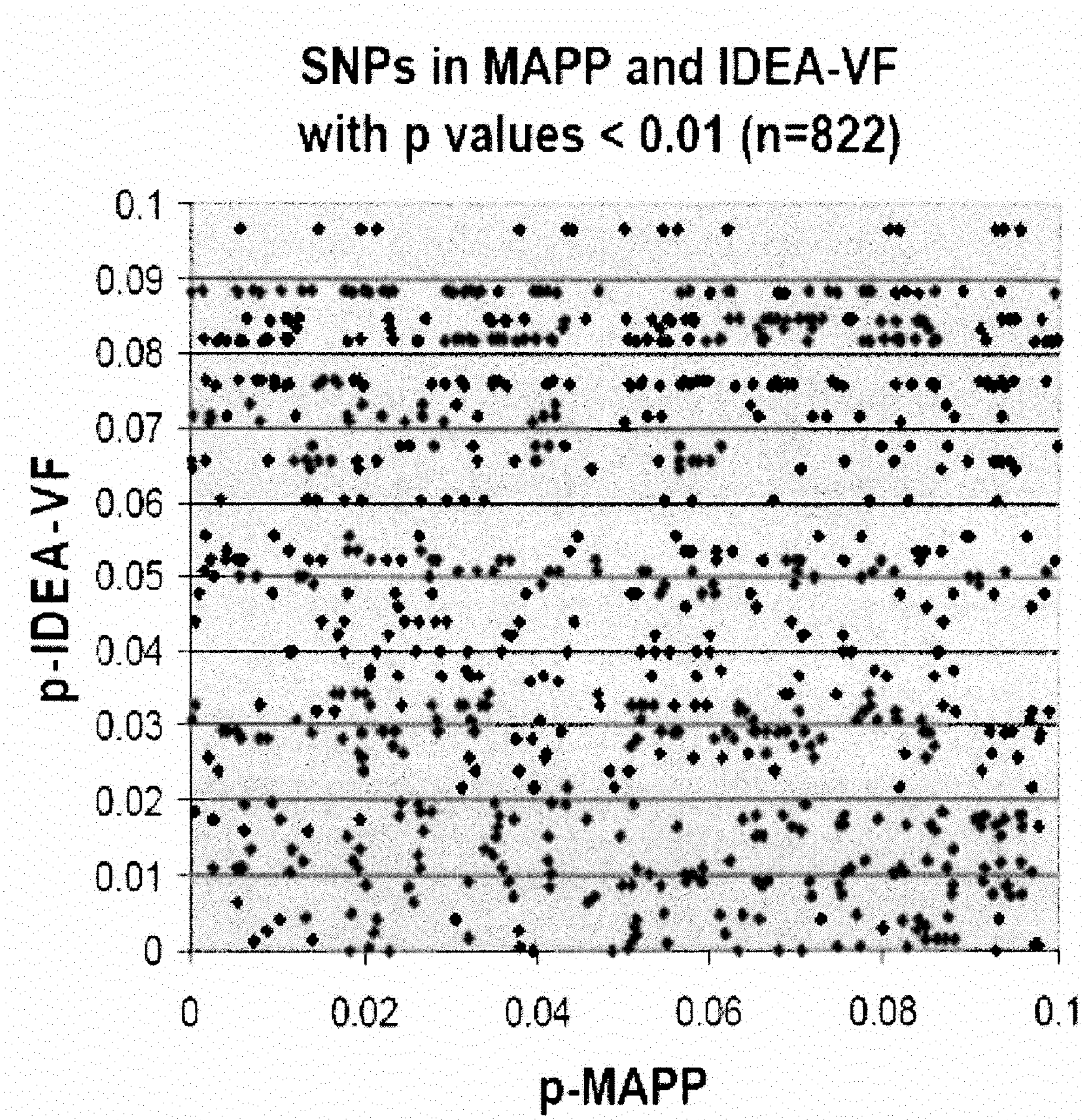
SNPs in MAPP and IDEA-VF
with p values < 0.01 (n=822)
0.1
0.09
0.08
0.07
0.06
0.05
0.04
0.03
0.02
0.01
0
p-IDEA-VF
0
0.02
0.04
0.06
0.08
0.1
p-MAPP

Fig. 10

# Fig. 11

Table of SEQ ID NO. and rs numbers

| rs no. | SEQIDNO. |
|---|---|
| rs12861247 | 1 |
| rs12558527 | 2 |
| rs12556578 | 3 |
| rs12388064 | 4 |
| rs10521726 | 5 |
| rs9803530 | 6 |
| rs7891488 | 7 |
| rs7882590 | 8 |
| rs7877387 | 9 |
| rs6649680 | 10 |
| rs6649251 | 11 |
| rs6633902 | 12 |
| rs6529997 | 13 |
| rs6525877 | 14 |
| rs6418096 | 15 |
| rs5988003 | 16 |
| rs5974731 | 17 |
| rs5967664 | 18 |
| rs5962157 | 19 |
| rs5961385 | 20 |
| rs5958298 | 21 |
| rs5958032 | 22 |
| rs5935513 | 23 |
| rs5935253 | 24 |
| rs5925077 | 25 |
| rs5923408 | 26 |
| rs5917614 | 27 |
| rs5916139 | 28 |
| rs5916138 | 29 |
| rs5911158 | 30 |
| rs5908645 | 31 |
| rs5908269 | 32 |
| rs5904750 | 33 |
| rs4830958 | 34 |
| rs4830955 | 35 |
| rs4830487 | 36 |
| rs4829348 | 37 |
| rs4520323 | 38 |
| rs4327974 | 39 |
| rs no | SEQIDNO. |
| rs4316308 | 40 |
| rs3860290 | 41 |
| rs3827440 | 42 |
| rs3788941 | 43 |
| rs2961430 | 44 |
| rs2411976 | 45 |
| rs2411975 | 46 |
| rs2317512 | 47 |
| rs2239473 | 48 |
| rs2211472 | 49 |
| rs2182289 | 50 |
| rs2157372 | 51 |
| rs2143844 | 52 |
| rs2103520 | 53 |
| rs2038193 | 54 |
| rs2018094 | 55 |
| rs1997630 | 56 |
| rs1986391 | 57 |
| rs1567894 | 58 |
| rs1565610 | 59 |
| rs1527536 | 60 |
| rs1503195 | 61 |
| rs1465067 | 62 |
| rs1458371 | 63 |
| rs1318655 | 64 |
| rs1206610 | 65 |
| rs1018368 | 66 |
| rs976845 | 67 |
| rs859941 | 68 |
| rs808576 | 69 |
| rs198780 | 70 |
| rs14312 | 71 |
| rs9620587 | 72 |
| rs3788329 | 73 |
| rs1894706 | 74 |
| rs1894704 | 75 |
| rs1001294 | 76 |
| rs738180 | 77 |
| rs713900 | 78 |
| rs no. | SEQIDNO. |
| rs133295 | 79 |
| rs9978739 | 80 |
| rs7275311 | 81 |
| rs2839347 | 82 |
| rs2839343 | 83 |
| rs2837851 | 84 |
| rs2830400 | 85 |
| rs2274808 | 86 |
| rs2238702 | 87 |
| rs2096509 | 88 |
| rs1984020 | 89 |
| rs1541103 | 90 |
| rs392840 | 91 |
| rs370850 | 92 |
| rs204740 | 93 |
| rs10485600 | 94 |
| rs8119972 | 95 |
| rs7262069 | 96 |
| rs7260918 | 97 |
| rs6073555 | 98 |
| rs6070373 | 99 |
| rs6069921 | 100 |
| rs6046403 | 101 |
| rs6031454 | 102 |
| rs4299400 | 103 |
| rs2424455 | 104 |
| rs1590202 | 105 |
| rs1004269 | 106 |
| rs880170 | 107 |
| rs869220 | 108 |
| rs10416550 | 109 |
| rs10415312 | 110 |
| rs10403334 | 111 |
| rs8113086 | 112 |
| rs8110654 | 113 |
| rs6509196 | 114 |
| rs4150992 | 115 |
| rs2303164 | 116 |
| rs2240887 | 117 |
| rs no. | SEQIDNO. |
| rs2195956 | 118 |
| rs1017205 | 119 |
| rs887029 | 120 |
| rs814528 | 121 |
| rs675136 | 122 |
| rs383216 | 123 |
| rs324125 | 124 |
| rs324121 | 125 |
| rs16949425 | 126 |
| rs12606960 | 127 |
| rs12456839 | 128 |
| rs11661310 | 129 |
| rs11564361 | 130 |
| rs11564355 | 131 |
| rs11082159 | 132 |
| rs10853232 | 133 |
| rs9962727 | 134 |
| rs9952567 | 135 |
| rs7350986 | 136 |
| rs7350983 | 137 |
| rs7244506 | 138 |
| rs7239567 | 139 |
| rs5026446 | 140 |
| rs4544324 | 141 |
| rs4456603 | 142 |
| rs4129316 | 143 |
| rs2852143 | 144 |
| rs2588844 | 145 |
| rs2430894 | 146 |
| rs2051301 | 147 |
| rs1893259 | 148 |
| rs1657385 | 149 |
| rs1657382 | 150 |
| rs1484700 | 151 |
| rs1352516 | 152 |

| | | | | | | | |
|---|---|---|---|---|---|---|---|
| rs1077388 | 153 | rs2210 | 203 | rs1950268 | 253 | rs4556627 | 303 |
| rs917673 | 154 | rs17441859 | 204 | rs1892555 | 254 | rs4244071 | 304 |
| rs752908 | 155 | rs12908846 | 205 | rs1886505 | 255 | rs4129443 | 305 |
| rs635488 | 156 | rs11073678 | 206 | rs1261795 | 256 | rs4083221 | 306 |
| rs584162 | 157 | rs10519131 | 207 | rs848692 | 257 | rs2839775 | 307 |
| rs12452861 | 158 | rs8041437 | 208 | rs807331 | 258 | rs2300719 | 308 |
| rs11871449 | 159 | rs8037430 | 209 | rs17189726 | 259 | rs2270151 | 309 |
| rs9911349 | 160 | rs8037172 | 210 | rs12876596 | 260 | rs1994283 | 310 |
| rs8079174 | 161 | rs8035983 | 211 | rs10508050 | 261 | rs1994090 | 311 |
| rs8074649 | 162 | rs8028632 | 212 | rs9563481 | 262 | rs1658693 | 312 |
| rs7223099 | 163 | rs4984479 | 213 | rs9520396 | 263 | rs1350515 | 313 |
| rs7220132 | 164 | rs4965520 | 214 | rs9301376 | 264 | rs1215765 | 314 |
| rs6503841 | 165 | rs4965238 | 215 | rs7339414 | 265 | rs1039302 | 315 |
| rs3744137 | 166 | rs4843143 | 216 | rs7338552 | 266 | rs939876 | 316 |
| rs3110496 | 167 | rs4777039 | 217 | rs4941887 | 267 | rs924417 | 317 |
| rs2945399 | 168 | rs4491457 | 218 | rs4907732 | 268 | rs904075 | 318 |
| rs2871647 | 169 | rs3957526 | 219 | rs4886014 | 269 | rs837473 | 319 |
| rs2526535 | 170 | rs3743123 | 220 | rs4771332 | 270 | rs733180 | 320 |
| rs2302762 | 171 | rs2957370 | 221 | rs1361206 | 271 | rs725399 | 321 |
| rs2165846 | 172 | rs2597909 | 222 | rs1334168 | 272 | rs724201 | 322 |
| rs1715489 | 173 | rs2570817 | 223 | rs983130 | 273 | rs708188 | 323 |
| rs1564821 | 174 | rs2460842 | 224 | rs912435 | 274 | rs640755 | 324 |
| rs7946 | 175 | rs2304900 | 225 | rs813328 | 275 | rs397496 | 325 |
| rs16947192 | 176 | rs1879894 | 226 | rs630943 | 276 | rs287024 | 326 |
| rs12918181 | 177 | rs1372347 | 227 | rs418543 | 277 | rs287016 | 327 |
| rs9931258 | 178 | rs999449 | 228 | rs391678 | 278 | rs242003 | 328 |
| rs9925917 | 179 | rs713469 | 229 | rs275946 | 279 | rs220599 | 329 |
| rs8061082 | 180 | rs17531821 | 230 | rs12298405 | 280 | rs177298 | 330 |
| rs8048495 | 181 | rs12881439 | 231 | rs11614358 | 281 | rs16934689 | 331 |
| rs8047991 | 182 | rs11629182 | 232 | rs11180765 | 282 | rs12792262 | 332 |
| rs7192812 | 183 | rs11624431 | 233 | rs11170657 | 283 | rs11035719 | 333 |
| rs6540246 | 184 | rs10498644 | 234 | rs10876488 | 284 | rs11030106 | 334 |
| rs6501048 | 185 | rs10483366 | 235 | rs10861034 | 285 | rs11024449 | 335 |
| rs4889180 | 186 | rs10146784 | 236 | rs10846448 | 286 | rs10502079 | 336 |
| rs4889176 | 187 | rs10139234 | 237 | rs10773594 | 287 | rs9667864 | 337 |
| rs4785755 | 188 | rs8009231 | 238 | rs10746116 | 288 | rs7946766 | 338 |
| rs4785426 | 189 | rs8004273 | 239 | rs10506676 | 289 | rs7934354 | 339 |
| rs2159318 | 190 | rs7493192 | 240 | rs10506596 | 290 | rs7122009 | 340 |
| rs2078548 | 191 | rs7154732 | 241 | rs7980489 | 291 | rs7121901 | 341 |
| rs1948073 | 192 | rs7147797 | 242 | rs7972005 | 292 | rs7120737 | 342 |
| rs1872667 | 193 | rs7146722 | 243 | rs7957728 | 293 | rs7119152 | 343 |
| rs1544616 | 194 | rs7140212 | 244 | rs7304711 | 294 | rs7116632 | 344 |
| rs1540613 | 195 | rs4445834 | 245 | rs7138775 | 295 | rs6578748 | 345 |
| rs1540610 | 196 | rs4403996 | 246 | rs6582285 | 296 | rs4755844 | 346 |
| rs1518603 | 197 | rs3742480 | 247 | rs6538408 | 297 | rs4752791 | 347 |
| rs1424104 | 198 | rs3007033 | 248 | rs5745066 | 298 | rs4556497 | 348 |
| rs708262 | 199 | rs2332914 | 249 | rs4913391 | 299 | rs3758740 | 349 |
| rs235799 | 200 | rs2224439 | 250 | rs4765028 | 300 | rs2647582 | 350 |
| rs181694 | 201 | rs2000327 | 251 | rs4761528 | 301 | rs2631229 | 351 |
| rs11823 | 202 | rs1953263 | 252 | rs4759732 | 302 | rs2304731 | 352 |

| | | | | | | | |
|---|---|---|---|---|---|---|---|
| rs2246649 | 353 | rs1247451 | 403 | rs628873 | 453 | rs413667 | 503 |
| rs2242144 | 354 | rs1171837 | 404 | rs553653 | 454 | rs180204 | 504 |
| rs2155387 | 355 | rs877748 | 405 | rs407179 | 455 | rs12666315 | 505 |
| rs2060009 | 356 | rs814620 | 406 | rs13279994 | 456 | rs11765962 | 506 |
| rs1909260 | 357 | rs239863 | 407 | rs13272985 | 457 | rs11764339 | 507 |
| rs1583443 | 358 | rs13294002 | 408 | rs12550783 | 458 | rs10277213 | 508 |
| rs1474056 | 359 | rs12341391 | 409 | rs12549803 | 459 | rs10246707 | 509 |
| rs1455264 | 360 | rs11791472 | 410 | rs12543841 | 460 | rs10242397 | 510 |
| rs1455251 | 361 | rs11243406 | 411 | rs11995187 | 461 | rs10238918 | 511 |
| rs1391613 | 362 | rs10983571 | 412 | rs10958798 | 462 | rs7801603 | 512 |
| rs1391612 | 363 | rs10982585 | 413 | rs10755897 | 463 | rs7785760 | 513 |
| rs931859 | 364 | rs10974530 | 414 | rs10504794 | 464 | rs7778311 | 514 |
| rs906528 | 365 | rs10972872 | 415 | rs10102788 | 465 | rs6974082 | 515 |
| rs662199 | 366 | rs10965597 | 416 | rs10099034 | 466 | rs6965038 | 516 |
| rs601904 | 367 | rs10964155 | 417 | rs10097563 | 467 | rs6949236 | 517 |
| rs589149 | 368 | rs10820441 | 418 | rs10085952 | 468 | rs6467917 | 518 |
| rs579687 | 369 | rs10818161 | 419 | rs7833003 | 469 | rs5745709 | 519 |
| rs541821 | 370 | rs10809523 | 420 | rs7818404 | 470 | rs4725617 | 520 |
| rs12784234 | 371 | rs10780770 | 421 | rs7818051 | 471 | rs4719155 | 521 |
| rs12775410 | 372 | rs10760793 | 422 | rs7010127 | 472 | rs4629773 | 522 |
| rs11511683 | 373 | rs10511470 | 423 | rs7000981 | 473 | rs4621704 | 523 |
| rs11201011 | 374 | rs10491952 | 424 | rs7000815 | 474 | rs4265116 | 524 |
| rs11198846 | 375 | rs7866610 | 425 | rs6999664 | 475 | rs3113173 | 525 |
| rs11013998 | 376 | rs7859758 | 426 | rs6991834 | 476 | rs2734189 | 526 |
| rs11008264 | 377 | rs7853023 | 427 | rs6989793 | 477 | rs2717351 | 527 |
| rs10828726 | 378 | rs7851794 | 428 | rs6989593 | 478 | rs2353815 | 528 |
| rs10823406 | 379 | rs7045026 | 429 | rs6981869 | 479 | rs2237432 | 529 |
| rs9424099 | 380 | rs7029465 | 430 | rs6530854 | 480 | rs2214827 | 530 |
| rs7912419 | 381 | rs6477107 | 431 | rs6471335 | 481 | rs2214825 | 531 |
| rs7905024 | 382 | rs4838271 | 432 | rs6468154 | 482 | rs2107710 | 532 |
| rs7903424 | 383 | rs3829078 | 433 | rs4467944 | 483 | rs2072175 | 533 |
| rs7099403 | 384 | rs3780708 | 434 | rs4310223 | 484 | rs1734729 | 534 |
| rs7091141 | 385 | rs3750490 | 435 | rs2979715 | 485 | rs1439098 | 535 |
| rs4962653 | 386 | rs2997664 | 436 | rs2922066 | 486 | rs1362128 | 536 |
| rs4935225 | 387 | rs2796460 | 437 | rs2517105 | 487 | rs1075042 | 537 |
| rs4339947 | 388 | rs2381672 | 438 | rs2217827 | 488 | rs940597 | 538 |
| rs3740259 | 389 | rs2380906 | 439 | rs2169385 | 489 | rs205717 | 539 |
| rs3026720 | 390 | rs2026362 | 440 | rs1994469 | 490 | rs172310 | 540 |
| rs2459068 | 391 | rs1991624 | 441 | rs1909446 | 491 | rs8 | 541 |
| rs2393469 | 392 | rs1984007 | 442 | rs1835851 | 492 | rs17679624 | 542 |
| rs2388569 | 393 | rs1858095 | 443 | rs1566861 | 493 | rs16868805 | 543 |
| rs2243639 | 394 | rs1434250 | 444 | rs1542827 | 494 | rs12175530 | 544 |
| rs1910534 | 395 | rs1361117 | 445 | rs1506869 | 495 | rs11962675 | 545 |
| rs1904031 | 396 | rs1335420 | 446 | rs1481646 | 496 | rs11759651 | 546 |
| rs1875789 | 397 | rs1326800 | 447 | rs1386689 | 497 | rs10945462 | 547 |
| rs1833044 | 398 | rs1156793 | 448 | rs1347001 | 498 | rs9386934 | 548 |
| rs1773877 | 399 | rs1004604 | 449 | rs1075493 | 499 | rs9320884 | 549 |
| rs1751280 | 400 | rs958804 | 450 | rs883429 | 500 | rs9320475 | 550 |
| rs1751277 | 401 | rs943174 | 451 | rs725173 | 501 | rs7760851 | 551 |
| rs1475418 | 402 | rs735262 | 452 | rs717406 | 502 | rs7754676 | 552 |

| | | | | | | | |
|---|---|---|---|---|---|---|---|
| rs7744878 | 553 | rs2052482 | 603 | rs1587734 | 653 | rs792841 | 703 |
| rs7744524 | 554 | rs2048646 | 604 | rs1470645 | 654 | rs660075 | 704 |
| rs7741540 | 555 | rs1808380 | 605 | rs1459151 | 655 | rs547432 | 705 |
| rs6920474 | 556 | rs1547531 | 606 | rs1439376 | 656 | rs544704 | 706 |
| rs4479955 | 557 | rs1031006 | 607 | rs1039539 | 657 | rs496486 | 707 |
| rs3763215 | 558 | rs950864 | 608 | rs998460 | 658 | rs17005910 | 708 |
| rs3012519 | 559 | rs780188 | 609 | rs993380 | 659 | rs12618696 | 709 |
| rs2812152 | 560 | rs736998 | 610 | rs916874 | 660 | rs12618382 | 710 |
| rs2097130 | 561 | rs459131 | 611 | rs230526 | 661 | rs12617566 | 711 |
| rs1954925 | 562 | rs454886 | 612 | rs17331632 | 662 | rs12468086 | 712 |
| rs1954920 | 563 | rs350089 | 613 | rs13322750 | 663 | rs12467276 | 713 |
| rs1934124 | 564 | rs296277 | 614 | rs13319027 | 664 | rs12373663 | 714 |
| rs1329521 | 565 | rs295999 | 615 | rs13091270 | 665 | rs11883500 | 715 |
| rs1321582 | 566 | rs246486 | 616 | rs12628984 | 666 | rs10519034 | 716 |
| rs1211554 | 567 | rs17816553 | 617 | rs10865566 | 667 | rs10496450 | 717 |
| rs997607 | 568 | rs17343504 | 618 | rs10513805 | 668 | rs10496340 | 718 |
| rs857477 | 569 | rs12645274 | 619 | rs10510564 | 669 | rs10496220 | 719 |
| rs802682 | 570 | rs11930193 | 620 | rs10440133 | 670 | rs10180461 | 720 |
| rs713084 | 571 | rs11726463 | 621 | rs9876832 | 671 | rs7596894 | 721 |
| rs638540 | 572 | rs11721729 | 622 | rs9876789 | 672 | rs7583169 | 722 |
| rs628572 | 573 | rs11099852 | 623 | rs9875303 | 673 | rs7580162 | 723 |
| rs588067 | 574 | rs10938745 | 624 | rs9872799 | 674 | rs7565358 | 724 |
| rs384366 | 575 | rs10517901 | 625 | rs9850416 | 675 | rs7559811 | 725 |
| rs239832 | 576 | rs10006010 | 626 | rs9845033 | 676 | rs6759510 | 726 |
| rs17635284 | 577 | rs10003889 | 627 | rs9817739 | 677 | rs6716246 | 727 |
| rs16903629 | 578 | rs9790702 | 628 | rs7648625 | 678 | rs6712744 | 728 |
| rs16898178 | 579 | rs7691894 | 629 | rs7637944 | 679 | rs6546754 | 729 |
| rs13357969 | 580 | rs7689919 | 630 | rs7625409 | 680 | rs4854135 | 730 |
| rs12653539 | 581 | rs7675016 | 631 | rs7622789 | 681 | rs4851235 | 731 |
| rs12516171 | 582 | rs7664824 | 632 | rs7620956 | 682 | rs4674039 | 732 |
| rs11957867 | 583 | rs7660418 | 633 | rs7618693 | 683 | rs4344923 | 733 |
| rs11750519 | 584 | rs7376535 | 634 | rs7617041 | 684 | rs4335989 | 734 |
| rs10520944 | 585 | rs6832047 | 635 | rs6783129 | 685 | rs4286327 | 735 |
| rs10491495 | 586 | rs6824427 | 636 | rs6763419 | 686 | rs4240205 | 736 |
| rs10065788 | 587 | rs6817090 | 637 | rs6442164 | 687 | rs3917289 | 737 |
| rs9686533 | 588 | rs6534743 | 638 | rs4916425 | 688 | rs3821053 | 738 |
| rs7730091 | 589 | rs6448011 | 639 | rs4643650 | 689 | rs3751109 | 739 |
| rs7719763 | 590 | rs4975147 | 640 | rs4608697 | 690 | rs3751107 | 740 |
| rs7712871 | 591 | rs4861175 | 641 | rs4453850 | 691 | rs3749010 | 741 |
| rs7701642 | 592 | rs4835490 | 642 | rs2654417 | 692 | rs2890537 | 742 |
| rs7379403 | 593 | rs4699769 | 643 | rs1864895 | 693 | rs2689828 | 743 |
| rs6556865 | 594 | rs4699128 | 644 | rs1595489 | 694 | rs2617394 | 744 |
| rs6452027 | 595 | rs4540026 | 645 | rs1503161 | 695 | rs2588873 | 745 |
| rs4535467 | 596 | rs3822030 | 646 | rs1488171 | 696 | rs2435962 | 746 |
| rs3087980 | 597 | rs3755862 | 647 | rs1466123 | 697 | rs2321733 | 747 |
| rs2337130 | 598 | rs3021146 | 648 | rs1403719 | 698 | rs2292884 | 748 |
| rs2287749 | 599 | rs2305593 | 649 | rs1378796 | 699 | rs2049197 | 749 |
| rs2244541 | 600 | rs2298752 | 650 | rs1357086 | 700 | rs2037284 | 750 |
| rs2242223 | 601 | rs2139209 | 651 | rs1282540 | 701 | rs1980291 | 751 |
| rs2133764 | 602 | rs1598859 | 652 | rs977103 | 702 | rs1896284 | 752 |

| | | | | | | | |
|---|---|---|---|---|---|---|---|
| rs1568786 | 753 | rs10465632 | 782 | rs1065754 | 811 | rs7305353 | 840 |
| rs1559931 | 754 | rs7529979 | 783 | rs984779 | 812 | rs7591633 | 841 |
| rs1522823 | 755 | rs7414734 | 784 | rs904910 | 813 | rs9983892 | 842 |
| rs1429272 | 756 | rs6683502 | 785 | rs823673 | 814 | rs10487115 | 843 |
| rs1257200 | 757 | rs6657442 | 786 | rs730645 | 815 | rs10505726 | 844 |
| rs1233258 | 758 | rs6429822 | 787 | rs728864 | 816 | rs10823151 | 845 |
| rs1196152 | 759 | rs6429732 | 788 | rs542214 | 817 | rs10919336 | 846 |
| rs1036165 | 760 | rs4920513 | 789 | rs500586 | 818 | rs12891099 | 847 |
| rs1002016 | 761 | rs4658340 | 790 | rs488150 | 819 | rs16952330 | 848 |
| rs962052 | 762 | rs4656680 | 791 | rs359937 | 820 | rs17694397 | 849 |
| rs935480 | 763 | rs4653474 | 792 | rs241250 | 821 | rs5443 | 850 |
| rs935374 | 764 | rs4651286 | 793 | rs223198 | 822 | rs72466454 | 851 |
| rs843677 | 765 | rs4507975 | 794 | rs248670 | 823 | rs72466453 | 852 |
| rs746701 | 766 | rs4354529 | 795 | rs486427 | 824 | rs7121 | 853 |
| rs732227 | 767 | rs3813804 | 796 | rs564275 | 825 | rs6026584 | 854 |
| rs718159 | 768 | rs3790688 | 797 | rs1346964 | 826 | rs6123837 | 855 |
| rs521095 | 769 | rs3789433 | 798 | rs1500325 | 827 | rs12143842 | 856 |
| rs384526 | 770 | rs3761916 | 799 | rs1679414 | 828 | rs3864180 | 857 |
| rs359980 | 771 | rs2861630 | 800 | rs2060117 | 829 | no rs number | 858 |
| rs346831 | 772 | rs2802031 | 801 | rs2270801 | 830 | | |
| rs17028972 | 773 | rs2760494 | 802 | rs2716727 | 831 | | |
| rs12566190 | 774 | rs2740170 | 803 | rs2839372 | 832 | | |
| rs12563141 | 775 | rs2502826 | 804 | rs3775296 | 833 | | |
| rs12410385 | 776 | rs2481627 | 805 | rs4691391 | 834 | | |
| rs12121994 | 777 | rs2420506 | 806 | rs4878412 | 835 | | |
| rs12025142 | 778 | rs2378013 | 807 | rs6480311 | 836 | | |
| rs11165877 | 779 | rs2089432 | 808 | rs6790359 | 837 | | |
| rs10923673 | 780 | rs1357337 | 809 | rs6828580 | 838 | | |
| rs10797444 | 781 | rs1122816 | 810 | rs7241111 | 839 | | |

Fig. 12
Microarray Technology

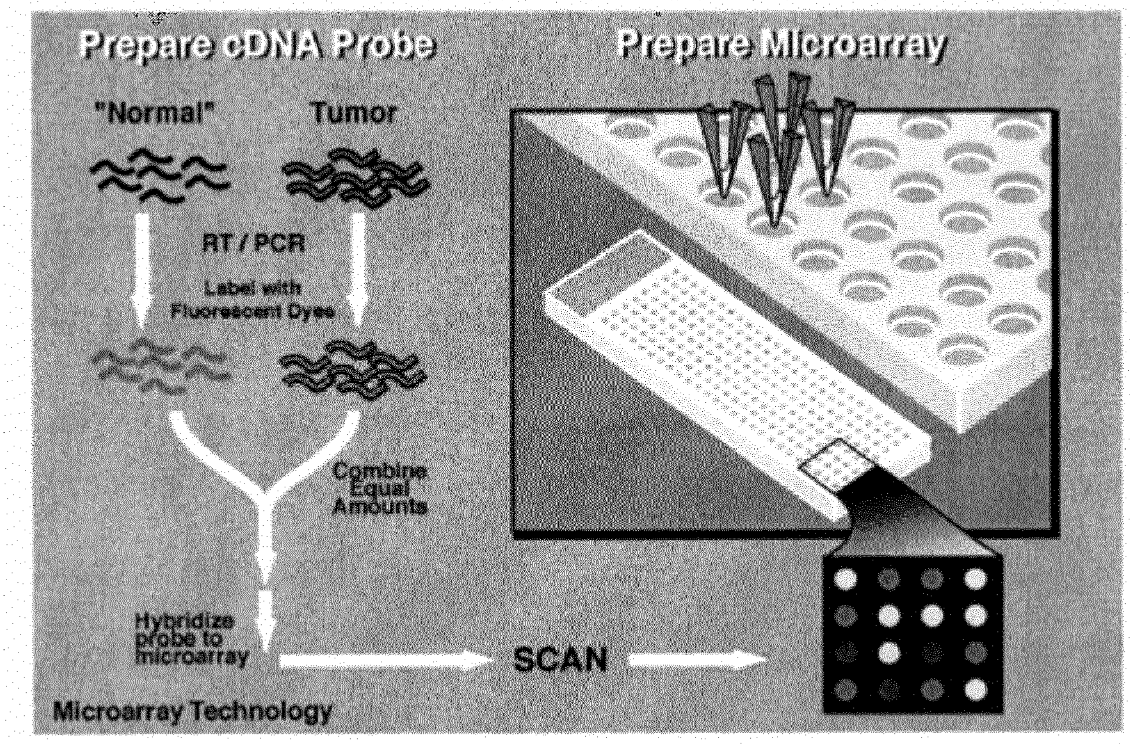

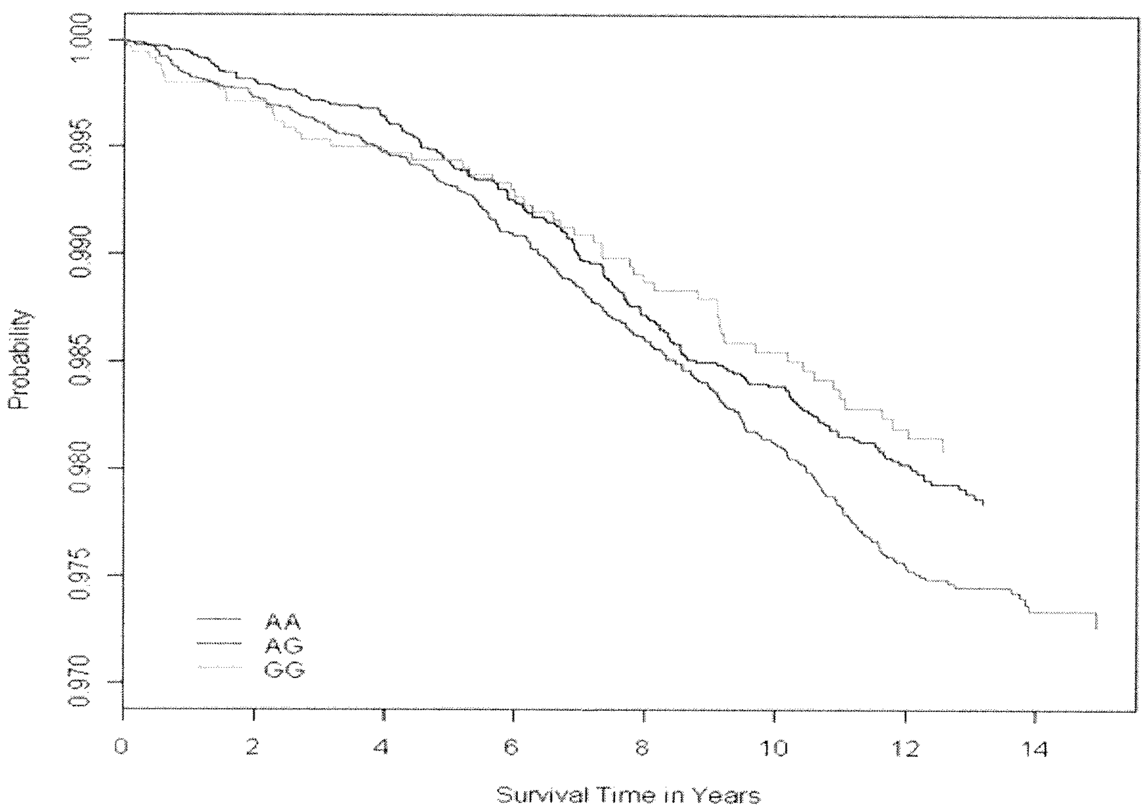
1.000
0.995
0.990
0.985
0.980
0.975
0.970
Probability
AA
AG
GG
0
2
4
6
8
10
12
14
Survival Time in Years

Fig. 13

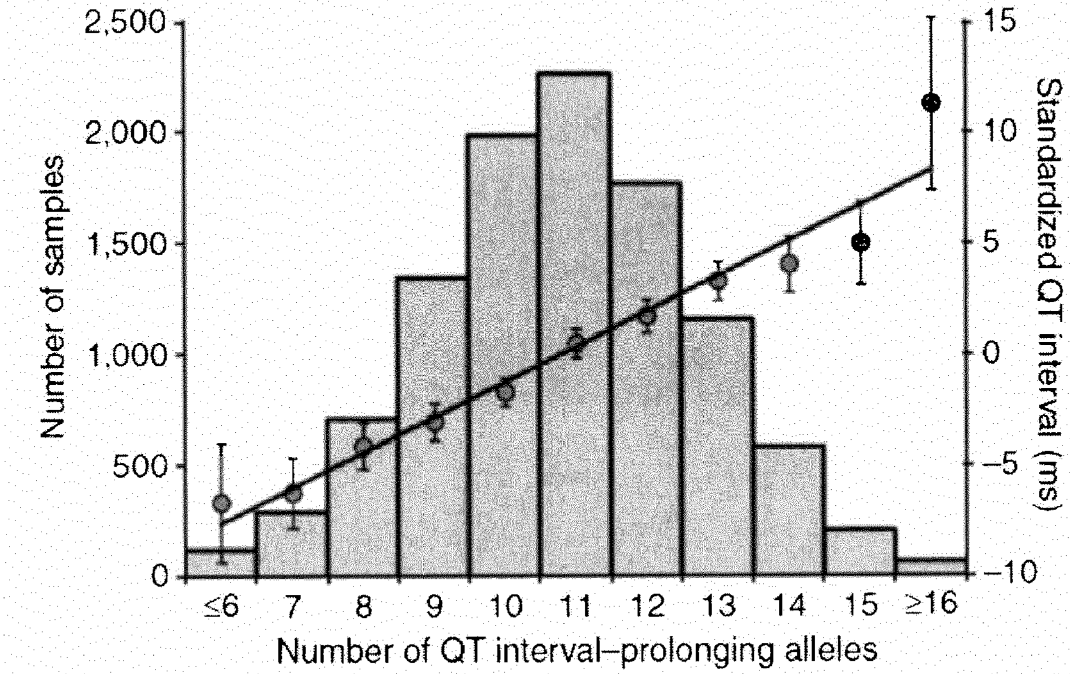
2,500
2,000
1,500
1,000
500
0
Number of samples
15
10
5
0
−5
−10
Standardized QT interval (ms)
≤6
7
8
9
10
11
12
13
14
15
≥16
Number of QT interval–prolonging alleles

Fig. 14

The NCBI dbSNP data model.

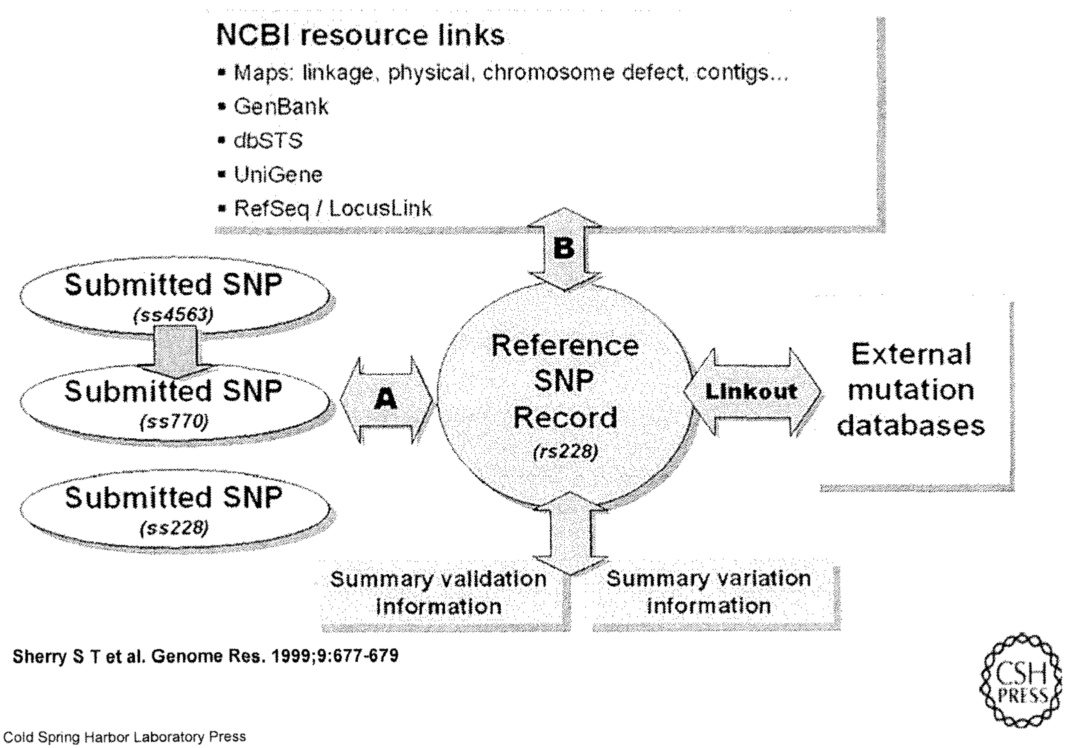


Sherry S T et al. Genome Res. 1999;9:677-679

©1999 by Cold Spring Harbor Laboratory Press

Fig. 15

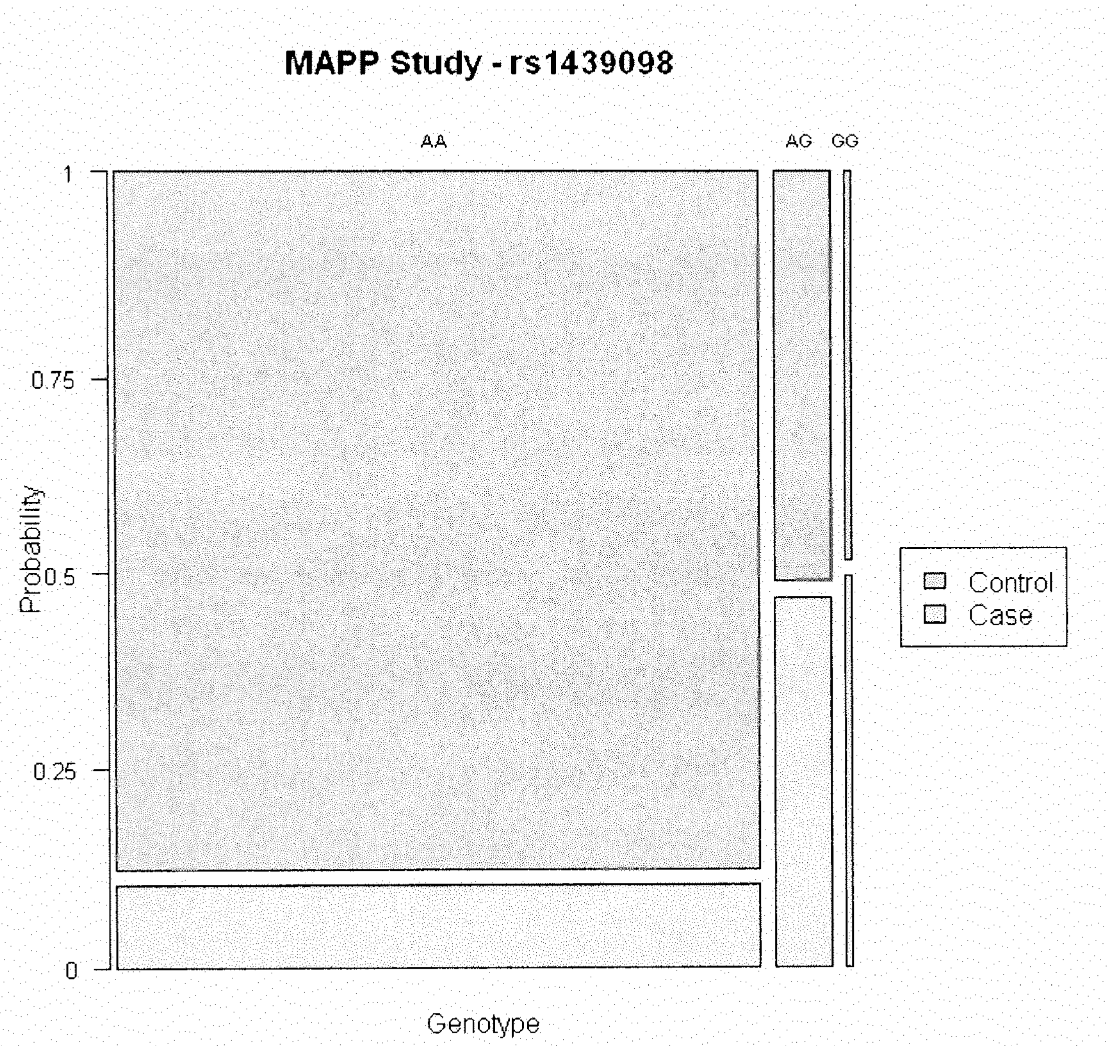
MAPP Study - rs1439098
AA
AG
GG
1
0.75
0.5
0.25
0
Probability
Genotype
Control
Case

Fig. 16

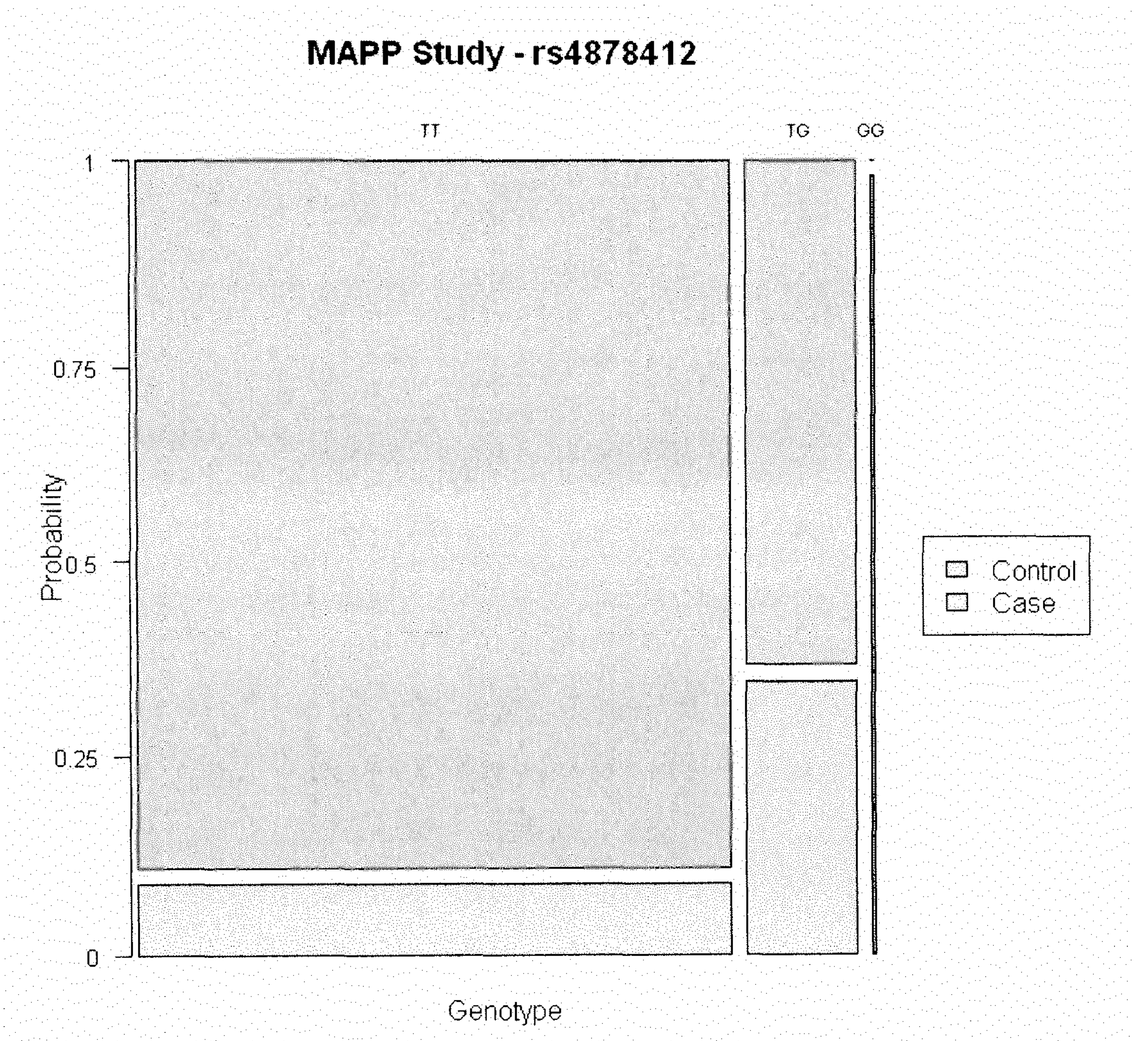
MAPP Study - rs4878412
TT
TG
GG
1
0.75
0.5
0.25
0
Probability
Genotype
Control
Case

Fig. 17

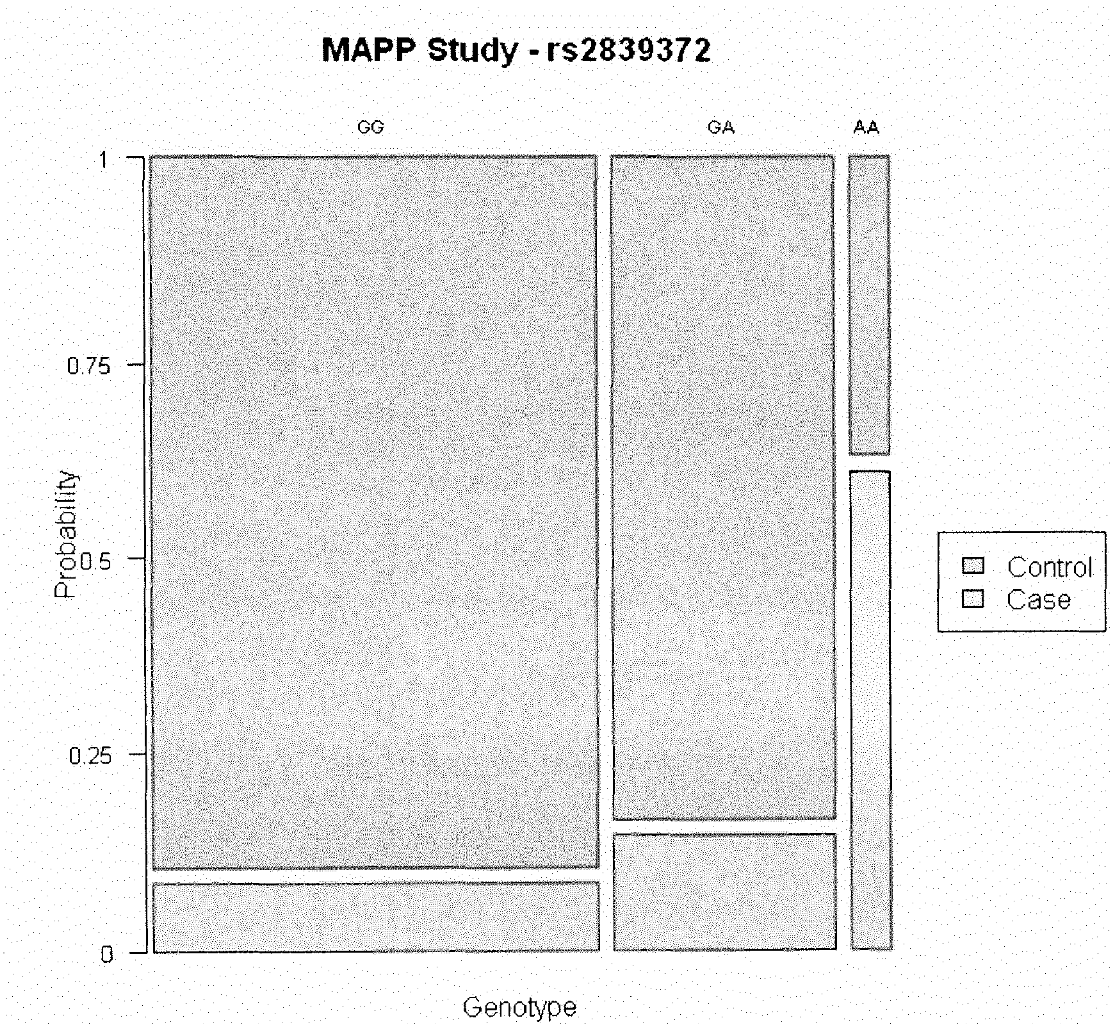
MAPP Study - rs2839372
GG
GA
AA
1
0.75
0.5
0.25
0
Probability
Genotype
Control
Case

Fig. 18

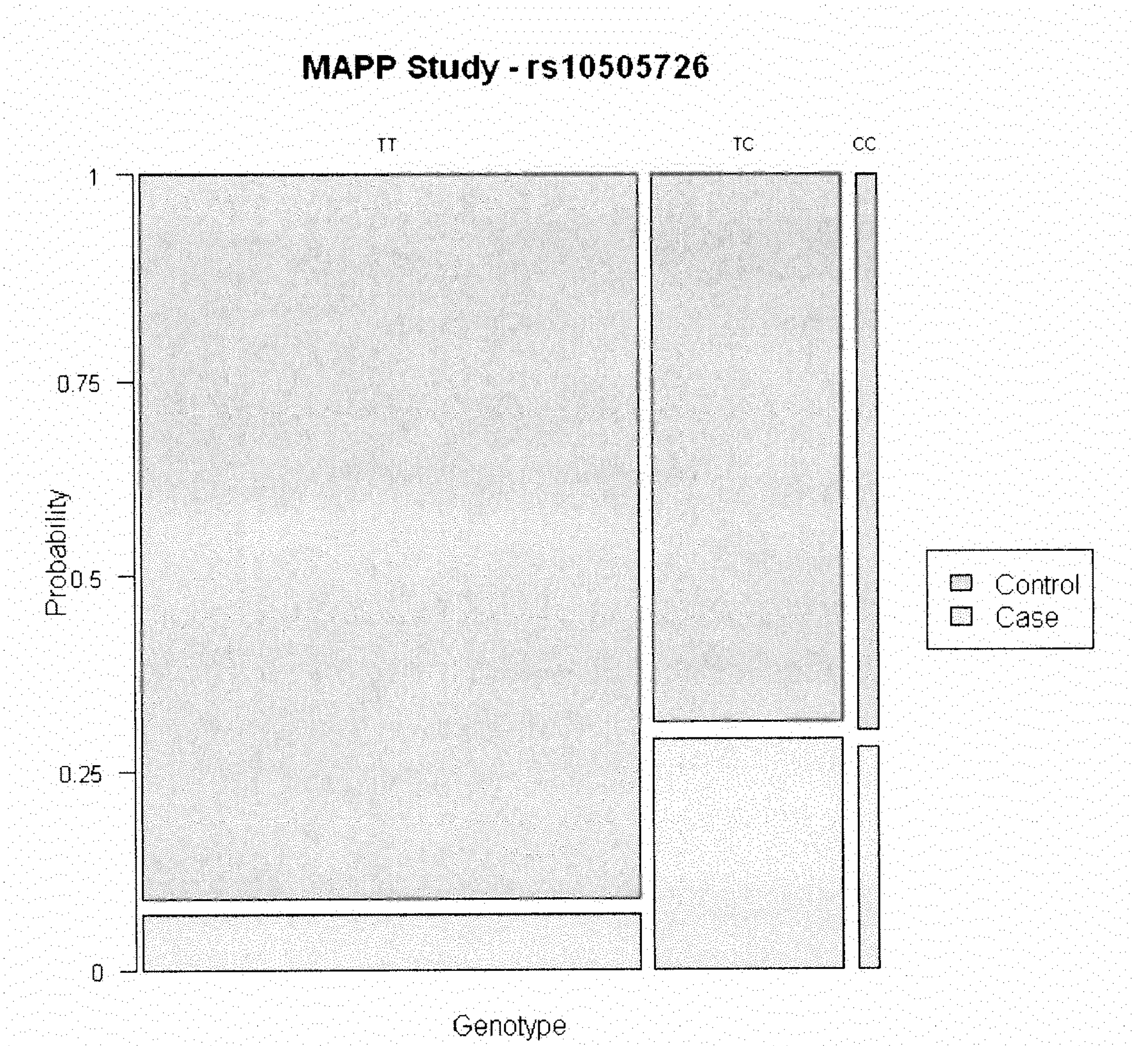
MAPP Study - rs10505726
TT
TC
CC
1
0.75
0.5
0.25
0
Probability
Genotype
Control
Case

Fig. 19

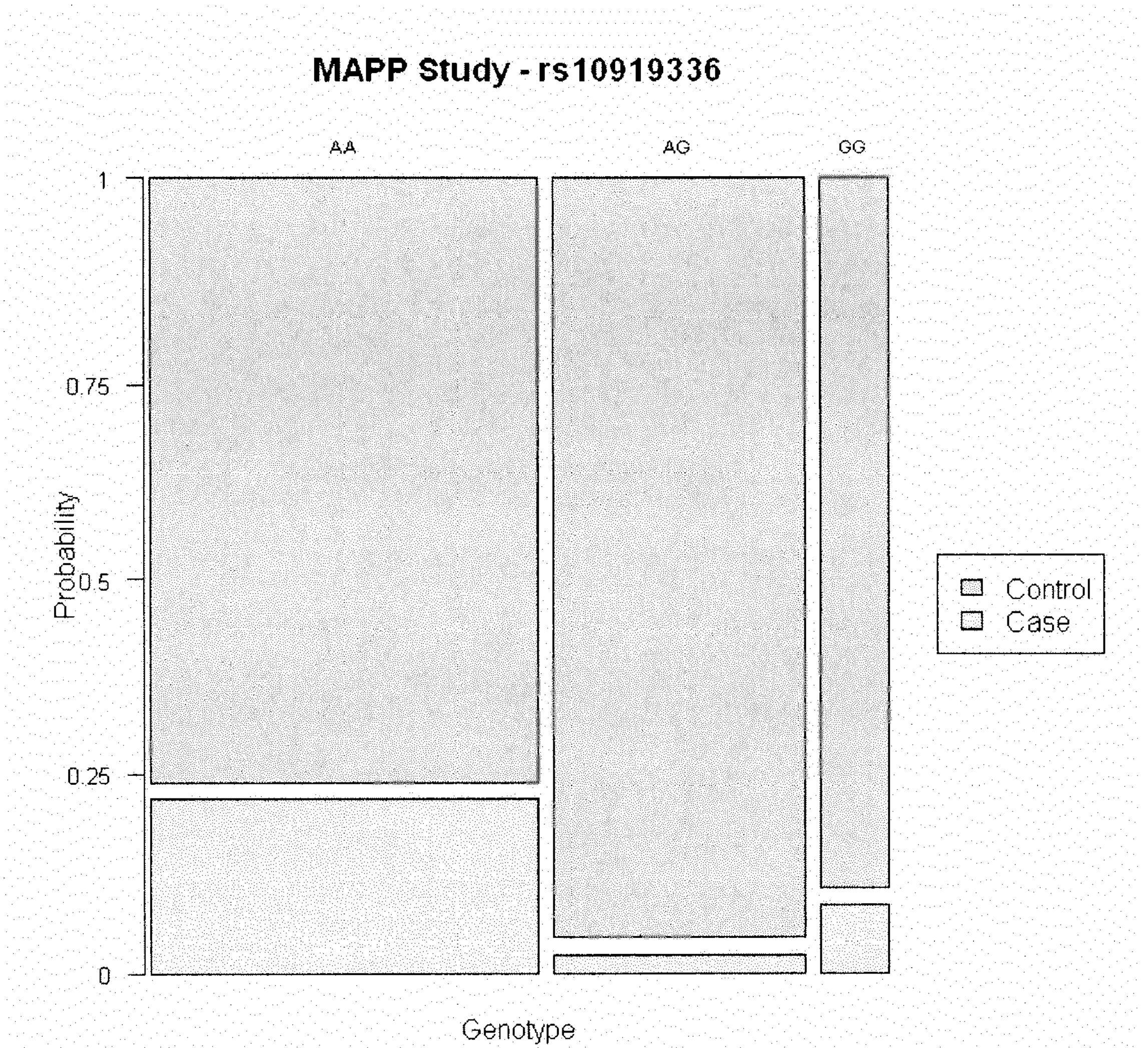
MAPP Study - rs10919336
AA
AG
GG
1
0.75
0.5
0.25
0
Probability
Genotype
Control
Case

Fig. 20

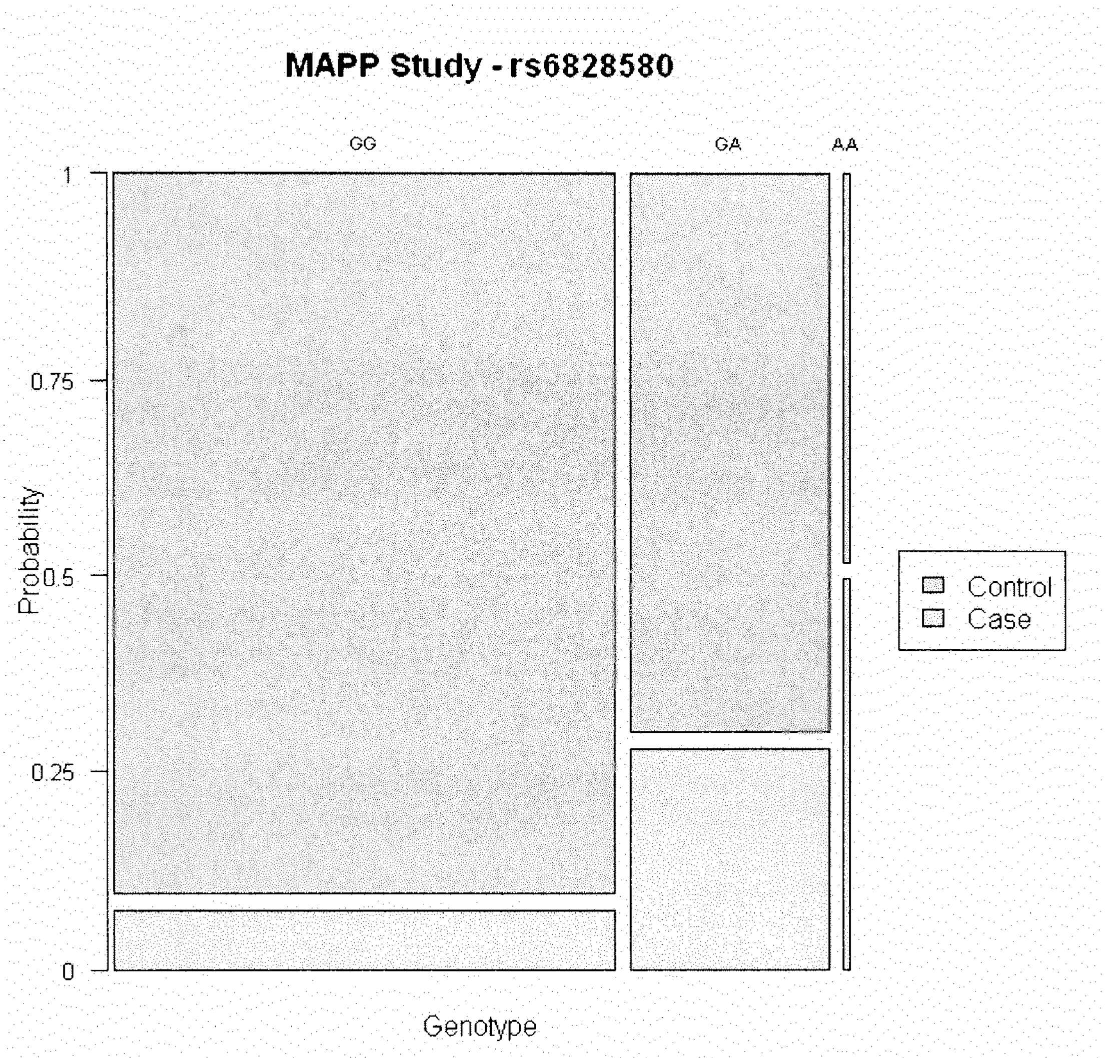
MAPP Study - rs6828580
GG
GA
AA
1
0.75
0.5
0.25
0
Probability
Genotype
Control
Case

Fig. 21

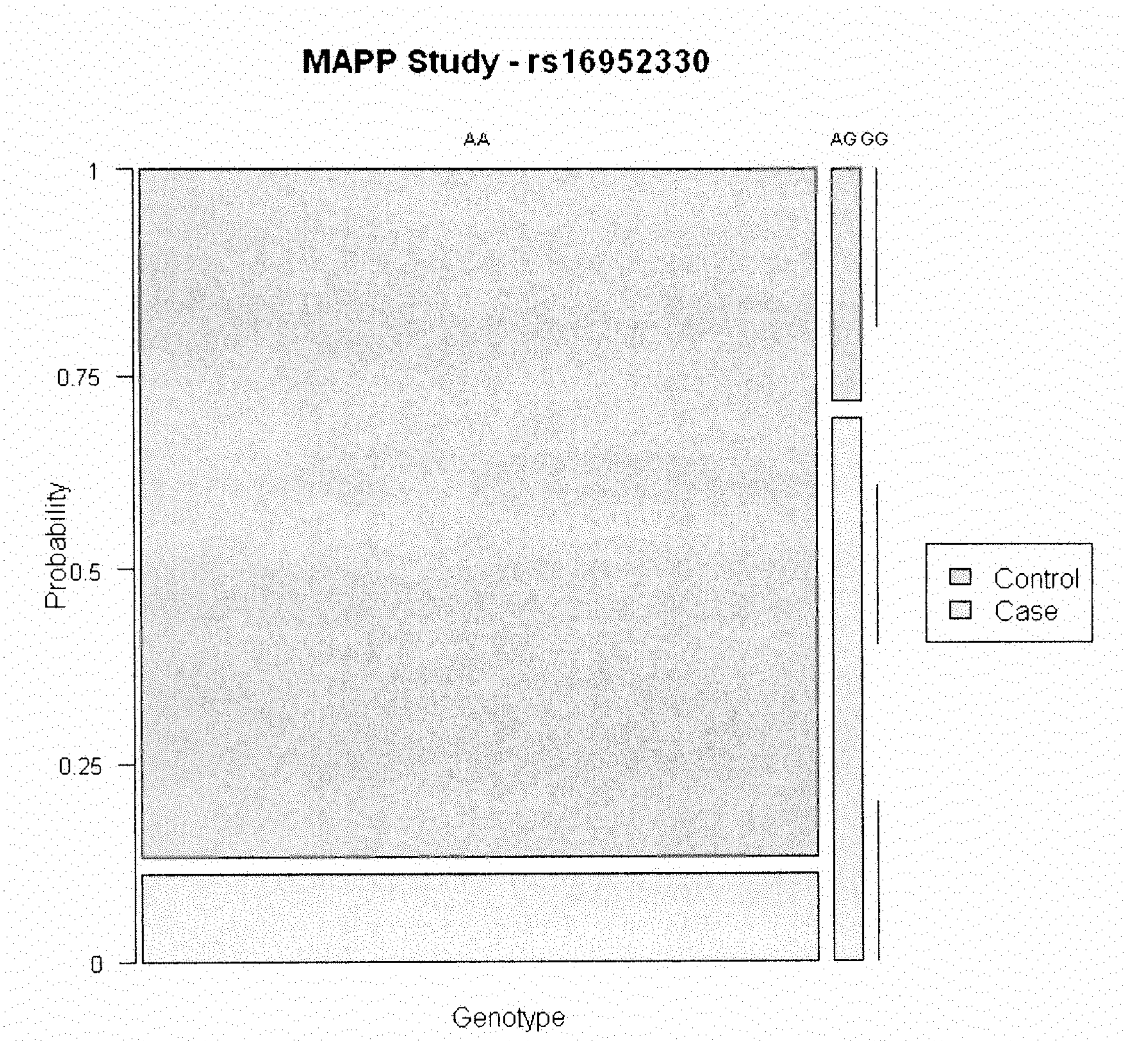
MAPP Study - rs16952330
AA
AG GG
1
0.75
0.5
0.25
0
Probability
Genotype
Control
Case

Fig. 22

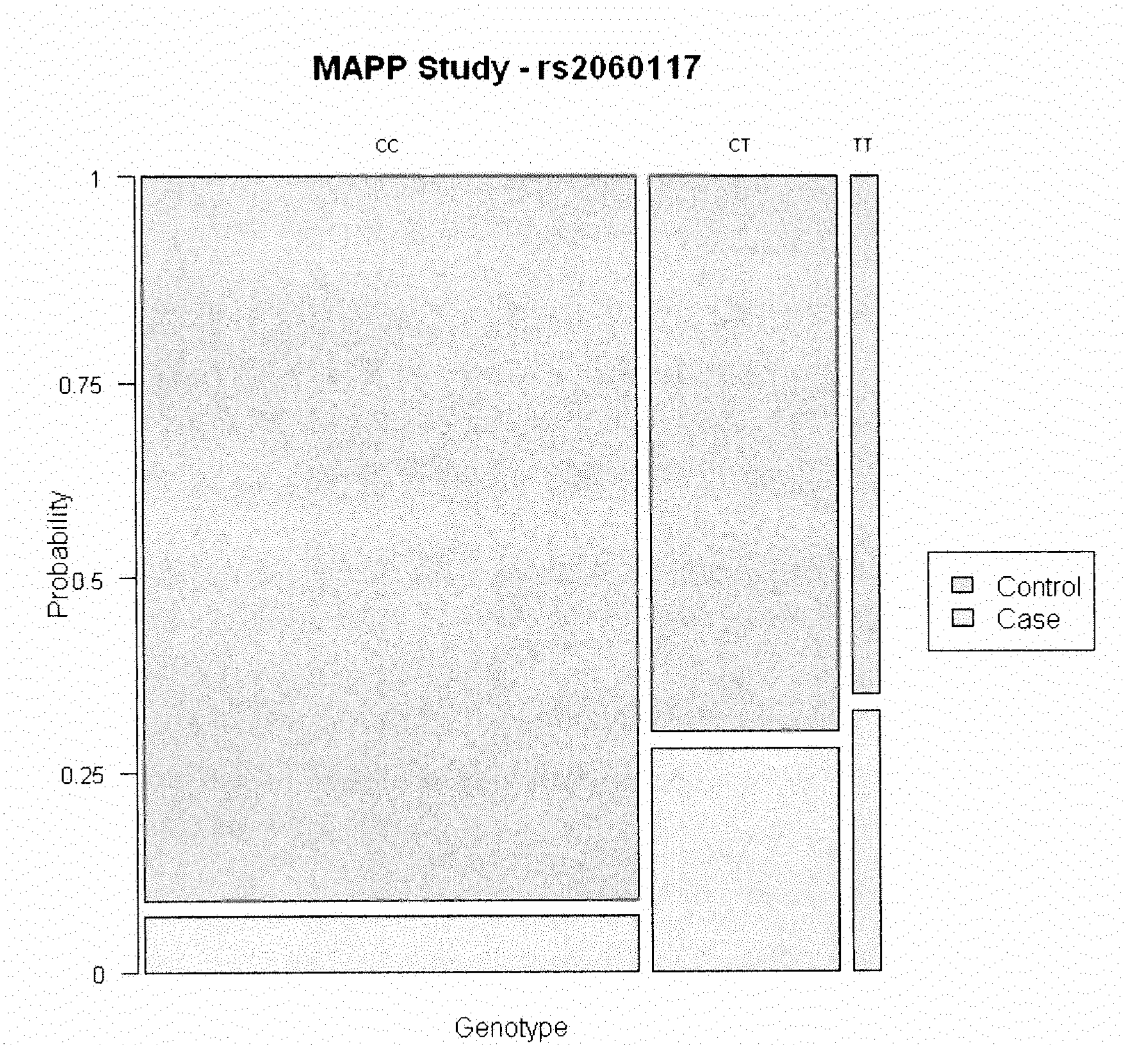
MAPP Study - rs2060117
CC
CT
TT
1
0.75
0.5
0.25
0
Probability
Genotype
Control
Case

Fig. 23

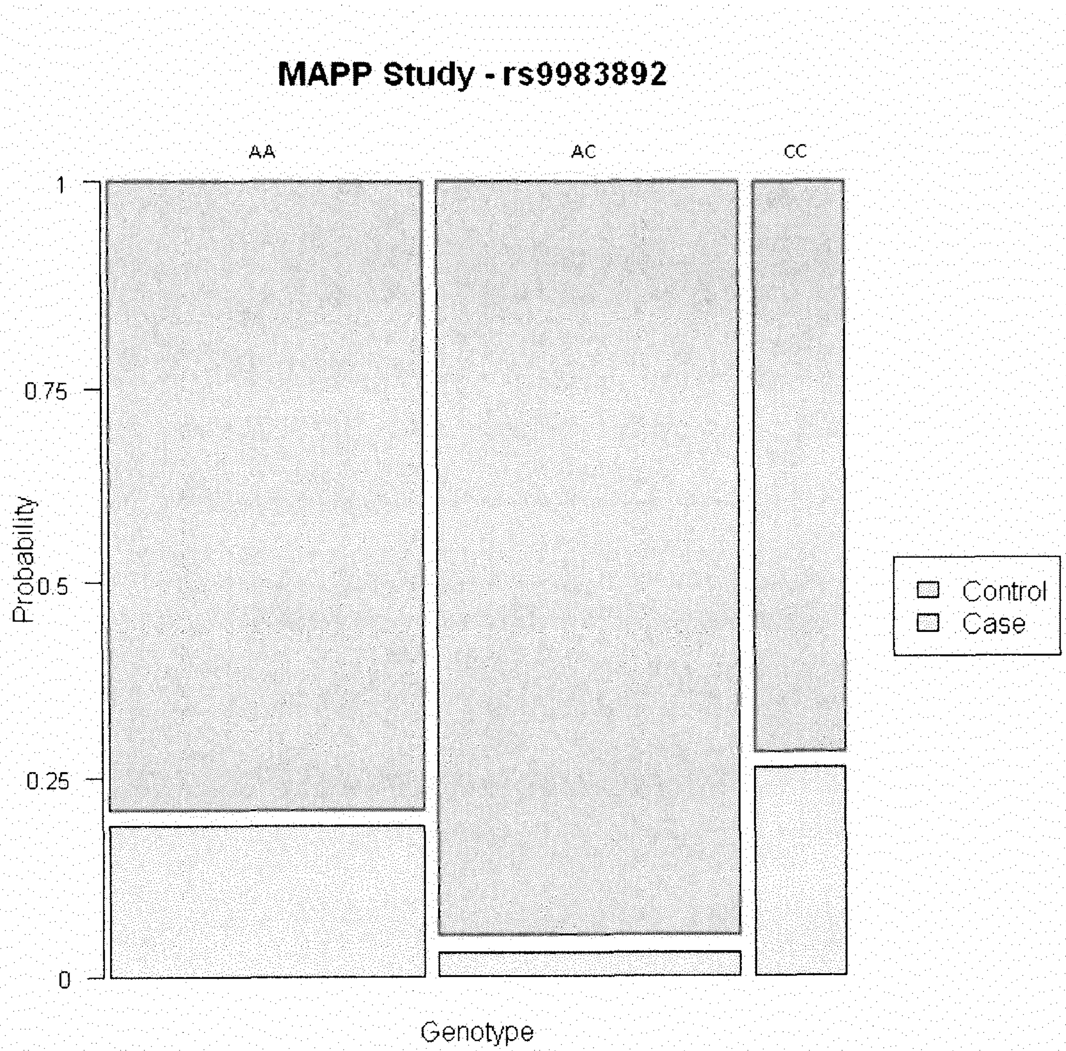
MAPP Study - rs9983892
AA
AC
CC
1
0.75
0.5
0.25
0
Probability
Genotype
Control
Case

Fig. 24

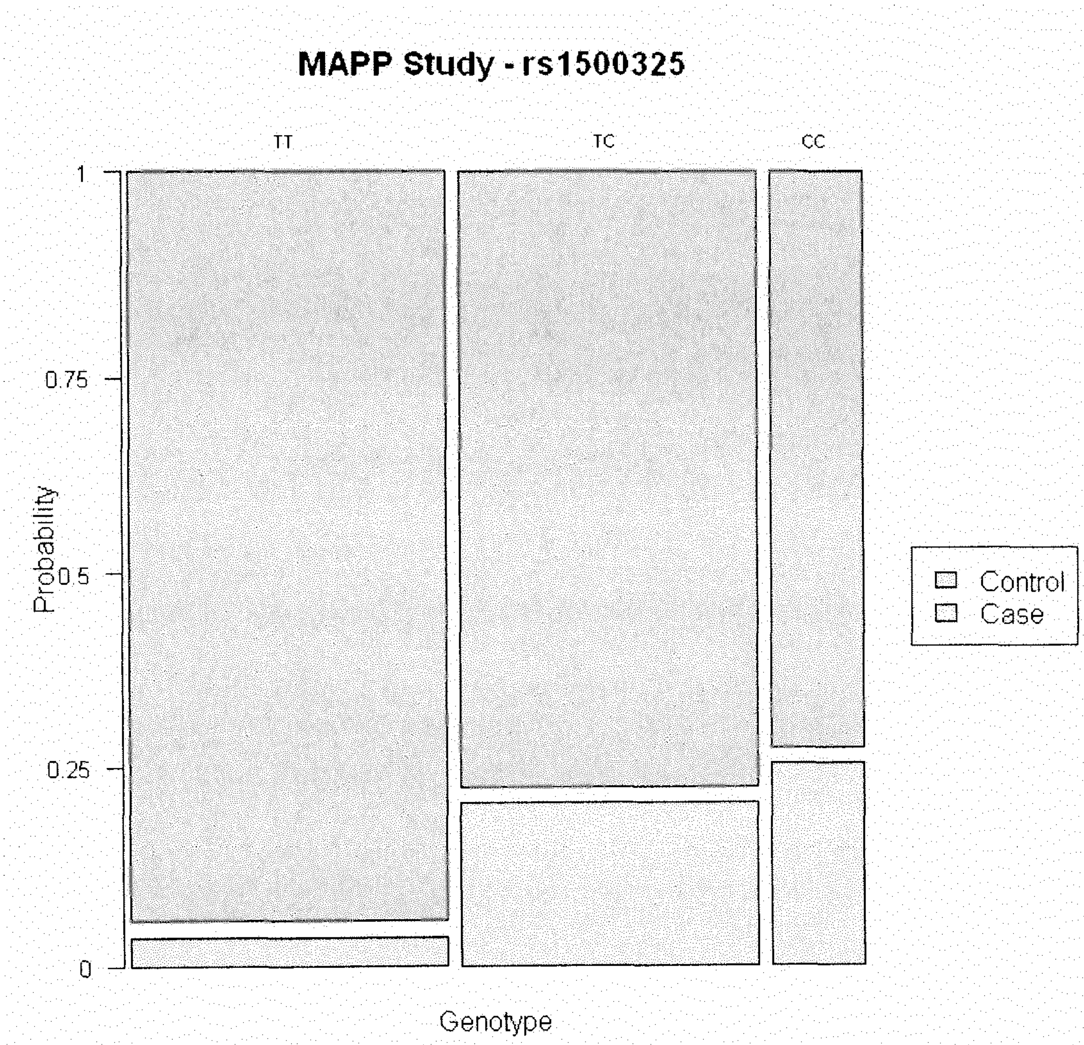
MAPP Study - rs1500325
TT
TC
CC
1
0.75
0.5
0.25
0
Probability
Genotype
Control
Case

Fig. 25

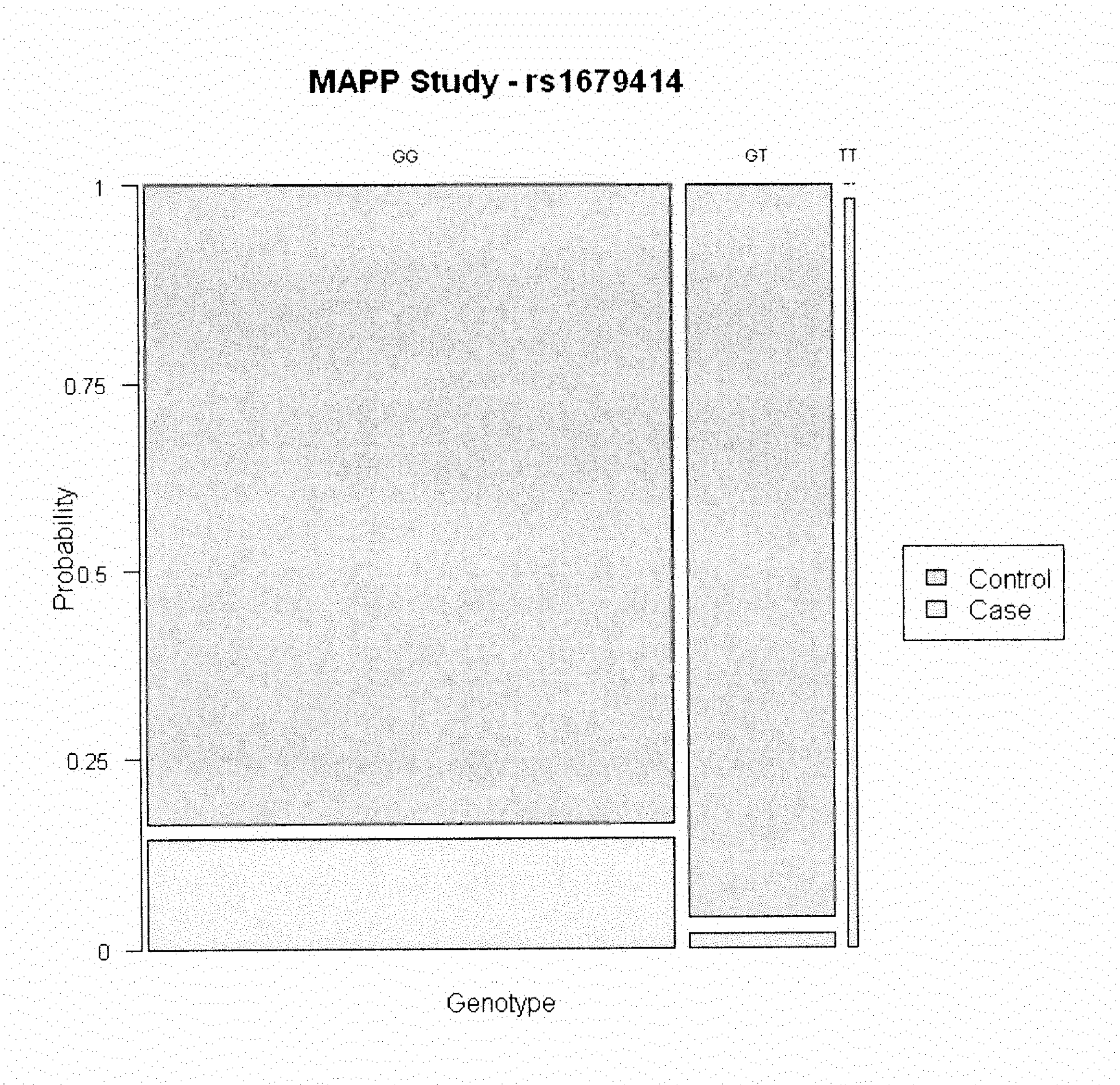
MAPP Study - rs1679414
GG
GT
TT
1
0.75
0.5
0.25
0
Probability
Genotype
Control
Case

Fig. 26

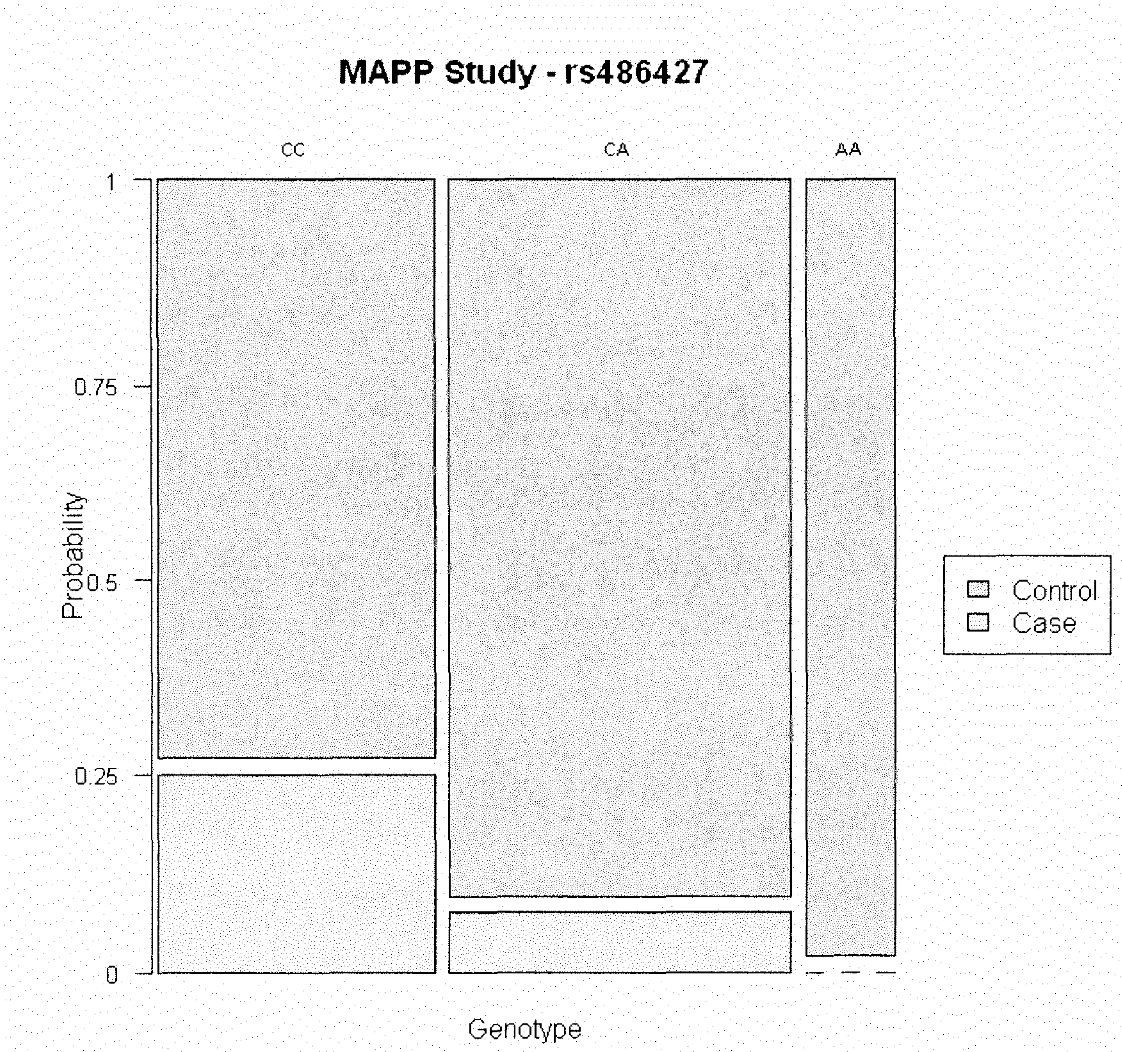
MAPP Study - rs486427
CC
CA
AA
1
0.75
0.5
0.25
0
Probability
Genotype
Control
Case

Fig. 27

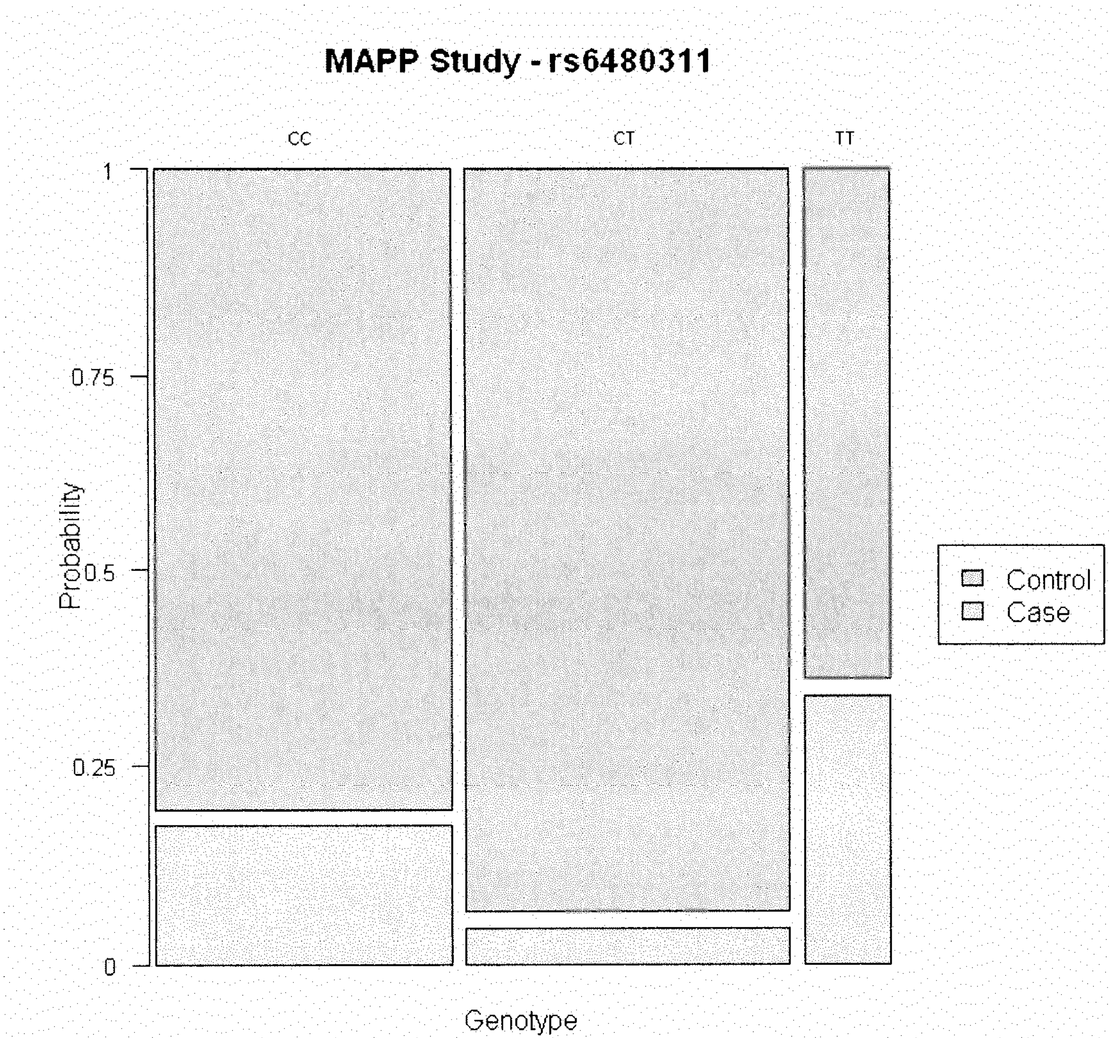
MAPP Study - rs6480311
CC
CT
TT
1
0.75
0.5
0.25
0
Probability
Control
Case
Genotype

Fig. 28

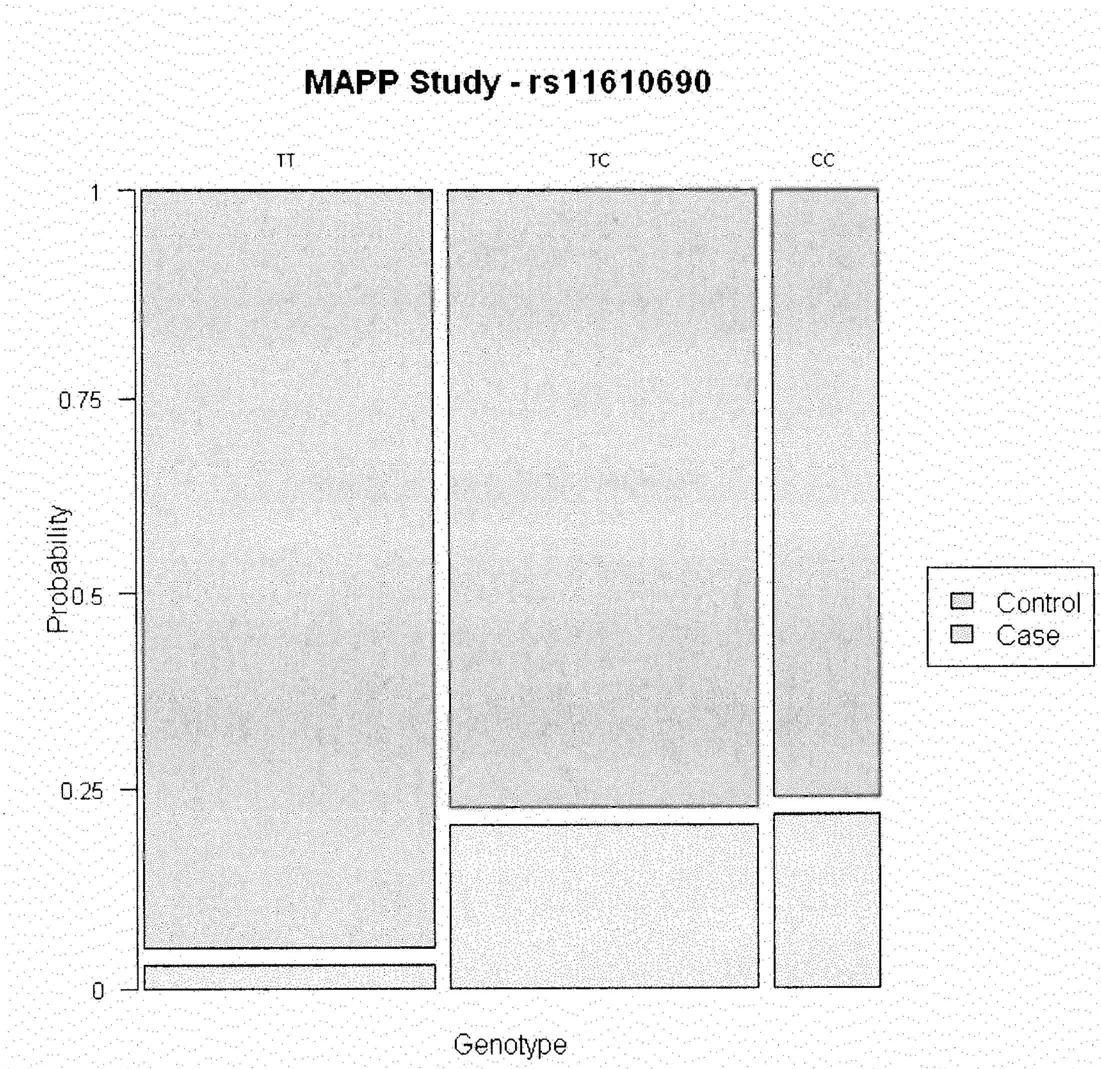
MAPP Study - rs11610690
TT
TC
CC
1
0.75
0.5
0.25
0
Probability
Genotype
Control
Case

Fig. 29

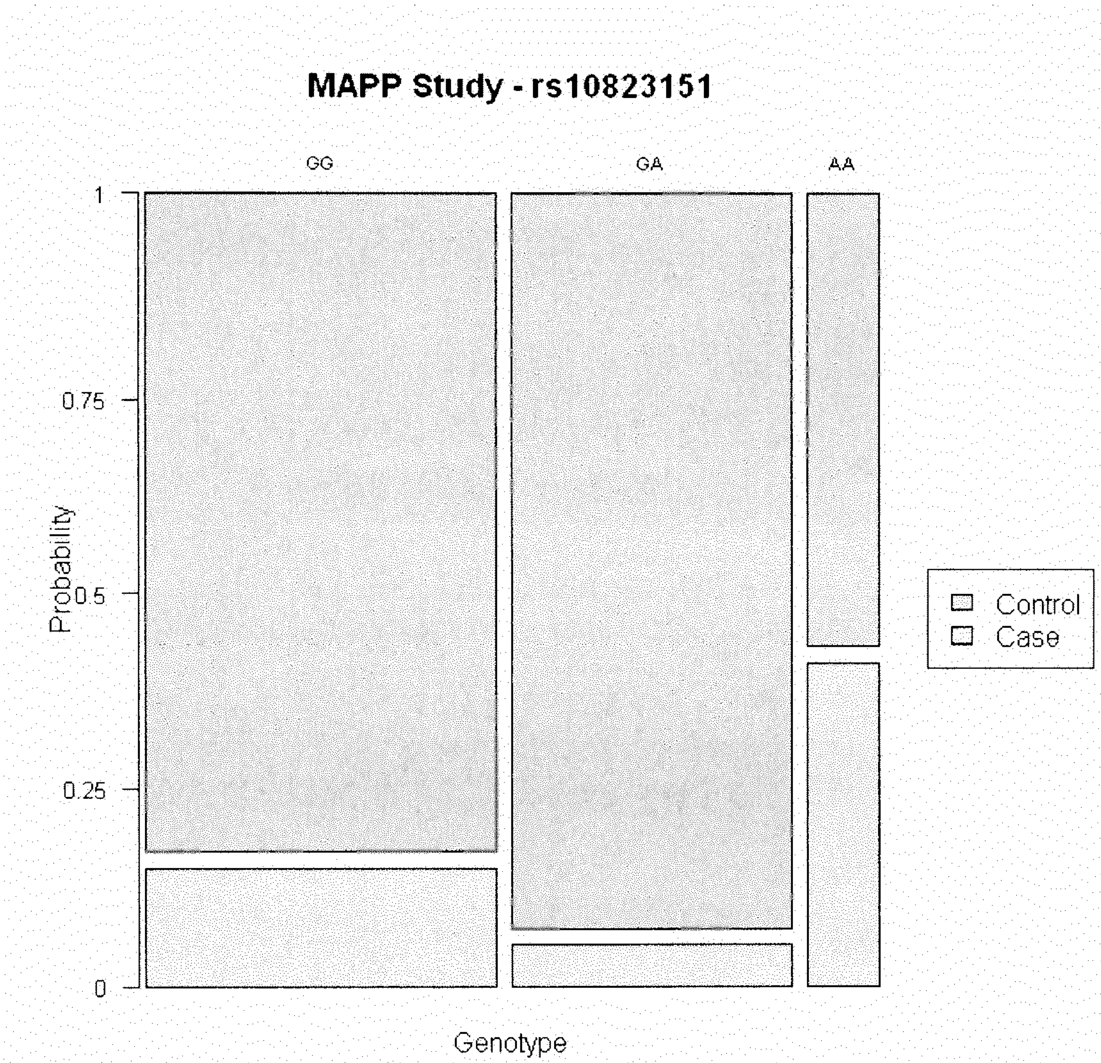
MAPP Study - rs10823151
GG
GA
AA
1
0.75
0.5
0.25
0
Probability
Genotype
Control
Case

Fig. 30

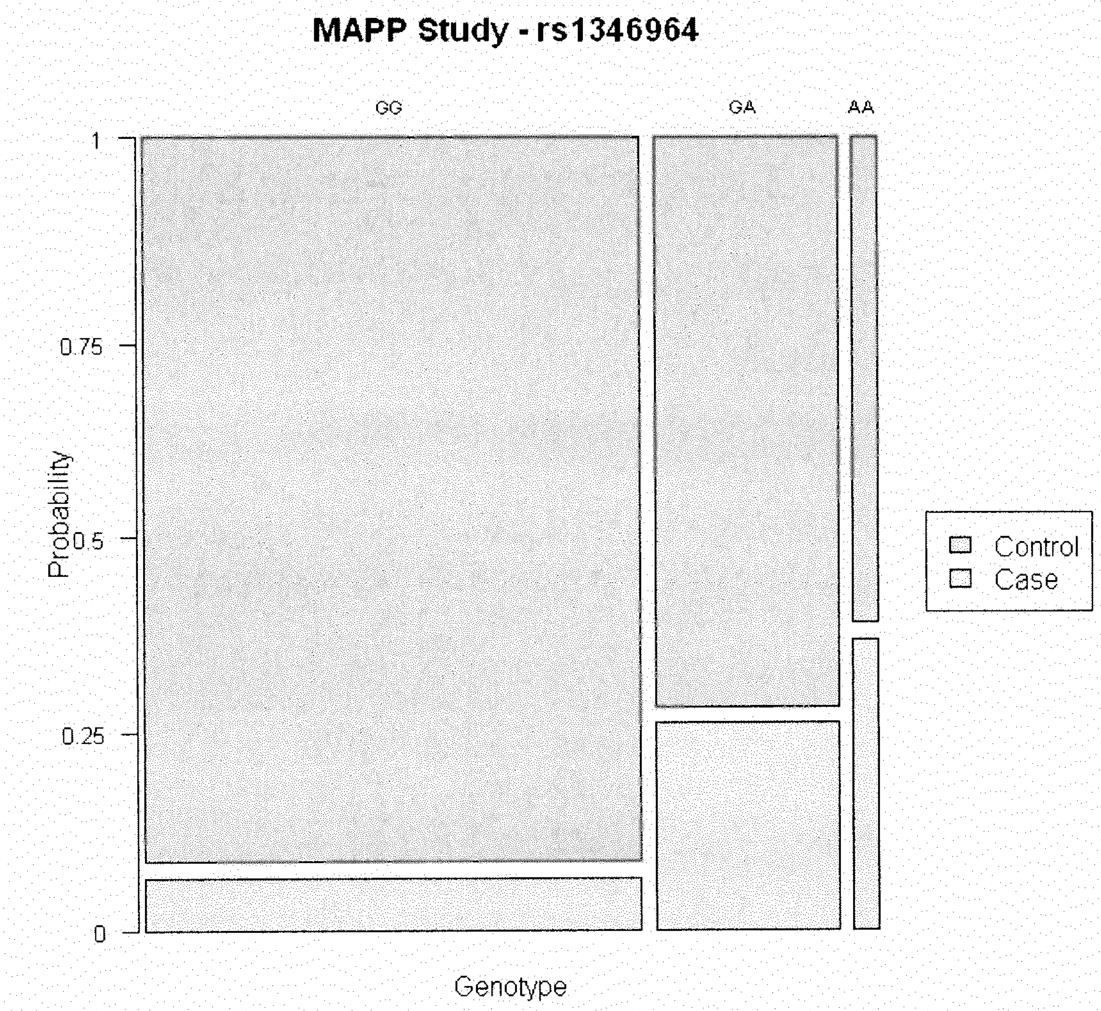
MAPP Study - rs1346964
GG
GA
AA
1
0.75
0.5
0.25
0
Probability
Genotype
Control
Case

Fig. 31

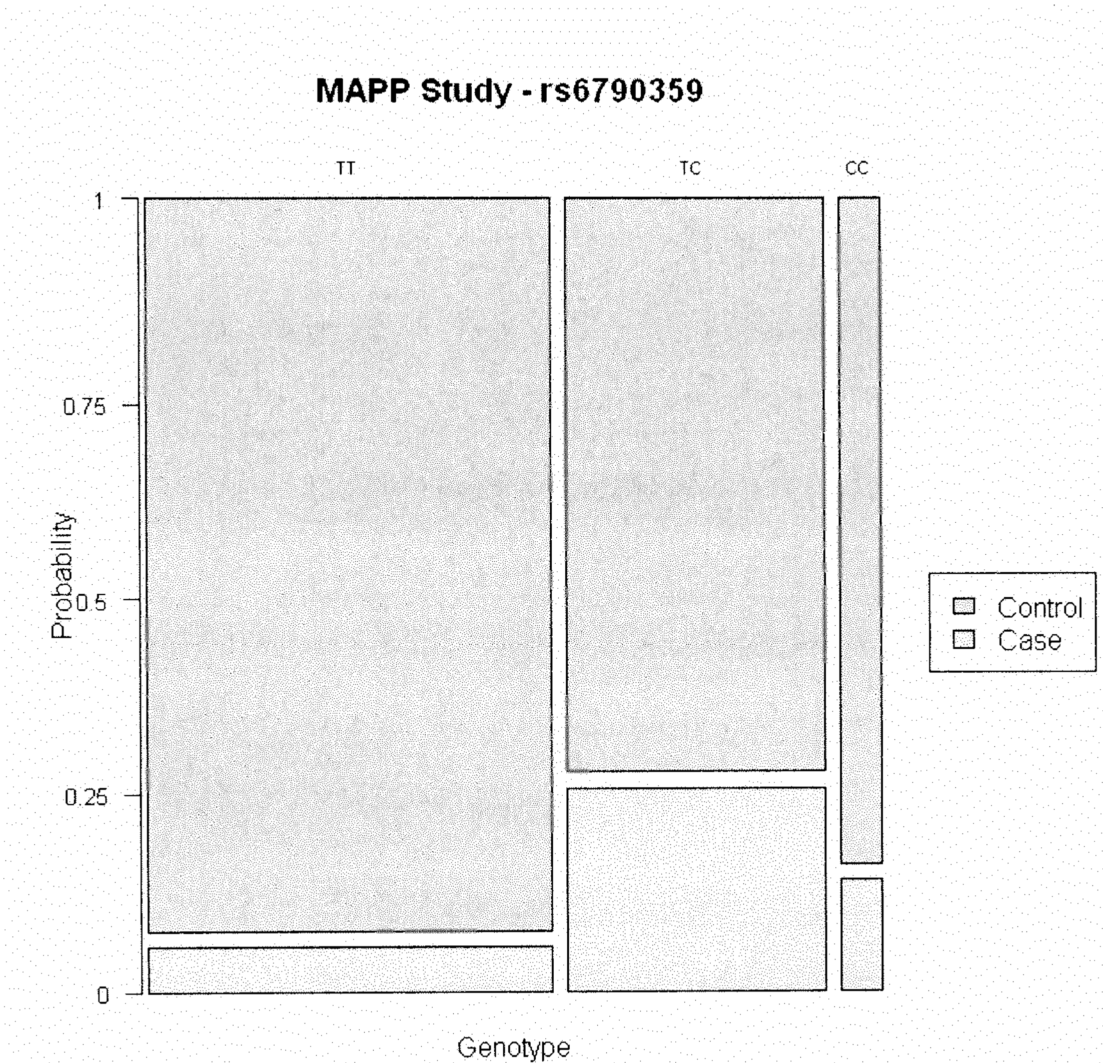
MAPP Study - rs6790359
TT
TC
CC
1
0.75
0.5
0.25
0
Probability
Genotype
Control
Case

Fig. 32

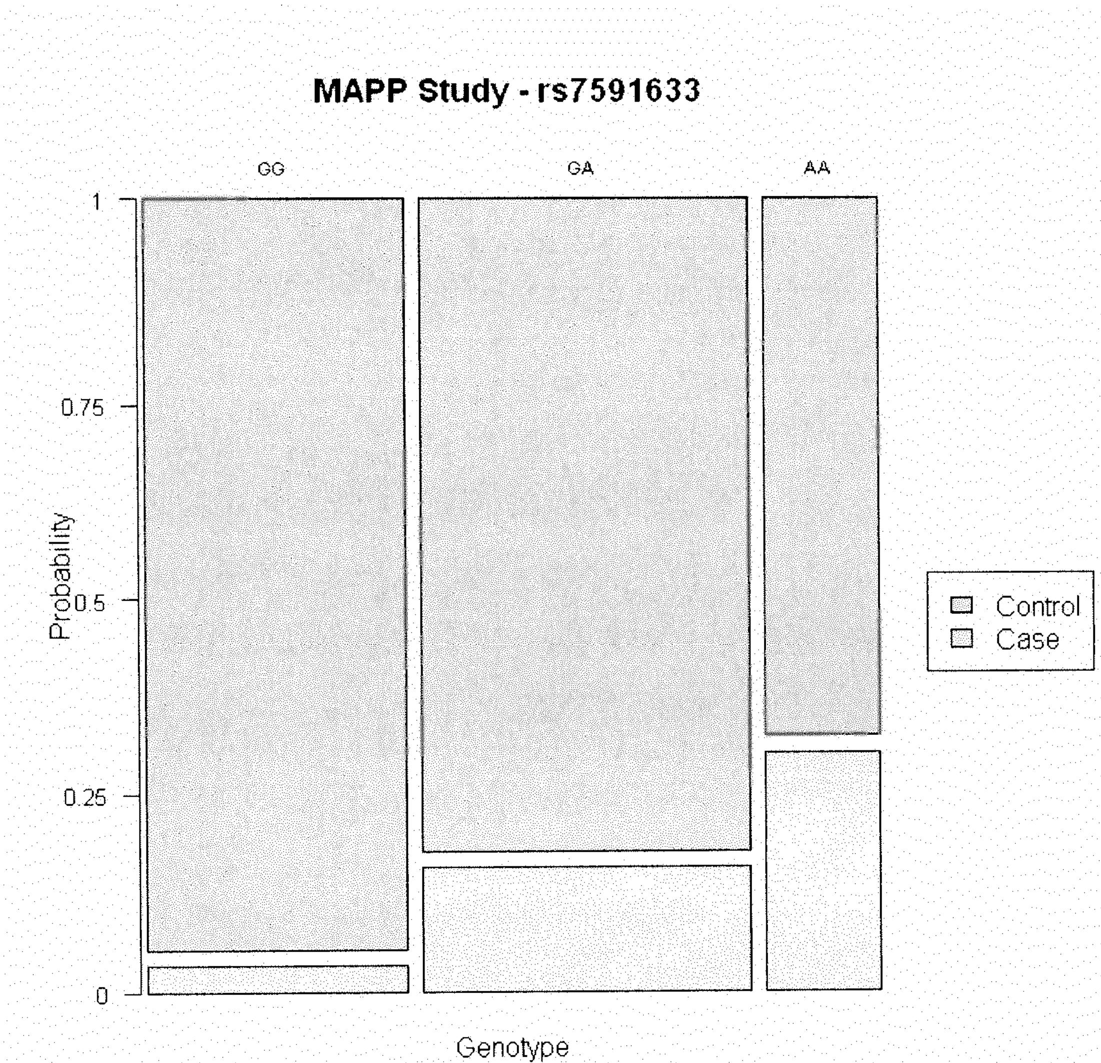
MAPP Study - rs7591633
GG
GA
AA
1
0.75
0.5
0.25
0
Probability
Control
Case
Genotype

Fig. 33

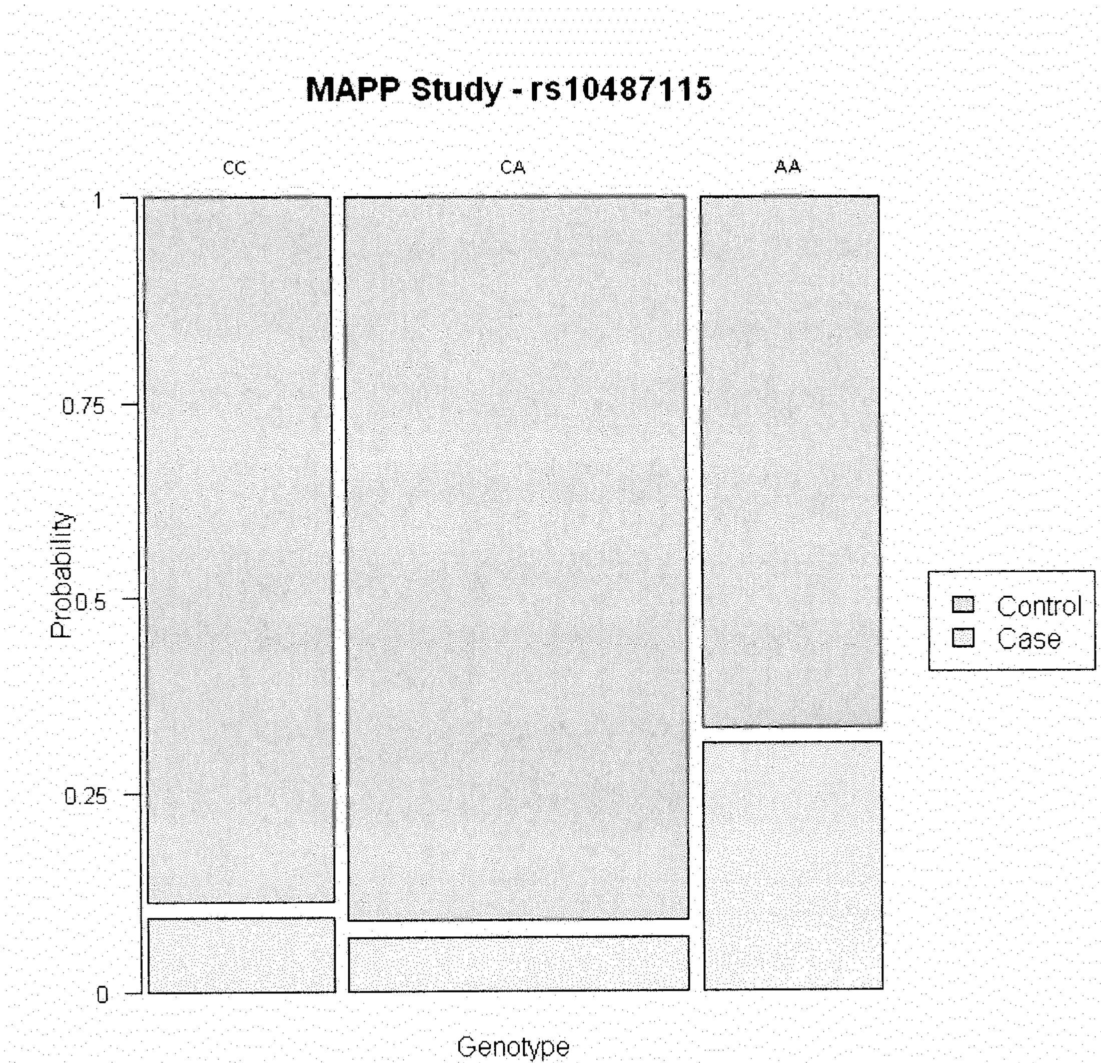
MAPP Study - rs10487115
CC
CA
AA
1
0.75
0.5
0.25
0
Probability
Genotype
Control
Case

Fig. 34

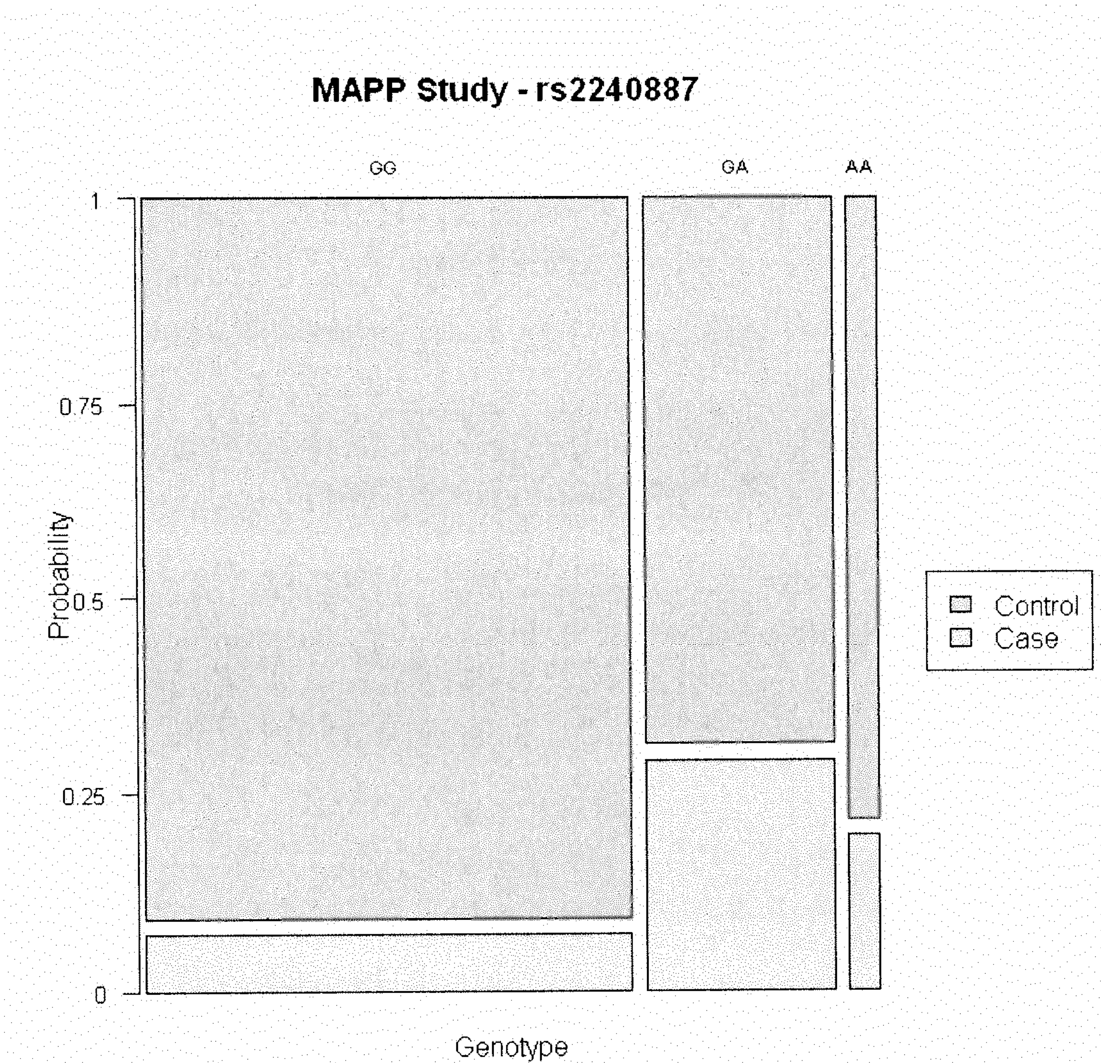
MAPP Study - rs2240887
GG
GA
AA
1
0.75
0.5
0.25
0
Probability
Genotype
Control
Case

Fig. 35

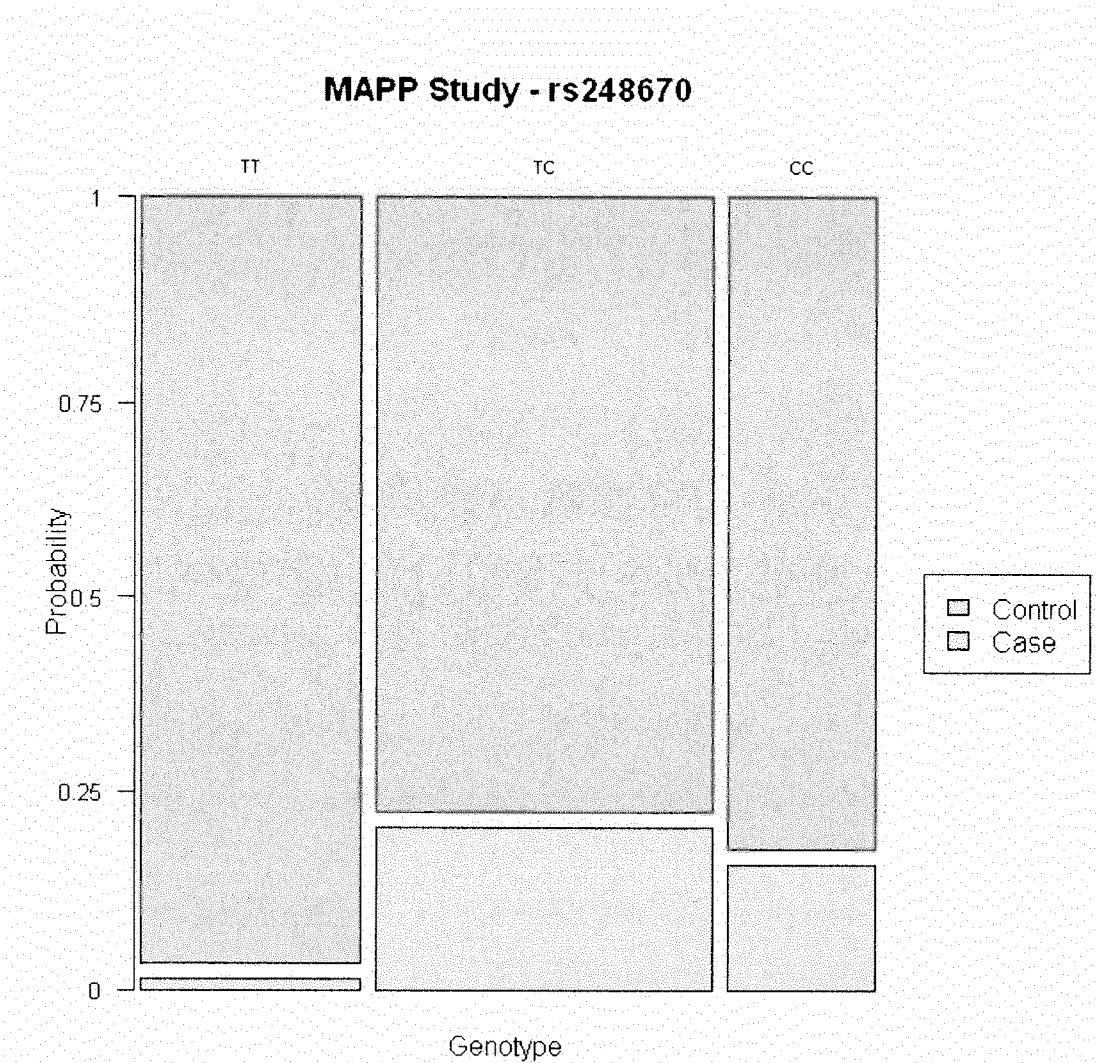
MAPP Study - rs248670
TT
TC
CC
1
0.75
0.5
0.25
0
Probability
Genotype
Control
Case

Fig. 36

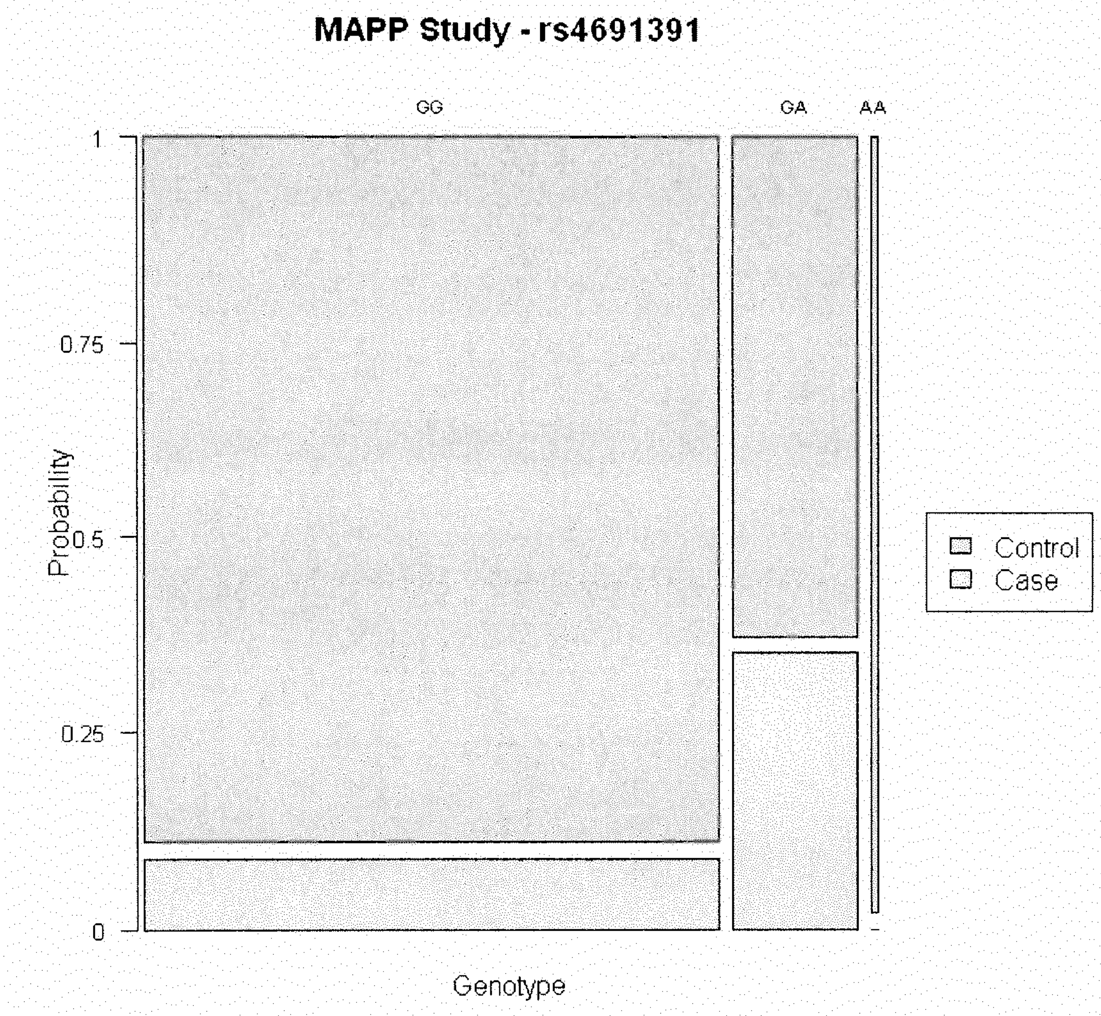
MAPP Study - rs4691391
GG
GA
AA
1
0.75
0.5
0.25
0
Probability
Genotype
Control
Case

Fig. 37

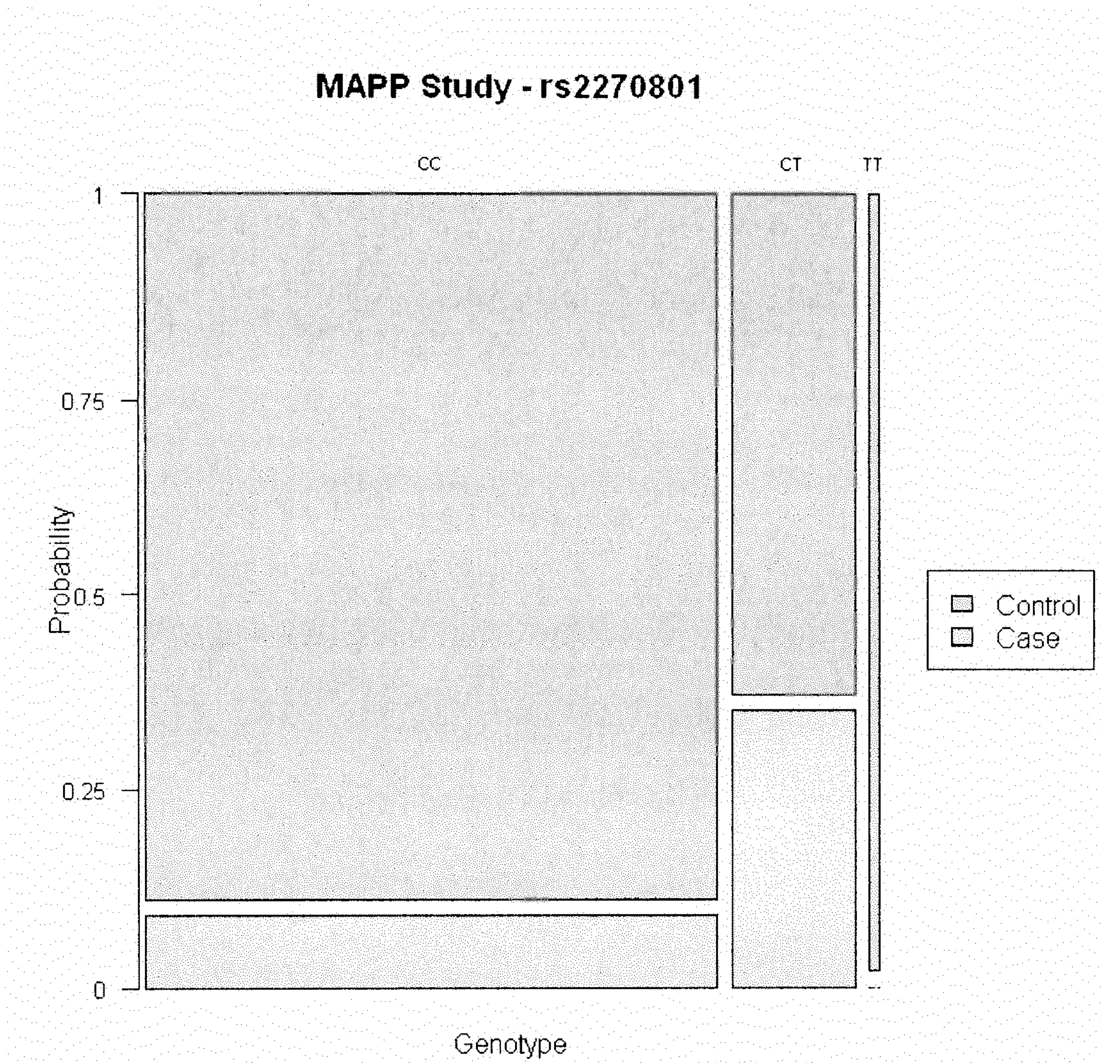
MAPP Study - rs2270801
CC
CT
TT
1
0.75
0.5
0.25
0
Probability
Genotype
Control
Case

Fig. 38

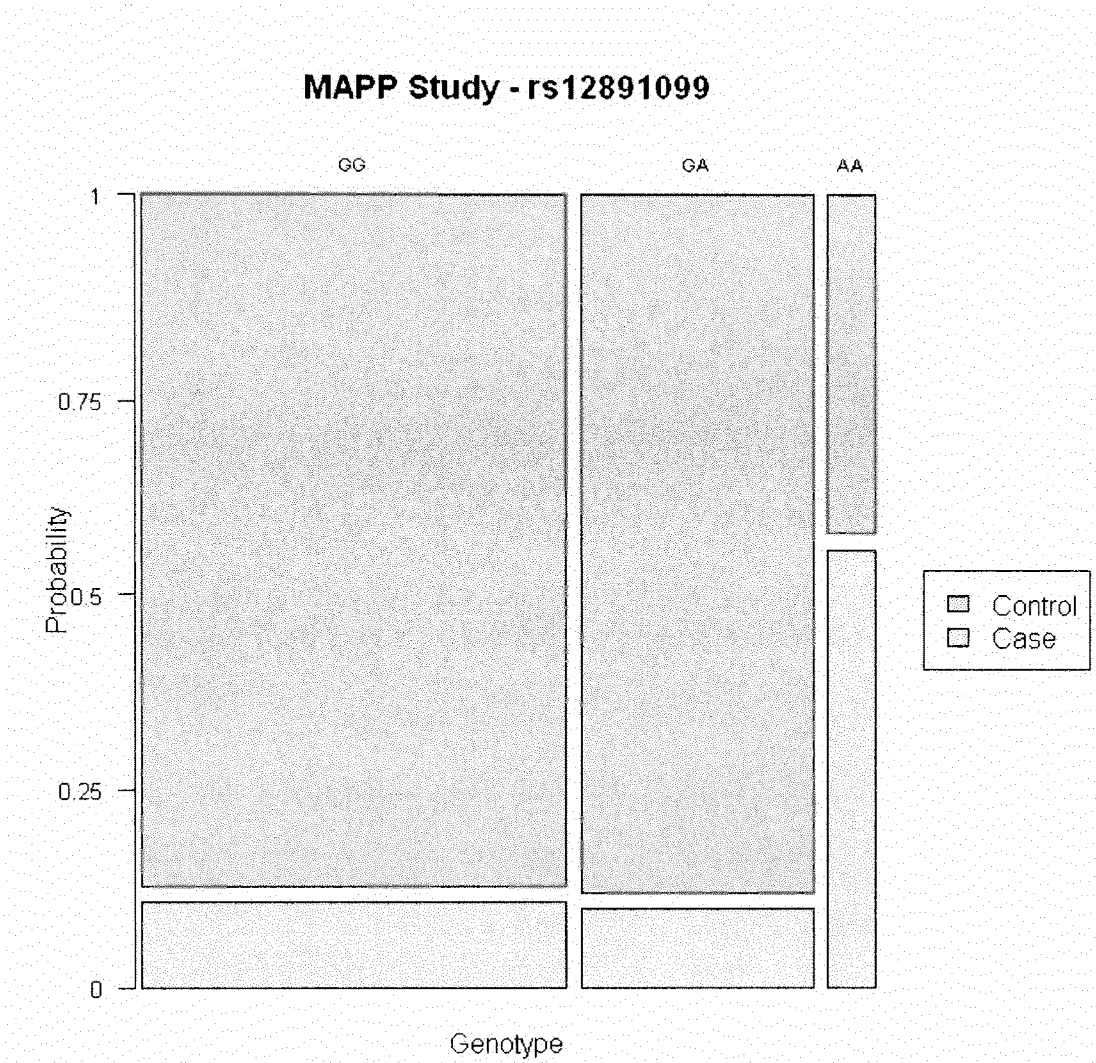
MAPP Study - rs12891099
GG
GA
AA
1
0.75
0.5
0.25
0
Probability
Genotype
Control
Case

Fig. 39

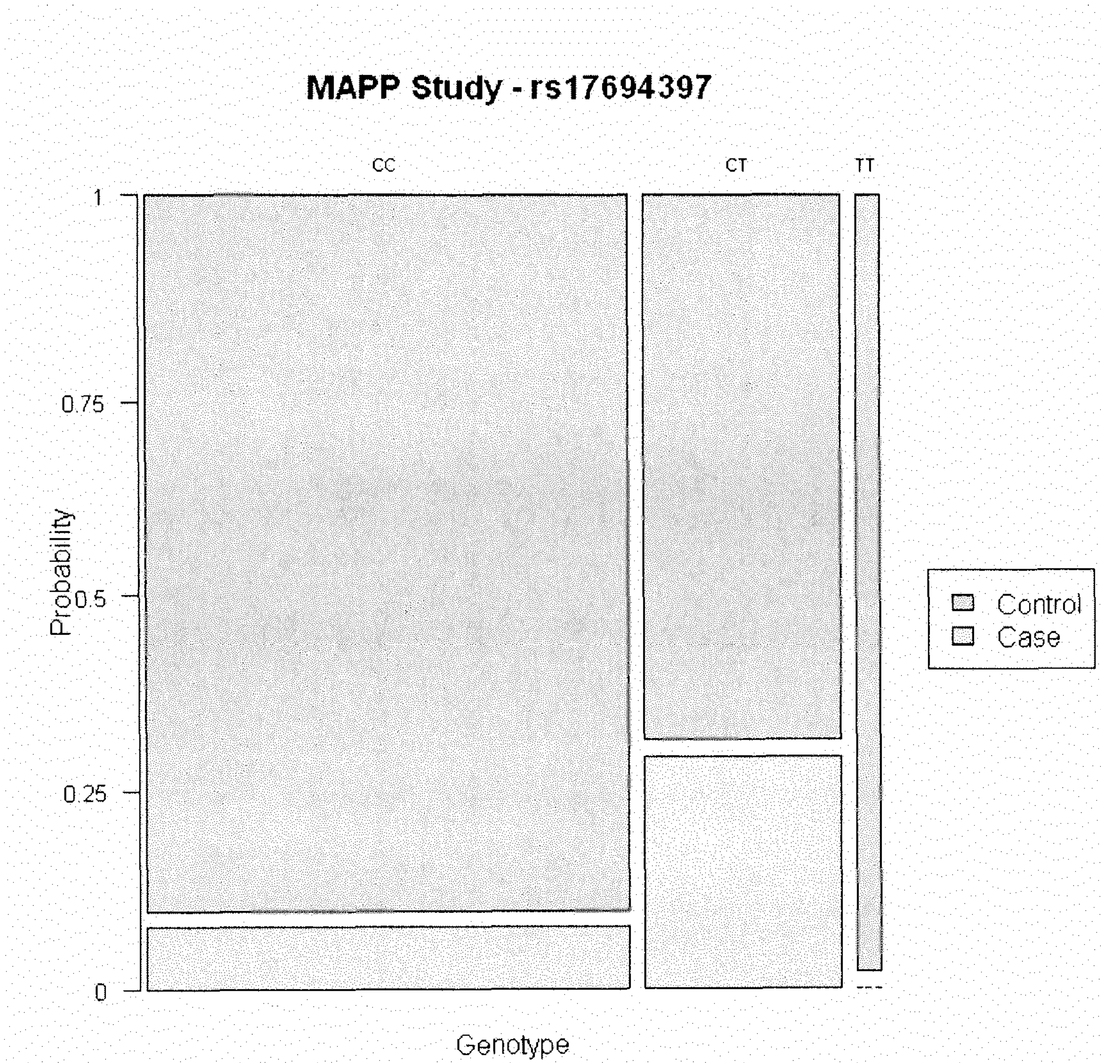
MAPP Study - rs17694397
CC
CT
TT
1
0.75
0.5
0.25
0
Probability
Genotype
Control
Case

Fig. 40

Fig. 41

Table of Risk Alleles and rs numbers

| rs no. | Risk Allele |
|---|---|
| rs10003889 | A |
| rs10006010 | C |
| rs1001294 | T |
| rs1002016 | A |
| rs1004269 | A |
| rs1004604 | T |
| rs10065788 | T |
| rs10085952 | C |
| rs10097563 | G |
| rs10099034 | T |
| rs10102788 | T |
| rs10139234 | A |
| rs10146784 | T |
| rs1017205 | A |
| rs10180461 | C |
| rs1018368 | A |
| rs10238918 | T |
| rs10242397 | A |
| rs10246707 | C |
| rs10277213 | A |
| rs1031006 | C |
| rs1036165 | T |
| rs1039302 | T |
| rs1039539 | T |
| rs10403334 | G |
| rs10415312 | T |
| rs10416550 | T |
| rs10440133 | A |
| rs10442399 | G |
| rs10465632 | C |
| rs10483366 | A |
| rs10485600 | A |
| rs10487115 | A |
| rs10491495 | G |
| rs10491952 | A |
| rs10496220 | C |
| rs10496340 | C |
| rs10496450 | T |
| rs10498644 | C |
| rs10502079 | T |
| rs10504794 | T |
| rs10505726 | C |
| rs10506596 | C |
| rs10506676 | G |
| rs no. | Risk Allele |
| rs10508050 | G |
| rs10510564 | A |
| rs10511470 | T |
| rs10513805 | T |
| rs10517901 | C |
| rs10519034 | T |
| rs10519131 | C |
| rs10520944 | C |
| rs10521726 | G |
| rs1065754 | C |
| rs10746116 | G |
| rs1075042 | G |
| rs10753137 | G |
| rs1075493 | C |
| rs10755897 | G |
| rs10760793 | T |
| rs10773594 | C |
| rs1077388 | A |
| rs10780770 | T |
| rs10797444 | A |
| rs10809523 | T |
| rs10818161 | C |
| rs10820441 | G |
| rs10823151 | A |
| rs10823406 | A |
| rs10828726 | G |
| rs10846448 | G |
| rs10853232 | A |
| rs10861034 | A |
| rs10865566 | T |
| rs10876488 | G |
| rs10919336 | A |
| rs10923673 | G |
| rs10938745 | A |
| rs10945462 | G |
| rs10958798 | A |
| rs10964155 | C |
| rs10965597 | C |
| rs10972872 | G |
| rs10974530 | T |
| rs10982585 | T |
| rs10983571 | A |
| rs11008264 | C |
| rs11013998 | A |
| rs no. | Risk Allele |
| rs11024449 | T |
| rs11030106 | A |
| rs11035719 | C |
| rs11073678 | A |
| rs11082159 | T |
| rs11099852 | A |
| rs11165877 | G |
| rs11170657 | A |
| rs11180765 | A |
| rs11198846 | G |
| rs11201011 | A |
| rs1122816 | G |
| rs11243406 | C |
| rs11564355 | A |
| rs11564361 | T |
| rs1156793 | A |
| rs11614358 | A |
| rs11624431 | G |
| rs11629182 | G |
| rs11661310 | A |
| rs1171837 | C |
| rs11721729 | A |
| rs11726463 | C |
| rs11750519 | T |
| rs11759651 | T |
| rs11764339 | G |
| rs11765962 | G |
| rs11791472 | G |
| rs11823 | G |
| rs11871449 | C |
| rs11883500 | C |
| rs11930193 | A |
| rs11957867 | C |
| rs1196152 | T |
| rs11962675 | A |
| rs11995187 | T |
| rs12025142 | A |
| rs1206610 | G |
| rs1211554 | C |
| rs12121994 | C |
| rs1215765 | A |
| rs12175530 | G |
| rs12298405 | C |
| rs1233258 | C |
| rs no. | Risk Allele |
| rs12341391 | G |
| rs12356112 | A |
| rs12373663 | C |
| rs12388064 | G |
| rs12410385 | T |
| rs1244655 | A |
| rs12452861 | T |
| rs12456839 | C |
| rs12467276 | T |
| rs12468086 | C |
| rs1247451 | A |
| rs12516171 | G |
| rs12543841 | A |
| rs12549803 | G |
| rs12550783 | G |
| rs12556578 | A |
| rs12558527 | C |
| rs12563141 | T |
| rs12566190 | A |
| rs1257200 | C |
| rs12606960 | G |
| rs12617566 | A |
| rs1261795 | A |
| rs12618382 | G |
| rs12618696 | A |
| rs12628984 | C |
| rs12645274 | T |
| rs12653539 | C |
| rs12666315 | G |
| rs12696410 | T |
| rs12775410 | T |
| rs12784234 | G |
| rs12792262 | A |
| rs1282540 | A |
| rs12861247 | A |
| rs12876596 | T |

| | | | | | | | |
|---|---|---|---|---|---|---|---|
| rs12881439 | G | rs1488171 | T | rs17694397 | C | rs205717 | T |
| rs12891099 | A | rs1500325 | C | rs177298 | G | rs2060009 | G |
| rs12908846 | A | rs1503161 | A | rs1773877 | T | rs2060117 | T |
| rs12918181 | A | rs1503195 | C | rs17816553 | T | rs2072175 | A |
| rs13091270 | G | rs1506869 | G | rs180204 | T | rs2078548 | G |
| rs1318655 | T | rs1518603 | A | rs1808380 | A | rs2089432 | C |
| rs1321582 | A | rs1522823 | G | rs181694 | A | rs2096509 | C |
| rs1326800 | C | rs1527536 | T | rs1833044 | T | rs2097130 | G |
| rs13272985 | C | rs1540610 | C | rs1835851 | C | rs2103520 | A |
| rs13279994 | C | rs1540613 | C | rs1858095 | C | rs2107710 | T |
| rs13294002 | G | rs1541103 | G | rs1864895 | A | rs2133764 | T |
| rs1329521 | C | rs1542827 | C | rs1872667 | G | rs2139209 | C |
| rs13319027 | T | rs1544616 | G | rs1875789 | A | rs2143844 | C |
| rs13322750 | C | rs1547531 | C | rs1879894 | G | rs2155387 | C |
| rs133295 | A | rs1559931 | A | rs1886505 | G | rs2157372 | T |
| rs1334168 | C | rs1564821 | A | rs1892555 | G | rs2159318 | G |
| rs1335420 | A | rs1565610 | T | rs1893259 | T | rs2165846 | G |
| rs13357969 | A | rs1566861 | C | rs1894704 | C | rs2169385 | C |
| rs1346964 | A | rs1567894 | A | rs1894706 | G | rs2182289 | C |
| rs1347001 | A | rs1568786 | G | rs1896284 | A | rs2195956 | T |
| rs1350515 | A | rs1583443 | G | rs1904031 | C | rs220599 | G |
| rs1352516 | A | rs1587734 | C | rs1909260 | A | rs2210 | C |
| rs1357086 | A | rs1590202 | C | rs1909446 | G | rs2211472 | T |
| rs1357337 | A | rs1595489 | A | rs1910534 | G | rs2214825 | T |
| rs1361117 | G | rs1598859 | C | rs1934124 | A | rs2214827 | G |
| rs1361206 | T | rs1657382 | A | rs1948073 | A | rs2217827 | G |
| rs1362128 | G | rs1657385 | A | rs1950268 | T | rs2224439 | T |
| rs1372347 | C | rs1658693 | T | rs1953263 | C | rs223198 | G |
| rs1378796 | A | rs1679414 | T | rs1954920 | C | rs2237432 | G |
| rs1386689 | T | rs16868805 | C | rs1954925 | C | rs2238702 | C |
| rs1391612 | A | rs16898178 | C | rs1980291 | A | rs2239473 | A |
| rs1391613 | C | rs16903629 | T | rs1984007 | T | rs2240887 | A |
| rs1403719 | T | rs16934689 | G | rs1984020 | A | rs2240887 | A |
| rs1424104 | A | rs16947192 | T | rs1986391 | T | rs2242144 | A |
| rs1429272 | T | rs16949425 | C | rs198780 | C | rs2242223 | C |
| rs14312 | T | rs16952330 | A | rs1991624 | C | rs2243639 | C |
| rs1434250 | G | rs17005910 | T | rs1994090 | G | rs2244541 | G |
| rs1439098 | G | rs17028972 | G | rs1994283 | G | rs2246649 | T |
| rs1439376 | T | rs1715489 | C | rs1994469 | T | rs2270151 | A |
| rs1455251 | A | rs17189726 | C | rs1997630 | T | rs2270801 | C |
| rs1455264 | T | rs172310 | A | rs2000327 | C | rs2274808 | C |
| rs1458371 | A | rs17331632 | G | rs2018094 | G | rs2287749 | T |
| rs1459151 | C | rs17343504 | G | rs2026362 | A | rs2292884 | A |
| rs1465067 | G | rs1734729 | T | rs2037284 | G | rs2298752 | T |
| rs1466123 | G | rs17441859 | C | rs2038193 | G | rs2300719 | A |
| rs1470645 | T | rs1751277 | C | rs204740 | G | rs2302762 | C |
| rs1474056 | C | rs1751280 | G | rs2048646 | T | rs2303164 | G |
| rs1475418 | A | rs17531821 | A | rs2049197 | C | rs2304731 | A |
| rs1481646 | C | rs17635284 | A | rs2051301 | G | rs2304900 | A |
| rs1484700 | C | rs17679624 | A | rs2052482 | G | rs230526 | T |

| | | | | | | | |
|---|---|---|---|---|---|---|---|
| rs2305593 | T | rs2839347 | A | rs3821053 | G | rs4653474 | T |
| rs2317512 | G | rs2839372 | A | rs3822030 | T | rs4656680 | T |
| rs2321733 | A | rs2839775 | C | rs3827440 | A | rs4658340 | T |
| rs2332914 | T | rs2852143 | A | rs3829078 | G | rs4674039 | T |
| rs2337130 | A | rs2861630 | T | rs383216 | T | rs4691391 | G |
| rs2353815 | G | rs287016 | A | rs384366 | T | rs4699128 | G |
| rs235799 | T | rs287024 | A | rs384526 | C | rs4699769 | A |
| rs2378013 | A | rs2871647 | C | rs3860290 | T | rs4719155 | G |
| rs2380906 | T | rs2890537 | C | rs391678 | G | rs4725617 | G |
| rs2381672 | G | rs2922066 | A | rs3917289 | T | rs4752791 | T |
| rs2388569 | G | rs2945399 | A | rs392840 | C | rs4755844 | T |
| rs2393469 | T | rs2957370 | G | rs3957526 | G | rs4759732 | C |
| rs239832 | T | rs295999 | G | rs397496 | T | rs4761528 | T |
| rs239863 | C | rs2961430 | C | rs407179 | C | rs4765028 | G |
| rs2411975 | G | rs296277 | A | rs4083221 | C | rs4771332 | C |
| rs2411976 | G | rs2979715 | C | rs4129316 | C | rs4777039 | C |
| rs241250 | T | rs2997664 | T | rs4129443 | A | rs4785426 | G |
| rs242003 | C | rs3007033 | T | rs413667 | G | rs4785755 | G |
| rs2420506 | C | rs3012519 | A | rs4150992 | C | rs4829348 | A |
| rs2424455 | A | rs3021146 | C | rs418543 | T | rs4830487 | C |
| rs2430894 | C | rs3026720 | C | rs4240205 | A | rs4830955 | T |
| rs2435962 | C | rs3087980 | A | rs4244071 | T | rs4830958 | G |
| rs2459068 | T | rs3110496 | G | rs4265116 | T | rs4835490 | A |
| rs2460842 | A | rs3113173 | T | rs4286327 | T | rs4838271 | T |
| rs246486 | A | rs324121 | A | rs4310223 | T | rs4843143 | A |
| rs2481627 | C | rs324125 | G | rs4316308 | C | rs4851235 | C |
| rs248670 | C | rs346831 | G | rs4327974 | G | rs4854135 | T |
| rs2502826 | T | rs350089 | G | rs4335989 | G | rs4861175 | G |
| rs2526535 | T | rs359937 | G | rs4339947 | G | rs486427 | C |
| rs2570817 | C | rs359980 | T | rs4344923 | T | rs4878412 | G |
| rs2588844 | T | rs370850 | G | rs4354529 | A | rs488150 | G |
| rs2588873 | T | rs3740259 | G | rs4403996 | A | rs4889176 | A |
| rs2597909 | A | rs3742480 | C | rs4445834 | G | rs4889180 | T |
| rs2617394 | G | rs3743123 | T | rs4453850 | T | rs4907732 | T |
| rs2631229 | G | rs3744137 | G | rs4456603 | G | rs4913391 | G |
| rs2647582 | A | rs3749010 | T | rs4467944 | A | rs4916425 | C |
| rs2654417 | T | rs3750490 | A | rs4479955 | C | rs4920513 | C |
| rs2689828 | C | rs3751107 | C | rs4491457 | G | rs4935225 | C |
| rs2716727 | C | rs3751109 | A | rs4520323 | T | rs4941887 | A |
| rs2717351 | C | rs3755862 | G | rs4535467 | A | rs4962653 | A |
| rs2734189 | G | rs3758740 | C | rs4540026 | T | rs496486 | G |
| rs2740170 | C | rs3761916 | A | rs4544324 | T | rs4965238 | T |
| rs275946 | T | rs3763215 | G | rs454886 | C | rs4965520 | G |
| rs2760494 | C | rs3775296 | T | rs4556497 | G | rs4975147 | G |
| rs2796460 | A | rs3780708 | C | rs4556627 | A | rs4984479 | G |
| rs2802031 | G | rs3788329 | T | rs459131 | T | rs500586 | A |
| rs2812152 | G | rs3788941 | A | rs4608697 | C | rs5026446 | T |
| rs2830400 | A | rs3789433 | A | rs4621704 | C | rs521095 | G |
| rs2837851 | G | rs3790688 | G | rs4643650 | G | rs541821 | G |
| rs2839343 | G | rs3813804 | A | rs4651286 | C | rs542214 | C |

| | | | | | | | |
|---|---|---|---|---|---|---|---|
| rs544704 | C | rs6503841 | G | rs7119152 | A | rs7580162 | A |
| rs547432 | A | rs6509196 | T | rs7120737 | A | rs7583169 | A |
| rs553653 | C | rs6525877 | T | rs7121901 | T | rs7591633 | A |
| rs564275 | C | rs6529997 | T | rs7122009 | T | rs7596894 | A |
| rs5745066 | G | rs6530854 | C | rs713084 | T | rs7617041 | G |
| rs5745709 | A | rs6534743 | G | rs713469 | G | rs7618693 | C |
| rs579687 | C | rs6538408 | C | rs7138775 | A | rs7620956 | A |
| rs584162 | A | rs6540246 | A | rs713900 | A | rs7622789 | C |
| rs588067 | A | rs6546754 | G | rs7140212 | T | rs7625409 | C |
| rs589149 | G | rs6556865 | A | rs7146722 | C | rs7637944 | T |
| rs5904750 | A | rs6578748 | A | rs7147797 | T | rs7648625 | T |
| rs5908269 | A | rs6582285 | C | rs7154732 | C | rs7660418 | C |
| rs5908645 | C | rs6586604 | C | rs717406 | T | rs7664824 | C |
| rs5911158 | T | rs660075 | T | rs718159 | G | rs7675016 | A |
| rs5916138 | C | rs662199 | T | rs7192812 | A | rs7689919 | T |
| rs5916139 | C | rs6633902 | C | rs7220132 | A | rs7691894 | G |
| rs5917614 | G | rs6649251 | C | rs7223099 | T | rs7701642 | G |
| rs5925077 | C | rs6649680 | G | rs7239567 | C | rs7712871 | C |
| rs5935253 | T | rs6657442 | T | rs7241111 | A | rs7719763 | A |
| rs5935513 | T | rs6683502 | A | rs724201 | G | rs7730091 | T |
| rs5958032 | A | rs6712744 | G | rs7244506 | A | rs7741540 | C |
| rs5958298 | C | rs6716246 | G | rs725173 | T | rs7744524 | T |
| rs5961385 | C | rs675136 | G | rs725399 | T | rs7744878 | A |
| rs5962157 | C | rs6759510 | A | rs7260918 | G | rs7754676 | G |
| rs5967664 | G | rs6763419 | T | rs7262069 | T | rs7760851 | A |
| rs5974731 | C | rs6783129 | C | rs7275311 | A | rs7778311 | A |
| rs5988003 | A | rs6790359 | C | rs728864 | A | rs7785760 | G |
| rs601904 | G | rs6817090 | C | rs7304711 | G | rs7801603 | G |
| rs6031454 | T | rs6824427 | T | rs730645 | C | rs780188 | G |
| rs6046403 | T | rs6828580 | A | rs732227 | C | rs7818051 | G |
| rs6069921 | T | rs6832047 | C | rs733180 | T | rs7818404 | C |
| rs6070373 | T | rs6920474 | A | rs7338552 | A | rs7833003 | G |
| rs6073555 | T | rs6949236 | T | rs7339414 | T | rs7851794 | T |
| rs628572 | G | rs6965038 | C | rs7350983 | C | rs7853023 | A |
| rs628873 | T | rs6974082 | G | rs7350986 | G | rs7859758 | G |
| rs630943 | T | rs6981869 | G | rs735262 | G | rs7866610 | T |
| rs638540 | T | rs6989593 | G | rs736998 | G | rs7877387 | C |
| rs640755 | A | rs6989793 | G | rs7376535 | G | rs7882590 | T |
| rs6418096 | C | rs6991834 | G | rs7379403 | C | rs7891488 | A |
| rs6429732 | C | rs6999664 | G | rs738180 | A | rs7903424 | G |
| rs6429822 | G | rs7000815 | A | rs7399511 | G | rs7905024 | A |
| rs6442164 | A | rs7000981 | T | rs7414734 | A | rs7912419 | C |
| rs6448011 | C | rs7010127 | T | rs7457368 | G | rs792841 | G |
| rs6452027 | T | rs7029465 | A | rs746701 | T | rs7934354 | G |
| rs6467917 | A | rs7045026 | A | rs7493192 | G | rs7946 | T |
| rs6468154 | T | rs708188 | A | rs7509408 | G | rs7946766 | C |
| rs6471335 | G | rs708262 | T | rs752908 | C | rs7957728 | A |
| rs6477107 | T | rs7091141 | C | rs7529979 | C | rs7972005 | T |
| rs6480311 | T | rs7099403 | T | rs7559811 | G | rs7980489 | T |
| rs6501048 | C | rs7116632 | G | rs7565358 | G | rs8 | T |

| | | | |
|---|---|---|---|
| rs8004273 | G | rs950864 | A |
| rs8009231 | T | rs9520396 | C |
| rs802682 | C | rs9563481 | A |
| rs8028632 | C | rs958804 | T |
| rs8035983 | T | rs962052 | A |
| rs8037172 | G | rs9620587 | C |
| rs8037430 | T | rs9667864 | G |
| rs8041437 | A | rs9686533 | C |
| rs8047991 | T | rs976845 | A |
| rs8048495 | T | rs977103 | A |
| rs8061082 | C | rs9790702 | T |
| rs807331 | A | rs9803530 | C |
| rs8074649 | T | rs9817739 | A |
| rs8079174 | G | rs983130 | A |
| rs808576 | A | rs9845033 | T |
| rs8110654 | T | rs984779 | A |
| rs8113086 | G | rs9850416 | G |
| rs8119972 | A | rs9872799 | T |
| rs813328 | G | rs9876789 | A |
| rs814528 | T | rs9876832 | G |
| rs814620 | G | rs9911349 | G |
| rs823673 | G | rs9925917 | T |
| rs837473 | C | rs9931258 | A |
| rs843677 | G | rs993380 | A |
| rs848692 | C | rs9952567 | A |
| rs857477 | A | rs9962727 | C |
| rs859941 | C | rs997607 | T |
| rs869220 | T | rs9978739 | A |
| rs877748 | A | rs9983892 | C |
| rs880170 | A | rs998460 | A |
| rs883429 | T | rs999449 | A |
| rs887029 | G | | |
| rs904075 | T | | |
| rs904910 | G | | |
| rs906528 | T | | |
| rs912435 | A | | |
| rs916874 | A | | |
| rs917673 | A | | |
| rs924417 | T | | |
| rs9301376 | C | | |
| rs931859 | G | | |
| rs9320475 | T | | |
| rs9320884 | C | | |
| rs935374 | T | | |
| rs935480 | G | | |
| rs9386934 | C | | |
| rs939876 | C | | |
| rs940597 | A | | |
| rs9424099 | T | | |
| rs943174 | T | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 96159610 A
- US 96169410 A
- US 6924100 B
- US 6242181 B


### Non-patent literature cited in the description


- **SHERRY et al.** dbSNP— Database for Single Polymorphism Polynucleotides and Other Classes of Minor Genetic Variation. *GENOME RES.,* 1999, vol. 9, 677-679
- **YOU et al.** BatchPrimer3: A high throughput web application for PCR and sequencing primer design. *BMC BIOINFORMATICS,* 2008, vol. 9, 253
- **YANG X ; SCHEFFLER BE ; WESTON LA.** Recent developments in primer design for DNA polymorphism and mRNA profiling in higher plants. *PLANT METHODS,* 2006, vol. 2 (1), 4
- **ABD-ELSALAM KA.** Bioinformatics tools and guideline for PCR primer design. *AFRICA JOURNAL OF BIOTECHNOLOGY,* 2003, vol. 2 (5), 91-95
- General concepts for PCR primer design. **DIEFFENBATCH CW ; LOWE TMJ ; DVEKSLER GS.** PCR PRIMER, A LABORATORY MANUAL. Cold Spring Harbor Laboratory Press, 1995, 133-155
- **SAMBROOK et al.** *MOLECULAR CLONING,* 1989
- **RIEGER et al.** GLOSSARY OF GENETICS. Springer-herlag, 1991, 16
- **RIEGER et al.** GLOSSARY OF GENETICS. Springer-Verlag, 1991, 16
- **FULTON R ; MCDADE R ; SMITH P ; KIENKER L ; KELTMAN J.** J. Advanced multiplexed analysis with the FlowMetrix system. *CLIN. CHEM.,* 1997, vol. 43, 1749-1756
- **MICHAEL K. ; TAYLOR L. ; SCHULTZ S ; WALT D.** Randomly ordered addressable high-density optical sensor arrays. *ANAL. CHEM.,* 1998, vol. 70, 1242-1248
- **STEEMERS F. ; FERGUSON J ; WALT D.** Screening unlabeled DNA targets with randomly ordered fiber-optic gene arrays. *NAT. BIOTECHNOL.,* 2000, vol. 18, 91-94
- **DEVLIN ; RISCH.** A comparison of linkage disequilibrium measures for fine-scale mapping. *Genomics,* 20 September 1995, vol. 29 (2), 311-22
- Cardiac arrest and sudden cardiac death. **MYERBURG, RJ.** A TEXTBOOK OF CARDIOVASCULAR MEDICINE. Saunders, WB, 2001, 890-931
- *CANCER FACTS AND FIGURES,* 2003, 4
- *Center for Disease Control,* 2004
- **PHAM, Q. et al.** T-wave alternans: marker, mechanism, and methodology for predicting sudden cardiac death. *JOURNAL OF ELECTROCARDIOLOGY,* vol. 36, 75-81
- Heart rate variability and sudden cardiac death. **BIGGER, JT.** CARDIAC ELECTROPHYSIOLOGY: FROM CELL TO BEDSIDE. WB Saunders, 1999
- **BUXTON, AE et al.** Risk stratification for sudden death: do we need anything more than ejection fraction?. *CARD. ELECTROPHYSIOLOGY REV.,* 2003, vol. 7, 434-7
- **ZIPES, DP et al.** Sudden Cardiac Death. *CIRCULATION,* 1998, vol. 98, 2334-2351
- **FISHER, R. A.** The Logic of Inductive Inference. *JOURNAL OF THE ROYAL STATISTICAL SOCIETY SERIES A,* 1935, vol. 98, 39-54
- **SIFFERT, W. et al.** Association of a Human G-Protein beta3 Subunit Variant with Hypertension. *NAT. GENET.,* 1998, vol. 18, 45-48
- **HENGSTENBERG, C. et al.** Association Between a Polymorphism in the G protein beta3 Subunit Gene (GNB3) with Arterial Hypertension but not with Myocardial Infarction. *CARDIVASC. RES.,* 2001, vol. 49, 820-827
- **GUTERSOHN, A. et al.** G Protein beta3 Subunit 825 TT Genotype and Post-Pregnancy Weight Retention. *LANCET,* 2000, vol. 355, 1240-1241
- **MITCHELL, A. et al.** Increased Haemodynamic Response to Clonidine in Subjects Carrying the 825T-allele of the G Protein beta3 Subunit. *NAUNYN-SCHMIEDEBERG'S ARCH. PHARACOL.,* 2002, vol. 265, 1
- **MITCHELL, A. et al.** Insulin-mediated Venodilation Is Impaired in Young, Healthy Carriers of the 825T-allele of the G-protein beta3 Subunit Gene (GNB3). *CLIN. PHARMACOL. THER.,* 2005, vol. 77, 495-502

- **MITCHELL, A. et al.** Effects of Systemic Endothelin A Receptor Antagonism in Various Vascular Beds in Men: In Vivo Interactions of the Major Blood Pressure-regulating Systems and Associations with the GNB3 C825T Polymorphism. *CLIN. PHARMACOL. THER.,* 2004, vol. 76, 396-408
- **SARRAZIN, C. et al.** GNB3 C825T Polymorphism and Response to Interferon-alfa/ribavirin Treatment in Patients with Hepatitis C Virus Genotype 1 (CHV-1) Injection. *J. HEPATOL.,* 2005, vol. 43, 388-393
- **SPERLING, H.** Sildenafil Response is Influenced by the G Protein beta3 Subunit GNB3 C825T Polymorphism: A Pilot Study. *J. UROL.,* 2003, vol. 169, 1048-1051
- **DOBREV, D. et al.** G-Protein beta(3)-Subunit 825T Allele Is Associated with Enhanced Human Atrial Inward Rectifier Potassium Currents. *CIRCULATION,* 2000, vol. 102, 692-697
- **SCHREIECK, J. et al.** C825T Polymorphism of the G-protein beta3 Subunit Gene and Atrial Fibrillation: Association of the TT Genotype with a Reduced Risk for Atrial Fibrillation. *AM. HEART. J.,* 2004, vol. 148, 545-550
- **WIENEKE, H. et al.** Better Identification of Patients Who Benefit from Implantable Cardioverter Defibrillators by Genotyping the G Protein beta3 Subunit (GNB3) C825T Polymorphism. *BASIC RES. CARDIOL.,* 2006
- **ADAMS, J. W. et al.** Enhanced Galphaq signaling: A Common Pathway Mediates Cardiac Hypertrophy and Apoptotic Heart Failure. *Proc. Natl. Acad. Sci. U.S.A.,* 1998, vol. 95, 10140-10145
- **WETTSCHURECK, N. et al.** Absence of Pressure Overload Induced Myocardial Hypertrophy After Conditional Inactivation of Galphaq/Galpha11 in Cardiomyocytes. *NAT. MED.,* 2001, vol. 7, 1236-1240
- **WEINSTEIN, L.S. et al.** Genetic Diseases associated with Heterotrimeric G Proteins. *TRENDS PHARMACOL. SCI.,* 2006, vol. 27, 260-266
- **JIA, H et al.** Association of the G(s)alpha Gene with Essential Hypertension and Response to beta-blockade. *HYPERTENSION,* 1999, vol. 34, 8-14
- **ARKING et al.** Genome-Wide Association Study Identifies GPC5 as a Novel Genetic Locus Protective against Sudden Cardiac Arrest. *PLosOne,* 2010, http://www.plosone.org/article/info:doi%2F10.1371%2Fjournal.pone.0009879
- **A. PFEUFER et al.** *Nature Genetics,* 2009
- **SAOUDI, N. et al.** How Smart Should Pacemakers Be?. *AM. J. CARDIOL.,* 1999, vol. 83, 180-186
- **MARSHALL, A.J. et al.** Pacemaker Diagnostics to Determine Treatment and Outcome in Sick Sinus Syndrome with Paroxysmal Atrial Fibrillation. *PACE,* 2004, vol. 27, 1130-1135
- **MITRANI, R.D. et al.** *The Use Of Pacemaker Diagnostic Data To Guide Clinical Decision Making, Presented at Cardiostim,* 2006
- **BENJAMIN, E.J.** Impact Of Atrial Fibrillation On The Risk Of Death: The Framingham Heart Study. *Circulation,* 1998, vol. 98, 946-952
- **GLOTZER, T.V. et al.** Atrial High Rate Episodes Detected By Pacemaker Diagnostics Predicts Death And Stroke. *Circulation,* 2003, vol. 107 (12), 1614-1619
- **NOWAK, B.** Taking Advantage of Sophisticated Pacemaker Diagnostics. *AM. J. CARDIOL.,* 1999, vol. 83, 172-179
- A report of the ACC/AHA Task Force and the ESC Committee for Practice Guidelines: ACC/AHA/ESC 2006 Guidelines For Management Of Patients With Ventricular Arrhythmia and the Prevention of Sudden Cardiac Death - Executive Summary. *European Heart J,* 2006, vol. 27, 2099-2140
- Nomenclature and Criteria for Diagnosis of Diseases of the Heart and Great Vessels. Little, Brown & Co, 04 March 1994
- Appropriate and Inappropriate Ventricular Therapies, Quality of Life, and Mortality Among Primary and Secondary Prevention Implantable Cardioverter Defibrillator Patients: Results From the Pacing Fast VT Reduces Shock Therapies [PinFREE Rx II] Trial. *CIRCULATION,* 2005, vol. 111, 2898-2905
- **WILKOFF, B. L. et al.** A Comparison of Empiric to Physician-Tailored Programming of Implantable Cardioverter-Defibrillators. *J. AM. COLL. CARDIOL.,* 2006, vol. 48, 3330-339
- **JOHNSON ; KOTZ.** Discrete Distributions. Houghton Mifflin Company, 1969, 58-60

SEQUENCE LISTING

<110> Medtronic, Inc.

<120> DIAGNOSTIC KITS, GENETIC MARKERS AND METHODS FOR SCD OR SCA THERAPY SELECTION

<130> EP79570FZ200pau

<140> not yet assigned
<141> herewith

<150> 12/961,596
<151> 2010-12-07

<150> 12/961,694
<151> 2010-12-07

<160> 858

<170> PatentIn version 3.5

<210> 1
<211> 101
<212> DNA
<213> Homo sapiens

<400> 1
tgtacctgga actgaaggat ggcagatgac aagccaggca gggaggaatg racctggatt 60

cctggtgaag gacgtggata tatcttgtgg ggtataactt g 101

<210> 2
<211> 101
<212> DNA
<213> Homo sapiens

<400> 2
ttatttttaa gtaaaaaaaa aaaaaagagg aacagaggtt atattttttg ytatactcaa 60

tgagctatct ttggatacca ataccctata ctattcactc c 101

<210> 3
<211> 101
<212> DNA
<213> Homo sapiens

<400> 3
gatgttttag tttgctccat gaggaaatag tttccctttt tctatttggc rtataaattt 60

tcttgctgat tataattctt atgtacatta catttttatt a 101

<210> 4
<211> 101
<212> DNA
<213> Homo sapiens

<400> 4
atagtggtca ctggtgacgc caataagagc aacaaaattg gaacttttct ragcacacaa 60

gtgccattgt attaaagaga atatttgctt tggatgacag a 101

<210> 5
<211> 101
<212> DNA
<213> Homo sapiens

<400> 5
ttatataaac tattttgatt atgcttctta tatctaaggc agcctgtata rttcattttt 60

ttccaaagtt agatcctagc tgagtagtga ggtatgtacg g 101

<210> 6
<211> 101
<212> DNA
<213> Homo sapiens

<400> 6
aaacaataca taacatttca cctttttcta aaacctccaa cttttgtctg mtctttgaaa 60

ataccaaagt ctacctagtc tgtgagtaca ccccaaattg t 101

<210> 7
<211> 101
<212> DNA
<213> Homo sapiens

<400> 7
atgctgcctt actctttaag aagatactca tcttcaatag aagaaactgt rtctgtggaa 60

cagtaaaaag gccagacact gttcaagata tgtaacacaa a 101

<210> 8
<211> 101
<212> DNA
<213> Homo sapiens

<400> 8
atgctgttct ctttgagatc ctaaattgtt tatgagcttg gggagctagt yatataatga 60

tgttattttc taaaatgtct gtgtgaacat cttttttttt c 101

<210> 9
<211> 101
<212> DNA
<213> Homo sapiens

<400> 9
tgattatttt tggaaaatga agtcacatac atatagttct gcatcttgct yttcttactt 60

ttcaatatcc tgtggaaccc tctccaagtc aactggtata g 101

<210> 10
<211> 101
<212> DNA
<213> Homo sapiens

<400> 10
atattcaaaa taaaaaattt gtaataatca aagaatatct tctcccatat kagattaatt 60

tggcccccaa tcaaccacac cacagcctag taaggtctta a 101

<210> 11
<211> 101
<212> DNA
<213> Homo sapiens

<400> 11
aaaaaaaaac tgcacaggct tgaaaactac atttacttta cacagctaga ygagagaact 60

tgtcatgtct tctaagaaca atgacaatga tacaaactag a 101

<210> 12
<211> 101
<212> DNA
<213> Homo sapiens

<400> 12
tacatgtgag gcagacctga aagacataaa tgccagtttc cctcttcccc mtttggagtg 60

tgtgcttttg atttccctgg aggctacatt tcctgatctg c 101

<210> 13
<211> 101
<212> DNA
<213> Homo sapiens

<400> 13
tctgaagtca gaggttttgg atatgaggca gtgctctgac attcactgga ytctgaggct 60

catatgtgtc ccagttgagt aatcacaggt tccttaaccc t 101

<210> 14
<211> 101
<212> DNA
<213> Homo sapiens

<400> 14
ctttatggta tggggaaaag gaggatgatg acagcctctg ctgcagtgta yaccatccac 60

tatggttgct gttcactctc acctccatgg agcatcctct g 101

<210> 15
<211> 101
<212> DNA
<213> Homo sapiens

<400> 15
gatccaaaag ccatgtttgc tgtctcagtg gggtagatca cggcctaggg yatggtctga 60

gtggggcact gcagaagtga gactgacctc aacaactgca t 101

<210> 16
<211> 101
<212> DNA
<213> Homo sapiens

<400> 16
tcttattgat cctattttat cagtctcctc tactagaatg tgacctccac rtgaatcggg 60

atttttttg gtctatttac tggctttact ttctacatta a 101

<210> 17
<211> 101
<212> DNA
<213> Homo sapiens

<400> 17
caagactccg tctcaagcaa acaaacaaaa gagcagtgac agcttggtta yggttctgtg 60

aattgaaatg ctaggcttcc cttagggtta gttcctccat a 101

<210> 18
<211> 101
<212> DNA
<213> Homo sapiens

<400> 18
ttttattatc aggccttggc agccacactt caacttttta caggtaactc rctggaccct 60

cacagtgact cttcaaatga ggttttgcac tagacccatt t 101

<210> 19
<211> 101
<212> DNA
<213> Homo sapiens

<400> 19
tgctggtgga actcactggt caatattcct tttacccata tatagacatc ytgtgtcagt 60

gaacttcaaa gctgctgatt agtttttcc tccataatat t 101

<210> 20
<211> 101
<212> DNA
<213> Homo sapiens

<400> 20
atgtcaagat aagctgatta tcctagaata tccaagtggg tccatgatac ygagaagcag 60

gaaaatggta atggaacaat cagtccagac aagccatgca a 101

<210> 21
<211> 101
<212> DNA
<213> Homo sapiens

<400> 21
tgctcaaatc tgctctccat acccactaga aaatcctaaa agaaatatag ycttaaatac 60

agtttttagg ctccatcacc ttacctatcc tggctgttgt g 101

<210> 22
<211> 101
<212> DNA
<213> Homo sapiens

<400> 22
ttccccaggg gaaaagtgga ctgcagaaag acactcactc accctctctc rtagtgggga 60

ttcactctca gttcctggtc tatcatggtc atataagctg c 101

<210> 23
<211> 101
<212> DNA
<213> Homo sapiens

<400> 23
gggcctgatg tatcacaagg gtccttataa gaaagaagtg gggattgaaa katgttatgc 60

tttgcagatg gaggaagggg ccacaaacca agaaatgcca g 101

<210> 24
<211> 101
<212> DNA
<213> Homo sapiens

<400> 24
gttgttggtg taatgaacgt atttaacctt ttcctgatag tcaagttctt yctcaattta 60

ggcatcaatc tcatctgtgc tgtctatggt gattgccttc a 101

<210> 25
<211> 101
<212> DNA
<213> Homo sapiens

<400> 25
cgcagtgctt ctgagagcgg gaatccgcga actggagtcc cgtcttcctt ytggcgtcct 60

gtcttccttt tggagctccc cctcaaggac cccgggagcc c 101

<210> 26
<211> 101
<212> DNA
<213> Homo sapiens

<400> 26
aaatgacagg aacacatgga cacatagagg gaaaaaatag atgctgggac rtacctgaag 60

gtgcaggatg ggagaagggt gaggactgag aaactagaaa a 101

<210> 27
<211> 101
<212> DNA
<213> Homo sapiens

<400> 27
aaagaagatt ctccctttttg aaaataatgg aactccagga aagccaaata kgttcaacat 60

aattatgaga aagaagtgtg ccactgtcag attggcattt a 101

<210> 28
<211> 101
<212> DNA
<213> Homo sapiens

<400> 28

ggtagatcac aattccatga agagcaagca aatatgaatg gagttggatg mtaaacagca 60

aagtgatatt taagtgatca gactacatca cacttttttt c 101

<210> 29
<211> 101
<212> DNA
<213> Homo sapiens

<400> 29
ccagttgtct cacttttttt tttttaccca cgtctgtgtt cctcatctca yagcaacctg 60

gctttaactt ccatcccttc acaaaaatta cagaagccac c 101

<210> 30
<211> 101
<212> DNA
<213> Homo sapiens

<400> 30
ccaagttgta cactttaaat acaattttg ggttaatcta attcctttgc ygtttcatgt 60

aaatttttga attagattgt ttacatctat aaaaaataag c 101

<210> 31
<211> 101
<212> DNA
<213> Homo sapiens

<400> 31
tgcatacaga cctaaaatat cgtagttttg aaatgtgcat tgagggaaag mtaaggatta 60

gcctggtggc ataaaatatg ggcagcagct ggaggtgaag t 101

<210> 32
<211> 101
<212> DNA
<213> Homo sapiens

<400> 32
cctctgctca ttggcatgcc ccctgacatc tgtttcccct gtctttcact rttggaagtc 60

tcagagccta gaaacaattg gacacagaca tttccaattc t 101

<210> 33
<211> 101
<212> DNA
<213> Homo sapiens

<400> 33
actgtctcta caaggaaaac tataaaacaa caatgaaagt tactgaagag racattaaat 60

aatagaaagt tattccatgc tcatgctttg aaaaaattat t 101

<210> 34
<211> 101
<212> DNA
<213> Homo sapiens

<400> 34
caggaagcac tggaagtagc tagcaagaat aatagttcct tgaggatggg rccgatgcta 60

tgcttttta tgatgctcca ctgaacttac aataattctg t 101

<210> 35
<211> 101
<212> DNA
<213> Homo sapiens

<400> 35
ttactttctg ggctgatgaa agtgttctgg aatcagcagt gatggttgtg yaatcctata 60

agtacataaa ccactacttt ttaaaaagct ttgtaaatac a 101

<210> 36
<211> 101
<212> DNA
<213> Homo sapiens

<400> 36
acctcagttt taccatcttt aaattgggtg taataatgag atctatctta yagctctgtg 60

aagattaaag gagttgaaat tggaaatggt aggtgctcaa c 101

<210> 37
<211> 101
<212> DNA
<213> Homo sapiens

<400> 37
gtttctacat tctgaccttg ttctgtgctc tgcggggctg atctcaatgg maagtgtctc 60

tggatttccc ttatccctta ttggctttga ctaactgggg g 101

<210> 38
<211> 101
<212> DNA
<213> Homo sapiens

<400> 38
tgggttgaca tatgagaaca tggaagggcc atgtaacagg tttagtctag kcagccaagc 60

cttttcaaag tgatttctaa attgggtaat ggagggtggg t 101

<210> 39
<211> 101
<212> DNA
<213> Homo sapiens

<400> 39
aagaaaacaa aaacagactc tttcttacag agtaagagga aaaacagaaa rtgaaggcaa 60

aaaacaaatg aaaagatgcc ctttctattt tctgaagcca g 101

<210> 40
<211> 101
<212> DNA
<213> Homo sapiens

<400> 40
aaggcagaac gccagaacag gtggatatga gtcccaagcc actgtgctca mtcaccgtgc 60

taattctgcc tccctgcagc tgctgtggct gataaggagg a 101

<210> 41
<211> 101
<212> DNA
<213> Homo sapiens

<400> 41
ctaccaacct gccttccttc ctgttaactt aatgagctgt tagtgctcaa yctaatggtg 60

agttcattgt ccttatctta tttgacccag caacagcttg t 101

<210> 42
<211> 101
<212> DNA
<213> Homo sapiens

<400> 42
gttgacattc ctggtaggaa gatccacaaa gcatttggtc ctattgccag rggtatcatc 60

actggttctg aggagtggaa agagtacaca ggcaaggcag a 101

<210> 43
<211> 101
<212> DNA
<213> Homo sapiens

<400> 43
caacatcatg tcatttctgt gagcagagcc aaacattgtt gcctgagaga rccccaagga 60

gggcttgaaa agagtttctc atcagcaatc tcatactcat t 101

<210> 44
<211> 101
<212> DNA
<213> Homo sapiens

<400> 44
ttccccacta gagtggaagc atcctgagga cagggacctt tgctgctttg ytcaccactc 60

aatcatcttg cccagaactg agcttggtac attgtaagat g 101

<210> 45
<211> 101
<212> DNA
<213> Homo sapiens

<400> 45
tgactgtata tacaaaggtg gaattgctgg atcctatgtg ttaaaccttt racctcttga 60

agaattggca gactgtttgc caaagtagct gcactaataa a 101

<210> 46
<211> 101
<212> DNA

<213> Homo sapiens

<400> 46
tctttttggc tattcccctt ctgtgccctt tttgcagaag taaactctgt kgggaagggt 60

aaatgtgtag cctcaaattc ctcattaagg ttttatttta t 101

<210> 47
<211> 101
<212> DNA
<213> Homo sapiens

<400> 47
gctcagtaaa tatttgttaa atgaatgaag tgtatgtttc tgcaaatgct rtcagaatct 60

cattttatct ctctgacaag actgcacctt tagtgcaggg a 101

<210> 48
<211> 101
<212> DNA
<213> Homo sapiens

<400> 48
agagaacagc attgacagag acgattagtt tcctcccccg cccccagtcc rttggcctct 60

gttgctaata acgcttggtt gaggattata ttaaaatgag t 101

<210> 49
<211> 101
<212> DNA
<213> Homo sapiens

<400> 49
atgcttccag ctctgttatt tttcttaaaa ttgcttgggc cattcgagct yttttttttg 60

tttaatatga attttagggt ttgagtacat tgaagctttt t 101

<210> 50
<211> 101
<212> DNA
<213> Homo sapiens

<400> 50
acaaagtttg tgtataatac atgccaagag ggtaggaata aaataccatt ygctgtcaag 60

atatatttct aaacaagttt attaggaagg cagtagcaga t 101

<210> 51
<211> 101
<212> DNA
<213> Homo sapiens

<400> 51
agtgtttatt aatgaactag ccatagtaaa attacagccc atttaaacat ycctctttga 60

ctaacactag tgtctatccc ttgccattgc agcaatgatc t 101

<210> 52
<211> 101

<212> DNA
<213> Homo sapiens

<400> 52
gaggaaaaca atttctcaat ccacggttat ttctttgtta tactaagaac mgtgcccaat 60

acttatggaa caaaataagc ctatcatttg gacgtctcct a 101

<210> 53
<211> 101
<212> DNA
<213> Homo sapiens

<400> 53
agaggcaagt gtcagaaatt aagcaagtaa acaacagaac actgtgagcc rttggtttgt 60

aacatgacag ctgcctgtct gtgcctctta ctgtgtctgt g 101

<210> 54
<211> 101
<212> DNA
<213> Homo sapiens

<400> 54
gagctaggct aaaatcagga cccaagaacc tcacctaaga tattttacag rgataaaacc 60

attatctatt catttttcaa aatccccctt taatccaaat t 101

<210> 55
<211> 101
<212> DNA
<213> Homo sapiens

<400> 55
cctttttcct ctctctagaa agggaggatc accaggaaga aataagtcca rattccccat 60

cagttcagtg gtatggagtc cagagtcaga atataatttt t 101

<210> 56
<211> 101
<212> DNA
<213> Homo sapiens

<400> 56
cttatatgag ctatgaatta gcccgaccac catcactgct actgctacta ygccccagac 60

tctctgtgct gctgccttgc cagcctgctg tgccctgctg a 101

<210> 57
<211> 101
<212> DNA
<213> Homo sapiens

<400> 57
ggtgtttggc agtgctgttg ttcaaaaata tggccaaggc ttcttaaata yactgactgt 60

tggattccct tccctgcctc cactccctca tctgctgaat c 101

<210> 58

<211> 101
<212> DNA
<213> Homo sapiens

<400> 58
cttgactaag tggagggtat tgtggagtag agcccttctg aataacagca rctaacattc 60

tcatagcact aactgcaccc ctttgaggta ggcggtctta t 101

<210> 59
<211> 101
<212> DNA
<213> Homo sapiens

<400> 59
gcaacagaga aaaaaatgtt ttttgtttat tttagcatgt ttatttttgg yccaagcctt 60

tatcaggttg gagttggagg ctggggagga agaataacaa a 101

<210> 60
<211> 101
<212> DNA
<213> Homo sapiens

<400> 60
ttttaaaaat acaaattaaa aattatctat tggacagagc catgtgtaga ycttagcctt 60

tgcacttgca aatcaaagct ttacaagaga tgctctccaa a 101

<210> 61
<211> 101
<212> DNA
<213> Homo sapiens

<400> 61
ttaaaaaaac ttcatttaca ccagaatgat ttccgtctgt cactcattga ytttacctct 60

ttttttctac ctctaattac tataaaaata tttgggatgg t 101

<210> 62
<211> 101
<212> DNA
<213> Homo sapiens

<400> 62
ggcaaagggg ttaggtgtca atgcctggct gatttcctgc attacaaaat ktacctctta 60

cttttctgtc ttcctgatgt taccccctct tttctttcac c 101

<210> 63
<211> 101
<212> DNA
<213> Homo sapiens

<400> 63
tttccctgat aaaaaggcat cttgtccaca gctgtacttg ttttcttatt ragtgatcct 60

ggttatagaa catgtgactt caggcataaa attctttcta c 101

<210> 64
<211> 101
<212> DNA
<213> Homo sapiens

<400> 64
aggaaacaca aacttctaga acttttaaat tgttaaacat ctttgtggaa ktaactacca 60

ttttcaccaa atctgcaaat catattccaa caagttgtaa a 101

<210> 65
<211> 101
<212> DNA
<213> Homo sapiens

<400> 65
tgtggctgtt aagtggtgac tgaagtagaa tggaggtgaa aataattcaa ratggaaagc 60

taaaacaacc gagaggcttg gaagctgaag aattccttca t 101

<210> 66
<211> 101
<212> DNA
<213> Homo sapiens

<400> 66
cacatacgca tatcctcctc aattttataa agaaatagaa gcaccattcc rcaccttcat 60

attccaccct taatcattgt taagttggtt gcatgtcttc c 101

<210> 67
<211> 101
<212> DNA
<213> Homo sapiens

<400> 67
gcaaagaggg ccagtagtta cactgcacca ttgtggtgac atcaccctat rtatgtattt 60

tttaaataac ttgttaatgc atatttccct agctagacta a 101

<210> 68
<211> 101
<212> DNA
<213> Homo sapiens

<400> 68
ttttggctgt taggctgtag agactttatg agggtgccaa acttggaaga matattgaag 60

gtagactcaa cagaattttc acaatatgaa ccctgtgaga c 101

<210> 69
<211> 101
<212> DNA
<213> Homo sapiens

<400> 69
ctattgtgag gcagggtgtg gaaatcgtga ttgagatgac aaggcaccca rttgtactca 60

tataaagaac actgcttgcg cgtatgattg ctgttcaggt c 101

<210> 70
<211> 101
<212> DNA
<213> Homo sapiens

<400> 70
tagtatgctt attaaatctg cagatgaatg catcttgtca aggaaaattt yctatgttac 60

aactgaattt cttctatttc acatgttgag gtctctttgg a 101

<210> 71
<211> 101
<212> DNA
<213> Homo sapiens

<400> 71
gacaggtctt ctttcctgcc agagggagct ctgaagacaa ctagagaatt ytgggcctga 60

aatttcaatc tagttagaaa gaaaaatgag gcaatgattt t 101

<210> 72
<211> 101
<212> DNA
<213> Homo sapiens

<400> 72
gacagggcac gtaggaatat ggaagtcaga aggacaacac agctctgcta ygtcccggtt 60

cttggtaact ttcttaaccc cactatgctt tatctttagt t 101

<210> 73
<211> 101
<212> DNA
<213> Homo sapiens

<400> 73
tgaggagagt tcctgggcca agggctggct ggcccatgtg acttttgggg kctcaggagg 60

agcctgttgt gttggggagt ctctctgctc aggtcctgtg t 101

<210> 74
<211> 101
<212> DNA
<213> Homo sapiens

<400> 74
gccccttggc tggttcttac ccatcagcaa gctctgaatg cggtcgtaat rtgtgaagtt 60

gtaggtgctg ctcgtggagg ctgcctcatc cctgggcagc g 101

<210> 75
<211> 101
<212> DNA
<213> Homo sapiens

<400> 75
tgggcaaatt cgctatgcat caggctgacg gcctggagga agcggcgatc mtgcggggtg 60

gccacctgcg gcaggtttgc ttccagaaga ggacacagag t 101

<210> 76
<211> 101
<212> DNA
<213> Homo sapiens

<400> 76
gggttcccac ccagacagac ggactcaaga actcacgcac tgcctctgca ycctctgctg 60

ccaatgaaaa tttaaatgag ggcaacagga gatcagagat g 101

<210> 77
<211> 101
<212> DNA
<213> Homo sapiens

<400> 77
tgaaatctac aaggtgcctt tcatcacgag agctgagcga tgacccctga rtgaggaggg 60

ccaggagctt agtcccatct cagagacaga cactgactca g 101

<210> 78
<211> 101
<212> DNA
<213> Homo sapiens

<400> 78
tccttgaccc cattcgccct cttacaaata atgaggttca gaaggcaggt rcaccagatg 60

ggagggagaa acaaaaataa agataaacga aacaacattt a 101

<210> 79
<211> 101
<212> DNA
<213> Homo sapiens

<400> 79
gcacttcatt tattcaccaa atacctgctt tggaaaataa ttggagtcgg rgggagcagc 60

aagaagggtg aaatagggca gtgcagggct cctggattgg g 101

<210> 80
<211> 101
<212> DNA
<213> Homo sapiens

<400> 80
ttcataggca tgcaagcctt cttatgaact aactgcacgt gccagggatc raggttgcac 60

actccttata agaatctaat gcctgatgat ctgaggtggg a 101

<210> 81
<211> 101
<212> DNA
<213> Homo sapiens

<400> 81
atcatggcag aaggcaaagg agaagcagga accttcttca taagggggca rgacaatgtg 60

agtgccagca gggaaaatgc cagattctta taaagccatc a 101

<210> 82
<211> 101
<212> DNA
<213> Homo sapiens

<400> 82
gctgaactgg ccatggaaat ggcagcctgg gcaacaggtt catgaaaaca ractttcac 60

acctggtcct gctctccagg cctgagcgaa ctccatgtgt t 101

<210> 83
<211> 101
<212> DNA
<213> Homo sapiens

<400> 83
ggctcttgtg ggacagggct agtggaacct acttgggtgt ctccattgcg rgcagaacgt 60

aatagctgtg tgtagaaggt cccactggat gaagggccag t 101

<210> 84
<211> 101
<212> DNA
<213> Homo sapiens

<400> 84
tggctggagg aacccaggaa caccctgagc atccatgttc ttaatgacaa ragagggaac 60

acagatttgg cttccctttc ttcataagaa aagaaagaaa a 101

<210> 85
<211> 101
<212> DNA
<213> Homo sapiens

<400> 85
catgcatatc cagaaactac agtaatttac aggggcaaac tctgcaacta rgaaaaggag 60

acagaactgt ttccactcaa tgcattcctc catcaaagaa c 101

<210> 86
<211> 101
<212> DNA
<213> Homo sapiens

<400> 86
ttgtgtttct gtgtggctga aatcgtgtcg taaagttaga agaaaggctg ytgtggggcc 60

tgcgttgctt ggcagaatgt tccttacctt ttgatttgca g 101

<210> 87
<211> 101
<212> DNA
<213> Homo sapiens

<400> 87
gtgccaagca gagcaggtag ttggctaagt ttgcctccag gaaagaagtc yctggagagc 60

gagctggttc tagaaagctc cattattata ttcctattgc t 101

<210> 88
<211> 101
<212> DNA
<213> Homo sapiens

<400> 88
gtcagtggtg atattctctt tatcattttc attgtgtcca tttgattctt ytcacttttc 60

tttgtctagc tagcagtcta tctattttat taattttttt c 101

<210> 89
<211> 101
<212> DNA
<213> Homo sapiens

<400> 89
cccatgtaag acacccatga aacaatgctc tggtcataat tagtctctaa mctttcaaaa 60

tgcctgcttc agtgacctca cctgctattg aacacgatgc c 101

<210> 90
<211> 101
<212> DNA
<213> Homo sapiens

<400> 90
agccacctct catttgcatg gtggacagct gcggctgaca ggcaaacaaa ratgtctgcg 60

gccatggcag ctcctagaga aactcttctc tccttactct c 101

<210> 91
<211> 101
<212> DNA
<213> Homo sapiens

<400> 91
ctgcgcttcc cccagaaagc atgcctgggt gaggggccag gtgacacttc ytacgatctg 60

gattttaaaa tatgtttgct tatgccttca ccctccacca a 101

<210> 92
<211> 101
<212> DNA
<213> Homo sapiens

<400> 92
gcgctcacgg gagggcggat gtggagaggg cagaggagca atggtgacct rggaaggtac 60

cctgagcggc tacgctagga tctctgttct gcagacttct g 101

<210> 93
<211> 101
<212> DNA
<213> Homo sapiens

<400> 93

agggaagcat cagatgtcac tggcttggga aagatattcc agaaggaagg racaggttgt 60

acaaagtaag gtaattttgt ttggggaagc tccagcaggt c 101

<210> 94
<211> 101
<212> DNA
<213> Homo sapiens

<400> 94
agttatcagc ttattgctat taaaaataac actaaacttt tgtttatcta magagtgtca 60

ggtaagcaag tgaacatttt gatgcaaaaa gaaatcactt t 101

<210> 95
<211> 101
<212> DNA
<213> Homo sapiens

<400> 95
ggctgagtaa attaaggtac atctgtatta aggaataaaa tgcaactacg raaaatgata 60

aactagatgg aggggtgcct atgacactgt aaagtttaac a 101

<210> 96
<211> 101
<212> DNA
<213> Homo sapiens

<400> 96
tggctgtgtt ctgagtggga gtgtcctaag agtgagagtt cctagtgacc yaggcagaag 60

ttgggttgac acttcttgca agatttctga tgacctagcc t 101

<210> 97
<211> 101
<212> DNA
<213> Homo sapiens

<400> 97
ggtctctgtg gattcccaaa ggaggtttca aatggagtca ttgtaaagac rattcatgat 60

cttagaagtg tctcatgcag tttcctcgtg atggtcttgt t 101

<210> 98
<211> 101
<212> DNA
<213> Homo sapiens

<400> 98
caggaatccc aattatgggg aaagaagatg agcttctgag actattccga kccacaagat 60

ttttcaaatt cttcacaatc tctgtctcat ggatcagaga g 101

<210> 99
<211> 101
<212> DNA
<213> Homo sapiens

<400> 99
cactgtacct tcgcagcacg aggagaggag agttcgaaac cacaaagctc yttcctttct 60

ttcaggagaa agaaaatgga ggatgggaac gtcatcagcc c 101

<210> 100
<211> 101
<212> DNA
<213> Homo sapiens

<400> 100
gggcctcaat tttctcagct ataatatggg ctgacaagag taaacgacaa kagcaaatga 60

gttaatatgt gttgcccctg atgttacagt ggataacgat g 101

<210> 101
<211> 101
<212> DNA
<213> Homo sapiens

<400> 101
aatcttaaac agtaaagttt cacgaagaca aaaatctttt tgatcaatca ygtctctttt 60

acaaagttta caaggaaagt attcatccct aaaactattt t 101

<210> 102
<211> 101
<212> DNA
<213> Homo sapiens

<400> 102
gagttactta tacaaaatta cacactaaga gatttgtatg tataattgtg kgtacacatt 60

cctagtattt tcctgatata aaaaaattat tcctatataa g 101

<210> 103
<211> 101
<212> DNA
<213> Homo sapiens

<400> 103
gaaggagttt ggatatattc cctcttcttt aatttttttg aagaatttga rtagaattag 60

tgttagttct ttacatgttt gttagaattc agctgtgaag c 101

<210> 104
<211> 101
<212> DNA
<213> Homo sapiens

<400> 104
agttagtaca ggagcggggc caggagagtg ctgtcccctc agctccagtg rgtggctgcc 60

catccagagc aagcctgcag cccccacccg cctcctcctt t 101

<210> 105
<211> 101
<212> DNA
<213> Homo sapiens

<400> 105
tcttgaatgc aggaactatt atataaaagc attgcagctc ttggtggttg yggcagagac 60

gcagagaaag ccagtttgca ttgaaggaag ggtacagcag a 101

<210> 106
<211> 101
<212> DNA
<213> Homo sapiens

<400> 106
tgctatagta cacatagcaa atctgcaaaa gtgctagcta tcattattat mtgaggcttt 60

tgacccagct ctcagagaag ctggaaattt gcatttttat g 101

<210> 107
<211> 101
<212> DNA
<213> Homo sapiens

<400> 107
ggagaatgca taatgaggct gaatgagaat tagatgctta attgaggcct rgaaaaggga 60

aagaaaaagc cagacatgtg gaatgtgatc agaatgcagc t 101

<210> 108
<211> 101
<212> DNA
<213> Homo sapiens

<400> 108
acagactgtc cttggaatgt tggaaagtta tttggaaagt ccttatgagc ytggggcaca 60

ttcttctgaa gagctttctt gattaggaaa atcctgtgct t 101

<210> 109
<211> 101
<212> DNA
<213> Homo sapiens

<400> 109
tacacacaaa ttcatgccca cacccataga cacacatata catatataca ygcatgtata 60

tgtccgtata gagagctcta tgctggaata tacaaaaaca t 101

<210> 110
<211> 101
<212> DNA
<213> Homo sapiens

<400> 110
gagcttcagg acttcaagta gatcacaaaa aaagtgtgga atttccattt yggtgcagaa 60

ggacagcctc aaaacagtca aggtctcgag cagggaaccc a 101

<210> 111
<211> 101
<212> DNA

<213> Homo sapiens

<400> 111
gcctgggggg tggtaatttg ggagccactg aaatgaactt gcaaaaggtt ktgggactat 60

tcatttatct gcagaaggct cagaaatttc attagattct c 101

<210> 112
<211> 101
<212> DNA
<213> Homo sapiens

<400> 112
tttgtttttt tgtattttca caataaatat gaaaacagtt ttaatttaat kattatgaac 60

aaaaaaggat gaaaaccaat agtcagtttc tttgtaaaat t 101

<210> 113
<211> 101
<212> DNA
<213> Homo sapiens

<400> 113
caccacacag gaagggattt tgtctgtcat gttcactgct gtgtccccag yatgctaagt 60

aggggccagg gtcaaagtaa atgcttgatg aatctttgcc g 101

<210> 114
<211> 101
<212> DNA
<213> Homo sapiens

<400> 114
tccccacttc ttgcataaag ggtagcattc atgagcatac cgttctgcac yttgcttttt 60

tcatttgtgt cttgaaacct gttccctgtt ggctaagaga g 101

<210> 115
<211> 101
<212> DNA
<213> Homo sapiens

<400> 115
gccttggacc tgctgggccc agccactggc tgtctactgg acgatgggct ygagggcctg 60

tttgaggata ttgacacctc tatgtatgac aatgaacttt g 101

<210> 116
<211> 101
<212> DNA
<213> Homo sapiens

<400> 116
ggccctcatg ctgtaaagaa gttgagttct ggaaactcca agttatcatc rtccaagttt 60

agcaatccca tcagcagcag taagaggaat gtctccctcc t 101

<210> 117
<211> 101

<212> DNA
<213> Homo sapiens

<400> 117
aagagtgcat aggagttttc taggcagaga aaacaaccct gcaggcgcac rttggctccc 60

attcctggat tgagggcgtg gccatgaagt ctgggtgctg c 101

<210> 118
<211> 101
<212> DNA
<213> Homo sapiens

<400> 118
caggaggggt caacttggag ggccaagcaa ccaggggtca catgggcata yggctgagcc 60

tggacccatc cacctgacta ctatgctatt atagggctcc c 101

<210> 119
<211> 101
<212> DNA
<213> Homo sapiens

<400> 119
agaagtttct ttattgagaa tgatattcat tagtaggcat tcaatgataa rgacacagcc 60

tgattttaaa gatttccttt tttttttttt ttttgcacat g 101

<210> 120
<211> 101
<212> DNA
<213> Homo sapiens

<400> 120
ctccaagggc ggatggcctg accgggataa gacccgtgaa cagatagtaa rtgtgggttt 60

ggcatttggc aggaaatgct tgtggaattc aggaggcaac t 101

<210> 121
<211> 101
<212> DNA
<213> Homo sapiens

<400> 121
tgtgctcagg caagattatg gagcgagctt ggttttgtcc tactccatcg yggtcagagt 60

ggccccatct gatatgagcg ttctgtgagt tttttttatt a 101

<210> 122
<211> 101
<212> DNA
<213> Homo sapiens

<400> 122
gattacaagc gtgagccacc acacctggcc ttgaggtcac ctttgcatgc raaggctgta 60

tactgctaac acctgtgaca tctcctgtct gatggtgtcc t 101

<210> 123

<211> 101
<212> DNA
<213> Homo sapiens

<400> 123
aaatttttcc tgtaattgac caagtagcaa atatattcag ctttgctggc ygtaaatttc 60

ctggcaatga ctcagtcctg ccgcggcagt gtggttaaca g 101

<210> 124
<211> 101
<212> DNA
<213> Homo sapiens

<400> 124
tgtcgaaaaa cctatcaaca attccttagt ttcaccactt caaaaaattt rttctagtgt 60

caaatcccac attttaaata aatacagaaa tgattttgat g 101

<210> 125
<211> 101
<212> DNA
<213> Homo sapiens

<400> 125
gaaggaggga tttggagcca gggcagacag agcagcatgg tgctgggaga rcaagagggg 60

cagccagtga taaggagagc acagggagaa ccacagcctg g 101

<210> 126
<211> 101
<212> DNA
<213> Homo sapiens

<400> 126
gcacattatc tatgctgttt gttataggta atagtttcag caaactagac mggaaggaaa 60

aaatgcatta agagtgaagg tgaaagagag agcgagagtg t 101

<210> 127
<211> 101
<212> DNA
<213> Homo sapiens

<400> 127
acaagatatt ccctctgatc tctggccctc tcctccagcc ctctccaaga rggacattgt 60

ccttgcctcc tatcccagag agctggcaaa tattccccta c 101

<210> 128
<211> 101
<212> DNA
<213> Homo sapiens

<400> 128
gatttctcct gtgtgggcaa gtcacacaca aaactccaga aatacatatt yaaaatgctc 60

ctagcttccc tctgcattag tcacaataac actaaatgct g 101

<210> 129
<211> 101
<212> DNA
<213> Homo sapiens

<400> 129
agcaagactc catctcaaaa acaaaaaagg caaattaaat ttatactaac rtcagcaaac 60

tagagaattt aatggctcat gtaactacag gtagagatgg g 101

<210> 130
<211> 101
<212> DNA
<213> Homo sapiens

<400> 130
atagctcctc ttttattact cggtcctggg gttaacctca attgtatcca yttactcaac 60

tagtgtttaa tgagttgcca tggtgtgcct cgtacttgtg a 101

<210> 131
<211> 101
<212> DNA
<213> Homo sapiens

<400> 131
tcatagcttc ctttgtacct caaactaagt agcttcatat tcctttgctc rtgcaaccca 60

atcatatttg ggaagctgca gatgaaaagc atactgactt t 101

<210> 132
<211> 101
<212> DNA
<213> Homo sapiens

<400> 132
gggtcatctg acaataaggc cacctaaggt ccgccagtag tagttgtaga ygaactggtg 60

acttctggca tggtcattag ggcaattgtt aaaactttta t 101

<210> 133
<211> 101
<212> DNA
<213> Homo sapiens

<400> 133
tgtttgctga gccttctctg cgctgtgtat agtactcagg gaagcttcac rtaagtgtct 60

tccttcactc atgtgttcgc tcaggaaata cgtatttact g 101

<210> 134
<211> 101
<212> DNA
<213> Homo sapiens

<400> 134
gccatggaca ttccgggttc ccaagtcagg tggggcccag ggataagcat ytatttttga 60

tcagcacctc aggtaactcc tgtcttcacc atagtttgaa a 101

<210> 135
<211> 101
<212> DNA
<213> Homo sapiens

<400> 135
tatcttattt attttcaagt cacaccaaag gaaaggcaag gctcagagaa rtggattaat 60

ttgctggagg ctacatagta agcagagggg gtgggatatg a 101

<210> 136
<211> 101
<212> DNA
<213> Homo sapiens

<400> 136
tataagtgta tatgtagaag aaaatgtccg gagtctggag acagaaccaa kagagagaat 60

tagaggttag atttccagtg cttacacaga gccagtgtta t 101

<210> 137
<211> 101
<212> DNA
<213> Homo sapiens

<400> 137
ctgtacaaag tctgaatttt gggggaatct gaagagtctc atttaaatat ycagctgatt 60

aattataagt gtatatgtag aagaaaatgt ccggagtctg g 101

<210> 138
<211> 101
<212> DNA
<213> Homo sapiens

<400> 138
tcttctcatt acttcagaat acagacatcc agtgtttaat tctgtttgtg rttatctcat 60

aattattaag atatattcat aactatttgt ttattaatca a 101

<210> 139
<211> 101
<212> DNA
<213> Homo sapiens

<400> 139
agaacaaaag taggtgattg atatagtttg gatatttgtc ccctcttaat yttatgttgg 60

aatgtggttc ccaatgttgg acatggagcc tggtgggaga t 101

<210> 140
<211> 101
<212> DNA
<213> Homo sapiens

<400> 140
acaggacatg ctcaatgtgg gcttttttta aatttttttt ccttctcttg ytttctttt 60

atttctgtgc gattacctgc tcctctgtgg tttctttatt g 101

<210> 141
<211> 101
<212> DNA
<213> Homo sapiens

<400> 141
ctgacaggca gaaatatatg ccacccccaaa atatgtcagc ctaaaagatg ycttctcaat 60

tgaaggcaat tgagaagaag cagatacaag aaaagctctc t 101

<210> 142
<211> 101
<212> DNA
<213> Homo sapiens

<400> 142
gaggttgata aacatgatgg tgaagatgtt gagcagtttt ccttaaaact rgttctcaat 60

tcactgctga tttgtggaaa tctggcactg tctataccag g 101

<210> 143
<211> 101
<212> DNA
<213> Homo sapiens

<400> 143
tacagtgtct agatgtgcta gtgtatccag aatggtgccc aagagagaaa mgtaggttag 60

gaatatattg agctgaccta ttttccatac gtaagtatgg g 101

<210> 144
<211> 101
<212> DNA
<213> Homo sapiens

<400> 144
aatataaaaa catttgactt aagattttct gaggaagctt aagtagtttc rttgaaggct 60

gaactggttt ggtcctgaat ctcatcctct atggcataat t 101

<210> 145
<211> 101
<212> DNA
<213> Homo sapiens

<400> 145
cccaaactct cctttcgatc ctttaatctc ccttaatcat ctcttgaatc ygcctcttcc 60

tgtctattct cacacactct gttctaacct agaaccactt t 101

<210> 146
<211> 101
<212> DNA
<213> Homo sapiens

<400> 146
gaaaagacct caaatttgct agtaagattc aacgataaat gcaaaataca yacatctaca 60

cacacttact tagaagggta gtaagataga catatttgac a 101

<210> 147
<211> 101
<212> DNA
<213> Homo sapiens

<400> 147
atgccccgt ttaacctctg aaaccttgtc attaaactac agggaattaa rtccaataat 60

aaacccttcc attgtcaaca gaactctcaa tgaactgtac c 101

<210> 148
<211> 101
<212> DNA
<213> Homo sapiens

<400> 148
gatgattgta gagcataaag aaactaattc acgtaaaaca ttttcatgtc yaggatacag 60

gtttcaataa atattagtca gaagcatcgt gatcattttg t 101

<210> 149
<211> 101
<212> DNA
<213> Homo sapiens

<400> 149
catcgtcact gggttaggtc tcaatgtcgg cagggctggc tgaggctctc rggaggatta 60

tctttccttg ccttttcca gcttctagaa gccaccttca a 101

<210> 150
<211> 101
<212> DNA
<213> Homo sapiens

<400> 150
actgcccgct ctccttgcct tcatggggcc acaactttct gacttctccc rtttgctttt 60

gcagacacct cctcttcctc tagatattct tctccagaga g 101

<210> 151
<211> 101
<212> DNA
<213> Homo sapiens

<400> 151
ggcaagtcca gcaagtctac atatttctag tcacatttcc ttgcctataa yttattaatc 60

catttatcaa atatttattg agcacatact tactatcatg t 101

<210> 152
<211> 101
<212> DNA
<213> Homo sapiens

<400> 152
cacaggatgg aaacaaaata tcatgagggt ccagcagtct tcagagcagt rtttttcag 60

ctggggacag aaacaccagg aggcttatga ggagtttcta g 101

<210> 153
<211> 101
<212> DNA
<213> Homo sapiens

<400> 153
ttgatgtcat ttgggacaat ggcagaaccg tctccttctc caagttctaa maatgaactt 60

agatgactgg caaaaccccc agagtgtgaa ggcttgtagc t 101

<210> 154
<211> 101
<212> DNA
<213> Homo sapiens

<400> 154
catgtgacag gaatatacta gatgtatcta caagttttct tatgacacag rtattcatga 60

catcaatctc atgacacagg tagtaggaat atattttaaa g 101

<210> 155
<211> 101
<212> DNA
<213> Homo sapiens

<400> 155
aactggaact gctggttaat cttgaatcag acaaagagca ccatggacac ytcgaggaag 60

tgcccacagc ccagcaacaa aagtttctgc agagatttct t 101

<210> 156
<211> 101
<212> DNA
<213> Homo sapiens

<400> 156
aagtcaaact atccgtgttt gcagatgaca tgatcctata tctagaaaac yccctaatct 60

tagcccagag cttcttaggc tcataaacaa cttcagcaaa g 101

<210> 157
<211> 101
<212> DNA
<213> Homo sapiens

<400> 157
ggtggcatta tttaaaatgt actaaggtat gactcagtca tcatgctaaa rcattattgt 60

accttatata aacatgactg taattcgatg ttttaaattc t 101

<210> 158
<211> 101
<212> DNA
<213> Homo sapiens

<400> 158

aaggaaaagt ccttctaact tctacagggc caaagcatgc atgtatcata ytaatgtcaa 60

tcctgtgcca gaccctttgt aaaattaagt acttcaaact t 101

<210> 159
<211> 101
<212> DNA
<213> Homo sapiens

<400> 159
cctagttggc cacagggagg gctggtcaac tgcaggggca ggcaggggta yacatgaccc 60

aggcctagcc tggaagtgtt ctcagcctgg tcctgctccg t 101

<210> 160
<211> 101
<212> DNA
<213> Homo sapiens

<400> 160
cattttctac aattgtgaaa atcagacacc gcagtaggat tagtgtaagc rtcgtggttt 60

ctaggtagtc ttctctgaca cctaggcaga atcagggccc t 101

<210> 161
<211> 101
<212> DNA
<213> Homo sapiens

<400> 161
gccttcaaag cggcagtggc cacccacaca gggaactagt gtttgtgaga rgagaatgaa 60

cgttgtttgt aatatgttgg tgtgaattgt cagcagagca c 101

<210> 162
<211> 101
<212> DNA
<213> Homo sapiens

<400> 162
gctgaaaggt ttccatgtgg aagcccctga ctaccaccaa ccagttcagg ygagagacct 60

gaatcctttc cccctttttct ttttaccttt tctgaatcct a 101

<210> 163
<211> 101
<212> DNA
<213> Homo sapiens

<400> 163
atctcaatat atttcaacaa tgggaacttc tgcggggcac aactcatgtc yacagcctcg 60

tctatgtaca gagcccaaag cagcaccact atcagtttgg g 101

<210> 164
<211> 101
<212> DNA
<213> Homo sapiens

<400> 164
ttctaccacc gtagatccgt tttgcctttt gtgtctggtt tcaatgcatc rtaggtccac 60

gacatccttc cacaggtacc ggccactcat tcctttcctt g 101

<210> 165
<211> 101
<212> DNA
<213> Homo sapiens

<400> 165
ataggcacat atcggatctc ccagcctggt gactcttccg tggtctaatc kgaacacctc 60

tggcctgcca cacctctggc cagcctccag ttagctgctt t 101

<210> 166
<211> 101
<212> DNA
<213> Homo sapiens

<400> 166
tcctagggaa cgccctcttc tcgctgcggc cctggcgtgt gtcgctggat kgtgagggcc 60

ccactgcatt ggtctccatg tgctctgcct tctcaatgtc c 101

<210> 167
<211> 101
<212> DNA
<213> Homo sapiens

<400> 167
agatgggggc agtcctttgg caggggtgct caagttggtc gattatccca rcggtgccag 60

agcggcagtg atttgtgggt gggcaggctc cttccctagg g 101

<210> 168
<211> 101
<212> DNA
<213> Homo sapiens

<400> 168
tctgctgcag ttcatagggt tcttcctgtt ggtctccata ccactcaccc raagcatgcg 60

agaagctgca ggggcttggg ggcagttgga gttcatgtgg g 101

<210> 169
<211> 101
<212> DNA
<213> Homo sapiens

<400> 169
gatgtatgtg tataaattgc actcatggct ctaaaacaaa tcagcagaac mcattctaga 60

aaaaatcgca ttcaagagat actatactaa tagattatgt a 101

<210> 170
<211> 101
<212> DNA
<213> Homo sapiens

<400> 170
aaaattactc ctggcctcag ctgcctcatg tctgggtccc tccctgccaa yagatttgtg 60

atggatattt acacgctgga agtgactggg ccatggtctc a 101

<210> 171
<211> 101
<212> DNA
<213> Homo sapiens

<400> 171
gggagaacta cagttcccag aagagtgtgc ggaagaagcg gcccatgctc ycggaagacg 60

ctgtggttga gcatcatggg agttgtagta ctcctgctgc t 101

<210> 172
<211> 101
<212> DNA
<213> Homo sapiens

<400> 172
ggccatccgt ggggcctgca ggagaacaag tggaatctgc agcatgggac rtctctgcct 60

agagcctgtg caaacaatgg cactgtcctc atcattgagg g 101

<210> 173
<211> 101
<212> DNA
<213> Homo sapiens

<400> 173
aaacacaagg aggcaccgag gctgctgtac aagagttggt tcctgctcac yccacaaact 60

ctacttccac ctactgcaaa aggttctgtc ctttttttta a 101

<210> 174
<211> 101
<212> DNA
<213> Homo sapiens

<400> 174
tgctgaccag ggaatacctc cccattgaag cctaggccag attccagtcc rttttgacca 60

taccccatca tggtatttta gagtacacct gaataagata c 101

<210> 175
<211> 101
<212> DNA
<213> Homo sapiens

<400> 175
cacgccccca cccgccgcag cccctactca ctcttcgtat aggagagcca ytatgtaggt 60

gagggccacc agcaccgtca ggagcaggcc cgtggggctg g 101

<210> 176
<211> 101
<212> DNA

<213> Homo sapiens

<400> 176
cagtccccac atttgcattg tccccaaatc taacccaagc tgaaagacat yaggcctatc 60

ttcttgcttt atgcataatg gcagatctcc agggagggag a 101

<210> 177
<211> 101
<212> DNA
<213> Homo sapiens

<400> 177
gccttttcat tcccctcttt ttttaataaa ggaaagccaa ttttaccggg rgtggcaaag 60

tgtctggaga aaacataaca tttcttagtt tcctttgtag c 101

<210> 178
<211> 101
<212> DNA
<213> Homo sapiens

<400> 178
tgtgtgcgtt ttcctgagtg tgcaggagta cgtgataatt tcctgctagg rtggaatgac 60

ttccgggtcc atgagtgtgg aattagggtc agctctgggt t 101

<210> 179
<211> 101
<212> DNA
<213> Homo sapiens

<400> 179
cagtttctga ggcccggttc tcccccaggg gctgggctgc aatcagcagg kactaaatct 60

cactgccaag ggcctgggcc aaggcatcca actctctgtg c 101

<210> 180
<211> 101
<212> DNA
<213> Homo sapiens

<400> 180
ctgaacagca aacccagagg ccattgcagc tgcctcggta ttctacaccc yccttgggtc 60

tggaagttgt tggaggcagg cataccagac tgtttataat a 101

<210> 181
<211> 101
<212> DNA
<213> Homo sapiens

<400> 181
gtgctctcat cctaatttag ggcccctttc tgcctagaac tctgtagatt yccgccgtct 60

gtgtttttcc atcatcccag accctcagct gcaagctcag g 101

<210> 182
<211> 101

<212> DNA
<213> Homo sapiens

<400> 182
cccacttgtt ctgcagagaa agtgagaggg aaaggttgct gatcagatgc ygctttaaaa 60

tgtaatcata agttttggct cagggagaga gagagagaga g 101

<210> 183
<211> 101
<212> DNA
<213> Homo sapiens

<400> 183
gttctagggc ctggaccagg ggcttaccta aagcccatgg tgcctcctcc rtctgaatgg 60

gagcctccac agccagtaat gagtatcctt cctcaaacct g 101

<210> 184
<211> 101
<212> DNA
<213> Homo sapiens

<400> 184
agtagtttcg tctctcagaa ccttataaaa tggataatag agtagtaccc mtccgatagg 60

gctgttgtca gggacaagga actaataccc atgaagcact g 101

<210> 185
<211> 101
<212> DNA
<213> Homo sapiens

<400> 185
tcagaaaata tttgcacaca cattgtctct tctggccctt gaaacattcc ytgtgtggct 60

gaagaaagtc aatagtggaa ccatttaata gataaggaca t 101

<210> 186
<211> 101
<212> DNA
<213> Homo sapiens

<400> 186
aaaatctttt agttcctaaa aagcacaaac ttaaaaaaaa aagggggaaa ygaaagggac 60

ttcttcaatt tggcaaagaa catctacaaa atacctacag a 101

<210> 187
<211> 101
<212> DNA
<213> Homo sapiens

<400> 187
atgttttcca tgatgagtgg gcaacagtta ccacccaggg ctgctccaca ragggaatga 60

actggagact tcacatgtgt tcaatttctt gaaagaaaat g 101

<210> 188

<211> 101
<212> DNA
<213> Homo sapiens

<400> 188
acacctgggg ggtgtactca ccttcttcga tgatgctttt cagcatttct rtgtacatgt 60

ccttgttgct gggagctgcg ctgttcatct tgaagtgggg c 101

<210> 189
<211> 101
<212> DNA
<213> Homo sapiens

<400> 189
ttaagagatg atttgagaaa gaataaatgt tgaatgagca tttattatag rgtcgtttat 60

gctacatttg cattttgact ctatttctgc catgcaggat g 101

<210> 190
<211> 101
<212> DNA
<213> Homo sapiens

<400> 190
gctcatcagc tgtagttagt gtatgtgtac tttatgtgtg gtccaagtca rttctttcag 60

tgtgtcccag ggaaaccaaa agattggacg cccctgtgtc t 101

<210> 191
<211> 101
<212> DNA
<213> Homo sapiens

<400> 191
acctgcagtg gactttgagc aagaaatcag cttttatgtg tcaatccacc rgaatttagg 60

gctttctctt aattgcagca aagcctagcc caccgtgagt a 101

<210> 192
<211> 101
<212> DNA
<213> Homo sapiens

<400> 192
tcatcctatt aaggccaggc tgcagaggcg ttgcgatgga gcagagattg rggagggggt 60

acggtgcgag tctctgcaag atgcacagca aggcagggag t 101

<210> 193
<211> 101
<212> DNA
<213> Homo sapiens

<400> 193
gagtgaggtg gaaatgtcgg tgcagcctgc agctcacctg gttgtcactc rcagatcggc 60

ctcggaaagc tccaggaagt tgatttggga tgagccagcc a 101

<210> 194
<211> 101
<212> DNA
<213> Homo sapiens

<400> 194
ttttctacaa aactaaacac tccaaacaca ggcacagcaa actgcatttc kaaaggtttt 60

gtaagttaaa caagccaagg aagttacatg gaaaaaaaaa a 101

<210> 195
<211> 101
<212> DNA
<213> Homo sapiens

<400> 195
agtgaaaagt tattgtgttc acttgaaagt ctaactggcc tttagaaggg ytatgcaact 60

agactcaggc ttcaagcata gcaagtggca tcaccaacat t 101

<210> 196
<211> 101
<212> DNA
<213> Homo sapiens

<400> 196
acatttgaaa cagcatgtta aactgtaagt acatcctcaa aatgcagaaa yctccattct 60

catcaagtta catgctcaca gtgacagcct gagaaggtag a 101

<210> 197
<211> 101
<212> DNA
<213> Homo sapiens

<400> 197
aagctgcctt ccttcttgaa aaatgttaat gtctccagta gccctaagaa rtccataggc 60

tccattctgt tattcaagat gccaaccaat ggttttgacc t 101

<210> 198
<211> 101
<212> DNA
<213> Homo sapiens

<400> 198
ccgagttctg gtaccatgac tgtgccgttc accattgttc ttcagcacct rgcactgggc 60

tggcactcaa caagaacttg ctagatcatg aagatgagca a 101

<210> 199
<211> 101
<212> DNA
<213> Homo sapiens

<400> 199
ctctgttagc taaactgagg aaccacaggc agggtggcct tgaatttcag kctgaaggac 60

ccatcaccca agagtcttgg cagcttcctc agcaaagatg a 101

<210> 200
<211> 101
<212> DNA
<213> Homo sapiens

<400> 200
ccagctgtct aaaaacatat atattttaga gtttgttttc ccaaataaga yctcatacac 60

ggttcatcca ctgtgtttgg ttattgggtc tctcaagctt a 101

<210> 201
<211> 101
<212> DNA
<213> Homo sapiens

<400> 201
atcacttcca ggctaaatgt cacactcaga tactcagctg cctacttact rgacacctct 60

actgagatgt ctgaattctg gaccctcctc ccaagccttc t 101

<210> 202
<211> 101
<212> DNA
<213> Homo sapiens

<400> 202
gggaagctct ggagcatttt gtgagcaccg tctcggtgga tgggaaagcc raagtctctg 60

cccgtctctt actggaggca ctaaaccccc tccctgggtt g 101

<210> 203
<211> 101
<212> DNA
<213> Homo sapiens

<400> 203
agtcaccacc ctggactata gtctgttgat tttctacctc tattctctta ytaaactttt 60

ggatacattc caaagcatca tggtcacttc cagttatgaa a 101

<210> 204
<211> 101
<212> DNA
<213> Homo sapiens

<400> 204
agcccagaga cctctttgga aagattacca aaccttgtta aaaacagaca yccttggggc 60

cagacacggt ggctcacgcc tgtaatccca gcactttggg a 101

<210> 205
<211> 101
<212> DNA
<213> Homo sapiens

<400> 205
tgaagaaagt ttaatgatgg atttttgttt aagtatgcat tcatccagaa racactttaa 60

ctgttcttca gagagacatg atgtggactc taactgatga a 101

<210> 206
<211> 101
<212> DNA
<213> Homo sapiens

<400> 206
tcagctatca caaaaaataa acgcaattct gaagatagca atagctcata racatcaggt 60

caaatctgca aagatgagca ttgtcctagg tgctaaggat a 101

<210> 207
<211> 101
<212> DNA
<213> Homo sapiens

<400> 207
ttaggtaaag cgaaaaatga cagaattaca ttaacttgac aaatcaacac mgatagcagg 60

aattttttca cacatttatt agtaagcaat tgtattagtc c 101

<210> 208
<211> 101
<212> DNA
<213> Homo sapiens

<400> 208
gagctttaaa aaaaaaaatg cctggactcc acccctaaag cttctgattt mattggccca 60

tttgtttaac tatcaatgac aatacagaga gatgctaaag t 101

<210> 209
<211> 101
<212> DNA
<213> Homo sapiens

<400> 209
aatggatgaa aagtaggatt ggtttgtttg ttttcaggaa gtgaggcaat ygtaaaaggg 60

aaaaatggga aaggcgaaac aagcaggatg tctttttttt t 101

<210> 210
<211> 101
<212> DNA
<213> Homo sapiens

<400> 210
tgaagagggc tatctgccta ttccagactt tatttccctg gaaacaaaaa rgaatatgca 60

caaatcactg tattttggat ttgaatatta tatttaaaaa a 101

<210> 211
<211> 101
<212> DNA
<213> Homo sapiens

<400> 211
aactcttgag caaggcatca agagttggtc cttaccccac gcttggtaca yttcagccac 60

acttaaggtt taccgttcct tttctcatgc catttcctca g 101

<210> 212
<211> 101
<212> DNA
<213> Homo sapiens

<400> 212
cgtgagacct catggttgtc ttgtcagtca aatgctctga aaccccattg yctgaagctc 60

taggttcaaa ctttgctcct tcaggtgttc agagctgccc c 101

<210> 213
<211> 101
<212> DNA
<213> Homo sapiens

<400> 213
tggatataag ttcctgtttt tctgattaat gtgcatgatc agacaagaaa rttatataca 60

ggaatcttaa actaatcatt gctacagaaa agaatgggaa g 101

<210> 214
<211> 101
<212> DNA
<213> Homo sapiens

<400> 214
acacagtagt gtaatcctaa tctttattgt gttagaaagt tcctcaagac rtagatggaa 60

gtccataccc caggagaatt actcataaaa atgaaatttc c 101

<210> 215
<211> 101
<212> DNA
<213> Homo sapiens

<400> 215
ttcgatatgc atttattagc aaagcttctg aaggtgtcgt aagctgaacg ygaggcagct 60

gcctctagaa gtgagattca catgcagggt ggaaatggta g 101

<210> 216
<211> 101
<212> DNA
<213> Homo sapiens

<400> 216
gggcccttta aacatagcct tgttttaata attagacccc ccaccccaga rgagagaggg 60

aggaaatgaa gcaaggcatc caccctcagg tgtaacatca a 101

<210> 217
<211> 101
<212> DNA
<213> Homo sapiens

<400> 217
atgatctgtg ccaatactct gttcttctta gcataaaggt gaacagcacc yctgcactgt 60

agcgtgaaag agtggatttg agtcttggct ccacgggctc c 101

<210> 218
<211> 101
<212> DNA
<213> Homo sapiens

<400> 218
agtagcagca gtttcacaaa gactatctca tttattcctt taataatcct rggcaggaaa 60

ttattagcag tcccatttta tagctaagaa aactgaggct c 101

<210> 219
<211> 101
<212> DNA
<213> Homo sapiens

<400> 219
tacatgggac taaactgata atggattata atttttatga cttttattta raatattgct 60

aattctttaa tattttattt tccagattta aggaaacttt t 101

<210> 220
<211> 101
<212> DNA
<213> Homo sapiens

<400> 220
ggtctacgca ctgcatcaaa atccaagctc agaaggcagg aaggcatctc ycgcttctac 60

attatccaag tggtgttccg aaatgccctg gaaattgggt t 101

<210> 221
<211> 101
<212> DNA
<213> Homo sapiens

<400> 221
ttattttcct aactccttgt tacttcagtt tagcaaattt tttaaaaagt raaagtataa 60

atatattaag acttttttgt agggggggctc tggaatgtga a 101

<210> 222
<211> 101
<212> DNA
<213> Homo sapiens

<400> 222
tggacagccc tggggctcct gctcctcccc tacacatcag gcttcttcct rtggagcttt 60

ctgtaccttc ccaagccctc aatgaatgca aaggaaaaaa t 101

<210> 223
<211> 101
<212> DNA
<213> Homo sapiens

<400> 223

ccaccacata cacagtaaac attctctctt ctcagtggtt gaagttgttc ytgattacag 60

ctctcttatc tgttctccct ttgatttgct gactgatgga t 101

<210> 224
<211> 101
<212> DNA
<213> Homo sapiens

<400> 224
tgtgcgcatt tcttatatct tcaatttata agtgcagaaa ttgagaatga raggtctaga 60

attaaacagt ccaggattca ggatcttggt tctgctactg a 101

<210> 225
<211> 101
<212> DNA
<213> Homo sapiens

<400> 225
gttgcttttc ccaggaggtg tgagcctacc tggaggaggc ttaggcacag rgatacctgc 60

tggaggtctg agcgttggtt gagcacctcc tgtttgtagg a 101

<210> 226
<211> 101
<212> DNA
<213> Homo sapiens

<400> 226
caattatctt ccatcatcac cctctcccca actggctgcc gtttccacct rtgatagatc 60

agtgttacac atgtgcattt tccagaactc ccagctgtga g 101

<210> 227
<211> 101
<212> DNA
<213> Homo sapiens

<400> 227
ctgacattta ctatatgcca aaacagggct gtttaaagtt catggtggtt ycatctactc 60

cttctgaggc tacttcaagg tagggaggct acttcaaggt a 101

<210> 228
<211> 101
<212> DNA
<213> Homo sapiens

<400> 228
attctaggaa aagcacctgc agttattaat gcattaaacc agtgttctga matgactaaa 60

tgcattattt ctgctgtaga agaaaacgct gaggtgaggc c 101

<210> 229
<211> 101
<212> DNA
<213> Homo sapiens

<400> 229
cacacgccag gcatggacgc tttccattgt tgtcaacaaa aactcatgca rctcaaatac 60

ttaaatgaat tctcaaacat gtggttcaca attgaaaaaa a 101

<210> 230
<211> 101
<212> DNA
<213> Homo sapiens

<400> 230
caactaagat cgtgtgcctt gtgttggtgg taaagcaata tcagagcccc rgtatggtaa 60

ttctcaatct aatgcctgtc tatgtgatca ggcttctccc c 101

<210> 231
<211> 101
<212> DNA
<213> Homo sapiens

<400> 231
gaatttgata aaaacaagaa atagaagcat aattattttt gaaaattaca rttaaaactg 60

ttagaatcag aagcagaaac cattagcagc atagagaggg g 101

<210> 232
<211> 101
<212> DNA
<213> Homo sapiens

<400> 232
tctttctgag ctttctgagc tttgcaatcc ccagctcacc cccccaacac rcccccacag 60

tccttcttcc caacagttgc cagcccaccc tggccataaa c 101

<210> 233
<211> 101
<212> DNA
<213> Homo sapiens

<400> 233
atgacccact acaacttcac ctcatgtatc ttgaacttta gggatatagc rccatttaaa 60

gagactaacc tctcttggtt cttgtcagtg aaactgggaa g 101

<210> 234
<211> 101
<212> DNA
<213> Homo sapiens

<400> 234
aaacttaagg tcagatattt cctcgagaca tcagaagtta aagcccatga yataatgagt 60

gaaaacatgc atagtaaact gtaaagctgt ctacatatgt a 101

<210> 235
<211> 101
<212> DNA
<213> Homo sapiens

<400> 235
gtgtgttctt tttagtttat cctttcatac atatatgtca agtctcccta rctcaattgt 60

aagccctaca atggtaaggg ctatgtttta tgcattttgg c 101

<210> 236
<211> 101
<212> DNA
<213> Homo sapiens

<400> 236
gcagagaaag acttctaata aaattccctc catatggaag gaaaaggaga yatcgggagt 60

tacgttaatc atgctcattt cttaacagtg caaatatcaa g 101

<210> 237
<211> 101
<212> DNA
<213> Homo sapiens

<400> 237
tccaaatggc caatctggcc actccaaagt cccgcttcca gactgaggaa rgggtgttaa 60

tgaagattcc agcaaacaac agctctgtcc taccaacttt t 101

<210> 238
<211> 101
<212> DNA
<213> Homo sapiens

<400> 238
agagaactgg agacaatgta gtataatatt cggatgtaca aagtacaaac yataaagtct 60

attttgtttt aataattaac aaaggtgcac ctagtacaca c 101

<210> 239
<211> 101
<212> DNA
<213> Homo sapiens

<400> 239
taagtacatg acattatcta atattggaaa taagagtgca aagccaaatc rtagccgtgt 60

atagcagtga atgttaggtt gtcaggttca ttcaaatgaa c 101

<210> 240
<211> 101
<212> DNA
<213> Homo sapiens

<400> 240
aggttaccgt gtatgtcaag gtcacccagg ggaatgactt aggagtcaaa ragcatggat 60

cctactgccc actgtggtgt caagttgctg ttcacccttg a 101

<210> 241
<211> 101
<212> DNA

<213> Homo sapiens

<400> 241
aaattgcacg caatgcatac aggaacaaag agagggtcaa gatggttatc yttcctcctg 60

gcttccaaca caacctgctt tgtaaaagcc ccacactgtt a 101

<210> 242
<211> 101
<212> DNA
<213> Homo sapiens

<400> 242
catgtcaaca acatctttca gaattggttt tctttcacga tgtcgtccag ytatgaaaac 60

gagcctcaca tgaaatatgc tccaagcctt ttgagggcaa c 101

<210> 243
<211> 101
<212> DNA
<213> Homo sapiens

<400> 243
ctactccctc tatgcttgtg gtgattcagt tgcagaaaga cacatctata yttcatagct 60

gtagaaaaat tctttttttg tggttgattt catgtggttt a 101

<210> 244
<211> 101
<212> DNA
<213> Homo sapiens

<400> 244
agtcaccagc tggtgacctt gagcaagtct ttagacctct ctgagctttt ycctcatgtg 60

taaaatgggg acagacggag cccaacccaa gatgttcctg t 101

<210> 245
<211> 101
<212> DNA
<213> Homo sapiens

<400> 245
gtcagatgtt acacaacttt gcaatttcca atatgtgaat attaacatag rccaatgaca 60

ttattacaga agcttactag aaatatattc tgctggtcac c 101

<210> 246
<211> 101
<212> DNA
<213> Homo sapiens

<400> 246
ctggcccaaa tgccagcatt tgctctcctg cctatttccc aggccgtggt raggggcttt 60

tcctcagggt cttcatgggg agagtcaggg gatgagtgcc t 101

<210> 247
<211> 101

<212> DNA
<213> Homo sapiens

<400> 247
agggagaagc cagtacagag gccccagcta gagtctgaat gaggacgatc mctctcccct 60

gtcctgggga gcctggggtc accttgcaga acaagatggt c 101

<210> 248
<211> 101
<212> DNA
<213> Homo sapiens

<400> 248
tctcccattt tcctccttta tgctcctgcc agttctgcaa atgtgggagt ygcccaaggc 60

tttgttcatc agccctctta cctaatcaca tttcttccaa g 101

<210> 249
<211> 101
<212> DNA
<213> Homo sapiens

<400> 249
ccaaggcagg cacctcctgg tgctgccaaa aggcatcaga ccccatgccc ygctccttcc 60

tcatcctgga ctagaactgc tttggggtgg agacgttacc t 101

<210> 250
<211> 101
<212> DNA
<213> Homo sapiens

<400> 250
atccatttac tgaagttatc tgacatggct ctcgagtccc ttctacccca ygactcccct 60

tttttccctt tatccttgtg aattatctgt tgaagaagcc a 101

<210> 251
<211> 101
<212> DNA
<213> Homo sapiens

<400> 251
taaaaataaa atagttatgc tatttacaag acacacctgt tgaaataagg yagtgtaaat 60

ataaataaaa gggtggaata tttatcatgt aaatgccaaa a 101

<210> 252
<211> 101
<212> DNA
<213> Homo sapiens

<400> 252
tgtcagatta tttaggccca atccattctg ttgattggac ctagtataag yggaaggata 60

aagatttcta tccctacatt aacacatttt atgggttgca a 101

<210> 253

<211> 101
<212> DNA
<213> Homo sapiens

<400> 253
tctggagatt cagctgaaca cctggagagt ctattgaggt ctttgtccct ygtctgttca 60

gaatggcacc aggtactagc actgtataat tttcaaaatc t 101

<210> 254
<211> 101
<212> DNA
<213> Homo sapiens

<400> 254
atacaaaaaa gtagcaaaaa gtgggatggg gaaaataaga ttagataact rggtaataac 60

cataaacgat gcccttttta agaaatccaa ttgttgttta t 101

<210> 255
<211> 101
<212> DNA
<213> Homo sapiens

<400> 255
ccaaagacct tgttacagtg tttttaggca tggctcactt tataaaggtc rtcacagttg 60

gccaagctat ctggtattta ttactcattt gatactcaca c 101

<210> 256
<211> 101
<212> DNA
<213> Homo sapiens

<400> 256
taagttctag agtgacagtg gcttgctcaa ggtcatatgt ctaattcagt rgttccaggg 60

acaattggat aatgtctgga gacatttttg gttgtcacaa c 101

<210> 257
<211> 101
<212> DNA
<213> Homo sapiens

<400> 257
atagggcatt ttgattatta aaactgtgaa ctgcttcctg gaagggcaaa yagaggtaac 60

tttggctgca tgttacaatc cacaattcaa tttggcatag c 101

<210> 258
<211> 101
<212> DNA
<213> Homo sapiens

<400> 258
ctcagctcta aatgcactgg tataactgtt gccatttctg gacatgccac rtgaaatttt 60

tcctttgctc atactattca tgcagtttgg aattgattcc c 101

<210> 259
<211> 101
<212> DNA
<213> Homo sapiens

<400> 259
aaggtttaag gaactttcat tttattagcc agtggttaag tgcctgtgag mgcaatcatc 60

agcaggtgca gtggtagaag ataacaagct tcctaataaa t 101

<210> 260
<211> 101
<212> DNA
<213> Homo sapiens

<400> 260
ccccattttc tgggcacacc ccaaacatct tccatgggag aaattggtca ygtgagccca 60

tccctgatgc ccgaggaggg atgggcttgc caaggctctt c 101

<210> 261
<211> 101
<212> DNA
<213> Homo sapiens

<400> 261
cctccctggg aatgacaggt tctgtttttc ccttcaacta ttttagcaca kggagttcac 60

aactcattcc agctacaatg ggaaatgttt agtcccgact c 101

<210> 262
<211> 101
<212> DNA
<213> Homo sapiens

<400> 262
atgaaatgga acaaggaaaa agaaagatta gaatacatgt gaaacctcta maatttttac 60

catatagagc aggaaagaaa cataatctaa accatatttt t 101

<210> 263
<211> 101
<212> DNA
<213> Homo sapiens

<400> 263
taaccgaaat accctgtgtg tgtgtgtgta catatgatcg agccagcctc ytcagtgcct 60

tgcattgctg ttaagagggg aagttctagg ctaagacttt g 101

<210> 264
<211> 101
<212> DNA
<213> Homo sapiens

<400> 264
gccttccatt tttaagcaaa cattttacaa gcttgtactc attctctcca ygttgtatta 60

agttttatat ttgacattgt atttaaagca tttaccatat t 101

<210> 265
<211> 101
<212> DNA
<213> Homo sapiens

<400> 265
tgtgaaaaac attgttagct tgaagaatgt gcaaaaacaa gctgtgtgcc ygatttggct 60

ttcaggctgt agtttgccaa cttgtgacct aggccttgag t 101

<210> 266
<211> 101
<212> DNA
<213> Homo sapiens

<400> 266
gagattgtgt cttaaaaagt tttgctctct cctcagaacc tagctcattt rgtaacttgt 60

tattgctgaa taaaaaccaa tttattgata aatgaatgtc a 101

<210> 267
<211> 101
<212> DNA
<213> Homo sapiens

<400> 267
atatataaca tagatagtat tttttcttgt atcttagtgt tctgagttca mctttcttct 60

tctcttcttc ctgaagtaca tacttgaaac ctcattcaca g 101

<210> 268
<211> 101
<212> DNA
<213> Homo sapiens

<400> 268
ttgtggtagg ctgcttaata attaattccc tcacctcagt ttttgaatgt ygttctgttt 60

atgcctcagt atcaaaaaca actgagaaag gggccgcagc t 101

<210> 269
<211> 101
<212> DNA
<213> Homo sapiens

<400> 269
cttaatattt ggctctgtgt ccccaaccaa atctcacctt gaattgtaat ratcctaacg 60

tgtcatggga ggtaaatggt gggaagtaat tgaatcatgg g 101

<210> 270
<211> 101
<212> DNA
<213> Homo sapiens

<400> 270
gatgaaaagg tcctatctta tcatacacct ttaccataaa cttcccctcc ygccaccccc 60

agaaggaaga gctgaggcag tttccaaagg tgcctgactt g 101

<210> 271
<211> 101
<212> DNA
<213> Homo sapiens

<400> 271
gcagagcgat ggttcagatc ccaggcagga aggagatgga tagcaaaaga ktttatcaca 60

ctactcagaa ttgtgcttaa tttaaaactt ttaaaatatt c 101

<210> 272
<211> 101
<212> DNA
<213> Homo sapiens

<400> 272
tttatccaaa gaagggaaat cagaatgatg aagagatact tttcctctta yatttttagg 60

tttatcacct tcatattgtc aaagcatgat gccaataacc t 101

<210> 273
<211> 101
<212> DNA
<213> Homo sapiens

<400> 273
ctctgcaatt tgagtttgtt gtgttctaaa gaggtacaaa aaaacatgca rctggttagc 60

agcatgctcc agagacccag aactgcccca gaatgatggg t 101

<210> 274
<211> 101
<212> DNA
<213> Homo sapiens

<400> 274
gccaatatcc aagacagacg ttcaattttc caaaaagccc aagaaattct raaaagtggc 60

ctcacaaaca ggtttttctg aggcttagac aaaaattcaa g 101

<210> 275
<211> 101
<212> DNA
<213> Homo sapiens

<400> 275
ttggagaaat gttaattcac tctctctagt gtcctgaaat ggattggatg rtgcagtatg 60

ttgtattgca tggctcctaa cccaattcca gggagtttct t 101

<210> 276
<211> 101
<212> DNA
<213> Homo sapiens

<400> 276
gtacttaggc actaattggc atttttcaac atttctgtta atgtagaaca ygtctttcga 60

accctcaggg gccttgcttt ggagctaatg aaaataaagc a 101

<210> 277
<211> 101
<212> DNA
<213> Homo sapiens

<400> 277
tttggggatg tggagggaaa gcgagctggg agctgagccc agaccagctc yggtaggagt 60

cagaagaatg tgccctgctg ccagtctgag ggtcaaagtg c 101

<210> 278
<211> 101
<212> DNA
<213> Homo sapiens

<400> 278
tggttaatca ttcactcaat catttgataa atatttgcca agaactgtct rtgtgtaagg 60

tacataatag acactcattt atgtgattat gaatccctct a 101

<210> 279
<211> 101
<212> DNA
<213> Homo sapiens

<400> 279
acctctccta cattctaaaa gaatggcctg aactatccat gagaacatga yatccgaact 60

tgtaaactta tttccctcat cacagcccat aaagaattat a 101

<210> 280
<211> 101
<212> DNA
<213> Homo sapiens

<400> 280
tgcaacttgg taaaaatatt ttaacttcat atgctacgaa tttgattttc yttgtattaa 60

ctacacatgt aattagattt ttttctttcc aaatcatctt t 101

<210> 281
<211> 101
<212> DNA
<213> Homo sapiens

<400> 281
agagagatcc ctgtctctcc tcttcttata aggctaccca tttttatcaa rttagtactc 60

catccttatg accccttttg attttttttt cttttgaaaa g 101

<210> 282
<211> 101
<212> DNA
<213> Homo sapiens

<400> 282
ttatataaag ggatcttacc tctctggatg gaagagactg aaatggaatt rccaaagtcc 60

aaatatgtgt atctgttgca tttaaagtag cacagtttct c 101

<210> 283
<211> 101
<212> DNA
<213> Homo sapiens

<400> 283
ttcacctccc aaaatgttgg gattacaggc gtgggccact acacctggcc rtaagtacag 60

tacacgtcac ccctgcttga aaaatcatca aagcctttca c 101

<210> 284
<211> 101
<212> DNA
<213> Homo sapiens

<400> 284
tcgaaagatt tacatagttt tagaaaggag gaaaggcaaa gagggagttg rgaaatgaaa 60

gaaacaggga gaagacatgg cttctaaatt cagggttggg a 101

<210> 285
<211> 101
<212> DNA
<213> Homo sapiens

<400> 285
taaattgcct gagagcttag agacaatcag gtcaccaccg ccctcacaag rgaaaagctt 60

cttacttccg agcagaacgg ttcagctggg aagagaggaa g 101

<210> 286
<211> 101
<212> DNA
<213> Homo sapiens

<400> 286
atttccaaga caatttttca tcctttcgta taatattcca ggtttgttgg kgcctcttct 60

ctgtatttcc cagaaaataa ttctaccctc tggagaactg t 101

<210> 287
<211> 101
<212> DNA
<213> Homo sapiens

<400> 287
aaagatgtgg ccatcaagga gaagtctttc ccatcgtaaa tatccaaggg ygtgactgag 60

ccatcactga actggaccca gcaactgatg gctgcttcct a 101

<210> 288
<211> 101
<212> DNA
<213> Homo sapiens

<400> 288

ttgtccttgt tttaaggatc ttcctgcagg atccactccc tagcacttct kgatggcctg 60

gctcagggaa atcttcagga aagagaccca ggcttgcact a 101

<210> 289
<211> 101
<212> DNA
<213> Homo sapiens

<400> 289
gtttttgctt tgaggaaact tgatatgatg ttaaatttct aaaagggcaa rgaaagtaga 60

attgatcagg tagcagaaat tttacacagt tttggacatc a 101

<210> 290
<211> 101
<212> DNA
<213> Homo sapiens

<400> 290
tgcccctacc ctgagtgctg agagtagaac tattgagaga cctctttatg mgaaattttc 60

agaaatccaa catggttctt ggtctagaaa gtgggatcaa g 101

<210> 291
<211> 101
<212> DNA
<213> Homo sapiens

<400> 291
gtggtcacat ttatctgctt ctttgtattt ctactaatcg ttctattaga kgctggacat 60

tatggatatc ctgttgttgc gtgtctggat tttgggtttt t 101

<210> 292
<211> 101
<212> DNA
<213> Homo sapiens

<400> 292
caaataaaat attttttctt ttacatagta catgaaagta aatctaatct kggagctcat 60

ttaggatgct gagcagagta actggagtta gactataaga t 101

<210> 293
<211> 101
<212> DNA
<213> Homo sapiens

<400> 293
aacaggctga ggttcagtaa gctgtcatag ctgagctgag acttgaatgc mggtcagatt 60

tcagaatctg ggctcctcgc acttctcacc acactgcctg t 101

<210> 294
<211> 101
<212> DNA
<213> Homo sapiens

<400> 294
ggactctcca acagcataaa ttggctccag cccgcaagcc caactttccc kcagctgagc 60

ccctttcaga cttctgcccc tgcctctgat ctatacttta t 101

<210> 295
<211> 101
<212> DNA
<213> Homo sapiens

<400> 295
cttaatctat ttagactgac tacagggatc tttgattgcc taaaacaaca rtatagcaat 60

ttctctatct gctctcgtct tcctcccgtc atactcatac a 101

<210> 296
<211> 101
<212> DNA
<213> Homo sapiens

<400> 296
ctcttttgat atccccttca aaatgtctgc tccacacaca gagcatcaca yatgtggttt 60

atatgtagct ggctgaattt ctttcctttc tctctttctt t 101

<210> 297
<211> 101
<212> DNA
<213> Homo sapiens

<400> 297
gatagcgcta ttaactgttt acacagtaag cacaattttc tattctctct ytctctctca 60

ctggtttcaa agcagccaaa agctttgagc cccccagcaa c 101

<210> 298
<211> 101
<212> DNA
<213> Homo sapiens

<400> 298
ataagctgaa ccgagacctg cttcgcctgg tggatgtcgg cgagttctcc raggaggccc 60

agttccgaga cccctgccgc tcctacgtgc ttcctgaggt c 101

<210> 299
<211> 101
<212> DNA
<213> Homo sapiens

<400> 299
ggaactttca agcttgtgtt ggggacatgg atctctataa gtaaccacat rtaagtgtaa 60

caagttttga tatgaaagaa aagaacagag tgccctacaa g 101

<210> 300
<211> 101
<212> DNA
<213> Homo sapiens

<400> 300
tgccacctca ttagcaaagt tcctgggagc cactgacatg gaagaccccc kgtttccgcc 60

tctcggtttc cgagcctcag aaagatggac tgtgaggcct c 101

<210> 301
<211> 101
<212> DNA
<213> Homo sapiens

<400> 301
acatttctat ggggctagac ttttccttgt caagattata atttttctta ygagttttta 60

cctgaaaccc ctattttcta agaccccatg gttaatgagt c 101

<210> 302
<211> 101
<212> DNA
<213> Homo sapiens

<400> 302
ataagccgtg ggtgtaacca tgtcccccac ggagtgagaa ggggagggtc ytctggtttg 60

ttactttctg ctcatgaggc ggggcgatgg ggagatgcct t 101

<210> 303
<211> 101
<212> DNA
<213> Homo sapiens

<400> 303
ctcaaataaa gagaaattta aatcaaaatg acttggcttt gtagagtact mctaattttg 60

atttttgtaa tcatttcatc ttcctatata tgtcctttta c 101

<210> 304
<211> 101
<212> DNA
<213> Homo sapiens

<400> 304
gaagtgatag gtggaaatga taattgttct gtaagagata ttctaagggg yaatttaaaa 60

catgtcaata taggcttctt ctaaggtggt aaactcagct t 101

<210> 305
<211> 101
<212> DNA
<213> Homo sapiens

<400> 305
actcactaac ttattctttg taaaaaggag agcaggtgca caggtgtaga racaagaaac 60

aacttggaga gtgttggcgt tgctggagca ccaagtagaa a 101

<210> 306
<211> 101
<212> DNA

<213> Homo sapiens

<400> 306
ttcagaactt acgttagtag agtttgaata gttaagactt gaaattaaga yccttgcttt 60

agtacataat ctcacaaatg actttcagaa aatggtgcat c 101

<210> 307
<211> 101
<212> DNA
<213> Homo sapiens

<400> 307
gaaattgctg ggccatacat agcgatgcgt ttgtaaacca gctcactgaa yaagaaagcc 60

ttgattagca tttgctaaca tctgtgatgt taatactcct a 101

<210> 308
<211> 101
<212> DNA
<213> Homo sapiens

<400> 308
ctgacaacca gaactcaagt ctctaacctt ctctgctgtc ccagtaatcc rtgcctgcct 60

tttctctgcc ttcagccctt tttgctccat cagtactttt a 101

<210> 309
<211> 101
<212> DNA
<213> Homo sapiens

<400> 309
gtcatgcggc ttgctaatgg gtttcaagga gcaagctgca aagagcccct rgacttgctc 60

tgatgggttt caagggacaa gatattagta acgcactcac a 101

<210> 310
<211> 101
<212> DNA
<213> Homo sapiens

<400> 310
acactgtgct ccgcttttcc tcttagcctc ttcccctcaa cgaaatggta rgagttcagc 60

tgacaacagg gtaaacagat tattgtgtta ttgctggctg a 101

<210> 311
<211> 101
<212> DNA
<213> Homo sapiens

<400> 311
caaataacta taaaataaac tcaaaatctt tttttcctgc attagttcac kgaaaataaa 60

aagggttagc aattagaatc aatagattct ttgaaaacac t 101

<210> 312
<211> 101

<212> DNA
<213> Homo sapiens

<400> 312
attatcatac tgctaaacac catgaaacac tgtgtaagtt tgcgctatta yagttatttt 60

aaactgtttt tatatttagt tgcttacttt taaatttata t 101

<210> 313
<211> 101
<212> DNA
<213> Homo sapiens

<400> 313
aaataagctt ggacatgacc ttttttagca taatgactac tgtcatttca rtgtcaacct 60

ttgaaagcat ccattcttgt taaaaacatt tgccactgct g 101

<210> 314
<211> 101
<212> DNA
<213> Homo sapiens

<400> 314
gggtttacac tgctcccctc tgctagagca tggactacca gctgacctgc mgagtcactc 60

accttaaatg ttagcagtag ctatggggtg tgtgtgtgtg t 101

<210> 315
<211> 101
<212> DNA
<213> Homo sapiens

<400> 315
attagttcca caacaaacta gatgtagtat tttgcatata tttcccctgc yaacgcacct 60

gtggtagttt ctagtacatg gtttcacttc tatgatcttt t 101

<210> 316
<211> 101
<212> DNA
<213> Homo sapiens

<400> 316
tccagcatat tcccagctgt agtggctacg gtaaaagact cattctgtat yagagcagac 60

ggaatctaga aagacagcca tcatctacaa gttgggttta a 101

<210> 317
<211> 101
<212> DNA
<213> Homo sapiens

<400> 317
ctgaacagac tgtgctttag agcctctgga agacacccaa cagaatgttc ygaaaaatgc 60

gattattttt acacaaaatt gccaatgtaa attcaacttc t 101

<210> 318

<211> 101
<212> DNA
<213> Homo sapiens

<400> 318
tgctgtgtga tgaggaagcc aagaactgaa ctgtaaccca aacacaaaca ygttgcattg 60

ccaggaaatg gctaatgcgg cctcccatta cacagagctt t 101

<210> 319
<211> 101
<212> DNA
<213> Homo sapiens

<400> 319
ttctaaagtc atccatcccc ttgacttaag ctccaggatg gatgcagaca yggacggacg 60

cctgtgcaca gacaggagtc tggaagagca cctgagccct g 101

<210> 320
<211> 101
<212> DNA
<213> Homo sapiens

<400> 320
tttaatggaa agttaattgt tatgcaaata tgcattcaca tgttattttg yttgtttgtt 60

tgtttgagac agggtcttcc tctgtcgccc aggctggagt g 101

<210> 321
<211> 101
<212> DNA
<213> Homo sapiens

<400> 321
actccaagtg ctataagcct gcaatggact gtatgtttgt ccccctccac ygcaaatgtg 60

tatgttgaaa tcctaacccc caatgtgatg gggtctttgg g 101

<210> 322
<211> 101
<212> DNA
<213> Homo sapiens

<400> 322
tgaacttaaa cccgagtata ctagaaatat aaattattat atacaaatgg rtgtctttta 60

cagcaataga ctccagccta aattgatggt aggggtttta t 101

<210> 323
<211> 101
<212> DNA
<213> Homo sapiens

<400> 323
ctttactatt tagtctagcc tgggattctg tatgtgctgg ctaactgcaa mcccgaacag 60

gcaggccttg gtgtgggatt ctctagttga gctgggtcac t 101

<210> 324
<211> 101
<212> DNA
<213> Homo sapiens

<400> 324
tcttataata aagattattg ttattattat aaccaccttt cagtgtttct rtcttaccct 60

cacatcttca cttttcccct aatctcaaga tagagtggag g 101

<210> 325
<211> 101
<212> DNA
<213> Homo sapiens

<400> 325
aagtggtaag gttgtttgtc tgaggtaggt gattaataga cagccttcct yagcacgtgc 60

aaattaaaat agaagaagga attatgattg gagctctcct t 101

<210> 326
<211> 101
<212> DNA
<213> Homo sapiens

<400> 326
cctgatcaac cttcaaagga atcctcctga gtttacatga gttggaaaat rtgttttcct 60

ggctcgttaa agtggaacca atctcctccg tgtggtagag a 101

<210> 327
<211> 101
<212> DNA
<213> Homo sapiens

<400> 327
cgggatatag tagccatgag gaaaacaatg agggctaccc ttacagcacc rgactccaga 60

tggtcttcag tgcattcttt gggtagcagc tccccaggag c 101

<210> 328
<211> 101
<212> DNA
<213> Homo sapiens

<400> 328
gacttgttca aggtcatata agcagcagtg gagtccagaa gccaggtttc ygtatgccct 60

cttccacatc acattgcaag acaccctctg aaaacactcc t 101

<210> 329
<211> 101
<212> DNA
<213> Homo sapiens

<400> 329
ggtccaccct ctcagttagg cagtagtaaa agatctaaac ataatcaatc rggcacattg 60

tatgtagctg tgagggttag aagtacaaaa tgtagttgtg a 101

<210> 330
<211> 101
<212> DNA
<213> Homo sapiens

<400> 330
ggaaataagc tcatagctgg acagacagca acgacataga tccggtggag rtgaatctgc 60

agatagagga taattggtct tggcttcaag gatggaaaga a 101

<210> 331
<211> 101
<212> DNA
<213> Homo sapiens

<400> 331
acatatgcat aatgatcctc aattacgtgc caagcattat ggaagtcatc rctaactcct 60

ctgtcacctt tactttcctg atagcacctg ttgatgctgt c 101

<210> 332
<211> 101
<212> DNA
<213> Homo sapiens

<400> 332
aaaaggcccc cagggaggaa ttgatcaaac caaaatgtgg atgagtagat rttaggcgaa 60

caccaggcaa atggtggtga gagaagggag caaagtgtat t 101

<210> 333
<211> 101
<212> DNA
<213> Homo sapiens

<400> 333
aaaataatct aaatcttatt gagcatgata ggattaagtg ggaattggac mgatagtgga 60

gttggggatg gattgtaatt atactacact gcgaaaaagc a 101

<210> 334
<211> 101
<212> DNA
<213> Homo sapiens

<400> 334
ctactttagc cactctcaaa actttgtgat aaatctgcaa tagaggtatt rtatatacat 60

gcagaaagct gtgggaagcc cagaggagta agtgactaac c 101

<210> 335
<211> 101
<212> DNA
<213> Homo sapiens

<400> 335
acaaaataat tccttcttaa aaattatgta ttagaaaact tttcaaaatt yatcccatcc 60

tccagaaacc aataaaataa cacacactag aggtccttca g 101

<210> 336
<211> 101
<212> DNA
<213> Homo sapiens

<400> 336
cagagctcta ccaatcataa cagagaaggc atggaaagct ggtgaaaatg ytggaacgag 60

tttcttttta catgttgttc aatttttatt tttgcaatta g 101

<210> 337
<211> 101
<212> DNA
<213> Homo sapiens

<400> 337
cttcccccaa aggccctgga aactatcatt ctactttcca tctctatgaa kgttatactc 60

taagtacctc atgtaagtgg agtcatgcag tgcttgtctt t 101

<210> 338
<211> 101
<212> DNA
<213> Homo sapiens

<400> 338
gtaaatttat tgcttgctca atccttcctt gtatttcatt agcatattgc yactctacac 60

ttgtcctgta tttagatatt tccttcctct atggtttgtt c 101

<210> 339
<211> 101
<212> DNA
<213> Homo sapiens

<400> 339
aaaccatggg gttgagtgca ggtgggataa caatgtagag attggcaaac rtgatgtgga 60

aggtgcgaga gacattgtgt ccaaagcgat gggcgaggat g 101

<210> 340
<211> 101
<212> DNA
<213> Homo sapiens

<400> 340
cttaacatat gcaaaatgaa taagtgacaa ccccaaccct caccattggc yccttagaac 60

tgaaaataat ggcagttgca gtgtttaagg gcaacatgaa t 101

<210> 341
<211> 101
<212> DNA
<213> Homo sapiens

<400> 341
gataatgact gggaattttc tagaattgga aatcctcctg tttgggacca ygaagaatcc 60

caggtaggat atgtaaaact aaatgcacat ctggcaatat t 101

<210> 342
<211> 101
<212> DNA
<213> Homo sapiens

<400> 342
aacaaaacaa aacaaaacaa aacaaaacaa aacacctctt attctagaat rttatgcttc 60

aggagagtgt agctctccta gttttagttt ggttcagaag a 101

<210> 343
<211> 101
<212> DNA
<213> Homo sapiens

<400> 343
ggcgttcagc cctgggctgt gctgtattca gggctctaaa aacgctggcc racttgaatg 60

tgtgaataca gttatggcag ggagggaggg gaggtgcttt g 101

<210> 344
<211> 101
<212> DNA
<213> Homo sapiens

<400> 344
tttgtgcata ctgtgatgat tttagaaggt aagaatgtca agctgtttga rctgaaagta 60

aagatagccc cttatcagga aagtgccagc cacccttgct g 101

<210> 345
<211> 101
<212> DNA
<213> Homo sapiens

<400> 345
aatgttgatg catttaacag cttagattaa atggacaaaa tttatgaaag rcacaaactt 60

tcaaagctta ctcaagaaga aaaagataac cagagtagcc c 101

<210> 346
<211> 101
<212> DNA
<213> Homo sapiens

<400> 346
gatctcgact cggagcttct tgcccctctt ctgtggaatg aaaggggagc kaaggaggag 60

ggtgtctgag gggcgagaga tgagcctgga agagaagcaa g 101

<210> 347
<211> 101
<212> DNA
<213> Homo sapiens

<400> 347
cgttgttgca taggactaga ctaaaccaag cgagctgcat tccatgcgaa ytattctatc 60

gtggggatca agatctccag ctgagaaaag atgccaccag a 101

<210> 348
<211> 101
<212> DNA
<213> Homo sapiens

<400> 348
tgatattact aactggaagt cctctataga atgcttttac catgatgtac rtagtctgtc 60

taggattcct tatgggaaac atacctaaaa ttgatggatt t 101

<210> 349
<211> 101
<212> DNA
<213> Homo sapiens

<400> 349
atcttattct gaaagcagat ggggcatcag aaacatcaaa caagttaaaa ycacaggaat 60

taaattataa attttaaact cccttttatt gaaatataag t 101

<210> 350
<211> 101
<212> DNA
<213> Homo sapiens

<400> 350
gctgtagatg gctataaagc ggtccaaaga catggccagc agcacagctg rctccatcat 60

ggataaagaa tggatggaga acatctggaa aaagacaagc t 101

<210> 351
<211> 101
<212> DNA
<213> Homo sapiens

<400> 351
gccttagtgg ggtttcagga gggagcagag ataaaaacac atgtcttcaa kccatcatct 60

tgaactggaa atcctaaata tcttttgatt ccttcttttg a 101

<210> 352
<211> 101
<212> DNA
<213> Homo sapiens

<400> 352
cagggaatgt ttcagaatga agggagggta catggataaa tcagtcagtt maaatattgg 60

tgagccccct gcagcacgcg cagatctttg cttaggtgta a 101

<210> 353
<211> 101
<212> DNA
<213> Homo sapiens

<400> 353

aggaagtacg gcatagcagt taggcactca ggcatggatt cagaaatacg yggaattcag 60

tagggctctg gcacctacta acaatttggt tactctccct g 101

<210> 354
<211> 101
<212> DNA
<213> Homo sapiens

<400> 354
gcactcaata ccctgaaaat tcgctcgtct ctcatgggcc tgcctctgaa rctgctatga 60

aagccggcaa ccacacagaa tttgcctccg gtaagaatta t 101

<210> 355
<211> 101
<212> DNA
<213> Homo sapiens

<400> 355
ctaagtatga tgtagccctc tgtaatgata atagtaatag caatagccag mactccagca 60

atagtaatag ccaccactga cttcattgtt aactacaggc c 101

<210> 356
<211> 101
<212> DNA
<213> Homo sapiens

<400> 356
gtgagacaca cacagagtct gcacagcatc tggctgcggg gtggattatg rttagccaag 60

ggttcctttt tatggatgac tgcggtagtg aagttgcaga c 101

<210> 357
<211> 101
<212> DNA
<213> Homo sapiens

<400> 357
acgataatag ctcctgtgcc aaagaccctg ggcagtgtca ggatagctgt rtagctcagt 60

gggctgtaga tggctataaa gcggtccaaa gacatggcca g 101

<210> 358
<211> 101
<212> DNA
<213> Homo sapiens

<400> 358
aaaactataa aaagagacaa aaattgtgat tatgtattga atgccaaagg rgtcaattct 60

gcaagaaaaa taataattga aaatatatgc accccacatt g 101

<210> 359
<211> 101
<212> DNA
<213> Homo sapiens

<400> 359
ttgggcagag ttctgtgcga ggggcagcag aggatgcaaa ggcctataat ytccctgtcc 60

tctttggcgc ttactgtcca ctgacaggga ggcagaatga c 101

<210> 360
<211> 101
<212> DNA
<213> Homo sapiens

<400> 360
ccaaaaaacg gttgggagca actgctctag aaatttgttg tcttcataaa ygtttctgac 60

tcttagtttc tgtttttatc ccttctctaa gtaccaactt c 101

<210> 361
<211> 101
<212> DNA
<213> Homo sapiens

<400> 361
tattctttct catcttccaa agctatttca tcctccaaag tgtttgttat rtacttttga 60

atgaatcaca atataccaat accaacacat attttcatta t 101

<210> 362
<211> 101
<212> DNA
<213> Homo sapiens

<400> 362
ttggtttcca ttgataattt ggaggcattg tcctctgtgg agttgtgtca yctatcagcg 60

ggctattaat ttagggtatg gttatagaca actgcagatc c 101

<210> 363
<211> 101
<212> DNA
<213> Homo sapiens

<400> 363
gtggatttac ttgcttggtt tccattgata atttggaggc attgtcctct rtggagttgt 60

gtcatctatc agcgggctat taatttaggg tatggttata g 101

<210> 364
<211> 101
<212> DNA
<213> Homo sapiens

<400> 364
aaaagcttta tatccttaca tgaaggacag aacaggcagc tatatggtga rgaaatgtac 60

agacacaaat atccatatat tgaataattg gctggctggg g 101

<210> 365
<211> 101
<212> DNA
<213> Homo sapiens

<400> 365
atctccgcgt cttcttcttc tgtgtgcccc agatataaat aagcctctat ygtatcgctg 60

gaaaaacaaa ctcaccaagt tctatattag gcctattgca c 101

<210> 366
<211> 101
<212> DNA
<213> Homo sapiens

<400> 366
ttgtgcacac ctattacagg aatggaggac tcctgtaatg tgtctattag ycttaattcg 60

ggctccatta tacattccta ttctgttccc tcccctttcc c 101

<210> 367
<211> 101
<212> DNA
<213> Homo sapiens

<400> 367
acaggctgtc aaatgagagc acgtacttaa gaggctaaca cagtatgacc rtatgtggca 60

ataaatgagt gctgagtaca tgtctatttc ttttccagtc t 101

<210> 368
<211> 101
<212> DNA
<213> Homo sapiens

<400> 368
accctcacag ctgctcccac tggagccagg ctcttgcctg gaagaactgc rggttccctg 60

ggagactccc cagagcccct ccttagtgga cccaggccca c 101

<210> 369
<211> 101
<212> DNA
<213> Homo sapiens

<400> 369
cagtgattac ctgcactttc tttctctgac ttctttggtt agctcttctg yttattgaaa 60

caggtaagca gagaaaagta tttaaaaata atctctctct c 101

<210> 370
<211> 101
<212> DNA
<213> Homo sapiens

<400> 370
gtaacacaac tacataatat ccaaagacaa agtagaatgg caagaacttc rcagagcgga 60

ataagccttg atggtaaagg gaaacatcca aataagcaag c 101

<210> 371
<211> 101
<212> DNA

<213> Homo sapiens

<400> 371
tcatcatctt cttgctgccc aagcctctgt tcagtccccc accagatgcg kcattcaagt 60

tgtaaagcaa atgtactatt tcttgacatt tctagaaaac t 101

<210> 372
<211> 101
<212> DNA
<213> Homo sapiens

<400> 372
gtttgagtca tggttttgga aaatcacatg atccatacca gaggagagct ktgtcttcaa 60

attatcttct agaaaggttc accagaaagt acaaaaatgt t 101

<210> 373
<211> 101
<212> DNA
<213> Homo sapiens

<400> 373
taagtcttga atttgggtag tgtgaatcct ccatatttgt ttttcctctt magtattgtt 60

ttggctattc ttggtctctt gtctttacat ttaaacttta g 101

<210> 374
<211> 101
<212> DNA
<213> Homo sapiens

<400> 374
cagtggtaac caggcagtaa gtaccatgga ttttggatga gactcagtac mttgctggca 60

tcatgtgcaa cccagcacat tcccagctct ggtggccaca g 101

<210> 375
<211> 101
<212> DNA
<213> Homo sapiens

<400> 375
tgtgtgtgtg tgtgtgtgta cacatgtgtg tgcgtgcatg ctttttcatg rggcacactt 60

attttcagat gttcacatgg actctttttg agattcccca g 101

<210> 376
<211> 101
<212> DNA
<213> Homo sapiens

<400> 376
caatgcaagg gatttgtaaa gaaacaggga aatgaatgat ctgacaggcc rtttgttacc 60

accaacattt ttcttaattt aacctgaact tacttgctct t 101

<210> 377
<211> 101

<212> DNA
<213> Homo sapiens

<400> 377
atccatgcaa tgcaataaac agccatagac agaagcgaag cgctgatcca ygctacagtg 60

tggagaaatc ttgaaaacac tagggaagtg aaagaaacca g 101

<210> 378
<211> 101
<212> DNA
<213> Homo sapiens

<400> 378
tataccaagg atagtttgtg cagttacacc ggaaataaga tatttcctgc rtttacagac 60

atctacatgc ttgccttttt ttccatttcc cactgaacca g 101

<210> 379
<211> 101
<212> DNA
<213> Homo sapiens

<400> 379
atgggggatg agacaaagaa cttcatgggt gcagcaggtc tcttggtgtc rtgtgggaaa 60

cacaagcaga atcagaagtt cccctggcct ctccctgggt c 101

<210> 380
<211> 101
<212> DNA
<213> Homo sapiens

<400> 380
aaagggagaa tggggtggag ggccagaaag caggagtgcc atagagtcag kaagtgaaaa 60

attgcaaatg tgggcaatgt gattaggcaa ctgggtgtgt a 101

<210> 381
<211> 101
<212> DNA
<213> Homo sapiens

<400> 381
caccctagaa atcctggagg gaggaccgaa aggtagcatg gagtcaataa ygagcctctt 60

tttatttaac tatgattaca tgtcaatcaa tgtctgattc t 101

<210> 382
<211> 101
<212> DNA
<213> Homo sapiens

<400> 382
cttggcatgc tagttaaccc aagggatggc tctacaatgc cttacagttt rtaaagtact 60

tccttctgta ttatttcatc tgaccttcgc aataaggcta t 101

<210> 383

<211> 101
<212> DNA
<213> Homo sapiens

<400> 383
aaatccacag ccattcaggt ggcttatgtt actggcactt agcattccgc raccatggtc 60

cccagaggct ctgtggacag aggtgccctg cagttccttt g 101

<210> 384
<211> 101
<212> DNA
<213> Homo sapiens

<400> 384
aacagcctta ttctttctta tttccagtaa gtattccaaa gaaaaacatg ytgactggcc 60

cagctcactt ttgcacatct ctgggtcatg aatctatgtc t 101

<210> 385
<211> 101
<212> DNA
<213> Homo sapiens

<400> 385
taatgcatct aaagttcagg atgtataatg aaatctagga atgtgaacta ytcaggagaa 60

aaacagacat gatctaagag ttcaaaagaa aaacattagc a 101

<210> 386
<211> 101
<212> DNA
<213> Homo sapiens

<400> 386
gtgagatcat ggacttgggc cccctaggcc agcccagtct ctttgcagcc raggaaagtg 60

aggcttagct gtcgggggct gtgggggat gcagcttgcc a 101

<210> 387
<211> 101
<212> DNA
<213> Homo sapiens

<400> 387
ctacactaac accatgagat aggtattctt attagcatca gtttttcgaa ygagtacttc 60

aagtttcagg aaagtaaaga aacttccctg aagacagtat c 101

<210> 388
<211> 101
<212> DNA
<213> Homo sapiens

<400> 388
ttctttatca ttgaatttca aaatctttac taggacaaat cttggtggta rgctttctat 60

atcgaatttc cctaggcaca ttttgctttt gcgatttgca g 101

<210> 389
<211> 101
<212> DNA
<213> Homo sapiens

<400> 389
caggtgtgt ccacactctg ctcacaggtg gatccacggc tttccagtgc rgagagtcga 60

gatgctccct gcagcccagg ccccgggcac ctcctgcaac c 101

<210> 390
<211> 101
<212> DNA
<213> Homo sapiens

<400> 390
tacagaaaat tgccaacctc tggaagcctc agcaggacca atgtcctcca ygcagagccc 60

ttcttatccc ctaggaccgc aggcccaggc tcctctgggg g 101

<210> 391
<211> 101
<212> DNA
<213> Homo sapiens

<400> 391
actgaaactc tctgcccaca ttccacattc tccctctccc caacccttga kaacctttc 60

tttccttctc tccttccttt cctctttccc tccttccttc c 101

<210> 392
<211> 101
<212> DNA
<213> Homo sapiens

<400> 392
ttctgaacca ggcaaaggat gatggggaat gcagtcttac gacgtgatgt ygcgtttaga 60

gggttttcat cagttttaat gaaatacaaa tgcacccaaa g 101

<210> 393
<211> 101
<212> DNA
<213> Homo sapiens

<400> 393
ctgtccccgt cgtccttcct atgctcacgg cagtcacgtg agcctaaaga rgtcatgaaa 60

ggaacatagc gaccactcca tgatgtggat taactcatcc t 101

<210> 394
<211> 101
<212> DNA
<213> Homo sapiens

<400> 394
actggaccca gcccagccca gctctttcca ctgctcacct gctgcccctg ygtttccagg 60

gactccacgc tcaccaggga cacctcgctc tcccttaggg c 101

<210> 395
<211> 101
<212> DNA
<213> Homo sapiens

<400> 395
cataaataac aaaaagtcta ctaaaacaga taccttggga tagatttatt rtgccatttt 60

aggatttcac tttcaagttg cttaatagaa aatcagtgac t 101

<210> 396
<211> 101
<212> DNA
<213> Homo sapiens

<400> 396
aagaaagatt ttgatacaga ggcacacgca gagggaaaac agccatgtga mgacagtgac 60

agaaactaaa gtgatgtagc tccaagacaa aaaatgccaa g 101

<210> 397
<211> 101
<212> DNA
<213> Homo sapiens

<400> 397
actagttaca aggcagaatt atctttctga ttgcatgaaa cccatagatc rttttctctc 60

caacagaaat cttttcagta acctcaatcc acgttttggc t 101

<210> 398
<211> 101
<212> DNA
<213> Homo sapiens

<400> 398
acagtgtctg cccaggtcag acactgtgtt tagaattgct ggtgattttg kagttcagaa 60

ttactggtga ttctgtgtct ccatccttct tcattccaaa t 101

<210> 399
<211> 101
<212> DNA
<213> Homo sapiens

<400> 399
ctctattaca aagataaaat ggcaagctac agagtggtag aaagtattta yaaaccacac 60

gtctggcaaa gcacgagtat ctagaataca caaagaattt t 101

<210> 400
<211> 101
<212> DNA
<213> Homo sapiens

<400> 400
atcctaacag aagtcacatg gctttatttc atggccagaa ccaccaggct rttacaggaa 60

agccaaaaag accagacaga gaagaatgtt tccttacagt a 101

<210> 401
<211> 101
<212> DNA
<213> Homo sapiens

<400> 401
ggtgacagcc atatgctcct gatcacaaga agaaattata tcgggtccag yggcggctgt 60

cacaaagcca tatggggtgg catggcagcc ttctgcaggt g 101

<210> 402
<211> 101
<212> DNA
<213> Homo sapiens

<400> 402
ctagtaaccc tttgtgaggc tacaaaaaaa aaaggcatat ttgcttgccc rgggggcttc 60

tcttccagtt cacctgggta gaattctggg tgtagtcccc a 101

<210> 403
<211> 101
<212> DNA
<213> Homo sapiens

<400> 403
gtaggactta ctttgtgcct gagttcagtg accttgtgct cactctctta mttctccttc 60

ctccctggct ggccattcct tctcagtttg ctttgtaact c 101

<210> 404
<211> 101
<212> DNA
<213> Homo sapiens

<400> 404
tttgcttcct ctctcacaat gtgatctctg cacatgttgg tcccttgtca mcttctgcca 60

taaggagaag cagcctgtgg ctcgcaccag aagcagatgc t 101

<210> 405
<211> 101
<212> DNA
<213> Homo sapiens

<400> 405
agtgttgttc tgtgttatta ttctctaatg tagaatcgca ccatcctggg rgtcaggcat 60

cttccgcctc ctctttgacc tagtttgtgg cacacagcag g 101

<210> 406
<211> 101
<212> DNA
<213> Homo sapiens

<400> 406
aagtgaaact taaatcttga atcatgagta aaacgtacca agcaaaaaac rgacaatttg 60

atctttgacg aacctgacac aagcaatggg gaaaggattc t 101

<210> 407
<211> 101
<212> DNA
<213> Homo sapiens

<400> 407
atctgccttc tagtatgtga ggcaaccttc atcagcatgt agtagcatgt yggtgctggc 60

tagttacttt ccaagaggga gataaacacc tcaaaataag c 101

<210> 408
<211> 101
<212> DNA
<213> Homo sapiens

<400> 408
tagtgaggag tgagaattat atcacaggat ttttgcaaaa gctgtaataa kataactaat 60

actactgcat tttgttccca acattcacaa ttgaagaaaa t 101

<210> 409
<211> 101
<212> DNA
<213> Homo sapiens

<400> 409
aaataaaaag tcataaaaag aggaaagaat aaaaatttcc attcaatagg rattgatctt 60

aaacatagat ggagggatca gacaagggaa gtcatgtgat t 101

<210> 410
<211> 101
<212> DNA
<213> Homo sapiens

<400> 410
acaagtggtt aggtagacag aagctatcgg gaacattctg gactgctgga rattgctata 60

gtctcaacat tttctaagac agtcgggtat agagctttgt a 101

<210> 411
<211> 101
<212> DNA
<213> Homo sapiens

<400> 411
gttgcagccc ccctgagccc ccattcacag gaggtctcct gctacattga mtataacatc 60

tccatgcccg cccagaacct ctggagactg gtgagtaagg c 101

<210> 412
<211> 101
<212> DNA
<213> Homo sapiens

<400> 412
ggagtaaggt aagtatgcat ggctgacttg aaaagatact ttctatatac rttgcttaat 60

aaactatcaa attgctgcag aatgatatat gtggatgaga t 101

<210> 413
<211> 101
<212> DNA
<213> Homo sapiens

<400> 413
atataaggca aagctcataa ccatcctcca gtgttcaggc tcagcataag ycctctagga 60

aacctttgta cctttctttg ggcctccccc accatagccc t 101

<210> 414
<211> 101
<212> DNA
<213> Homo sapiens

<400> 414
gtccttaaaa ggaagggagc tcccgtattc ccctcttctt ccttcctctg kgctggcata 60

tgaacacaat gactggaagc tgaggagtca tcctggatca t 101

<210> 415
<211> 101
<212> DNA
<213> Homo sapiens

<400> 415
ctcggttgtc ctcaagcaaa aggaatgcta tcaataagcc ttcctaccac rtattgaaaa 60

ttaaagtcct tccttttac actttaagac cttctaataa g 101

<210> 416
<211> 101
<212> DNA
<213> Homo sapiens

<400> 416
ccctaattga gaataaatct gtctgaggca gatgtttggc aaaagtagtg ygagtgggtt 60

ttcgttaggt cttttaccgt tcttagaaat gctgtcagca t 101

<210> 417
<211> 101
<212> DNA
<213> Homo sapiens

<400> 417
ctgcctcagc ctggagacca ggatggcacc cccaagtcct ttcaaagtca yctgcaatgg 60

aaactctctt gcttttagtt tttcccagga cagtcagcca a 101

<210> 418
<211> 101
<212> DNA
<213> Homo sapiens

<400> 418

caaataaccc acactttcct tacaaatatg aattgacata tttatcaccc rtcggtctgg 60

ttttaggttt tctattctgc gttgttctct gcctgactat t 101

<210> 419
<211> 101
<212> DNA
<213> Homo sapiens

<400> 419
gaagtatgga gacaaaaagt taaggagggt gagaggatag aggagtctca ytgaagatcc 60

cctggttaaa accactgcct catttctgtg aacagcctac t 101

<210> 420
<211> 101
<212> DNA
<213> Homo sapiens

<400> 420
ccatgtccct gtgtcatttt tactcttggt gcttgtcgcc tttcaacata ytatatatct 60

catttgtttt ccttgtgtat taaccatttc ccacattaaa a 101

<210> 421
<211> 101
<212> DNA
<213> Homo sapiens

<400> 421
cacacagctg caattgagtc ctccactgat gctaccagga gctctagaac kgggatgggg 60

ccttcagggt gttctgaatt tgggcaagga ggctgggctt t 101

<210> 422
<211> 101
<212> DNA
<213> Homo sapiens

<400> 422
aactcagagt ggatttggcc atgaaagata aagtaaaagc aagtataaca ygaaagaaca 60

aaaaagcatg actcatatct gtgcaggctt tttaatatgt t 101

<210> 423
<211> 101
<212> DNA
<213> Homo sapiens

<400> 423
gccctataag agaggacagc agaaacaaca gaggaaaaag tgacagggtc kgctgttgaa 60

atgcttatca aagagtgggc atttgaacta agttatgaaa g 101

<210> 424
<211> 101
<212> DNA
<213> Homo sapiens

<400> 424
gctatcataa aacaaatatt aagcacagcc cctaaataat ctttggcagt rtatgtcttg 60

gcaattttga tgtaattatg tttcatcatt ttctactttc c 101

<210> 425
<211> 101
<212> DNA
<213> Homo sapiens

<400> 425
acttacactg aatgcaatac atagtaattt gaacaggagt ttaatctagt yaatggggac 60

cctatggagg gtcagaggac tccaatagcc agtgtgagtt g 101

<210> 426
<211> 101
<212> DNA
<213> Homo sapiens

<400> 426
tagaaaaaga aagtaaaaaa ggaaaattca tgaactgaaa aaagagtgac rttttcataa 60

aatgagagaa aataaggtct atttataggt ggaagggctg a 101

<210> 427
<211> 101
<212> DNA
<213> Homo sapiens

<400> 427
atgaataata ttcccttctg tatatgcacc acatcttaaa aaattcattt rtctgtagtt 60

agacaagtag gttgattcca aatcttgact attgtgaaca g 101

<210> 428
<211> 101
<212> DNA
<213> Homo sapiens

<400> 428
aaggagataa tagtgggtgg gtgattactt gaaactgatt tttggagaag ktcattaatt 60

aaatattcat tcattaatta aagaaacaat gtatgtcaat a 101

<210> 429
<211> 101
<212> DNA
<213> Homo sapiens

<400> 429
ttctaaccca gaagctttct attttttgt tttcagaaga tccccagata rcatctatcc 60

aaactaaaat gagaacacag tctgacggac atgaggggat t 101

<210> 430
<211> 101
<212> DNA
<213> Homo sapiens

<400> 430
actcgtggag agtgcttctg cattttgata ctctgaagtg attcctgcaa rcaacagttg 60

tttcacattc tagactagaa cttcagagtc atgtacaact g 101

<210> 431
<211> 101
<212> DNA
<213> Homo sapiens

<400> 431
gcttggtgat actctttcaa gccttgaagg ggcctgttga tctttcccta ytccactgcc 60

aacttcagtt ctccagttct ctaaagtggg gctttattct a 101

<210> 432
<211> 101
<212> DNA
<213> Homo sapiens

<400> 432
gttcaagagt tgggcatctt aactacttta tcctctgctg tcaaagttct yaaaggtctc 60

ttggtctctg atctgctgcc agcctctgcc tggctggtaa a 101

<210> 433
<211> 101
<212> DNA
<213> Homo sapiens

<400> 433
aggactggac atatctgcac tcctgccctc tgacttcagc cgctacttcc ratatgaggg 60

gtctctgact acaccgccct gtgcccaggg tgtcatctgg a 101

<210> 434
<211> 101
<212> DNA
<213> Homo sapiens

<400> 434
gggtctggaa ggacctctgc ctgggtgttt gacttggaag gggacagtgg ytctgggctt 60

gggttggaat tcagaaccca tccccgggca gctgcgtggg c 101

<210> 435
<211> 101
<212> DNA
<213> Homo sapiens

<400> 435
gtctttacag aactagagtt caggggggaat atcagaggta aaaaagctga raaaagcatt 60

gacttcaaat gccagatacc attttgattt ttggcagagc a 101

<210> 436
<211> 101
<212> DNA

<213> Homo sapiens

<400> 436
tggaggtgtg ggatagccag tattacaacc aagagtttac atctgtgttc yccaggccca 60

cttaaataga accacagcta ccaatcactg ccatttatca t 101

<210> 437
<211> 101
<212> DNA
<213> Homo sapiens

<400> 437
atccagtgtc tggggtgggg aacgagagtt atcatatggc caaataactt maagctgagc 60

gatgggcatg tggcatttat tgtacaattc tctgtgcttt c 101

<210> 438
<211> 101
<212> DNA
<213> Homo sapiens

<400> 438
ctttctaaat ggaccctaag cttctctagg tcaagaacca tgatttaggg ktcttcgatg 60

tgcctatcac ttgagtcaaa aaccttaaaa tagtaatggg c 101

<210> 439
<211> 101
<212> DNA
<213> Homo sapiens

<400> 439
tgagattaca acctagtaga agcctgtaag tcagtgtcta catgacagca ytttgcatgc 60

caagtccagg ccatgactgc tcattgtaga cgttgcttgt g 101

<210> 440
<211> 101
<212> DNA
<213> Homo sapiens

<400> 440
ctgtatagtt tgtgagttat tgcaaaggga ggattgccca ggaaccatac raggctgctg 60

tggagcagac tcagccagtg ctctcatatc catggtctcc c 101

<210> 441
<211> 101
<212> DNA
<213> Homo sapiens

<400> 441
tagttatgaa gttttagggg aaatatgtcc cccttttca cttggtacca mgttttgaga 60

taggcaattt tctttgtagt cccctgagga aggatttggg g 101

<210> 442
<211> 101

<212> DNA
<213> Homo sapiens

<400> 442
gttacaagtc agccgtctgg gtgttaaatc tacacgtacc aaataaccaa ytgtactttt 60

ttcactgaaa tgttagtatt atgtagagac agccacgact c 101

<210> 443
<211> 101
<212> DNA
<213> Homo sapiens

<400> 443
ttctctctta catgaacaat tgaacatttg ttagacatag tgatgctcct yagtattacc 60

cattcacttt tttgggaggc acaagaaagg attgcacttc a 101

<210> 444
<211> 101
<212> DNA
<213> Homo sapiens

<400> 444
ttgaatccag aagctggcca gctgttccaa atcagctatt gttatcaatc kcctctgaaa 60

atcaacttat caagcagttc acagctatca gatgttaaaa a 101

<210> 445
<211> 101
<212> DNA
<213> Homo sapiens

<400> 445
cctgctaatt ctttctccat ctgaggggtg agaaagactt ctttttagct rtctctttca 60

ctgccaacct gctttgataa tgttctgggg gctttaccag a 101

<210> 446
<211> 101
<212> DNA
<213> Homo sapiens

<400> 446
aaggcccttg agactgaggt ctcaacagat tgggacaaag aaggcaacag rataagggca 60

taggtgttac cctgggaccc cagagacctg aattctggct c 101

<210> 447
<211> 101
<212> DNA
<213> Homo sapiens

<400> 447
ccagggtttc agacaagtct agagcaagtc aggatatcaa taagacccaa yaggatgtag 60

ggctgcctgt ctagggagac atttagctta tcttccccgg c 101

<210> 448

<211> 101
<212> DNA
<213> Homo sapiens

<400> 448
ctcagctgga gagcaaccct ttcggtttaa aataaactaa tgaaatccct raggacaaat 60

atcactatga tatgcacaaa aacagcacat taatgcaaca a 101

<210> 449
<211> 101
<212> DNA
<213> Homo sapiens

<400> 449
ttttctctta aaagactcag tacattatta gaaatgcctt tcactaacat ytaacaaata 60

aaacagttct atagggacaa tgaagttgac atttccattg t 101

<210> 450
<211> 101
<212> DNA
<213> Homo sapiens

<400> 450
tctactggtc ccatgtccca gagatcacaa tgccttccta tctatcactg ycggccattg 60

ctggtattta agggtatatc tctcttctgc ctccacccta g 101

<210> 451
<211> 101
<212> DNA
<213> Homo sapiens

<400> 451
acagtcttca gtttatttct cactgaactg atcctttgtt tccctccccc yaccacctac 60

agaatctaaa ttagagtgat ttcctcccgc agaaaagtca g 101

<210> 452
<211> 101
<212> DNA
<213> Homo sapiens

<400> 452
gcatctttag gacttctccc ttgggattat cttcactatt agcttttctc rttttgtttt 60

attttttcac atcccctcaa tggaaggcaa tacacttagc a 101

<210> 453
<211> 101
<212> DNA
<213> Homo sapiens

<400> 453
ttctaatcat tcagataaag gtttaatttg taccaagatt atcctcaaaa yatcactgaa 60

tacagtaaac actggcaatt gccattaaaa acaaattata t 101

<210> 454
<211> 101
<212> DNA
<213> Homo sapiens

<400> 454
tcatgttcct aaaaggacaa catgaagtat aaacccaaac aatagatgta mactaatcat 60

ccctaacaat atccatagtg aatggttcca acagagtgca c 101

<210> 455
<211> 101
<212> DNA
<213> Homo sapiens

<400> 455
catgtactag catcaagaaa catctgactc ccattctgtc attctgtacc yacgtcatct 60

tgactagaca tcaattaaga gtttcctgga aaactcggaa c 101

<210> 456
<211> 101
<212> DNA
<213> Homo sapiens

<400> 456
gaccagacta acccttttc cttcttttgg aggttatgat taggattgtc mgagggcaaa 60

gggtttaatt ttttcattaa actaacaaca tgttttgagc a 101

<210> 457
<211> 101
<212> DNA
<213> Homo sapiens

<400> 457
atctcctagc ctacaaaatt attctttaga gaatccattt tcccacaaga yatgcaaaaa 60

ctaaaacaaa ccacaacacg tgggccagat gtttcttcaa t 101

<210> 458
<211> 101
<212> DNA
<213> Homo sapiens

<400> 458
gaagaacgag ccgtttaaat cacacatcag accataccat tcctctgctc raaaccctgc 60

aatggtttcc tgtttcactc agggtaaaag ctaaaggtcc t 101

<210> 459
<211> 101
<212> DNA
<213> Homo sapiens

<400> 459
ggaattttta gagaaactac atgttctaac atgttctctt agggtgcttc rtacagatcg 60

tcaaggaagt atcccaaaaa aaatcaatga acacccggaa t 101

<210> 460
<211> 101
<212> DNA
<213> Homo sapiens

<400> 460
ttttgtcccc atttttttctc ccatgtaaga catttttaat ctaccttgca rtgaagaggc 60

tgttaaacac ttgtaccagc accacccagc ttttccatgt c 101

<210> 461
<211> 101
<212> DNA
<213> Homo sapiens

<400> 461
ctggaagtta ggatttgtac aaaagattga attagttctc agtgacccct ygacctaacc 60

cttggtccct cactgagtgg gctccttgga gcgctgtgat c 101

<210> 462
<211> 101
<212> DNA
<213> Homo sapiens

<400> 462
ttgaaacatt gtttttgtag aatttgcaag tggattttac agcgctttga rgattttaga 60

gcaaatgaat aaatgtaatc caccatataa agagaaccaa a 101

<210> 463
<211> 101
<212> DNA
<213> Homo sapiens

<400> 463
ttttaagcaa taagcatgct gtgcttaggc tgtctcagca ctattgttaa rtgctttaat 60

tatgtaactt ttgatacatt catgttatca tatgttgtaa t 101

<210> 464
<211> 101
<212> DNA
<213> Homo sapiens

<400> 464
ctgaggcagt gcatacccaa gactgtcact tctgctctgc atacctttaa kattcttcct 60

taggattctc tagtacacag tggtctcatc caccagctgc c 101

<210> 465
<211> 101
<212> DNA
<213> Homo sapiens

<400> 465
caaggagagg agataagcat cctcactaca acctgaccaa ttcttaacca yagaatctgt 60

aaataaaaca aaatggttgt ttgcctctga gtctggggat g 101

<210> 466
<211> 101
<212> DNA
<213> Homo sapiens

<400> 466
atgtcaaaat attgcaaagc tcctactgca aatggctcat gtaaccaaca ytattagaga 60

atatttcctg tttagaaatt tattttaaaa attgaaatta a 101

<210> 467
<211> 101
<212> DNA
<213> Homo sapiens

<400> 467
cagaggtgtc acttgtttta aaagtgagaa actaaccagt gcttagaact rtaacccca 60

gagcattgcc tatgaatacc aaggacctag aaatctcctc a 101

<210> 468
<211> 101
<212> DNA
<213> Homo sapiens

<400> 468
ggctgaacag atgaaattgc tttagctaaa ggaagtggca cgaatttact yatttattag 60

atgtgcagga tacatccatc acaccgacct ctggatcaac t 101

<210> 469
<211> 101
<212> DNA
<213> Homo sapiens

<400> 469
ttcctcataa acatcaagta atgtgctggt aactgggaaa tactgcagtt kgttagtaga 60

attttatcag aagtcaacaa aatattccgt tttgcatgcc t 101

<210> 470
<211> 101
<212> DNA
<213> Homo sapiens

<400> 470
cacatcatct ggaaataaag aacattttgc ttcttccttt caaagctaca ygctgatcta 60

tcttgaagtt tatgggtgtg ggttcttctg ccatctcaaa t 101

<210> 471
<211> 101
<212> DNA
<213> Homo sapiens

<400> 471
gcagtatctc ctgggtatgt ccatctggtt atgtaaagtg aattattggt rgctttcccc 60

agctctttca atttttaaaa aataagtaat acatccaatg c 101

<210> 472
<211> 101
<212> DNA
<213> Homo sapiens

<400> 472
caggtgatag attaaaaact atggttactt aaaaaatgac cattgaactt yataaaacta 60

ttctgcctga tttccaactg gtatcaaaat tttaagtgat c 101

<210> 473
<211> 101
<212> DNA
<213> Homo sapiens

<400> 473
caaggataat tatggctatc ttttgtgtct taattttgtt tgtagtttca ygtgaaagtc 60

ttcattctgg ggggcttaga attaaagccc tctttattta g 101

<210> 474
<211> 101
<212> DNA
<213> Homo sapiens

<400> 474
tgccaagcat aatcttacca tagggccttt gaacgggcta tgcctccacc mgaaccactt 60

ttcccgttta tctgatcact ccttcacctt caagtcttga t 101

<210> 475
<211> 101
<212> DNA
<213> Homo sapiens

<400> 475
ttgtatatac tggaatagag taaaccatac aacaaaacag aactctgtct rtatcaggaa 60

accttgttta attttaggga aaatgatata catttgaata c 101

<210> 476
<211> 101
<212> DNA
<213> Homo sapiens

<400> 476
tatcaaaatt ttaagtgatc aagagtaaaa gaactttatc aagaattata rcacttaaca 60

ggtcgacaca gatgcagccc ttttattata taggtataat g 101

<210> 477
<211> 101
<212> DNA
<213> Homo sapiens

<400> 477
taaaatgttg ggtggagatg gtgccttttc cagtggaagc tactcatggc rtcagaacaa 60

acccacccca cggacaaatt cacaaagggt gtaaaactgg a 101

<210> 478
<211> 101
<212> DNA
<213> Homo sapiens

<400> 478
tgtcatacat tggcccagca catatgtgtg attgtgactc taatatacac rctcaactaa 60

aagttaaagg tgtcaccctc aaagatcagg agattgtgtc a 101

<210> 479
<211> 101
<212> DNA
<213> Homo sapiens

<400> 479
cgaagaacag agggccagga agctaattaa taaatgactt gctcaagaca rcacagctag 60

caaaggcagc ctgatgtgga gcacagccca gcctcttccc t 101

<210> 480
<211> 101
<212> DNA
<213> Homo sapiens

<400> 480
tggttaattt ctactattac agtggtccat agactcattt gaagcaaatt yatgaaagga 60

atattgccgt aaattcgatg ggatttcatc aatatcttaa a 101

<210> 481
<211> 101
<212> DNA
<213> Homo sapiens

<400> 481
agtttaaatg cctacagcaa tcttccaaga cacaggtgct atttttgata rcactatgga 60

actgtacaaa actatacaaa caacattatg actctgcact t 101

<210> 482
<211> 101
<212> DNA
<213> Homo sapiens

<400> 482
gccagtactg atggccctgt gccttcagtc tagcgtcctg gagtctgaaa ygggagatgg 60

aagacagtag cttgaataca gagggtgaaa gattttcctc c 101

<210> 483
<211> 101
<212> DNA
<213> Homo sapiens

<400> 483

acaagcccag agaaaacatc catacaacag gcttgaaaga ctccaagaat mtctcgccta 60

aaaaattggt atcatatttc cccagacaaa agccaactta a 101

<210> 484
<211> 101
<212> DNA
<213> Homo sapiens

<400> 484
aaagatacag ggagtggact gggctttgga acaactcagt tttacttcca yggtattctg 60

atgctcaagc agccacagaa ctcagatttc agggcagatg a 101

<210> 485
<211> 101
<212> DNA
<213> Homo sapiens

<400> 485
ttgagttcag tgtgaggagg tttatgccta gaaaaggtgc tcaccaataa ygtgcctcag 60

ttcccataat agcaagatcg agaaggttct ttagtctccc g 101

<210> 486
<211> 101
<212> DNA
<213> Homo sapiens

<400> 486
aaaacttcat acctctccag ggagacagtt cccagaaacc tccctcccct rcaaagcact 60

cctataacaa ataaataaac tacatttccc aaagttctct t 101

<210> 487
<211> 101
<212> DNA
<213> Homo sapiens

<400> 487
ttctccttca ggaattctta tcgtgcataa gttagttctc tagatagggt yccataatcc 60

cataggcctt ctccattttt tttcactcct ctgactagaa a 101

<210> 488
<211> 101
<212> DNA
<213> Homo sapiens

<400> 488
atccctaact ggagatcatc tcctcagtgc tggacttgag attcaaattc rggaccttac 60

ttctgagtct gctcaaaagc actctgaaac agcatccaga g 101

<210> 489
<211> 101
<212> DNA
<213> Homo sapiens

<400> 489
tttattctgt aatgtgatta taagccatta gcaggattta tgcaagggag ygatatggta 60

gatttacacg cttaagagat tattttgcct gttgggtaga g 101

<210> 490
<211> 101
<212> DNA
<213> Homo sapiens

<400> 490
gatactgatc tataaaatat aagccaaata ctgttaagaa aagttaacca ygaataagcc 60

aggtatggtg gctcatgcct gtaatcccag cactttggga g 101

<210> 491
<211> 101
<212> DNA
<213> Homo sapiens

<400> 491
atctggaaga cccaccctca agtggtacat accagtgcca ttcacattct rctgcctaaa 60

ttactcactt tgcctcaccc aactttcaca aagcatggca a 101

<210> 492
<211> 101
<212> DNA
<213> Homo sapiens

<400> 492
tgtgtcattt aaccttgcag aagtttaaat tctaccagta tttcctgtta yagtttctgc 60

ctttggtgtc atgtgaaaaa aaaagaccat tactatagca a 101

<210> 493
<211> 101
<212> DNA
<213> Homo sapiens

<400> 493
tattccatta actaaacagc aacctcgaaa gaaatcaata ctcggaaggt yctgtagtag 60

cagccattcc atggatggga caccagaggt ggggcaggag c 101

<210> 494
<211> 101
<212> DNA
<213> Homo sapiens

<400> 494
gctcccagca gctcacccct ccagtggctg ttctttctac ctgtcaaagc ytgtgctgac 60

acatatactg ggaggtgacc cccagctgcg gctgccccac c 101

<210> 495
<211> 101
<212> DNA
<213> Homo sapiens

<400> 495
tcaatatgga agaacttgtc caggcttgtg cagaccacca tgtctctgcc ktacaggctg 60

acatttaaca atggtgaagg caatctcttc ttggaaaaaa t 101

<210> 496
<211> 101
<212> DNA
<213> Homo sapiens

<400> 496
agactgtgca gtgtccagtt cttttattaa gtacatgggg tctgtagtca yacttcctgg 60

ggcaaaatcc tgcctcttat gtttttgacc ttcggcaagt t 101

<210> 497
<211> 101
<212> DNA
<213> Homo sapiens

<400> 497
gtttagcatc tgtggtaagt gtgttcgaag gccgtgtaag cacattttat yatgagcatg 60

tcttacttcc aagttaagat aaagatttgg aaattaatgt a 101

<210> 498
<211> 101
<212> DNA
<213> Homo sapiens

<400> 498
atccagaatc tacctacatt cattgttatt aatttgtacc cctggtgttc rgccagtatc 60

accttctccc aatctatttc agccagtgac aatgaggaca t 101

<210> 499
<211> 101
<212> DNA
<213> Homo sapiens

<400> 499
agagatgccc ccgccctcca gggaaactgc acagacatta caaacaagca ygctcttatc 60

aagcaggaga ggtctgggtc gggggctgg ggggaaggat t 101

<210> 500
<211> 101
<212> DNA
<213> Homo sapiens

<400> 500
ttattgctga attggtataa agatgaatat atgcctggct gcattctact yattcttctt 60

atttcaagag aaattaaatc atttcatggg cccctaaaat t 101

<210> 501
<211> 101
<212> DNA

<213> Homo sapiens

<400> 501
ggctaatcaa tttgatgtct tttaaaacta atattcttca aattttttt yagtgtctat 60

ttaggggaat ggctgatggc tgcatgaagt gggggactca g 101

<210> 502
<211> 101
<212> DNA
<213> Homo sapiens

<400> 502
tcttgcttcc aggggaagct gccaggtaga agtagtgagg aatctggtat ygcactgtcc 60

caaggggcgg gacacctgcc tttgaagacc cctgggttct g 101

<210> 503
<211> 101
<212> DNA
<213> Homo sapiens

<400> 503
ctactgatct ttcagactgc actgttcatt ctaattctta taatacaaag kcagagcagc 60

agatactcta gggaaagaat gcttgcaccg tgaaatccac a 101

<210> 504
<211> 101
<212> DNA
<213> Homo sapiens

<400> 504
aacctccctc cctgctgcta tcttatgtac actcttaatg tgcctaacct yccacgagtg 60

tgcagagatg ctgctagagc agtccctgct tagatcactg g 101

<210> 505
<211> 101
<212> DNA
<213> Homo sapiens

<400> 505
ttgttcaaaa tgtatatttt ctcgttttta aattatgtaa ttttggctgg rcgtggtggt 60

ttacgcctgt aattccagca ttttgggagg ccgaggcggg t 101

<210> 506
<211> 101
<212> DNA
<213> Homo sapiens

<400> 506
attcacacct caggtcttca ctttggggag cgaagccttt tagcagaaat rccagaagta 60

ccatcttgcc aaatggtcag gaactgtctg atagagatgg a 101

<210> 507
<211> 101

<212> DNA
<213> Homo sapiens

<400> 507
aggcactgtt ttatcatggc tcatctagat tccaaagtcc acaataaccc rgatgatcca 60

tgtggtcata tcatgctctt cacaagtaca tgcctctgct t 101

<210> 508
<211> 101
<212> DNA
<213> Homo sapiens

<400> 508
tcaactacag gtgtgttcct gatggccttt agctggagcg tactgacaca rtaacaggct 60

ttgaaattca agtgattcag tttggcatct tagctccacc a 101

<210> 509
<211> 101
<212> DNA
<213> Homo sapiens

<400> 509
catgaatatg tacaatgatt atttgccaat caaaattctg catcctccag magcatgcta 60

tccaaacttc tttcatcatc cctctccctc tggaggagga c 101

<210> 510
<211> 101
<212> DNA
<213> Homo sapiens

<400> 510
ttaacaaaaa acgaatatta taaattgatt atgtttcctt gcagctggat rgcttagcct 60

gaagtatgga ttgctagtaa ttcctccagt cactcaacat t 101

<210> 511
<211> 101
<212> DNA
<213> Homo sapiens

<400> 511
ttcatcctta ataaaaagaa aattgcatag ctttttatat tgttgcaaat kcatctccca 60

atatcattgt cagcttagtg atattctcca tattttaaaa t 101

<210> 512
<211> 101
<212> DNA
<213> Homo sapiens

<400> 512
aatgaagtaa agcaagtttc agctgtttct ttccccaatg cacaacctta rtttcctttt 60

atcttaaaca ccaggaatca aacaatctca accatctgaa a 101

<210> 513

<211> 101
<212> DNA
<213> Homo sapiens

<400> 513
tagtgtagcc atccaatgga ctactatcta gcaatgaaaa agagtgcacc rttggccaca 60

tggcaacagg gataaatctc aggagtgtta gagcaggtga a 101

<210> 514
<211> 101
<212> DNA
<213> Homo sapiens

<400> 514
cctttcctct tccccgcacc aacaccagct ccatgtgcat ttattgttgg rttttaacac 60

ccgtgtcctc cctccctctc cccagtgttc tttcacagct t 101

<210> 515
<211> 101
<212> DNA
<213> Homo sapiens

<400> 515
gaagagacca attgcccttt ttacagatat tatgattgcc aacacttaac rtgtaaacaa 60

attattagaa caacattgtt cagcaagatt accgagtgca a 101

<210> 516
<211> 101
<212> DNA
<213> Homo sapiens

<400> 516
ttgtattttt gacgtcacta gtgtcatttt ttgagtcctc taccaatttt ycaagggtat 60

atcatcttca gttccaattg aacatacagc ccttttttgaa t 101

<210> 517
<211> 101
<212> DNA
<213> Homo sapiens

<400> 517
aaaaaaaaga atacattttg tttagatgtg gaaaatgagt agcttgaaag yaaagccaaa 60

caacaacaaa aacaatgaca aaaaatctgt atgtcgtaat c 101

<210> 518
<211> 101
<212> DNA
<213> Homo sapiens

<400> 518
cattttaata cgtgtcacac tgaataaatt tatgcacatt tattcatgtc raaaggaaaa 60

attaatgttg tgatgttgac tccttgatga agtttttgaa g 101

<210> 519
<211> 101
<212> DNA
<213> Homo sapiens

<400> 519
ttacagattt ttaccataga tatactcata gagaaggtag cacccactca rccctagcaa 60

tagtgctagt gtttacaaaa ttgcaaaaga agtatgacac a 101

<210> 520
<211> 101
<212> DNA
<213> Homo sapiens

<400> 520
tcaggcggcc cagagagcag cgctccacat tcagcttcac ggagccagac rcaaggtctc 60

gaatggtgaa gctctggtct gcagccaccg tggttacctg g 101

<210> 521
<211> 101
<212> DNA
<213> Homo sapiens

<400> 521
cccaaagaat ccttccctta cagcaggcca gaaagctatt gtcctagcct rtggaaacac 60

ctaaaacaca ctggggagat gtggacactc agcccattgc a 101

<210> 522
<211> 101
<212> DNA
<213> Homo sapiens

<400> 522
caccactgac actatttaca gccaaagaaa tcatatgaaa ccgtactagc rcatgcacca 60

gaaccaaatc caaagtgccc caccaaaaca acaccataaa t 101

<210> 523
<211> 101
<212> DNA
<213> Homo sapiens

<400> 523
aattctcatt ctcctaatcc aaggctctgt gtgatacatc acactgtgtt yattacttta 60

ttacagagca agtaaacaga tgcttagtgt agatcacgca g 101

<210> 524
<211> 101
<212> DNA
<213> Homo sapiens

<400> 524
ctggattttg tggtcttatg ctatttccac tcattctcca aatgtaaccg kaaagaccat 60

cccaaaatgt aatacaaacc tttttaaatg cccatttaaa a 101

<210> 525
<211> 101
<212> DNA
<213> Homo sapiens

<400> 525
caggagcagg gtggacgtca aaaaataatc ctgatgctat ttggctcatg katgattcag 60

agcaggtgct gtcagagccc taatttccct tgtttttgaa c 101

<210> 526
<211> 101
<212> DNA
<213> Homo sapiens

<400> 526
agcaaactga taagtcaaag atgcatatgt aattcccaga tcaaatacta raaacagcaa 60

aaagaggata aaatagcctt ttcagcaaat ggttctagaa a 101

<210> 527
<211> 101
<212> DNA
<213> Homo sapiens

<400> 527
tctattcaat tttgtttctt ttttcaaagt aaacactgtt ttgtaaataa yacagaactg 60

aaccccaaaa tacataactg ggcattggag gattagaaca t 101

<210> 528
<211> 101
<212> DNA
<213> Homo sapiens

<400> 528
agaggaaatg tcacaaaact cttcatagtt acaaagacat tgtgacactc rgtagaggta 60

aaggttccag tattttaaaa acatgagaaa tatgggttaa a 101

<210> 529
<211> 101
<212> DNA
<213> Homo sapiens

<400> 529
cttgctgctt tctgcagaaa cccctggaag cagtccaaat gcaaagttag rgcttcagag 60

aatgcaccct gtaaatggta gttgtgtata cccttaccat t 101

<210> 530
<211> 101
<212> DNA
<213> Homo sapiens

<400> 530
ttcaaggaaa caggtgaaca tataaacgat gtaacagttt atatgtagga rtgccctttg 60

gctctgtcta ttgctgtcag tacattattt acctgctcca g 101

<210> 531
<211> 101
<212> DNA
<213> Homo sapiens

<400> 531
ttttattagc aggtctagat tgagagagat ttacctcggc agtaccatag ygtggataat 60

attcagttag gtttgttcag aggaacttcc ccatcattct g 101

<210> 532
<211> 101
<212> DNA
<213> Homo sapiens

<400> 532
acatgaatag atgggacctg tatttgctta attccagtag actaaatact ytggcctaaa 60

tagagttgtc aatctcataa acccaagaaa tactcagaaa c 101

<210> 533
<211> 101
<212> DNA
<213> Homo sapiens

<400> 533
agagctgact tttacccaag gggctgtggg tggaaaccag atgaaatggg rtatgtagtt 60

gatggtatgt gaggacctaa tactgtctta taaacattta t 101

<210> 534
<211> 101
<212> DNA
<213> Homo sapiens

<400> 534
atcgccgttc ccgaggtcgt cccctttgca cctgtccgcg ggtcctcggg ygtgtggctt 60

ccgggcacac agaaaaccgt gtggttctag gatacatggg g 101

<210> 535
<211> 101
<212> DNA
<213> Homo sapiens

<400> 535
attagtcatg gaaaaggaat aaaaggcatc caagttggaa aagaagaaat raaattatct 60

ctgcttaaag atggtatgat ctatatgtag aaaatcctaa a 101

<210> 536
<211> 101
<212> DNA
<213> Homo sapiens

<400> 536
tctttctaat acacatattg catctattcc atgccttcaa tgaatttccc rttgtttaaa 60

ctataggtca agaaactgtc caattgctat acttgtttgg g 101

<210> 537
<211> 101
<212> DNA
<213> Homo sapiens

<400> 537
taaagaaaaa aatctgtaca tgttttggac agacacaatt tttttcccag rctatttttg 60

acctatagtt atttgaatcc acagatgcag aacccacgga t 101

<210> 538
<211> 101
<212> DNA
<213> Homo sapiens

<400> 538
gccttgtgtg agcattttta tctctggcaa gctcccttcc tctcttagat ratagagatt 60

atctcccggg attacaagga cagcttctcc acaacgaatc c 101

<210> 539
<211> 101
<212> DNA
<213> Homo sapiens

<400> 539
cctagaccag tgtggcgtat aggctataga agattggtcc tgaatgctaa yggcagggat 60

gagtgtaaga tcatcaaaac ttatttgtgg gaaggagagc t 101

<210> 540
<211> 101
<212> DNA
<213> Homo sapiens

<400> 540
gggaaggtcg ttgttttcct cctatttcaa ggtgttgcac ctttggccaa mgggcccaca 60

gcactgcttg gaggaaccac agggcttcag gacgtgaccg t 101

<210> 541
<211> 101
<212> DNA
<213> Homo sapiens

<400> 541
ccaaaagaga aaaaattctg acgggggcat aactggagaa taaagtgatc ytaaaatact 60

gctgaaacaa aaagtcatct gccccctgga ccgttgtctt a 101

<210> 542
<211> 101
<212> DNA
<213> Homo sapiens

<400> 542
aaatcttggc taatcattta atctttgggc atcaatttct tcactgttaa matgacagtt 60

gtagtatttc tccttaaaat acttcagggc agaattaaat c 101

<210> 543
<211> 101
<212> DNA
<213> Homo sapiens

<400> 543
tacatgatat aagaaaataa taagaatgtg gtttcgttta ggaagattct yaatacacaa 60

agatatatct gcaaatatat tttcctagct ttggttttct t 101

<210> 544
<211> 101
<212> DNA
<213> Homo sapiens

<400> 544
ccaagtaact ataagattca tgtattagag aaaatcatat taaatttgct rttatgtgat 60

cctttagaca tataaaaatg gtatatgtta tggttcaacc t 101

<210> 545
<211> 101
<212> DNA
<213> Homo sapiens

<400> 545
acaattatat gccaacaaat tggataccct agaaaaatga aaaaaatcct rgatacaacc 60

taccaagaat gaatagtaaa aaaaaaattc ttactcaaca t 101

<210> 546
<211> 101
<212> DNA
<213> Homo sapiens

<400> 546
cagcagatac ccttaattcc tatttcccag tgagaacaaa gggcagaaaa ygtgaccgtg 60

cccacattct ctgctcccta accccctaaa caatcagcac c 101

<210> 547
<211> 101
<212> DNA
<213> Homo sapiens

<400> 547
atagcagccc ttagcccagc gacctccaga agcctcgccc acccccggat rgtataccca 60

ccctagagag tacgagtcct ggcatttgag gaagtaccac t 101

<210> 548
<211> 101
<212> DNA
<213> Homo sapiens

<400> 548

taaactgttc agtaataaca ttgatttgat tttaagaaat aatagaaaaa yagagtttat 60

actacagcag tgatttccag tagaaatata ctgggagcca c 101

<210> 549
<211> 101
<212> DNA
<213> Homo sapiens

<400> 549
agctagtgtc cagtagtcct cccaggatta taggtgaaag atggaggaga mggttcggta 60

tgcagggaat cacgcgacac agtgtccaat taatttttgt t 101

<210> 550
<211> 101
<212> DNA
<213> Homo sapiens

<400> 550
tatgtagcag caatcttaaa aaatttttat ttactaaaaa tctcatcatc yaataattat 60

ttaaatacct tttcatacta tctgtataag ttagctaatg t 101

<210> 551
<211> 101
<212> DNA
<213> Homo sapiens

<400> 551
ttatccctta tagatgccta agagcttatt tataaaatgg taatactaat rtatttaatg 60

tcatcttaca gttaccatgt acttttcagt ttacaaaata c 101

<210> 552
<211> 101
<212> DNA
<213> Homo sapiens

<400> 552
attttttacc tgcaacccct gatgtggaca ttctcagaaa aagccagcca raggaagtct 60

ttcattaatc ccaggcatgt cacataacct cagacctttt t 101

<210> 553
<211> 101
<212> DNA
<213> Homo sapiens

<400> 553
tgagctccaa gcaggcaagg aattcacctg aaagcatgaa tgaaagacag rtctggaatg 60

caccaaatga ctaggatcag gagtgtctgt aagtgtcaga a 101

<210> 554
<211> 101
<212> DNA
<213> Homo sapiens

<400> 554
catgcctgga cttcacttgt agcacatcat ttgtggaagg ctgcagtaag yactcaatac 60

tttgctgttg attgatttca gaacggattg atcagattgc a 101

<210> 555
<211> 101
<212> DNA
<213> Homo sapiens

<400> 555
tcatggaaat ataaatggaa ttttagattc atgttaaacc tctcttgtaa mgttctcaat 60

gtctatgtgt atacttcaaa ctgtaacttt ttttaaaaaa a 101

<210> 556
<211> 101
<212> DNA
<213> Homo sapiens

<400> 556
ttcatcacac cactctgact tgctacaatg actgcctgga catgctgact mcagtgagtt 60

ccaggcatca gtagggtctg aaaatataag caaaggaaaa c 101

<210> 557
<211> 101
<212> DNA
<213> Homo sapiens

<400> 557
atctgtgccc tcttagaatg taacactgga aagtggtctc cctcttatgg yttttaaaat 60

tgtgaatgtg ctggtttgag caataaactc tgaaggttga g 101

<210> 558
<211> 101
<212> DNA
<213> Homo sapiens

<400> 558
atggatcact gcccagcaga tagctggtcc tccaaacgta tttgctgaat raatcaactg 60

ccttagaggc agagatattc ttactgcatt ccttagtcta t 101

<210> 559
<211> 101
<212> DNA
<213> Homo sapiens

<400> 559
ttctgttacc taggagatgt tacttacata tgtaatactg tatcctgcac rtggaaatat 60

tcagaattgt agatagcata actctccctg ctcctattct t 101

<210> 560
<211> 101
<212> DNA
<213> Homo sapiens

<400> 560
aaggcagctt gaccacaggc aatagcttgc tgattcctgc ataaagttta rcatactctt 60

gaaatttcat ttgtctaata ttttaacctc aaactgtgcc t 101

<210> 561
<211> 101
<212> DNA
<213> Homo sapiens

<400> 561
tacagaaagc cctctgtcct tgtaacaagg tagacgctct aattgagttg rttaacacaa 60

ggtgcccgta ggcaaactaa gagaacaccc tgtaacacac g 101

<210> 562
<211> 101
<212> DNA
<213> Homo sapiens

<400> 562
ttgtgcaaat cttctgattt gtgcaaagtc ccagaagaaa tgacgataga mtgctgctct 60

cctcctaagt aaaatgaaga agtatctaag agaaacagat g 101

<210> 563
<211> 101
<212> DNA
<213> Homo sapiens

<400> 563
cagataaccc ttaaagtgaa gaactaggtg tctcaggtag ttttaggtac ytcacctgct 60

tcctgtaatc tctacagaca tttgcttaaa tatatactaa t 101

<210> 564
<211> 101
<212> DNA
<213> Homo sapiens

<400> 564
gacctcaggt gatgtttaga cttacttctt ggcctagact tatgttaaca raaccccaaa 60

aggtctaaag cactaaagag gtttgccaac tacacttaga t 101

<210> 565
<211> 101
<212> DNA
<213> Homo sapiens

<400> 565
tattttagta ccaaatgaaa tttccattca gatataattt gcgaacccct ygggtgacac 60

ttccatgcaa tgaaataata ctataatgac acaatgacag a 101

<210> 566
<211> 101
<212> DNA

<213> Homo sapiens

<400> 566
tcactcagct aatagacaga gaatgatgta taaaatcata atgccaactt rtaaatttat 60

aaatagaaat atggttgtca tacctcctta aacactgaca t 101

<210> 567
<211> 101
<212> DNA
<213> Homo sapiens

<400> 567
aagctggctg aatttttaca aggcaggaat gaaatactga agagagacat mttcttgaac 60

caaaacaagc tgaagaagag tattgtccca aatattgcac a 101

<210> 568
<211> 101
<212> DNA
<213> Homo sapiens

<400> 568
ggaatatact gtctctcagt aagtgatact gggacatctg gatatgcata yaggggggga 60

aaaaaagaaa cgactcctac attacatcgt acacaaaaat c 101

<210> 569
<211> 101
<212> DNA
<213> Homo sapiens

<400> 569
tcaatttctg ttcctttagg ccagtcagtc tgtgttacct tcttacagcg rccccaggaa 60

acgaacaaga aaccagtcca aactgcttag catgatactt a 101

<210> 570
<211> 101
<212> DNA
<213> Homo sapiens

<400> 570
tgtggatgca gaacccatag atagagaggg ctgactgtac taaagattac mtttccttct 60

ccacgagtct caacatattc atctactcag cagtaaataa a 101

<210> 571
<211> 101
<212> DNA
<213> Homo sapiens

<400> 571
ggaaaagaaa agaaatggca acctgaggtc agctgtgtgt gacccacatg yaagactgaa 60

gtagaacttg cctccttgtg aacgaaacag ggcaacaaga g 101

<210> 572
<211> 101

<212> DNA
<213> Homo sapiens

<400> 572
catcactctg ctccatctct tacctagatt ccagaactct tctttctcca yctacccaaa 60

cttttacttc tgctagtctc tattacccat gcctttctac a 101

<210> 573
<211> 101
<212> DNA
<213> Homo sapiens

<400> 573
atcctcacca ctgcaagcat taaggagaaa cccctaaaat tattctgagt rtaaacacag 60

caaaaggcgc atggacctta accaacatgt atgacaccaa a 101

<210> 574
<211> 101
<212> DNA
<213> Homo sapiens

<400> 574
tggcacaata actaactgta tttttagagt ttatcaataa atatgatgtt rccataaaca 60

cacatgaaca cactgatctc tttaaaagat ttacaatgga a 101

<210> 575
<211> 101
<212> DNA
<213> Homo sapiens

<400> 575
cccaaaggtt gttatgaggt gcatgacttt acttatttgt gtggattgat ygttaatcag 60

tatgccatat tcctaaaaat gagcacttgc tccaggtctt t 101

<210> 576
<211> 101
<212> DNA
<213> Homo sapiens

<400> 576
cagtcagaac atctttggta cacccatgga atgaatagta atgactggca ygggcagagg 60

tgaagccata tccatatgtt tattttttat taaaaaatgt a 101

<210> 577
<211> 101
<212> DNA
<213> Homo sapiens

<400> 577
tttcacaggc tcctgggccc aagatacagt gagtacaatg ggtcccacgc rgttctcccc 60

tttgagtttg aaagcttaga gttcttgagc tgaagcaagc a 101

<210> 578

<211> 101
<212> DNA
<213> Homo sapiens

<400> 578
gggaaggcac ttgtttcgtg gaggagtagg atttgtgtct ctggcagttg ycctgcacat 60

tcaagatgca agagctttct gtgcaacaca agcaaagcag a 101

<210> 579
<211> 101
<212> DNA
<213> Homo sapiens

<400> 579
caggtcatgt tttcacaaaa tgtgacattt catgtcgttg ttatgaaaac mgtggcacca 60

aattcaatct gcaccaatca tatttttatt ttaatatttt a 101

<210> 580
<211> 101
<212> DNA
<213> Homo sapiens

<400> 580
gatgggaact ggcctccttt taatagcaca ttaacaacat tattctaccc raaggaagac 60

agcttccctt tggccttagc tgccttgtga gtttggtgaa c 101

<210> 581
<211> 101
<212> DNA
<213> Homo sapiens

<400> 581
aaaattctgt caatagacac ataggtaggg agactattcc tgagtggtgc mtgcctctag 60

aaaaacaaac ctataagtga gataaagttt agatttcata a 101

<210> 582
<211> 101
<212> DNA
<213> Homo sapiens

<400> 582
tacatatgct tcagaagaag gctaagggtt cgttatctta aaggggggaaa rgagtgtctt 60

ggacaccagc cttagctgtc agacaggtct catcttaatt c 101

<210> 583
<211> 101
<212> DNA
<213> Homo sapiens

<400> 583
actattcccc tcagtctcct cactatgcat caaaactagc aggtaaatcc ytggctcatg 60

atgcatccat aagcttttct ctcacttttc taaaatatta g 101

<210> 584
<211> 101
<212> DNA
<213> Homo sapiens

<400> 584
tctcaaactt ctgctctaaa ctggcaacat ttaaagagtc tatttgggaa ytttggggaa 60

cccagtactc tcctattggt gaaaatgaga gaggatgcag c 101

<210> 585
<211> 101
<212> DNA
<213> Homo sapiens

<400> 585
aagtgtaatt tacaagacag aaaggccaag atactcgaat tgatttaaca mgtacaggca 60

aagtattttt gaagaagtta tttaacccat ttgaaactga t 101

<210> 586
<211> 101
<212> DNA
<213> Homo sapiens

<400> 586
tcccatgttt acacatatat tcattataca ttttatgtac ctattatgat rtgccagtca 60

ccttgttagg ctttgggtat aaaaagaata caaagatgaa a 101

<210> 587
<211> 101
<212> DNA
<213> Homo sapiens

<400> 587
gaattgcaaa aggcatttca aagcaccttc ccacattccc agaaagatgt yttcccctct 60

ttccaaacag ctgagacaga agtacaacgt gtggtccctg c 101

<210> 588
<211> 101
<212> DNA
<213> Homo sapiens

<400> 588
gtcatatatc aattatactt caattaagtt gtaaaaatag ttataaaagc maaaggtatg 60

tctgcactgt tttatatata ttcattttaa tttaaaatgt g 101

<210> 589
<211> 101
<212> DNA
<213> Homo sapiens

<400> 589
cgtcatccct taacagaact gctgcaacag cagtaactga tgttccatgc ycccacccct 60

tatagtgggt taccaaccca gatgccagag ttacgctttt c 101

<210> 590
<211> 101
<212> DNA
<213> Homo sapiens

<400> 590
gaggatatgg actgaagagt agtatttaca cagtaaatgc taccagccag rggaagaaga 60

ggaagatgtg tgtgaacctg agcagtccca cagtcctgtc g 101

<210> 591
<211> 101
<212> DNA
<213> Homo sapiens

<400> 591
gagagctgtt aaagggtttg gagcagagga gggacatgac ccaaccagcc yattaacaag 60

agcacaggct gatgtgttag gactgaactg gagaagacag g 101

<210> 592
<211> 101
<212> DNA
<213> Homo sapiens

<400> 592
ttatttaatg ttgctcttgt atccagcaac cttgctgaat ttttttattt ktaatagttt 60

ggagtagata ctccagtttt acaggtaaat cgtcattttc a 101

<210> 593
<211> 101
<212> DNA
<213> Homo sapiens

<400> 593
ggaaaagctg ttaggaggtg ctgaataata atcacagttg agtcactttc ygacactgct 60

gtcttgcatg atttactgaa tataatcctt caaatgatct t 101

<210> 594
<211> 101
<212> DNA
<213> Homo sapiens

<400> 594
tggccacatg tgcctgttga gcacttgaaa tgtggctagt ccaaattgag ragttgtgct 60

ataagtgtat aatacacact ggacttcaaa gacttatctt t 101

<210> 595
<211> 101
<212> DNA
<213> Homo sapiens

<400> 595
tgcttcaatg ctttctgatt tcatacctgc ataataaaat tcctgattcg yccatcacat 60

tttggcaaac aaccaccgcc acatctctct ggatactggc t 101

<210> 596
<211> 101
<212> DNA
<213> Homo sapiens

<400> 596
gtagcttttg gcaaatcttc tactgcatct caccactgtg ggaaattgca rcttccaagg 60

aaaaggagta gaaactacag gctcaaaaaa atgagatcag t 101

<210> 597
<211> 101
<212> DNA
<213> Homo sapiens

<400> 597
aaaatagaaa tgtattttat attctaaatc ttaagagtca ttaggttgat rtttgcaatt 60

ttttatagtt aatgcaaggc atgttaaaat ataatttgtc t 101

<210> 598
<211> 101
<212> DNA
<213> Homo sapiens

<400> 598
ccggaataga actcaggcta aatgctggtg gtatggaatt gggaacatgt rccaagtaaa 60

gacagaggct tgtttggaag gaatagcaga ggaagatgaa a 101

<210> 599
<211> 101
<212> DNA
<213> Homo sapiens

<400> 599
atgacgtccc catgacacag agaagccaga acccagcacg caccccatgg ycattgcact 60

tcttcccaca gccttcagtt tcaaagaagg aggtgttcct g 101

<210> 600
<211> 101
<212> DNA
<213> Homo sapiens

<400> 600
cattaccaga tattctgtag ttctttattt ctgaaattcc ttaattggaa racaaaacaa 60

tagtaatagc caaaataaaa gttacatgga tatagtttca t 101

<210> 601
<211> 101
<212> DNA
<213> Homo sapiens

<400> 601
ggttaagaag cttaattgca atccctatga ataacaaaag ttgttagaac yacaacatat 60

cattttcctt tctctttagt agcagattga caaaaactgg g 101

<210> 602
<211> 101
<212> DNA
<213> Homo sapiens

<400> 602
atgtatcctg taggcagtag gtcgtgtgga tggtttttaa tgtaaaagtg yggcacgatg 60

acagcattgc tttataatga ttattctggt ggcattattc a 101

<210> 603
<211> 101
<212> DNA
<213> Homo sapiens

<400> 603
catggcaatg tggagaatga attggaaagg aggtgtggag gtcacctagc rgttcaactg 60

aggtaatata aaggtttgaa atcaagcagt gatgagcaag a 101

<210> 604
<211> 101
<212> DNA
<213> Homo sapiens

<400> 604
cttgtatcaa cttgttgttt atgctctcta ctaaatacat cctgtatgtt ycaatccttg 60

tgtcttttct tctctccttt aatttaaata ttacttcttg c 101

<210> 605
<211> 101
<212> DNA
<213> Homo sapiens

<400> 605
tggcccacct gggatcttct aggtctttct atcacaatac tgctttagaa ragtctgtgt 60

gaaggagggg actctggtat ttaactccat ccatcaatgt c 101

<210> 606
<211> 101
<212> DNA
<213> Homo sapiens

<400> 606
atttgaatt gtacaataca tcataattat tggagatagt cactccacta ygcaatagac 60

tccaaaggta ttccatctgt ttacctgaaa ctcttgggcc a 101

<210> 607
<211> 101
<212> DNA
<213> Homo sapiens

<400> 607
ggagaatttt cccttgctcc ggcttcccac tgacggacgt ttcacttaac ygtattaatt 60

cctctgcact attagttacg catgatgcat gacaagcaga t 101

<210> 608
<211> 101
<212> DNA
<213> Homo sapiens

<400> 608
taaatccttc ctactgacca gtgatgaaga cagtgtccat ttctagggta mattgtctgc 60

gattgctgca ctctgataca tgagaaatac atgggaggga g 101

<210> 609
<211> 101
<212> DNA
<213> Homo sapiens

<400> 609
aacagttttc ttaagttact ttttctgtcc ttttagtggc ttcatttaaa ktacagtaaa 60

atctcagaca caaaattatc aaggatttag gaataaaggg a 101

<210> 610
<211> 101
<212> DNA
<213> Homo sapiens

<400> 610
attccagaaa tggtaaaagg tagattcaaa gtgtagcagg ataaaaggaa ragctatttc 60

agggtctctg ttaatgagga catcaaccaa agttttccca g 101

<210> 611
<211> 101
<212> DNA
<213> Homo sapiens

<400> 611
gcattccagg tagaaggcaa gggtcagagt gccccttcct agtttctctc yatccatcat 60

tgggacaaaa tcttccccag acgcctcagt atacttcccc t 101

<210> 612
<211> 101
<212> DNA
<213> Homo sapiens

<400> 612
ctcctttctt tggctatttt tgatatgcct cattttgtat catataaaac ygtggctctt 60

cttctcttac tgcatataac tttaccttct actttataga a 101

<210> 613
<211> 101
<212> DNA
<213> Homo sapiens

<400> 613

gtcttcagga ggtaagaaat agtaggagct tcttgaattt tggaaatcag racacaaaat 60

agaggatacc cctctgcagc agaattttaa ttcaacatca t 101

<210> 614
<211> 101
<212> DNA
<213> Homo sapiens

<400> 614
agttatcact gacccatttt ctatgttatc ctaagcatcc tttgaacgat rtcctctaaa 60

ctcttctcac atattgactt caagctcaat agcctgtgat t 101

<210> 615
<211> 101
<212> DNA
<213> Homo sapiens

<400> 615
ctctttgtac ttttctctcc caaaggagca ttccttgaga agccggagga rttctactga 60

ttacatctcc agcacagcca cattccagcg ggtaggaggg t 101

<210> 616
<211> 101
<212> DNA
<213> Homo sapiens

<400> 616
gaagcagaga taatgacaga gagtgggata ctagagaaac gcccaagacc rtctttagct 60

gcagagttct atcctggatt tcatgtgtga ccttagacaa a 101

<210> 617
<211> 101
<212> DNA
<213> Homo sapiens

<400> 617
taagaggggg cactgctgga tttggtccat gttataggat ttgctgcaca kcccgttact 60

cagaaaatgg ggctgtggta tcagacccgg ctttgaaact g 101

<210> 618
<211> 101
<212> DNA
<213> Homo sapiens

<400> 618
agcatggtta taatagaata agttaagttc caaataggat tacttatttc rtgttgtagc 60

cctaattttg cctcaaccac tcaccctctg gtaaattcct c 101

<210> 619
<211> 101
<212> DNA
<213> Homo sapiens

<400> 619
accatgagta attcagtatt cattcaactt gaataactac agggttagga kagtcatttt 60

gaaaatggtt aggattatta gttagtgtta agaaaatatt t 101

<210> 620
<211> 101
<212> DNA
<213> Homo sapiens

<400> 620
tgaaaagaga aatgcatata gattttttag atgaaagagg ggagcacaca rcatcccaaa 60

ttgtgatatc gtttttgcct aagcaccagg ggttttaggg a 101

<210> 621
<211> 101
<212> DNA
<213> Homo sapiens

<400> 621
gaaacccgag cgaaagacat ttcaaagagg gtttagattt aaagcaaata yctattcact 60

ctaatctgct ttaaaatctg ttgttttcct ggagagactt a 101

<210> 622
<211> 101
<212> DNA
<213> Homo sapiens

<400> 622
gtgagaatgt tgatttttga aaaaatgatc cctcaaatgc ttacagcccc rtgcatgtac 60

aaagatgaaa aatcagtgca attggagaaa aaaacaatgg t 101

<210> 623
<211> 101
<212> DNA
<213> Homo sapiens

<400> 623
taaaggattc taagtcacct ttttccctca ttcaaaatga aaacctctct rtttttattt 60

attttttgag acaaggtgtc tatcacccat gctgcagtac a 101

<210> 624
<211> 101
<212> DNA
<213> Homo sapiens

<400> 624
gacaatttcc ctgatataaa ggaaagatga atttgccaaa tgagcagcaa rtaattttcc 60

agggtaaggt gatggagaat gagccacact gatacaaatc c 101

<210> 625
<211> 101
<212> DNA
<213> Homo sapiens

<400> 625
caggctttac cacattaatt cccagggtat tttcctaaat taacatcaac mttacactta 60

ccattgtttc tttagtttct caaaacttta tcataatgtg a 101

<210> 626
<211> 101
<212> DNA
<213> Homo sapiens

<400> 626
taccattttg tgtctttaca tcttttactc ctggcaaaat gaaataattt mttgatgaat 60

gtattagttt ttgtctttta ataaatatgc tgtaagtgtt g 101

<210> 627
<211> 101
<212> DNA
<213> Homo sapiens

<400> 627
tactcaccat tatctctcta tggaaataat ctgcctatta ttgcctccct rtggaatctg 60

cctctttatg gaaataatcc ccaacataaa gcagcaactc c 101

<210> 628
<211> 101
<212> DNA
<213> Homo sapiens

<400> 628
ctcagtaagt ggcactctca tgttttaaag ttattcaggc cgaaacttca ytctttctat 60

gtctctcact gtgtaaccag tacattagat aatcctactg a 101

<210> 629
<211> 101
<212> DNA
<213> Homo sapiens

<400> 629
ctaattgact gctgctgaag caattaactg attatgtttt cccctcattt raaagtttct 60

gtgatataga caagtaactt tgtgttacaa aagtaatcta g 101

<210> 630
<211> 101
<212> DNA
<213> Homo sapiens

<400> 630
aagctggctt cctcagccat cttgattttg aatactttgc cacttctgaa yagtttagtg 60

tttttctgtt ctatccatat ggtgacatca gctcttagtt c 101

<210> 631
<211> 101
<212> DNA

<213> Homo sapiens

<400> 631
caagacttgc tagacacaag gtccaagctg acatagatac ctgggaggcc raaagcagca 60

acactctcct gcttgggaga ggatggtact tattaaatgg a 101

<210> 632
<211> 101
<212> DNA
<213> Homo sapiens

<400> 632
gctgatttaa ctatgtttct ttttggagca attattttta tgataagtaa magaaaagtt 60

tactcataca gagaaaaatt caagaaggta tgaggcactg a 101

<210> 633
<211> 101
<212> DNA
<213> Homo sapiens

<400> 633
atgattctga atgtgattgg atgagttcct aggaagatgg gtcatacaga yaaaatgatc 60

attgtagaga agatgctatt tcctcctgtg ggaaagaaac a 101

<210> 634
<211> 101
<212> DNA
<213> Homo sapiens

<400> 634
ggatgcatag agttattcta tgtaaattac ccaccagaga gaattcaggc rtgtttcaaa 60

atctataaaa ccgtgtgctg ggggaccatg aaaagttgta g 101

<210> 635
<211> 101
<212> DNA
<213> Homo sapiens

<400> 635
aatttgataa ttaaaatttc attgatgtgt ttgcacttat tctcttaaaa ytgtaacatt 60

taataagtaa aaagttatgc tcattaactc aaacagattt t 101

<210> 636
<211> 101
<212> DNA
<213> Homo sapiens

<400> 636
ctcaggtaaa ttcacctatg tgtgtatggt aagacactgc ttctactctg ytcatcagca 60

aaacacttat tatcattttc ataactttcc tagaatttta g 101

<210> 637
<211> 101

<212> DNA
<213> Homo sapiens

<400> 637
tctcagggtg aaattcagta caacttcatt ttacagtaag gatcttgggg yccgcaggag 60

atttctgtg agaaaattgt aagagagggc ccctgagaag g 101

<210> 638
<211> 101
<212> DNA
<213> Homo sapiens

<400> 638
tcataatggg actgcagaac cagaagcaaa agagtaaaat gcttattttc rtacaacatt 60

gagttttggg gtccttggtt tgtaacatta ttgcagtaaa a 101

<210> 639
<211> 101
<212> DNA
<213> Homo sapiens

<400> 639
aaaaccatat gccattgtat ctgaaatgtt ggccccccttc aagactctca mccaagaaat 60

tgcaccataa tttacctcat tgttgaagcc aagaaaatgg a 101

<210> 640
<211> 101
<212> DNA
<213> Homo sapiens

<400> 640
gcatcatctt ccataggcac agtgatcatt gccagccagt ggcacttcta rgtgaggagg 60

ctcttaggcg aggcccccag gatttgccct gtaggaaccg c 101

<210> 641
<211> 101
<212> DNA
<213> Homo sapiens

<400> 641
acacattaaa tcaccacttc tagggaaagg ttgagctcac tcatagctct rttgatagtg 60

acactgagag ggtattaaat gttgaaaggt ctaaaaggga g 101

<210> 642
<211> 101
<212> DNA
<213> Homo sapiens

<400> 642
gcatctggat gaatagatct acgatgacca tattgccttc actgtacatg rcctaaactc 60

atctctctgg aaagttaatc tttcataaca ttaacatcag t 101

<210> 643

<211> 101
<212> DNA
<213> Homo sapiens

<400> 643
ttctcttctg ttgtttctac ccgtgttctt ctccgggata ttatcagaaa rtaaacacac 60

caaaggaaat aaacaaaata tgcatttcca atatattttc c 101

<210> 644
<211> 101
<212> DNA
<213> Homo sapiens

<400> 644
atggaatttg cacattatat atgttattta tggaatacag atcattcatt kaggcatttt 60

tctagattgt ctttgagctt ccctgaccaa cttgcagttt a 101

<210> 645
<211> 101
<212> DNA
<213> Homo sapiens

<400> 645
ctgaacttaa acattataga cacacgctat gtctataatt tttgacatta yagacatgaa 60

ggtccttaat gggctagtgg gcaaaagcca tctaggaatc a 101

<210> 646
<211> 101
<212> DNA
<213> Homo sapiens

<400> 646
gttacctgat cggctgatcc gggagttgaa ctgtaatcag gggcttgtag kagttagagc 60

tgtgtgggcc tctgaggagc tcccagcctc ccaggagcgg c 101

<210> 647
<211> 101
<212> DNA
<213> Homo sapiens

<400> 647
tcactgccgt taagttgtag agttgctcta ggtccctgca ttcggctgtc rtatttcact 60

gaacttactt tgaagttgct tatgtcactc tcaccattgc c 101

<210> 648
<211> 101
<212> DNA
<213> Homo sapiens

<400> 648
gtatttttgt ttttttttta agttttcaga actttaagat ggtgtgtaga yagatgcttt 60

tatgggccaa gaaagcatgt tgatatccat tattttattt g 101

<210> 649
<211> 101
<212> DNA
<213> Homo sapiens

<400> 649
ggtgctactg cttccagaga cagcaaggta aaagatgaga cccttacaga ygcaaatagt 60

tgacctgcat gtcaaatttt acttattttt taagaaaata a 101

<210> 650
<211> 101
<212> DNA
<213> Homo sapiens

<400> 650
attcatgtct aagcatttcg tagaaggatg cacgtgagaa aaagcacctg ygctgtcata 60

gcgatccttt ggtgttttaa gatgaaaaag ttcaaagcat t 101

<210> 651
<211> 101
<212> DNA
<213> Homo sapiens

<400> 651
aaacttccat taggaagtat gtgaaagaaa ctttccttta aataaaaatg ygtaagtgtt 60

tagaattgcc cttgcaaagc tctaaatcaa tcacccaggg c 101

<210> 652
<211> 101
<212> DNA
<213> Homo sapiens

<400> 652
tagtcacctc ctttgaacag ctttctagta acaggtccct ggatccatgg ygcttatttt 60

tagaagagac agtagtatat tattttgagg tcatggaatt a 101

<210> 653
<211> 101
<212> DNA
<213> Homo sapiens

<400> 653
tatgcttgtt cccaatctcc ttgggagaaa gcagtgtcaa tcttttacca ycaagtataa 60

ttttagctat agattttgta caaataactt ttatgagtct a 101

<210> 654
<211> 101
<212> DNA
<213> Homo sapiens

<400> 654
gcatgtagat gcaagacata gcatttaaga atatcaatgt gtgtgcctac yatgccttac 60

tagctaaata ttctactgtt gtataacagg atgatttggt t 101

<210> 655
<211> 101
<212> DNA
<213> Homo sapiens

<400> 655
tgtcccccaa ccatctgtag acattcccaa aagcctccat cgcatatgct ygtgcaccca 60

cttgtcagaa gcatacccat gctgcaccgc cccggatttg c 101

<210> 656
<211> 101
<212> DNA
<213> Homo sapiens

<400> 656
aataccaagg agagcagagc tgtgctgtca agcccctgac aattcgtgaa yttctgctgc 60

tgaaattatt agtgctgcct tggatcaagt tccatttgta t 101

<210> 657
<211> 101
<212> DNA
<213> Homo sapiens

<400> 657
tttttaatat caattggaat tgccgcaaca cccaacactg acacacagtt yccagagcaa 60

agctccgtgg tcagactccc aagctcctta gtagtggtgg c 101

<210> 658
<211> 101
<212> DNA
<213> Homo sapiens

<400> 658
gttcaataca tctcaatgag aagcatgcaa ccttaatcca tgacgcttgt ragtggagct 60

atttttcaat ctacgttaat tttgaattta actgtgtcaa g 101

<210> 659
<211> 101
<212> DNA
<213> Homo sapiens

<400> 659
acaaaattct tgaaggtcaa tatgggatag cctcaagcct cggacacaaa rgagtttgta 60

ttcacactca agcttttctt tagggcccct aactgggtgc t 101

<210> 660
<211> 101
<212> DNA
<213> Homo sapiens

<400> 660
ctggattcaa ttctttcttt gtttccatat ccaatcctcc atggatcatt mtttttcctt 60

agcacttctg atgatgtttc ccaggataca tccttagcct c 101

<210> 661
<211> 101
<212> DNA
<213> Homo sapiens

<400> 661
taaacaagaa tcacttttcc cgtaatctta ctacgaaaaa tggtattaat ygatatttgt 60

acactaagat atggctaaaa agccaggtac ctaagcccat g 101

<210> 662
<211> 101
<212> DNA
<213> Homo sapiens

<400> 662
ttatgcttct ttacaacttg tgcaactatt acctaagata aagccctgaa rgaaaagaaa 60

ctgtagtctg agtgactgtg agaaatcata aatgacagtc c 101

<210> 663
<211> 101
<212> DNA
<213> Homo sapiens

<400> 663
cccacacttc tcccatatct gtaacctctc catctctttt gttctgtcta ytggcatata 60

aacagattaa aatttctccc accctaaaaa ttaagaataa g 101

<210> 664
<211> 101
<212> DNA
<213> Homo sapiens

<400> 664
gcatataaac agattaaaat ttctcccacc ctaaaaatta agaataagaa ytctgtcaaa 60

tcaataacca ccctgacttt ctcctcttca caacccaaaa t 101

<210> 665
<211> 101
<212> DNA
<213> Homo sapiens

<400> 665
aatacatcac atccatttta tccatatcac ttttcctggg tttggctacc rgcgcagatt 60

aatagttgtc tttgcattat gcagtggaac ttaatttcta t 101

<210> 666
<211> 101
<212> DNA
<213> Homo sapiens

<400> 666
atcagaacaa gattctgaat gaaaacgtgt tcccccaggt gagccatatg yagacgaatg 60

cttgggatgc tgggtagatg ttgaaaaaaa gttttgcccg a 101

<210> 667
<211> 101
<212> DNA
<213> Homo sapiens

<400> 667
gctcaggttt gcttcttaaa cacagatttg aatacattac tgtaaatctc ygttttgctt 60

ttaggtcaaa tagaaatggt catggaatga cagcccagat g 101

<210> 668
<211> 101
<212> DNA
<213> Homo sapiens

<400> 668
gaatcaatca catccttgtt gcctcccttt tcttcaaccc catgttcaat yagtcgctga 60

gctgctggta aatccctagg agaaggagag tgatgtgtct c 101

<210> 669
<211> 101
<212> DNA
<213> Homo sapiens

<400> 669
caaccctttc aaaaaatctc tgggagttga accaggattg atcttgtggc raagaatctt 60

catcggctgc taggacagcc attcagtctc actttcccat t 101

<210> 670
<211> 101
<212> DNA
<213> Homo sapiens

<400> 670
ttctaccaag ctcctaggtg atgatgttgg ggattcatgg accacgcttt ragaggcaag 60

gataaagaaa actactgtat acgaattagg gccacgatgt g 101

<210> 671
<211> 101
<212> DNA
<213> Homo sapiens

<400> 671
aataaggaag cccatttatt ttatcattat tacttttatc actaataaca rgctctttac 60

acctacacat gagaatgaca atagcaaagg aaacaatcat t 101

<210> 672
<211> 101
<212> DNA
<213> Homo sapiens

<400> 672
gaaaaagtat taatacttcc tcagggtaac ctccttcagc actatcagca rttacaatga 60

gattgaatac taattaacct ttaaatatag gctttggggc t 101

<210> 673
<211> 101
<212> DNA
<213> Homo sapiens

<400> 673
tgtatcattc tatggtaaga ctacgtttag ctttgcaaga aactgtcaaa ytgtcattca 60

acgtggctgt gtcatgttac attccctaca atgattggga g 101

<210> 674
<211> 101
<212> DNA
<213> Homo sapiens

<400> 674
ccatcttgct gatttccagg ttgcttcggg gaccccaaga gaattcatat kctggtggat 60

tggtgtgagg cacccgcctg taactgagat atcgctgctg c 101

<210> 675
<211> 101
<212> DNA
<213> Homo sapiens

<400> 675
accgcaaaat gtaccttgtt gggtatttag cagaaggaaa tgtgttgact rttacacatc 60

ccttatctac agtgcttgag actgttttga atttcttatt c 101

<210> 676
<211> 101
<212> DNA
<213> Homo sapiens

<400> 676
gttgaatgat ttcattttac atagattgcc ttttatgatt tttatgattt yttcaacttt 60

cattttaggt tcagggttac gtgtgtaggt ttgttatata g 101

<210> 677
<211> 101
<212> DNA
<213> Homo sapiens

<400> 677
cctaggcgaa taaacaaagg aatgatttct ccacttggat ggacatacca rttgtagcct 60

gttggtctgt ttctcaccct acttatcaga gtaacctctc c 101

<210> 678
<211> 101
<212> DNA
<213> Homo sapiens

<400> 678

ttggcttaga ttatttttta agtttcatat tgtgccacca cgggcgggtc ytctccatac 60

agcagtgact gtaaaatcaa accccacttt cagtgagtga g 101

<210> 679
<211> 101
<212> DNA
<213> Homo sapiens

<400> 679
cctgaaaatc agtttcttcc cttcgattga caaccaagga ggaagtcagt kggaagacct 60

ggggcattca taaagggaca agaatctttt tctcattaag t 101

<210> 680
<211> 101
<212> DNA
<213> Homo sapiens

<400> 680
acctttgtga tgctttatct cccaactgac actgaactac atactaaata ygtattgcta 60

ctatgttctc ctaagctttt ttatacatgc tactttcttt a 101

<210> 681
<211> 101
<212> DNA
<213> Homo sapiens

<400> 681
actggccctg cagcactgag acactcagga gcccatgatc ctccaccagc ygtgaagcag 60

cagagaaact catggtccga aaccgcaacc aaagcctcca g 101

<210> 682
<211> 101
<212> DNA
<213> Homo sapiens

<400> 682
aatactttta ttaatataca ggaatccccc cttacctgca gggcatccaa ractcccgag 60

tgaatgccta aaaccacaga tagtaccaag ccctacacat a 101

<210> 683
<211> 101
<212> DNA
<213> Homo sapiens

<400> 683
agtgttggca gatgtcaaat aactgcattt attcaaccag aactgatcat yatttagagt 60

gaaatgatca attattggag taaaatgcat tttgtttgca a 101

<210> 684
<211> 101
<212> DNA
<213> Homo sapiens

<400> 684
gcctgggttc aaatttggac tctgccattt ccttatctgt gacttggaga rctcatttaa 60

acttctcaat tcttccattc cctcatctat aatggaaatg t 101

<210> 685
<211> 101
<212> DNA
<213> Homo sapiens

<400> 685
tggtttctct ctagttaaaa aggaatgttc aaaataactc aagaggttcg ytttctggca 60

atttgcctct ctagcaattc agaatttcct tgtagttttt t 101

<210> 686
<211> 101
<212> DNA
<213> Homo sapiens

<400> 686
gtttttcctt aagaatggtg aagttgtttt ttttttttaa aaaaaggaaa ygcatatgag 60

ttctggatag tttgaatact tggaaaaatt attgtcctgg a 101

<210> 687
<211> 101
<212> DNA
<213> Homo sapiens

<400> 687
aaaccatcag aaaaaaaaaa ctatattccc ctttccactc tttatcataa rtataacttc 60

aattaaagga aataactttg atttatagtt agaccacaac a 101

<210> 688
<211> 101
<212> DNA
<213> Homo sapiens

<400> 688
cagttcacaa cccataccca cagagaaaca tacacatata ccttatatta yattggttct 60

tttttttcct gaaacaaaag gtctcacata tttattactg a 101

<210> 689
<211> 101
<212> DNA
<213> Homo sapiens

<400> 689
ggaagtcaaa agttataagc caagtttcaa ccgcttgcaa atgtacccct raaccccatg 60

ttgtacaagg gtcaactgta ctgttactgt cccctgttac a 101

<210> 690
<211> 101
<212> DNA
<213> Homo sapiens

<400> 690
caaacctagg aggcaatatt gcccagctgt aaggagcatg ggctttagaa yctctggttg 60

ctcttgttaa tggtgcgact ttaggcatgt tatttaacct c 101

<210> 691
<211> 101
<212> DNA
<213> Homo sapiens

<400> 691
ttggagttag tgtcagtagt gttgaatcat tcaggactgg atattaagta ygtaagggca 60

atagaagagc ctggagcata tttcatatcc ctctatccct c 101

<210> 692
<211> 101
<212> DNA
<213> Homo sapiens

<400> 692
cagcataatg cttggtattt gacatgttat caagtatgaa taggggagta kcaagggata 60

tgaaaggggt cagaccaaaa agggattcat tttataccta g 101

<210> 693
<211> 101
<212> DNA
<213> Homo sapiens

<400> 693
ttacaccctt cacagaattg cttgagggca caagtacaaa gaattaatat rttaattatc 60

ataagtgaat cattaaacag caacagtaat taacagctta a 101

<210> 694
<211> 101
<212> DNA
<213> Homo sapiens

<400> 694
aagcacttta ggtttttcag ataacataat cagagaggca agagtatatt rtatttgctt 60

ttctgcctct tgtctgggct taaaatattt cacttggagt g 101

<210> 695
<211> 101
<212> DNA
<213> Homo sapiens

<400> 695
tctgatcgtc tagttccaat atattctctg cctcttcctt gatagcttaa rtcctgaatt 60

ctgttcttaa atactgttgc agcttaagct gtcctgcctg a 101

<210> 696
<211> 101
<212> DNA

<213> Homo sapiens

<400> 696
gtggaaagta tagggactaa gccaaaccag gagaaagtgt caactccagt yaagatccag 60

cagaaccctc tggattggat aagggaccca gaataatcca t 101

<210> 697
<211> 101
<212> DNA
<213> Homo sapiens

<400> 697
ccaaagcagt ttatctgtgt accccaagac tgcaaataaa tttatagaac rgtgttgcct 60

ggtagaattt tctataatga tagaaatgtt ttatgatctg t 101

<210> 698
<211> 101
<212> DNA
<213> Homo sapiens

<400> 698
aaagcacagc ttaacaagta ctctgacacc cagaaaaggc ctacataaac ycagtaggaa 60

agaaacctaa aatagcagaa gtgctggatg agagtaagga a 101

<210> 699
<211> 101
<212> DNA
<213> Homo sapiens

<400> 699
caatctcaac aaacattgga agaaaactgt tcaaagccac tggctcatag mctgctatct 60

ctatgaggat gtttaggatg atgtcattat gggttgaatc c 101

<210> 700
<211> 101
<212> DNA
<213> Homo sapiens

<400> 700
tatttaattt ggggctcaga agggctgaaa actgcattcc atgaataaga raactggaaa 60

taatcaaaga actatatgga ctgcagcatc tctctgccat c 101

<210> 701
<211> 101
<212> DNA
<213> Homo sapiens

<400> 701
acagatgcaa gtaaaaaaat taaaaagtat tacggaacca caatatttat ragggacagt 60

cctaagaatc ccatgatttc ccagattgat aagggaacag t 101

<210> 702
<211> 101

<212> DNA
<213> Homo sapiens

<400> 702
ggataaggga gaatgtatat acaccaccaa aaaggagaga gtcacaccga raagtcagtt 60

ttgagatcag tttagagaaa atgcaggcca aggcagtgtc a 101

<210> 703
<211> 101
<212> DNA
<213> Homo sapiens

<400> 703
cccttccctt caagcaaaac tcttgtgatt cccctacact attttatggc kccatgtgct 60

tgtatattct gatccctctc cccaaatgcc ctatcctgac t 101

<210> 704
<211> 75
<212> DNA
<213> Homo sapiens

<400> 704
accaataatt tgattttgtt gatayatcca gatttgacca tttcaaggaa gtaattcgtg 60

tttatttaaa ttctc 75

<210> 705
<211> 101
<212> DNA
<213> Homo sapiens

<400> 705
taagtatttc tatatgctac tattttttct tagattaagg tcctgaggat mtccaacttt 60

tgggttttag agaggtaacg tgttgccttt aacctctatt a 101

<210> 706
<211> 101
<212> DNA
<213> Homo sapiens

<400> 706
ccagccccac cttcctcttc tttgaatcct gcccctccct tgctccagac ytcaccaagt 60

ctctgcatta cagttcacat caaccctaag ttgctctttc c 101

<210> 707
<211> 101
<212> DNA
<213> Homo sapiens

<400> 707
gataggaaca aaaatggaat ggtattcatc tacatattat ttgggcctct ktacttttta 60

tgttgtaaat gaaggagata atttattctt accacatact g 101

<210> 708

<211> 101
<212> DNA
<213> Homo sapiens

<400> 708
agctacaaca ggaaaaatgt gtggacatga agggaacttg tgagtaggtg ytgttgagta 60

catgcctgtg tgtgtatatg tgctagggac acctaccagg g 101

<210> 709
<211> 101
<212> DNA
<213> Homo sapiens

<400> 709
tacattttac tcttgtacca gtatcacagg ttttgaatcc aagaaatgtg rgtctatcta 60

cattgttctt tttctaatta ttctgacgat tttgtgtcct t 101

<210> 710
<211> 101
<212> DNA
<213> Homo sapiens

<400> 710
ccaaggatgt tcccatcaaa tccttccctc atttgatttt cacaacctgc raggaaggca 60

aggcaactgg catccatatg gacatggaaa ccgagggcca g 101

<210> 711
<211> 101
<212> DNA
<213> Homo sapiens

<400> 711
atctgattaa ttcagattag tttatggatt agttcctctg gggttggata rcttctcttg 60

gctcaatcag ccatgtcagg ggaatgacat tgctaatgaa g 101

<210> 712
<211> 101
<212> DNA
<213> Homo sapiens

<400> 712
aagtagggtc tgtatggcaa ggacattacc tatcttgttt accatgaaat ygccagtgcc 60

tagtggatca ccacctagta cacgctcaat aaacactagg t 101

<210> 713
<211> 101
<212> DNA
<213> Homo sapiens

<400> 713
acacgaaact gttacccatg ccttttcatt ttccccttca ttatcctctg yaccttacat 60

ttctaaatgg aaacccttca atgactacct acttaactct c 101

<210> 714
<211> 101
<212> DNA
<213> Homo sapiens

<400> 714
gatgatgtgc ttacattttt ctgcaaccga tcttctgaca ttttctcgtt yccccagcca 60

cgagattgta atttaacctc aactttttgt gtgtgtgcaa g 101

<210> 715
<211> 101
<212> DNA
<213> Homo sapiens

<400> 715
cctggctgag ctctgcccgc ctggaggctc ccacaggatg gccctgggga ytgctgctgc 60

actcggtagg tgcccttggc cagggtcttc ctgatgggct c 101

<210> 716
<211> 101
<212> DNA
<213> Homo sapiens

<400> 716
tggcacacac aggaagcttg catctgacaa caggaaggct ggaacgccac ktggatttgc 60

tcaaggaggg tacaagcatc tcctgctcat tgtctccttt g 101

<210> 717
<211> 101
<212> DNA
<213> Homo sapiens

<400> 717
aaaggatttt ccccacattt atagctctga agttgagctt tttatcacct ygctttttgg 60

ctcccaagtc ttgctgctgg gtagaattac ctggaaagct g 101

<210> 718
<211> 101
<212> DNA
<213> Homo sapiens

<400> 718
tgagtattta gattctcaag atgactattt caaaggacag tagttccttg yatgcactaa 60

aaataccccg aaacatgaat acttcttttt taaaatgaat c 101

<210> 719
<211> 101
<212> DNA
<213> Homo sapiens

<400> 719
tgagtgtctt tgacagtaac tccttcatag atgctttctt atgatgtacc mtttaatttt 60

gatgaaggtc ctgtgaaata agcagagcag attttatgat c 101

<210> 720
<211> 101
<212> DNA
<213> Homo sapiens

<400> 720
gttttggaaa tgttgttgca ttgtcacttt ctgcagtaga aactgaaaaa ygagaaacac 60

actgtgtttg actggaagcc caaaggagac aaaatgtttt c 101

<210> 721
<211> 101
<212> DNA
<213> Homo sapiens

<400> 721
caaaatagca tataatctag tttggttgac cctttgcttt ccacaggcac rgaatgggaa 60

ataaggatgg aaatgagaat tggggatgta ttgcagagga a 101

<210> 722
<211> 101
<212> DNA
<213> Homo sapiens

<400> 722
cagagcagga aagtgagctc ctcagcagag accaggctgg gatgaggaca mcgcggtgca 60

gaagaaaatc tgcctggccg tggtgcctaa agctgccatg c 101

<210> 723
<211> 101
<212> DNA
<213> Homo sapiens

<400> 723
cacgatatag gaagaccaac caattcttga aaagcttttt tcttttccca rttgcttcag 60

tgatagccac acatttcaat aaacccaatt ttcctccatc t 101

<210> 724
<211> 101
<212> DNA
<213> Homo sapiens

<400> 724
tctgggccat aagatatacc ttaacagatt taaacaagta gaaatgatac raagtgtgct 60

ctaataatgc cataatggag ctaaatgaga aatgtaaaaa a 101

<210> 725
<211> 101
<212> DNA
<213> Homo sapiens

<400> 725
cctggtccct ggaggaacag tagcctctgt ctgagtccta aactggggca rcaggccggg 60

cacaatgtct caagcttgta atcctagcac tttgaggcac c 101

<210> 726
<211> 101
<212> DNA
<213> Homo sapiens

<400> 726
gaaataggat ttcctcaata aggacaaaat ggctcagggc caaaatgaaa rcatcactca 60

gcactttttt ttttttttta cttttatagt caatgcaaag a 101

<210> 727
<211> 101
<212> DNA
<213> Homo sapiens

<400> 727
gtctggtgtc cgagcagcgt gtggtcctgg gaacatctta catgaagtga rgtgtccatc 60

cttgggtggg tccctctgac tcaaggcgag tcttgtggag g 101

<210> 728
<211> 101
<212> DNA
<213> Homo sapiens

<400> 728
gtaaaaaaaa ctgaaggtag taaatgtggt cgttcagaga aattcagagt raaatgaagg 60

agaatgaggg acaggatggc aatactaata gataagggag c 101

<210> 729
<211> 101
<212> DNA
<213> Homo sapiens

<400> 729
tacttctagg tatacttcta ggtaaaactc cccaagaaac actcatatat rtgcacaagg 60

aaacaaacat aagtatgttc catgaagtac tatttgcgac a 101

<210> 730
<211> 101
<212> DNA
<213> Homo sapiens

<400> 730
actgaagact ccaagctata tggactgaat ccaccccaa ttcccccgcc yaattcatac 60

actgaagccc tagacccagt gtgactgtac tggagacaga g 101

<210> 731
<211> 101
<212> DNA
<213> Homo sapiens

<400> 731
tacttacccc cttcagataa acagaaaatg caactctatg taaatattcc ytaagaatat 60

tttgcagcac actggaatta aattagtgct aaagatgatg a 101

<210> 732
<211> 101
<212> DNA
<213> Homo sapiens

<400> 732
gttccactta cacaaacgtc cacaacacat aaatctagaa acagaaacta ygttagtggc 60

tgcctagggt ttaggatgag gagggtagat gtgaagaatg a 101

<210> 733
<211> 101
<212> DNA
<213> Homo sapiens

<400> 733
atgtggtgat gattaacctt gtcaacttat tttttaaata atcctcatcg yttataccat 60

tgtagtaaag ggttcccctc tcccatgcag caagtccaga a 101

<210> 734
<211> 101
<212> DNA
<213> Homo sapiens

<400> 734
gaggaaccac ccctctccct ctctctgcca atctgtattg gggcaaggtt kggaagtact 60

ggcgagggta ttacatttca agaaacatga ccagggaagc c 101

<210> 735
<211> 101
<212> DNA
<213> Homo sapiens

<400> 735
aagtcaaaag actagataga gaaatgatgt ccagggagct cataatctgc ytgtgcaaga 60

attctagttt ctagaaagtc actgattaat aaattcatgt g 101

<210> 736
<211> 101
<212> DNA
<213> Homo sapiens

<400> 736
ctacacaaag ccctcttcaa cagatagcat aaacgctacc ctgtaaaatc rccagcaagc 60

ctttgtctcc ttgcagtcag tttctctctg ctgcctgcct a 101

<210> 737
<211> 101
<212> DNA
<213> Homo sapiens

<400> 737
tattgttttc tctttaatgg tgaaacttga tagggaacct aaaaagaatt ktaagactgc 60

attcacttaa tttgaagctt aactagaaat ttgtttgctg t 101

<210> 738
<211> 101
<212> DNA
<213> Homo sapiens

<400> 738
ccactctact gcttgggagt aagcggccac caaaaccccg cttccagcag rtgctaggag 60

caacatgaca ggaaaaacac aacctaatta aaatggtaga g 101

<210> 739
<211> 101
<212> DNA
<213> Homo sapiens

<400> 739
ctccatcctc atctgtctgg tcgctgtctc cacttctctc ttcagatatc rgttcaggcc 60

cagctgcaat agatacctgc atgactccac ccaaggacaa a 101

<210> 740
<211> 101
<212> DNA
<213> Homo sapiens

<400> 740
gatgacttac tttgctgcca aagggctggg cctgggcctg ggcctctgag ycaggttctc 60

catcctcatc tgtctggtcg ctgtctccac ttctctcttc a 101

<210> 741
<211> 101
<212> DNA
<213> Homo sapiens

<400> 741
acttctaaat taccaccatc caggttgcat ctatttatgg ttccattccc ygaactgatc 60

caataaagct tgttttccac atagtctatc gatagacctg t 101

<210> 742
<211> 101
<212> DNA
<213> Homo sapiens

<400> 742
tcacagtaac ccccagtcct caaaacatca acaataaaca cagacctgca ytgattgtgg 60

tattctgggt atttctataa catttctagg tttctgtaga t 101

<210> 743
<211> 101
<212> DNA
<213> Homo sapiens

<400> 743

atcttggttt ttctgccttg acctttggct ctttctaatg taattggctc mgactccatt 60

tctggccatc tgaactctgg ttccaagaat taatccaggt g 101

<210> 744
<211> 101
<212> DNA
<213> Homo sapiens

<400> 744
atctctcctt aattattaca gaaaaaaatg ttattaaaga aacaatcagg kgatccagca 60

aaagctgaca atgcacagta gtttagaaac cataagatgc a 101

<210> 745
<211> 101
<212> DNA
<213> Homo sapiens

<400> 745
gaggttatta gcatcccctt ttacagaaga aaaaactgag aaaccaagca yatacagctg 60

gtaagtaacg tagtctgggt gcaaaaccac gaagctcatg a 101

<210> 746
<211> 101
<212> DNA
<213> Homo sapiens

<400> 746
cttctgcttt caaaaggaat tgaagaaacc ctaagataaa agagacaaga yacactcaag 60

ccattcaaag aacaaggacg gcacagaaag tacaggttat a 101

<210> 747
<211> 101
<212> DNA
<213> Homo sapiens

<400> 747
tctgggaaca gactacttgc tgaaacgaac aaattcccag gcagttgaaa rcctttgtgt 60

ttcctactgg gaataacctg cattcacaaa ttcattagcc t 101

<210> 748
<211> 101
<212> DNA
<213> Homo sapiens

<400> 748
tctgcccact gtttctctcc ttctgctgca gatctttgag ctgaataagc rtatttcagc 60

tgtggaatgc ctgctgacct acctggagaa cacagttgtg c 101

<210> 749
<211> 101
<212> DNA
<213> Homo sapiens

<400> 749
tctcagtttt ggagaccaaa agttggctgt tttggtgggc tgaaatagag ytgtgggaag 60

ggccccactc cagatggagg ctctggggga gaatcctttt t 101

<210> 750
<211> 101
<212> DNA
<213> Homo sapiens

<400> 750
tatatatgtc aagcaatacc ttagtaaggt actcacttat tttatcccta rtggcatatt 60

aatcaggcaa tgtcatagat ctctggttac tattccacct c 101

<210> 751
<211> 101
<212> DNA
<213> Homo sapiens

<400> 751
cactagttat tggcggtggt gaattcagtt tacatggctc tgaattcata rcaagtttat 60

ttctttagga aaatgcaaat agttattgtg gttggcagaa t 101

<210> 752
<211> 101
<212> DNA
<213> Homo sapiens

<400> 752
tctgaagggc taagcaaggg taagttgttt atgctgttgc aggaaccaca rtgatgggaa 60

agaaaaatga tatggtattt ccatcccggg ccttaaaata a 101

<210> 753
<211> 101
<212> DNA
<213> Homo sapiens

<400> 753
aaatgttgac tatatacctg cttgataata agaaacattc acctctcttc rtttaagttc 60

aacttaaaga agaaacattt ttgaaaagtg agaagtgtgt t 101

<210> 754
<211> 101
<212> DNA
<213> Homo sapiens

<400> 754
caagatagcc ttctttagaa tatgatttgg ctagaaagat tcttaaatat rtggaatatg 60

attattctta gctggaatat tttctctact tcctgtctgc a 101

<210> 755
<211> 101
<212> DNA
<213> Homo sapiens

<400> 755
gctttataac tgagatgtgt acttcaggct tgcatgggaa ttgtctgtac rgcccacaaa 60

ctggcccccca ggtctttggg actccttcct gtaacttagt g 101

<210> 756
<211> 101
<212> DNA
<213> Homo sapiens

<400> 756
ttattatctc tgaatcacag atgagtaaac tgaggcacag aggtttttg ktttttttt 60

cccttaagga cagaaaacag catattcaaa ccgaggcatg t 101

<210> 757
<211> 102
<212> DNA
<213> Homo sapiens

<400> 757
aatcacaggt ttttatcaat aaatgtccag ctgggtacat tcctccctct mtctaaacac 60

aactcctgcc ggtcaggcac tgtgtcctag aacctttgcc at 102

<210> 758
<211> 101
<212> DNA
<213> Homo sapiens

<400> 758
cactttgctg ctgctctttc tgcctctgtg accactcctt ataggttcct yttcttcttg 60

tgcctgcccc tttaatgctg atattgatgt tttctcccaa g 101

<210> 759
<211> 101
<212> DNA
<213> Homo sapiens

<400> 759
cacaaaagaa atgtttcctc tcacagttgg tgaagctaga tgtctaaaaa ycaaggtatc 60

agtagggcca tgctcccact gaaggctgta gggaagattc c 101

<210> 760
<211> 101
<212> DNA
<213> Homo sapiens

<400> 760
ccttgtactt ctccttggtg tcatgaagac aaatagcatt aaaaaaagtt ytcccagtga 60

agcagctctc attttctcct ctctcatccc cttccaaaca t 101

<210> 761
<211> 79
<212> DNA

<213> Homo sapiens

<400> 761
gagcgtagct ttctagagtg tgcgagtggt ggctagatgt gctttgtttc rtgtgctgtg 60

catttcagtg ctagtgtga 79

<210> 762
<211> 101
<212> DNA
<213> Homo sapiens

<400> 762
ttccctcatt gccaatcacc ccatttagtt atgaaaatac ttcattggta rtagtggcca 60

aacaggcaaa tatctattca gtaattagat gaataaatgg g 101

<210> 763
<211> 101
<212> DNA
<213> Homo sapiens

<400> 763
aaaaacaaca aaaatacaaa attttcatga tgatataata ggaagctctc raaggttgga 60

ttcaggtaag gaaatggggg aaagtttcct gataccctga c 101

<210> 764
<211> 101
<212> DNA
<213> Homo sapiens

<400> 764
cagcaggagt ggactgaata gcgtgcccct gggaggtttg tcttcctaag yagatccaat 60

cggtcttctt gttctgatga agtaaaacag agtggatatc c 101

<210> 765
<211> 101
<212> DNA
<213> Homo sapiens

<400> 765
aaacaaactg ttctaaattc aaggagtctc tgccagttat gtgactttgc rtgactgact 60

ctgctttacc cctccaggcc caagagacaa ggctgtccag a 101

<210> 766
<211> 101
<212> DNA
<213> Homo sapiens

<400> 766
taatctccca gaggtgtttc cttttgttac tctccaaaat gaaaagtcta ytttttttctt 60

atcaaagcca tacatgcttc ctgtaaaatc aactcagata a 101

<210> 767
<211> 101

<212> DNA
<213> Homo sapiens

<400> 767
tcacagggaa tggggtttct tttatcactg acgatagcaa gacctacttt yttgctctgg 60

acagctccta tgaaaatatg gcattcagaa ctgcttccct g 101

<210> 768
<211> 101
<212> DNA
<213> Homo sapiens

<400> 768
gaaaggatga taaatcttag gaataatacc aatggcatta atgtaatccc rcgtaagttt 60

cgaaaaacct ttccaagtat aaattcagta agaaaagctg g 101

<210> 769
<211> 101
<212> DNA
<213> Homo sapiens

<400> 769
tgccgttctt ggcatcattt ctatttggct gtgagtcgtc cgcttgatgc rtggtccaca 60

gctgattttc atgccccaaa caatccccat cgaaggtcac a 101

<210> 770
<211> 101
<212> DNA
<213> Homo sapiens

<400> 770
caatggttaa gaattaattt ctatgtgttt tgttatccgt taaacacagg ytgtgagcta 60

gcaagaaaca agatactttt ggaggcttag tgactttttt t 101

<210> 771
<211> 101
<212> DNA
<213> Homo sapiens

<400> 771
aacagaggac attctgtttt ggagccatgt tcccctgtcc ctggaatacc ycgctactta 60

ttagaaaagc agaaatgcaa aaaatcacag acatgtgggg g 101

<210> 772
<211> 101
<212> DNA
<213> Homo sapiens

<400> 772
tctttctggg ctaacaccaa gggggtggca gggctgtctg tgttcctgct rgtggttata 60

agggagaaat tccttccttg ctttttccag atcctagagg c 101

<210> 773

<211> 101
<212> DNA
<213> Homo sapiens

<400> 773
gaaaagcttc ctagagaagg ggcagctgga acctgaagaa caaaaccaga rctgacgacg 60

acggatgagg caggtgtttc aggtggcaga gcaacacagg c 101

<210> 774
<211> 101
<212> DNA
<213> Homo sapiens

<400> 774
caatttctcc atttttaaaa ttggtaagtc ccccagccca aggatatggt ragtgattgt 60

gtgacctcca gaaaccacac ttctcccatg gatctttgca g 101

<210> 775
<211> 101
<212> DNA
<213> Homo sapiens

<400> 775
cttcctcttt tcctttgttc tctattgcct ttacctattt taaaaagttt yaaattatta 60

gccagtcggg ttttagttta aattgtaagg tctagctcca g 101

<210> 776
<211> 101
<212> DNA
<213> Homo sapiens

<400> 776
ataggtgaga gggatctaga ttacgaaagg cctctgaagc cagggagaaa ytgaacttaa 60

tatgacaggt agtgaggagt cagtgtgagt tcctcctggg c 101

<210> 777
<211> 101
<212> DNA
<213> Homo sapiens

<400> 777
cttttccatt tccattttta cttcctctcc tacagtctct tttaaatcca yaaccaatta 60

ggttttcatt ccaccaaagc tgctcattaa aatcccttac t 101

<210> 778
<211> 101
<212> DNA
<213> Homo sapiens

<400> 778
aaggcattta ggtcctgggc atgcaggtct gtctcctctc actagaatgc magttctgga 60

tggtcagcaa ttttgtttca ttcactgtca tgggctgtga c 101

<210> 779
<211> 101
<212> DNA
<213> Homo sapiens

<400> 779
tctctctcgc tgctatcagg ttgtcagtgt ttgtccttgc tgagccaggt ragcaggctt 60

ctgatgtatt tacgtaggtc aatggtctct aaaattattt g 101

<210> 780
<211> 101
<212> DNA
<213> Homo sapiens

<400> 780
ggcatcacat tagagactcc aaaatcagac tacctacttc aaatattaac kctgtggcct 60

taagatatta aacccttatg tgtctcagtt tcttcatcta t 101

<210> 781
<211> 101
<212> DNA
<213> Homo sapiens

<400> 781
aagacaagca aatttttcat caatgaagtt atacaaatgt gaaacataca raaagatgtt 60

caacactatt cattattgga gaaatgcaaa ttaaaaccac a 101

<210> 782
<211> 101
<212> DNA
<213> Homo sapiens

<400> 782
tatgtgtgag tgtacatata tgttttaaaa atccctagca agagtaagta ygttatttgg 60

tcagtcagct gttaaaactt ccactttctc cagttgtctg g 101

<210> 783
<211> 101
<212> DNA
<213> Homo sapiens

<400> 783
taactggcaa cacatgcact ttcttttgag cttttaaaaa cattgctcca ytgctatcat 60

tgtagacccc caaggagaag gtaccccagc ctcctggaaa c 101

<210> 784
<211> 101
<212> DNA
<213> Homo sapiens

<400> 784
ggtccaaaag ggccacagtt tgctggcaga aaccatacga agtagatttt rttgttaccc 60

ccattttaaa gatgaagaaa ctgagtccca gagaggttca g 101

<210> 785
<211> 101
<212> DNA
<213> Homo sapiens

<400> 785
tctaaccttt ggtgtgcgct gtccctaagg gaggaaggag tgcagctcac maaagccccc 60

ttgaaacaaa ggaaatgtga acgcaacacc aaccactgaa g 101

<210> 786
<211> 101
<212> DNA
<213> Homo sapiens

<400> 786
gcctttatct ctgcttcttc tacccaacag gtgactcctt ttagctaggg yatcacttat 60

acctaacagg ggactcaatt tagccaggat ttcactctgg c 101

<210> 787
<211> 101
<212> DNA
<213> Homo sapiens

<400> 787
atctttccac tggagggaaa ttgggttcat agagtagaaa tactttgccc ragcctcaac 60

agctgctaag aggtgcaatg aaaactcaac ttgaggctgt c 101

<210> 788
<211> 101
<212> DNA
<213> Homo sapiens

<400> 788
ccatcttggc atcattaaaa agggccaacc aagatgttac atgtccacga ygtgacacag 60

gaggaatcaa acagcctgcc tatgaagtag tcttgacaac a 101

<210> 789
<211> 101
<212> DNA
<213> Homo sapiens

<400> 789
tttagctatc tgccatttcc agacacttca tgctctctga gtcttatctt ycactcccag 60

aagattgtca aagtattttc caaaacaaag atagtttccc c 101

<210> 790
<211> 101
<212> DNA
<213> Homo sapiens

<400> 790
aacataactt tggggaatag ctatagatac taaagggggca acataaaaca kttattgatt 60

acaaagtgta tgaagaccca gttgcttggc agagtgatat c 101

<210> 791
<211> 101
<212> DNA
<213> Homo sapiens

<400> 791
aatggggcag gaggtagaat ggcacaggaa ttcaagtaga ggaggattta ycatgaagct 60

aatgaagtct aagtttcagg gcttctcacc tgtgcaggcc a 101

<210> 792
<211> 101
<212> DNA
<213> Homo sapiens

<400> 792
tatagcatct atttataagc cacacacacc atcttatatt aatgcttata ytgtcttggc 60

tcacttagat acaaataaag gtttgcatct gatagaggaa t 101

<210> 793
<211> 101
<212> DNA
<213> Homo sapiens

<400> 793
ttaatggatg aagatatgta gacatctatg gtgttctggg aagctgagca ygtctgatat 60

aaggcatgtg aggtttaaat gcatgcatgt gttagatatg t 101

<210> 794
<211> 101
<212> DNA
<213> Homo sapiens

<400> 794
gccaagttcc caaggtcgca gcaaggtaaa tgggattcca cttgtgttcg raaaatctgt 60

ttataggcct tctcctgaat caaaacacac aggggaaaag c 101

<210> 795
<211> 101
<212> DNA
<213> Homo sapiens

<400> 795
ataagggtga ggctagatct gctatgtccg aaatggcagc cactggatgc rtgactagat 60

ttacattaat tacaatgatt ctaataaaaa atgaagttct c 101

<210> 796
<211> 101
<212> DNA
<213> Homo sapiens

<400> 796
aaccatgcct tgttttgcgt cttctcaaag aaccccgggg gcacgtggcc racaatgtac 60

acctacaagg gaggggttcc cagaagaggc tcacagatgc c 101

<210> 797
<211> 101
<212> DNA
<213> Homo sapiens

<400> 797
aaccccctcc tttctcctgt actgatgact ctgtagcttt aaccagggcg rcggtgtcac 60

tctaaatgtc accttggcat tcagccccat agagtgggga a 101

<210> 798
<211> 101
<212> DNA
<213> Homo sapiens

<400> 798
caagcaaaag aaccttgaat aagccaatat ttcactcata atgtgagtgc raaacatgaa 60

acccaacttt ccgggttcaa atgccaagta cagctagagt c 101

<210> 799
<211> 101
<212> DNA
<213> Homo sapiens

<400> 799
gccctggggc taggataagc ttcttctctg attcaaagaa gcattctcca ragttgcttg 60

ccagatacca ggttctgagc tagttggcct cccaaaaacc c 101

<210> 800
<211> 101
<212> DNA
<213> Homo sapiens

<400> 800
aaatatttat gatgttgtct aaaaatgagt aggtaacaca ctccacatta ycagtacaga 60

gactattcta gcatcaatga atgccaccat agataactat t 101

<210> 801
<211> 101
<212> DNA
<213> Homo sapiens

<400> 801
atggcagtaa gtcataccca aatttggttc acttcactca aatatttgtg rggcacttaa 60

cgattaaagg gtttgtaggt actttgttta atgaataaat t 101

<210> 802
<211> 101
<212> DNA
<213> Homo sapiens

<400> 802
gggactttct ggacactacc acatggagac tgaagatgaa gctaacactt yccagagcaa 60

gctgagtgac agacagaaat caaagcctga tgataccatt t 101

<210> 803
<211> 101
<212> DNA
<213> Homo sapiens

<400> 803
taataatgga ggaaacccgg tgggtgtgag gtatatggga gttttctgga ytctttgcag 60

tttttcagca atctaaaact gttccaaaat aaagtttaga c 101

<210> 804
<211> 101
<212> DNA
<213> Homo sapiens

<400> 804
ttaggtcttc ctaagaatgt atttctgcct cagaatgcac aatgttttca yataaatgtc 60

agtatgatta gggtttatta gcaattgtaa aaaattcaac a 101

<210> 805
<211> 101
<212> DNA
<213> Homo sapiens

<400> 805
tctactccaa cttgttggaa agtagtagta gaatacaaac tagtcaaata maccacgttg 60

tgtaatgaac tgaaacttta acttattttg ttggagtcaa a 101

<210> 806
<211> 101
<212> DNA
<213> Homo sapiens

<400> 806
tgccctggag acgttttccc cattgtcttg gtaactaaca ttcagctccg ygtgcagcac 60

caacttactt atgcaaattt ctgtcactgg tttgaatttc t 101

<210> 807
<211> 101
<212> DNA
<213> Homo sapiens

<400> 807
tcaggtctac tcatctgtaa aatgagaata ataactgcca ccaactccct rgattatgtt 60

gaggatttga ttaggtagtg tttatggagc atggcacgtg t 101

<210> 808
<211> 101
<212> DNA
<213> Homo sapiens

<400> 808

actccacctc tctctgatga gaagaggtaa gtaggattta cagataagca yacgagaagc 60

aggggaagat gctaaggcaa agaaggggcc tgaacaccac c 101

<210> 809
<211> 101
<212> DNA
<213> Homo sapiens

<400> 809
tgtgaaattt caagttttat tcttccattg ttccatttgt gacctaatct rtgaagcctt 60

tcttatcttt tccaagtaaa ggatcactct cttcttactt c 101

<210> 810
<211> 101
<212> DNA
<213> Homo sapiens

<400> 810
acagaggctg tccttaaagg agctgagcct cccttctctc aagggcatct rtgtctgcga 60

atccatccag gctgatgact gtcaacctgg ggctttttgt t 101

<210> 811
<211> 101
<212> DNA
<213> Homo sapiens

<400> 811
tcttctctga aagctgaatt aactagtcag gaatcgcaga tctccactta ygaagaagaa 60

ttggcaaaag ctagagaaga gctgagccgt ctacagcaag a 101

<210> 812
<211> 101
<212> DNA
<213> Homo sapiens

<400> 812
tggcaggagt gatgctggcc taatgacaac ctcagccaat gaccaacctc rtggagatac 60

tttagagcta aaaccgtatc ttaatgttgt catgcattgg c 101

<210> 813
<211> 101
<212> DNA
<213> Homo sapiens

<400> 813
ggcagaccat aatgattcaa ccacttggat tctacaaaca atactttaac rtggaaatgt 60

gtacttggat gaagaagaga gaaggcaatg cctgattttt c 101

<210> 814
<211> 101
<212> DNA
<213> Homo sapiens

<400> 814
agcactgaag aggtctttgc tgactctggc tcaggaatta gaagtttctc rgcaagggcc 60

atttaaattg ggccttgatg gatgtatagg agctcaataa g 101

<210> 815
<211> 101
<212> DNA
<213> Homo sapiens

<400> 815
agttacaaga gctcactgac caacacaaac tcaggttagc agagcttttc ygaaattcca 60

ccctttcct ctgcgttcag tgctacttac tcccttcact t 101

<210> 816
<211> 101
<212> DNA
<213> Homo sapiens

<400> 816
gctccgctat tcagtttcag gtagggacat agagtcttag agagggtgag rcatttatac 60

aaggctacat agcaagtaga aggcaaaacc aggacaggat c 101

<210> 817
<211> 101
<212> DNA
<213> Homo sapiens

<400> 817
ctttactgag ctcctattag atgctttgca tgaggtattt caatttaatg ygtaggaact 60

gtggcttatt tgacttgtta cacccaacag cgcctggcac c 101

<210> 818
<211> 101
<212> DNA
<213> Homo sapiens

<400> 818
cagtgctccc agtggtgggt acctacccca gagaactctc acatgtatca raagtgggca 60

tgtatacagt tcagaactgt ccatcatggt cagagttgaa g 101

<210> 819
<211> 101
<212> DNA
<213> Homo sapiens

<400> 819
tgcaaactgg gaagctgagg gtgcccatgt tttctgttat gtggactagg ragcagagaa 60

tgccacccac aaggaaagag aaaactcaag catatgttca g 101

<210> 820
<211> 101
<212> DNA
<213> Homo sapiens

<400> 820
caccagcctc attggccttg tccctgaatc ttacacacct aaatgcaaac rcaccttcca 60

attatctgct tgttcttctt tttatccact tctttgtctc c 101

<210> 821
<211> 101
<212> DNA
<213> Homo sapiens

<400> 821
cttacatttc atttctttca ttcttattcc ttaatagata tgtttgttct ytctgcctgt 60

tctttttctt attcctccat ctttgcttgt ctatcccacc c 101

<210> 822
<211> 101
<212> DNA
<213> Homo sapiens

<220>
<221> allele
<222> (51)..(51)

<400> 822
tcagagaccc cagggaattc acatgtgtat tgctttgtaa atgttctgac ratgctctca 60

ggtgccaggg ctgtgtcttc agcatcagtt tgggagttcc t 101

<210> 823
<211> 101
<212> DNA
<213> Homo sapiens

<400> 823
ataaaacact ctctgaagaa aatacatttc tctatatcta tgtcttgtta yttacttata 60

catctataaa ttttttgatt aacctatgtt ctttgttttc t 101

<210> 824
<211> 101
<212> DNA
<213> Homo sapiens

<400> 824
tgacttccag aactagtaat ttggtgtcgg agaactacgt gtatatggca mctttgtaca 60

ttcattaagt tggcacaatt tgaccttttg ttgcatgaca g 101

<210> 825
<211> 101
<212> DNA
<213> Homo sapiens

<400> 825
cagatttcac taacatagga gattgtactg atttgcactc tcactagcca yatatgagca 60

tgtcaatttc tcctacatct caccaacaca gtatattagc a 101

<210> 826
<211> 101
<212> DNA
<213> Homo sapiens

<400> 826
ccaagaaagt ctctagaaga agggtgacat aaattgcaag gacgtgggtt rgtgtgaggt 60

tttcatgact tcatctgcca cccaaattat ttattttcca t 101

<210> 827
<211> 101
<212> DNA
<213> Homo sapiens

<400> 827
tacggttttt tgcctttttc tatttgattt agaatggata caagcggcct ytaggactga 60

ttcatggaag gtcaggcaga gtctccctgg atgcctggaa a 101

<210> 828
<211> 101
<212> DNA
<213> Homo sapiens

<400> 828
tcaagaaagt catttcctaa tgaaaaatgg ttctggtcat cttccctctg kctgtatgtg 60

ttcatcaaca gagtttgact tcagattaat tgagattaaa g 101

<210> 829
<211> 101
<212> DNA
<213> Homo sapiens

<400> 829
gtttctggaa gagattcaca tttgcatcag taggctgagt aaagaagatc yacctcacca 60

atgtgggcca acagcatcca atccaaatag aataaaaagg a 101

<210> 830
<211> 101
<212> DNA
<213> Homo sapiens

<400> 830
cgagttttgc gtttagcaga tggacctgac gaagttcatc tttcagcaat ygcaacaatg 60

gagctgcggg accaagccaa aagactgaca gccaagatat a 101

<210> 831
<211> 101
<212> DNA
<213> Homo sapiens

<400> 831
ataaaatctg gcttacagaa aaacaggcaa aatacctttg gcccagtgaa ygaattgtcc 60

ttggtttcca tgttaatacc cagaggccag tactgggcgt t 101

<210> 832
<211> 101
<212> DNA
<213> Homo sapiens

<400> 832
gttaatgaaa agtaaggcta ccaaatatat caagctatat ctgcttggac rtagctttgt 60

ttactttgtg acaggaattt cttagagtca gcctccagta a 101

<210> 833
<211> 101
<212> DNA
<213> Homo sapiens

<400> 833
aaaaggcccc cccaaaagta gatacaaggc aaagtctgtc tcatgattct kctgtagaaa 60

agaaacatta aaaagtatga gcaacattaa tagcagatgg c 101

<210> 834
<211> 101
<212> DNA
<213> Homo sapiens

<400> 834
tgggagcaaa gggaagcact gaagcaaacc acatgaagta agagcaggaa rtagctctca 60

cccccaggac tgggaaagaa agggaagata atagagtaac c 101

<210> 835
<211> 101
<212> DNA
<213> Homo sapiens

<400> 835
tgcaaatagc aggggaacat cctctagtta ttttaataat tcaagggagg kacaaggttt 60

tccacttttt ctcccctgca aagcactctc taaattgcag c 101

<210> 836
<211> 101
<212> DNA
<213> Homo sapiens

<400> 836
cttttgaata actggtcgaa ctcatagcaa ggggagctga gcccacttaa yccagaatga 60

cagaaagaag ctaagcctgt ttatttgatt gcagatctcg c 101

<210> 837
<211> 101
<212> DNA
<213> Homo sapiens

<400> 837
ggtttccttt ctgggagaat ggggctggtg acaattaatg cagaaaactt ytattggaga 60

tagacatgca aaaacactca gagatagagt tgaggaattt g 101

<210> 838
<211> 101
<212> DNA
<213> Homo sapiens

<400> 838
ctgtgtgccc atggggttct tcgtgaagag tttcatggtt gctgcttcag ractactgag 60

atttgggaat tctcctttct tgttctttta aaataagcaa t 101

<210> 839
<211> 101
<212> DNA
<213> Homo sapiens

<400> 839
tttgttaatc tcagaaaaga atgtatcatt ttaagaaata cactaaaagg rccagtcgta 60

agcaaagagc tctgttattt agtaagtaga aagtgtagat a 101

<210> 840
<211> 101
<212> DNA
<213> Homo sapiens

<400> 840
gaaagcagag gtagagtggg aaggactttt tattgtgctt tgggtaccag ytcagctgca 60

gtagaataga gcaccaggta gctttctaag gttcctgact c 101

<210> 841
<211> 101
<212> DNA
<213> Homo sapiens

<400> 841
tgtggtgaat aatgacgaaa taacaaagca ataaaagcag cagcagcatt rtatgtattt 60

tgttttcgat atgcaaatga agggcagact tatttttgtc a 101

<210> 842
<211> 101
<212> DNA
<213> Homo sapiens

<400> 842
agtgatttgt cctcaatctc acccagaaga gagtaagaac tgacctcata matttaagga 60

acaaagtggg acgaaggaag cctgatgtga ctggggttgg g 101

<210> 843
<211> 101
<212> DNA
<213> Homo sapiens

<400> 843

gtgggctgta atctttgaat ctagacttta tctttgcagt tccagtcttt mggcaaccgt 60

ccaggaggac aagactcatt cagtgaaagt ggcttcaatt t 101

<210> 844
<211> 101
<212> DNA
<213> Homo sapiens

<400> 844
acatagtcca agtcaccttc gtctctaccc tggactacac tataacctcc yaattagtct 60

tcctacagcc ccttatcccc ctcagtattt tcttttgctg c 101

<210> 845
<211> 101
<212> DNA
<213> Homo sapiens

<400> 845
ctgttggatc atggcccata cctcagcagt gccatatcca attaggatgc rtcattacat 60

tatggttcgc aacagaaaaa ctgtctccac caattggaag g 101

<210> 846
<211> 101
<212> DNA
<213> Homo sapiens

<400> 846
atgtggaaca gagtactcct ttatttgtgc catgattagg ccaactctga rgtgaagaag 60

aggaatagaa aaaagcaaaa gttagaagca tttaatgaaa t 101

<210> 847
<211> 101
<212> DNA
<213> Homo sapiens

<400> 847
ttggaagagt acaaatttaa aggagccaat gaggtgcata catgagaagg ragccttgca 60

aaaatatact agtttattac tgtgggtgaa gacaaatgtt a 101

<210> 848
<211> 101
<212> DNA
<213> Homo sapiens

<400> 848
atagaagaag cagcggagac tactgctgct aatgactgta gacatgttga rcattagaat 60

ttgtagcagg agttgccctg ccatagcaag aaatgacaat g 101

<210> 849
<211> 101
<212> DNA
<213> Homo sapiens

<400> 849
ttttgttatt taaatcccct ataattatat ttttaatttt ctcgatttga ytatttctga 60

gaacagagtt ggggtaggca gaataatgat ccaccaaaga c 101

<210> 850
<211> 52
<212> DNA
<213> Homo sapiens

<400> 850
gagagcatca tctgcggcat cacgtcygtg gccttctccc tcagtggccg cc 52

<210> 851
<211> 52
<212> DNA
<213> Homo sapiens

<400> 851
cgccgccagg cgcacggcgt aggggarcct cgcaggcggc ggcggcggcg gc 52

<210> 852
<211> 52
<212> DNA
<213> Homo sapiens

<400> 852
gctctcgccg ccaggcgcac ggcgtarggg agcctcgcag gcggcggcgg cg 52

<210> 853
<211> 52
<212> DNA
<213> Homo sapiens

<400> 853
gagaaccagt tcagagtgga ctacatyctg agtgtgatga acgtgcctga ct 52

<210> 854
<211> 52
<212> DNA
<213> Homo sapiens

<400> 854
atctgcagct taagccagtg acacaayatt ttgcattttt aaatggtgat tc 52

<210> 855
<211> 52
<212> DNA
<213> Homo sapiens

<400> 855
ctggtcttct cggtgcgcag cccctcrtgg gtgctcaact tcctgctgca ga 52

<210> 856
<211> 52
<212> DNA
<213> Homo sapiens

<400> 856
ttagcaccca gggtcacatc ccagttyaaa aatatcccat ggagtgcagt ca 52

<210> 857
<211> 52
<212> DNA
<213> Homo sapiens

<400> 857
tgttcatcta ttcaaaatgt agtatarttt tatttgagat tgtctttttt ta 52

<210> 858
<211> 52
<212> DNA
<213> Homo sapiens

<400> 858
gcgtccgcag agcccgcggg ggccgkycca gcccgggagc cgcgcgggcg ag 52